# EUROPEAN PATENT SPECIFICATION

(11) **EP 2 942 403 B1**
(45) Date of publication and mention of the grant of the patent: **12.04.2017**
(21) Application number: 15168304.2
(22) Date of filing: 01.12.2011
(51) Int. Cl.: C12Q 1/68

(54) **METHOD FOR PREDICTING THE OUTCOME OF A CANCER BY ANALYSING MIRNA EXPRESSION**
VERFAHREN ZUR ERGEBNISPROGNOSE EINES TUMORS MITTELS ANALYSE VON MIRNA-EXPRESSION
PROCÉDÉ DE PRÉVISION DU RÉSULTAT D'UN CANCER PAR L'ANALYSE DE L'EXPRESSION DU MIARN

(30) Priority: 01.12.2010 EP 10306329; 20.01.2011 US 201161434498 P
(43) Date of publication of application: 11.11.2015
(62) Divisional of application: 11790625.5
(73) Proprietor: INSERM (Institut National de la Santé et de la Recherche Médicale), 75013 Paris (FR); Université Paris Descartes, 75006 Paris (FR); ASSISTANCE PUBLIQUE-HOPITAUX DE PARIS, 75004 Paris (FR)
(72) Inventor: Galon, Jérôme, 75006 Paris (FR); Pages, Franck, 75006 Paris (FR); Fridman, Hervé, 75006 Paris (FR); Mlecnik, Bernhard, 75006 Paris (FR)
(74) Representative: Cabinet Plasseraud

(56) References cited:
- WO-A2-2006/137941
- WO-A2-2008/008430
- WO-A2-2009/008720
- WO-A2-2009/111643
- WO-A2-2009/140670
- WO-A2-2010/058393
- DÍAZ RAQUEL ET AL: "Deregulated expression of miR-106a predicts survival in human colon cancer patients.", GENES, CHROMOSOMES & CANCER SEP 2008 LNKD- PUBMED:18521848, vol. 47, no. 9, September 2008 (2008-09), pages 794-802, XP002629635, ISSN: 1098-2264
- SCHETTER AARON J ET AL: "MicroRNA expression profiles associated with prognosis and therapeutic outcome in colon adenocarcinoma", JAMA THE JOURNAL OF THE AMERICAN MEDICAL ASSOCIATION, AMERICAN MEDICAL ASSOCIATION, US, vol. 299, no. 4, 30 January 2008 (2008-01-30), pages 425-436, XP002529936, ISSN: 0098-7484, DOI: DOI:10.1001/JAMA.299.4.425
- LIU M ET AL: "The role of microRNAs in colorectal cancer", JOURNAL OF GENETICS AND GENOMICS, ELSEVIER BV, NL; CN, vol. 37, no. 6, 1 June 2010 (2010-06-01), pages 347-358, XP027119586, ISSN: 1673-8527 [retrieved on 2010-06-01]
- SCHEPELER TROELS ET AL: "Diagnostic and prognostic microRNAs in stage II colon cancer", CANCER RESEARCH, AMERICAN ASSOCIATION FOR CANCER RESEARCH, US, vol. 68, no. 15, 1 August 2008 (2008-08-01), pages 6416-6424, XP002529939, ISSN: 0008-5472, DOI: 10.1158/0008-5472.CAN-07-6110
- CUMMINS JORDAN M ET AL: "The colorectal microRNAome", PROCEEDINGS OF THE NATIONAL ACADEMY OF SCIENCES OF THE UNITED STATES (PNAS), NATIONAL ACADEMY OF SCIENCE, US, vol. 103, no. 10, 7 March 2006 (2006-03-07), pages 3687-3692, XP002539256, ISSN: 0027-8424, DOI: DOI:10.1073/PNAS.0511155103
- ZHAOHUI HUANG ET AL: "Plasma microRNAs are promising novel biomarkers for early detection of colorectal cancer", INTERNATIONAL JOURNAL OF CANCER, vol. 127, no. 1, 28 October 2009 (2009-10-28), pages 118-126, XP055024209, ISSN: 0020-7136, DOI: 10.1002/ijc.25007
- BANDRÉS E ET AL: "Identification by Real-time PCR of 13 mature microRNAs differentially expressed in colorectal cancer and non-tumoral tissues", MOLECULAR CANCER, BIOMED CENTRAL, LONDON, GB, vol. 5, no. 1, 19 July 2006 (2006-07-19), page 29, XP021018291, ISSN: 1476-4598, DOI: 10.1186/1476-4598-5-29
- HOL L ET AL: "W1985 MicroRNA Expression Profiling of Colorectal Cancer and Its Precancerous Lesions Using Lna(TM) Oligonucleotide Arrays", GASTROENTEROLOGY, ELSEVIER, PHILADELPHIA, PA, vol. 134, no. 4, 1 April 2008 (2008-04-01) , pages A-748, XP023435289, ISSN: 0016-5085, DOI: 10.1016/S0016-5085(08)63492-7 [retrieved on 2008-04-01]

## Description

### FIELD OF THE INVENTION:

The present invention relates to a method for predicting the outcome of a cancer.

### BACKGROUND OF THE INVENTION:

Cancer remains a serious public health problem in developed countries. Accordingly, to be most effective, cancer treatment requires not only early detection and treatment or removal of the malignancy, but a reliable assessment of the severity of the malignancy and a prediction of the likelihood of cancer recurrence. The stage of a cancer indicates how far a cancer has spread and as consequences what the oucome of the canser will be. Staging is important because treatment is often decided according to the stage of a cancer. To date, cancers are generally classified according to the UICC-TNM system. The TNM (for "Tumor-Node-Metastasis") classification system uses the size of the tumor, the presence or absence of tumor in regional lymph nodes, and the presence or absence of distant metastases, to assign a stage and an outcome to the tumor. The TNM system developed from the observation that patients with small tumours have better prognosis than those with tumours of greater size at the primary site. In general, patients with tumours confined to the primary site have better prognosis than those with regional lymph node involvement, which in turn is better than for those with distant spread of disease to other organs. Accordingly, cancer can be generally divided into four stages. Stage I is very localized cancer with no cancer in the lymph nodes. Stage II cancer has spread to the lymph nodes. Stage III cancer has spread near to where the cancer started. Stage IV cancer has spread to another part of the body. The assigned stage is used as a basis for selection of appropriate therapy and for prognostic purposes.

The above clinical classifications, although they are to be useful, are imperfect and do not allow a reliable prognosis of the outcome of the cancers. Recently, a study has suggested that density, and location of immune cells in colorectal cancer had a prognostic value that was superior to and independent of those of the UICC-TNM classification (Science. 2006 Sep 29;313(5795):1960-4). However there is still a need for other reliable methods that will help physicians for predicting the outcome of a cancer in a patient.

miRNAs have been suggested to be helpful for the diagnosis and predicting the outcome of a cancer. For example Cummins et al. (2006) describe miRNAs that are differentially expressed in colon cancer and some that are associated with clinical outcome (Cummins et al, Proc. Natl. Acad. Sci. USA, 103 (10):3687-3692, 2006). Michael et al. (2003) describe miRNAs that are down-regulated in colorectal adenocarcinomas as compared to matched normal tissues, in particular miR.143 and miR145 (Michael et al, Mol. Cancer. Res., 1:882-891, 2003). Bandres et al. (2006) describe miRNA in paired colorectal tumor and normal adjacent tissue and reported the differential expression of miR.31 in Stage II and Stage IV colorectal cancer cells (Bandres et al, Molec. Cancer, 5:29, 2006. Xi et al. (2006a, 2006b)), evaluated the prognostic value of several miRNAs in colorectal cancer and showed that higher expression of miR.200c was associated with shorter survival time (Xi et al, BiomarkInsights, 2:113-121, 2006a. Xi et al, Clin Cancer Res., 12:2014-2024, 2006b). Schetter et al., (2008) reported that a high expression of miR.21 is associated with a poor survival and poor therapeutic response in colorectal cancer patients (Schetter et al, JAMA, 299(4):425-436, 2008).

However, there is no current method based on miRNAs that allow having a prognostic value that is superior to and independent of those of the UICC-TNM classification.

### SUMMARY OF THE INVENTION:

The present invention relates to a method for predicting the outcome of a cancer in a patient comprising a step consisting of determining the expression level of a miRNA cluster in a sample obtained from said patient, wherein said miRNA cluster comprises:
- miR.609 or,
- miR.518c or,
- miR.520f or,
- miR.220a or,
- miR.362 or,
- miR.29a or,
- miR.660 or,
- miR.603 or,
- miR.558 or,
- miR519b or,
- miR.494 or,
- miR.130a or,
- miR.639.

### DETAILED DESCRIPTION OF THE INVENTION:

From a cohort of 77 Stage I/II/III patients the inventors have determined various miRNA clusters that can be suitable for predicting the outcome of a cancer in a patient (Table 1 to Table 17). They demonstrated, by determining the ROC curves for each of said clusters that most of them provide greater sensitivities and selectivities than the UICC-TNM classification does for predicting the outcome of a cancer.

Accordingly, the invention relates to methods for predicting the outcome of a cancer in a patient comprising a step consisting of determining the expression level of said miRNA clusters in a sample obtained from said patient.

The term "miRNAs" refers to microRNA molecules that are generally 21 to 22 nucleotides in length, even though lengths of 19 and up to 23 nucleotides have been reported. miRNAs are each processed from a longer precursor RNA molecule ("precursor miRNA"). Precursor miRNAs are transcribed from non-protein-encoding genes. The precursor miRNAs have two regions of complementarity that enables them to form a stem-loop- or fold-back-like structure, which is cleaved in animals by a ribonuclease Ill-like nuclease enzyme called Dicer. The processed miRNA is typically a portion of the stem. The processed miRNA (also referred to as "mature miRNA") become part of a large complex to down-regulate a particular target gene. All the miRNAs pertaining to the invention are known per se and sequences of them are publicly available from the data base http://microrna.sanger.ac.uk/sequences/. The miRNAs of the invention are listed in Table A:

**Table A: list of the miRNAs according to the invention**

| **miRNA** | **miRBase** |
|---|---|
| miR.609 | MI0003622 |
| miR.519b | MI0003151 |
| miR.520f | MI0003146 |
| miR.558 | MI0003564 |
| miR.603 | MI0003616 |
| miR.220a | MI0000297 |
| miR.376a* | MI0000784 |
| miR.639 | MI0003654 |
| miR.130a | MI0000448 |
| miR.338 | MI0000814 |
| miR.26a | MI0000083 |
| miR.29a | MI0000087 |
| miR.494 | MI0003134 |
| miR.518c | MI0003159 |
| miR.660 | MI0003684 |
| miR.369-3p | MI0000777 |
| miR.362 | MI0000762 |

The term "miRNA cluster" refers to a set of at least one miRNA selected from the group consisting in the miRNAs of Table A. Accordingly, said miRNA cluster may comprise 1, 2, 3, 4, 5, 6, 7, 8, 9, 10, 11, 12, 13, 14, 15, 16, or 17 miRNAs of Table A.

As used herein, the term "patient" denotes a mammal. In a preferred embodiment of the invention, a patient according to the invention is a human.

As used herein, the term "sample" refers to a sample that contains nucleic acid materials.

As used herein, the term "nucleic acid" is meant a polymeric compound comprising nucleoside or nucleoside analogs which have nitrogenous heterocyclic bases, or base analogs, linked together by nucleic acid backbone linkages (e.g., phosphodiester bonds) to form a polynucleotide. Conventional RNA and DNA are included in the term "nucleic acid" as are analogs thereof. The nucleic acid backbone may include a variety of linkages, for example, one or more of sugar-phosphodiester linkages, peptide-nucleic acid bonds, phosphorothioate or methylphosphonate linkages or mixtures of such linkages in a single oligonucleotide. Sugar moieties in the nucleic acid may be either ribose or deoxyribose, or similar compounds with known substitutions. Conventional nitrogenous bases (A, G, C, T, U), known base analogs (eg inosine), derivatives of purine or pyrimidine bases and "abasic" residues (i.e., no nitrogenous base for one or more backbone positions) are included in the term nucleic acid. That is, a nucleic acid may comprise only conventional sugars, bases and linkages found in RNA and DNA, or may include both conventional components and substitutions (e.g., conventional bases and analogs linked via a methoxy backbone, or conventional bases and one or more base analogs linked via an RNA or DNA backbone).

Typically the "sample" means any tissue sample derived from the patient. Said tissue sample is obtained for the purpose of the in vitro evaluation. The sample can be fresh, frozen, fixed (e.g., formalin fixed), or embedded (e.g., paraffin embedded). In a particular embodiment the sample results from biopsy performed in the tumour sample of the patient. For example an endoscopical biopsy performed in the bowel of the patient affected by a colorectal cancer.

### miRNA clusters based on miR.609:

An aspect of the invention relates to a method for predicting the outcome of a cancer in a patient comprising a step consisting of determining the expression level of a miRNA cluster in a sample obtained from said patient, wherein said miRNA cluster comprises miR.609.

The inventors have been indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.519b, miR.520f, miR.558, miR.603, miR.220a, miR.376a*, miR.639, miR.130a, miR.338, miR.26a, miR.29a, miR.494, miR.518c, miR.660, miR.369-3p and miR.362.

In a particular embodiment, the miRNA cluster consists in the combination of 2 miRNAs. For example, said miRNA cluster may be selected according to any combination described in Table 2 which comprises miR.609. In a particular embodiment, the miRNA cluster consists in the combination miR.609 + miR.29a.

In a particular embodiment, the miRNA cluster consists in the combination of 3 miRNAs. For example, said miRNA cluster may be selected according to any combination described in Table 3 which comprises miR.609. In a particular embodiment, the miRNA cluster consists in the combination miR.558 + miR.609 + miR.518c.

In a particular embodiment, the miRNA cluster consists in the combination of 4 miRNAs. For example, said miRNA cluster may be selected according to any combination described in Table 4 which comprises miR.609. In a particular embodiment, the miRNA cluster consists in the combination miR.609 + miR.29a + miR.376a* + miR.518c.

In a particular embodiment, the miRNA cluster consists in the combination of 5 miRNAs. For example, said miRNA cluster may be selected according to any combination described in Table 5 which comprises miR.609. In a particular embodiment, the miRNA cluster consists in the combination miR.558 + miR.609 + miR.29a + miR.376a* + miR.518c.

In a particular embodiment, the miRNA cluster consists of in the combination of 6 miRNAs. For example, said miRNA cluster may be selected according to any combination described in Table 6 which comprises miR.609. In a particular embodiment, the miRNA cluster consists in the combination miR.558 + miR.609 + miR.220a + miR.29a + miR.376a* + miR.518c.

In a particular embodiment, the miRNA cluster consists in the combination of 7 miRNAs. For example, said miRNA cluster may be selected according to any combination described in Table 7 which comprises miR.609. In a particular embodiment, the miRNA cluster consists in the combination miR.558 + miR.609 + miR.220a + miR.29a + miR.369.3p + miR.376a* + miR.518c.

In a particular embodiment, the miRNA cluster consists in the combination of 8 miRNAs. For example, said miRNA cluster may be selected according to any combination described in Table 8 which comprises miR.609. In a particular embodiment, the miRNA cluster consists in the combination miR.558 + miR.609 + miR.603 + miR.220a + miR.29a + miR.369.3p + miR.376a* + miR.518c.

In a particular embodiment, the miRNA cluster consists in the combination of 9 miRNAs. For example, said miRNA cluster may be selected according to any combination described in Table 9 which comprises miR.609. In a particular embodiment, the miRNA cluster consists in the combination miR.558 + miR.609 + miR.603 + miR.220a + miR.26a + miR.29a + miR.369.3p + miR.376a* + miR.518c.

In a particular embodiment, the miRNA cluster consists in the combination of 10 miRNAs. For example, said miRNA cluster may be selected according to any combination described in Table 10 which comprises miR.609. In a particular embodiment, the miRNA cluster consists in the combination miR.558 + miR.609 + miR.603 + miR.220a + miR.26a + miR.29a + miR.369.3p + miR.376a* + miR.518c + miR.660.

In a particular embodiment, the miRNA cluster consists in the combination of 11 miRNAs. For example, said miRNA cluster may be selected according to any combination described in Table 11 which comprises miR.609. In a particular embodiment, the miRNA cluster consists in the combination miR.558 + miR.609 + miR.520f + miR.29a + miR.362 + miR.369.3p + miR.376a* + miR.518c + miR.639 + miR.660 + miR.338.

In a particular embodiment, the miRNA cluster consists in the combination of 12 miRNAs. For example, said miRNA cluster may be selected according to any combination described in Table 12 which comprises miR.609. In a particular embodiment, the miRNA cluster consists in the combination miR.558 + miR.609 + miR.520f + miR.220a + miR.29a + miR.362 + miR.369.3p + miR.376a* + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.609. In a particular embodiment, the miRNA cluster consists in the combination miR.558 + miR.609 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.609. In a particular embodiment, the miRNA cluster consists in the combination miR.558 + miR.609 + miR.603 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.609. In a particular embodiment, the miRNA cluster consists in the combination miR.558 + miR.609 + miR.603 + miR.520f + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.609. In a particular embodiment, the miRNA cluster consists in the combination miR.558 + miR.609 + miR.603 + miR.520f + miR.220a + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a*+ miR.494 + miR.518c + miR.639 + miR.660 + miR.338.

### miRNA clusters based on miR.558:

A further aspect of the invention relates to a method for predicting the outcome of a cancer in a patient comprising a step consisting of determining the expression level of a miRNA cluster in a sample obtained from said patient, wherein said miRNA cluster comprises miR.558.

The inventors have been indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.519b, miR.520f, miR.609, miR.603, miR.220a, miR.376a*, miR.639, miR.130a, miR.338, miR.26a, miR.29a, miR.494, miR.518c, miR.660, miR.369-3p and miR.362.

In a particular embodiment, the miRNA cluster consists in the combination of 2 miRNAs. For example, said miRNA cluster may be selected according to any combination described in Table 2 which comprises miR.558. In a particular embodiment, the miRNA cluster consists in the combination miR.558 + miR.518c.

In a particular embodiment, the miRNA cluster consists in the combination of 3 miRNAs. For example, said miRNA cluster may be selected according to any combination described in Table 3 which comprises miR.558. In a particular embodiment, the miRNA cluster consists in the combination miR.558 + miR.609 + miR.518c.

In a particular embodiment, the miRNA cluster consists in the combination of 4 miRNAs. For example, said miRNA cluster may be selected according to any combination described in Table 4 which comprises miR.558. In a particular embodiment, the miRNA cluster consists in the combination miR.558 + miR.609 + miR.29a + miR.518c.

In a particular embodiment, the miRNA cluster consists in the combination of 5 miRNAs. For example, said miRNA cluster may be selected according to any combination described in Table 5 which comprises miR.558. In a particular embodiment, the miRNA cluster consists in the combination miR.558 + miR.609 + miR.29a + miR.376a* + miR.518c.

In a particular embodiment, the miRNA cluster consists of in the combination of 6 miRNAs. For example, said miRNA cluster may be selected according to any combination described in Table 6 which comprises miR.558. In a particular embodiment, the miRNA cluster consists in the combination miR.558 + miR.609 + miR.220a + miR.29a + miR.376a* + miR.518c.

In a particular embodiment, the miRNA cluster consists in the combination of 7 miRNAs. For example, said miRNA cluster may be selected according to any combination described in Table 7 which comprises miR.558. In a particular embodiment, the miRNA cluster consists in the combination miR.558 + miR.609 + miR.220a + miR.29a + miR.369.3p + miR.376a* + miR.518c.

In a particular embodiment, the miRNA cluster consists in the combination of 8 miRNAs. For example, said miRNA cluster may be selected according to any combination described in Table 8 which comprises miR.558. In a particular embodiment, the miRNA cluster consists in the combination miR.558 + miR.609 + miR.603 + miR.220a + miR.29a + miR.369.3p + miR.376a* + miR.518c.

In a particular embodiment, the miRNA cluster consists in the combination of 9 miRNAs. For example, said miRNA cluster may be selected according to any combination described in Table 9 which comprises miR.558. In a particular embodiment, the miRNA cluster consists in the combination miR.558 + miR.609 + miR.603 + miR.220a + miR.26a + miR.29a + miR.369.3p + miR.376a* + miR.518c.

In a particular embodiment, the miRNA cluster consists in the combination of 10 miRNAs. For example, said miRNA cluster may be selected according to any combination described in Table 10 which comprises miR.558. In a particular embodiment, the miRNA cluster consists in the combination miR.558 + miR.609 + miR.603 + miR.220a + miR.26a + miR.29a + miR.369.3p + miR.376a* + miR.518c + miR.660.

In a particular embodiment, the miRNA cluster consists in the combination of 11 miRNAs. For example, said miRNA cluster may be selected according to any combination described in Table 11 which comprises miR.558. In a particular embodiment, the miRNA cluster consists in the combination miR.558 + miR.609 + miR.520f + miR.29a + miR.362 + miR.369.3p + miR.376a* + miR.518c + miR.639 + miR.660 + miR.338.

In a particular embodiment, the miRNA cluster consists in the combination of 12 miRNAs. For example, said miRNA cluster may be selected according to any combination described in Table 12 which comprises miR.558. In a particular embodiment, the miRNA cluster consists in the combination miR.558 + miR.609 + miR.520f + miR.220a + miR.29a + miR.362 + miR.369.3p + miR.376a* + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.558. In a particular embodiment, the miRNA cluster consists in the combination miR.558 + miR.609 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.558. In a particular embodiment, the miRNA cluster consists in the combination miR.558 + miR.609 + miR.603 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.558. In a particular embodiment, the miRNA cluster consists in the combination miR.558 + miR.609 + miR.603 + miR.520f + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.558. In a particular embodiment, the miRNA cluster consists in the combination miR.558 + miR.609 + miR.603 + miR.520f + miR.220a + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338.

### miRNA clusters based on miR.603:

A further aspect of the invention relates to a method for predicting the outcome of a cancer in a patient comprising a step consisting of determining the expression level of a miRNA cluster in a sample obtained from said patient, wherein said miRNA cluster comprises miR.603.

The inventors have been indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.519b, miR.520f, miR.609, miR.558, miR.220a, miR.376a*, miR.639, miR.130a, miR.338, miR.26a, miR.29a, miR.494, miR.518c, miR.660, miR.369-3p and miR.362.

In a particular embodiment, the miRNA cluster consists in the combination of 2 miRNAs. For example, said miRNA cluster may be selected according to any combination described in Table 2 which comprises miR.603. In a particular embodiment, the miRNA cluster consists in the combination miR.603 + miR.518c.

In a particular embodiment, the miRNA cluster consists in the combination of 3 miRNAs. For example, said miRNA cluster may be selected according to any combination described in Table 3 which comprises miR.603. In a particular embodiment, the miRNA cluster consists in the combination miR.558 + miR.603 + miR.518c.

In a particular embodiment, the miRNA cluster consists in the combination of 4 miRNAs. For example, said miRNA cluster may be selected according to any combination described in Table 4 which comprises miR.603. In a particular embodiment, the miRNA cluster consists in the combination miR.558 + miR.609 + miR.603 + miR.518c.

In a particular embodiment, the miRNA cluster consists in the combination of 5 miRNAs. For example, said miRNA cluster may be selected according to any combination described in Table 5 which comprises miR.603. In a particular embodiment, the miRNA cluster consists in the combination miR.609 + miR.603 + miR.29a + miR.376a* + miR.518c.

In a particular embodiment, the miRNA cluster consists of in the combination of 6 miRNAs. For example, said miRNA cluster may be selected according to any combination described in Table 6 which comprises miR.603. In a particular embodiment, the miRNA cluster consists in the combination miR.558 + miR.609 + miR.603 + miR.29a + miR.376a* + miR.518c.

In a particular embodiment, the miRNA cluster consists in the combination of 7 miRNAs. For example, said miRNA cluster may be selected according to any combination described in Table 7 which comprises miR.603. In a particular embodiment, the miRNA cluster consists in the combination miR.558 + miR.609 + miR.603 + miR.220a + miR.29a + miR.376a* + miR.518c.

In a particular embodiment, the miRNA cluster consists in the combination of 8 miRNAs. For example, said miRNA cluster may be selected according to any combination described in Table 8 which comprises miR.603. In a particular embodiment, the miRNA cluster consists in the combination miR.558 + miR.609 + miR.603 + miR.220a + miR.29a + miR.369.3p + miR.376a* + miR.518c.

In a particular embodiment, the miRNA cluster consists in the combination of 9 miRNAs. For example, said miRNA cluster may be selected according to any combination described in Table 9 which comprises miR.603. In a particular embodiment, the miRNA cluster consists in the combination miR.558 + miR.609 + miR.603 + miR.220a + miR.26a + miR.29a + miR.369.3p + miR.376a* + miR.518c.

In a particular embodiment, the miRNA cluster consists in the combination of 10 miRNAs. For example, said miRNA cluster may be selected according to any combination described in Table 10 which comprises miR.603. In a particular embodiment, the miRNA cluster consists in the combination miR.558 + miR.609 + miR.603 + miR.220a + miR.26a + miR.29a + miR.369.3p + miR.376a* + miR.518c + miR.660.

In a particular embodiment, the miRNA cluster consists in the combination of 11 miRNAs. For example, said miRNA cluster may be selected according to any combination described in Table 11 which comprises miR.603. In a particular embodiment, the miRNA cluster consists in the combination miR.558 + miR.609 + miR.603 + miR.220a + miR.26a + miR.29a + miR.369.3p + miR.376a* + miR.518c + miR.660 + miR.338.

In a particular embodiment, the miRNA cluster consists in the combination of 12 miRNAs. For example, said miRNA cluster may be selected according to any combination described in Table 12 which comprises miR.603. In a particular embodiment, the miRNA cluster consists in the combination miR.558 + miR.609 + miR.603 + miR.520f + miR.29a + miR.362 + miR.369.3p + miR.376a* + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.603. In a particular embodiment, the miRNA cluster consists in the combination miR.558 + miR.609 + miR.603 + miR.520f + miR.220a + miR.29a + miR.362 + miR.369.3p + miR.376a* + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.603. In a particular embodiment, the miRNA cluster consists in the combination miR.558 + miR.609 + miR.603 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.603. In a particular embodiment, the miRNA cluster consists in the combination miR.558 + miR.609 + miR.603 + miR.520f + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.603. In a particular embodiment, the miRNA cluster consists in the combination miR.558 + miR.609 + miR.603 + miR.520f + miR.220a + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338.

### miRNA clusters based on miR.518c:

A further aspect of the invention relates to a method for predicting the outcome of a cancer in a patient comprising a step consisting of determining the expression level of a miRNA cluster in a sample obtained from said patient, wherein said miRNA cluster comprises miR.518c.

The inventors have been indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.519b, miR.520f, miR.609, miR.558, miR.220a, miR.376a*, miR.639, miR.130a, miR.338, miR.26a, miR.29a, miR.494, miR.603, miR.660, miR.369-3p and miR.362.

In a particular embodiment, the miRNA cluster consists in the combination of 2 miRNAs. For example, said miRNA cluster may be selected according to any combination described in Table 2 which comprises miR.518c. In a particular embodiment, the miRNA cluster consists in the combination miR.603 + miR.518c.

In a particular embodiment, the miRNA cluster consists in the combination of 3 miRNAs. For example, said miRNA cluster may be selected according to any combination described in Table 3 which comprises miR.518c. In a particular embodiment, the miRNA cluster consists in the combination miR.558 + miR.609 + miR.518c.

In a particular embodiment, the miRNA cluster consists in the combination of 4 miRNAs. For example, said miRNA cluster may be selected according to any combination described in Table 4 which comprises miR.518c. In a particular embodiment, the miRNA cluster consists in the combination miR.609 + miR.29a + miR.376a* + miR.518c.

In a particular embodiment, the miRNA cluster consists in the combination of 5 miRNAs. For example, said miRNA cluster may be selected according to any combination described in Table 5 which comprises miR.518c. In a particular embodiment, the miRNA cluster consists in the combination miR.558 + miR.609 + miR.29a + miR.376a* + miR.518c.

In a particular embodiment, the miRNA cluster consists of in the combination of 6 miRNAs. For example, said miRNA cluster may be selected according to any combination described in Table 6 which comprises miR.518c. In a particular embodiment, the miRNA cluster consists in the combination miR.558 + miR.609 + miR.220a + miR.29a + miR.376a* + miR.518c.

In a particular embodiment, the miRNA cluster consists in the combination of 7 miRNAs. For example, said miRNA cluster may be selected according to any combination described in Table 7 which comprises miR.518c. In a particular embodiment, the miRNA cluster consists in the combination miR.558 + miR.609 + miR.220a + miR.29a + miR.369.3p + miR.376a* + miR.518c.

In a particular embodiment, the miRNA cluster consists in the combination of 8 miRNAs. For example, said miRNA cluster may be selected according to any combination described in Table 8 which comprises miR.518c. In a particular embodiment, the miRNA cluster consists in the combination miR.558 + miR.609 + miR.603 + miR.220a + miR.29a + miR.369.3p + miR.376a* + miR.518c.

In a particular embodiment, the miRNA cluster consists in the combination of 9 miRNAs. For example, said miRNA cluster may be selected according to any combination described in Table 9 which comprises miR.518c. In a particular embodiment, the miRNA cluster consists in the combination miR.558 + miR.609 + miR.603 + miR.220a + miR.26a + miR.29a + miR.369.3p + miR.376a* + miR.518c.

In a particular embodiment, the miRNA cluster consists in the combination of 10 miRNAs. For example, said miRNA cluster may be selected according to any combination described in Table 10 which comprises miR.518c. In a particular embodiment, the miRNA cluster consists in the combination miR.558 + miR.609 + miR.603 + miR.220a + miR.26a + miR.29a + miR.369.3p + miR.376a* + miR.518c + miR.660.

In a particular embodiment, the miRNA cluster consists in the combination of 11 miRNAs. For example, said miRNA cluster may be selected according to any combination described in Table 11 which comprises miR.518c. In a particular embodiment, the miRNA cluster consists in the combination miR.558 + miR.609 + miR.520f + miR.29a + miR.362 + miR.369.3p + miR.376a* + miR.518c + miR.639 + miR.660 + miR.338.

In a particular embodiment, the miRNA cluster consists in the combination of 12 miRNAs. For example, said miRNA cluster may be selected according to any combination described in Table 12 which comprises miR.518c. In a particular embodiment, the miRNA cluster consists in the combination miR.558 + miR.609 + miR.520f + miR.220a + miR.29a + miR.362 + miR.369.3p + miR.376a* + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.518c. In a particular embodiment, the miRNA cluster consists in the combination miR.558 + miR.609 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.518c. In a particular embodiment, the miRNA cluster consists in the combination miR.558 + miR.609 + miR.603 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.518c. In a particular embodiment, the miRNA cluster consists in the combination miR.558 + miR.609 + miR.603 + miR.520f + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.518c. In a particular embodiment, the miRNA cluster consists in the combination miR.558 + miR.609 + miR.603 + miR.520f + miR.220a + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338.

### miRNA clusters based on miR520f:

A further aspect of the invention relates to a method for predicting the outcome of a cancer in a patient comprising a step consisting of determining the expression level of a miRNA cluster in a sample obtained from said patient, wherein said miRNA cluster comprises miR.520f.

The inventors have been indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.519b, miR.518c, miR.609, miR.558, miR.220a, miR.376a*, miR.639, miR.130a, miR.338, miR.26a, miR.29a, miR.494, miR.603, miR.660, miR.369-3p and miR.362.

In a particular embodiment, the miRNA cluster consists in the combination of 2 miRNAs. For example, said miRNA cluster may be selected according to any combination described in Table 2 which comprises miR.520f. In a particular embodiment, the miRNA cluster consists in the combination miR.558 + miR.520f.

In a particular embodiment, the miRNA cluster consists in the combination of 3 miRNAs. For example, said miRNA cluster may be selected according to any combination described in Table 3 which comprises miR.520f. In a particular embodiment, the miRNA cluster consists in the combination miR.558 + miR.520f + miR.518c.

In a particular embodiment, the miRNA cluster consists in the combination of 4 miRNAs. For example, said miRNA cluster may be selected according to any combination described in Table 4 which comprises miR.520f. In a particular embodiment, the miRNA cluster consists in the combination miR.609 + miR.520f + miR.29a + miR.376a*

In a particular embodiment, the miRNA cluster consists in the combination of 5 miRNAs. For example, said miRNA cluster may be selected according to any combination described in Table 5 which comprises miR.520f. In a particular embodiment, the miRNA cluster consists in the combination miR.558 + miR.609 + miR.520f + miR.29a + miR.376a*.

In a particular embodiment, the miRNA cluster consists of in the combination of 6 miRNAs. For example, said miRNA cluster may be selected according to any combination described in Table 6 which comprises miR.520f. In a particular embodiment, the miRNA cluster consists in the combination miR.558 + miR.609 + miR.520f + miR.29a + miR.376a* + miR.518c.

In a particular embodiment, the miRNA cluster consists in the combination of 7 miRNAs. For example, said miRNA cluster may be selected according to any combination described in Table 7 which comprises miR.520f. In a particular embodiment, the miRNA cluster consists in the combination miR.558 + miR.609 + miR.520f + miR.220a + miR.29a + miR.376a* + miR.518c.

In a particular embodiment, the miRNA cluster consists in the combination of 8 miRNAs. For example, said miRNA cluster may be selected according to any combination described in Table 8 which comprises miR.520f. In a particular embodiment, the miRNA cluster consists in the combination miR.558 + miR.609 + miR.520f + miR.220a + miR.29a + miR.369.3p + miR.376a* + miR.518c.

In a particular embodiment, the miRNA cluster consists in the combination of 9 miRNAs. For example, said miRNA cluster may be selected according to any combination described in Table 9 which comprises miR.520f. In a particular embodiment, the miRNA cluster consists in the combination miR.558 + miR.609 + miR.603 + miR.520f + miR.220a + miR.29a + miR.369.3p + miR.376a* + miR.518c.

In a particular embodiment, the miRNA cluster consists in the combination of 10 miRNAs. For example, said miRNA cluster may be selected according to any combination described in Table 10 which comprises miR.520f. In a particular embodiment, the miRNA cluster consists in the combination. miR.558 + miR.609 + miR.603 + miR.520f + miR.220a + miR.29a + miR.369.3p + miR.376a* + miR.518c + miR.660

In a particular embodiment, the miRNA cluster consists in the combination of 11 miRNAs. For example, said miRNA cluster may be selected according to any combination described in Table 11 which comprises miR.520f. In a particular embodiment, the miRNA cluster consists in the combination miR.558 + miR.609 + miR.520f + miR.29a + miR.362 + miR.369.3p + miR.376a* + miR.518c + miR.639 + miR.660 + miR.338.

In a particular embodiment, the miRNA cluster consists in the combination of 12 miRNAs. For example, said miRNA cluster may be selected according to any combination described in Table 12 which comprises miR.520f. In a particular embodiment, the miRNA cluster consists in the combination miR.558 + miR.609 + miR.520f + miR.220a + miR.29a + miR.362 + miR.369.3p + miR.376a* + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.520f. In a particular embodiment, the miRNA cluster consists in the combination miR.558 + miR.609 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.520f. In a particular embodiment, the miRNA cluster consists in the combination miR.558 + miR.609 + miR.603 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.520f. In a particular embodiment, the miRNA cluster consists in the combination miR.558 + miR.609 + miR.603 + miR.520f + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.520f. In a particular embodiment, the miRNA cluster consists in the combination miR.558 + miR.609 + miR.603 + miR.520f + miR.220a + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338.

### miRNA clusters based on miR.362:

A further aspect of the invention relates to a method for predicting the outcome of a cancer in a patient comprising a step consisting of determining the expression level of a miRNA cluster in a sample obtained from said patient, wherein said miRNA cluster comprises miR.362.

The inventors have been indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.519b, miR.518c, miR.609, miR.558, miR.220a, miR.376a*, miR.639, miR.130a, miR.338, miR.26a, miR.29a, miR.494, miR.603, miR.660, miR.369-3p and miR.520f.

In a particular embodiment, the miRNA cluster consists in the combination of 2 miRNAs. For example, said miRNA cluster may be selected according to any combination described in Table 2 which comprises miR.362. In a particular embodiment, the miRNA cluster consists in the combination miR.558 + miR.362.

In a particular embodiment, the miRNA cluster consists in the combination of 3 miRNAs. For example, said miRNA cluster may be selected according to any combination described in Table 3 which comprises miR.362. In a particular embodiment, the miRNA cluster consists in the combination miR.603 + miR.362 + miR.518c.

In a particular embodiment, the miRNA cluster consists in the combination of 4 miRNAs. For example, said miRNA cluster may be selected according to any combination described in Table 4 which comprises miR.362. In a particular embodiment, the miRNA cluster consists in the combination miR.558 + miR.609 + miR.362 + miR.518c.

In a particular embodiment, the miRNA cluster consists in the combination of 5 miRNAs. For example, said miRNA cluster may be selected according to any combination described in Table 5 which comprises miR.362. In a particular embodiment, the miRNA cluster consists in the combination miR.609 + miR.29a + miR.362 + miR.376a* + miR.518c.

In a particular embodiment, the miRNA cluster consists of in the combination of 6 miRNAs. For example, said miRNA cluster may be selected according to any combination described in Table 6 which comprises miR.362. In a particular embodiment, the miRNA cluster consists in the combination miR.558 + miR.609 + miR.29a + miR.362 + miR.376a* + miR.518c.

In a particular embodiment, the miRNA cluster consists in the combination of 7 miRNAs. For example, said miRNA cluster may be selected according to any combination described in Table 7 which comprises miR.362. In a particular embodiment, the miRNA cluster consists in the combination miR.558 + miR.609 + miR.220a + miR.29a + miR.362 + miR.376a* + miR.518c.

In a particular embodiment, the miRNA cluster consists in the combination of 8 miRNAs. For example, said miRNA cluster may be selected according to any combination described in Table 8 which comprises miR.362. In a particular embodiment, the miRNA cluster consists in the combination miR.558 + miR.609 + miR.603 + miR.220a + miR.29a + miR.362 + miR.376a* + miR.518c.

In a particular embodiment, the miRNA cluster consists in the combination of 9 miRNAs. For example, said miRNA cluster may be selected according to any combination described in Table 9 which comprises miR.362. In a particular embodiment, the miRNA cluster consists in the combination miR.558 + miR.609 + miR.603 + miR.220a + miR.519b + miR.29a + miR.362 + miR.376a* + miR.518c.

In a particular embodiment, the miRNA cluster consists in the combination of 10 miRNAs. For example, said miRNA cluster may be selected according to any combination described in Table 10 which comprises miR.362. In a particular embodiment, the miRNA cluster consists in the combination miR.558 + miR.609 + miR.603 + miR.220a + miR.519b + miR.29a + miR.362 + miR.369.3p + miR.376a* + miR.518c.

In a particular embodiment, the miRNA cluster consists in the combination of 11 miRNAs. For example, said miRNA cluster may be selected according to any combination described in Table 11 which comprises miR.362. In a particular embodiment, the miRNA cluster consists in the combination miR.558 + miR.609 + miR.520f + miR.29a + miR.362 + miR.369.3p + miR.376a* + miR.518c + miR.639 + miR.660 + miR.338.

In a particular embodiment, the miRNA cluster consists in the combination of 12 miRNAs. For example, said miRNA cluster may be selected according to any combination described in Table 12 which comprises miR.362. In a particular embodiment, the miRNA cluster consists in the combination miR.558 + miR.609 + miR.520f + miR.220a + miR.29a + miR.362 + miR.369.3p + miR.376a* + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.362. In a particular embodiment, the miRNA cluster consists in the combination miR.558 + miR.609 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.362. In a particular embodiment, the miRNA cluster consists in the combination miR.558 + miR.609 + miR.603 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.362. In a particular embodiment, the miRNA cluster consists in the combination miR.558 + miR.609 + miR.603 + miR.520f + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.362. In a particular embodiment, the miRNA cluster consists in the combination miR.558 + miR.609 + miR.603 + miR.520f + miR.220a + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338.

### miRNA clusters based on miR.220a:

A further aspect of the invention relates to a method for predicting the outcome of a cancer in a patient comprising a step consisting of determining the expression level of a miRNA cluster in a sample obtained from said patient, wherein said miRNA cluster comprises miR.220a.

The inventors have been indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.519b, miR.518c, miR.609, miR.558, miR.362, miR.376a*, miR.639, miR.130a, miR.338, miR.26a, miR.29a, miR.494, miR.603, miR.660, miR.369-3p and miR.520f.

In a particular embodiment, the miRNA cluster consists in the combination of 2 miRNAs. For example, said miRNA cluster may be selected according to any combination described in Table 2 which comprises miR.220a. In a particular embodiment, the miRNA cluster consists in the combination miR.220a + miR.518c.

In a particular embodiment, the miRNA cluster consists in the combination of 3 miRNAs. For example, said miRNA cluster may be selected according to any combination described in Table 3 which comprises miR.220a. In a particular embodiment, the miRNA cluster consists in the combination miR.603 + miR.220a + miR.518c.

In a particular embodiment, the miRNA cluster consists in the combination of 4 miRNAs. For example, said miRNA cluster may be selected according to any combination described in Table 4 which comprises miR.220a. In a particular embodiment, the miRNA cluster consists in the combination miR.558 + miR.609 + miR.220a + miR.518c.

In a particular embodiment, the miRNA cluster consists in the combination of 5 miRNAs. For example, said miRNA cluster may be selected according to any combination described in Table 5 which comprises miR.220a. In a particular embodiment, the miRNA cluster consists in the combination miR.609 + miR.220a + miR.29a + miR.376a* + miR.518c.

In a particular embodiment, the miRNA cluster consists of in the combination of 6 miRNAs. For example, said miRNA cluster may be selected according to any combination described in Table 6 which comprises miR.220a. In a particular embodiment, the miRNA cluster consists in the combination miR.558 + miR.609 + miR.220a + miR.29a + miR.376a* + miR.518c.

In a particular embodiment, the miRNA cluster consists in the combination of 7 miRNAs. For example, said miRNA cluster may be selected according to any combination described in Table 7 which comprises miR.220a. In a particular embodiment, the miRNA cluster consists in the combination miR.558 + miR.609 + miR.220a + miR.29a + miR.369.3p + miR.376a* + miR.518c.

In a particular embodiment, the miRNA cluster consists in the combination of 8 miRNAs. For example, said miRNA cluster may be selected according to any combination described in Table 8 which comprises miR.220a. In a particular embodiment, the miRNA cluster consists in the combination miR.558 + miR.609 + miR.603 + miR.220a + miR.29a + miR.369.3p + miR.376a* + miR.518c.

In a particular embodiment, the miRNA cluster consists in the combination of 9 miRNAs. For example, said miRNA cluster may be selected according to any combination described in Table 9 which comprises miR.220a. In a particular embodiment, the miRNA cluster consists in the combination miR.558 + miR.609 + miR.603 + miR.220a + miR.26a + miR.29a + miR.369.3p + miR.376a* + miR.518c.

In a particular embodiment, the miRNA cluster consists in the combination of 10 miRNAs. For example, said miRNA cluster may be selected according to any combination described in Table 10 which comprises miR.220a. In a particular embodiment, the miRNA cluster consists in the combination miR.558 + miR.609 + miR.603 + miR.220a + miR.26a + miR.29a + miR.369.3p + miR.376a* + miR.518c + miR.660.

In a particular embodiment, the miRNA cluster consists in the combination of 11 miRNAs. For example, said miRNA cluster may be selected according to any combination described in Table 11 which comprises miR.220a. In a particular embodiment, the miRNA cluster consists in the combination miR.558 + miR.609 + miR.603 + miR.220a + miR.26a + miR.29a + miR.369.3p + miR.376a* + miR.518c + miR.660 + miR.338.

In a particular embodiment, the miRNA cluster consists in the combination of 12 miRNAs. For example, said miRNA cluster may be selected according to any combination described in Table 12 which comprises miR.220a. In a particular embodiment, the miRNA cluster consists in the combination miR.558 + miR.609 + miR.520f + miR.220a + miR.29a + miR.362 + miR.369.3p + miR.376a* + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.220a. In a particular embodiment, the miRNA cluster consists in the combination miR.558 + miR.609 + miR.603 + miR.520f + miR.220a + miR.29a + miR.362 + miR.369.3p + miR.376a* + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.220a. In a particular embodiment, the miRNA cluster consists in the combination miR.558 + miR.609 + miR.520f + miR.220a + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.220a. In a particular embodiment, the miRNA cluster consists in the combination miR.558 + miR.609 + miR.603 + miR.220a + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.220a. In a particular embodiment, the miRNA cluster consists in the combination miR.558 + miR.609 + miR.603 + miR.520f + miR.220a + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338.

### miRNA clusters based on miR.29a:

A further aspect of the invention relates to a method for predicting the outcome of a cancer in a patient comprising a step consisting of determining the expression level of a miRNA cluster in a sample obtained from said patient, wherein said miRNA cluster comprises miR.29a.

The inventors have been indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.519b, miR.518c, miR.609, miR.558, miR.362, miR.376a*, miR.639, miR.130a, miR.338, miR.26a, miR.220a, miR.494, miR.603, miR.660, miR.369-3p and miR.520f.

In a particular embodiment, the miRNA cluster consists in the combination of 2 miRNAs. For example, said miRNA cluster may be selected according to any combination described in Table 2 which comprises miR.29a. In a particular embodiment, the miRNA cluster consists in the combination miR.609 + miR.29a.

In a particular embodiment, the miRNA cluster consists in the combination of 3 miRNAs. For example, said miRNA cluster may be selected according to any combination described in Table 3 which comprises miR.29a. In a particular embodiment, the miRNA cluster consists in the combination miR.609 + miR.29a + miR.518c.

In a particular embodiment, the miRNA cluster consists in the combination of 4 miRNAs. For example, said miRNA cluster may be selected according to any combination described in Table 4 which comprises miR.29a. In a particular embodiment, the miRNA cluster consists in the combination miR.609 + miR.29a + miR.376a* + miR.518c.

In a particular embodiment, the miRNA cluster consists in the combination of 5 miRNAs. For example, said miRNA cluster may be selected according to any combination described in Table 5 which comprises miR.29a. In a particular embodiment, the miRNA cluster consists in the combination miR.558 + miR.609 + miR.29a + miR.376a* + miR.518c.

In a particular embodiment, the miRNA cluster consists of in the combination of 6 miRNAs. For example, said miRNA cluster may be selected according to any combination described in Table 6 which comprises miR.29a. In a particular embodiment, the miRNA cluster consists in the combination miR.558 + miR.609 + miR.220a + miR.29a + miR.376a* + miR.518c.

In a particular embodiment, the miRNA cluster consists in the combination of 7 miRNAs. For example, said miRNA cluster may be selected according to any combination described in Table 7 which comprises miR.29a. In a particular embodiment, the miRNA cluster consists in the combination miR.558 + miR.609 + miR.220a + miR.29a + miR.369.3p + miR.376a* + miR.518c.

In a particular embodiment, the miRNA cluster consists in the combination of 8 miRNAs. For example, said miRNA cluster may be selected according to any combination described in Table 8 which comprises miR.29a. In a particular embodiment, the miRNA cluster consists in the combination miR.558 + miR.609 + miR.603 + miR.220a + miR.29a + miR.369.3p + miR.376a* + miR.518c.

In a particular embodiment, the miRNA cluster consists in the combination of 9 miRNAs. For example, said miRNA cluster may be selected according to any combination described in Table 9 which comprises miR.29a. In a particular embodiment, the miRNA cluster consists in the combination miR.558 + miR.609 + miR.603 + miR.220a + miR.26a + miR.29a + miR.369.3p + miR.376a* + miR.518c.

In a particular embodiment, the miRNA cluster consists in the combination of 10 miRNAs. For example, said miRNA cluster may be selected according to any combination described in Table 10 which comprises miR.29a. In a particular embodiment, the miRNA cluster consists in the combination miR.558 + miR.609 + miR.603 + miR.220a + miR.26a + miR.29a + miR.369.3p + miR.376a* + miR.518c + miR.660.

In a particular embodiment, the miRNA cluster consists in the combination of 11 miRNAs. For example, said miRNA cluster may be selected according to any combination described in Table 11 which comprises miR.29a. In a particular embodiment, the miRNA cluster consists in the combination miR.558 + miR.609 + miR.520f + miR.29a + miR.362 + miR.369.3p + miR.376a* + miR.518c + miR.639 + miR.660 + miR.338.

In a particular embodiment, the miRNA cluster consists in the combination of 12 miRNAs. For example, said miRNA cluster may be selected according to any combination described in Table 12 which comprises miR.29a. In a particular embodiment, the miRNA cluster consists in the combination miR.558 + miR.609 + miR.520f + miR.220a + miR.29a + miR.362 + miR.369.3p + miR.376a* + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.29a. In a particular embodiment, the miRNA cluster consists in the combination miR.558 + miR.609 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.29a. In a particular embodiment, the miRNA cluster consists in the combination miR.558 + miR.609 + miR.603 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.29a. In a particular embodiment, the miRNA cluster consists in the combination miR.558 + miR.609 + miR.603 + miR.520f + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.29a. In a particular embodiment, the miRNA cluster consists in the combination miR.558 + miR.609 + miR.603 + miR.520f + miR.220a + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338.

### miRNA clusters based on miR.519b:

A further aspect of the invention relates to a method for predicting the outcome of a cancer in a patient comprising a step consisting of determining the expression level of a miRNA cluster in a sample obtained from said patient, wherein said miRNA cluster comprises miR.519b.

The inventors have been indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.29a, miR.518c, miR.609, miR.558, miR.362, miR.376a*, miR.639, miR.130a, miR.338, miR.26a, miR.220a, miR.494, miR.603, miR.660, miR.369-3p and miR.520f.

In a particular embodiment, the miRNA cluster consists in the combination of 2 miRNAs. For example, said miRNA cluster may be selected according to any combination described in Table 2 which comprises miR.519b. In a particular embodiment, the miRNA cluster consists in the combination miR.519b + miR.518c.

In a particular embodiment, the miRNA cluster consists in the combination of 3 miRNAs. For example, said miRNA cluster may be selected according to any combination described in Table 3 which comprises miR.519b. In a particular embodiment, the miRNA cluster consists in the combination miR.603 + miR.519b + miR.518c.

In a particular embodiment, the miRNA cluster consists in the combination of 4 miRNAs. For example, said miRNA cluster may be selected according to any combination described in Table 4 which comprises miR.519b. In a particular embodiment, the miRNA cluster consists in the combination miR.558 + miR.609 + miR.519b + miR.518c.

In a particular embodiment, the miRNA cluster consists in the combination of 5 miRNAs. For example, said miRNA cluster may be selected according to any combination described in Table 5 which comprises miR.519b. In a particular embodiment, the miRNA cluster consists in the combination miR.609 + miR.519b + miR.29a + miR.376a* + miR.518c.

In a particular embodiment, the miRNA cluster consists of in the combination of 6 miRNAs. For example, said miRNA cluster may be selected according to any combination described in Table 6 which comprises miR.519b. In a particular embodiment, the miRNA cluster consists in the combination miR.558 + miR.609 + miR.519b + miR.29a + miR.376a* + miR.518c.

In a particular embodiment, the miRNA cluster consists in the combination of 7 miRNAs. For example, said miRNA cluster may be selected according to any combination described in Table 7 which comprises miR.519b. In a particular embodiment, the miRNA cluster consists in the combination miR.558 + miR.609 + miR.220a + miR.519b + miR.29a + miR.376a* + miR.518c.

In a particular embodiment, the miRNA cluster consists in the combination of 8 miRNAs. For example, said miRNA cluster may be selected according to any combination described in Table 8 which comprises miR.519b. In a particular embodiment, the miRNA cluster consists in the combination miR.558 + miR.609 + miR.603 + miR.220a + miR.519b + miR.29a + miR.376a* + miR.518c.

In a particular embodiment, the miRNA cluster consists in the combination of 9 miRNAs. For example, said miRNA cluster may be selected according to any combination described in Table 9 which comprises miR.519b. In a particular embodiment, the miRNA cluster consists in the combination miR.558 + miR.609 + miR.603 + miR.220a + miR.519b + miR.29a + miR.369.3p + miR.376a* + miR.518c.

In a particular embodiment, the miRNA cluster consists in the combination of 10 miRNAs. For example, said miRNA cluster may be selected according to any combination described in Table 10 which comprises miR.519b. In a particular embodiment, the miRNA cluster consists in the combination miR.558 + miR.609 + miR.603 + miR.220a + miR.519b + miR.29a + miR.369.3p + miR.376a* + miR.518c + miR.660.

In a particular embodiment, the miRNA cluster consists in the combination of 11 miRNAs. For example, said miRNA cluster may be selected according to any combination described in Table 11 which comprises miR.519b. In a particular embodiment, the miRNA cluster consists in the combination miR.558 + miR.609 + miR.603 + miR.220a + miR.519b + miR.26a + miR.29a + miR.369.3p + miR.376a* + miR.518c + miR.660.

In a particular embodiment, the miRNA cluster consists in the combination of 12 miRNAs. For example, said miRNA cluster may be selected according to any combination described in Table 12 which comprises miR.519b. In a particular embodiment, the miRNA cluster consists in the combination miR.558 + miR.609 + miR.520f + miR.519b + miR.29a + miR.362 + miR.369.3p + miR.376a* + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.519b. In a particular embodiment, the miRNA cluster consists in the combination miR.558 + miR.609 + miR.520f + miR.519b + miR.26a + miR.29a + miR.362 + miR.369.3p + miR.376a* + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.519b. In a particular embodiment, the miRNA cluster consists in the combination miR.558 + miR.609 + miR.520f + miR.519b + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.519b. In a particular embodiment, the miRNA cluster consists in the combination miR.558 + miR.609 + miR.603 + miR.520f + miR.519b + miR.130a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.519b. In a particular embodiment, the miRNA cluster consists in the combination miR.558 + miR.609 + miR.603 + miR.520f + miR.519b + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338.

### miRNA clusters based on miR.494:

A further aspect of the invention relates to a method for predicting the outcome of a cancer in a patient comprising a step consisting of determining the expression level of a miRNA cluster in a sample obtained from said patient, wherein said miRNA cluster comprises miR.494.

The inventors have been indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.29a, miR.518c, miR.609, miR.558, miR.362, miR.376a*, miR.639, miR.130a, miR.338, miR.26a, miR.220a, miR.519b, miR.603, miR.660, miR.369-3p and miR.520f.

In a particular embodiment, the miRNA cluster consists in the combination of 2 miRNAs. For example, said miRNA cluster may be selected according to any combination described in Table 2 which comprises miR.494. In a particular embodiment, the miRNA cluster consists in the combination miR.603 + miR.494.

In a particular embodiment, the miRNA cluster consists in the combination of 3 miRNAs. For example, said miRNA cluster may be selected according to any combination described in Table 3 which comprises miR.494. In a particular embodiment, the miRNA cluster consists in the combination miR.603 + miR.494 + miR.518c.

In a particular embodiment, the miRNA cluster consists in the combination of 4 miRNAs. For example, said miRNA cluster may be selected according to any combination described in Table 4 which comprises miR.494. In a particular embodiment, the miRNA cluster consists in the combination miR.558 + miR.609 + miR.494 + miR.518c.

In a particular embodiment, the miRNA cluster consists in the combination of 5 miRNAs. For example, said miRNA cluster may be selected according to any combination described in Table 5 which comprises miR.494. In a particular embodiment, the miRNA cluster consists in the combination miR.609 + miR.520f + miR.29a + miR.376a* + miR.494.

In a particular embodiment, the miRNA cluster consists of in the combination of 6 miRNAs. For example, said miRNA cluster may be selected according to any combination described in Table 6 which comprises miR.494. In a particular embodiment, the miRNA cluster consists in the combination miR.558 + miR.609 + miR.29a + miR.376a* + miR.494 + miR.518c.

In a particular embodiment, the miRNA cluster consists in the combination of 7 miRNAs. For example, said miRNA cluster may be selected according to any combination described in Table 7 which comprises miR.494. In a particular embodiment, the miRNA cluster consists in the combination miR.558 + miR.609 + miR.220a + miR.29a + miR.376a* + miR.494 + miR.518c.

In a particular embodiment, the miRNA cluster consists in the combination of 8 miRNAs. For example, said miRNA cluster may be selected according to any combination described in Table 8 which comprises miR.494. In a particular embodiment, the miRNA cluster consists in the combination miR.558 + miR.609 + miR.220a + miR.26a + miR.29a + miR.376a* + miR.494 + miR.518c.

In a particular embodiment, the miRNA cluster consists in the combination of 9 miRNAs. For example, said miRNA cluster may be selected according to any combination described in Table 9 which comprises miR.494. In a particular embodiment, the miRNA cluster consists in the combination miR.558 + miR.609 + miR.603 + miR.220a + miR.29a + miR.369.3p + miR.376a* + miR.494 + miR.518c.

In a particular embodiment, the miRNA cluster consists in the combination of 10 miRNAs. For example, said miRNA cluster may be selected according to any combination described in Table 10 which comprises miR.494. In a particular embodiment, the miRNA cluster consists in the combination miR.558 + miR.609 + miR.603 + miR.220a + miR.26a + miR.29a + miR.369.3p + miR.376a* + miR.494 + miR.518c.

In a particular embodiment, the miRNA cluster consists in the combination of 11 miRNAs. For example, said miRNA cluster may be selected according to any combination described in Table 11 which comprises miR.494. In a particular embodiment, the miRNA cluster consists in the combination miR.558 + miR.609 + miR.603 + miR.220a + miR.26a + miR.29a + miR.369.3p + miR.376a* + miR.494 + miR.518c + miR.660.

In a particular embodiment, the miRNA cluster consists in the combination of 12 miRNAs. For example, said miRNA cluster may be selected according to any combination described in Table 12 which comprises miR.494. In a particular embodiment, the miRNA cluster consists in the combination miR.558 + miR.609 + miR.520f + miR.29a + miR.362 + miR.369.3p + miR.376a* + miR.494 + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.494. In a particular embodiment, the miRNA cluster consists in the combination miR.558 + miR.609 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.494. In a particular embodiment, the miRNA cluster consists in the combination miR.558 + miR.609 + miR.603 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.494. In a particular embodiment, the miRNA cluster consists in the combination miR.558 + miR.609 + miR.603 + miR.520f + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.494. In a particular embodiment, the miRNA cluster consists in the combination miR.558 + miR.609 + miR.603 + miR.520f + miR.519b + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338.

### miRNA clusters based on miR.130a:

A further aspect of the invention relates to a method for predicting the outcome of a cancer in a patient comprising a step consisting of determining the expression level of a miRNA cluster in a sample obtained from said patient, wherein said miRNA cluster comprises miR.130a.

The inventors have been indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.29a, miR.518c, miR.609, miR.558, miR.362, miR.376a*, miR.639, miR.494, miR.338, miR.26a, miR.220a, miR.519b, miR.603, miR.660, miR.369-3p and miR.520f.

In a particular embodiment, the miRNA cluster consists in the combination of 2 miRNAs. For example, said miRNA cluster may be selected according to any combination described in Table 2 which comprises miR.130a. In a particular embodiment, the miRNA cluster consists in the combination miR.520f + miR.130a.

In a particular embodiment, the miRNA cluster consists in the combination of 3 miRNAs. For example, said miRNA cluster may be selected according to any combination described in Table 3 which comprises miR.130a. In a particular embodiment, the miRNA cluster consists in the combination miR.609 + miR.130a + miR.518c.

In a particular embodiment, the miRNA cluster consists in the combination of 4 miRNAs. For example, said miRNA cluster may be selected according to any combination described in Table 4 which comprises miR.130a. In a particular embodiment, the miRNA cluster consists in the combination miR.558 + miR.609 + miR.130a + miR.518c.

In a particular embodiment, the miRNA cluster consists in the combination of 5 miRNAs. For example, said miRNA cluster may be selected according to any combination described in Table 5 which comprises miR.130a. In a particular embodiment, the miRNA cluster consists in the combination miR.558 + miR.609 + miR.130a + miR.376a* + miR.518c.

In a particular embodiment, the miRNA cluster consists of in the combination of 6 miRNAs. For example, said miRNA cluster may be selected according to any combination described in Table 6 which comprises miR.130a. In a particular embodiment, the miRNA cluster consists in the combination miR.558 + miR.609 + miR.130a + miR.29a + miR.376a* + miR.518c.

In a particular embodiment, the miRNA cluster consists in the combination of 7 miRNAs. For example, said miRNA cluster may be selected according to any combination described in Table 7 which comprises miR.130a. In a particular embodiment, the miRNA cluster consists in the combination miR.558 + miR.609 + miR.220a + miR.130a + miR.29a + miR.376a* + miR.518c.

In a particular embodiment, the miRNA cluster consists in the combination of 8 miRNAs. For example, said miRNA cluster may be selected according to any combination described in Table 8 which comprises miR.130a. In a particular embodiment, the miRNA cluster consists in the combination miR.558 + miR.609 + miR.220a + miR.130a + miR.29a + miR.369.3p + miR.376a* + miR.518c.

In a particular embodiment, the miRNA cluster consists in the combination of 9 miRNAs. For example, said miRNA cluster may be selected according to any combination described in Table 9 which comprises miR.130a. In a particular embodiment, the miRNA cluster consists in the combination miR.558 + miR.609 + miR.603 + miR.220a + miR.130a + miR.29a + miR.369.3p + miR.376a* + miR.518c.

In a particular embodiment, the miRNA cluster consists in the combination of 10 miRNAs. For example, said miRNA cluster may be selected according to any combination described in Table 10 which comprises miR.130a. In a particular embodiment, the miRNA cluster consists in the combination miR.558 + miR.609 + miR.603 + miR.220a + miR.130a + miR.26a + miR.29a + miR.369.3p + miR.376a* + miR.518c.

In a particular embodiment, the miRNA cluster consists in the combination of 11 miRNAs. For example, said miRNA cluster may be selected according to any combination described in Table 11 which comprises miR.130a. In a particular embodiment, the miRNA cluster consists in the combination miR.558 + miR.609 + miR.603 + miR.220a + miR.130a + miR.26a + miR.29a + miR.369.3p + miR.376a* + miR.518c + miR.660.

In a particular embodiment, the miRNA cluster consists in the combination of 12 miRNAs. For example, said miRNA cluster may be selected according to any combination described in Table 12 which comprises miR.130a. In a particular embodiment, the miRNA cluster consists in the combination miR.558 + miR.609 + miR.520f + miR.130a + miR.29a + miR.362 + miR.369.3p + miR.376a* + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.130a. In a particular embodiment, the miRNA cluster consists in the combination miR.558 + miR.609 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.130a. In a particular embodiment, the miRNA cluster consists in the combination miR.558 + miR.609 + miR.603 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.130a. In a particular embodiment, the miRNA cluster consists in the combination miR.558 + miR.609 + miR.603 + miR.520f + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.130a. In a particular embodiment, the miRNA cluster consists in the combination miR.558 + miR.609 + miR.603 + miR.520f + miR.220a + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338.

### miRNA clusters based on miR.639:

A further aspect of the invention relates to a method for predicting the outcome of a cancer in a patient comprising a step consisting of determining the expression level of a miRNA cluster in a sample obtained from said patient, wherein said miRNA cluster comprises miR.639.

The inventors have been indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.29a, miR.518c, miR.609, miR.558, miR.362, miR.376a*, miR.130a, miR.494, miR.338, miR.26a, miR.220a, miR.519b, miR.603, miR.660, miR.369-3p and miR.520f.

In a particular embodiment, the miRNA cluster consists in the combination of 2 miRNAs. For example, said miRNA cluster may be selected according to any combination described in Table 2 which comprises miR.639 In a particular embodiment, the miRNA cluster consists in the combination miR.603 + miR.639.

In a particular embodiment, the miRNA cluster consists in the combination of 3 miRNAs. For example, said miRNA cluster may be selected according to any combination described in Table 3 which comprises miR.639. In a particular embodiment, the miRNA cluster consists in the combination miR.609 + miR.518c + miR.639.

In a particular embodiment, the miRNA cluster consists in the combination of 4 miRNAs. For example, said miRNA cluster may be selected according to any combination described in Table 4 which comprises miR.639. In a particular embodiment, the miRNA cluster consists in the combination miR.558 + miR.609 + miR.518c + miR.639.

In a particular embodiment, the miRNA cluster consists in the combination of 5 miRNAs. For example, said miRNA cluster may be selected according to any combination described in Table 5 which comprises miR.639. In a particular embodiment, the miRNA cluster consists in the combination miR.558 + miR.609 + miR.376a* + miR.518c + miR.639.

In a particular embodiment, the miRNA cluster consists of in the combination of 6 miRNAs. For example, said miRNA cluster may be selected according to any combination described in Table 6 which comprises miR.639. In a particular embodiment, the miRNA cluster consists in the combination miR.558 + miR.609 + miR.29a + miR.376a* + miR.518c + miR.639.

In a particular embodiment, the miRNA cluster consists in the combination of 7 miRNAs. For example, said miRNA cluster may be selected according to any combination described in Table 7 which comprises miR.639. In a particular embodiment, the miRNA cluster consists in the combination miR.558 + miR.609 + miR.520f + miR.29a + miR.376a* + miR.518c + miR.639.

In a particular embodiment, the miRNA cluster consists in the combination of 8 miRNAs. For example, said miRNA cluster may be selected according to any combination described in Table 8 which comprises miR.639. In a particular embodiment, the miRNA cluster consists in the combination miR.558 + miR.609 + miR.220a + miR.29a + miR.369.3p + miR.376a* + miR.518c + miR.639.

In a particular embodiment, the miRNA cluster consists in the combination of 9 miRNAs. For example, said miRNA cluster may be selected according to any combination described in Table 9 which comprises miR.639. In a particular embodiment, the miRNA cluster consists in the combination miR.558 + miR.609 + miR.603 + miR.220a + miR.26a + miR.29a + miR.376a* + miR.518c + miR.639.

In a particular embodiment, the miRNA cluster consists in the combination of 10 miRNAs. For example, said miRNA cluster may be selected according to any combination described in Table 10 which comprises miR.639. In a particular embodiment, the miRNA cluster consists in the combination miR.558 + miR.609 + miR.603 + miR.220a + miR.26a + miR.29a + miR.369.3p + miR.376a* + miR.518c + miR.639.

In a particular embodiment, the miRNA cluster consists in the combination of 11 miRNAs. For example, said miRNA cluster may be selected according to any combination described in Table 11 which comprises miR.639. In a particular embodiment, the miRNA cluster consists in the combination miR.558 + miR.609 + miR.520f + miR.29a + miR.362 + miR.369.3p + miR.376a* + miR.518c + miR.639 + miR.660 + miR.338.

In a particular embodiment, the miRNA cluster consists in the combination of 12 miRNAs. For example, said miRNA cluster may be selected according to any combination described in Table 12 which comprises miR.639. In a particular embodiment, the miRNA cluster consists in the combination miR.558 + miR.609 + miR.520f + miR.220a + miR.29a + miR.362 + miR.369.3p + miR.376a* + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.639. In a particular embodiment, the miRNA cluster consists in the combination miR.558 + miR.609 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.639. In a particular embodiment, the miRNA cluster consists in the combination miR.558 + miR.609 + miR.603 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.639. In a particular embodiment, the miRNA cluster consists in the combination miR.558 + miR.609 + miR.603 + miR.520f + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.639. In a particular embodiment, the miRNA cluster consists in the combination miR.558 + miR.609 + miR.603 + miR.520f + miR.220a + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338.

### miRNA clusters based on miR.660:

A further aspect of the invention relates to a method for predicting the outcome of a cancer in a patient comprising a step consisting of determining the expression level of a miRNA cluster in a sample obtained from said patient, wherein said miRNA cluster comprises miR.660.

The inventors have been indeed demonstrated that said miRNA provides a greater accuracy for predicting the outcome of a cancer than the UICC-TNM classification (i.e. the miRNA provides a ROC curve which results in a larger area under the curve than the one with TNM) (see Table 1).

In a particular embodiment, the miRNA cluster further comprises at least one miRNA selected from the group consisting of miR.29a, miR.518c, miR.609, miR.558, miR.362, miR.376a*, miR.130a, miR.494, miR.338, miR.26a, miR.220a, miR.519b, miR.603, miR.639, miR.369-3p and miR.520f.

In a particular embodiment, the miRNA cluster consists in the combination of 2 miRNAs. For example, said miRNA cluster may be selected according to any combination described in Table 2 which comprises miR.660 In a particular embodiment, the miRNA cluster consists in the combination miR.603 + miR.660.

In a particular embodiment, the miRNA cluster consists in the combination of 3 miRNAs. For example, said miRNA cluster may be selected according to any combination described in Table 3 which comprises miR.660. In a particular embodiment, the miRNA cluster consists in the combination miR.603 + miR.518c + miR.660.

In a particular embodiment, the miRNA cluster consists in the combination of 4 miRNAs. For example, said miRNA cluster may be selected according to any combination described in Table 4 which comprises miR.660. In a particular embodiment, the miRNA cluster consists in the combination miR.558 + miR.609 + miR.518c + miR.660.

In a particular embodiment, the miRNA cluster consists in the combination of 5 miRNAs. For example, said miRNA cluster may be selected according to any combination described in Table 5 which comprises miR.660. In a particular embodiment, the miRNA cluster consists in the combination miR.558 + miR.609 + miR.29a + miR.518c + miR.660.

In a particular embodiment, the miRNA cluster consists of in the combination of 6 miRNAs. For example, said miRNA cluster may be selected according to any combination described in Table 6 which comprises miR.660. In a particular embodiment, the miRNA cluster consists in the combination miR.558 + miR.609 + miR.29a + miR.376a* + miR.518c + miR.660.

In a particular embodiment, the miRNA cluster consists in the combination of 7 miRNAs. For example, said miRNA cluster may be selected according to any combination described in Table 7 which comprises miR.660. In a particular embodiment, the miRNA cluster consists in the combination miR.558 + miR.609 + miR.520f + miR.29a + miR.376a* + miR.518c + miR.660.

In a particular embodiment, the miRNA cluster consists in the combination of 8 miRNAs. For example, said miRNA cluster may be selected according to any combination described in Table 8 which comprises miR.660. In a particular embodiment, the miRNA cluster consists in the combination miR.558 + miR.609 + miR.220a + miR.29a + miR.369.3p + miR.376a* + miR.518c + miR.660.

In a particular embodiment, the miRNA cluster consists in the combination of 9 miRNAs. For example, said miRNA cluster may be selected according to any combination described in Table 9 which comprises miR.660. In a particular embodiment, the miRNA cluster consists in the combination miR.558 + miR.609 + miR.603 + miR.220a + miR.29a + miR.369.3p + miR.376a* + miR.518c + miR.660.

In a particular embodiment, the miRNA cluster consists in the combination of 10 miRNAs. For example, said miRNA cluster may be selected according to any combination described in Table 10 which comprises miR.660. In a particular embodiment, the miRNA cluster consists in the combination miR.558 + miR.609 + miR.603 + miR.220a + miR.26a + miR.29a + miR.369.3p + miR.376a* + miR.518c + miR.660.

In a particular embodiment, the miRNA cluster consists in the combination of 11 miRNAs. For example, said miRNA cluster may be selected according to any combination described in Table 11 which comprises miR.660. In a particular embodiment, the miRNA cluster consists in the combination miR.558 + miR.609 + miR.520f + miR.29a + miR.362 + miR.369.3p + miR.376a* + miR.518c + miR.639 + miR.660 + miR.338.

In a particular embodiment, the miRNA cluster consists in the combination of 12 miRNAs. For example, said miRNA cluster may be selected according to any combination described in Table 12 which comprises miR.660. In a particular embodiment, the miRNA cluster consists in the combination miR.558 + miR.609 + miR.520f + miR.220a + miR.29a + miR.362 + miR.369.3p + miR.376a* + miR.518c + miR.639 + miR.660 + miR.338.

In a particular embodiment, the miRNA cluster consists in the combination of 13 miRNAs. For example, said miRNA cluster may be selected according to any combination described in Table 13 which comprises miR.660. In a particular embodiment, the miRNA cluster consists in the combination miR.558 + miR.609 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 14 miRNAs. For example, said miRNA cluster may be selected according to any combination described in Table 14 which comprises miR.660. In a particular embodiment, the miRNA cluster consists in the combination miR.558 + miR.609 + miR.603 + miR.520f + miR.130a + miR.29a + miR.362 + miR.369.3p + miR.376a* + miR.494 + miR.518c + miR.639 + miR.660 + miR.338.

In a particular embodiment, the miRNA cluster consists in the combination of 15 miRNAs. For example, said miRNA cluster may be selected according to any combination described in Table 15 which comprises miR.660. In a particular embodiment, the miRNA cluster consists in the combination miR.558 + miR.609 + miR.603 + miR.520f + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 16 miRNAs. For example, said miRNA cluster may be selected according to any combination described in Table 16 which comprises miR.639. In a particular embodiment, the miRNA cluster consists in the combination miR.558 + miR.609 + miR.603 + miR.520f + miR.220a + miR.130a + miR.26a + miR.29a + miR.362 + miR.369.3p + miR.376a* + miR.494 + miR.518c + miR.639 + miR.660 + miR.338.

In a particular embodiment, the miRNA cluster consists in the combination of 17 miRNAs. In a particular embodiment, the miRNA cluster consists in the combination miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338.

### Techniques for measuring the expression level of the miRNA clusters:

Measuring the expression level of the miRNA clusters of the invention in the sample obtained form the patient can be performed by a variety of techniques.

For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted miRNAs is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous. Other methods of Amplification include ligase chain reaction (LCR), transcription-mediated amplification (TMA), strand displacement amplification (SDA) and nucleic acid sequence based amplification (NASBA).

In a particular embodiment, the determination comprises contacting the sample with selective reagents such as probes or primers and thereby detecting the presence, or measuring the amount of miRNAs originally in the sample. Contacting may be performed in any suitable device, such as a plate, microtiter dish, test tube, well, glass, column, and so forth In specific embodiments, the contacting is performed on a substrate coated with the reagent, such as a miRNA array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a miRNAs hybrid, to be formed between the reagent and the miRNAs of the sample.

Nucleic acids exhibiting sequence complementarity or homology to the miRNAs of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

The probes and primers are "specific" to the miRNAs they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

Accordingly, the present invention concerns the preparation and use of miRNA arrays or miRNA probe arrays, which are macroarrays or microarrays of nucleic acid molecules (probes) that are fully or nearly complementary or identical to a plurality of miRNA molecules positioned on a support or support material in a spatially separated organization. Macroarrays are typically sheets of nitrocellulose or nylon upon which probes have been spotted. Microarrays position the nucleic acid probes more densely such that up to 10,000 nucleic acid molecules can be fit into a region typically 1 to 4 square centimeters. Microarrays can be fabricated by spotting nucleic acid molecules, e.g., genes, oligonucleotides, etc., onto substrates or fabricating oligonucleotide sequences in situ on a substrate. Spotted or fabricated nucleic acid molecules can be applied in a high density matrix pattern of up to about 30 non-identical nucleic acid molecules per square centimeter or higher, e.g. up to about 100 or even 1000 per square centimeter. Microarrays typically use coated glass as the solid support, in contrast to the nitrocellulose-based material of filter arrays. By having an ordered array of miRNA-complementing nucleic acid samples, the position of each sample can be tracked and linked to the original sample. A variety of different array devices in which a plurality of distinct nucleic acid probes are stably associated with the surface of a solid support are known to those of skill in the art. Useful substrates for arrays include nylon, glass, metal, plastic, latex, and silicon. Such arrays may vary in a number of different ways, including average probe length, sequence or types of probes, nature of bond between the probe and the array surface, e.g. covalent or non-covalent, and the like.

After an array or a set of miRNA probes is prepared and/or the miRNA in the sample or miRNA probe is labeled, the population of target nucleic acids is contacted with the array or probes under hybridization conditions, where such conditions can be adjusted, as desired, to provide for an optimum level of specificity in view of the particular assay being performed. Suitable hybridization conditions are well known to those of skill in the art and reviewed in Sambrook et al. (2001). Of particular interest in many embodiments is the use of stringent conditions during hybridization. Stringent conditions are known to those of skill in the art.

Alternatively, miRNAs quantification method may be performed by using stem-loop primers for reverse transcription (RT) followed by a real-time TaqMan® probe. Typically, said method comprises a first step wherein the stem-loop primers are annealed to miRNA targets and extended in the presence of reverse transcriptase. Then miRNA-specific forward primer, TaqMan® probe, and reverse primer are used for PCR reactions. Quantitation of miRNAs is estimated based on measured CT values.

Many miRNA quantification assays are commercially available from Qiagen (S. A. Courtaboeuf, France) or Applied Biosystems (Foster City, USA).

Expression level of a miRNA may be expressed as absolute expression level or normalized expression level. Typically, expression levels are normalized by correcting the absolute expression level of a miRNA by comparing its expression to the expression of a mRNA that is not a relevant for determining the outcome of the cancer in the patient, e.g., a housekeeping mRNA that is constitutively expressed. Suitable mRNA for normalization include housekeeping mRNAs such as the U6, U24, U48 and S18. This normalization allows the comparison of the expression level in one sample, e.g., a patient sample, to another sample, or between samples from different sources.

### Reference levels

It is specifically contemplated that the invention can be used for predicting the outcome of a cancer in a patient.

Therefore the methods of the invention further comprises a step consisting of comparing the expression level of one miRNA cluster (as above described) determined in the sample of the patient with a reference expression level of said miRNA cluster, wherein a difference between said expression levels is indicative of the outcome of the cancer in the patient.

Typically, the reference expression levels according to the invention are correlated with an outcome. The reference expression levels may thus consist in the expression level of said miRNA cluster in a tissue representative of an outcome. Accordingly, the reference levels may be predetermined by carrying out a method comprising the steps of a) providing at least one collection of tumor tissue samples selected from the group consisting of a collection of tumor tissue samples from cancer patients having different outcomes, b) determining for each tumor tissue sample comprised in a collection of tumor tissue samples provided at step a), the expression level of said miRNA clusters.

Typically, the expression level determined for one miRNA cluster may be also expressed as a score. Typically said score may be obtained by i) determining for every gene of the cluster their expression level in the sample ii) allocating for every miRNA a positive coefficient (e.g. +1) when the miRNA is associated with a good prognosis and a negative coefficient (e.g. -1) when the miRNA is associated with a bad prognosis and iii) multiplying the expression level of one miRNA with the coefficient allocated in step ii) and iv) adding up all values determining at step iii) for obtaining said score. Typically a coefficient of +1 is allocated to the miRNAs associated with a good prognosis, namely miR.609, miR.519b, miR.520f, miR.558, miR.603 and miR.220a and a coefficient of -1 is allocated to the genes associates with a bad prognosis, namely miR.376a*, miR.639, miR.130a, miR.338, miR.26a, miR.29a, miR.494, miR.518c, miR.660, miR.369-3p and miR.362. Preferably, the expression levels are expressed as normalized expression levels as above described.

The advantage of said score is to make easier the comparison step with the reference levels that may be expressed as "cut-off values". For example the reference ("cut-off") value represents the score calculated for the miRNA cluster of interest in a tissue sample representative of a specific cancer outcome. The cut-off values may also be predetermined by carrying out a method comprising the steps of:
a) selecting a miRNA cluster for which a reference value is to be determined;
b) providing a collection of tumor tissue samples from cancer patients;
c) providing, for each tumor sample provided at step b), information relating to the actual clinical outcome for the corresponding cancer patient;
d) providing a serial of arbitrary values for said miRNA cluster obtained at step a)
e) calculated the score of said miRNA cluster provided at step a) for each tumor tissue sample contained in the collection provided at step b)
f) classifying said tumor samples in two groups for one specific arbitrary value provided at step c), respectively :
   (i) a first group comprising tumor samples that exhibit a score for said miRNA cluster that is lower than the said arbitrary value contained in the said serial of values;
   (ii) a second group comprising tumor samples that exhibit a score for said miRNA cluster that is higher than said arbitrary value contained in the said serial of values;
      whereby two groups of tumor samples are obtained for the said specific value, wherein the tumors samples of each group are separately enumerated;
g) calculating the statistical significance between (i) the score for said miRNA cluster obtained at step e) and (ii) the actual clinical outcome of the patients from which tumor samples contained in the first and second groups defined at step f) derive;
h) reiterating steps f) and g) until every arbitrary value provided at step d) is tested;
i) setting the said reference value ("cut-off" value) as consisting of the arbitrary value for which the highest statistical significance (most significant) has been calculated at step g). As it is disclosed above, said method allows the setting of a single "cut-off" value permitting discrimination between bad and good outcome prognosis. Practically, high statistical significance values (e.g. low P values) are generally obtained for a range of successive arbitrary values, and not only for a single arbitrary value. Thus, in one alternative embodiment of the method of determining "cut-off" values above, a minimal statistical significance value (minimal threshold of significance, e.g. maximal threshold P value) is arbitrarily set and the range of arbitrary values for which the statistical significance value calculated at step g) is higher (more significant, e.g. lower P value) are retained, whereby a range of values is provided. Said range of values consist of a "cut-off" value according to the invention. According to this specific embodiment of a "cut-off" value, bad or good clinical outcome prognosis can be determined by comparing the score obtained for the miRNA cluster with the range of values delimiting said "cut-off" value. In certain embodiments, a cut-off value consisting of a range of values for the considered miRNA cluster, consists of a range of values centred on the value for which the highest statistical significance value is found (e.g. generally the minimum P value which is found). Typically, the cut-off values as above described are determined from a cohort of patient that has a sufficient size enough for allowing a reproducible and accurate discrimination between patients with good prognosis and patients with bad prognosis.

In particular embodiment, the cut-off values as described above may be reported in a table, so that the physician can compare the score obtained for a patient with said values and can easily determined the cancer outcome for said patient.

### Cancers:

The methods of the invention may be performed for any type of cancers selected from the group consisting of adrenal cortical cancer, anal cancer, bile duct cancer (e.g. periphilar cancer, distal bile duct cancer, intrahepatic bile duct cancer), bladder cancer, bone cancer (e.g. osteoblastoma, osteochrondroma, hemangioma, chondromyxoid fibroma, osteosarcoma, chondrosarcoma, fibrosarcoma, malignant fibrous histiocytoma, giant cell tumor of the bone, chordoma, lymphoma, multiple myeloma), brain and central nervous system cancer (e.g. meningioma, astocytoma, oligodendrogliomas, ependymoma, gliomas, medulloblastoma, ganglioglioma, Schwannoma, germinoma, craniopharyngioma), breast cancer (e.g. ductal carcinoma in situ, infiltrating ductal carcinoma, infiltrating, lobular carcinoma, lobular carcinoma in, situ, gynecomastia), Castleman disease (e.g. giant lymph node hyperplasia, angiofollicular lymph node hyperplasia), cervical cancer, colorectal cancer, endometrial cancer (e.g. endometrial adenocarcinoma, adenocanthoma, papillary serous adnocarcinroma, clear cell), esophagus cancer, gallbladder cancer (mucinous adenocarcinoma, small cell carcinoma), gastrointestinal carcinoid tumors (e.g. choriocarcinoma, chorioadenoma destruens), Hodgkin's disease, non-Hodgkin's lymphoma, Kaposi's sarcoma, kidney cancer (e.g. renal cell cancer), laryngeal and hypopharyngeal cancer, liver cancer (e.g. hemangioma, hepatic adenoma, focal nodular hyperplasia, hepatocellular carcinoma), lung cancer (e.g. small cell lung cancer, non-small cell lung cancer), mesothelioma, plasmacytoma, nasal cavity and paranasal sinus cancer (e.g. esthesioneuroblastoma, midline granuloma), nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma (e.g. embryonal rhabdomyosarcoma, alveolar rhabdomyosarcoma, pleomorphic rhabdomyosarcoma), salivary gland cancer, skin cancer (e.g. melanoma, nonmelanoma skin cancer), stomach cancer, testicular cancer (e.g. seminoma, nonseminoma germ cell cancer), thymus cancer, thyroid cancer (e.g. follicular carcinoma, anaplastic carcinoma, poorly differentiated carcinoma, medullary thyroid carcinoma, thyroid lymphoma), vaginal cancer, vulvar cancer, and uterine cancer (e.g. uterine leiomyosarcoma). In a particular embodiment, the cancer is a colorectal cancer.

In a particular embodiment, the cancer is at Stage I, II, III, or IV as determined by the TNM classification, but however the present invention is accurately useful for predicting the outcome of the cancer when said cancer has been classified as Stage I, II or III by the TNM classification.

Methods of the invention can be applied for monitoring the treatment (e.g., drug compounds) of the patient. For example, the effectiveness of an agent to affect the expression level of the miRNA cluster (as herein after described) according to the invention can be monitored during treatments of patients receiving anti-cancer treatments.

The "anti-cancer treatment" that is referred to in the definition of step a) above relate to any type of cancer therapy undergone by the cancer patients previously to collecting the tumor tissue samples, including radiotherapy, chemotherapy and surgery, e.g. surgical resection of the tumor.

Accordingly, the present invention relates to a method for monitoring the treatment of patient affected with a cancer, said method comprising the steps consisting of:
i) predicting the outcome of said cancer before said treatment by performing the method of the invention
ii) predicting the outcome of said cancer after said treatment by performing the method of the invention
iii) and comparing the outcome predicted a step i) with the outcome predicted at step ii) wherein a difference between said outcomes is indicative of the effectiveness of the treatment.

The present invention also relates to a method for predicting the outcome of a cancer in a patient, wherein said method may be used independently from conventional clinicopatholological cancer staging methods, and which method comprising determining the expression level of a miRNA cluster according to the invention.

### Kits:

A further object of the invention relates to kits for performing the methods of the invention, wherein said kits comprise means for measuring the expression level of the miRNA clusters of the invention in the sample obtained from the patient. The kits may include probes, primers macroarrays or microarrays as above described.

For example, the kit may comprise a set of miRNA probes as above defined, usually made of DNA, and that may be pre-labelled. Alternatively, probes may be unlabelled and the ingredients for labelling may be included in the kit in separate containers. The kit may further comprise hybridization reagents or other suitably packaged reagents and materials needed for the particular hybridization protocol, including solid-phase matrices, if applicable, and standards.

Alternatively the kit of the invention may comprise amplification primers (e.g. stem-loop primers) that may be pre-labelled or may contain an affinity purification or attachment moiety. The kit may further comprise amplification reagents and also other suitably packaged reagents and materials needed for the particular amplification protocol.

In a particular embodiment, the kit of the invention comprises means for determining the expression level of a miRNA cluster in a sample obtained from said patient, wherein said miRNA cluster comprises miR.609 or miR.558, or miR.603, or miR.518c, or miR.520f, or miR.362, or miR.220a, or miR.29a, or miR.519b, or miR.494, or miR.130a, or miR.639, or miR.660.

In a particular embodiment, the kit of the invention comprises means for determining the expression level of a miRNA represented by any combination described in Table 2, 3, 4, 5, 6, 7, 8, 9, 10, 11, 12, 13, 14, 15, or 16 which comprises miR.609 or miR.558, or miR.603, or miR.518c, or miR.520f, or miR.362, or miR.220a, or miR.29a, or miR.519b, or miR.494, or miR.130a, or miR.639, or miR.660.

The invention will be further illustrated by the following figures and examples. However, these examples and figures should not be interpreted in any way as limiting the scope of the present invention.

### FIGURES:

**Figure 1****: DFS KM curves for the two clusters. Cluster1 and Cluster 2 with good outcome are merged.** The hazard ratio (HR) is 10.1, the concordance index (CI) is 70.2 and the Brier Score (BS) is 0.23.
**Figure 2****: Pearson correlation matrix for the final selected 17 miRNAs for the deltaCt qPCR data on 77 patients, clustered using euclidean distance.**
**Figure 3****: DFS KM curves based on the dichotomized linear predictor of the fitted Cox model for all 17 miRNAs at 0 months follow-up.** The all over hazard ratio (HR) is 5.12 (logrank P value < 0.001) and the Brier Score (BS) is 0.205.

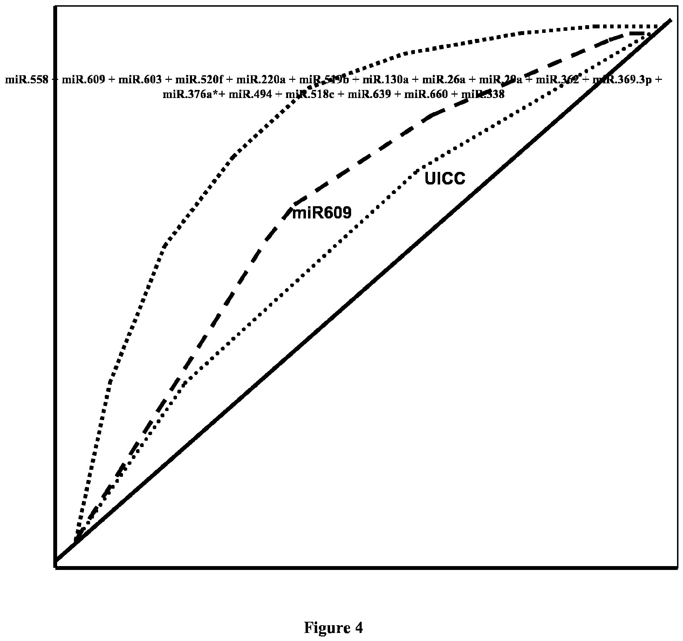

**Figure 4****: ROC curves for the final selected 17 miRNAs, miR609, and UICC.**

### EXAMPLE:

### Material & Methods

**Patients and data:** The records of colorectal cancer (CRC) patients who underwent a primary resection of their tumor at the Laennec-HEGP Hospitals between 1996 and 2004 were reviewed and previously described (Galon et al. 2006). All frozen tumor samples (n=77) from UICC-TNM Stage I-III patients available from Laennec-HEGP Hospitals from 1996-2004, with sufficient RNA quality and quantity, were selected. The RNA samples analyzed were from 77 different patients. These patients were used for miRNA expression experiments (Taqman qPCR). The observation time in the cohorts was the interval between diagnosis and last contact (death or last follow-up). Data were censored at the last follow-up for patients without relapse, or death. The min:max values until progression/death or last follow-up were (0:136) months, respectively. Eight patients with incomplete data records were excluded from the analysis. Time to recurrence or disease-free time was defined as the interval from the date of surgery to confirmed tumor relapse date for relapsed patients and from the date of surgery to the date of last follow-up for disease-free patients.

Histopathological and clinical findings were scored according to the UICC-TNM staging system. Follow-up data were collected prospectively and updated. A secure Web-based database, TME.db (Tumor MicroEnvironment Database), was built on a 3-tier architecture using Java-2 Enterprise-Edition (J2EE) to integrate the clinical data and the data from high-throughput technologies. Ethical, Legal and Social Implications were approved by ethical review board.

**Statistical analysis:** All signatures were build based on 69 patients (8 patients were removed due to missing values) from a UICC-TNM Stage I-III CRC patient cohort and all 17 selected final miRs. The predictive performance for each miRNA combination was assessed by the Harrel's concordance index (c-index) and time-dependent c-index (C_{τ} index) (R risksetROC package) and correspond to the area under the receiver operator curve (AUC) (c-index (Harrell FE et al. 1996) and iAUC (Heagerty PJ et al., 2005), respectively). A summary table for each possible model combination length from 1 to 16 miRs was created and sorted by the highest C_{τ} index. Additionally each table shows the number of miRNAs remaining significant in the cox model and a P-value (U-statistics, Pencina MJ et al., 2008) comparing the performance of all models of a certain number of miRNAs with the one with the highest performance (largest C_{τ} index) (R risksetROC and Hmisc package).

Time-dependent area under the ROC(t) (iAUC): Since the event occurrence is time-dependent, time-dependent ROC curves are more appropriate than conventional ones (Heagerty PJ et al., 2005). Heagerty PJ et al. proposed to summarize the discrimination potential of a risk score, estimated at the diagnosis/surgery time t=0, by calculating ROC curves for cumulative event occurrence by time t. From the ROC curve ROC(t) the integrated area under the curve over time (iAUC) can be calculated. The larger the iAUC at time t, the better is the predictability of the time to event (TTE) at time t as well as the average predictability of the TTE.

The concordance index (c-index): The concordance index (c-index) calculates the probability that, for a pair of randomly chosen comparable patients, the patient with the higher risk prediction will experience an event before the lower risk patient. The c-index is a generalization of the AUC(t) (iAUC), an can not represent the evolution of performance with respect to time (Harrell FE et al., 1996). The larger c-index, the better is the predictability of the time to event (TTE).

### Results

**Selection of the relevant miRNAs:** In a cohort of 77 Stage I/II/III patients for 365 micro-RNAs (miRNAs) we determined the logrank significance of each single marker using the minimal P-value approach for the dichotomization. The obtained P-values were corrected using Altman et al. (Altman, D. G., Lausen, B., Sauerbrei, W. & Schumacher, M. Dangers of using "optimal" cutpoints in the evaluation of prognostic factors. J Natl Cancer Inst, 1994;86:829-35.) and were additionally cross validated. The Hazard ratios obtained with the minimal P-value dichotomization were corrected using Hollaender et al. (Hollaender N, Sauerbrei W, Schumacher M. Confidence intervals for the effect of a prognostic factor after selection of an 'optimal' cutpoint. Stat Med, 2004;23(11):1701-13.). The final selection of the miRNAs was based on the median cross validation P-value. We removed from the final miRNA signature miRNAs which were not expressed (none of the determined values were larger than deltaCt > 32) although they where logrank significant. We finally end up with 17 markers, where 6 show a good out come when high expressed and 11 show a bad outcome when high expressed (see Tablle B). All analyzes were performed using the R survival package.

**Table B: Final selected miRNAs**

| **Marker** | **median Hazard ratio (HR)** | **median logrank P value** | **optimal Hazard ratio (HR)** | **optimal Hazard ratio corrected** | **optimal logrank P value** | **optimal logrank P value corrected** | **optimal logrank P value cross validated** |
|---|---|---|---|---|---|---|---|
| **miR.609** | 0.239 | 0.0057 | 0.194 | 0.24 | 0.0012 | 0.0576 | 0.0276 |
| **miR.519b** | 0.312 | 0.0209 | 0.251 | 0.31 | 0.0033 | 0.1359 | 0.0431 |
| **miR.520f** | 0.321 | 0.0217 | 0.252 | 0.31 | 0.0016 | 0.0731 | 0.0651 |
| **miR.558** | 0.334 | 0.0198 | 0.137 | 0.19 | 0.0019 | 0.0866 | 0.0389 |
| **miR.603** | 0.337 | 0.0213 | 0.132 | 0.18 | 0.0014 | 0.0682 | 0.0299 |
| **miR.220a** | 0.351 | 0.029 | 0.144 | 0.20 | 0.0027 | 0.1162 | 0.0531 |
| **miR.376a*** | 1.199 | 0.6935 | 3.812 | 3.23 | 0.0023 | 0.1022 | 0.0336 |
| **miR.639** | 1.339 | 0.5253 | 6.338 | 4.55 | 0.0047 | 0.1774 | 0.0231 |
| **miR.130a** | 1.635 | 0.2868 | 6.545 | 4.76 | 0.0041 | 0.1596 | 0.0316 |
| **miR.338** | 1.687 | 0.2861 | 4.863 | 3.70 | 0.0005 | 0.0278 | 0.0146 |
| **miR.26a** | 1.861 | 0.1857 | 5.04 | 3.85 | 0.0045 | 0.1707 | 0.0416 |
| **miR.29a** | 2.562 | 0.0483 | 7.257 | 5.26 | 0.0019 | 0.0877 | 0.0547 |
| **miR.494** | 3.333 | 0.0149 | 4.415 | 3.57 | 0.0042 | 0.1616 | 0.0406 |
| **miR.518c** | 3.514 | 0.0103 | 4.694 | 3.85 | 0.0011 | 0.0563 | 0.0214 |
| **miR.660** | 3.743 | 0.0119 | 4.67 | 3.85 | 0.0026 | 0.1118 | 0.054: |
| **miR.369-3p** | 4.533 | 0.0033 | 5.934 | 4.76 | 0.0013 | 0.0649 | 0.012 |
| **miR.362** | 4.634 | 0.0028 | 4.899 | 4.00 | 0.0018 | 0.0818 | 0.0215 |

6 miRNAs show a good out come (HR < 1) when high expressed: miR.609, miR.519b, miR.520f, miR.558, miR.603 and miR.220a.

11 miRNAs show a bad outcome (HR > 1) when high expressed: miR.376a*, miR.639, miR.130a, miR.338, miR.26a, miR.29a, miR.494, miR.518c, miR.660, miR.369-3p and miR.362.

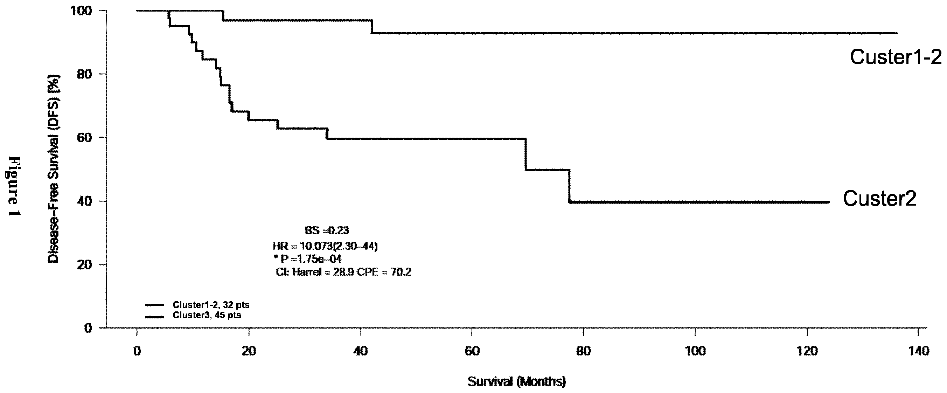

**Cluster for the 17 final selected signature miRNA qPCR data clustered on 77 patients (Stage I-III):** Before clustering the deltaCt qPCR data for each miRNA was mean centered and divided by its standard deviation. The miRNAs where hierarchical clustered using the complete linkage algorithm and Pearson correlation as distance measurement. Three major patient clusters were defined, two with good outcome (Cluster1-2) and one with poor outcome (Cluster3) (Figure 1).

**Time-dependent ROC Curves and iAUC (Time-dependent predictive accuracy) using Cox regression models for disease-free survival (DFS):** In the cohorts of Stage I/II, Stage I/II/III and Stage III patients using the 17 final selected micro-RNAs (miRNAs) as well as Version1 and Version2 of the clustered data we determined the time-dependent predictive accuracy (integrated Area Under the ROC curves, iAUC) (Heagerty PJ, Zheng Y. Survival model predictive accuracy and ROC curves. Biometrics, 2005, Mar;61(1):92-105.)

All signatures show stable time-dependent ROC curves and none of the signatures violate the cox proportional hazards assumption.

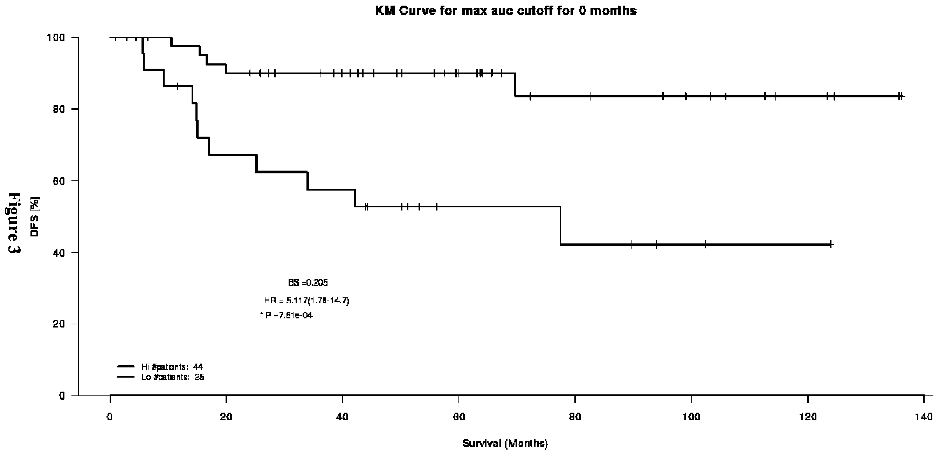

The ROC and AUC curves were drawn based on the linear predictor of a fitted Cox regression model for the respective miRNA or miRNA combination were the miRNA data entered un-dichotimized into the model. Each circle in the iAUC plot indicates an event (relapse) of a patient (Figure3). The ROC and AUC curves were calculated based on the LocalCox method in case the marker was violating the Hazards assumptions, otherwise the Cox method was used. The analyzes were performed using the R survival and risksetROC packages. All AUC characteristics for all combination markers are described in Table 1, 2, 3, 4, 5, 6, 7, 8, 9, 10, 11, 12, 13, 14, 15, and 16. The best combinations are described in Table 17.

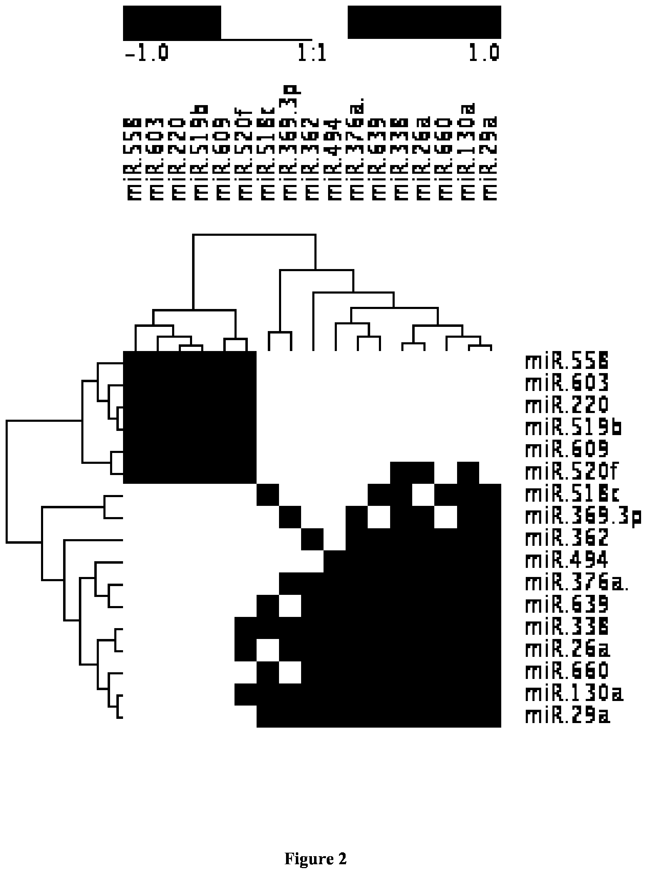

**Correlation matrix cluster for the 17 final selected signature miRNA qPCR data clustered on 77patients (Stage I-III):** The correlation matrix cluster show two major cluster (Figure 2). A high correlation cluster with the six miRs with good outcome and a high correlation cluster with the poor outcome miRNAs. Negative correlation can be seen between those two clusters showing an inverse expression of those miRNAs. A high correlation pattern between miRNAs show a probable redundancy among those miRNAs. E.g. miR.29a and miR130a have a highly similar correlation pattern as well as miR.26a and miR.338, which would suggest a possible replacement by one of them. Other examples are described in the followings Tables:

**Table C**

| 4 Marker_Combination all miRs | c_tau |
|---|---|
| miR.558 + **miR.603** + **miR.220a** + miR.518c | 0.7646 |
| miR.558 + **miR.609** + **miR.519b** + miR.518c | 0.7639 |

**Table D**

| 4 Marker Combination all miRs | c_tau |
|---|---|
| miR.609 + miR.26a + **miR.29a** + miR.518c | 0.7609 |
| miR.609 + miR.603 + **miR.130a** + miR.518c | 0.7608 |

**Table E**

| 5 Marker Combination all miRs | c_tau |
|---|---|
| miR.609 + **miR.603** + miR.29a + miR.376a* + miR.518c | 0.7983 |
| miR.609 + **miR.558** + miR.29a + miR.376a* + miR.518c | 0.7913 |

**Table F**

| 6 Marker Combination all miRs | c_tau |
|---|---|
| miR.558 + miR.609 + **miR.220a** + miR.29a + miR.376a* + miR.518c | 0.8060 |
| miR.558 + miR.609 + **miR.520f +** miR.29a + miR.376a* + miR.518c | 0.8050 |

**Table G**

| 7 Marker_Combination all patients | c_tau |
|---|---|
| miR.558 + miR.609 + miR.220a + miR.29a + **miR.660** + miR.376a* + miR.518c | 0.8066 |
| miR.558 + miR.609 + miR.220a + miR.29a + **miR.130a** + miR.376a* + miR.518c | 0.8063 |

**Table H**

| 8 Marker_Combination all patients | c_tau |
|---|---|
| miR.558 + miR.609 + miR.220a + **miR.26a** + miR.29a + miR.369.3p + miR.376a* + miR.518c | 0.8113 |
| miR.558 + miR.609 + miR.220a + **miR.660** + miR.29a + miR.369.3p + miR.376a* + miR.518c | 0.8111 |

Some of the miRNAs are redundant and may share common biological functions which makes them replaceable among each other. This is of advantage in case some the miRNAs are not expressed or are not measurable because of technical reasons.

**Table 1: clusters of 1 miRNA**

| **Cluster of 1 miR** | **c_tau level** | **pv compared to top signature miR.609** |
|---|---|---|
| miR.609 | 0,66 | NaN |
| miR.558 | 0,66 | 0,418938974 |
| miR.603 | 0,66 | 0,190043508 |
| miR.518c | 0,65 | 0,394113397 |
| miR.520f | 0,64 | 0,483494013 |
| miR.362 | 0,64 | 0,432657709 |
| miR.220a | 0,63 | 0,436890827 |
| miR.29a | 0,62 | 0,312018624 |
| miR.519b | 0,62 | 0,226161064 |
| miR.494 | 0,61 | 0,158371367 |
| miR.130a | 0,6 | 0,189293501 |
| miR.639 | 0,6 | 0,168935983 |
| miR.660 | 0,59 | 0,170064551 |
| **UICC** | **0,59** | **NaN** |
| miR.26a | 0,57 | 0,059277098 |
| miR.369.3p | 0,55 | 0,08341555 |
| miR.338 | 0,54 | 0,054392511 |
| miR.376a* | 0,54 | 0,015577754 |

**Table 2: clusters of 2 miRNAs**

| **Cluster of 2 miRs** | **c_tau level** | **pv compared to top signature miR.603 + miR.518c** |
|---|---|---|
| miR.603 + miR.518c | 0,73 | NaN |
| miR.558 + miR.518c | 0,73 | 0,009268576 |
| miR.609 + miR.29a | 0,72 | 0,423370924 |
| miR.558 + miR.609 | 0,72 | 0,482705956 |
| miR.220a + miR.518c | 0,72 | 0,001073457 |
| miR.609 + miR.518c | 0,72 | 0,134186365 |
| miR.558 + miR.520f | 0,7 | 0,171004128 |
| miR.609 + miR.520f | 0,7 | 0,167116274 |
| miR.558 + miR.603 | 0,7 | 0,188187249 |
| miR.519b + miR.518c | 0,69 | 0,000904262 |
| miR.558 + miR.362 | 0,69 | 0,086851866 |
| miR.558 + miR.29a | 0,69 | 0,122648778 |
| miR.520f + miR.29a | 0,69 | 0,14900018 |
| miR.603 + miR.29a | 0,68 | 0,02436506 |
| miR.603 + miR.494 | 0,68 | 0,066852154 |
| miR.520f + miR.130a | 0,68 | 0,1378426 |
| miR.520f + miR.26a | 0,68 | 0,138396835 |
| miR.603 + miR.130a | 0,68 | 0,044534014 |
| miR.603 + miR.362 | 0,68 | 0,01236297 |
| miR.558 + miR.130a | 0,68 | 0,104285811 |
| miR.603 + miR.639 | 0,68 | 0,038208676 |
| miR.603 + miR.369.3p | 0,67 | 0,094691498 |
| miR.603 + miR.660 | 0,67 | 0,01385758 |
| miR.603 + miR.220a | 0,67 | 0,033359616 |
| miR.29a + miR.518c | 0,67 | 0,038492703 |
| miR.220a + miR.29a | 0,66 | 0,03205418 |
| miR.369.3p + miR.518c | 0,66 | 0,014816909 |
| miR.603 + miR.26a | 0,66 | 0,044908534 |
| miR.220a + miR.362 | 0,66 | 0,011719433 |
| miR.603 + miR.338 | 0,66 | 0,04870573 |
| miR.519b + miR.29a | 0,65 | 0,009625124 |
| miR.220a + miR.130a | 0,65 | 0,032322123 |
| miR.130a + miR.362 | 0,65 | 0,033378678 |

**Table 3: clusters of 3 miRNAs**

| **Cluster of 3 miRs** | **c_tau level** | **pv compared to top signature miR.558 + miR.609 + miR.518c** |
|---|---|---|
| miR.558 + miR.609 + miR.518c | 0,76 | NaN |
| miR.558 + miR.603 + miR.518c | 0,75 | 0,247784245 |
| miR.609 + miR.29a + miR.518c | 0,75 | 0,164812866 |
| miR.603 + miR.220a + miR.518c | 0,75 | 0,488976647 |
| miR.609 + miR.603 + miR.518c | 0,75 | 0,34714351 |
| miR.558 + miR.520f + miR.518c | 0,75 | 0,362175562 |
| miR.558 + miR.609 + miR.29a | 0,75 | 0,355216853 |
| miR.609 + miR.520f + miR.29a | 0,75 | 0,348153894 |
| miR.603 + miR.520f + miR.518c | 0,74 | 0,425658902 |
| miR.609 + miR.29a + miR.376a* | 0,74 | 0,194993588 |
| miR.609 + miR.220a + miR.518c | 0,74 | 0,049347643 |
| miR.558 + miR.220a + miR.518c | 0,74 | 0,101338262 |
| miR.603 + miR.376a* + miR.518c | 0,74 | 0,346484887 |
| miR.558 + miR.609 + miR.520f | 0,74 | 0,140417039 |
| miR.609 + miR.518c + miR.639 | 0,74 | 0,351186289 |
| miR.603 + miR.518c + miR.639 | 0,74 | 0,280054707 |
| miR.609 + miR.130a + miR.518c | 0,74 | 0,449352384 |
| miR.520f + miR.220a + miR.518c | 0,74 | 0,402122181 |
| miR.603 + miR.369.3p + miR.518c | 0,74 | 0,273671635 |
| miR.558 + miR.609 + miR.603 | 0,73 | 0,108306893 |
| miR.603 + miR.362 + miR.518c | 0,73 | 0,293305717 |
| miR.520f + miR.518c + miR.639 | 0,73 | 0,35243061 |
| miR.609 + miR.520f + miR.518c | 0,73 | 0,30176526 |
| miR.603 + miR.29a + miR.518c | 0,73 | 0,271112606 |
| miR.603 + miR.26a + miR.518c | 0,73 | 0,285703084 |
| miR.558 + miR.376a* + miR.518c | 0,73 | 0,315129807 |
| miR.558 + miR.609 + miR.639 | 0,73 | 0,104170851 |
| miR.609 + miR.520f + miR.130a | 0,73 | 0,370027972 |
| miR.603 + miR.518c + miR.660 | 0,73 | 0,216991425 |
| miR.603 + miR.130a + miR.518c | 0,73 | 0,251376103 |
| miR.603 + miR.518c + miR.338 | 0,73 | 0,212164512 |
| miR.603 + miR.519b + miR.518c | 0,73 | 0,185914027 |
| miR.558 + miR.609 + miR.130a | 0,73 | 0,078091571 |
| miR.603 + miR.494 + miR.518c | 0,73 | 0,173052451 |
| miR.558 + miR.362 + miR.518c | 0,73 | 0,069189409 |
| miR.558 + miR.609 + miR.220a | 0,73 | 0,039602125 |
| miR.558 + miR.609 + miR.362 | 0,73 | 0,021740781 |
| miR.609 + miR.603 + miR.29a | 0,73 | 0,397990999 |
| miR.520f + miR.29a + miR.518c | 0,73 | 0,404704361 |
| miR.558 + miR.518c + miR.639 | 0,73 | 0,091698305 |
| miR.558 + miR.26a + miR.518c | 0,73 | 0,299248291 |
| miR.558 + miR.29a + miR.518c | 0,73 | 0,09574238 |
| miR.609 + miR.519b + miR.518c | 0,73 | 0,005919178 |
| miR.558 + miR.609 + miR.369.3p | 0,73 | 0,265320988 |
| miR.609 + miR.29a + miR.494 | 0,73 | 0,329298298 |
| miR.609 + miR.220a + miR.29a | 0,73 | 0,471997025 |
| miR.609 + miR.362 + miR.518c | 0,73 | 0,256139437 |
| miR.558 + miR.369.3p + miR.518c | 0,73 | 0,153094875 |
| miR.558 + miR.519b + miR.518c | 0,73 | 0,202589552 |
| miR.558 + miR.518c + miR.660 | 0,73 | 0,090211228 |
| miR.609 + miR.520f + miR.639 | 0,73 | 0,248436815 |
| miR.520f + miR.26a + miR.518c | 0,73 | 0,248670032 |
| miR.558 + miR.494 + miR.518c | 0,73 | 0,102215116 |
| miR.558 + miR.609 + miR.494 | 0,73 | 0,112580737 |
| miR.558 + miR.518c + miR.338 | 0,73 | 0,111295798 |
| miR.558 + miR.130a + miR.518c | 0,73 | 0,097771847 |
| miR.609 + miR.369.3p + miR.518c | 0,73 | 0,292047668 |
| miR.520f + miR.130a + miR.518c | 0,73 | 0,325326497 |
| miR.558 + miR.609 + miR.376a* | 0,73 | 0,260189737 |
| miR.609 + miR.494 + miR.518c | 0,73 | 0,098487012 |
| miR.609 + miR.29a + miR.369.3p | 0,72 | 0,335139361 |
| miR.558 + miR.609 + miR.660 | 0,72 | 0,025079336 |
| miR.609 + miR.26a + miR.29a | 0,72 | 0,43507738 |
| miR.609 + miR.519b + miR.29a | 0,72 | 0,332887111 |
| miR.609 + miR.29a + miR.639 | 0,72 | 0,363228171 |
| miR.520f + miR.519b + miR.518c | 0,72 | 0,170474636 |
| miR.609 + miR.29a + miR.338 | 0,72 | 0,321558317 |
| miR.220a + miR.518c + miR.338 | 0,72 | 0,178086869 |
| miR.220a + miR.29a + miR.518c | 0,72 | 0,137410978 |
| miR.609 + miR.29a + miR.362 | 0,72 | 0,37955572 |
| miR.220a + miR.369.3p + miR.518c | 0,72 | 0,153896555 |
| miR.609 + miR.29a + miR.660 | 0,72 | 0,284984411 |
| miR.558 + miR.609 + miR.26a | 0,72 | 0,057440468 |
| miR.220a + miR.518c + miR.639 | 0,72 | 0,125459545 |
| miR.520f + miR.362 + miR.518c | 0,72 | 0,301355412 |
| miR.609 + miR.520f + miR.26a | 0,72 | 0,312363545 |
| miR.558 + miR.609 + miR.338 | 0,72 | 0,047008735 |
| miR.609 + miR.603 + miR.130a | 0,72 | 0,23533357 |
| miR.609 + miR.130a + miR.29a | 0,72 | 0,370784436 |
| miR.220a + miR.362 + miR.518c | 0,72 | 0,16166902 |
| miR.609 + miR.518c + miR.660 | 0,72 | 0,112066562 |
| miR.220a + miR.376a* + miR.518c | 0,72 | 0,198135987 |
| miR.220a + miR.130a + miR.518c | 0,72 | 0,148439546 |
| miR.558 + miR.609 + miR.519b | 0,72 | 0,088041913 |
| miR.220a + miR.26a + miR.518c | 0,72 | 0,190004241 |
| miR.220a + miR.519b + miR.518c | 0,72 | 0,142304577 |
| miR.220a + miR.494 + miR.518c | 0,72 | 0,157006033 |
| miR.220a + miR.518c + miR.660 | 0,72 | 0,161323893 |
| miR.609 + miR.603 + miR.494 | 0,72 | 0,241519739 |
| miR.609 + miR.603 + miR.639 | 0,72 | 0,251517332 |
| miR.609 + miR.26a + miR.518c | 0,72 | 0,066030989 |
| miR.558 + miR.603 + miR.520f | 0,72 | 0,142937829 |
| miR.520f + miR.518c + miR.660 | 0,72 | 0,243462668 |
| miR.558 + miR.520f + miR.29a | 0,72 | 0,061445518 |
| miR.520f + miR.369.3p + miR.518c | 0,72 | 0,221082215 |
| miR.520f + miR.518c + miR.338 | 0,72 | 0,277129663 |
| miR.609 + miR.362 + miR.369.3p | 0,72 | 0,256027229 |
| miR.609 + miR.603 + miR.520f | 0,72 | 0,165161932 |
| miR.558 + miR.520f + miR.639 | 0,72 | 0,10668753 |
| miR.609 + miR.518c + miR.338 | 0,72 | 0,049918113 |
| miR.609 + miR.130a + miR.376a* | 0,72 | 0,314938145 |
| miR.520f + miR.494 + miR.518c | 0,72 | 0,161170186 |
| miR.609 + miR.603 + miR.220a | 0,72 | 0,17485756 |
| miR.609 + miR.376a* + miR.518c | 0,71 | 0,021158932 |
| miR.558 + miR.520f + miR.362 | 0,71 | 0,042970193 |
| miR.609 + miR.220a + miR.130a | 0,71 | 0,128890701 |
| miR.609 + miR.603 + miR.369.3p | 0,71 | 0,196936889 |
| miR.609 + miR.520f + miR.494 | 0,71 | 0,067113092 |
| miR.558 + miR.520f + miR.130a | 0,71 | 0,032963824 |
| miR.609 + miR.362 + miR.494 | 0,71 | 0,132878392 |
| miR.609 + miR.520f + miR.220a | 0,71 | 0,044047133 |
| miR.609 + miR.603 + miR.362 | 0,71 | 0,081082555 |
| miR.558 + miR.520f + miR.26a | 0,71 | 0,037521396 |
| miR.609 + miR.494 + miR.639 | 0,71 | 0,073255244 |
| miR.609 + miR.220a + miR.494 | 0,71 | 0,17258061 |
| miR.609 + miR.220a + miR.369.3p | 0,71 | 0,105201478 |
| miR.558 + miR.520f + miR.338 | 0,71 | 0,064777816 |
| miR.609 + miR.520f + miR.362 | 0,71 | 0,035840094 |
| miR.558 + miR.520f + miR.660 | 0,71 | 0,043413436 |
| miR.558 + miR.362 + miR.369.3p | 0,71 | 0,037425892 |
| miR.558 + miR.603 + miR.362 | 0,71 | 0,043710943 |
| miR.609 + miR.603 + miR.376a* | 0,71 | 0,128251061 |
| miR.609 + miR.220a + miR.376a* | 0,71 | 0,080434388 |
| miR.558 + miR.603 + miR.29a | 0,71 | 0,050887026 |
| miR.609 + miR.130a + miR.369.3p | 0,71 | 0,097832454 |
| miR.609 + miR.130a + miR.494 | 0,71 | 0,07652331 |
| miR.609 + miR.603 + miR.660 | 0,71 | 0,069081805 |
| miR.609 + miR.603 + miR.26a | 0,71 | 0,119585833 |
| miR.609 + miR.220a + miR.362 | 0,71 | 0,050963096 |
| miR.609 + miR.603 + miR.519b | 0,71 | 0,070308772 |
| miR.558 + miR.603 + miR.130a | 0,71 | 0,028731659 |
| miR.558 + miR.603 + miR.369.3p | 0,71 | 0,051261759 |
| miR.558 + miR.603 + miR.639 | 0,71 | 0,034357969 |
| miR.558 + miR.520f + miR.494 | 0,71 | 0,048911277 |
| miR.603 + miR.520f + miR.29a | 0,71 | 0,216340461 |
| miR.558 + miR.603 + miR.660 | 0,71 | 0,027394144 |
| miR.609 + miR.520f + miR.519b | 0,71 | 0,033679188 |
| miR.609 + miR.520f + miR.338 | 0,7 | 0,052551862 |
| miR.603 + miR.362 + miR.369.3p | 0,7 | 0,198969083 |
| miR.558 + miR.520f + miR.220a | 0,7 | 0,044708648 |
| miR.603 + miR.520f + miR.639 | 0,7 | 0,167994772 |
| miR.609 + miR.603 + miR.338 | 0,7 | 0,084883885 |
| miR.603 + miR.520f + miR.130a | 0,7 | 0,141242618 |
| miR.609 + miR.520f + miR.660 | 0,7 | 0,016329201 |
| miR.609 + miR.369.3p + miR.494 | 0,7 | 0,049507017 |
| miR.558 + miR.520f + miR.369.3p | 0,7 | 0,128349981 |
| miR.609 + miR.520f + miR.369.3p | 0,7 | 0,056625869 |
| miR.362 + miR.369.3p + miR.518c | 0,7 | 0,240334825 |
| miR.558 + miR.603 + miR.494 | 0,7 | 0,021795267 |
| miR.519b + miR.518c + miR.639 | 0,7 | 0,007041137 |
| miR.603 + miR.520f + miR.26a | 0,7 | 0,181375394 |
| miR.609 + miR.519b + miR.362 | 0,7 | 0,017616618 |
| miR.609 + miR.26a + miR.362 | 0,7 | 0,049968858 |
| miR.609 + miR.220a + miR.519b | 0,7 | 0,027688308 |
| miR.558 + miR.603 + miR.220a | 0,7 | 0,04012403 |
| miR.519b + miR.362 + miR.518c | 0,7 | 0,026035969 |
| miR.603 + miR.520f + miR.494 | 0,7 | 0,119510911 |
| miR.519b + miR.376a* + miR.518c | 0,7 | 0,007101906 |
| miR.558 + miR.520f + miR.376a* | 0,7 | 0,148510611 |
| miR.519b + miR.369.3p + miR.518c | 0,7 | 0,006727401 |
| miR.609 + miR.362 + miR.376a* | 0,7 | 0,065078334 |
| miR.558 + miR.520f + miR.519b | 0,7 | 0,159181427 |
| miR.519b + miR.29a + miR.518c | 0,7 | 0,025523668 |
| miR.603 + miR.520f + miR.362 | 0,7 | 0,142734333 |
| miR.609 + miR.362 + miR.338 | 0,7 | 0,039368124 |
| miR.603 + miR.362 + miR.494 | 0,7 | 0,09934405 |
| miR.609 + miR.494 + miR.660 | 0,7 | 0,041673346 |
| miR.558 + miR.29a + miR.362 | 0,7 | 0,004731426 |
| miR.609 + miR.520f + miR.376a* | 0,7 | 0,020124337 |
| miR.558 + miR.362 + miR.494 | 0,7 | 0,003012638 |
| miR.520f + miR.29a + miR.362 | 0,7 | 0,089888022 |
| miR.558 + miR.603 + miR.338 | 0,7 | 0,015964208 |
| miR.519b + miR.130a + miR.518c | 0,7 | 0,00436048 |
| miR.362 + miR.518c + miR.639 | 0,7 | 0,074343301 |
| miR.558 + miR.362 + miR.639 | 0,7 | 0,003521047 |
| miR.520f + miR.362 + miR.639 | 0,7 | 0,089557122 |
| miR.519b + miR.518c + miR.660 | 0,7 | 0,004070438 |
| miR.519b + miR.494 + miR.518c | 0,7 | 0,002940958 |
| miR.558 + miR.603 + miR.26a | 0,7 | 0,014992291 |
| miR.519b + miR.518c + miR.338 | 0,7 | 0,00107145 |
| miR.558 + miR.603 + miR.519b | 0,7 | 0,03465954 |
| miR.520f + miR.26a + miR.639 | 0,7 | 0,07962049 |
| miR.558 + miR.603 + miR.376a* | 0,7 | 0,031039049 |
| miR.520f + miR.130a + miR.362 | 0,69 | 0,085235769 |
| miR.558 + miR.130a + miR.362 | 0,69 | 0,00601709 |
| miR.520f + miR.26a + miR.29a | 0,69 | 0,087651916 |
| miR.603 + miR.520f + miR.660 | 0,69 | 0,077140142 |
| miR.520f + miR.29a + miR.639 | 0,69 | 0,081746407 |
| miR.520f + miR.29a + miR.494 | 0,69 | 0,065088308 |
| miR.520f + miR.220a + miR.29a | 0,69 | 0,071074473 |
| miR.520f + miR.519b + miR.29a | 0,69 | 0,052203253 |
| miR.603 + miR.520f + miR.220a | 0,69 | 0,13159188 |
| miR.603 + miR.29a + miR.494 | 0,69 | 0,057716574 |
| miR.603 + miR.369.3p + miR.494 | 0,69 | 0,063429906 |
| miR.558 + miR.26a + miR.29a | 0,69 | 0,016454956 |
| miR.520f + miR.26a + miR.494 | 0,69 | 0,037334593 |
| miR.520f + miR.519b + miR.639 | 0,69 | 0,032303599 |
| miR.520f + miR.29a + miR.376a* | 0,69 | 0,135732892 |
| miR.558 + miR.220a + miR.362 | 0,69 | 0,004264036 |
| miR.520f + miR.26a + miR.362 | 0,69 | 0,080410499 |
| miR.362 + miR.494 + miR.518c | 0,69 | 0,07811005 |
| miR.558 + miR.220a + miR.29a | 0,69 | 0,003817785 |
| miR.603 + miR.130a + miR.362 | 0,69 | 0,107693542 |
| miR.520f + miR.362 + miR.369.3p | 0,69 | 0,064838129 |
| miR.603 + miR.520f + miR.369.3p | 0,69 | 0,097355846 |
| miR.603 + miR.520f + miR.338 | 0,69 | 0,083688787 |
| miR.609 + miR.376a* + miR.660 | 0,69 | 0,030919011 |
| miR.558 + miR.29a + miR.369.3p | 0,69 | 0,008209665 |
| miR.603 + miR.220a + miR.494 | 0,69 | 0,09413129 |
| miR.603 + miR.362 + miR.639 | 0,69 | 0,092399431 |
| miR.603 + miR.494 + miR.639 | 0,69 | 0,037277169 |
| miR.603 + miR.29a + miR.362 | 0,69 | 0,097411564 |
| miR.558 + miR.362 + miR.338 | 0,69 | 0,001934736 |
| miR.558 + miR.29a + miR.494 | 0,69 | 0,002735141 |
| miR.558 + miR.29a + miR.639 | 0,69 | 0,00511974 |
| miR.558 + miR.362 + miR.376a* | 0,69 | 0,004511201 |
| miR.520f + miR.362 + miR.494 | 0,69 | 0,026077672 |
| miR.520f + miR.494 + miR.639 | 0,69 | 0,028291164 |
| miR.558 + miR.29a + miR.376a* | 0,69 | 0,022595613 |
| miR.520f + miR.130a + miR.26a | 0,69 | 0,032662435 |
| miR.558 + miR.26a + miR.362 | 0,69 | 0,001724111 |
| miR.520f + miR.26a + miR.376a* | 0,69 | 0,102288809 |
| miR.520f + miR.130a + miR.639 | 0,69 | 0,037949646 |
| miR.558 + miR.29a + miR.660 | 0,69 | 0,00269176 |
| miR.558 + miR.519b + miR.362 | 0,69 | 0,007170633 |
| miR.520f + miR.29a + miR.338 | 0,69 | 0,113544366 |
| miR.603 + miR.494 + miR.660 | 0,69 | 0,030503802 |
| miR.558 + miR.130a + miR.29a | 0,69 | 0,00294133 |
| miR.603 + miR.130a + miR.369.3p | 0,69 | 0,058256247 |
| miR.520f + miR.29a + miR.369.3p | 0,69 | 0,074005777 |
| miR.603 + miR.29a + miR.369.3p | 0,69 | 0,094414449 |
| miR.520f + miR.130a + miR.29a | 0,69 | 0,06100135 |
| miR.520f + miR.29a + miR.660 | 0,69 | 0,077631374 |
| miR.520f + miR.519b + miR.130a | 0,69 | 0,016231919 |
| miR.558 + miR.29a + miR.338 | 0,69 | 0,005031265 |
| miR.603 + miR.220a + miR.29a | 0,69 | 0,090210804 |
| miR.558 + miR.519b + miR.29a | 0,69 | 0,00734062 |
| miR.603 + miR.29a + miR.639 | 0,69 | 0,086681916 |
| miR.520f + miR.639 + miR.338 | 0,69 | 0,062844863 |
| miR.603 + miR.130a + miR.494 | 0,69 | 0,033425556 |
| miR.603 + miR.369.3p + miR.639 | 0,69 | 0,079970621 |
| miR.520f + miR.220a + miR.26a | 0,69 | 0,064168511 |
| miR.603 + miR.220a + miR.130a | 0,69 | 0,064383883 |
| miR.603 + miR.220a + miR.369.3p | 0,69 | 0,102676466 |
| miR.520f + miR.220a + miR.130a | 0,69 | 0,032168586 |
| miR.558 + miR.369.3p + miR.660 | 0,69 | 0,005397536 |
| miR.520f + miR.220a + miR.639 | 0,69 | 0,060100112 |
| miR.520f + miR.639 + miR.660 | 0,69 | 0,044269744 |
| miR.520f + miR.130a + miR.376a* | 0,69 | 0,054980261 |
| miR.558 + miR.369.3p + miR.639 | 0,69 | 0,016041004 |
| miR.603 + miR.376a* + miR.494 | 0,69 | 0,046186987 |
| miR.603 + miR.26a + miR.29a | 0,69 | 0,063535409 |
| miR.603 + miR.369.3p + miR.660 | 0,69 | 0,053000268 |
| miR.603 + miR.29a + miR.660 | 0,69 | 0,078229291 |
| miR.603 + miR.220a + miR.362 | 0,69 | 0,061173635 |
| miR.603 + miR.130a + miR.660 | 0,69 | 0,059182476 |
| miR.603 + miR.130a + miR.639 | 0,69 | 0,04451678 |
| miR.520f + miR.130a + miR.494 | 0,69 | 0,031419644 |
| miR.558 + miR.376a* + miR.660 | 0,68 | 0,008666668 |
| miR.220a + miR.362 + miR.369.3p | 0,68 | 0,044803856 |
| miR.603 + miR.29a + miR.338 | 0,68 | 0,061647642 |
| miR.558 + miR.220a + miR.369.3p | 0,68 | 0,006032894 |
| miR.603 + miR.130a + miR.29a | 0,68 | 0,065069428 |
| miR.603 + miR.29a + miR.376a* | 0,68 | 0,098148148 |
| miR.603 + miR.519b + miR.29a | 0,68 | 0,079745901 |
| miR.520f + miR.519b + miR.26a | 0,68 | 0,021950393 |
| miR.520f + miR.130a + miR.338 | 0,68 | 0,034120701 |
| miR.558 + miR.220a + miR.130a | 0,68 | 0,001355518 |
| miR.520f + miR.26a + miR.660 | 0,68 | 0,03716572 |
| miR.520f + miR.519b + miR.362 | 0,68 | 0,018306926 |
| miR.520f + miR.130a + miR.369.3p | 0,68 | 0,025465139 |
| miR.603 + miR.639 + miR.660 | 0,68 | 0,051673284 |
| miR.558 + miR.130a + miR.660 | 0,68 | 0,00227285 |
| miR.603 + miR.130a + miR.376a* | 0,68 | 0,087534658 |
| miR.520f + miR.369.3p + miR.639 | 0,68 | 0,031523393 |
| miR.520f + miR.26a + miR.338 | 0,68 | 0,03608043 |
| miR.603 + miR.220a + miR.639 | 0,68 | 0,074165337 |
| miR.558 + miR.220a + miR.494 | 0,68 | 0,00165927 |
| miR.603 + miR.519b + miR.494 | 0,68 | 0,033679942 |
| miR.603 + miR.494 + miR.338 | 0,68 | 0,032721745 |
| miR.520f + miR.220a + miR.494 | 0,68 | 0,020323857 |
| miR.603 + miR.26a + miR.494 | 0,68 | 0,032072768 |
| miR.558 + miR.130a + miR.369.3p | 0,68 | 0,007685614 |
| miR.29a + miR.518c + miR.639 | 0,68 | 0,023364438 |
| miR.494 + miR.518c + miR.639 | 0,68 | 0,008490205 |
| miR.520f + miR.130a + miR.660 | 0,68 | 0,029924621 |
| miR.603 + miR.220a + miR.660 | 0,68 | 0,082252214 |
| miR.520f + miR.26a + miR.369.3p | 0,68 | 0,026372464 |
| miR.369.3p + miR.518c + miR.639 | 0,68 | 0,02039752 |
| miR.362 + miR.369.3p + miR.494 | 0,68 | 0,026195497 |
| miR.603 + miR.519b + miR.130a | 0,68 | 0,030867499 |
| miR.603 + miR.130a + miR.338 | 0,68 | 0,028259552 |
| miR.603 + miR.130a + miR.26a | 0,68 | 0,021419132 |
| miR.603 + miR.519b + miR.639 | 0,68 | 0,036692848 |
| miR.558 + miR.130a + miR.639 | 0,68 | 0,00230415 |
| miR.603 + miR.519b + miR.362 | 0,68 | 0,062185979 |
| miR.603 + miR.26a + miR.362 | 0,68 | 0,058826941 |
| miR.603 + miR.362 + miR.338 | 0,68 | 0,063726262 |
| miR.558 + miR.130a + miR.376a* | 0,68 | 0,006780287 |
| miR.519b + miR.362 + miR.369.3p | 0,68 | 0,031917711 |
| miR.603 + miR.362 + miR.660 | 0,68 | 0,06167808 |
| miR.558 + miR.130a + miR.494 | 0,68 | 0,001593409 |
| miR.603 + miR.362 + miR.376a* | 0,68 | 0,063688629 |
| miR.558 + miR.130a + miR.26a | 0,68 | 0,003314831 |
| miR.603 + miR.376a* + miR.660 | 0,68 | 0,062752031 |
| miR.520f + miR.362 + miR.376a* | 0,68 | 0,037891073 |
| miR.29a + miR.369.3p + miR.518c | 0,68 | 0,026641203 |
| miR.603 + miR.376a* + miR.639 | 0,68 | 0,062767214 |
| miR.558 + miR.130a + miR.338 | 0,68 | 0,001298161 |
| miR.29a + miR.494 + miR.518c | 0,68 | 0,015993401 |
| miR.603 + miR.639 + miR.338 | 0,68 | 0,038471506 |
| miR.558 + miR.519b + miR.130a | 0,68 | 0,001359148 |
| miR.603 + miR.26a + miR.639 | 0,68 | 0,036012679 |
| miR.220a + miR.362 + miR.494 | 0,68 | 0,021666565 |
| miR.520f + miR.369.3p + miR.494 | 0,68 | 0,01293201 |
| miR.362 + miR.369.3p + miR.376a* | 0,68 | 0,047295371 |
| miR.603 + miR.26a + miR.369.3p | 0,68 | 0,041274316 |
| miR.603 + miR.519b + miR.660 | 0,68 | 0,042202556 |
| miR.603 + miR.220a + miR.26a | 0,67 | 0,050004442 |
| miR.362 + miR.369.3p + miR.639 | 0,67 | 0,042589232 |
| miR.603 + miR.369.3p + miR.338 | 0,67 | 0,037912775 |
| miR.603 + miR.369.3p + miR.376a* | 0,67 | 0,056124246 |
| miR.603 + miR.220a + miR.519b | 0,67 | 0,046039626 |
| miR.603 + miR.660 + miR.338 | 0,67 | 0,032875331 |
| miR.603 + miR.26a + miR.660 | 0,67 | 0,034332136 |
| miR.603 + miR.220a + miR.338 | 0,67 | 0,045592948 |
| miR.558 + miR.376a* + miR.494 | 0,67 | 0,01210817 |
| miR.369.3p + miR.494 + miR.518c | 0,67 | 0,00381182 |
| miR.558 + miR.369.3p + miR.338 | 0,67 | 0,012678172 |
| miR.29a + miR.362 + miR.369.3p | 0,67 | 0,051664943 |
| miR.130a + miR.362 + miR.369.3p | 0,67 | 0,041646443 |
| miR.29a + miR.518c + miR.660 | 0,67 | 0,014200948 |
| miR.603 + miR.220a + miR.376a* | 0,67 | 0,063739599 |
| miR.220a + miR.29a + miR.494 | 0,67 | 0,010435825 |
| miR.220a + miR.29a + miR.362 | 0,67 | 0,013267597 |
| miR.130a + miR.369.3p + miR.518c | 0,67 | 0,013797868 |
| miR.130a + miR.494 + miR.518c | 0,67 | 0,003426413 |
| miR.220a + miR.29a + miR.369.3p | 0,67 | 0,010805745 |
| miR.520f + miR.376a* + miR.660 | 0,67 | 0,023277242 |
| miR.220a + miR.130a + miR.362 | 0,67 | 0,015581923 |
| miR.220a + miR.362 + miR.639 | 0,67 | 0,008960577 |
| miR.220a + miR.26a + miR.29a | 0,67 | 0,015919194 |
| miR.519b + miR.29a + miR.362 | 0,66 | 0,007917993 |
| miR.220a + miR.29a + miR.338 | 0,66 | 0,006269301 |
| miR.220a + miR.29a + miR.639 | 0,66 | 0,013210616 |
| miR.220a + miR.519b + miR.29a | 0,66 | 0,006033479 |
| miR.603 + miR.519b + miR.26a | 0,66 | 0,016181215 |
| miR.519b + miR.130a + miR.362 | 0,66 | 0,0029988 |
| miR.220a + miR.130a + miR.369.3p | 0,66 | 0,004916638 |
| miR.220a + miR.29a + miR.660 | 0,66 | 0,009183359 |
| miR.26a + miR.369.3p + miR.518c | 0,66 | 0,009051473 |
| miR.220a + miR.130a + miR.29a | 0,66 | 0,007773035 |
| miR.220a + miR.29a + miR.376a* | 0,66 | 0,02391839 |
| miR.369.3p + miR.518c + miR.338 | 0,66 | 0,008593343 |
| miR.603 + miR.26a + miR.338 | 0,66 | 0,017783329 |
| miR.220a + miR.369.3p + miR.639 | 0,66 | 0,00521421 |
| miR.519b + miR.26a + miR.29a | 0,66 | 0,002257527 |
| miR.519b + miR.29a + miR.494 | 0,66 | 0,001399685 |
| miR.519b + miR.29a + miR.369.3p | 0,66 | 0,003631973 |
| miR.603 + miR.26a + miR.376a* | 0,66 | 0,022188973 |
| miR.220a + miR.26a + miR.362 | 0,66 | 0,004094142 |
| miR.220a + miR.362 + miR.338 | 0,66 | 0,004256069 |
| miR.519b + miR.29a + miR.639 | 0,66 | 0,001554 |
| miR.220a + miR.362 + miR.376a* | 0,66 | 0,009279042 |
| miR.603 + miR.376a* + miR.338 | 0,66 | 0,032502963 |
| miR.29a + miR.362 + miR.639 | 0,66 | 0,008171297 |
| miR.220a + miR.130a + miR.639 | 0,66 | 0,001492459 |
| miR.220a + miR.519b + miR.130a | 0,66 | 0,000761264 |
| miR.220a + miR.130a + miR.660 | 0,66 | 0,002601161 |
| miR.519b + miR.29a + miR.376a* | 0,66 | 0,007472073 |
| miR.519b + miR.130a + miR.29a | 0,65 | 0,001009144 |
| miR.519b + miR.29a + miR.660 | 0,65 | 0,001366399 |
| miR.220a + miR.130a + miR.376a* | 0,65 | 0,005951799 |
| miR.220a + miR.130a + miR.26a | 0,65 | 0,000508695 |
| miR.519b + miR.130a + miR.376a* | 0,65 | 0,000560668 |
| miR.220a + miR.130a + miR.338 | 0,65 | 0,000874004 |
| miR.130a + miR.29a + miR.362 | 0,65 | 0,005112882 |

**Table 4: clusters of 4 miRNAs**

| **Cluster of 4 miRs** | **c_tau level** | **pv compared to top signature miR.609** + **miR.29a + miR.376a*** + **miR**.**518c** |
|---|---|---|
| miR.609 + miR.29a + miR.376a* + miR.518c | 0,77 | NaN |
| miR.558 + miR.609 + miR.29a + miR.518c | 0,77 | 0,095190878 |
| miR.558 + miR.609 + miR.29a + miR.376a* | 0,77 | 0,234133564 |
| miR.609 + miR.520f + miR.29a + miR.376a* | 0,77 | 0,398192778 |
| miR.558 + miR.609 + miR.603 + miR.518c | 0,77 | 0,152400827 |
| miR.609 + miR.520f + miR.29a + miR.518c | 0,77 | 0,273655288 |
| miR.558 + miR.609 + miR.520f + miR.518c | 0,77 | 0,120996525 |
| miR.558 + miR.609 + miR.518c + miR.639 | 0,76 | 0,083980327 |
| miR.558 + miR.609 + miR.220a + miR.518c | 0,76 | 0,090562295 |
| miR.558 + miR.609 + miR.376a* + miR.518c | 0,76 | 0,073563814 |
| miR.558 + miR.609 + miR.520f + miR.29a | 0,76 | 0,212166867 |
| miR.609 + miR.603 + miR.29a + miR.518c | 0,76 | 0,069410938 |
| miR.558 + miR.609 + miR.369.3p + miR.518c | 0,76 | 0,073717701 |
| miR.558 + miR.609 + miR.362 + miR.518c | 0,76 | 0,050881463 |
| miR.558 + miR.609 + miR.130a + miR.518c | 0,76 | 0,071838125 |
| miR.609 + miR.603 + miR.220a + miR.518c | 0,76 | 0,101227072 |
| miR.609 + miR.220a + miR.29a + miR.518c | 0,76 | 0,05942434 |
| miR.558 + miR.603 + miR.220a + miR.518c | 0,76 | 0,131622103 |
| miR.558 + miR.609 + miR.519b + miR.518c | 0,76 | 0,041357661 |
| miR.609 + miR.520f + miR.518c + miR.639 | 0,76 | 0,147820503 |
| miR.558 + miR.609 + miR.494 + miR.518c | 0,76 | 0,059097293 |
| miR.603 + miR.220a + miR.376a* + miR.518c | 0,76 | 0,251129292 |
| miR.558 + miR.609 + miR.26a + miR.518c | 0,76 | 0,061349034 |
| miR.558 + miR.603 + miR.520f + miR.518c | 0,76 | 0,195773827 |
| miR.609 + miR.520f + miR.130a + miR.518c | 0,76 | 0,116815227 |
| miR.558 + miR.609 + miR.518c + miR.338 | 0,76 | 0,05045062 |
| miR.558 + miR.609 + miR.518c + miR.660 | 0,76 | 0,039220441 |
| miR.609 + miR.603 + miR.518c + miR.639 | 0,76 | 0,071585762 |
| miR.558 + miR.603 + miR.376a* + miR.518c | 0,76 | 0,202745975 |
| miR.609 + miR.603 + miR.376a* + miR.518c | 0,76 | 0,084566847 |
| miR.609 + miR.29a + miR.518c + miR.660 | 0,76 | 0,006818281 |
| miR.609 + miR.26a + miR.29a + miR.518c | 0,76 | 0,033615265 |
| miR.609 + miR.603 + miR.130a + miR.518c | 0,76 | 0,064304505 |
| miR.609 + miR.603 + miR.520f + miR.518c | 0,76 | 0,073345843 |
| miR.558 + miR.609 + miR.26a + miR.29a | 0,75 | 0,07199124 |
| miR.609 + miR.29a + miR.518c + miR.338 | 0,75 | 0,043991625 |
| miR.609 + miR.520f + miR.130a + miR.376a* | 0,75 | 0,275240376 |
| miR.558 + miR.603 + miR.519b + miR.518c | 0,75 | 0,145532044 |
| miR.609 + miR.220a + miR.29a + miR.376a* | 0,75 | 0,092195252 |
| miR.609 + miR.29a + miR.376a* + miR.494 | 0,75 | 0,268412151 |
| miR.609 + miR.603 + miR.369.3p + miR.518c | 0,75 | 0,05151607 |
| miR.558 + miR.520f + miR.518c + miR.639 | 0,75 | 0,160078581 |
| miR.558 + miR.609 + miR.520f + miR.639 | 0,75 | 0,153716109 |
| miR.609 + miR.603 + miR.29a + miR.376a* | 0,75 | 0,121083107 |
| miR.603 + miR.220a + miR.519b + miR.518c | 0,75 | 0,122942194 |
| miR.603 + miR.520f + miR.220a + miR.518c | 0,75 | 0,182075349 |
| miR.558 + miR.609 + miR.520f + miR.130a | 0,75 | 0,219179588 |
| miR.558 + miR.603 + miR.26a + miR.518c | 0,75 | 0,15839003 |
| miR.609 + miR.29a + miR.369.3p + miR.376a* | 0,75 | 0,213298508 |
| miR.603 + miR.220a + miR.369.3p + miR.518c | 0,75 | 0,126725345 |
| miR.558 + miR.603 + miR.518c + miR.639 | 0,75 | 0,159516149 |
| miR.558 + miR.520f + miR.220a + miR.518c | 0,75 | 0,154099639 |
| miR.609 + miR.603 + miR.494 + miR.518c | 0,75 | 0,094774889 |
| miR.609 + miR.29a + miR.494 + miR.518c | 0,75 | 0,042714709 |
| miR.609 + miR.220a + miR.130a + miR.518c | 0,75 | 0,033957969 |
| miR.558 + miR.603 + miR.369.3p + miR.518c | 0,75 | 0,139052213 |
| miR.609 + miR.29a + miR.369.3p + miR.518c | 0,75 | 0,02790735 |
| miR.609 + miR.519b + miR.29a + miR.518c | 0,75 | 0,026540884 |
| miR.603 + miR.220a + miR.518c + miR.338 | 0,75 | 0,109761825 |
| miR.558 + miR.520f + miR.29a + miR.518c | 0,75 | 0,169971614 |
| miR.558 + miR.603 + miR.362 + miR.518c | 0,75 | 0,125790751 |
| miR.609 + miR.130a + miR.29a + miR.518c | 0,75 | 0,017242261 |
| miR.603 + miR.220a + miR.26a + miR.518c | 0,75 | 0,090332806 |
| miR.558 + miR.603 + miR.29a + miR.518c | 0,75 | 0,137256379 |
| miR.603 + miR.520f + miR.518c + miR.639 | 0,75 | 0,155894054 |
| miR.603 + miR.220a + miR.29a + miR.518c | 0,75 | 0,105622688 |
| miR.558 + miR.603 + miR.494 + miR.518c | 0,75 | 0,120489954 |
| miR.558 + miR.520f + miR.376a* + miR.518c | 0,75 | 0,193976453 |
| miR.558 + miR.603 + miR.518c + miR.338 | 0,75 | 0,125210123 |
| miR.609 + miR.603 + miR.362 + miR.518c | 0,75 | 0,051557056 |
| miR.609 + miR.29a + miR.518c + miR.639 | 0,75 | 0,025353784 |
| miR.609 + miR.520f + miR.220a + miR.518c | 0,75 | 0,034932887 |
| miR.558 + miR.603 + miR.518c + miR.660 | 0,75 | 0,120602125 |
| miR.603 + miR.220a + miR.518c + miR.639 | 0,75 | 0,089640403 |
| miR.609 + miR.29a + miR.362 + miR.518c | 0,75 | 0,022807144 |
| miR.609 + miR.220a + miR.376a* + miR.518c | 0,75 | 0,015135288 |
| miR.558 + miR.603 + miR.130a + miR.518c | 0,75 | 0,128050808 |
| miR.609 + miR.220a + miR.518c + miR.639 | 0,75 | 0,019525674 |
| miR.558 + miR.609 + miR.603 + miR.29a | 0,75 | 0,11995271 |
| miR.603 + miR.220a + miR.130a + miR.518c | 0,75 | 0,101475598 |
| miR.609 + miR.520f + miR.26a + miR.518c | 0,75 | 0,091970396 |
| miR.603 + miR.220a + miR.494 + miR.518c | 0,75 | 0,102280329 |
| miR.609 + miR.603 + miR.519b + miR.518c | 0,75 | 0,033387808 |
| miR.558 + miR.520f + miR.362 + miR.518c | 0,75 | 0,17757916 |
| miR.558 + miR.520f + miR.130a + miR.518c | 0,75 | 0,164603281 |
| miR.609 + miR.603 + miR.518c + miR.338 | 0,75 | 0,040306705 |
| miR.603 + miR.520f + miR.376a* + miR.518c | 0,75 | 0,178218301 |
| miR.609 + miR.520f + miR.29a + miR.660 | 0,75 | 0,108259038 |
| miR.609 + miR.603 + miR.26a + miR.518c | 0,75 | 0,049760274 |
| miR.603 + miR.220a + miR.362 + miR.518c | 0,75 | 0,084267929 |
| miR.603 + miR.376a* + miR.518c + miR.639 | 0,75 | 0,110692495 |
| miR.558 + miR.609 + miR.29a + miR.660 | 0,75 | 0,062339195 |
| miR.609 + miR.603 + miR.518c + miR.660 | 0,75 | 0,03753584 |
| miR.558 + miR.520f + miR.519b + miR.518c | 0,75 | 0,140569368 |
| miR.558 + miR.609 + miR.130a + miR.376a* | 0,75 | 0,155334179 |
| miR.603 + miR.220a + miR.518c + miR.660 | 0,75 | 0,086588446 |
| miR.609 + miR.519b + miR.29a + miR.376a* | 0,75 | 0,083277641 |
| miR.558 + miR.609 + miR.376a* + miR.639 | 0,75 | 0,130880406 |
| miR.558 + miR.220a + miR.376a* + miR.518c | 0,75 | 0,108015506 |
| miR.558 + miR.520f + miR.26a + miR.518c | 0,75 | 0,15060931 |
| miR.558 + miR.609 + miR.220a + miR.29a | 0,75 | 0,099431509 |
| miR.558 + miR.520f + miR.369.3p + miR.518c | 0,75 | 0,145736289 |
| miR.558 + miR.520f + miR.494 + miR.518c | 0,75 | 0,122357549 |
| miR.609 + miR.520f + miR.29a + miR.494 | 0,75 | 0,172325158 |
| miR.558 + miR.609 + miR.29a + miR.369.3p | 0,75 | 0,09238121 |
| miR.558 + miR.609 + miR.130a + miR.29a | 0,75 | 0,072519142 |
| miR.609 + miR.220a + miR.369.3p + miR.518c | 0,75 | 0,020967329 |
| miR.603 + miR.520f + miR.29a + miR.518c | 0,75 | 0,151377195 |
| miR.558 + miR.520f + miR.518c + miR.338 | 0,75 | 0,150058461 |
| miR.558 + miR.520f + miR.518c + miR.660 | 0,75 | 0,132520189 |
| miR.603 + miR.369.3p + miR.376a* + miR.518c | 0,75 | 0,179261881 |
| miR.609 + miR.130a + miR.376a* + miR.518c | 0,75 | 0,043347291 |
| miR.558 + miR.609 + miR.29a + miR.338 | 0,75 | 0,06854081 |
| miR.558 + miR.609 + miR.520f + miR.26a | 0,75 | 0,156943535 |
| miR.609 + miR.603 + miR.520f + miR.29a | 0,75 | 0,095775145 |
| miR.558 + miR.609 + miR.519b + miR.29a | 0,75 | 0,061975286 |
| miR.558 + miR.609 + miR.29a + miR.639 | 0,75 | 0,076366259 |
| miR.558 + miR.609 + miR.29a + miR.494 | 0,75 | 0,103217378 |
| miR.558 + miR.609 + miR.603 + miR.520f | 0,75 | 0,170065251 |
| miR.558 + miR.220a + miR.26a + miR.518c | 0,75 | 0,062062634 |
| miR.609 + miR.29a + miR.376a* + miR.639 | 0,75 | 0,045578904 |
| miR.609 + miR.220a + miR.494 + miR.518c | 0,75 | 0,026701873 |
| miR.609 + miR.29a + miR.362 + miR.376a* | 0,75 | 0,05758252 |
| miR.609 + miR.520f + miR.519b + miR.29a | 0,75 | 0,071806723 |
| miR.609 + miR.520f + miR.220a + miR.29a | 0,75 | 0,066879617 |
| miR.609 + miR.520f + miR.29a + miR.369.3p | 0,75 | 0,105980196 |
| miR.603 + miR.520f + miR.130a + miR.518c | 0,75 | 0,146050405 |
| miR.609 + miR.520f + miR.29a + miR.338 | 0,75 | 0,084787236 |
| miR.609 + miR.520f + miR.29a + miR.639 | 0,75 | 0,105287948 |
| miR.603 + miR.520f + miR.369.3p + miR.518c | 0,75 | 0,15502797 |
| miR.603 + miR.29a + miR.376a* + miR.518c | 0,74 | 0,095638325 |
| miR.609 + miR.376a* + miR.518c + miR.639 | 0,74 | 0,037647912 |
| miR.603 + miR.520f + miR.362 + miR.518c | 0,74 | 0,128652026 |
| miR.558 + miR.609 + miR.29a + miR.362 | 0,74 | 0,073016857 |
| miR.603 + miR.520f + miR.519b + miR.518c | 0,74 | 0,116485645 |
| miR.609 + miR.520f + miR.130a + miR.29a | 0,74 | 0,11856521 |
| miR.609 + miR.519b + miR.518c + miR.639 | 0,74 | 0,01994205 |
| miR.558 + miR.220a + miR.519b + miR.518c | 0,74 | 0,053231829 |
| miR.609 + miR.520f + miR.26a + miR.29a | 0,74 | 0,103809182 |
| miR.603 + miR.520f + miR.26a + miR.518c | 0,74 | 0,121981164 |
| miR.609 + miR.29a + miR.376a* + miR.660 | 0,74 | 0,05046512 |
| miR.609 + miR.520f + miR.29a + miR.362 | 0,74 | 0,092715547 |
| miR.603 + miR.376a* + miR.518c + miR.660 | 0,74 | 0,12721963 |
| miR.603 + miR.520f + miR.518c + miR.338 | 0,74 | 0,12431959 |
| miR.603 + miR.520f + miR.518c + miR.660 | 0,74 | 0,12431959 |
| miR.609 + miR.220a + miR.362 + miR.518c | 0,74 | 0,016326012 |
| miR.603 + miR.520f + miR.494 + miR.518c | 0,74 | 0,136298214 |
| miR.609 + miR.220a + miR.518c + miR.338 | 0,74 | 0,013512608 |
| miR.609 + miR.29a + miR.376a* + miR.338 | 0,74 | 0,050304492 |
| miR.609 + miR.220a + miR.519b + miR.518c | 0,74 | 0,018397 |
| miR.558 + miR.609 + miR.603 + miR.639 | 0,74 | 0,134932573 |
| miR.609 + miR.130a + miR.29a + miR.376a* | 0,74 | 0,078618906 |
| miR.609 + miR.220a + miR.26a + miR.518c | 0,74 | 0,013136055 |
| miR.609 + miR.26a + miR.29a + miR.376a* | 0,74 | 0,033752402 |
| miR.609 + miR.220a + miR.518c + miR.660 | 0,74 | 0,009578358 |
| miR.603 + miR.26a + miR.518c + miR.639 | 0,74 | 0,082856643 |
| miR.558 + miR.220a + miR.518c + miR.338 | 0,74 | 0,051067743 |
| miR.558 + miR.609 + miR.362 + miR.369.3p | 0,74 | 0,098473275 |
| miR.603 + miR.362 + miR.376a* + miR.518c | 0,74 | 0,119681093 |
| miR.520f + miR.220a + miR.518c + miR.639 | 0,74 | 0,066460344 |
| miR.603 + miR.26a + miR.29a + miR.518c | 0,74 | 0,081998228 |
| miR.603 + miR.130a + miR.376a* + miR.518c | 0,74 | 0,089401331 |
| miR.558 + miR.609 + miR.520f + miR.338 | 0,74 | 0,134256712 |
| miR.609 + miR.603 + miR.130a + miR.376a* | 0,74 | 0,104954096 |
| miR.520f + miR.220a + miR.29a + miR.518c | 0,74 | 0,085293212 |
| miR.558 + miR.609 + miR.520f + miR.494 | 0,74 | 0,135588189 |
| miR.609 + miR.369.3p + miR.518c + miR.639 | 0,74 | 0,030410317 |
| miR.609 + miR.519b + miR.130a + miR.518c | 0,74 | 0,030907058 |
| miR.558 + miR.609 + miR.520f + miR.376a* | 0,74 | 0,099852691 |
| miR.558 + miR.609 + miR.520f + miR.220a | 0,74 | 0,120841026 |
| miR.558 + miR.609 + miR.603 + miR.130a | 0,74 | 0,086267578 |
| miR.558 + miR.26a + miR.29a + miR.518c | 0,74 | 0,068441857 |
| miR.603 + miR.376a* + miR.494 + miR.518c | 0,74 | 0,116351712 |
| miR.558 + miR.609 + miR.520f + miR.362 | 0,74 | 0,106771094 |
| miR.558 + miR.220a + miR.369.3p + miR.518c | 0,74 | 0,041229229 |
| miR.609 + miR.130a + miR.518c + miR.639 | 0,74 | 0,013478854 |
| miR.558 + miR.609 + miR.520f + miR.369.3p | 0,74 | 0,115909957 |
| miR.558 + miR.220a + miR.518c + miR.639 | 0,74 | 0,042333948 |
| miR.558 + miR.376a* + miR.518c + miR.639 | 0,74 | 0,074901972 |
| miR.603 + miR.362 + miR.369.3p + miR.518c | 0,74 | 0,117819056 |
| miR.558 + miR.220a + miR.494 + miR.518c | 0,74 | 0,032449642 |
| miR.558 + miR.220a + miR.29a + miR.518c | 0,74 | 0,034843117 |
| miR.609 + miR.494 + miR.518c + miR.639 | 0,74 | 0,030646365 |
| miR.558 + miR.609 + miR.603 + miR.369.3p | 0,74 | 0,17745831 |
| miR.609 + miR.362 + miR.518c + miR.639 | 0,74 | 0,020828142 |
| miR.609 + miR.520f + miR.369.3p + miR.518c | 0,74 | 0,036347494 |
| miR.558 + miR.220a + miR.362 + miR.518c | 0,74 | 0,035052722 |
| miR.558 + miR.26a + miR.518c + miR.639 | 0,74 | 0,055224505 |
| miR.609 + miR.130a + miR.26a + miR.518c | 0,74 | 0,011711735 |
| miR.609 + miR.130a + miR.362 + miR.518c | 0,74 | 0,015579647 |
| miR.558 + miR.609 + miR.603 + miR.220a | 0,74 | 0,094494537 |
| miR.609 + miR.520f + miR.519b + miR.518c | 0,74 | 0,032444677 |
| miR.558 + miR.609 + miR.220a + miR.376a* | 0,74 | 0,077934955 |
| miR.603 + miR.26a + miR.369.3p + miR.518c | 0,74 | 0,078701326 |
| miR.558 + miR.220a + miR.518c + miR.660 | 0,74 | 0,051369854 |
| miR.603 + miR.369.3p + miR.518c + miR.639 | 0,74 | 0,063261821 |
| miR.609 + miR.362 + miR.369.3p + miR.518c | 0,74 | 0,034899386 |
| miR.609 + miR.520f + miR.494 + miR.518c | 0,74 | 0,07781653 |
| miR.558 + miR.29a + miR.376a* + miR.518c | 0,74 | 0,04969725 |
| miR.558 + miR.220a + miR.130a + miR.518c | 0,74 | 0,044650196 |
| miR.603 + miR.518c + miR.639 + miR.338 | 0,74 | 0,055957384 |
| miR.520f + miR.220a + miR.376a* + miR.518c | 0,74 | 0,094204525 |
| miR.558 + miR.609 + miR.369.3p + miR.376a* | 0,74 | 0,11312355 |
| miR.609 + miR.520f + miR.26a + miR.376a* | 0,74 | 0,114301319 |
| miR.609 + miR.603 + miR.520f + miR.130a | 0,74 | 0,103585291 |
| miR.558 + miR.362 + miR.376a* + miR.518c | 0,74 | 0,075181584 |
| miR.609 + miR.520f + miR.362 + miR.518c | 0,74 | 0,034998525 |
| miR.603 + miR.26a + miR.376a* + miR.518c | 0,74 | 0,075475153 |
| miR.603 + miR.376a* + miR.518c + miR.338 | 0,74 | 0,092567256 |
| miR.558 + miR.376a* + miR.518c + miR.660 | 0,74 | 0,058226264 |
| miR.603 + miR.362 + miR.518c + miR.639 | 0,74 | 0,074247111 |
| miR.558 + miR.369.3p + miR.376a* + miR.518c | 0,74 | 0,08001079 |
| miR.603 + miR.519b + miR.376a* + miR.518c | 0,74 | 0,084991001 |
| miR.603 + miR.130a + miR.26a + miR.518c | 0,74 | 0,069975625 |
| miR.520f + miR.220a + miR.130a + miR.518c | 0,74 | 0,064535284 |
| miR.558 + miR.609 + miR.603 + miR.362 | 0,74 | 0,09356382 |
| miR.558 + miR.609 + miR.603 + miR.376a* | 0,74 | 0,100172643 |
| miR.558 + miR.609 + miR.520f + miR.660 | 0,74 | 0,110938722 |
| miR.520f + miR.376a* + miR.518c + miR.639 | 0,74 | 0,098515677 |
| miR.558 + miR.609 + miR.520f + miR.519b | 0,74 | 0,125171619 |
| miR.609 + miR.603 + miR.29a + miR.494 | 0,74 | 0,101151086 |
| miR.609 + miR.518c + miR.639 + miR.338 | 0,74 | 0,01521563 |
| miR.520f + miR.220a + miR.26a + miR.518c | 0,74 | 0,070228502 |
| miR.609 + miR.130a + miR.369.3p + miR.518c | 0,74 | 0,02414883 |
| miR.558 + miR.609 + miR.362 + miR.639 | 0,74 | 0,076219252 |
| miR.520f + miR.220a + miR.369.3p + miR.518c | 0,74 | 0,066978308 |
| miR.558 + miR.609 + miR.362 + miR.376a* | 0,74 | 0,056518304 |
| miR.609 + miR.130a + miR.518c + miR.338 | 0,74 | 0,009388196 |
| miR.609 + miR.26a + miR.518c + miR.639 | 0,74 | 0,012827359 |
| miR.558 + miR.609 + miR.603 + miR.494 | 0,74 | 0,124215101 |
| miR.609 + miR.520f + miR.518c + miR.338 | 0,74 | 0,030672998 |
| miR.603 + miR.29a + miR.369.3p + miR.518c | 0,74 | 0,071151405 |
| miR.603 + miR.29a + miR.518c + miR.639 | 0,74 | 0,058594387 |
| miR.603 + miR.519b + miR.518c + miR.639 | 0,74 | 0,066748811 |
| miR.520f + miR.519b + miR.518c + miR.639 | 0,74 | 0,054222827 |
| miR.603 + miR.494 + miR.518c + miR.639 | 0,74 | 0,076089769 |
| miR.558 + miR.609 + miR.220a + miR.369.3p | 0,74 | 0,090077117 |
| miR.603 + miR.369.3p + miR.518c + miR.338 | 0,74 | 0,071015847 |
| miR.558 + miR.609 + miR.369.3p + miR.639 | 0,74 | 0,095032576 |
| miR.603 + miR.130a + miR.518c + miR.639 | 0,74 | 0,072705347 |
| miR.609 + miR.518c + miR.639 + miR.660 | 0,74 | 0,013137355 |
| miR.558 + miR.609 + miR.376a* + miR.494 | 0,74 | 0,159926065 |
| miR.520f + miR.220a + miR.362 + miR.518c | 0,74 | 0,055403772 |
| miR.558 + miR.609 + miR.130a + miR.362 | 0,74 | 0,05851125 |
| miR.603 + miR.518c + miR.639 + miR.660 | 0,74 | 0,064717331 |
| miR.609 + miR.520f + miR.130a + miR.639 | 0,74 | 0,083646158 |
| miR.609 + miR.520f + miR.518c + miR.660 | 0,74 | 0,026861186 |
| miR.603 + miR.26a + miR.362 + miR.518c | 0,74 | 0,063115644 |
| miR.520f + miR.220a + miR.518c + miR.338 | 0,74 | 0,047670481 |
| miR.603 + miR.519b + miR.369.3p + miR.518c | 0,74 | 0,073788459 |
| miR.520f + miR.220a + miR.519b + miR.518c | 0,74 | 0,052030294 |
| miR.609 + miR.603 + miR.520f + miR.639 | 0,74 | 0,094165599 |
| miR.609 + miR.520f + miR.376a* + miR.639 | 0,74 | 0,118141535 |
| miR.558 + miR.609 + miR.220a + miR.639 | 0,74 | 0,072866358 |
| miR.603 + miR.29a + miR.518c + miR.338 | 0,74 | 0,043282333 |
| miR.609 + miR.130a + miR.518c + miR.660 | 0,74 | 0,010119594 |
| miR.558 + miR.362 + miR.369.3p + miR.518c | 0,74 | 0,076227533 |
| miR.558 + miR.609 + miR.220a + miR.130a | 0,74 | 0,05483949 |
| miR.603 + miR.29a + miR.362 + miR.518c | 0,74 | 0,05582789 |
| miR.558 + miR.609 + miR.220a + miR.362 | 0,74 | 0,047620739 |
| miR.520f + miR.220a + miR.494 + miR.518c | 0,74 | 0,06173118 |
| miR.520f + miR.29a + miR.518c + miR.639 | 0,74 | 0,084401779 |
| miR.603 + miR.369.3p + miR.518c + miR.660 | 0,74 | 0,066259739 |
| miR.603 + miR.130a + miR.369.3p + miR.518c | 0,74 | 0,066259739 |
| miR.558 + miR.26a + miR.362 + miR.518c | 0,74 | 0,064332146 |
| miR.520f + miR.26a + miR.518c + miR.639 | 0,74 | 0,074182159 |
| miR.558 + miR.609 + miR.130a + miR.369.3p | 0,74 | 0,066572075 |
| miR.520f + miR.29a + miR.376a* + miR.518c | 0,74 | 0,128861379 |
| miR.520f + miR.362 + miR.518c + miR.639 | 0,74 | 0,05445848 |
| miR.558 + miR.609 + miR.362 + miR.494 | 0,74 | 0,09032679 |
| miR.558 + miR.609 + miR.603 + miR.660 | 0,74 | 0,101202769 |
| miR.609 + miR.520f + miR.26a + miR.639 | 0,74 | 0,087783637 |
| miR.558 + miR.609 + miR.130a + miR.639 | 0,74 | 0,063069995 |
| miR.520f + miR.220a + miR.518c + miR.660 | 0,74 | 0,065363815 |
| miR.603 + miR.369.3p + miR.494 + miR.518c | 0,74 | 0,070213824 |
| miR.603 + miR.519b + miR.362 + miR.518c | 0,74 | 0,045382411 |
| miR.603 + miR.26a + miR.518c + miR.660 | 0,74 | 0,060830051 |
| miR.609 + miR.130a + miR.494 + miR.518c | 0,74 | 0,024287975 |
| miR.558 + miR.130a + miR.29a + miR.518c | 0,74 | 0,045640866 |
| miR.558 + miR.609 + miR.494 + miR.639 | 0,74 | 0,099348006 |
| miR.603 + miR.130a + miR.29a + miR.518c | 0,74 | 0,045398461 |
| miR.603 + miR.362 + miR.518c + miR.338 | 0,74 | 0,069621505 |
| miR.603 + miR.519b + miR.29a + miR.518c | 0,74 | 0,055764584 |
| miR.558 + miR.609 + miR.603 + miR.26a | 0,74 | 0,109309598 |
| miR.558 + miR.609 + miR.603 + miR.338 | 0,74 | 0,107471247 |
| miR.609 + miR.519b + miR.369.3p + miR.518c | 0,74 | 0,024341477 |
| miR.603 + miR.130a + miR.362 + miR.518c | 0,73 | 0,055400192 |
| miR.609 + miR.362 + miR.494 + miR.518c | 0,73 | 0,043500436 |
| miR.520f + miR.369.3p + miR.518c + miR.639 | 0,73 | 0,066107433 |
| miR.558 + miR.609 + miR.603 + miR.519b | 0,73 | 0,101532949 |
| miR.603 + miR.362 + miR.494 + miR.518c | 0,73 | 0,080810581 |
| miR.609 + miR.603 + miR.220a + miR.29a | 0,73 | 0,030911624 |
| miR.558 + miR.26a + miR.376a* + miR.518c | 0,73 | 0,065170768 |
| miR.558 + miR.130a + miR.376a* + miR.518c | 0,73 | 0,062083572 |
| miR.558 + miR.26a + miR.369.3p + miR.518c | 0,73 | 0,073399404 |
| miR.520f + miR.518c + miR.639 + miR.660 | 0,73 | 0,075619421 |
| miR.609 + miR.520f + miR.130a + miR.26a | 0,73 | 0,097353957 |
| miR.603 + miR.26a + miR.494 + miR.518c | 0,73 | 0,077581338 |
| miR.558 + miR.362 + miR.518c + miR.639 | 0,73 | 0,033826398 |
| miR.603 + miR.29a + miR.494 + miR.518c | 0,73 | 0,060683834 |
| miR.609 + miR.603 + miR.29a + miR.369.3p | 0,73 | 0,065959119 |
| miR.520f + miR.494 + miR.518c + miR.639 | 0,73 | 0,074944414 |
| miR.520f + miR.26a + miR.376a* + miR.518c | 0,73 | 0,058205606 |
| miR.520f + miR.130a + miR.518c + miR.639 | 0,73 | 0,060947304 |
| miR.603 + miR.518c + miR.660 + miR.338 | 0,73 | 0,056606581 |
| miR.609 + miR.603 + miR.376a* + miR.639 | 0,73 | 0,115496164 |
| miR.520f + miR.518c + miR.639 + miR.338 | 0,73 | 0,063945903 |
| miR.603 + miR.362 + miR.518c + miR.660 | 0,73 | 0,05818279 |
| miR.558 + miR.519b + miR.376a* + miR.518c | 0,73 | 0,045554382 |
| miR.558 + miR.376a* + miR.518c + miR.338 | 0,73 | 0,055855034 |
| miR.558 + miR.130a + miR.26a + miR.518c | 0,73 | 0,042176299 |
| miR.558 + miR.609 + miR.376a* + miR.660 | 0,73 | 0,044024045 |
| miR.603 + miR.519b + miR.26a + miR.518c | 0,73 | 0,062257825 |
| miR.609 + miR.519b + miR.376a* + miR.518c | 0,73 | 0,015982589 |
| miR.609 + miR.520f + miR.130a + miR.660 | 0,73 | 0,101833469 |
| miR.609 + miR.520f + miR.130a + miR.369.3p | 0,73 | 0,109412444 |
| miR.609 + miR.519b + miR.362 + miR.518c | 0,73 | 0,013056845 |
| miR.603 + miR.29a + miR.518c + miR.660 | 0,73 | 0,059519308 |
| miR.609 + miR.520f + miR.130a + miR.362 | 0,73 | 0,068097694 |
| miR.558 + miR.609 + miR.130a + miR.26a | 0,73 | 0,057495804 |
| miR.609 + miR.26a + miR.29a + miR.494 | 0,73 | 0,042038841 |
| miR.603 + miR.26a + miR.518c + miR.338 | 0,73 | 0,051744542 |
| miR.609 + miR.130a + miR.362 + miR.376a* | 0,73 | 0,082310119 |
| miR.609 + miR.520f + miR.220a + miR.130a | 0,73 | 0,0878706 |
| miR.609 + miR.220a + miR.130a + miR.376a* | 0,73 | 0,044324572 |
| miR.609 + miR.520f + miR.519b + miR.130a | 0,73 | 0,090034683 |
| miR.609 + miR.520f + miR.130a + miR.494 | 0,73 | 0,096237724 |
| miR.220a + miR.26a + miR.29a + miR.518c | 0,73 | 0,028305867 |
| miR.558 + miR.376a* + miR.494 + miR.518c | 0,73 | 0,049466424 |
| miR.558 + miR.609 + miR.519b + miR.639 | 0,73 | 0,059730051 |
| miR.603 + miR.130a + miR.518c + miR.338 | 0,73 | 0,037054069 |
| miR.558 + miR.609 + miR.26a + miR.639 | 0,73 | 0,062921851 |
| miR.558 + miR.29a + miR.362 + miR.518c | 0,73 | 0,030396599 |
| miR.603 + miR.519b + miR.518c + miR.660 | 0,73 | 0,036802339 |
| miR.558 + miR.609 + miR.220a + miR.494 | 0,73 | 0,089461022 |
| miR.603 + miR.519b + miR.494 + miR.518c | 0,73 | 0,054177507 |
| miR.558 + miR.609 + miR.639 + miR.338 | 0,73 | 0,063619561 |
| miR.603 + miR.494 + miR.518c + miR.338 | 0,73 | 0,073653458 |
| miR.609 + miR.520f + miR.376a* + miR.518c | 0,73 | 0,022392935 |
| miR.609 + miR.520f + miR.130a + miR.338 | 0,73 | 0,102375209 |
| miR.609 + miR.29a + miR.362 + miR.660 | 0,73 | 0,005505747 |
| miR.558 + miR.26a + miR.518c + miR.660 | 0,73 | 0,049765564 |
| miR.609 + miR.520f + miR.494 + miR.639 | 0,73 | 0,085074805 |
| miR.558 + miR.609 + miR.130a + miR.494 | 0,73 | 0,054027886 |
| miR.603 + miR.130a + miR.518c + miR.660 | 0,73 | 0,051150114 |
| miR.558 + miR.519b + miR.362 + miR.518c | 0,73 | 0,037444677 |
| miR.603 + miR.519b + miR.518c + miR.338 | 0,73 | 0,047404685 |
| miR.558 + miR.609 + miR.639 + miR.660 | 0,73 | 0,067591843 |
| miR.603 + miR.519b + miR.130a + miR.518c | 0,73 | 0,050878399 |
| miR.558 + miR.609 + miR.362 + miR.660 | 0,73 | 0,048363097 |
| miR.603 + miR.494 + miR.518c + miR.660 | 0,73 | 0,070813234 |
| miR.609 + miR.603 + miR.29a + miR.660 | 0,73 | 0,017966954 |
| miR.558 + miR.518c + miR.639 + miR.338 | 0,73 | 0,031060775 |
| miR.609 + miR.603 + miR.376a* + miR.494 | 0,73 | 0,155790044 |
| miR.609 + miR.220a + miR.29a + miR.494 | 0,73 | 0,037062083 |
| miR.520f + miR.519b + miR.29a + miR.518c | 0,73 | 0,067754735 |
| miR.609 + miR.603 + miR.29a + miR.639 | 0,73 | 0,037384146 |
| miR.603 + miR.130a + miR.494 + miR.518c | 0,73 | 0,069744604 |
| miR.609 + miR.603 + miR.26a + miR.29a | 0,73 | 0,028652026 |
| miR.558 + miR.609 + miR.130a + miR.338 | 0,73 | 0,038234098 |
| miR.558 + miR.609 + miR.519b + miR.130a | 0,73 | 0,042415851 |
| miR.558 + miR.609 + miR.130a + miR.660 | 0,73 | 0,045579859 |
| miR.558 + miR.609 + miR.220a + miR.338 | 0,73 | 0,058298105 |
| miR.609 + miR.603 + miR.29a + miR.338 | 0,73 | 0,020377726 |
| miR.520f + miR.29a + miR.518c + miR.660 | 0,73 | 0,075863818 |
| miR.558 + miR.362 + miR.518c + miR.660 | 0,73 | 0,047026908 |
| miR.220a + miR.369.3p + miR.376a* + miR.518c | 0,73 | 0,068113783 |
| miR.558 + miR.609 + miR.362 + miR.338 | 0,73 | 0,042500706 |
| miR.520f + miR.29a + miR.362 + miR.518c | 0,73 | 0,09084255 |
| miR.558 + miR.362 + miR.494 + miR.518c | 0,73 | 0,030321617 |
| miR.558 + miR.609 + miR.220a + miR.26a | 0,73 | 0,058239393 |
| miR.558 + miR.362 + miR.518c + miR.338 | 0,73 | 0,038933989 |
| miR.609 + miR.519b + miR.494 + miR.518c | 0,73 | 0,017166781 |
| miR.558 + miR.609 + miR.220a + miR.519b | 0,73 | 0,05863021 |
| miR.558 + miR.519b + miR.26a + miR.518c | 0,73 | 0,05010813 |
| miR.609 + miR.603 + miR.520f + miR.26a | 0,73 | 0,09381255 |
| miR.558 + miR.609 + miR.220a + miR.660 | 0,73 | 0,058239393 |
| miR.609 + miR.130a + miR.29a + miR.494 | 0,73 | 0,037976088 |
| miR.220a + miR.29a + miR.376a* + miR.518c | 0,73 | 0,025191606 |
| miR.558 + miR.609 + miR.376a* + miR.338 | 0,73 | 0,057703944 |
| miR.609 + miR.29a + miR.494 + miR.660 | 0,73 | 0,035778842 |
| miR.220a + miR.29a + miR.518c + miR.338 | 0,73 | 0,027143718 |
| miR.558 + miR.130a + miR.518c + miR.639 | 0,73 | 0,036393513 |
| miR.609 + miR.520f + miR.26a + miR.494 | 0,73 | 0,097028554 |
| miR.558 + miR.369.3p + miR.518c + miR.639 | 0,73 | 0,049119107 |
| miR.558 + miR.609 + miR.26a + miR.362 | 0,73 | 0,038073012 |
| miR.558 + miR.29a + miR.518c + miR.639 | 0,73 | 0,038052152 |
| miR.558 + miR.519b + miR.518c + miR.639 | 0,73 | 0,032629944 |
| miR.609 + miR.520f + miR.519b + miR.639 | 0,73 | 0,069677371 |
| miR.558 + miR.29a + miR.518c + miR.338 | 0,73 | 0,023717845 |
| miR.558 + miR.130a + miR.362 + miR.518c | 0,73 | 0,033933391 |
| miR.558 + miR.609 + miR.369.3p + miR.494 | 0,73 | 0,09121593 |
| miR.609 + miR.603 + miR.130a + miR.29a | 0,73 | 0,03420256 |
| miR.520f + miR.26a + miR.29a + miR.518c | 0,73 | 0,067269802 |
| miR.520f + miR.29a + miR.518c + miR.338 | 0,73 | 0,076669475 |
| miR.609 + miR.603 + miR.519b + miR.29a | 0,73 | 0,028546438 |
| miR.609 + miR.369.3p + miR.494 + miR.518c | 0,73 | 0,028830831 |
| miR.558 + miR.609 + miR.519b + miR.362 | 0,73 | 0,038229586 |
| miR.558 + miR.494 + miR.518c + miR.639 | 0,73 | 0,032661435 |
| miR.558 + miR.519b + miR.29a + miR.518c | 0,73 | 0,033339954 |
| miR.609 + miR.29a + miR.369.3p + miR.494 | 0,73 | 0,056013402 |
| miR.520f + miR.29a + miR.369.3p + miR.518c | 0,73 | 0,08584984 |
| miR.558 + miR.609 + miR.26a + miR.376a* | 0,73 | 0,045281352 |
| miR.520f + miR.130a + miR.29a + miR.518c | 0,73 | 0,08778147 |
| miR.609 + miR.220a + miR.26a + miR.29a | 0,73 | 0,011664266 |
| miR.609 + miR.362 + miR.369.3p + miR.376a* | 0,73 | 0,148943316 |
| miR.609 + miR.220a + miR.29a + miR.369.3p | 0,73 | 0,024156115 |
| miR.558 + miR.26a + miR.494 + miR.518c | 0,73 | 0,054062325 |
| miR.558 + miR.518c + miR.639 + miR.660 | 0,73 | 0,038428075 |
| miR.609 + miR.29a + miR.362 + miR.369.3p | 0,73 | 0,040814635 |
| miR.609 + miR.603 + miR.29a + miR.362 | 0,73 | 0,034280118 |
| miR.558 + miR.609 + miR.369.3p + miR.660 | 0,73 | 0,068825487 |
| miR.609 + miR.29a + miR.494 + miR.338 | 0,73 | 0,026047031 |
| miR.558 + miR.26a + miR.518c + miR.338 | 0,73 | 0,055344824 |
| miR.558 + miR.29a + miR.494 + miR.518c | 0,73 | 0,022491443 |
| miR.609 + miR.519b + miR.518c + miR.660 | 0,73 | 0,00782303 |
| miR.520f + miR.29a + miR.494 + miR.518c | 0,73 | 0,078245802 |
| miR.609 + miR.220a + miR.29a + miR.660 | 0,73 | 0,006263507 |
| miR.558 + miR.29a + miR.369.3p + miR.518c | 0,73 | 0,046525542 |
| miR.558 + miR.609 + miR.369.3p + miR.338 | 0,73 | 0,086904488 |
| miR.609 + miR.29a + miR.362 + miR.494 | 0,73 | 0,054141582 |
| miR.609 + miR.603 + miR.520f + miR.494 | 0,73 | 0,104015054 |
| miR.609 + miR.520f + miR.362 + miR.639 | 0,73 | 0,072460936 |
| miR.609 + miR.520f + miR.639 + miR.338 | 0,73 | 0,074374157 |
| miR.609 + miR.29a + miR.494 + miR.639 | 0,73 | 0,042566365 |
| miR.609 + miR.519b + miR.26a + miR.518c | 0,73 | 0,006647307 |
| miR.520f + miR.130a + miR.376a* + miR.518c | 0,73 | 0,04859414 |
| miR.609 + miR.519b + miR.518c + miR.338 | 0,73 | 0,006807561 |
| miR.609 + miR.220a + miR.29a + miR.338 | 0,73 | 0,015999341 |
| miR.609 + miR.519b + miR.29a + miR.494 | 0,73 | 0,040745552 |
| miR.558 + miR.29a + miR.518c + miR.660 | 0,73 | 0,030602068 |
| miR.558 + miR.609 + miR.26a + miR.369.3p | 0,73 | 0,081066424 |
| miR.609 + miR.519b + miR.130a + miR.376a* | 0,73 | 0,060025821 |
| miR.558 + miR.609 + miR.519b + miR.369.3p | 0,73 | 0,077410062 |
| miR.558 + miR.519b + miR.369.3p + miR.518c | 0,73 | 0,053466843 |
| miR.609 + miR.26a + miR.362 + miR.518c | 0,73 | 0,009256496 |
| miR.609 + miR.369.3p + miR.518c + miR.660 | 0,73 | 0,010362334 |
| miR.558 + miR.369.3p + miR.494 + miR.518c | 0,73 | 0,042544066 |
| miR.558 + miR.519b + miR.518c + miR.660 | 0,73 | 0,033810946 |
| miR.609 + miR.520f + miR.220a + miR.639 | 0,73 | 0,057658231 |
| miR.520f + miR.519b + miR.130a + miR.518c | 0,73 | 0,043576783 |
| miR.609 + miR.362 + miR.376a* + miR.518c | 0,73 | 0,003926436 |
| miR.520f + miR.362 + miR.369.3p + miR.518c | 0,73 | 0,07167175 |
| miR.558 + miR.369.3p + miR.518c + miR.338 | 0,73 | 0,03659566 |
| miR.220a + miR.518c + miR.639 + miR.338 | 0,73 | 0,008625653 |
| miR.520f + miR.26a + miR.362 + miR.518c | 0,73 | 0,042129104 |
| miR.609 + miR.603 + miR.362 + miR.369.3p | 0,73 | 0,066110609 |
| miR.520f + miR.130a + miR.362 + miR.518c | 0,73 | 0,066757079 |
| miR.558 + miR.369.3p + miR.518c + miR.660 | 0,73 | 0,04576864 |
| miR.609 + miR.362 + miR.518c + miR.660 | 0,73 | 0,005445028 |
| miR.609 + miR.520f + miR.369.3p + miR.639 | 0,73 | 0,060200502 |
| miR.609 + miR.220a + miR.519b + miR.29a | 0,73 | 0,017829862 |
| miR.220a + miR.376a* + miR.518c + miR.639 | 0,73 | 0,024536373 |
| miR.609 + miR.220a + miR.29a + miR.639 | 0,73 | 0,017469826 |
| miR.609 + miR.220a + miR.130a + miR.29a | 0,73 | 0,016035157 |
| miR.558 + miR.518c + miR.660 + miR.338 | 0,73 | 0,026559768 |
| miR.220a + miR.26a + miR.369.3p + miR.518c | 0,73 | 0,023284351 |
| miR.520f + miR.130a + miR.26a + miR.518c | 0,73 | 0,049486625 |
| miR.609 + miR.519b + miR.376a* + miR.639 | 0,73 | 0,074001601 |
| miR.609 + miR.362 + miR.518c + miR.338 | 0,73 | 0,005669306 |
| miR.609 + miR.494 + miR.518c + miR.660 | 0,73 | 0,017339014 |
| miR.558 + miR.130a + miR.369.3p + miR.518c | 0,73 | 0,04801593 |
| miR.558 + miR.519b + miR.518c + miR.338 | 0,73 | 0,022642243 |
| miR.609 + miR.603 + miR.494 + miR.639 | 0,73 | 0,099375105 |
| miR.609 + miR.519b + miR.26a + miR.29a | 0,73 | 0,009115276 |
| miR.558 + miR.603 + miR.520f + miR.639 | 0,73 | 0,111635217 |
| miR.609 + miR.130a + miR.369.3p + miR.376a* | 0,73 | 0,094334817 |
| miR.520f + miR.519b + miR.26a + miR.518c | 0,73 | 0,036616858 |
| miR.558 + miR.494 + miR.518c + miR.660 | 0,73 | 0,0209133 |
| miR.558 + miR.519b + miR.494 + miR.518c | 0,73 | 0,029778652 |
| miR.220a + miR.369.3p + miR.518c + miR.338 | 0,73 | 0,024792375 |
| miR.609 + miR.220a + miR.29a + miR.362 | 0,73 | 0,017908865 |
| miR.220a + miR.130a + miR.26a + miR.518c | 0,73 | 0,020876153 |
| miR.609 + miR.520f + miR.639 + miR.660 | 0,73 | 0,055728073 |
| miR.609 + miR.26a + miR.29a + miR.362 | 0,73 | 0,006368033 |
| miR.558 + miR.519b + miR.130a + miR.518c | 0,73 | 0,027551883 |
| miR.558 + miR.609 + miR.494 + miR.660 | 0,73 | 0,065683851 |
| miR.609 + miR.603 + miR.130a + miR.369.3p | 0,73 | 0,058944345 |
| miR.220a + miR.26a + miR.518c + miR.639 | 0,73 | 0,01481234 |
| miR.609 + miR.369.3p + miR.376a* + miR.518c | 0,73 | 0,014701023 |
| miR.520f + miR.26a + miR.369.3p + miR.518c | 0,73 | 0,034621629 |
| miR.520f + miR.26a + miR.518c + miR.660 | 0,73 | 0,029220716 |
| miR.609 + miR.519b + miR.29a + miR.369.3p | 0,73 | 0,023972747 |
| miR.609 + miR.603 + miR.220a + miR.130a | 0,73 | 0,034829252 |
| miR.558 + miR.130a + miR.518c + miR.660 | 0,73 | 0,030952624 |
| miR.220a + miR.130a + miR.376a* + miR.518c | 0,73 | 0,028758485 |
| miR.609 + miR.26a + miR.29a + miR.639 | 0,73 | 0,007463068 |
| miR.609 + miR.26a + miR.29a + miR.369.3p | 0,73 | 0,015898228 |
| miR.558 + miR.609 + miR.26a + miR.494 | 0,73 | 0,068523648 |
| miR.520f + miR.26a + miR.494 + miR.518c | 0,73 | 0,044919939 |
| miR.520f + miR.519b + miR.362 + miR.518c | 0,73 | 0,048769905 |
| miR.520f + miR.26a + miR.518c + miR.338 | 0,73 | 0,029715495 |
| miR.609 + miR.603 + miR.369.3p + miR.376a* | 0,73 | 0,077237275 |
| miR.558 + miR.609 + miR.494 + miR.338 | 0,73 | 0,077101971 |
| miR.609 + miR.26a + miR.494 + miR.518c | 0,73 | 0,017895554 |
| miR.558 + miR.603 + miR.520f + miR.29a | 0,73 | 0,109254688 |
| miR.609 + miR.376a* + miR.494 + miR.639 | 0,73 | 0,136796764 |
| miR.520f + miR.130a + miR.369.3p + miR.518c | 0,73 | 0,054306405 |
| miR.609 + miR.603 + miR.130a + miR.494 | 0,73 | 0,066990008 |
| miR.609 + miR.29a + miR.369.3p + miR.338 | 0,73 | 0,012834623 |

**Table 5: clusters of 5 miRNAs**

| **Cluster of 5 miRs** | **c_tau level** | **pv compared to top signature miR.558 + miR.609 + miR.29a + miR.376a* + miR.518c** |
|---|---|---|
| miR.558 + miR.609 + miR.29a + miR.376a* + miR.518c | 0,79 | NaN |
| miR.609 + miR.603 + miR.29a + miR.376a* + miR.518c | 0,79 | 0,327442837 |
| miR.558 + miR.609 + miR.520f + miR.29a + miR.376a* | 0,79 | 0,327799124 |
| miR.609 + miR.220a + miR.29a + miR.376a* + miR.518c | 0,79 | 0,01115903 |
| miR.609 + miR.520f + miR.29a + miR.376a* + miR.518c | 0,79 | 0,450779905 |
| miR.558 + miR.609 + miR.520f + miR.29a + miR.518c | 0,78 | 0,059817433 |
| miR.558 + miR.609 + miR.26a + miR.29a + miR.518c | 0,78 | 0,043671541 |
| miR.558 + miR.609 + miR.376a* + miR.518c + miR.639 | 0,78 | 0,23022043 |
| miR.609 + miR.519b + miR.29a + miR.376a* + miR.518c | 0,78 | 0,188539884 |
| miR.558 + miR.609 + miR.29a + miR.518c + miR.660 | 0,78 | 0,002653657 |
| miR.609 + miR.520f + miR.29a + miR.376a* + miR.494 | 0,78 | 0,249066295 |
| miR.558 + miR.609 + miR.29a + miR.369.3p + miR.376a* | 0,78 | 0,158036688 |
| miR.558 + miR.609 + miR.520f + miR.518c + miR.639 | 0,78 | 0,063513262 |
| miR.609 + miR.29a + miR.369.3p + miR.376a* + miR.518c | 0,78 | 0,143839599 |
| miR.558 + miR.609 + miR.130a + miR.376a* + miR.518c | 0,78 | 0,110267568 |
| miR.558 + miR.609 + miR.603 + miR.376a* + miR.518c | 0,78 | 0,035625688 |
| miR.609 + miR.520f + miR.29a + miR.518c + miR.660 | 0,78 | 0,067617987 |
| miR.609 + miR.29a + miR.376a* + miR.494 + miR.518c | 0,77 | 0,350446224 |
| miR.558 + miR.609 + miR.603 + miR.29a + miR.518c | 0,77 | 0,016877074 |
| miR.609 + miR.603 + miR.130a + miR.376a* + miR.518c | 0,77 | 0,041540235 |
| miR.609 + miR.603 + miR.376a* + miR.518c + miR.639 | 0,77 | 0,026897842 |
| miR.558 + miR.609 + miR.220a + miR.29a + miR.376a* | 0,77 | 0,017879839 |
| miR.558 + miR.609 + miR.220a + miR.29a + miR.518c | 0,77 | 0,006280085 |
| miR.558 + miR.609 + miR.29a + miR.518c + miR.338 | 0,77 | 0,011101684 |
| miR.558 + miR.609 + miR.29a + miR.376a* + miR.494 | 0,77 | 0,062612995 |
| miR.558 + miR.609 + miR.603 + miR.520f + miR.518c | 0,77 | 0,03443809 |
| miR.558 + miR.609 + miR.220a + miR.376a* + miR.518c | 0,77 | 0,033997596 |
| miR.558 + miR.609 + miR.130a + miR.29a + miR.518c | 0,77 | 0,002887257 |
| miR.558 + miR.609 + miR.520f + miR.130a + miR.518c | 0,77 | 0,037877132 |
| miR.558 + miR.609 + miR.603 + miR.220a + miR.518c | 0,77 | 0,017976338 |
| miR.609 + miR.520f + miR.29a + miR.376a* + miR.338 | 0,77 | 0,407308292 |
| miR.609 + miR.29a + miR.376a* + miR.518c + miR.639 | 0,77 | 0,17469374 |
| miR.558 + miR.609 + miR.26a + miR.29a + miR.376a* | 0,77 | 0,008922532 |
| miR.558 + miR.609 + miR.520f + miR.130a + miR.376a* | 0,77 | 0,140289539 |
| miR.609 + miR.520f + miR.130a + miR.376a* + miR.518c | 0,77 | 0,059066796 |
| miR.558 + miR.609 + miR.603 + miR.518c + miR.639 | 0,77 | 0,027137149 |
| miR.609 + miR.520f + miR.29a + miR.369.3p + miR.376a* | 0,77 | 0,373692037 |
| miR.609 + miR.520f + miR.26a + miR.29a + miR.376a* | 0,77 | 0,366136004 |
| miR.558 + miR.609 + miR.29a + miR.376a* + miR.639 | 0,77 | 0,020223844 |
| miR.558 + miR.609 + miR.369.3p + miR.376a* + miR.518c | 0,77 | 0,167914542 |
| miR.609 + miR.29a + miR.376a* + miR.518c + miR.660 | 0,77 | 0,113571149 |
| miR.609 + miR.220a + miR.130a + miR.376a* + miR.518c | 0,77 | 0,028574731 |
| miR.609 + miR.26a + miR.29a + miR.376a* + miR.518c | 0,77 | 0,157501218 |
| miR.609 + miR.520f + miR.130a + miR.29a + miR.376a* | 0,77 | 0,231742772 |
| miR.609 + miR.29a + miR.362 + miR.376a* + miR.518c | 0,77 | 0,185874123 |
| miR.609 + miR.520f + miR.519b + miR.29a + miR.376a* | 0,77 | 0,192784408 |
| miR.609 + miR.29a + miR.376a* + miR.518c + miR.338 | 0,77 | 0,133790333 |
| miR.609 + miR.130a + miR.29a + miR.376a* + miR.518c | 0,77 | 0,174916686 |
| miR.558 + miR.609 + miR.603 + miR.29a + miR.376a* | 0,77 | 0,031799615 |
| miR.558 + miR.609 + miR.29a + miR.369.3p + miR.518c | 0,77 | 0,005160508 |
| miR.609 + miR.603 + miR.220a + miR.376a* + miR.518c | 0,77 | 0,04671063 |
| miR.609 + miR.603 + miR.520f + miR.29a + miR.518c | 0,77 | 0,099573877 |
| miR.609 + miR.603 + miR.520f + miR.29a + miR.376a* | 0,77 | 0,37830202 |
| miR.558 + miR.609 + miR.603 + miR.369.3p + miR.518c | 0,77 | 0,028220566 |
| miR.558 + miR.603 + miR.220a + miR.376a* + miR.518c | 0,77 | 0,03980927 |
| miR.558 + miR.609 + miR.519b + miR.29a + miR.518c | 0,77 | 0,007674366 |
| miR.558 + miR.609 + miR.29a + miR.362 + miR.518c | 0,77 | 0,004377824 |
| miR.558 + miR.609 + miR.29a + miR.518c + miR.639 | 0,77 | 0,005411378 |
| miR.558 + miR.609 + miR.29a + miR.494 + miR.518c | 0,77 | 0,004831169 |
| miR.558 + miR.609 + miR.603 + miR.130a + miR.518c | 0,77 | 0,017870286 |
| miR.558 + miR.609 + miR.603 + miR.519b + miR.518c | 0,77 | 0,027427599 |
| miR.609 + miR.520f + miR.220a + miR.29a + miR.518c | 0,77 | 0,048133374 |
| miR.558 + miR.609 + miR.520f + miR.220a + miR.518c | 0,77 | 0,026797673 |
| miR.558 + miR.609 + miR.520f + miR.376a* + miR.518c | 0,77 | 0,100903248 |
| miR.558 + miR.609 + miR.26a + miR.518c + miR.639 | 0,77 | 0,095109646 |
| miR.558 + miR.609 + miR.29a + miR.362 + miR.376a* | 0,77 | 0,013602017 |
| miR.558 + miR.609 + miR.29a + miR.376a* + miR.660 | 0,77 | 0,018468784 |
| miR.558 + miR.609 + miR.29a + miR.376a* + miR.338 | 0,77 | 0,0121737 |
| miR.558 + miR.609 + miR.520f + miR.29a + miR.660 | 0,77 | 0,066139496 |
| miR.609 + miR.520f + miR.220a + miR.29a + miR.376a* | 0,77 | 0,225976847 |
| miR.609 + miR.520f + miR.29a + miR.376a* + miR.639 | 0,77 | 0,28280271 |
| miR.558 + miR.609 + miR.520f + miR.26a + miR.518c | 0,77 | 0,024846111 |
| miR.558 + miR.609 + miR.520f + miR.369.3p + miR.518c | 0,77 | 0,041924518 |
| miR.609 + miR.520f + miR.29a + miR.518c + miR.639 | 0,77 | 0,033274671 |
| miR.609 + miR.520f + miR.29a + miR.376a* + miR.660 | 0,77 | 0,253849844 |
| miR.609 + miR.220a + miR.29a + miR.518c + miR.660 | 0,77 | 0,001466597 |
| miR.558 + miR.609 + miR.519b + miR.29a + miR.376a* | 0,77 | 0,021908997 |
| miR.558 + miR.609 + miR.130a + miR.29a + miR.376a* | 0,77 | 0,01948068 |
| miR.609 + miR.520f + miR.29a + miR.518c + miR.338 | 0,77 | 0,022546602 |
| miR.609 + miR.520f + miR.29a + miR.362 + miR.518c | 0,77 | 0,035845969 |
| miR.609 + miR.520f + miR.29a + miR.362 + miR.376a* | 0,77 | 0,235677599 |
| miR.609 + miR.603 + miR.220a + miR.29a + miR.518c | 0,77 | 0,02363119 |
| miR.558 + miR.609 + miR.603 + miR.26a + miR.518c | 0,77 | 0,029204349 |
| miR.609 + miR.520f + miR.29a + miR.494 + miR.518c | 0,77 | 0,034015223 |
| miR.558 + miR.609 + miR.603 + miR.518c + miR.338 | 0,77 | 0,023944369 |
| miR.558 + miR.603 + miR.220a + miR.519b + miR.518c | 0,77 | 0,046578651 |
| miR.609 + miR.603 + miR.369.3p + miR.376a* + miR.518c | 0,77 | 0,036178907 |
| miR.609 + miR.520f + miR.29a + miR.369.3p + miR.518c | 0,77 | 0,026716689 |
| miR.558 + miR.609 + miR.603 + miR.494 + miR.518c | 0,77 | 0,020979226 |
| miR.558 + miR.609 + miR.520f + miR.519b + miR.518c | 0,77 | 0,075018623 |
| miR.558 + miR.609 + miR.603 + miR.362 + miR.518c | 0,77 | 0,01311257 |
| miR.603 + miR.220a + miR.369.3p + miR.376a* + miR.518c | 0,77 | 0,090956912 |
| miR.609 + miR.520f + miR.130a + miR.29a + miR.518c | 0,77 | 0,030110896 |
| miR.609 + miR.520f + miR.26a + miR.29a + miR.518c | 0,77 | 0,033978099 |
| miR.558 + miR.609 + miR.220a + miR.518c + miR.639 | 0,77 | 0,010358514 |
| miR.609 + miR.520f + miR.519b + miR.29a + miR.518c | 0,77 | 0,029936391 |
| miR.609 + miR.520f + miR.376a* + miR.518c + miR.639 | 0,77 | 0,0426317 |
| miR.558 + miR.609 + miR.520f + miR.362 + miR.518c | 0,77 | 0,037379284 |
| miR.558 + miR.609 + miR.520f + miR.518c + miR.338 | 0,77 | 0,032694828 |
| miR.558 + miR.609 + miR.603 + miR.518c + miR.660 | 0,77 | 0,022460856 |
| miR.558 + miR.609 + miR.220a + miR.369.3p + miR.518c | 0,77 | 0,015591292 |
| miR.558 + miR.609 + miR.376a* + miR.518c + miR.338 | 0,77 | 0,072802745 |
| miR.609 + miR.603 + miR.520f + miR.518c + miR.639 | 0,77 | 0,058039953 |
| miR.558 + miR.609 + miR.220a + miR.519b + miR.518c | 0,77 | 0,008198503 |
| miR.609 + miR.603 + miR.29a + miR.518c + miR.660 | 0,77 | 0,004944248 |
| miR.558 + miR.609 + miR.520f + miR.494 + miR.518c | 0,77 | 0,046137579 |
| miR.558 + miR.609 + miR.518c + miR.639 + miR.338 | 0,77 | 0,035275149 |
| miR.558 + miR.609 + miR.376a* + miR.518c + miR.660 | 0,77 | 0,05650841 |
| miR.558 + miR.609 + miR.362 + miR.376a* + miR.518c | 0,77 | 0,064266389 |
| miR.609 + miR.603 + miR.26a + miR.29a + miR.518c | 0,77 | 0,0346868 |
| miR.609 + miR.603 + miR.29a + miR.376a* + miR.494 | 0,77 | 0,109005651 |
| miR.609 + miR.220a + miR.29a + miR.518c + miR.338 | 0,77 | 0,00542927 |
| miR.558 + miR.609 + miR.220a + miR.130a + miR.518c | 0,77 | 0,006191584 |
| miR.558 + miR.609 + miR.520f + miR.518c + miR.660 | 0,77 | 0,039493571 |
| miR.558 + miR.609 + miR.518c + miR.639 + miR.660 | 0,77 | 0,075839077 |
| miR.609 + miR.603 + miR.29a + miR.518c + miR.338 | 0,77 | 0,012245334 |
| miR.609 + miR.220a + miR.26a + miR.29a + miR.518c | 0,77 | 0,009385259 |
| miR.558 + miR.609 + miR.362 + miR.518c + miR.639 | 0,77 | 0,015266454 |
| miR.558 + miR.609 + miR.520f + miR.26a + miR.29a | 0,77 | 0,056607697 |
| miR.603 + miR.220a + miR.29a + miR.376a* + miR.518c | 0,77 | 0,068683646 |
| miR.558 + miR.609 + miR.220a + miR.26a + miR.518c | 0,77 | 0,011596267 |
| miR.558 + miR.609 + miR.369.3p + miR.518c + miR.639 | 0,77 | 0,039044417 |
| miR.609 + miR.520f + miR.130a + miR.376a* + miR.338 | 0,77 | 0,227383044 |
| miR.558 + miR.609 + miR.220a + miR.518c + miR.660 | 0,77 | 0,008650483 |
| miR.558 + miR.609 + miR.520f + miR.130a + miR.29a | 0,76 | 0,049657608 |
| miR.558 + miR.609 + miR.130a + miR.26a + miR.518c | 0,76 | 0,032765546 |
| miR.558 + miR.609 + miR.220a + miR.518c + miR.338 | 0,76 | 0,007071801 |
| miR.558 + miR.609 + miR.494 + miR.518c + miR.639 | 0,76 | 0,024604157 |
| miR.558 + miR.609 + miR.520f + miR.519b + miR.29a | 0,76 | 0,088466268 |
| miR.609 + miR.603 + miR.520f + miR.130a + miR.518c | 0,76 | 0,060820074 |
| miR.558 + miR.609 + miR.519b + miR.518c + miR.639 | 0,76 | 0,037860542 |
| miR.603 + miR.220a + miR.130a + miR.376a* + miR.518c | 0,76 | 0,059374762 |
| miR.558 + miR.603 + miR.520f + miR.376a* + miR.518c | 0,76 | 0,05220861 |
| miR.558 + miR.609 + miR.603 + miR.520f + miR.29a | 0,76 | 0,043855643 |
| miR.609 + miR.603 + miR.29a + miR.369.3p + miR.376a* | 0,76 | 0,181642701 |
| miR.558 + miR.609 + miR.220a + miR.362 + miR.518c | 0,76 | 0,007746776 |
| miR.558 + miR.609 + miR.130a + miR.518c + miR.639 | 0,76 | 0,023485054 |
| miR.558 + miR.609 + miR.520f + miR.29a + miR.639 | 0,76 | 0,062774075 |
| miR.558 + miR.609 + miR.220a + miR.494 + miR.518c | 0,76 | 0,007759013 |
| miR.558 + miR.609 + miR.26a + miR.376a* + miR.518c | 0,76 | 0,042891276 |
| miR.558 + miR.609 + miR.519b + miR.376a* + miR.518c | 0,76 | 0,044007041 |
| miR.558 + miR.609 + miR.520f + miR.376a* + miR.639 | 0,76 | 0,155435952 |
| miR.609 + miR.603 + miR.220a + miR.130a + miR.518c | 0,76 | 0,023795849 |
| miR.609 + miR.603 + miR.520f + miR.130a + miR.376a* | 0,76 | 0,144793046 |
| miR.558 + miR.609 + miR.376a* + miR.494 + miR.518c | 0,76 | 0,107352539 |
| miR.558 + miR.609 + miR.520f + miR.29a + miR.494 | 0,76 | 0,063864386 |
| miR.558 + miR.609 + miR.362 + miR.369.3p + miR.518c | 0,76 | 0,014633171 |
| miR.558 + miR.609 + miR.520f + miR.29a + miR.362 | 0,76 | 0,066015305 |
| miR.558 + miR.609 + miR.520f + miR.29a + miR.369.3p | 0,76 | 0,06352373 |
| miR.609 + miR.603 + miR.220a + miR.518c + miR.639 | 0,76 | 0,018261185 |
| miR.603 + miR.220a + miR.376a* + miR.518c + miR.639 | 0,76 | 0,054394079 |
| miR.558 + miR.609 + miR.520f + miR.29a + miR.338 | 0,76 | 0,057639817 |
| miR.558 + miR.609 + miR.520f + miR.220a + miR.29a | 0,76 | 0,068562148 |
| miR.609 + miR.220a + miR.29a + miR.369.3p + miR.376a* | 0,76 | 0,168648268 |
| miR.558 + miR.603 + miR.520f + miR.220a + miR.518c | 0,76 | 0,020501787 |
| miR.609 + miR.520f + miR.220a + miR.130a + miR.518c | 0,76 | 0,017413575 |
| miR.609 + miR.603 + miR.29a + miR.369.3p + miR.518c | 0,76 | 0,011994811 |
| miR.609 + miR.220a + miR.376a* + miR.518c + miR.639 | 0,76 | 0,002219588 |
| miR.558 + miR.603 + miR.376a* + miR.518c + miR.639 | 0,76 | 0,027563503 |
| miR.558 + miR.603 + miR.520f + miR.519b + miR.518c | 0,76 | 0,07252901 |
| miR.609 + miR.603 + miR.220a + miR.369.3p + miR.518c | 0,76 | 0,019700311 |
| miR.609 + miR.603 + miR.29a + miR.494 + miR.518c | 0,76 | 0,022769325 |
| miR.558 + miR.603 + miR.220a + miR.518c + miR.338 | 0,76 | 0,01179661 |
| miR.558 + miR.603 + miR.369.3p + miR.376a* + miR.518c | 0,76 | 0,065638767 |
| miR.558 + miR.603 + miR.220a + miR.26a + miR.518c | 0,76 | 0,033907502 |
| miR.609 + miR.603 + miR.520f + miR.220a + miR.518c | 0,76 | 0,01732095 |
| miR.609 + miR.603 + miR.376a* + miR.494 + miR.518c | 0,76 | 0,058385957 |
| miR.609 + miR.603 + miR.220a + miR.519b + miR.518c | 0,76 | 0,018677282 |
| miR.558 + miR.609 + miR.26a + miR.369.3p + miR.518c | 0,76 | 0,064077303 |
| miR.609 + miR.603 + miR.29a + miR.518c + miR.639 | 0,76 | 0,016265908 |
| miR.609 + miR.603 + miR.220a + miR.494 + miR.518c | 0,76 | 0,023047739 |
| miR.558 + miR.603 + miR.520f + miR.518c + miR.639 | 0,76 | 0,029622658 |
| miR.558 + miR.609 + miR.130a + miR.518c + miR.338 | 0,76 | 0,014299426 |
| miR.609 + miR.603 + miR.519b + miR.29a + miR.518c | 0,76 | 0,016726432 |
| miR.558 + miR.609 + miR.130a + miR.369.3p + miR.518c | 0,76 | 0,014416164 |
| miR.609 + miR.26a + miR.29a + miR.518c + miR.660 | 0,76 | 0,000646569 |
| miR.558 + miR.520f + miR.376a* + miR.518c + miR.639 | 0,76 | 0,089500075 |
| miR.603 + miR.520f + miR.376a* + miR.518c + miR.639 | 0,76 | 0,035843726 |
| miR.609 + miR.603 + miR.130a + miR.29a + miR.518c | 0,76 | 0,013363391 |
| miR.609 + miR.603 + miR.29a + miR.362 + miR.518c | 0,76 | 0,013168181 |
| miR.603 + miR.220a + miR.519b + miR.376a* + miR.518c | 0,76 | 0,076073782 |
| miR.609 + miR.520f + miR.220a + miR.518c + miR.639 | 0,76 | 0,019407372 |
| miR.609 + miR.220a + miR.29a + miR.376a* + miR.494 | 0,76 | 0,114865704 |
| miR.609 + miR.520f + miR.130a + miR.518c + miR.639 | 0,76 | 0,01746451 |
| miR.603 + miR.520f + miR.220a + miR.376a* + miR.518c | 0,76 | 0,071800437 |
| miR.609 + miR.603 + miR.220a + miR.518c + miR.338 | 0,76 | 0,016855614 |
| miR.558 + miR.603 + miR.29a + miR.376a* + miR.518c | 0,76 | 0,013240032 |
| miR.609 + miR.29a + miR.362 + miR.369.3p + miR.376a* | 0,76 | 0,220411833 |
| miR.558 + miR.603 + miR.220a + miR.369.3p + miR.518c | 0,76 | 0,011574552 |
| miR.609 + miR.520f + miR.518c + miR.639 + miR.660 | 0,76 | 0,026277595 |
| miR.558 + miR.609 + miR.519b + miR.369.3p + miR.518c | 0,76 | 0,048179323 |
| miR.558 + miR.609 + miR.130a + miR.362 + miR.518c | 0,76 | 0,009488054 |
| miR.609 + miR.520f + miR.130a + miR.518c + miR.660 | 0,76 | 0,011782281 |
| miR.558 + miR.609 + miR.362 + miR.518c + miR.660 | 0,76 | 0,021648874 |
| miR.609 + miR.603 + miR.130a + miR.26a + miR.518c | 0,76 | 0,011152616 |
| miR.558 + miR.609 + miR.520f + miR.26a + miR.376a* | 0,76 | 0,051931099 |
| miR.558 + miR.603 + miR.376a* + miR.518c + miR.660 | 0,76 | 0,035240698 |
| miR.558 + miR.609 + miR.519b + miR.362 + miR.518c | 0,76 | 0,025224668 |
| miR.609 + miR.220a + miR.29a + miR.369.3p + miR.518c | 0,76 | 0,003275441 |
| miR.558 + miR.609 + miR.369.3p + miR.518c + miR.338 | 0,76 | 0,035823594 |
| miR.609 + miR.520f + miR.26a + miR.518c + miR.639 | 0,76 | 0,032642896 |
| miR.558 + miR.609 + miR.369.3p + miR.494 + miR.518c | 0,76 | 0,024228242 |
| miR.558 + miR.609 + miR.26a + miR.362 + miR.518c | 0,76 | 0,0244115 |
| miR.609 + miR.603 + miR.220a + miR.518c + miR.660 | 0,76 | 0,025612318 |
| miR.609 + miR.603 + miR.220a + miR.26a + miR.518c | 0,76 | 0,022902934 |
| miR.609 + miR.220a + miR.29a + miR.494 + miR.518c | 0,76 | 0,003430325 |
| miR.558 + miR.609 + miR.130a + miR.518c + miR.660 | 0,76 | 0,023670606 |
| miR.609 + miR.29a + miR.369.3p + miR.376a* + miR.494 | 0,76 | 0,120811765 |
| miR.558 + miR.609 + miR.362 + miR.518c + miR.338 | 0,76 | 0,014571932 |
| miR.558 + miR.609 + miR.369.3p + miR.518c + miR.660 | 0,76 | 0,044847549 |
| miR.558 + miR.609 + miR.130a + miR.494 + miR.518c | 0,76 | 0,020151915 |
| miR.558 + miR.609 + miR.519b + miR.130a + miR.518c | 0,76 | 0,026257119 |
| miR.609 + miR.220a + miR.29a + miR.362 + miR.518c | 0,76 | 0,002018625 |
| miR.609 + miR.603 + miR.220a + miR.362 + miR.518c | 0,76 | 0,019735452 |
| miR.609 + miR.29a + miR.362 + miR.518c + miR.660 | 0,76 | 0,001531379 |
| miR.609 + miR.220a + miR.130a + miR.29a + miR.518c | 0,76 | 0,002969535 |
| miR.609 + miR.603 + miR.520f + miR.376a* + miR.518c | 0,76 | 0,021791801 |
| miR.558 + miR.603 + miR.220a + miR.518c + miR.660 | 0,76 | 0,013152501 |
| miR.558 + miR.603 + miR.220a + miR.29a + miR.518c | 0,76 | 0,007763354 |
| miR.558 + miR.609 + miR.362 + miR.494 + miR.518c | 0,76 | 0,014731694 |
| miR.609 + miR.520f + miR.130a + miR.26a + miR.376a* | 0,76 | 0,16200117 |
| miR.558 + miR.603 + miR.220a + miR.518c + miR.639 | 0,76 | 0,007508418 |
| miR.558 + miR.603 + miR.26a + miR.518c + miR.639 | 0,76 | 0,052154719 |
| miR.609 + miR.220a + miR.29a + miR.518c + miR.639 | 0,76 | 0,003091116 |
| miR.558 + miR.603 + miR.220a + miR.494 + miR.518c | 0,76 | 0,010178039 |
| miR.609 + miR.520f + miR.519b + miR.518c + miR.639 | 0,76 | 0,026238301 |
| miR.609 + miR.519b + miR.29a + miR.369.3p + miR.376a* | 0,76 | 0,107303707 |
| miR.609 + miR.220a + miR.519b + miR.29a + miR.518c | 0,76 | 0,003631923 |
| miR.609 + miR.220a + miR.369.3p + miR.376a* + miR.518c | 0,76 | 0,007208339 |
| miR.558 + miR.520f + miR.29a + miR.376a* + miR.518c | 0,76 | 0,049689549 |
| miR.558 + miR.603 + miR.520f + miR.29a + miR.518c | 0,76 | 0,021737337 |
| miR.609 + miR.26a + miR.29a + miR.494 + miR.518c | 0,76 | 0,002102546 |
| miR.603 + miR.220a + miR.376a* + miR.494 + miR.518c | 0,76 | 0,036759049 |
| miR.609 + miR.603 + miR.220a + miR.29a + miR.376a* | 0,76 | 0,053938304 |
| miR.558 + miR.603 + miR.519b + miR.376a* + miR.518c | 0,76 | 0,041720652 |
| miR.609 + miR.520f + miR.494 + miR.518c + miR.639 | 0,76 | 0,022405267 |
| miR.558 + miR.603 + miR.220a + miR.130a + miR.518c | 0,76 | 0,01231129 |
| miR.558 + miR.603 + miR.220a + miR.362 + miR.518c | 0,76 | 0,00903321 |
| miR.609 + miR.519b + miR.26a + miR.29a + miR.518c | 0,76 | 0,001598197 |
| miR.609 + miR.603 + miR.26a + miR.518c + miR.639 | 0,76 | 0,015889918 |
| miR.609 + miR.29a + miR.518c + miR.660 + miR.338 | 0,76 | 0,000161201 |
| miR.609 + miR.520f + miR.369.3p + miR.518c + miR.639 | 0,76 | 0,023386701 |
| miR.558 + miR.603 + miR.362 + miR.376a* + miR.518c | 0,76 | 0,027638813 |
| miR.609 + miR.603 + miR.369.3p + miR.518c + miR.639 | 0,76 | 0,022896138 |
| miR.609 + miR.520f + miR.130a + miR.376a* + miR.639 | 0,76 | 0,081231616 |
| miR.609 + miR.519b + miR.376a* + miR.518c + miR.639 | 0,76 | 0,008433744 |
| miR.558 + miR.609 + miR.519b + miR.518c + miR.338 | 0,76 | 0,041751735 |
| miR.558 + miR.603 + miR.26a + miR.29a + miR.518c | 0,76 | 0,014455142 |
| miR.609 + miR.603 + miR.518c + miR.639 + miR.338 | 0,76 | 0,005822029 |
| miR.609 + miR.603 + miR.130a + miR.518c + miR.639 | 0,76 | 0,010164618 |
| miR.609 + miR.603 + miR.376a* + miR.518c + miR.660 | 0,76 | 0,010253425 |
| miR.558 + miR.609 + miR.519b + miR.518c + miR.660 | 0,76 | 0,049471421 |
| miR.558 + miR.609 + miR.130a + miR.369.3p + miR.376a* | 0,76 | 0,149697262 |
| miR.558 + miR.609 + miR.26a + miR.494 + miR.518c | 0,76 | 0,046963722 |
| miR.603 + miR.220a + miR.376a* + miR.518c + miR.660 | 0,76 | 0,048900387 |
| miR.558 + miR.603 + miR.520f + miR.494 + miR.518c | 0,76 | 0,024856532 |
| miR.609 + miR.520f + miR.26a + miR.376a* + miR.518c | 0,76 | 0,021053337 |
| miR.609 + miR.520f + miR.518c + miR.639 + miR.338 | 0,76 | 0,019478405 |
| miR.609 + miR.519b + miR.29a + miR.518c + miR.660 | 0,76 | 0,000401115 |
| miR.609 + miR.29a + miR.494 + miR.518c + miR.660 | 0,76 | 0,002457844 |
| miR.609 + miR.520f + miR.130a + miR.369.3p + miR.376a* | 0,76 | 0,104284792 |
| miR.558 + miR.609 + miR.519b + miR.26a + miR.518c | 0,76 | 0,049598796 |
| miR.609 + miR.520f + miR.362 + miR.518c + miR.639 | 0,76 | 0,021826248 |
| miR.609 + miR.603 + miR.520f + miR.369.3p + miR.518c | 0,76 | 0,021265273 |
| miR.558 + miR.603 + miR.520f + miR.369.3p + miR.518c | 0,76 | 0,033445228 |
| miR.558 + miR.603 + miR.130a + miR.376a* + miR.518c | 0,76 | 0,028261204 |
| miR.609 + miR.603 + miR.376a* + miR.518c + miR.338 | 0,76 | 0,010570983 |
| miR.609 + miR.603 + miR.362 + miR.376a* + miR.518c | 0,76 | 0,008819616 |
| miR.609 + miR.26a + miR.29a + miR.369.3p + miR.518c | 0,76 | 0,0021862 |
| miR.558 + miR.603 + miR.520f + miR.130a + miR.518c | 0,76 | 0,027187735 |
| miR.609 + miR.520f + miR.130a + miR.369.3p + miR.518c | 0,76 | 0,009091034 |
| miR.609 + miR.29a + miR.362 + miR.376a* + miR.494 | 0,76 | 0,07611372 |
| miR.603 + miR.220a + miR.362 + miR.376a* + miR.518c | 0,76 | 0,048416338 |
| miR.558 + miR.603 + miR.520f + miR.362 + miR.518c | 0,76 | 0,032644745 |
| miR.603 + miR.220a + miR.376a* + miR.518c + miR.338 | 0,76 | 0,038486109 |
| miR.603 + miR.220a + miR.26a + miR.376a* + miR.518c | 0,76 | 0,046320209 |
| miR.558 + miR.520f + miR.220a + miR.376a* + miR.518c | 0,76 | 0,033038578 |
| miR.609 + miR.520f + miR.519b + miR.130a + miR.518c | 0,76 | 0,010550971 |
| miR.558 + miR.603 + miR.520f + miR.518c + miR.660 | 0,76 | 0,034114893 |
| miR.603 + miR.520f + miR.29a + miR.376a* + miR.518c | 0,76 | 0,023006145 |
| miR.558 + miR.603 + miR.520f + miR.518c + miR.338 | 0,76 | 0,036799544 |
| miR.558 + miR.609 + miR.519b + miR.494 + miR.518c | 0,76 | 0,06410699 |
| miR.609 + miR.220a + miR.130a + miR.26a + miR.518c | 0,76 | 0,002119052 |
| miR.558 + miR.609 + miR.26a + miR.518c + miR.338 | 0,76 | 0,042287284 |
| miR.609 + miR.519b + miR.130a + miR.376a* + miR.518c | 0,76 | 0,007989029 |
| miR.558 + miR.609 + miR.26a + miR.518c + miR.660 | 0,76 | 0,040582972 |
| miR.609 + miR.520f + miR.130a + miR.518c + miR.338 | 0,76 | 0,006938008 |
| miR.609 + miR.603 + miR.494 + miR.518c + miR.639 | 0,76 | 0,017767437 |
| miR.609 + miR.520f + miR.130a + miR.362 + miR.518c | 0,76 | 0,008980282 |
| miR.558 + miR.603 + miR.26a + miR.376a* + miR.518c | 0,76 | 0,042647738 |
| miR.609 + miR.520f + miR.130a + miR.26a + miR.518c | 0,76 | 0,010152129 |
| miR.609 + miR.520f + miR.130a + miR.494 + miR.518c | 0,76 | 0,010953139 |
| miR.609 + miR.603 + miR.130a + miR.518c + miR.338 | 0,76 | 0,006348197 |
| miR.558 + miR.603 + miR.520f + miR.26a + miR.518c | 0,76 | 0,035806694 |
| miR.609 + miR.603 + miR.518c + miR.639 + miR.660 | 0,76 | 0,010630384 |
| miR.558 + miR.609 + miR.369.3p + miR.376a* + miR.639 | 0,76 | 0,229021768 |
| miR.558 + miR.609 + miR.603 + miR.130a + miR.376a* | 0,76 | 0,03971776 |
| miR.609 + miR.603 + miR.26a + miR.376a* + miR.518c | 0,76 | 0,006742826 |
| miR.609 + miR.603 + miR.130a + miR.369.3p + miR.518c | 0,76 | 0,010159921 |
| miR.609 + miR.603 + miR.362 + miR.518c + miR.639 | 0,76 | 0,011672276 |
| miR.558 + miR.609 + miR.518c + miR.660 + miR.338 | 0,76 | 0,031801258 |
| miR.609 + miR.26a + miR.29a + miR.518c + miR.338 | 0,76 | 0,003025169 |
| miR.558 + miR.609 + miR.494 + miR.518c + miR.338 | 0,76 | 0,032582396 |
| miR.603 + miR.362 + miR.369.3p + miR.376a* + miR.518c | 0,76 | 0,067169531 |
| miR.609 + miR.519b + miR.29a + miR.376a* + miR.494 | 0,76 | 0,057015103 |
| miR.609 + miR.603 + miR.520f + miR.26a + miR.518c | 0,76 | 0,016606257 |
| miR.558 + miR.609 + miR.26a + miR.29a + miR.369.3p | 0,76 | 0,016964813 |
| miR.609 + miR.603 + miR.520f + miR.494 + miR.518c | 0,76 | 0,03216242 |
| miR.609 + miR.603 + miR.29a + miR.376a* + miR.639 | 0,76 | 0,030163877 |
| miR.558 + miR.603 + miR.376a* + miR.494 + miR.518c | 0,76 | 0,023924104 |
| miR.558 + miR.603 + miR.376a* + miR.518c + miR.338 | 0,76 | 0,026608271 |
| miR.609 + miR.603 + miR.519b + miR.518c + miR.639 | 0,76 | 0,01119512 |
| miR.558 + miR.609 + miR.29a + miR.362 + miR.660 | 0,76 | 0,009330873 |
| miR.558 + miR.609 + miR.494 + miR.518c + miR.660 | 0,76 | 0,047432705 |
| miR.558 + miR.520f + miR.220a + miR.519b + miR.518c | 0,76 | 0,039277549 |
| miR.609 + miR.29a + miR.369.3p + miR.518c + miR.660 | 0,76 | 0,00098718 |
| miR.609 + miR.520f + miR.519b + miR.130a + miR.376a* | 0,76 | 0,075797653 |
| miR.609 + miR.29a + miR.518c + miR.639 + miR.660 | 0,76 | 0,00073455 |
| miR.558 + miR.609 + miR.603 + miR.520f + miR.639 | 0,76 | 0,042887922 |
| miR.558 + miR.609 + miR.603 + miR.376a* + miR.639 | 0,76 | 0,058038116 |
| miR.558 + miR.609 + miR.26a + miR.29a + miR.660 | 0,76 | 0,011052131 |
| miR.609 + miR.26a + miR.29a + miR.518c + miR.639 | 0,76 | 0,001547257 |
| miR.558 + miR.609 + miR.220a + miR.26a + miR.29a | 0,76 | 0,013185857 |
| miR.609 + miR.130a + miR.29a + miR.376a* + miR.494 | 0,76 | 0,047466604 |
| miR.603 + miR.369.3p + miR.376a* + miR.518c + miR.639 | 0,76 | 0,020526146 |
| miR.609 + miR.603 + miR.519b + miR.376a* + miR.518c | 0,76 | 0,007223758 |
| miR.609 + miR.520f + miR.29a + miR.494 + miR.660 | 0,76 | 0,073589539 |
| miR.609 + miR.29a + miR.494 + miR.518c + miR.338 | 0,76 | 0,003462953 |
| miR.603 + miR.220a + miR.29a + miR.518c + miR.338 | 0,76 | 0,023023695 |
| miR.609 + miR.520f + miR.26a + miR.376a* + miR.639 | 0,76 | 0,117375859 |
| miR.603 + miR.520f + miR.220a + miR.518c + miR.639 | 0,76 | 0,025887464 |
| miR.603 + miR.520f + miR.220a + miR.519b + miR.518c | 0,76 | 0,025767911 |
| miR.558 + miR.603 + miR.519b + miR.362 + miR.518c | 0,76 | 0,024961269 |
| miR.609 + miR.130a + miR.29a + miR.518c + miR.660 | 0,76 | 0,000273592 |
| miR.609 + miR.603 + miR.130a + miR.518c + miR.660 | 0,76 | 0,011725317 |
| miR.609 + miR.603 + miR.130a + miR.362 + miR.518c | 0,76 | 0,010515123 |
| miR.609 + miR.29a + miR.369.3p + miR.518c + miR.338 | 0,76 | 0,003907214 |
| miR.558 + miR.603 + miR.26a + miR.369.3p + miR.518c | 0,76 | 0,050279892 |
| miR.603 + miR.220a + miR.519b + miR.518c + miR.338 | 0,76 | 0,01514577 |
| miR.609 + miR.603 + miR.520f + miR.519b + miR.518c | 0,76 | 0,014157426 |
| miR.558 + miR.609 + miR.520f + miR.130a + miR.639 | 0,76 | 0,045951688 |
| miR.558 + miR.609 + miR.603 + miR.520f + miR.130a | 0,76 | 0,036822881 |
| miR.609 + miR.519b + miR.29a + miR.518c + miR.338 | 0,76 | 0,002082521 |
| miR.609 + miR.26a + miR.29a + miR.362 + miR.518c | 0,76 | 0,000765601 |
| miR.558 + miR.609 + miR.362 + miR.369.3p + miR.376a* | 0,76 | 0,108386454 |
| miR.603 + miR.220a + miR.519b + miR.29a + miR.518c | 0,76 | 0,012790104 |
| miR.609 + miR.603 + miR.362 + miR.369.3p + miR.518c | 0,76 | 0,015538273 |
| miR.609 + miR.603 + miR.130a + miR.494 + miR.518c | 0,76 | 0,011597837 |
| miR.558 + miR.603 + miR.519b + miR.518c + miR.639 | 0,76 | 0,023795778 |
| miR.603 + miR.220a + miR.26a + miR.29a + miR.518c | 0,76 | 0,018423813 |
| miR.558 + miR.603 + miR.519b + miR.26a + miR.518c | 0,76 | 0,040091082 |
| miR.609 + miR.520f + miR.130a + miR.362 + miR.376a* | 0,76 | 0,073024904 |
| miR.609 + miR.603 + miR.519b + miR.130a + miR.518c | 0,76 | 0,008962594 |
| miR.609 + miR.220a + miR.130a + miR.518c + miR.338 | 0,76 | 0,002205947 |
| miR.609 + miR.130a + miR.26a + miR.29a + miR.518c | 0,76 | 0,000957283 |
| miR.609 + miR.520f + miR.220a + miR.130a + miR.376a* | 0,76 | 0,063686016 |
| miR.603 + miR.520f + miR.220a + miR.29a + miR.518c | 0,76 | 0,020043897 |
| miR.609 + miR.603 + miR.520f + miR.362 + miR.518c | 0,76 | 0,014416878 |
| miR.558 + miR.609 + miR.130a + miR.362 + miR.376a* | 0,76 | 0,024321151 |
| miR.558 + miR.609 + miR.26a + miR.29a + miR.639 | 0,76 | 0,009247032 |
| miR.558 + miR.609 + miR.603 + miR.26a + miR.29a | 0,76 | 0,009942144 |
| miR.609 + miR.130a + miR.376a* + miR.518c + miR.639 | 0,76 | 0,009279881 |
| miR.603 + miR.220a + miR.519b + miR.369.3p + miR.518c | 0,76 | 0,019144995 |
| miR.558 + miR.520f + miR.494 + miR.518c + miR.639 | 0,76 | 0,028787601 |
| miR.558 + miR.609 + miR.26a + miR.29a + miR.494 | 0,76 | 0,00790317 |
| miR.609 + miR.603 + miR.520f + miR.518c + miR.338 | 0,76 | 0,015771629 |
| miR.609 + miR.130a + miR.29a + miR.494 + miR.518c | 0,76 | 0,001342942 |
| miR.558 + miR.609 + miR.520f + miR.376a* + miR.338 | 0,76 | 0,109265275 |
| miR.558 + miR.603 + miR.519b + miR.518c + miR.660 | 0,76 | 0,02783213 |
| miR.603 + miR.220a + miR.369.3p + miR.518c + miR.338 | 0,76 | 0,011651358 |
| miR.558 + miR.520f + miR.130a + miR.376a* + miR.518c | 0,76 | 0,035536194 |
| miR.558 + miR.520f + miR.220a + miR.518c + miR.639 | 0,76 | 0,018069663 |
| miR.558 + miR.520f + miR.519b + miR.518c + miR.639 | 0,76 | 0,06482366 |
| miR.558 + miR.603 + miR.130a + miR.26a + miR.518c | 0,76 | 0,016575711 |
| miR.603 + miR.220a + miR.519b + miR.26a + miR.518c | 0,76 | 0,015828338 |
| miR.609 + miR.29a + miR.376a* + miR.494 + miR.639 | 0,76 | 0,037368818 |
| miR.558 + miR.609 + miR.520f + miR.26a + miR.639 | 0,76 | 0,032760971 |
| miR.558 + miR.603 + miR.519b + miR.369.3p + miR.518c | 0,76 | 0,042299566 |
| miR.603 + miR.520f + miR.130a + miR.376a* + miR.518c | 0,76 | 0,021525819 |
| miR.609 + miR.26a + miR.29a + miR.376a* + miR.494 | 0,76 | 0,043344291 |
| miR.609 + miR.520f + miR.130a + miR.376a* + miR.494 | 0,76 | 0,057857469 |
| miR.558 + miR.603 + miR.518c + miR.639 + miR.338 | 0,75 | 0,012562831 |
| miR.609 + miR.603 + miR.130a + miR.29a + miR.376a* | 0,75 | 0,038169185 |
| miR.558 + miR.520f + miR.518c + miR.639 + miR.660 | 0,75 | 0,032273928 |
| miR.558 + miR.603 + miR.519b + miR.29a + miR.518c | 0,75 | 0,015180046 |
| miR.558 + miR.609 + miR.220a + miR.130a + miR.376a* | 0,75 | 0,02221586 |
| miR.558 + miR.609 + miR.26a + miR.29a + miR.338 | 0,75 | 0,008220027 |
| miR.609 + miR.603 + miR.520f + miR.518c + miR.660 | 0,75 | 0,010092484 |
| miR.609 + miR.369.3p + miR.376a* + miR.518c + miR.639 | 0,75 | 0,029475112 |
| miR.558 + miR.609 + miR.26a + miR.29a + miR.362 | 0,75 | 0,007678726 |
| miR.558 + miR.609 + miR.520f + miR.639 + miR.660 | 0,75 | 0,089040477 |
| miR.603 + miR.520f + miR.220a + miR.369.3p + miR.518c | 0,75 | 0,022342134 |
| miR.609 + miR.603 + miR.369.3p + miR.494 + miR.518c | 0,75 | 0,016807895 |
| miR.609 + miR.603 + miR.26a + miR.369.3p + miR.518c | 0,75 | 0,009087212 |
| miR.603 + miR.220a + miR.518c + miR.639 + miR.338 | 0,75 | 0,0099053 |
| miR.609 + miR.29a + miR.518c + miR.639 + miR.338 | 0,75 | 0,001599317 |
| miR.558 + miR.603 + miR.362 + miR.369.3p + miR.518c | 0,75 | 0,010086123 |
| miR.558 + miR.609 + miR.519b + miR.26a + miR.29a | 0,75 | 0,008859121 |
| miR.603 + miR.220a + miR.26a + miR.369.3p + miR.518c | 0,75 | 0,018703057 |
| miR.558 + miR.520f + miR.220a + miR.29a + miR.518c | 0,75 | 0,017183184 |
| miR.609 + miR.220a + miR.376a* + miR.494 + miR.518c | 0,75 | 0,00489172 |
| miR.558 + miR.609 + miR.130a + miR.26a + miR.29a | 0,75 | 0,004334831 |
| miR.558 + miR.520f + miR.26a + miR.376a* + miR.518c | 0,75 | 0,028539114 |
| miR.558 + miR.609 + miR.520f + miR.639 + miR.338 | 0,75 | 0,050919535 |
| miR.609 + miR.29a + miR.362 + miR.518c + miR.338 | 0,75 | 0,001525708 |
| miR.609 + miR.29a + miR.369.3p + miR.376a* + miR.639 | 0,75 | 0,07072185 |
| miR.558 + miR.603 + miR.519b + miR.518c + miR.338 | 0,75 | 0,02210537 |
| miR.558 + miR.603 + miR.519b + miR.130a + miR.518c | 0,75 | 0,024636374 |
| miR.609 + miR.130a + miR.29a + miR.518c + miR.338 | 0,75 | 0,001253716 |
| miR.609 + miR.603 + miR.26a + miR.29a + miR.376a* | 0,75 | 0,019969573 |
| miR.609 + miR.220a + miR.519b + miR.29a + miR.376a* | 0,75 | 0,018820628 |
| miR.609 + miR.520f + miR.130a + miR.376a* + miR.660 | 0,75 | 0,04349445 |
| miR.558 + miR.609 + miR.130a + miR.376a* + miR.639 | 0,75 | 0,051369927 |
| miR.609 + miR.220a + miR.29a + miR.362 + miR.376a* | 0,75 | 0,02102776 |
| miR.603 + miR.520f + miR.369.3p + miR.376a* + miR.518c | 0,75 | 0,031199248 |
| miR.603 + miR.220a + miR.130a + miR.26a + miR.518c | 0,75 | 0,018175075 |
| miR.558 + miR.220a + miR.29a + miR.376a* + miR.518c | 0,75 | 0,011886225 |
| miR.603 + miR.220a + miR.130a + miR.518c + miR.338 | 0,75 | 0,011863166 |
| miR.609 + miR.220a + miR.26a + miR.29a + miR.376a* | 0,75 | 0,010887199 |
| miR.603 + miR.520f + miR.220a + miR.130a + miR.518c | 0,75 | 0,018897988 |
| miR.603 + miR.220a + miR.519b + miR.494 + miR.518c | 0,75 | 0,01581099 |
| miR.603 + miR.520f + miR.220a + miR.518c + miR.338 | 0,75 | 0,021372759 |
| miR.558 + miR.603 + miR.130a + miR.518c + miR.639 | 0,75 | 0,014618027 |
| miR.609 + miR.603 + miR.29a + miR.362 + miR.376a* | 0,75 | 0,050058787 |
| miR.609 + miR.603 + miR.369.3p + miR.518c + miR.338 | 0,75 | 0,010014277 |
| miR.558 + miR.609 + miR.520f + miR.369.3p + miR.639 | 0,75 | 0,038186301 |
| miR.558 + miR.609 + miR.362 + miR.376a* + miR.639 | 0,75 | 0,047674346 |
| miR.609 + miR.29a + miR.376a* + miR.494 + miR.338 | 0,75 | 0,053116412 |
| miR.558 + miR.520f + miR.130a + miR.518c + miR.639 | 0,75 | 0,022328503 |
| miR.558 + miR.520f + miR.519b + miR.29a + miR.518c | 0,75 | 0,052640607 |
| miR.558 + miR.603 + miR.26a + miR.362 + miR.518c | 0,75 | 0,021593994 |
| miR.609 + miR.220a + miR.29a + miR.376a* + miR.338 | 0,75 | 0,019694695 |
| miR.558 + miR.603 + miR.369.3p + miR.518c + miR.639 | 0,75 | 0,01007484 |
| miR.603 + miR.220a + miR.26a + miR.518c + miR.639 | 0,75 | 0,014638688 |
| miR.558 + miR.520f + miR.26a + miR.518c + miR.639 | 0,75 | 0,029269796 |
| miR.609 + miR.603 + miR.519b + miR.29a + miR.376a* | 0,75 | 0,04581019 |
| miR.558 + miR.520f + miR.362 + miR.518c + miR.639 | 0,75 | 0,017951532 |
| miR.609 + miR.220a + miR.29a + miR.376a* + miR.639 | 0,75 | 0,01913617 |
| miR.558 + miR.220a + miR.369.3p + miR.376a* + miR.518c | 0,75 | 0,049161847 |
| miR.558 + miR.603 + miR.130a + miR.29a + miR.518c | 0,75 | 0,007216532 |
| miR.603 + miR.220a + miR.519b + miR.130a + miR.518c | 0,75 | 0,01262932 |
| miR.558 + miR.609 + miR.520f + miR.130a + miR.26a | 0,75 | 0,043520581 |
| miR.558 + miR.603 + miR.362 + miR.518c + miR.639 | 0,75 | 0,006232439 |
| miR.558 + miR.603 + miR.519b + miR.494 + miR.518c | 0,75 | 0,022492489 |
| miR.609 + miR.520f + miR.220a + miR.376a* + miR.518c | 0,75 | 0,00384492 |
| miR.609 + miR.220a + miR.130a + miR.29a + miR.376a* | 0,75 | 0,028916521 |
| miR.558 + miR.520f + miR.518c + miR.639 + miR.338 | 0,75 | 0,01885151 |
| miR.609 + miR.130a + miR.362 + miR.376a* + miR.518c | 0,75 | 0,003891989 |
| miR.558 + miR.609 + miR.520f + miR.494 + miR.639 | 0,75 | 0,048260686 |
| miR.603 + miR.220a + miR.519b + miR.518c + miR.639 | 0,75 | 0,014390889 |
| miR.558 + miR.609 + miR.520f + miR.362 + miR.639 | 0,75 | 0,035567697 |
| miR.558 + miR.609 + miR.520f + miR.130a + miR.660 | 0,75 | 0,06507655 |
| miR.609 + miR.520f + miR.220a + miR.369.3p + miR.518c | 0,75 | 0,005772128 |
| miR.609 + miR.603 + miR.29a + miR.376a* + miR.338 | 0,75 | 0,03681333 |
| miR.558 + miR.520f + miR.376a* + miR.518c + miR.660 | 0,75 | 0,046044877 |
| miR.609 + miR.603 + miR.519b + miR.369.3p + miR.518c | 0,75 | 0,009001864 |
| miR.609 + miR.29a + miR.369.3p + miR.376a* + miR.338 | 0,75 | 0,087747794 |
| miR.609 + miR.220a + miR.29a + miR.376a* + miR.660 | 0,75 | 0,019498297 |
| miR.609 + miR.29a + miR.376a* + miR.494 + miR.660 | 0,75 | 0,036531086 |
| miR.558 + miR.520f + miR.376a* + miR.518c + miR.338 | 0,75 | 0,098259776 |
| miR.609 + miR.130a + miR.29a + miR.369.3p + miR.518c | 0,75 | 0,001741294 |
| miR.558 + miR.520f + miR.369.3p + miR.518c + miR.639 | 0,75 | 0,019447124 |
| miR.609 + miR.520f + miR.26a + miR.376a* + miR.494 | 0,75 | 0,076045797 |
| miR.603 + miR.220a + miR.519b + miR.518c + miR.660 | 0,75 | 0,017343411 |
| miR.609 + miR.220a + miR.130a + miR.518c + miR.660 | 0,75 | 0,001581982 |
| miR.558 + miR.603 + miR.26a + miR.518c + miR.660 | 0,75 | 0,027131535 |
| miR.558 + miR.520f + miR.29a + miR.518c + miR.639 | 0,75 | 0,019640508 |
| miR.609 + miR.130a + miR.29a + miR.518c + miR.639 | 0,75 | 0,001080999 |
| miR.558 + miR.609 + miR.520f + miR.130a + miR.369.3p | 0,75 | 0,053459123 |
| miR.558 + miR.609 + miR.520f + miR.519b + miR.639 | 0,75 | 0,053286342 |
| miR.603 + miR.520f + miR.220a + miR.518c + miR.660 | 0,75 | 0,022335483 |
| miR.558 + miR.609 + miR.520f + miR.519b + miR.130a | 0,75 | 0,064845424 |
| miR.558 + miR.520f + miR.362 + miR.376a* + miR.518c | 0,75 | 0,045030008 |
| miR.558 + miR.609 + miR.520f + miR.220a + miR.639 | 0,75 | 0,041778052 |
| miR.609 + miR.29a + miR.362 + miR.369.3p + miR.518c | 0,75 | 0,002486241 |
| miR.609 + miR.603 + miR.29a + miR.376a* + miR.660 | 0,75 | 0,044235947 |
| miR.603 + miR.220a + miR.369.3p + miR.518c + miR.639 | 0,75 | 0,011980221 |
| miR.558 + miR.609 + miR.520f + miR.130a + miR.362 | 0,75 | 0,027438706 |
| miR.603 + miR.220a + miR.29a + miR.369.3p + miR.518c | 0,75 | 0,010519744 |
| miR.609 + miR.519b + miR.29a + miR.369.3p + miR.518c | 0,75 | 0,00214254 |
| miR.558 + miR.520f + miR.220a + miR.494 + miR.518c | 0,75 | 0,012772203 |
| miR.609 + miR.519b + miR.29a + miR.376a* + miR.639 | 0,75 | 0,015007279 |
| miR.609 + miR.603 + miR.369.3p + miR.518c + miR.660 | 0,75 | 0,00793125 |
| miR.558 + miR.603 + miR.494 + miR.518c + miR.639 | 0,75 | 0,005636393 |
| miR.603 + miR.520f + miR.26a + miR.376a* + miR.518c | 0,75 | 0,024421876 |
| miR.609 + miR.520f + miR.220a + miR.26a + miR.518c | 0,75 | 0,010530365 |
| miR.558 + miR.609 + miR.520f + miR.130a + miR.338 | 0,75 | 0,046884652 |
| miR.558 + miR.520f + miR.29a + miR.494 + miR.518c | 0,75 | 0,017713046 |
| miR.603 + miR.369.3p + miR.376a* + miR.518c + miR.660 | 0,75 | 0,030292073 |
| miR.603 + miR.520f + miR.220a + miR.494 + miR.518c | 0,75 | 0,020658815 |
| miR.558 + miR.520f + miR.369.3p + miR.376a* + miR.518c | 0,75 | 0,088750924 |
| miR.609 + miR.220a + miR.130a + miR.369.3p + miR.518c | 0,75 | 0,002238117 |
| miR.558 + miR.603 + miR.369.3p + miR.494 + miR.518c | 0,75 | 0,01247287 |
| miR.609 + miR.603 + miR.362 + miR.494 + miR.518c | 0,75 | 0,014175895 |
| miR.603 + miR.220a + miR.519b + miR.362 + miR.518c | 0,75 | 0,013147374 |
| miR.609 + miR.29a + miR.494 + miR.518c + miR.639 | 0,75 | 0,001660365 |
| miR.603 + miR.520f + miR.220a + miR.26a + miR.518c | 0,75 | 0,019222776 |
| miR.520f + miR.220a + miR.29a + miR.376a* + miR.518c | 0,75 | 0,019991243 |
| miR.558 + miR.603 + miR.26a + miR.518c + miR.338 | 0,75 | 0,023494384 |
| miR.558 + miR.603 + miR.29a + miR.518c + miR.338 | 0,75 | 0,006133672 |

**Table 6 clusters of 6miRNAs**

| **Cluster of 6 miRs** | **c_tau level** | **pv compared to top signature miR.558 + miR.609 + miR.220a + miR.29a + miR.376a* + miR.518c** |
|---|---|---|
| miR.558 + miR.609 + miR.220a + miR.29a + miR.376a* + miR.518c | 0,8 | NaN |
| miR.558 + miR.609 + miR.520f + miR.29a + miR.376a* + miR.518c | 0,8 | 0,425262874 |
| miR.558 + miR.609 + miR.603 + miR.29a + miR.376a* + miR.518c | 0,8 | 0,000198569 |
| miR.558 + miR.609 + miR.29a + miR.369.3p + miR.376a* + miR.518c | 0,8 | 0,192669487 |
| miR.558 + miR.609 + miR.26a + miR.29a + miR.376a* + miR.518c | 0,8 | 0,357087852 |
| miR.558 + miR.609 + miR.29a + miR.376a* + miR.518c + miR.639 | 0,79 | 0,185758947 |
| miR.609 + miR.603 + miR.220a + miR.29a + miR.376a* + miR.518c | 0,79 | 0,462031556 |
| miR.558 + miR.609 + miR.29a + miR.376a* + miR.518c + miR.660 | 0,79 | 0,100722966 |
| miR.558 + miR.609 + miR.29a + miR.376a* + miR.518c + miR.338 | 0,79 | 0,176804634 |
| miR.558 + miR.609 + miR.130a + miR.29a + miR.376a* + miR.518c | 0,79 | 0,132965668 |
| miR.558 + miR.609 + miR.29a + miR.362 + miR.376a* + miR.518c | 0,79 | 0,112793246 |
| miR.558 + miR.609 + miR.519b + miR.29a + miR.376a* + miR.518c | 0,79 | 0,131995208 |
| miR.558 + miR.609 + miR.29a + miR.376a* + miR.494 + miR.518c | 0,79 | 0,292038462 |
| miR.609 + miR.603 + miR.29a + miR.369.3p + miR.376a* + miR.518c | 0,79 | 0,495842065 |
| miR.609 + miR.603 + miR.520f + miR.29a + miR.376a* + miR.518c | 0,79 | 0,164096863 |
| miR.609 + miR.220a + miR.29a + miR.369.3p + miR.376a* + miR.518c | 0,79 | 0,109937592 |
| miR.609 + miR.520f + miR.220a + miR.29a + miR.376a* + miR.518c | 0,79 | 0,465866529 |
| miR.558 + miR.609 + miR.520f + miR.29a + miR.376a* + miR.338 | 0,79 | 0,24024849 |
| miR.609 + miR.520f + miR.29a + miR.376a* + miR.494 + miR.518c | 0,79 | 0,362228462 |
| miR.558 + miR.609 + miR.520f + miR.29a + miR.518c + miR.660 | 0,79 | 0,061409925 |
| miR.558 + miR.609 + miR.520f + miR.29a + miR.376a* + miR.494 | 0,79 | 0,098769582 |
| miR.609 + miR.603 + miR.29a + miR.376a* + miR.494 + miR.518c | 0,79 | 0,062376144 |
| miR.558 + miR.609 + miR.520f + miR.29a + miR.369.3p + miR.376a* | 0,79 | 0,260005036 |
| miR.609 + miR.520f + miR.29a + miR.369.3p + miR.376a* + miR.518c | 0,79 | 0,496052539 |
| miR.558 + miR.609 + miR.520f + miR.376a* + miR.518c + miR.639 | 0,79 | 0,267576326 |
| miR.609 + miR.603 + miR.29a + miR.376a* + miR.518c + miR.639 | 0,79 | 0,011245226 |
| miR.558 + miR.609 + miR.26a + miR.29a + miR.518c + miR.660 | 0,79 | 0,04047064 |
| miR.558 + miR.609 + miR.603 + miR.376a* + miR.518c + miR.639 | 0,79 | 0,103974742 |
| miR.609 + miR.520f + miR.29a + miR.376a* + miR.518c + miR.660 | 0,79 | 0,266209089 |
| miR.609 + miR.220a + miR.519b + miR.29a + miR.376a* + miR.518c | 0,79 | 0,454399121 |
| miR.609 + miR.220a + miR.29a + miR.376a* + miR.494 + miR.518c | 0,79 | 0,422773766 |
| miR.609 + miR.520f + miR.29a + miR.376a* + miR.518c + miR.338 | 0,79 | 0,488487745 |
| miR.609 + miR.520f + miR.519b + miR.29a + miR.376a* + miR.518c | 0,79 | 0,221080827 |
| miR.558 + miR.609 + miR.603 + miR.520f + miR.29a + miR.376a* | 0,79 | 0,086973376 |
| miR.558 + miR.609 + miR.520f + miR.29a + miR.376a* + miR.660 | 0,79 | 0,15681019 |
| miR.609 + miR.603 + miR.26a + miR.29a + miR.376a* + miR.518c | 0,79 | 0,007221034 |
| miR.558 + miR.609 + miR.520f + miR.130a + miR.376a* + miR.518c | 0,79 | 0,111580627 |
| miR.609 + miR.220a + miR.29a + miR.362 + miR.376a* + miR.518c | 0,79 | 0,465457785 |
| miR.609 + miR.603 + miR.29a + miR.362 + miR.376a* + miR.518c | 0,79 | 0,002216649 |
| miR.609 + miR.220a + miR.26a + miR.29a + miR.376a* + miR.518c | 0,79 | 0,488707209 |
| miR.609 + miR.603 + miR.29a + miR.376a* + miR.518c + miR.660 | 0,79 | 0,002116363 |
| miR.609 + miR.603 + miR.130a + miR.29a + miR.376a* + miR.518c | 0,79 | 0,004761298 |
| miR.609 + miR.520f + miR.29a + miR.362 + miR.376a* + miR.518c | 0,79 | 0,309625965 |
| miR.609 + miR.520f + miR.26a + miR.29a + miR.376a* + miR.518c | 0,79 | 0,361650132 |
| miR.609 + miR.520f + miR.130a + miR.29a + miR.376a* + miR.518c | 0,79 | 0,26767295 |
| miR.609 + miR.603 + miR.29a + miR.376a* + miR.518c + miR.338 | 0,79 | 0,009226262 |
| miR.558 + miR.609 + miR.520f + miR.29a + miR.376a* + miR.639 | 0,79 | 0,120239833 |
| miR.609 + miR.220a + miR.29a + miR.376a* + miR.518c + miR.338 | 0,79 | 0,39600383 |
| miR.609 + miR.603 + miR.519b + miR.29a + miR.376a* + miR.518c | 0,79 | 0,003518071 |
| miR.558 + miR.609 + miR.520f + miR.519b + miR.29a + miR.376a* | 0,79 | 0,131613028 |
| miR.609 + miR.220a + miR.29a + miR.376a* + miR.518c + miR.660 | 0,79 | 0,293581541 |
| miR.609 + miR.520f + miR.29a + miR.376a* + miR.518c + miR.639 | 0,79 | 0,277735408 |
| miR.609 + miR.220a + miR.29a + miR.376a* + miR.518c + miR.639 | 0,79 | 0,409188251 |
| miR.558 + miR.609 + miR.520f + miR.130a + miR.29a + miR.376a* | 0,79 | 0,123312922 |
| miR.609 + miR.603 + miR.220a + miR.130a + miR.376a* + miR.518c | 0,79 | 0,093728469 |
| miR.558 + miR.609 + miR.520f + miR.26a + miR.29a + miR.376a* | 0,79 | 0,105948702 |
| miR.558 + miR.609 + miR.520f + miR.220a + miR.29a + miR.376a* | 0,78 | 0,115738585 |
| miR.609 + miR.220a + miR.130a + miR.29a + miR.376a* + miR.518c | 0,78 | 0,479361199 |
| miR.558 + miR.609 + miR.520f + miR.29a + miR.362 + miR.376a* | 0,78 | 0,129581995 |
| miR.558 + miR.609 + miR.603 + miR.130a + miR.376a* + miR.518c | 0,78 | 0,027048087 |
| miR.558 + miR.609 + miR.603 + miR.26a + miR.29a + miR.518c | 0,78 | 0,017201072 |
| miR.558 + miR.609 + miR.369.3p + miR.376a* + miR.518c + miR.639 | 0,78 | 0,268171173 |
| miR.558 + miR.609 + miR.220a + miR.130a + miR.376a* + miR.518c | 0,78 | 0,071004976 |
| miR.558 + miR.609 + miR.603 + miR.220a + miR.376a* + miR.518c | 0,78 | 0,037830085 |
| miR.558 + miR.609 + miR.220a + miR.26a + miR.29a + miR.518c | 0,78 | 0,018586641 |
| miR.558 + miR.609 + miR.520f + miR.26a + miR.29a + miR.518c | 0,78 | 0,023669412 |
| miR.558 + miR.609 + miR.220a + miR.376a* + miR.518c + miR.639 | 0,78 | 0,146858934 |
| miR.609 + miR.603 + miR.520f + miR.130a + miR.376a* + miR.518c | 0,78 | 0,029015602 |
| miR.558 + miR.609 + miR.26a + miR.29a + miR.362 + miR.518c | 0,78 | 0,018300028 |
| miR.609 + miR.519b + miR.29a + miR.369.3p + miR.376a* + miR.518c | 0,78 | 0,424917376 |
| miR.558 + miR.609 + miR.26a + miR.29a + miR.369.3p + miR.518c | 0,78 | 0,022564996 |
| miR.558 + miR.609 + miR.603 + miR.369.3p + miR.376a* + miR.518c | 0,78 | 0,088380038 |
| miR.558 + miR.609 + miR.520f + miR.29a + miR.518c + miR.338 | 0,78 | 0,015998239 |
| miR.558 + miR.609 + miR.26a + miR.29a + miR.518c + miR.338 | 0,78 | 0,023785496 |
| miR.558 + miR.609 + miR.130a + miR.29a + miR.518c + miR.660 | 0,78 | 0,001551724 |
| miR.558 + miR.609 + miR.130a + miR.376a* + miR.518c + miR.639 | 0,78 | 0,135766224 |
| miR.558 + miR.609 + miR.520f + miR.519b + miR.29a + miR.518c | 0,78 | 0,035621536 |
| miR.558 + miR.609 + miR.220a + miR.29a + miR.518c + miR.660 | 0,78 | 0,00600659 |
| miR.609 + miR.520f + miR.29a + miR.376a* + miR.494 + miR.338 | 0,78 | 0,218054449 |
| miR.558 + miR.609 + miR.520f + miR.29a + miR.362 + miR.518c | 0,78 | 0,017324965 |
| miR.609 + miR.603 + miR.369.3p + miR.376a* + miR.518c + miR.639 | 0,78 | 0,052726214 |
| miR.558 + miR.609 + miR.520f + miR.130a + miR.29a + miR.518c | 0,78 | 0,015419299 |
| miR.558 + miR.609 + miR.520f + miR.130a + miR.376a* + miR.338 | 0,78 | 0,303295878 |
| miR.558 + miR.609 + miR.26a + miR.29a + miR.518c + miR.639 | 0,78 | 0,013352756 |
| miR.558 + miR.609 + miR.520f + miR.29a + miR.518c + miR.639 | 0,78 | 0,019870772 |
| miR.558 + miR.609 + miR.130a + miR.26a + miR.29a + miR.518c | 0,78 | 0,019211323 |
| miR.558 + miR.609 + miR.520f + miR.518c + miR.639 + miR.660 | 0,78 | 0,141871505 |
| miR.558 + miR.609 + miR.520f + miR.220a + miR.29a + miR.518c | 0,78 | 0,015020769 |
| miR.558 + miR.609 + miR.520f + miR.29a + miR.369.3p + miR.518c | 0,78 | 0,010237864 |
| miR.558 + miR.609 + miR.520f + miR.29a + miR.494 + miR.518c | 0,78 | 0,009486224 |
| miR.558 + miR.609 + miR.603 + miR.29a + miR.518c + miR.660 | 0,78 | 0,006453536 |
| miR.558 + miR.609 + miR.29a + miR.362 + miR.369.3p + miR.376a* | 0,78 | 0,120744206 |
| miR.558 + miR.609 + miR.26a + miR.29a + miR.494 + miR.518c | 0,78 | 0,014994735 |
| miR.558 + miR.609 + miR.603 + miR.520f + miR.29a + miR.518c | 0,78 | 0,021035633 |
| miR.558 + miR.609 + miR.519b + miR.26a + miR.29a + miR.518c | 0,78 | 0,018119309 |
| miR.609 + miR.603 + miR.220a + miR.376a* + miR.518c + miR.639 | 0,78 | 0,028081925 |
| miR.609 + miR.603 + miR.130a + miR.369.3p + miR.376a* + miR.518c | 0,78 | 0,023330265 |
| miR.609 + miR.603 + miR.520f + miR.376a* + miR.518c + miR.639 | 0,78 | 0,039819944 |
| miR.609 + miR.520f + miR.220a + miR.130a + miR.376a* + miR.518c | 0,78 | 0,040364078 |
| miR.558 + miR.609 + miR.130a + miR.369.3p + miR.376a* + miR.518c | 0,78 | 0,147174396 |
| miR.609 + miR.29a + miR.369.3p + miR.376a* + miR.494 + miR.518c | 0,78 | 0,212674547 |
| miR.609 + miR.603 + miR.520f + miR.29a + miR.376a* + miR.494 | 0,78 | 0,095803005 |
| miR.558 + miR.609 + miR.29a + miR.362 + miR.518c + miR.660 | 0,78 | 0,005039364 |
| miR.609 + miR.603 + miR.130a + miR.376a* + miR.518c + miR.639 | 0,78 | 0,018369545 |
| miR.609 + miR.519b + miR.29a + miR.376a* + miR.518c + miR.639 | 0,78 | 0,019397867 |
| miR.558 + miR.609 + miR.220a + miR.29a + miR.369.3p + miR.376a* | 0,78 | 0,140691113 |
| miR.558 + miR.609 + miR.220a + miR.369.3p + miR.376a* + miR.518c | 0,78 | 0,124067642 |
| miR.558 + miR.609 + miR.603 + miR.520f + miR.518c + miR.639 | 0,78 | 0,043027803 |
| miR.558 + miR.609 + miR.29a + miR.369.3p + miR.376a* + miR.494 | 0,78 | 0,166394208 |
| miR.609 + miR.520f + miR.29a + miR.369.3p + miR.376a* + miR.494 | 0,78 | 0,225078086 |
| miR.609 + miR.520f + miR.26a + miR.29a + miR.376a* + miR.494 | 0,78 | 0,126890301 |
| miR.558 + miR.609 + miR.26a + miR.29a + miR.369.3p + miR.376a* | 0,78 | 0,142788727 |
| miR.558 + miR.609 + miR.29a + miR.518c + miR.660 + miR.338 | 0,78 | 0,00250342 |
| miR.558 + miR.609 + miR.603 + miR.29a + miR.369.3p + miR.376a* | 0,78 | 0,126575709 |
| miR.558 + miR.609 + miR.362 + miR.376a* + miR.518c + miR.639 | 0,78 | 0,133604741 |
| miR.609 + miR.520f + miR.220a + miR.29a + miR.518c + miR.660 | 0,78 | 0,033902659 |
| miR.609 + miR.519b + miR.29a + miR.376a* + miR.494 + miR.518c | 0,78 | 0,057426252 |
| miR.558 + miR.609 + miR.376a* + miR.518c + miR.639 + miR.338 | 0,78 | 0,119882578 |
| miR.609 + miR.29a + miR.376a* + miR.494 + miR.518c + miR.639 | 0,78 | 0,064820004 |
| miR.558 + miR.609 + miR.376a* + miR.494 + miR.518c + miR.639 | 0,78 | 0,235901663 |
| miR.609 + miR.603 + miR.220a + miR.369.3p + miR.376a* + miR.518c | 0,78 | 0,046852084 |
| miR.609 + miR.519b + miR.26a + miR.29a + miR.376a* + miR.518c | 0,78 | 0,010708884 |
| miR.558 + miR.609 + miR.26a + miR.376a* + miR.518c + miR.639 | 0,78 | 0,126690944 |
| miR.558 + miR.609 + miR.220a + miR.29a + miR.518c + miR.338 | 0,78 | 0,006155848 |
| miR.609 + miR.29a + miR.362 + miR.369.3p + miR.376a* + miR.518c | 0,78 | 0,303261509 |
| miR.609 + miR.520f + miR.130a + miR.376a* + miR.518c + miR.639 | 0,78 | 0,066060212 |
| miR.558 + miR.609 + miR.519b + miR.376a* + miR.518c + miR.639 | 0,78 | 0,128912262 |
| miR.558 + miR.609 + miR.603 + miR.220a + miR.519b + miR.518c | 0,78 | 0,052360911 |
| miR.558 + miR.609 + miR.376a* + miR.518c + miR.639 + miR.660 | 0,78 | 0,119939336 |
| miR.609 + miR.520f + miR.29a + miR.494 + miR.518c + miR.660 | 0,78 | 0,036862504 |
| miR.558 + miR.609 + miR.603 + miR.29a + miR.518c + miR.338 | 0,78 | 0,007693048 |
| miR.609 + miR.29a + miR.369.3p + miR.376a* + miR.518c + miR.639 | 0,78 | 0,328525056 |
| miR.609 + miR.520f + miR.29a + miR.376a* + miR.494 + miR.660 | 0,78 | 0,159619532 |
| miR.558 + miR.609 + miR.603 + miR.220a + miR.29a + miR.518c | 0,78 | 0,01005745 |
| miR.558 + miR.609 + miR.603 + miR.520f + miR.376a* + miR.518c | 0,78 | 0,043056915 |
| miR.609 + miR.520f + miR.29a + miR.376a* + miR.494 + miR.639 | 0,78 | 0,092755061 |
| miR.609 + miR.520f + miR.220a + miR.29a + miR.376a* + miR.494 | 0,78 | 0,088959415 |
| miR.609 + miR.520f + miR.29a + miR.518c + miR.639 + miR.660 | 0,78 | 0,03311913 |
| miR.558 + miR.609 + miR.130a + miR.362 + miR.376a* + miR.518c | 0,78 | 0,047174907 |
| miR.558 + miR.609 + miR.29a + miR.518c + miR.639 + miR.660 | 0,78 | 0,007003397 |
| miR.558 + miR.609 + miR.130a + miR.376a* + miR.518c + miR.338 | 0,78 | 0,054960555 |
| miR.609 + miR.519b + miR.130a + miR.29a + miR.376a* + miR.518c | 0,78 | 0,018045409 |
| miR.558 + miR.609 + miR.29a + miR.369.3p + miR.376a* + miR.338 | 0,78 | 0,105915537 |
| miR.609 + miR.519b + miR.29a + miR.362 + miR.376a* + miR.518c | 0,78 | 0,008708266 |
| miR.609 + miR.519b + miR.29a + miR.376a* + miR.518c + miR.338 | 0,78 | 0,008592583 |
| miR.609 + miR.520f + miR.519b + miR.29a + miR.376a* + miR.494 | 0,78 | 0,07554015 |
| miR.558 + miR.609 + miR.520f + miR.26a + miR.376a* + miR.518c | 0,78 | 0,033047054 |
| miR.558 + miR.609 + miR.519b + miR.29a + miR.518c + miR.660 | 0,78 | 0,0033052 |
| miR.609 + miR.519b + miR.29a + miR.376a* + miR.518c + miR.660 | 0,78 | 0,009079165 |
| miR.558 + miR.609 + miR.130a + miR.29a + miR.518c + miR.338 | 0,78 | 0,002325394 |
| miR.609 + miR.29a + miR.369.3p + miR.376a* + miR.518c + miR.660 | 0,78 | 0,275695159 |
| miR.609 + miR.603 + miR.376a* + miR.494 + miR.518c + miR.639 | 0,78 | 0,03123954 |
| miR.558 + miR.609 + miR.220a + miR.130a + miR.29a + miR.518c | 0,78 | 0,001458956 |
| miR.558 + miR.609 + miR.130a + miR.26a + miR.376a* + miR.518c | 0,78 | 0,067089746 |
| miR.609 + miR.520f + miR.130a + miR.376a* + miR.518c + miR.338 | 0,78 | 0,115491461 |
| miR.558 + miR.609 + miR.29a + miR.369.3p + miR.376a* + miR.639 | 0,78 | 0,168741776 |
| miR.609 + miR.520f + miR.130a + miR.29a + miR.376a* + miR.338 | 0,78 | 0,265488968 |
| miR.558 + miR.609 + miR.29a + miR.369.3p + miR.518c + miR.660 | 0,78 | 0,006670492 |
| miR.609 + miR.520f + miR.29a + miR.369.3p + miR.376a* + miR.338 | 0,78 | 0,370072661 |
| miR.609 + miR.520f + miR.29a + miR.362 + miR.376a* + miR.494 | 0,78 | 0,08150245 |
| miR.609 + miR.520f + miR.130a + miR.29a + miR.376a* + miR.494 | 0,78 | 0,089734587 |
| miR.558 + miR.609 + miR.603 + miR.376a* + miR.518c + miR.338 | 0,78 | 0,041849811 |
| miR.609 + miR.26a + miR.29a + miR.369.3p + miR.376a* + miR.518c | 0,78 | 0,263624916 |
| miR.558 + miR.609 + miR.603 + miR.519b + miR.29a + miR.518c | 0,78 | 0,012130373 |
| miR.609 + miR.520f + miR.29a + miR.362 + miR.518c + miR.660 | 0,78 | 0,025516226 |
| miR.558 + miR.609 + miR.603 + miR.519b + miR.376a* + miR.518c | 0,78 | 0,03013586 |
| miR.558 + miR.609 + miR.520f + miR.362 + miR.518c + miR.639 | 0,78 | 0,042176469 |
| miR.558 + miR.609 + miR.520f + miR.130a + miR.518c + miR.639 | 0,78 | 0,028306951 |
| miR.558 + miR.609 + miR.26a + miR.29a + miR.376a* + miR.494 | 0,78 | 0,046957121 |
| miR.609 + miR.220a + miR.130a + miR.369.3p + miR.376a* + miR.518c | 0,78 | 0,044898898 |
| miR.558 + miR.609 + miR.603 + miR.376a* + miR.518c + miR.660 | 0,78 | 0,030903885 |
| miR.558 + miR.609 + miR.362 + miR.369.3p + miR.376a* + miR.518c | 0,78 | 0,112556837 |
| miR.558 + miR.609 + miR.520f + miR.518c + miR.639 + miR.338 | 0,78 | 0,039709829 |
| miR.558 + miR.609 + miR.29a + miR.494 + miR.518c + miR.660 | 0,78 | 0,003625737 |
| miR.609 + miR.603 + miR.520f + miR.29a + miR.518c + miR.660 | 0,78 | 0,019161415 |
| miR.558 + miR.609 + miR.603 + miR.362 + miR.376a* + miR.518c | 0,78 | 0,028918491 |
| miR.558 + miR.609 + miR.603 + miR.130a + miR.29a + miR.518c | 0,78 | 0,004039676 |
| miR.558 + miR.609 + miR.603 + miR.376a* + miR.494 + miR.518c | 0,78 | 0,075790989 |
| miR.558 + miR.609 + miR.520f + miR.220a + miR.518c + miR.639 | 0,78 | 0,041798464 |
| miR.558 + miR.609 + miR.130a + miR.376a* + miR.518c + miR.660 | 0,78 | 0,037992011 |
| miR.558 + miR.609 + miR.520f + miR.369.3p + miR.518c + miR.639 | 0,78 | 0,042386029 |
| miR.558 + miR.609 + miR.520f + miR.494 + miR.518c + miR.639 | 0,78 | 0,047663269 |
| miR.558 + miR.609 + miR.520f + miR.26a + miR.518c + miR.639 | 0,78 | 0,040311664 |
| miR.558 + miR.609 + miR.603 + miR.520f + miR.130a + miR.376a* | 0,78 | 0,108630608 |
| miR.558 + miR.609 + miR.130a + miR.29a + miR.369.3p + miR.376a* | 0,78 | 0,121675959 |
| miR.558 + miR.609 + miR.519b + miR.29a + miR.369.3p + miR.376a* | 0,78 | 0,121675959 |
| miR.558 + miR.609 + miR.520f + miR.519b + miR.518c + miR.639 | 0,78 | 0,06245737 |
| miR.558 + miR.609 + miR.29a + miR.369.3p + miR.376a* + miR.660 | 0,78 | 0,120491127 |
| miR.609 + miR.603 + miR.130a + miR.362 + miR.376a* + miR.518c | 0,78 | 0,007456785 |
| miR.609 + miR.520f + miR.130a + miR.29a + miR.518c + miR.660 | 0,78 | 0,017846749 |
| miR.558 + miR.609 + miR.603 + miR.29a + miR.376a* + miR.494 | 0,78 | 0,017722258 |
| miR.558 + miR.609 + miR.220a + miR.29a + miR.376a* + miR.494 | 0,78 | 0,033479475 |
| miR.609 + miR.26a + miR.29a + miR.376a* + miR.494 + miR.518c | 0,78 | 0,03916501 |
| miR.609 + miR.603 + miR.130a + miR.376a* + miR.518c + miR.338 | 0,78 | 0,0151384 |
| miR.609 + miR.130a + miR.29a + miR.369.3p + miR.376a* + miR.518c | 0,78 | 0,237823936 |
| miR.609 + miR.603 + miR.130a + miR.26a + miR.376a* + miR.518c | 0,78 | 0,006029358 |
| miR.558 + miR.609 + miR.603 + miR.26a + miR.518c + miR.639 | 0,78 | 0,046808887 |
| miR.609 + miR.520f + miR.29a + miR.369.3p + miR.518c + miR.660 | 0,78 | 0,016655018 |
| miR.558 + miR.609 + miR.520f + miR.130a + miR.369.3p + miR.376a* | 0,78 | 0,219283343 |
| miR.609 + miR.29a + miR.362 + miR.376a* + miR.494 + miR.518c | 0,78 | 0,086495985 |
| miR.558 + miR.609 + miR.220a + miR.26a + miR.29a + miR.376a* | 0,78 | 0,009727333 |
| miR.558 + miR.609 + miR.603 + miR.29a + miR.369.3p + miR.518c | 0,78 | 0,006324878 |
| miR.609 + miR.603 + miR.130a + miR.376a* + miR.518c + miR.660 | 0,78 | 0,004947727 |
| miR.558 + miR.609 + miR.603 + miR.520f + miR.519b + miR.518c | 0,78 | 0,03236939 |
| miR.558 + miR.609 + miR.130a + miR.29a + miR.518c + miR.639 | 0,78 | 0,001807018 |
| miR.558 + miR.603 + miR.220a + miR.519b + miR.376a* + miR.518c | 0,78 | 0,071846662 |
| miR.609 + miR.603 + miR.220a + miR.29a + miR.518c + miR.660 | 0,78 | 0,011602529 |
| miR.558 + miR.603 + miR.220a + miR.369.3p + miR.376a* + miR.518c | 0,78 | 0,08367822 |
| miR.609 + miR.29a + miR.369.3p + miR.376a* + miR.518c + miR.338 | 0,78 | 0,314466464 |
| miR.609 + miR.603 + miR.520f + miR.29a + miR.376a* + miR.338 | 0,78 | 0,256073965 |
| miR.609 + miR.603 + miR.520f + miR.29a + miR.369.3p + miR.376a* | 0,78 | 0,199149406 |
| miR.558 + miR.609 + miR.130a + miR.376a* + miR.494 + miR.518c | 0,78 | 0,044155188 |
| miR.609 + miR.520f + miR.29a + miR.518c + miR.660 + miR.338 | 0,78 | 0,015927423 |
| miR.558 + miR.609 + miR.603 + miR.26a + miR.376a* + miR.518c | 0,78 | 0,033092463 |
| miR.609 + miR.130a + miR.29a + miR.376a* + miR.494 + miR.518c | 0,78 | 0,038978082 |
| miR.558 + miR.609 + miR.130a + miR.29a + miR.376a* + miR.494 | 0,78 | 0,024533495 |
| miR.558 + miR.609 + miR.603 + miR.520f + miR.130a + miR.518c | 0,78 | 0,038232842 |
| miR.558 + miR.609 + miR.519b + miR.130a + miR.29a + miR.518c | 0,78 | 0,002342319 |
| miR.558 + miR.609 + miR.519b + miR.130a + miR.376a* + miR.518c | 0,78 | 0,056517414 |
| miR.609 + miR.520f + miR.26a + miR.29a + miR.518c + miR.660 | 0,78 | 0,018204943 |
| miR.609 + miR.520f + miR.26a + miR.29a + miR.376a* + miR.338 | 0,78 | 0,258680925 |
| miR.609 + miR.29a + miR.376a* + miR.494 + miR.518c + miR.660 | 0,77 | 0,024560439 |
| miR.609 + miR.520f + miR.519b + miR.29a + miR.518c + miR.660 | 0,77 | 0,020408091 |
| miR.609 + miR.520f + miR.26a + miR.376a* + miR.518c + miR.639 | 0,77 | 0,064748083 |
| miR.558 + miR.609 + miR.603 + miR.520f + miR.220a + miR.518c | 0,77 | 0,021787736 |
| miR.558 + miR.609 + miR.220a + miR.519b + miR.376a* + miR.518c | 0,77 | 0,0447292 |
| miR.558 + miR.609 + miR.603 + miR.220a + miR.518c + miR.639 | 0,77 | 0,019775213 |
| miR.558 + miR.609 + miR.520f + miR.220a + miR.376a* + miR.518c | 0,77 | 0,056528715 |
| miR.558 + miR.609 + miR.220a + miR.519b + miR.29a + miR.518c | 0,77 | 0,00822999 |
| miR.558 + miR.609 + miR.520f + miR.130a + miR.26a + miR.376a* | 0,77 | 0,095906364 |
| miR.609 + miR.29a + miR.376a* + miR.494 + miR.518c + miR.338 | 0,77 | 0,047215942 |
| miR.558 + miR.609 + miR.603 + miR.220a + miR.518c + miR.660 | 0,77 | 0,018447378 |
| miR.609 + miR.520f + miR.130a + miR.26a + miR.376a* + miR.518c | 0,77 | 0,03724997 |
| miR.558 + miR.609 + miR.130a + miR.29a + miR.369.3p + miR.518c | 0,77 | 0,002453207 |
| miR.558 + miR.609 + miR.603 + miR.29a + miR.362 + miR.518c | 0,77 | 0,004641534 |
| miR.609 + miR.520f + miR.26a + miR.29a + miR.369.3p + miR.376a* | 0,77 | 0,388929625 |
| miR.609 + miR.603 + miR.520f + miR.130a + miR.29a + miR.376a* | 0,77 | 0,117512572 |
| miR.558 + miR.609 + miR.603 + miR.29a + miR.518c + miR.639 | 0,77 | 0,005046545 |
| miR.558 + miR.609 + miR.520f + miR.376a* + miR.518c + miR.338 | 0,77 | 0,161048175 |
| miR.558 + miR.609 + miR.520f + miR.369.3p + miR.376a* + miR.518c | 0,77 | 0,151625914 |
| miR.609 + miR.520f + miR.519b + miR.29a + miR.376a* + miR.338 | 0,77 | 0,20458255 |
| miR.558 + miR.609 + miR.520f + miR.376a* + miR.639 + miR.338 | 0,77 | 0,250373701 |
| miR.558 + miR.609 + miR.220a + miR.376a* + miR.518c + miR.338 | 0,77 | 0,083435977 |
| miR.609 + miR.520f + miR.519b + miR.29a + miR.369.3p + miR.376a* | 0,77 | 0,195417176 |
| miR.558 + miR.609 + miR.520f + miR.130a + miR.376a* + miR.639 | 0,77 | 0,134936459 |
| miR.609 + miR.603 + miR.519b + miR.376a* + miR.518c + miR.639 | 0,77 | 0,008328199 |
| miR.558 + miR.609 + miR.603 + miR.220a + miR.26a + miR.518c | 0,77 | 0,025277755 |
| miR.558 + miR.609 + miR.603 + miR.520f + miR.369.3p + miR.518c | 0,77 | 0,031808939 |
| miR.558 + miR.609 + miR.29a + miR.362 + miR.376a* + miR.494 | 0,77 | 0,030604567 |
| miR.558 + miR.609 + miR.603 + miR.220a + miR.518c + miR.338 | 0,77 | 0,022354317 |
| miR.558 + miR.609 + miR.603 + miR.220a + miR.29a + miR.376a* | 0,77 | 0,016896958 |
| miR.609 + miR.520f + miR.29a + miR.376a* + miR.639 + miR.338 | 0,77 | 0,19620365 |
| miR.609 + miR.603 + miR.362 + miR.376a* + miR.518c + miR.639 | 0,77 | 0,007184524 |
| miR.609 + miR.603 + miR.376a* + miR.518c + miR.639 + miR.338 | 0,77 | 0,007340145 |
| miR.558 + miR.609 + miR.603 + miR.29a + miR.494 + miR.518c | 0,77 | 0,003513241 |
| miR.609 + miR.603 + miR.519b + miR.130a + miR.376a* + miR.518c | 0,77 | 0,004945048 |
| miR.558 + miR.609 + miR.603 + miR.220a + miR.369.3p + miR.518c | 0,77 | 0,026723847 |
| miR.609 + miR.520f + miR.519b + miR.130a + miR.376a* + miR.518c | 0,77 | 0,017190898 |
| miR.609 + miR.603 + miR.130a + miR.376a* + miR.494 + miR.518c | 0,77 | 0,005030959 |
| miR.609 + miR.520f + miR.130a + miR.369.3p + miR.376a* + miR.518c | 0,77 | 0,037841858 |
| miR.609 + miR.520f + miR.130a + miR.29a + miR.369.3p + miR.376a* | 0,77 | 0,229369867 |
| miR.609 + miR.520f + miR.519b + miR.376a* + miR.518c + miR.639 | 0,77 | 0,033069795 |
| miR.558 + miR.609 + miR.29a + miR.376a* + miR.494 + miR.639 | 0,77 | 0,017693081 |
| miR.558 + miR.609 + miR.29a + miR.369.3p + miR.518c + miR.338 | 0,77 | 0,009476879 |
| miR.609 + miR.603 + miR.376a* + miR.518c + miR.639 + miR.660 | 0,77 | 0,004435959 |
| miR.558 + miR.609 + miR.520f + miR.130a + miR.518c + miR.660 | 0,77 | 0,029765067 |
| miR.558 + miR.609 + miR.369.3p + miR.376a* + miR.518c + miR.338 | 0,77 | 0,148815903 |
| miR.609 + miR.603 + miR.26a + miR.376a* + miR.518c + miR.639 | 0,77 | 0,00424272 |
| miR.558 + miR.609 + miR.130a + miR.29a + miR.494 + miR.518c | 0,77 | 0,001226394 |
| miR.558 + miR.609 + miR.29a + miR.376a* + miR.494 + miR.338 | 0,77 | 0,031320284 |
| miR.558 + miR.609 + miR.220a + miR.29a + miR.362 + miR.518c | 0,77 | 0,004050479 |
| miR.558 + miR.609 + miR.603 + miR.130a + miR.26a + miR.518c | 0,77 | 0,02497583 |
| miR.609 + miR.520f + miR.220a + miR.29a + miR.369.3p + miR.376a* | 0,77 | 0,152820685 |
| miR.609 + miR.520f + miR.29a + miR.376a* + miR.660 + miR.338 | 0,77 | 0,368688621 |
| miR.609 + miR.603 + miR.520f + miR.26a + miR.29a + miR.376a* | 0,77 | 0,127440633 |
| miR.609 + miR.520f + miR.220a + miR.29a + miR.376a* + miR.338 | 0,77 | 0,167521517 |
| miR.558 + miR.609 + miR.520f + miR.220a + miR.130a + miR.518c | 0,77 | 0,023300975 |
| miR.558 + miR.609 + miR.220a + miR.29a + miR.376a* + miR.338 | 0,77 | 0,009309358 |
| miR.558 + miR.609 + miR.520f + miR.519b + miR.130a + miR.518c | 0,77 | 0,020395383 |
| miR.558 + miR.609 + miR.29a + miR.362 + miR.518c + miR.338 | 0,77 | 0,008204675 |
| miR.558 + miR.609 + miR.220a + miR.29a + miR.376a* + miR.639 | 0,77 | 0,012364405 |
| miR.558 + miR.609 + miR.220a + miR.29a + miR.362 + miR.376a* | 0,77 | 0,015122956 |
| miR.558 + miR.609 + miR.220a + miR.29a + miR.518c + miR.639 | 0,77 | 0,002237474 |
| miR.558 + miR.609 + miR.220a + miR.29a + miR.494 + miR.518c | 0,77 | 0,001803869 |
| miR.558 + miR.609 + miR.220a + miR.376a* + miR.494 + miR.518c | 0,77 | 0,075716129 |
| miR.558 + miR.609 + miR.603 + miR.29a + miR.376a* + miR.639 | 0,77 | 0,010701564 |
| miR.558 + miR.609 + miR.29a + miR.518c + miR.639 + miR.338 | 0,77 | 0,008310265 |
| miR.558 + miR.609 + miR.220a + miR.130a + miR.29a + miR.376a* | 0,77 | 0,01373442 |
| miR.558 + miR.609 + miR.130a + miR.29a + miR.362 + miR.518c | 0,77 | 0,001299145 |
| miR.558 + miR.609 + miR.220a + miR.26a + miR.376a* + miR.518c | 0,77 | 0,033073663 |
| miR.558 + miR.609 + miR.520f + miR.130a + miR.362 + miR.518c | 0,77 | 0,01566809 |
| miR.558 + miR.609 + miR.220a + miR.29a + miR.376a* + miR.660 | 0,77 | 0,013804712 |
| miR.558 + miR.609 + miR.519b + miR.29a + miR.518c + miR.338 | 0,77 | 0,013321564 |
| miR.609 + miR.29a + miR.376a* + miR.518c + miR.639 + miR.660 | 0,77 | 0,024120709 |
| miR.558 + miR.609 + miR.29a + miR.494 + miR.518c + miR.338 | 0,77 | 0,006527235 |
| miR.558 + miR.609 + miR.603 + miR.520f + miR.26a + miR.518c | 0,77 | 0,023003976 |
| miR.558 + miR.609 + miR.220a + miR.29a + miR.369.3p + miR.518c | 0,77 | 0,001975563 |
| miR.609 + miR.603 + miR.220a + miR.376a* + miR.494 + miR.518c | 0,77 | 0,047933012 |
| miR.558 + miR.609 + miR.603 + miR.220a + miR.130a + miR.518c | 0,77 | 0,0140883 |
| miR.558 + miR.609 + miR.603 + miR.520f + miR.518c + miR.660 | 0,77 | 0,012731247 |
| miR.558 + miR.609 + miR.26a + miR.29a + miR.376a* + miR.639 | 0,77 | 0,015937405 |
| miR.558 + miR.609 + miR.220a + miR.362 + miR.376a* + miR.518c | 0,77 | 0,035003582 |
| miR.609 + miR.220a + miR.29a + miR.518c + miR.660 + miR.338 | 0,77 | 0,00097626 |
| miR.609 + miR.603 + miR.520f + miR.130a + miR.376a* + miR.338 | 0,77 | 0,218924232 |
| miR.609 + miR.220a + miR.26a + miR.29a + miR.518c + miR.660 | 0,77 | 0,001753314 |
| miR.558 + miR.609 + miR.220a + miR.376a* + miR.518c + miR.660 | 0,77 | 0,038367824 |
| miR.558 + miR.609 + miR.520f + miR.130a + miR.26a + miR.518c | 0,77 | 0,014789949 |
| miR.558 + miR.609 + miR.220a + miR.519b + miR.29a + miR.376a* | 0,77 | 0,021990482 |
| miR.558 + miR.609 + miR.603 + miR.220a + miR.362 + miR.518c | 0,77 | 0,025129012 |
| miR.558 + miR.609 + miR.603 + miR.520f + miR.518c + miR.338 | 0,77 | 0,02258038 |
| miR.558 + miR.609 + miR.603 + miR.520f + miR.494 + miR.518c | 0,77 | 0,016561973 |
| miR.558 + miR.609 + miR.603 + miR.520f + miR.362 + miR.518c | 0,77 | 0,021028373 |
| miR.558 + miR.609 + miR.29a + miR.376a* + miR.494 + miR.660 | 0,77 | 0,021775453 |
| miR.609 + miR.220a + miR.130a + miR.26a + miR.376a* + miR.518c | 0,77 | 0,007569947 |
| miR.558 + miR.603 + miR.220a + miR.29a + miR.376a* + miR.518c | 0,77 | 0,031755674 |
| miR.558 + miR.609 + miR.520f + miR.130a + miR.369.3p + miR.518c | 0,77 | 0,014593479 |
| miR.558 + miR.609 + miR.603 + miR.518c + miR.639 + miR.338 | 0,77 | 0,016413963 |
| miR.558 + miR.609 + miR.603 + miR.220a + miR.494 + miR.518c | 0,77 | 0,019203352 |
| miR.609 + miR.520f + miR.26a + miR.29a + miR.376a* + miR.639 | 0,77 | 0,110639251 |
| miR.558 + miR.609 + miR.520f + miR.130a + miR.494 + miR.518c | 0,77 | 0,017097309 |
| miR.558 + miR.609 + miR.603 + miR.369.3p + miR.518c + miR.639 | 0,77 | 0,020788841 |
| miR.609 + miR.520f + miR.29a + miR.362 + miR.376a* + miR.338 | 0,77 | 0,245277466 |
| miR.609 + miR.603 + miR.29a + miR.369.3p + miR.376a* + miR.494 | 0,77 | 0,101266137 |
| miR.609 + miR.520f + miR.130a + miR.26a + miR.29a + miR.376a* | 0,77 | 0,11994057 |
| miR.609 + miR.220a + miR.519b + miR.130a + miR.376a* + miR.518c | 0,77 | 0,008059565 |
| miR.609 + miR.520f + miR.29a + miR.369.3p + miR.376a* + miR.639 | 0,77 | 0,17954231 |
| miR.558 + miR.609 + miR.519b + miR.29a + miR.376a* + miR.494 | 0,77 | 0,028918961 |
| miR.609 + miR.603 + miR.220a + miR.29a + miR.518c + miR.338 | 0,77 | 0,013917486 |
| miR.558 + miR.609 + miR.603 + miR.518c + miR.639 + miR.660 | 0,77 | 0,028832708 |
| miR.558 + miR.609 + miR.603 + miR.26a + miR.29a + miR.376a* | 0,77 | 0,005596967 |
| miR.558 + miR.603 + miR.220a + miR.376a* + miR.518c + miR.639 | 0,77 | 0,044013585 |
| miR.609 + miR.520f + miR.519b + miR.130a + miR.29a + miR.376a* | 0,77 | 0,110347939 |
| miR.609 + miR.130a + miR.29a + miR.376a* + miR.518c + miR.639 | 0,77 | 0,03202496 |
| miR.558 + miR.609 + miR.603 + miR.519b + miR.518c + miR.639 | 0,77 | 0,025874765 |
| miR.609 + miR.603 + miR.362 + miR.369.3p + miR.376a* + miR.518c | 0,77 | 0,018929539 |
| miR.558 + miR.609 + miR.519b + miR.26a + miR.29a + miR.376a* | 0,77 | 0,006899444 |
| miR.558 + miR.603 + miR.520f + miR.376a* + miR.518c + miR.639 | 0,77 | 0,050784236 |
| miR.609 + miR.26a + miR.29a + miR.376a* + miR.518c + miR.639 | 0,77 | 0,031111936 |
| miR.603 + miR.220a + miR.29a + miR.369.3p + miR.376a* + miR.518c | 0,77 | 0,071892876 |
| miR.603 + miR.220a + miR.369.3p + miR.376a* + miR.518c + miR.639 | 0,77 | 0,056473226 |
| miR.609 + miR.220a + miR.369.3p + miR.376a* + miR.518c + miR.639 | 0,77 | 0,015191152 |
| miR.558 + miR.609 + miR.220a + miR.26a + miR.518c + miR.639 | 0,77 | 0,055363873 |
| miR.609 + miR.29a + miR.362 + miR.376a* + miR.518c + miR.639 | 0,77 | 0,032246525 |
| miR.609 + miR.29a + miR.376a* + miR.518c + miR.639 + miR.338 | 0,77 | 0,033895696 |
| miR.558 + miR.609 + miR.26a + miR.29a + miR.376a* + miR.660 | 0,77 | 0,009362703 |
| miR.609 + miR.603 + miR.520f + miR.220a + miR.29a + miR.518c | 0,77 | 0,027071432 |
| miR.558 + miR.609 + miR.520f + miR.130a + miR.362 + miR.376a* | 0,77 | 0,108536286 |
| miR.609 + miR.520f + miR.220a + miR.130a + miR.29a + miR.376a* | 0,77 | 0,099971942 |
| miR.609 + miR.520f + miR.26a + miR.29a + miR.376a* + miR.660 | 0,77 | 0,166622163 |
| miR.558 + miR.609 + miR.520f + miR.130a + miR.518c + miR.338 | 0,77 | 0,017531482 |
| miR.558 + miR.609 + miR.603 + miR.494 + miR.518c + miR.639 | 0,77 | 0,014891641 |
| miR.558 + miR.609 + miR.29a + miR.376a* + miR.639 + miR.338 | 0,77 | 0,013546542 |
| miR.609 + miR.26a + miR.29a + miR.376a* + miR.518c + miR.660 | 0,77 | 0,013272168 |
| miR.558 + miR.609 + miR.520f + miR.26a + miR.376a* + miR.639 | 0,77 | 0,094588654 |
| miR.609 + miR.520f + miR.220a + miR.26a + miR.29a + miR.376a* | 0,77 | 0,137256623 |
| miR.558 + miR.603 + miR.520f + miR.220a + miR.376a* + miR.518c | 0,77 | 0,041411027 |
| miR.609 + miR.520f + miR.519b + miR.26a + miR.29a + miR.376a* | 0,77 | 0,12140393 |
| miR.558 + miR.609 + miR.220a + miR.130a + miR.26a + miR.518c | 0,77 | 0,016521337 |
| miR.609 + miR.520f + miR.130a + miR.362 + miR.376a* + miR.518c | 0,77 | 0,012298954 |
| miR.558 + miR.609 + miR.369.3p + miR.376a* + miR.518c + miR.660 | 0,77 | 0,084244914 |
| miR.558 + miR.609 + miR.520f + miR.130a + miR.376a* + miR.494 | 0,77 | 0,113821271 |
| miR.558 + miR.609 + miR.29a + miR.362 + miR.376a* + miR.639 | 0,77 | 0,01408851 |
| miR.558 + miR.609 + miR.520f + miR.220a + miR.519b + miR.518c | 0,77 | 0,044301384 |
| miR.609 + miR.520f + miR.519b + miR.29a + miR.376a* + miR.639 | 0,77 | 0,078616685 |
| miR.609 + miR.520f + miR.29a + miR.362 + miR.369.3p + miR.376a* | 0,77 | 0,127243263 |
| miR.558 + miR.603 + miR.220a + miR.130a + miR.376a* + miR.518c | 0,77 | 0,032649417 |
| miR.609 + miR.520f + miR.29a + miR.369.3p + miR.376a* + miR.660 | 0,77 | 0,234423912 |
| miR.558 + miR.609 + miR.520f + miR.519b + miR.130a + miR.376a* | 0,77 | 0,107754432 |
| miR.558 + miR.609 + miR.520f + miR.130a + miR.376a* + miR.660 | 0,77 | 0,105306429 |
| miR.558 + miR.609 + miR.603 + miR.130a + miR.518c + miR.639 | 0,77 | 0,018065119 |
| miR.609 + miR.603 + miR.520f + miR.29a + miR.376a* + miR.639 | 0,77 | 0,083710321 |
| miR.609 + miR.220a + miR.130a + miR.362 + miR.376a* + miR.518c | 0,77 | 0,009445869 |
| miR.609 + miR.520f + miR.130a + miR.376a* + miR.518c + miR.660 | 0,77 | 0,010483661 |
| miR.558 + miR.609 + miR.603 + miR.362 + miR.518c + miR.639 | 0,77 | 0,018899261 |
| miR.609 + miR.520f + miR.130a + miR.376a* + miR.494 + miR.518c | 0,77 | 0,010472044 |
| miR.609 + miR.220a + miR.130a + miR.376a* + miR.518c + miR.639 | 0,77 | 0,00817285 |
| miR.558 + miR.609 + miR.130a + miR.29a + miR.376a* + miR.639 | 0,77 | 0,011349157 |
| miR.609 + miR.603 + miR.220a + miR.26a + miR.29a + miR.518c | 0,77 | 0,02045932 |
| miR.558 + miR.603 + miR.520f + miR.220a + miR.519b + miR.518c | 0,77 | 0,067945261 |
| miR.609 + miR.520f + miR.130a + miR.29a + miR.376a* + miR.660 | 0,77 | 0,090321044 |
| miR.609 + miR.603 + miR.520f + miR.29a + miR.518c + miR.338 | 0,77 | 0,01144112 |
| miR.609 + miR.603 + miR.520f + miR.220a + miR.29a + miR.376a* | 0,77 | 0,104546742 |
| miR.558 + miR.609 + miR.26a + miR.29a + miR.362 + miR.376a* | 0,77 | 0,00565928 |
| miR.609 + miR.520f + miR.220a + miR.376a* + miR.518c + miR.639 | 0,77 | 0,025482588 |
| miR.609 + miR.29a + miR.362 + miR.376a* + miR.518c + miR.660 | 0,77 | 0,028162606 |
| miR.558 + miR.609 + miR.520f + miR.220a + miR.130a + miR.376a* | 0,77 | 0,121940052 |
| miR.558 + miR.609 + miR.520f + miR.29a + miR.362 + miR.660 | 0,77 | 0,071950411 |
| miR.558 + miR.609 + miR.603 + miR.26a + miR.369.3p + miR.518c | 0,77 | 0,042242342 |
| miR.609 + miR.603 + miR.220a + miR.519b + miR.376a* + miR.518c | 0,77 | 0,00831626 |
| miR.609 + miR.520f + miR.130a + miR.29a + miR.376a* + miR.639 | 0,77 | 0,084943006 |
| miR.558 + miR.609 + miR.130a + miR.26a + miR.29a + miR.376a* | 0,77 | 0,010687046 |
| miR.609 + miR.603 + miR.520f + miR.29a + miR.376a* + miR.660 | 0,77 | 0,089051081 |
| miR.609 + miR.520f + miR.26a + miR.29a + miR.362 + miR.376a* | 0,77 | 0,125842842 |
| miR.558 + miR.609 + miR.26a + miR.29a + miR.376a* + miR.338 | 0,77 | 0,010653659 |
| miR.609 + miR.220a + miR.29a + miR.362 + miR.518c + miR.660 | 0,77 | 0,003228105 |
| miR.558 + miR.609 + miR.26a + miR.369.3p + miR.518c + miR.639 | 0,77 | 0,084756756 |
| miR.609 + miR.520f + miR.220a + miR.29a + miR.362 + miR.518c | 0,77 | 0,006958009 |
| miR.609 + miR.29a + miR.362 + miR.376a* + miR.518c + miR.338 | 0,77 | 0,011074393 |
| miR.609 + miR.603 + miR.520f + miR.29a + miR.518c + miR.639 | 0,77 | 0,017446179 |
| miR.558 + miR.609 + miR.520f + miR.29a + miR.639 + miR.660 | 0,77 | 0,064805339 |
| miR.558 + miR.609 + miR.519b + miR.29a + miR.376a* + miR.639 | 0,77 | 0,008827513 |
| miR.558 + miR.609 + miR.29a + miR.376a* + miR.639 + miR.660 | 0,77 | 0,009735835 |
| miR.609 + miR.220a + miR.130a + miR.376a* + miR.494 + miR.518c | 0,77 | 0,008170883 |
| miR.609 + miR.520f + miR.220a + miR.29a + miR.518c + miR.338 | 0,77 | 0,008962688 |
| miR.558 + miR.609 + miR.603 + miR.29a + miR.376a* + miR.660 | 0,77 | 0,002801981 |
| miR.609 + miR.29a + miR.376a* + miR.518c + miR.660 + miR.338 | 0,77 | 0,011595155 |
| miR.609 + miR.26a + miR.29a + miR.362 + miR.376a* + miR.518c | 0,77 | 0,011813893 |
| miR.609 + miR.220a + miR.130a + miR.376a* + miR.518c + miR.660 | 0,77 | 0,0051046 |
| miR.603 + miR.220a + miR.130a + miR.369.3p + miR.376a* + miR.518c | 0,77 | 0,07082274 |
| miR.609 + miR.26a + miR.29a + miR.376a* + miR.518c + miR.338 | 0,77 | 0,011217691 |
| miR.558 + miR.609 + miR.603 + miR.519b + miR.369.3p + miR.518c | 0,77 | 0,024556776 |
| miR.558 + miR.609 + miR.362 + miR.518c + miR.639 + miR.660 | 0,77 | 0,068240133 |
| miR.558 + miR.609 + miR.603 + miR.130a + miR.518c + miR.338 | 0,77 | 0,014304072 |
| miR.558 + miR.609 + miR.603 + miR.130a + miR.29a + miR.376a* | 0,77 | 0,004576634 |
| miR.609 + miR.520f + miR.130a + miR.29a + miR.362 + miR.376a* | 0,77 | 0,093750787 |
| miR.558 + miR.609 + miR.603 + miR.519b + miR.130a + miR.518c | 0,77 | 0,023536179 |
| miR.609 + miR.130a + miR.26a + miR.29a + miR.376a* + miR.518c | 0,77 | 0,021357078 |
| miR.609 + miR.603 + miR.26a + miR.29a + miR.518c + miR.660 | 0,77 | 0,002390266 |
| miR.609 + miR.130a + miR.29a + miR.362 + miR.376a* + miR.518c | 0,77 | 0,033894424 |
| miR.609 + miR.603 + miR.520f + miR.29a + miR.362 + miR.518c | 0,77 | 0,015426528 |
| miR.609 + miR.520f + miR.519b + miR.29a + miR.376a* + miR.660 | 0,77 | 0,098260698 |
| miR.609 + miR.603 + miR.520f + miR.519b + miR.29a + miR.376a* | 0,77 | 0,094345939 |
| miR.558 + miR.609 + miR.603 + miR.130a + miR.369.3p + miR.518c | 0,77 | 0,018185215 |
| miR.609 + miR.603 + miR.520f + miR.220a + miR.376a* + miR.518c | 0,77 | 0,018022844 |
| miR.609 + miR.603 + miR.520f + miR.519b + miR.29a + miR.518c | 0,77 | 0,016525501 |
| miR.558 + miR.603 + miR.220a + miR.519b + miR.26a + miR.518c | 0,77 | 0,045168349 |
| miR.558 + miR.609 + miR.520f + miR.220a + miR.518c + miR.660 | 0,77 | 0,010454011 |
| miR.558 + miR.609 + miR.130a + miR.26a + miR.518c + miR.639 | 0,77 | 0,04326088 |
| miR.558 + miR.609 + miR.603 + miR.29a + miR.362 + miR.376a* | 0,77 | 0,002518751 |
| miR.558 + miR.609 + miR.520f + miR.220a + miR.369.3p + miR.518c | 0,77 | 0,030428094 |
| miR.609 + miR.603 + miR.220a + miR.26a + miR.376a* + miR.518c | 0,77 | 0,011199454 |
| miR.558 + miR.609 + miR.603 + miR.29a + miR.376a* + miR.338 | 0,77 | 0,002040468 |
| miR.609 + miR.130a + miR.29a + miR.376a* + miR.518c + miR.338 | 0,77 | 0,017779127 |
| miR.558 + miR.609 + miR.520f + miR.376a* + miR.518c + miR.660 | 0,77 | 0,047455935 |
| miR.558 + miR.609 + miR.520f + miR.376a* + miR.494 + miR.518c | 0,77 | 0,07847662 |
| miR.609 + miR.603 + miR.520f + miR.26a + miR.376a* + miR.518c | 0,77 | 0,00854435 |
| miR.609 + miR.603 + miR.220a + miR.519b + miR.29a + miR.518c | 0,77 | 0,010617572 |
| miR.558 + miR.609 + miR.520f + miR.362 + miR.376a* + miR.518c | 0,77 | 0,053788243 |
| miR.609 + miR.603 + miR.220a + miR.376a* + miR.518c + miR.338 | 0,77 | 0,013059379 |
| miR.609 + miR.603 + miR.520f + miR.26a + miR.29a + miR.518c | 0,77 | 0,016119849 |
| miR.558 + miR.609 + miR.603 + miR.362 + miR.369.3p + miR.518c | 0,77 | 0,015781877 |
| miR.609 + miR.603 + miR.29a + miR.362 + miR.369.3p + miR.376a* | 0,77 | 0,079825069 |
| miR.558 + miR.609 + miR.519b + miR.29a + miR.369.3p + miR.518c | 0,77 | 0,003692251 |
| miR.609 + miR.603 + miR.369.3p + miR.376a* + miR.518c + miR.338 | 0,77 | 0,014175499 |
| miR.558 + miR.609 + miR.520f + miR.519b + miR.26a + miR.518c | 0,77 | 0,022487851 |
| miR.609 + miR.520f + miR.220a + miR.29a + miR.376a* + miR.660 | 0,77 | 0,093807333 |
| miR.558 + miR.609 + miR.26a + miR.369.3p + miR.376a* + miR.518c | 0,77 | 0,098169323 |
| miR.609 + miR.220a + miR.130a + miR.376a* + miR.518c + miR.338 | 0,77 | 0,007775701 |
| miR.609 + miR.520f + miR.369.3p + miR.376a* + miR.518c + miR.639 | 0,77 | 0,075449122 |
| miR.558 + miR.603 + miR.520f + miR.29a + miR.376a* + miR.518c | 0,77 | 0,01426733 |
| miR.558 + miR.609 + miR.29a + miR.369.3p + miR.518c + miR.639 | 0,77 | 0,002316902 |
| miR.609 + miR.603 + miR.520f + miR.518c + miR.639 + miR.660 | 0,77 | 0,037719953 |
| miR.558 + miR.609 + miR.603 + miR.130a + miR.518c + miR.660 | 0,77 | 0,024449524 |
| miR.558 + miR.609 + miR.29a + miR.369.3p + miR.494 + miR.518c | 0,77 | 0,0018993 |
| miR.558 + miR.609 + miR.603 + miR.369.3p + miR.518c + miR.338 | 0,77 | 0,021836721 |
| miR.558 + miR.609 + miR.29a + miR.362 + miR.369.3p + miR.518c | 0,77 | 0,002372279 |
| miR.558 + miR.609 + miR.520f + miR.519b + miR.376a* + miR.518c | 0,77 | 0,051807111 |
| miR.609 + miR.520f + miR.376a* + miR.494 + miR.518c + miR.639 | 0,77 | 0,043382601 |
| miR.609 + miR.603 + miR.220a + miR.376a* + miR.518c + miR.660 | 0,77 | 0,010569123 |
| miR.558 + miR.609 + miR.519b + miR.369.3p + miR.376a* + miR.518c | 0,77 | 0,12723108 |
| miR.558 + miR.609 + miR.520f + miR.29a + miR.494 + miR.660 | 0,77 | 0,052610624 |
| miR.558 + miR.609 + miR.220a + miR.518c + miR.639 + miR.660 | 0,77 | 0,023773521 |
| miR.558 + miR.603 + miR.220a + miR.519b + miR.518c + miR.338 | 0,77 | 0,04050716 |
| miR.609 + miR.603 + miR.220a + miR.362 + miR.376a* + miR.518c | 0,77 | 0,008313058 |
| miR.609 + miR.603 + miR.520f + miR.29a + miR.369.3p + miR.518c | 0,77 | 0,013175473 |
| miR.609 + miR.520f + miR.376a* + miR.518c + miR.639 + miR.338 | 0,77 | 0,060908541 |
| miR.558 + miR.609 + miR.130a + miR.29a + miR.362 + miR.376a* | 0,77 | 0,0053212 |
| miR.609 + miR.603 + miR.520f + miR.130a + miR.29a + miR.518c | 0,77 | 0,012872201 |
| miR.609 + miR.520f + miR.220a + miR.519b + miR.29a + miR.376a* | 0,77 | 0,078542643 |
| miR.558 + miR.609 + miR.603 + miR.520f + miR.29a + miR.660 | 0,77 | 0,047264387 |
| miR.609 + miR.603 + miR.520f + miR.29a + miR.494 + miR.518c | 0,77 | 0,017700054 |
| miR.609 + miR.520f + miR.220a + miR.519b + miR.29a + miR.518c | 0,77 | 0,007178292 |
| miR.558 + miR.609 + miR.603 + miR.369.3p + miR.518c + miR.660 | 0,77 | 0,030997545 |
| miR.609 + miR.130a + miR.29a + miR.376a* + miR.518c + miR.660 | 0,77 | 0,019251846 |
| miR.609 + miR.520f + miR.220a + miR.26a + miR.29a + miR.518c | 0,77 | 0,008605824 |
| miR.558 + miR.603 + miR.220a + miR.376a* + miR.518c + miR.660 | 0,77 | 0,029523797 |
| miR.609 + miR.603 + miR.520f + miR.29a + miR.362 + miR.376a* | 0,77 | 0,095268033 |
| miR.558 + miR.609 + miR.603 + miR.519b + miR.29a + miR.376a* | 0,77 | 0,001813366 |
| miR.558 + miR.609 + miR.520f + miR.220a + miR.362 + miR.518c | 0,77 | 0,019808849 |
| miR.558 + miR.609 + miR.369.3p + miR.376a* + miR.494 + miR.518c | 0,77 | 0,13727794 |
| miR.609 + miR.520f + miR.130a + miR.369.3p + miR.376a* + miR.338 | 0,77 | 0,136659491 |
| miR.558 + miR.609 + miR.603 + miR.519b + miR.362 + miR.518c | 0,77 | 0,020886818 |
| miR.558 + miR.609 + miR.29a + miR.362 + miR.376a* + miR.660 | 0,77 | 0,019395961 |
| miR.558 + miR.609 + miR.603 + miR.519b + miR.494 + miR.518c | 0,77 | 0,024375379 |
| miR.558 + miR.603 + miR.220a + miR.26a + miR.376a* + miR.518c | 0,77 | 0,038369815 |
| miR.609 + miR.520f + miR.220a + miR.29a + miR.518c + miR.639 | 0,77 | 0,004671713 |
| miR.558 + miR.603 + miR.220a + miR.376a* + miR.494 + miR.518c | 0,77 | 0,021836525 |
| miR.558 + miR.609 + miR.520f + miR.220a + miR.518c + miR.338 | 0,77 | 0,019232048 |
| miR.609 + miR.520f + miR.220a + miR.29a + miR.494 + miR.518c | 0,77 | 0,006824784 |
| miR.558 + miR.609 + miR.519b + miR.29a + miR.362 + miR.518c | 0,77 | 0,003863341 |
| miR.603 + miR.220a + miR.519b + miR.29a + miR.376a* + miR.518c | 0,77 | 0,039032708 |
| miR.558 + miR.603 + miR.220a + miR.376a* + miR.518c + miR.338 | 0,77 | 0,02199773 |
| miR.558 + miR.609 + miR.520f + miR.220a + miR.494 + miR.518c | 0,77 | 0,017215728 |
| miR.558 + miR.609 + miR.519b + miR.29a + miR.494 + miR.518c | 0,77 | 0,003871226 |
| miR.603 + miR.220a + miR.362 + miR.369.3p + miR.376a* + miR.518c | 0,77 | 0,066129138 |
| miR.558 + miR.609 + miR.520f + miR.220a + miR.26a + miR.518c | 0,77 | 0,018026819 |
| miR.609 + miR.520f + miR.29a + miR.362 + miR.518c + miR.639 | 0,77 | 0,008417738 |
| miR.558 + miR.609 + miR.29a + miR.362 + miR.494 + miR.518c | 0,77 | 0,001691111 |
| miR.558 + miR.609 + miR.29a + miR.362 + miR.518c + miR.639 | 0,77 | 0,002216628 |
| miR.558 + miR.609 + miR.603 + miR.519b + miR.518c + miR.338 | 0,77 | 0,018574053 |

**Table 7 clusters of 7 miRNAs**

| **Cluster of 7 miRs** | **c_tau level** | **pv compared to top signature miR.558 + miR.609 + miR.220aa + miR.29a + miR.369.3p + miR.376a*** **+ miR.518c** |
|---|---|---|
| miR.558 + miR.609 + miR.220aa + miR.29a + miR.369.3p + miR.376a* + miR.518c | 0,8 | NaN |
| miR.558 + miR.609 + miR.603 + miR.220aa + miR.29a + miR.376a* + miR.518c | 0,8 | 0,025002564 |
| miR.558 + miR.609 + miR.520f + miR.220aa + miR.29a + miR.376a* + miR.518c | 0,8 | 0,236514028 |
| miR.558 + miR.609 + miR.520f + miR.29a + miR.376a* + miR.518c + miR.660 | 0,8 | 0,234767626 |
| miR.558 + miR.609 + miR.520f + miR.29a + miR.369.3p + miR.376a* + miR.518c | 0,8 | 0,368604402 |
| miR.558 + miR.609 + miR.603 + miR.29a + miR.369.3p + miR.376a* + miR.518c | 0,8 | 4,66E-005 |
| miR.558 + miR.609 + miR.220aa + miR.26a + miR.29a + miR.376a* + miR.518c | 0,8 | 0,400443165 |
| miR.558 + miR.609 + miR.220aa + miR.29a + miR.362 + miR.376a* + miR.518c | 0,8 | 0,063808643 |
| miR.558 + miR.609 + miR.603 + miR.520f + miR.29a + miR.376a* + miR.518c | 0,8 | 0,061426174 |
| miR.558 + miR.609 + miR.220aa + miR.29a + miR.376a* + miR.494 + miR.518c | 0,8 | 0,009429917 |
| miR.558 + miR.609 + miR.520f + miR.29a + miR.376a* + miR.518c + miR.338 | 0,8 | 0,451228907 |
| miR.558 + miR.609 + miR.220aa + miR.29a + miR.376a* + miR.518c + miR.660 | 0,8 | 0,052632921 |
| miR.558 + miR.609 + miR.220aa + miR.130a + miR.29a + miR.376a* + miR.518c | 0,8 | 0,023756083 |
| miR.558 + miR.609 + miR.520f + miR.29a + miR.362 + miR.376a* + miR.518c | 0,8 | 0,362771469 |
| miR.558 + miR.609 + miR.520f + miR.29a + miR.376a* + miR.494 + miR.518c | 0,8 | 0,254988519 |
| miR.558 + miR.609 + miR.220aa + miR.519b + miR.29a + miR.376a* + miR.518c | 0,8 | 0,101848935 |
| miR.558 + miR.609 + miR.520f + miR.29a + miR.376a* + miR.518c + miR.639 | 0,8 | 0,173022543 |
| miR.558 + miR.609 + miR.220aa + miR.29a + miR.376a* + miR.518c + miR.338 | 0,8 | 0,022009342 |
| miR.558 + miR.609 + miR.220aa + miR.29a + miR.376a* + miR.518c + miR.639 | 0,8 | 0,014190264 |
| miR.558 + miR.609 + miR.26a + miR.29a + miR.369.3p + miR.376a* + miR.518c | 0,8 | 0,487971958 |
| miR.558 + miR.609 + miR.520f + miR.26a + miR.29a + miR.376a* + miR.518c | 0,8 | 0,263651262 |
| miR.558 + miR.609 + miR.520f + miR.130a + miR.29a + miR.376a* + miR.518c | 0,8 | 0,21029374 |
| miR.558 + miR.609 + miR.520f + miR.519b + miR.29a + miR.376a* + miR.518c | 0,8 | 0,262148524 |
| miR.609 + miR.603 + miR.220aa + miR.29a + miR.369.3p + miR.376a* + miR.518c | 0,8 | 0,287440685 |
| miR.558 + miR.609 + miR.603 + miR.26a + miR.29a + miR.376a* + miR.518c | 0,8 | 0,002876115 |
| miR.558 + miR.609 + miR.603 + miR.29a + miR.376a* + miR.518c + miR.639 | 0,8 | 0,001233164 |
| miR.558 + miR.609 + miR.26a + miR.29a + miR.376a* + miR.518c + miR.639 | 0,8 | 0,275744525 |
| miR.558 + miR.609 + miR.130a + miR.29a + miR.369.3p + miR.376a* + miR.518c | 0,8 | 0,255194458 |
| miR.558 + miR.609 + miR.29a + miR.369.3p + miR.376a* + miR.518c + miR.660 | 0,8 | 0,13651033 |
| miR.558 + miR.609 + miR.603 + miR.29a + miR.362 + miR.376a* + miR.518c | 0,8 | 0,000337044 |
| miR.558 + miR.609 + miR.603 + miR.29a + miR.376a* + miR.518c + miR.660 | 0,8 | 0,00089689 |
| miR.558 + miR.609 + miR.603 + miR.29a + miR.376a* + miR.518c + miR.338 | 0,8 | 0,000809225 |
| miR.558 + miR.609 + miR.603 + miR.519b + miR.29a + miR.376a* + miR.518c | 0,8 | 0,001161154 |
| miR.558 + miR.609 + miR.29a + miR.369.3p + miR.376a* + miR.518c + miR.639 | 0,8 | 0,171792923 |
| miR.558 + miR.609 + miR.603 + miR.130a + miR.29a + miR.376a* + miR.518c | 0,8 | 0,000360769 |
| miR.558 + miR.609 + miR.29a + miR.362 + miR.369.3p + miR.376a* + miR.518c | 0,8 | 0,24498342 |
| miR.558 + miR.609 + miR.29a + miR.369.3p + miR.376a* + miR.494 + miR.518c | 0,8 | 0,160522317 |
| miR.558 + miR.609 + miR.29a + miR.369.3p + miR.376a* + miR.518c + miR.338 | 0,8 | 0,3404612 |
| miR.558 + miR.609 + miR.603 + miR.29a + miR.376a* + miR.494 + miR.518c | 0,8 | 0,005187074 |
| miR.609 + miR.603 + miR.520f + miR.220aa + miR.29a + miR.376a* + miR.518c | 0,8 | 0,322541602 |
| miR.558 + miR.609 + miR.26a + miR.29a + miR.376a* + miR.494 + miR.518c | 0,8 | 0,156342193 |
| miR.558 + miR.609 + miR.519b + miR.29a + miR.369.3p + miR.376a* + miR.518c | 0,8 | 0,110020017 |
| miR.558 + miR.609 + miR.26a + miR.29a + miR.376a* + miR.518c + miR.338 | 0,8 | 0,261803986 |
| miR.609 + miR.603 + miR.220aa + miR.29a + miR.362 + miR.376a* + miR.518c | 0,8 | 0,244551383 |
| miR.609 + miR.603 + miR.220aa + miR.519b + miR.29a + miR.376a* + miR.518c | 0,8 | 0,198217239 |
| miR.609 + miR.603 + miR.220aa + miR.26a + miR.29a + miR.376a* + miR.518c | 0,8 | 0,155639038 |
| miR.609 + miR.603 + miR.520f + miR.29a + miR.369.3p + miR.376a* + miR.518c | 0,8 | 0,112953682 |
| miR.609 + miR.603 + miR.220aa + miR.29a + miR.376a* + miR.518c + miR.660 | 0,8 | 0,204298726 |
| miR.558 + miR.609 + miR.130a + miR.29a + miR.376a* + miR.494 + miR.518c | 0,8 | 0,150493528 |
| miR.558 + miR.609 + miR.130a + miR.29a + miR.376a* + miR.518c + miR.639 | 0,8 | 0,068254929 |
| miR.609 + miR.603 + miR.520f + miR.29a + miR.376a* + miR.494 + miR.518c | 0,8 | 0,094133593 |
| miR.558 + miR.609 + miR.26a + miR.29a + miR.376a* + miR.518c + miR.660 | 0,8 | 0,142031284 |
| miR.558 + miR.609 + miR.26a + miR.29a + miR.362 + miR.376a* + miR.518c | 0,8 | 0,210300462 |
| miR.558 + miR.609 + miR.519b + miR.26a + miR.29a + miR.376a* + miR.518c | 0,8 | 0,158773957 |
| miR.558 + miR.609 + miR.130a + miR.26a + miR.29a + miR.376a* + miR.518c | 0,8 | 0,141313551 |
| miR.558 + miR.609 + miR.29a + miR.376a* + miR.518c + miR.639 + miR.338 | 0,79 | 0,098937459 |
| miR.558 + miR.609 + miR.29a + miR.362 + miR.376a* + miR.518c + miR.639 | 0,79 | 0,114050461 |
| miR.609 + miR.520f + miR.220aa + miR.29a + miR.369.3p + miR.376a* + miR.518c | 0,79 | 0,386937705 |
| miR.609 + miR.603 + miR.220aa + miR.29a + miR.376a* + miR.494 + miR.518c | 0,79 | 0,09534330 7 |
| miR.609 + miR.520f + miR.220aa + miR.29a + miR.376a* + miR.494 + miR.518c | 0,79 | 0,34388918 |
| miR.609 + miR.603 + miR.220aa + miR.29a + miR.376a* + miR.518c + miR.639 | 0,79 | 0,080340683 |
| miR.558 + miR.609 + miR.519b + miR.29a + miR.376a* + miR.518c + miR.639 | 0,79 | 0,062136982 |
| miR.558 + miR.609 + miR.29a + miR.376a* + miR.494 + miR.518c + miR.639 | 0,79 | 0,059422498 |
| miR.609 + miR.603 + miR.220aa + miR.130a + miR.29a + miR.376a* + miR.518c | 0,79 | 0,12155247 |
| miR.609 + miR.603 + miR.220aa + miR.29a + miR.376a* + miR.518c + miR.338 | 0,79 | 0,092825736 |
| miR.558 + miR.609 + miR.29a + miR.362 + miR.376a* + miR.518c + miR.338 | 0,79 | 0,042112106 |
| miR.558 + miR.609 + miR.130a + miR.29a + miR.362 + miR.376a* + miR.518c | 0,79 | 0,057370027 |
| miR.558 + miR.609 + miR.29a + miR.376a* + miR.518c + miR.660 + miR.338 | 0,79 | 0,09140135 |
| miR.558 + miR.609 + miR.29a + miR.376a* + miR.518c + miR.639 + miR.660 | 0,79 | 0,14662121 |
| miR.558 + miR.609 + miR.130a + miR.29a + miR.376a* + miR.518c + miR.338 | 0,79 | 0,028655704 |
| miR.609 + miR.603 + miR.520f + miR.29a + miR.376a* + miR.518c + miR.639 | 0,79 | 0,01819747 |
| miR.558 + miR.609 + miR.519b + miR.29a + miR.376a* + miR.518c + miR.660 | 0,79 | 0,040305976 |
| miR.558 + miR.609 + miR.29a + miR.362 + miR.376a* + miR.518c + miR.660 | 0,79 | 0,023853552 |
| miR.558 + miR.609 + miR.130a + miR.29a + miR.376a* + miR.518c + miR.660 | 0,79 | 0,070099167 |
| miR.558 + miR.609 + miR.519b + miR.29a + miR.376a* + miR.518c + miR.338 | 0,79 | 0,047274439 |
| miR.558 + miR.609 + miR.29a + miR.376a* + miR.494 + miR.518c + miR.338 | 0,79 | 0,074655187 |
| miR.558 + miR.609 + miR.519b + miR.29a + miR.362 + miR.376a* + miR.518c | 0,79 | 0,051958259 |
| miR.558 + miR.609 + miR.603 + miR.369.3p + miR.376a* + miR.518c + miR.639 | 0,79 | 0,129365113 |
| miR.558 + miR.609 + miR.519b + miR.130a + miR.29a + miR.376a* + miR.518c | 0,79 | 0,062867155 |
| miR.609 + miR.603 + miR.29a + miR.369.3p + miR.376a* + miR.494 + miR.518c | 0,79 | 0,024020148 |
| miR.558 + miR.609 + miR.29a + miR.362 + miR.376a* + miR.494 + miR.518c | 0,79 | 0,075519808 |
| miR.609 + miR.603 + miR.29a + miR.369.3p + miR.376a* + miR.518c + miR.639 | 0,79 | 0,018578868 |
| miR.609 + miR.603 + miR.520f + miR.130a + miR.29a + miR.376a* + miR.518c | 0,79 | 0,030963519 |
| miR.609 + miR.603 + miR.26a + miR.29a + miR.369.3p + miR.376a* + miR.518c | 0,79 | 0,019120213 |
| miR.558 + miR.609 + miR.519b + miR.29a + miR.376a* + miR.494 + miR.518c | 0,79 | 0,161499486 |
| miR.609 + miR.220aa + miR.29a + miR.369.3p + miR.376a* + miR.518c + miR.660 | 0,79 | 0,347879436 |
| miR.558 + miR.609 + miR.520f + miR.29a + miR.369.3p + miR.376a* + miR.338 | 0,79 | 0,192029995 |
| miR.558 + miR.609 + miR.29a + miR.376a* + miR.494 + miR.518c + miR.660 | 0,79 | 0,056021885 |
| miR.609 + miR.603 + miR.520f + miR.29a + miR.376a* + miR.518c + miR.338 | 0,79 | 0,382763568 |
| miR.609 + miR.603 + miR.29a + miR.362 + miR.369.3p + miR.376a* + miR.518c | 0,79 | 0,110998575 |
| miR.609 + miR.520f + miR.220aa + miR.29a + miR.376a* + miR.518c + miR.660 | 0,79 | 0,341995375 |
| miR.609 + miR.520f + miR.220aa + miR.29a + miR.362 + miR.376a* * + miR.518c | 0,79 | 0,475144603 |
| miR.609 + miR.603 + miR.29a + miR.369.3p + miR.376a* + miR.518c + miR.660 | 0,79 | 0,005214757 |
| miR.609 + miR.603 + miR.519b + miR.29a + miR.369.3p + miR.376a* + miR.518c | 0,79 | 0,006677565 |
| miR.609 + miR.603 + miR.520f + miR.29a + miR.362 + miR.376a* + miR.518c | 0,79 | 0,078638928 |
| miR.609 + miR.520f + miR.220aa + miR.130a + miR.29a + miR.376a* + miR.518c | 0,79 | 0,279815891 |
| miR.558 + miR.609 + miR.520f + miR.29a + miR.376a* + miR.494 + miR.338 | 0,79 | 0,086095333 |
| miR.609 + miR.220aa + miR.519b + miR.29a + miR.369.3p + miR.376a* + miR.518c | 0,79 | 0,053108906 |
| miR.609 + miR.220aa + miR.26a + miR.29a + miR.369.3p + miR.376a* + miR.518c | 0,79 | 0,231379998 |
| miR.609 + miR.603 + miR.29a + miR.369.3p + miR.376a* + miR.518c + miR.338 | 0,79 | 0,095546627 |
| miR.609 + miR.603 + miR.520f + miR.26a + miR.29a + miR.376a* + miR.518c | 0,79 | 0,077713891 |
| miR.609 + miR.603 + miR.520f + miR.519b + miR.29a + miR.376a* + miR.518c | 0,79 | 0,054308627 |
| miR.609 + miR.520f + miR.29a + miR.376a* + miR.494 + miR.518c + miR.338 | 0,79 | 0,264931684 |
| miR.609 + miR.603 + miR.130a + miR.29a + miR.369.3p + miR.376a* + miR.518c | 0,79 | 0,004061965 |
| miR.558 + miR.609 + miR.603 + miR.520f + miR.376a* + miR.518c + miR.639 | 0,79 | 0,164459406 |
| miR.609 + miR.603 + miR.520f + miR.29a + miR.376a* + miR.518c + miR.660 | 0,79 | 0,04984183 |
| miR.609 + miR.520f + miR.29a + miR.369.3p + miR.376a* + miR.494 + miR.518c | 0,79 | 0,2712076 |
| miR.609 + miR.220aa + miR.29a + miR.369.3p + miR.376a* + miR.518c + miR.338 | 0,79 | 0,118569941 |
| miR.558 + miR.609 + miR.603 + miR.520f + miR.130a + miR.376a* + miR.518c | 0,79 | 0,03928753 |
| miR.609 + miR.220aa + miR.29a + miR.369.3p + miR.376a* + miR.494 + miR.518c | 0,79 | 0,441436661 |
| miR.609 + miR.603 + miR.29a + miR.376a* + miR.494 + miR.518c + miR.639 | 0,79 | 0,005325103 |
| miR.558 + miR.609 + miR.520f + miR.130a + miR.376a* + miR.518c + miR.338 | 0,79 | 0,208365173 |
| miR.609 + miR.520f + miR.220aa + miR.26a + miR.29a + miR.376a* + miR.518c | 0,79 | 0,336945692 |
| miR.609 + miR.520f + miR.29a + miR.376a* + miR.494 + miR.518c + miR.660 | 0,79 | 0,215220026 |
| miR.609 + miR.520f + miR.29a + miR.376a* + miR.494 + miR.518c + miR.639 | 0,79 | 0,168490783 |
| miR.558 + miR.609 + miR.220aa + miR.26a + miR.29a + miR.518c + miR.660 | 0,79 | 0,024360855 |
| miR.558 + miR.609 + miR.603 + miR.220aa + miR.376a* + miR.518c + miR.639 | 0,79 | 0,042927216 |
| miR.609 + miR.520f + miR.220aa + miR.519b + miR.29a + miR.376a* + miR.518c | 0,79 | 0,376416943 |
| miR.558 + miR.609 + miR.520f + miR.376a* + miR.518c + miR.639 + miR.338 | 0,79 | 0,36433758 |
| miR.558 + miR.609 + miR.520f + miR.29a + miR.362 + miR.518c + miR.660 | 0,79 | 0,069346328 |
| miR.609 + miR.220aa + miR.29a + miR.369.3p + miR.376a* + miR.518c + miR.639 | 0,79 | 0,105590463 |
| miR.609 + miR.520f + miR.519b + miR.29a + miR.369.3p + miR.376a* + miR.518c | 0,79 | 0,180385142 |
| miR.558 + miR.609 + miR.520f + miR.29a + miR.369.3p + miR.376a* + miR.494 | 0,79 | 0,078706196 |
| miR.609 + miR.520f + miR.220aa + miR.29a + miR.376a* + miR.518c + miR.338 | 0,79 | 0,437108605 |
| miR.558 + miR.609 + miR.520f + miR.29a + miR.518c + miR.639 + miR.660 | 0,79 | 0,081885245 |
| miR.609 + miR.220aa + miR.29a + miR.362 + miR.369.3p + miR.376a* + miR.518c | 0,79 | 0,151705294 |
| miR.558 + miR.609 + miR.520f + miR.130a + miR.376a* + miR.518c + miR.639 | 0,79 | 0,176315207 |
| miR.609 + miR.520f + miR.220aa + miR.29a + miR.376a* + miR.518c + miR.639 | 0,79 | 0,32112685 |
| miR.558 + miR.609 + miR.520f + miR.26a + miR.29a + miR.518c + miR.660 | 0,79 | 0,059516306 |
| miR.558 + miR.609 + miR.520f + miR.29a + miR.376a* + miR.660 + miR.338 | 0,79 | 0,255670703 |
| miR.558 + miR.609 + miR.520f + miR.130a + miR.29a + miR.376a* + miR.338 | 0,79 | 0,106841277 |
| miR.609 + miR.603 + miR.220aa + miR.130a + miR.369.3p + miR.376a* + miR.518c | 0,79 | 0,051594779 |
| miR.558 + miR.609 + miR.520f + miR.369.3p + miR.376a* + miR.518c + miR.639 | 0,79 | 0,302834571 |
| miR.558 + miR.609 + miR.520f + miR.29a + miR.376a* + miR.639 + miR.338 | 0,79 | 0,107168484 |
| miR.558 + miR.609 + miR.603 + miR.26a + miR.29a + miR.518c + miR.660 | 0,79 | 0,018705037 |
| miR.558 + miR.609 + miR.603 + miR.220aa + miR.130a + miR.376a* + miR.518c | 0,79 | 0,041655174 |
| miR.558 + miR.609 + miR.520f + miR.519b + miR.29a + miR.376a* + miR.338 | 0,79 | 0,146035682 |
| miR.558 + miR.609 + miR.603 + miR.520f + miR.29a + miR.376a* + miR.338 | 0,79 | 0,092270512 |
| miR.609 + miR.520f + miR.26a + miR.29a + miR.376a* + miR.494 + miR.518c | 0,79 | 0,151560335 |
| miR.558 + miR.609 + miR.520f + miR.26a + miR.29a + miR.376a* + miR.338 | 0,79 | 0,084840869 |
| miR.609 + miR.520f + miR.29a + miR.369.3p + miR.376a* + miR.518c + miR.338 | 0,79 | 0,442036117 |
| miR.609 + miR.220aa + miR.130a + miR.29a + miR.369.3p + miR.376a* + miR.518c | 0,79 | 0,13732403 |
| miR.558 + miR.609 + miR.520f + miR.220aa + miR.130a + miR.376a* + miR.518c | 0,79 | 0,050995659 |
| miR.558 + miR.609 + miR.520f + miR.220aa + miR.29a + miR.376a* + miR.338 | 0,79 | 0,081447451 |
| miR.609 + miR.520f + miR.29a + miR.362 + miR.376a* + miR.494 + miR.518c | 0,79 | 0,179772063 |
| miR.558 + miR.609 + miR.603 + miR.130a + miR.369.3p + miR.376a* + miR.518c | 0,79 | 0,025022285 |
| miR.609 + miR.520f + miR.519b + miR.29a + miR.376a* + miR.494 + miR.518c | 0,79 | 0,157354426 |
| miR.609 + miR.603 + miR.26a + miR.29a + miR.376a* + miR.494 + miR.518c | 0,79 | 0,019309474 |
| miR.558 + miR.609 + miR.520f + miR.519b + miR.29a + miR.518c + miR.660 | 0,79 | 0,052011918 |
| miR.558 + miR.609 + miR.520f + miR.130a + miR.29a + miR.518c + miR.660 | 0,79 | 0,062591102 |
| miR.609 + miR.220aa + miR.26a + miR.29a + miR.376a* + miR.494 + miR.518c | 0,79 | 0,056334367 |
| miR.558 + miR.609 + miR.520f + miR.220aa + miR.29a + miR.518c + miR.660 | 0,79 | 0,059394226 |
| miR.558 + miR.609 + miR.220aa + miR.369.3p + miR.376a* + miR.518c + miR.639 | 0,79 | 0,14915253 |
| miR.558 + miR.609 + miR.603 + miR.130a + miR.376a* + miR.518c + miR.639 | 0,79 | 0,036435405 |
| miR.558 + miR.609 + miR.603 + miR.520f + miR.29a + miR.518c + miR.660 | 0,79 | 0,046421266 |
| miR.609 + miR.520f + miR.130a + miR.29a + miR.376a* + miR.494 + miR.518c | 0,79 | 0,159193429 |
| miR.558 + miR.609 + miR.520f + miR.519b + miR.29a + miR.376a* + miR.494 | 0,79 | 0,108376704 |
| miR.609 + miR.520f + miR.29a + miR.369.3p + miR.376a* + miR.518c + miR.639 | 0,79 | 0,223681012 |
| miR.558 + miR.609 + miR.520f + miR.29a + miR.518c + miR.660 + miR.338 | 0,79 | 0,046748659 |
| miR.558 + miR.609 + miR.603 + miR.520f + miR.29a + miR.369.3p + miR.376a* | 0,79 | 0,0678819 |
| miR.558 + miR.609 + miR.520f + miR.29a + miR.376a* + miR.494 + miR.660 | 0,79 | 0,125273034 |
| miR.558 + miR.609 + miR.520f + miR.29a + miR.494 + miR.518c + miR.660 | 0,79 | 0,054337348 |
| miR.558 + miR.609 + miR.520f + miR.29a + miR.376a* + miR.494 + miR.639 | 0,79 | 0,063699307 |
| miR.558 + miR.609 + miR.603 + miR.220aa + miR.369.3p + miR.376a* + miR.518c | 0,79 | 0,033066672 |
| miR.558 + miR.609 + miR.520f + miR.29a + miR.369.3p + miR.518c + miR.660 | 0,79 | 0,057263944 |
| miR.609 + miR.603 + miR.519b + miR.29a + miR.376a* + miR.494 + miR.518c | 0,79 | 0,014393844 |
| miR.609 + miR.220aa + miR.29a + miR.376a* + miR.494 + miR.518c + miR.660 | 0,79 | 0,05588982 |
| miR.558 + miR.609 + miR.520f + miR.29a + miR.362 + miR.376a* + miR.338 | 0,79 | 0,152086774 |
| miR.609 + miR.520f + miR.29a + miR.369.3p + miR.376a* + miR.518c + miR.660 | 0,79 | 0,220871924 |
| miR.558 + miR.609 + miR.520f + miR.29a + miR.369.3p + miR.376a* + miR.639 | 0,79 | 0,092882264 |
| miR.558 + miR.609 + miR.603 + miR.520f + miR.29a + miR.376a* + miR.494 | 0,79 | 0,046125093 |
| miR.609 + miR.520f + miR.26a + miR.29a + miR.369.3p + miR.376a* + miR.518c | 0,79 | 0,183771477 |
| miR.558 + miR.609 + miR.520f + miR.26a + miR.29a + miR.376a* + miR.494 | 0,79 | 0,058610704 |
| miR.609 + miR.603 + miR.29a + miR.376a* + miR.494 + miR.518c + miR.338 | 0,79 | 0,03003348 |
| miR.609 + miR.603 + miR.220aa + miR.369.3p + miR.376a* + miR.518c + miR.639 | 0,79 | 0,03181683 |
| miR.558 + miR.609 + miR.520f + miR.130a + miR.29a + miR.369.3p + miR.376a* | 0,79 | 0,070136984 |
| miR.558 + miR.609 + miR.520f + miR.29a + miR.362 + miR.376a* + miR.494 | 0,79 | 0,066075059 |
| miR.609 + miR.520f + miR.130a + miR.29a + miR.369.3p + miR.376a* + miR.518c | 0,79 | 0,180389967 |
| miR.609 + miR.603 + miR.520f + miR.220aa + miR.130a + miR.376a* + miR.518c | 0,79 | 0,079715485 |
| miR.558 + miR.609 + miR.520f + miR.130a + miR.29a + miR.376a* + miR.494 | 0,79 | 0,057588097 |
| miR.609 + miR.603 + miR.130a + miR.29a + miR.376a* + miR.494 + miR.518c | 0,79 | 0,020795419 |
| miR.558 + miR.609 + miR.520f + miR.29a + miR.369.3p + miR.376a* + miR.660 | 0,79 | 0,104980423 |
| miR.609 + miR.520f + miR.519b + miR.29a + miR.376a* + miR.518c + miR.639 | 0,79 | 0,04536668 |
| miR.558 + miR.609 + miR.520f + miR.220aa + miR.29a + miR.376a* + miR.494 | 0,79 | 0,064514507 |
| miR.609 + miR.603 + miR.29a + miR.376a* + miR.494 + miR.518c + miR.660 | 0,79 | 0,018013249 |
| miR.558 + miR.609 + miR.520f + miR.376a* + miR.518c + miR.639 + miR.660 | 0,79 | 0,286575086 |
| miR.609 + miR.603 + miR.29a + miR.362 + miR.376a* + miR.494 + miR.518c | 0,79 | 0,016458536 |
| miR.609 + miR.520f + miR.29a + miR.376a* + miR.518c + miR.660 + miR.338 | 0,79 | 0,386922586 |
| miR.558 + miR.609 + miR.520f + miR.26a + miR.29a + miR.369.3p + miR.376a* | 0,79 | 0,061480526 |
| miR.609 + miR.603 + miR.26a + miR.29a + miR.376a* + miR.518c + miR.639 | 0,79 | 0,009034589 |
| miR.558 + miR.609 + miR.520f + miR.130a + miR.369.3p + miR.376a* + miR.518c | 0,79 | 0,057548194 |
| miR.609 + miR.520f + miR.519b + miR.130a + miR.29a + miR.376a* + miR.518c | 0,79 | 0,087144576 |
| miR.558 + miR.609 + miR.520f + miR.220aa + miR.29a + miR.369.3p + miR.376a* | 0,79 | 0,063034652 |
| miR.558 + miR.609 + miR.520f + miR.220aa + miR.376a* + miR.518c + miR.639 | 0,79 | 0,216787991 |
| miR.609 + miR.603 + miR.520f + miR.130a + miR.376a* + miR.518c + miR.639 | 0,79 | 0,033871147 |
| miR.609 + miR.520f + miR.26a + miR.29a + miR.376a* + miR.518c + miR.338 | 0,79 | 0,351173259 |
| miR.558 + miR.609 + miR.130a + miR.26a + miR.29a + miR.518c + miR.660 | 0,79 | 0,013327785 |
| miR.558 + miR.609 + miR.520f + miR.26a + miR.376a* + miR.518c + miR.639 | 0,79 | 0,130137736 |
| miR.558 + miR.609 + miR.520f + miR.29a + miR.362 + miR.369.3p + miR.376a* | 0,79 | 0,061385059 |
| miR.558 + miR.609 + miR.220aa + miR.130a + miR.369.3p + miR.376a* + miR.518c | 0,79 | 0,056436539 |
| miR.609 + miR.520f + miR.26a + miR.29a + miR.376a* + miR.518c + miR.660 | 0,79 | 0,146738814 |
| miR.558 + miR.609 + miR.520f + miR.519b + miR.29a + miR.369.3p + miR.376a* | 0,79 | 0,080909233 |
| miR.558 + miR.609 + miR.26a + miR.29a + miR.369.3p + miR.518c + miR.660 | 0,79 | 0,02075549 |
| miR.609 + miR.520f + miR.29a + miR.376a* + miR.518c + miR.639 + miR.338 | 0,79 | 0,216249971 |
| miR.558 + miR.609 + miR.520f + miR.29a + miR.376a* + miR.639 + miR.660 | 0,79 | 0,082945161 |
| miR.609 + miR.520f + miR.519b + miR.29a + miR.376a* + miR.518c + miR.338 | 0,79 | 0,324638105 |
| miR.558 + miR.609 + miR.26a + miR.29a + miR.518c + miR.639 + miR.660 | 0,79 | 0,044353924 |
| miR.609 + miR.220aa + miR.519b + miR.26a + miR.29a + miR.376a* + miR.518c | 0,79 | 0,062424972 |
| miR.609 + miR.603 + miR.130a + miR.26a + miR.29a + miR.376a* + miR.518c | 0,79 | 0,004990594 |
| miR.558 + miR.609 + miR.603 + miR.220aa + miR.519b + miR.376a* + miR.518c | 0,79 | 0,03147335 |
| miR.609 + miR.520f + miR.519b + miR.29a + miR.376a* + miR.518c + miR.660 | 0,79 | 0,119888614 |
| miR.609 + miR.603 + miR.130a + miR.29a + miR.376a* + miR.518c + miR.639 | 0,79 | 0,00067983 |
| miR.609 + miR.520f + miR.29a + miR.376a* + miR.518c + miR.639 + miR.660 | 0,79 | 0,134394809 |
| miR.558 + miR.609 + miR.26a + miR.29a + miR.494 + miR.518c + miR.660 | 0,79 | 0,014052264 |
| miR.558 + miR.609 + miR.26a + miR.29a + miR.518c + miR.660 + miR.338 | 0,79 | 0,010675371 |
| miR.609 + miR.603 + miR.29a + miR.376a* + miR.518c + miR.639 + miR.338 | 0,79 | 0,007014287 |
| miR.609 + miR.520f + miR.519b + miR.26a + miR.29a + miR.376a* + miR.518c | 0,79 | 0,099139978 |
| miR.609 + miR.603 + miR.520f + miR.130a + miR.376a* + miR.518c + miR.338 | 0,79 | 0,103316352 |
| miR.558 + miR.609 + miR.520f + miR.362 + miR.376a* + miR.518c + miR.639 | 0,79 | 0,232377675 |
| miR.609 + miR.520f + miR.29a + miR.362 + miR.376a* + miR.518c + miR.338 | 0,79 | 0,345706741 |
| miR.609 + miR.220aa + miR.519b + miR.29a + miR.362 + miR.376a* + miR.518c | 0,79 | 0,018034377 |
| miR.558 + miR.609 + miR.520f + miR.376a* + miR.494 + miR.518c + miR.639 | 0,79 | 0,285149788 |
| miR.558 + miR.609 + miR.520f + miR.519b + miR.376a* + miR.518c + miR.639 | 0,79 | 0,232964205 |
| miR.558 + miR.609 + miR.519b + miR.26a + miR.29a + miR.518c + miR.660 | 0,79 | 0,007457376 |
| miR.609 + miR.603 + miR.220aa + miR.130a + miR.376a* + miR.518c + miR.639 | 0,79 | 0,040593982 |
| miR.609 + miR.520f + miR.26a + miR.29a + miR.376a* + miR.518c + miR.639 | 0,79 | 0,07329894 |
| miR.558 + miR.609 + miR.520f + miR.130a + miR.26a + miR.376a* + miR.518c | 0,79 | 0,030507676 |
| miR.609 + miR.520f + miR.519b + miR.29a + miR.362 + miR.376a* + miR.518c | 0,79 | 0,165907826 |
| miR.609 + miR.520f + miR.130a + miR.29a + miR.376a* + miR.518c + miR.660 | 0,79 | 0,104030103 |
| miR.558 + miR.609 + miR.520f + miR.26a + miR.29a + miR.376a* + miR.660 | 0,79 | 0,072464296 |
| miR.609 + miR.603 + miR.519b + miR.29a + miR.376a* + miR.518c + miR.639 | 0,79 | 0,004981793 |
| miR.609 + miR.520f + miR.130a + miR.29a + miR.376a* + miR.518c + miR.639 | 0,79 | 0,120914979 |
| miR.558 + miR.609 + miR.603 + miR.26a + miR.376a* + miR.518c + miR.639 | 0,79 | 0,089934804 |
| miR.609 + miR.220aa + miR.26a + miR.29a + miR.362 + miR.376a* + miR.518c | 0,79 | 0,063933855 |
| miR.609 + miR.220aa + miR.29a + miR.362 + miR.376a* + miR.494 + miR.518c | 0,79 | 0,027539531 |
| miR.558 + miR.609 + miR.603 + miR.376a* + miR.518c + miR.639 + miR.338 | 0,79 | 0,054331874 |
| miR.609 + miR.520f + miR.130a + miR.29a + miR.376a* + miR.518c + miR.338 | 0,79 | 0,270368469 |
| miR.609 + miR.603 + miR.29a + miR.362 + miR.376a* + miR.518c + miR.639 | 0,79 | 0,00552345 |
| miR.609 + miR.520f + miR.130a + miR.26a + miR.29a + miR.376a* + miR.518c | 0,79 | 0,107840654 |
| miR.609 + miR.520f + miR.26a + miR.29a + miR.362 + miR.376a* + miR.518c | 0,79 | 0,17426981 |
| miR.609 + miR.220aa + miR.29a + miR.376a* + miR.494 + miR.518c + miR.338 | 0,79 | 0,016790109 |
| miR.558 + miR.609 + miR.603 + miR.362 + miR.376a* + miR.518c + miR.639 | 0,79 | 0,053886075 |
| miR.558 + miR.609 + miR.26a + miR.29a + miR.362 + miR.518c + miR.660 | 0,79 | 0,016111562 |
| miR.609 + miR.220aa + miR.519b + miR.29a + miR.376a* + miR.518c + miR.338 | 0,79 | 0,008169744 |
| miR.558 + miR.609 + miR.603 + miR.376a* + miR.518c + miR.639 + miR.660 | 0,79 | 0,061209527 |
| miR.609 + miR.220aa + miR.519b + miR.29a + miR.376a* + miR.494 + miR.518c | 0,79 | 0,024368321 |
| miR.609 + miR.520f + miR.29a + miR.362 + miR.369.3p + miR.376a* + miR.518c | 0,79 | 0,197937421 |
| miR.558 + miR.609 + miR.603 + miR.519b + miR.376a* + miR.518c + miR.639 | 0,79 | 0,059897897 |
| miR.609 + miR.220aa + miR.29a + miR.362 + miR.376a* + miR.518c + miR.338 | 0,79 | 0,012360346 |
| miR.609 + miR.220aa + miR.519b + miR.29a + miR.376a* + miR.518c + miR.660 | 0,79 | 0,012017444 |
| miR.558 + miR.609 + miR.603 + miR.520f + miR.29a + miR.376a* + miR.660 | 0,79 | 0,05638436 |
| miR.609 + miR.603 + miR.29a + miR.376a* + miR.518c + miR.639 + miR.660 | 0,79 | 0,006887135 |
| miR.558 + miR.609 + miR.603 + miR.376a* + miR.494 + miR.518c + miR.639 | 0,79 | 0,091458863 |
| miR.558 + miR.609 + miR.220aa + miR.130a + miR.376a* + miR.518c + miR.639 | 0,79 | 0,048328988 |
| miR.609 + miR.520f + miR.29a + miR.362 + miR.376a* + miR.518c + miR.639 | 0,79 | 0,155225133 |
| miR.609 + miR.220aa + miR.519b + miR.29a + miR.376a* + miR.518c + miR.639 | 0,79 | 0,011852535 |
| miR.609 + miR.520f + miR.29a + miR.362 + miR.376a* + miR.518c + miR.660 | 0,79 | 0,095588241 |
| miR.609 + miR.220aa + miR.130a + miR.29a + miR.376a* + miR.494 + miR.518c | 0,79 | 0,023573084 |
| miR.558 + miR.609 + miR.520f + miR.220aa + miR.29a + miR.376a* + miR.660 | 0,79 | 0,061842619 |
| miR.609 + miR.520f + miR.130a + miR.29a + miR.362 + miR.376a* + miR.518c | 0,79 | 0,130868338 |
| miR.558 + miR.609 + miR.520f + miR.130a + miR.376a* + miR.494 + miR.518c | 0,79 | 0,031074593 |
| miR.609 + miR.603 + miR.26a + miR.29a + miR.376a* + miR.518c + miR.338 | 0,79 | 0,008422824 |
| miR.609 + miR.220aa + miR.29a + miR.376a* + miR.494 + miR.518c + miR.639 | 0,79 | 0,028170458 |
| miR.558 + miR.609 + miR.603 + miR.520f + miR.29a + miR.376a* + miR.639 | 0,79 | 0,037375968 |
| miR.558 + miR.609 + miR.520f + miR.29a + miR.362 + miR.376a* + miR.660 | 0,79 | 0,06263938 |
| miR.558 + miR.609 + miR.603 + miR.520f + miR.519b + miR.29a + miR.376a* | 0,79 | 0,034434776 |
| miR.609 + miR.603 + miR.220aa + miR.130a + miR.26a + miR.376a* + miR.518c | 0,79 | 0,036223839 |
| miR.558 + miR.609 + miR.520f + miR.26a + miR.29a + miR.376a* + miR.639 | 0,79 | 0,043744427 |
| miR.609 + miR.603 + miR.519b + miR.26a + miR.29a + miR.376a* + miR.518c | 0,79 | 0,001746115 |
| miR.609 + miR.603 + miR.26a + miR.29a + miR.362 + miR.376a* + miR.518c | 0,79 | 0,002496086 |
| miR.558 + miR.609 + miR.603 + miR.220aa + miR.29a + miR.518c + miR.660 | 0,79 | 0,008586363 |
| miR.609 + miR.603 + miR.220aa + miR.519b + miR.130a + miR.376a* + miR.518c | 0,79 | 0,036722839 |
| miR.558 + miR.609 + miR.520f + miR.130a + miR.362 + miR.376a* + miR.518c | 0,79 | 0,031754317 |
| miR.558 + miR.609 + miR.520f + miR.519b + miR.29a + miR.376a* + miR.660 | 0,79 | 0,055797438 |
| miR.609 + miR.603 + miR.520f + miR.130a + miR.369.3p + miR.376a* + miR.518c | 0,79 | 0,013538666 |
| miR.558 + miR.609 + miR.603 + miR.520f + miR.130a + miR.29a + miR.376a* | 0,79 | 0,035530556 |
| miR.558 + miR.609 + miR.369.3p + miR.376a* + miR.518c + miR.639 + miR.338 | 0,79 | 0,135959601 |
| miR.609 + miR.603 + miR.130a + miR.369.3p + miR.376a* + miR.518c + miR.639 | 0,79 | 0,010841931 |
| miR.609 + miR.603 + miR.130a + miR.29a + miR.376a* + miR.518c + miR.338 | 0,79 | 0,004726664 |
| miR.609 + miR.603 + miR.26a + miR.29a + miR.376a* + miR.518c + miR.660 | 0,79 | 0,001776638 |
| miR.558 + miR.609 + miR.220aa + miR.26a + miR.29a + miR.362 + miR.518c | 0,79 | 0,006241375 |
| miR.558 + miR.609 + miR.520f + miR.130a + miR.376a* + miR.518c + miR.660 | 0,79 | 0,037090034 |
| miR.609 + miR.220aa + miR.29a + miR.362 + miR.376a* + miR.518c + miR.639 | 0,79 | 0,031439295 |
| miR.609 + miR.220aa + miR.130a + miR.26a + miR.29a + miR.376a* + miR.518c | 0,79 | 0,055177943 |
| miR.609 + miR.220aa + miR.26a + miR.29a + miR.376a* + miR.518c + miR.338 | 0,79 | 0,046300412 |
| miR.609 + miR.220aa + miR.519b + miR.130a + miR.29a + miR.376a* + miR.518c | 0,79 | 0,007592753 |
| miR.558 + miR.609 + miR.520f + miR.130a + miR.29a + miR.376a* + miR.639 | 0,79 | 0,04211244 |
| miR.558 + miR.609 + miR.220aa + miR.130a + miR.29a + miR.518c + miR.660 | 0,79 | 0,001843614 |
| miR.558 + miR.609 + miR.520f + miR.29a + miR.362 + miR.376a* + miR.639 | 0,79 | 0,053291837 |
| miR.609 + miR.603 + miR.130a + miR.29a + miR.362 + miR.376a* + miR.518c | 0,79 | 0,000162055 |
| miR.609 + miR.603 + miR.519b + miR.29a + miR.362 + miR.376a* + miR.518c | 0,79 | 0,000823729 |
| miR.609 + miR.603 + miR.29a + miR.362 + miR.376a* + miR.518c + miR.338 | 0,79 | 0,003948422 |
| miR.609 + miR.603 + miR.130a + miR.29a + miR.376a* + miR.518c + miR.660 | 0,79 | 8,98E-005 |
| miR.609 + miR.603 + miR.29a + miR.362 + miR.376a* + miR.518c + miR.660 | 0,79 | 0,001086233 |
| miR.609 + miR.220aa + miR.26a + miR.29a + miR.376a* + miR.518c + miR.639 | 0,79 | 0,062331349 |
| miR.558 + miR.609 + miR.520f + miR.519b + miR.130a + miR.376a* + miR.518c | 0,79 | 0,041258478 |
| miR.558 + miR.609 + miR.603 + miR.220aa + miR.26a + miR.29a + miR.518c | 0,79 | 0,030884002 |
| miR.609 + miR.220aa + miR.130a + miR.29a + miR.376a* + miR.518c + miR.660 | 0,79 | 0,030204084 |
| miR.558 + miR.609 + miR.520f + miR.220aa + miR.29a + miR.376a* + miR.639 | 0,79 | 0,03510479 |
| miR.609 + miR.220aa + miR.26a + miR.29a + miR.376a* + miR.518c + miR.660 | 0,79 | 0,034474487 |
| miR.609 + miR.603 + miR.519b + miR.130a + miR.29a + miR.376a* + miR.518c | 0,79 | 0,000183613 |
| miR.609 + miR.603 + miR.29a + miR.376a* + miR.518c + miR.660 + miR.338 | 0,79 | 0,002734206 |
| miR.609 + miR.603 + miR.519b + miR.29a + miR.376a* + miR.518c + miR.660 | 0,79 | 0,001358362 |
| miR.558 + miR.609 + miR.603 + miR.26a + miR.29a + miR.369.3p + miR.518c | 0,79 | 0,01577491 |
| miR.609 + miR.220aa + miR.29a + miR.376a* + miR.518c + miR.660 + miR.338 | 0,79 | 0,005252008 |
| miR.558 + miR.609 + miR.520f + miR.130a + miR.26a + miR.29a + miR.376a* | 0,79 | 0,034781279 |
| miR.609 + miR.220aa + miR.29a + miR.376a* + miR.518c + miR.639 + miR.338 | 0,79 | 0,034145139 |
| miR.609 + miR.603 + miR.519b + miR.29a + miR.376a* + miR.518c + miR.338 | 0,79 | 0,002255017 |
| miR.558 + miR.609 + miR.520f + miR.519b + miR.29a + miR.362 + miR.376a* | 0,79 | 0,047064737 |
| miR.558 + miR.609 + miR.520f + miR.519b + miR.29a + miR.376a* + miR.639 | 0,79 | 0,035807403 |
| miR.558 + miR.609 + miR.362 + miR.369.3p + miR.376a* + miR.518c + miR.639 | 0,79 | 0,147161344 |
| miR.558 + miR.609 + miR.520f + miR.130a + miR.29a + miR.376a* + miR.660 | 0,79 | 0,058152187 |
| miR.558 + miR.609 + miR.130a + miR.369.3p + miR.376a* + miR.518c + miR.639 | 0,79 | 0,128514837 |
| miR.558 + miR.609 + miR.603 + miR.520f + miR.26a + miR.29a + miR.376a* | 0,79 | 0,028503537 |
| miR.558 + miR.609 + miR.603 + miR.130a + miR.26a + miR.376a* + miR.518c | 0,79 | 0,027580709 |
| miR.558 + miR.609 + miR.603 + miR.26a + miR.29a + miR.518c + miR.338 | 0,79 | 0,02047927 |
| miR.558 + miR.609 + miR.603 + miR.130a + miR.376a* + miR.518c + miR.338 | 0,79 | 0,032979385 |
| miR.609 + miR.220aa + miR.29a + miR.376a* + miR.518c + miR.639 + miR.660 | 0,79 | 0,016361163 |
| miR.558 + miR.609 + miR.220aa + miR.130a + miR.26a + miR.376a* + miR.518c | 0,79 | 0,036616618 |
| miR.558 + miR.609 + miR.26a + miR.369.3p + miR.376a* + miR.518c + miR.639 | 0,79 | 0,144178297 |
| miR.558 + miR.609 + miR.520f + miR.130a + miR.29a + miR.362 + miR.376a* | 0,79 | 0,0455553 |
| miR.558 + miR.609 + miR.520f + miR.220aa + miR.130a + miR.29a + miR.376a* | 0,79 | 0,031594159 |
| miR.609 + miR.519b + miR.26a + miR.29a + miR.369.3p + miR.376a* + miR.518c | 0,79 | 0,056505964 |
| miR.609 + miR.220aa + miR.130a + miR.29a + miR.362 + miR.376a* + miR.518c | 0,79 | 0,032180945 |
| miR.609 + miR.220aa + miR.29a + miR.362 + miR.376a* + miR.518c + miR.660 | 0,79 | 0,01185485 |
| miR.558 + miR.609 + miR.220aa + miR.29a + miR.518c + miR.660 + miR.338 | 0,79 | 0,002784308 |
| miR.558 + miR.609 + miR.520f + miR.519b + miR.130a + miR.29a + miR.376a* | 0,79 | 0,035266528 |
| miR.558 + miR.609 + miR.520f + miR.26a + miR.29a + miR.362 + miR.376a* | 0,79 | 0,041576634 |
| miR.558 + miR.609 + miR.603 + miR.520f + miR.29a + miR.362 + miR.376a* | 0,79 | 0,042506952 |
| miR.558 + miR.609 + miR.603 + miR.520f + miR.26a + miR.29a + miR.518c | 0,79 | 0,019690085 |
| miR.558 + miR.609 + miR.603 + miR.130a + miR.362 + miR.376a* + miR.518c | 0,79 | 0,009913306 |
| miR.609 + miR.603 + miR.520f + miR.369.3p + miR.376a* + miR.518c + miR.639 | 0,79 | 0,020460122 |
| miR.609 + miR.603 + miR.220aa + miR.130a + miR.376a* + miR.518c + miR.338 | 0,79 | 0,10654041 |
| miR.558 + miR.609 + miR.603 + miR.520f + miR.220aa + miR.29a + miR.376a* | 0,79 | 0,033882422 |
| miR.558 + miR.609 + miR.520f + miR.220aa + miR.26a + miR.29a + miR.376a* | 0,79 | 0,027325355 |
| miR.558 + miR.609 + miR.220aa + miR.29a + miR.362 + miR.518c + miR.660 | 0,79 | 0,003900385 |
| miR.609 + miR.220aa + miR.130a + miR.29a + miR.376a* + miR.518c + miR.338 | 0,79 | 0,012269227 |
| miR.558 + miR.609 + miR.520f + miR.220aa + miR.519b + miR.29a + miR.376a* | 0,79 | 0,032081583 |
| miR.558 + miR.609 + miR.520f + miR.130a + miR.369.3p + miR.376a* + miR.338 | 0,79 | 0,295223702 |
| miR.558 + miR.609 + miR.220aa + miR.26a + miR.29a + miR.518c + miR.338 | 0,79 | 0,012301699 |
| miR.609 + miR.603 + miR.220aa + miR.130a + miR.376a* + miR.494 + miR.518c | 0,79 | 0,046543256 |
| miR.609 + miR.603 + miR.220aa + miR.130a + miR.376a* + miR.518c + miR.660 | 0,79 | 0,075257701 |
| miR.558 + miR.609 + miR.603 + miR.130a + miR.376a* + miR.518c + miR.660 | 0,79 | 0,012535871 |
| miR.558 + miR.609 + miR.520f + miR.519b + miR.26a + miR.29a + miR.376a* | 0,79 | 0,032383598 |
| miR.609 + miR.603 + miR.220aa + miR.130a + miR.362 + miR.376a* + miR.518c | 0,79 | 0,053512435 |
| miR.609 + miR.519b + miR.29a + miR.369.3p + miR.376a* + miR.518c + miR.639 | 0,79 | 0,047045854 |
| miR.558 + miR.609 + miR.603 + miR.26a + miR.29a + miR.362 + miR.518c | 0,79 | 0,01624326 |
| miR.558 + miR.609 + miR.520f + miR.220aa + miR.29a + miR.362 + miR.376a* | 0,79 | 0,04227083 |
| miR.558 + miR.609 + miR.29a + miR.362 + miR.369.3p + miR.376a* + miR.660 | 0,78 | 0,170577351 |
| miR.558 + miR.609 + miR.603 + miR.26a + miR.29a + miR.518c + miR.639 | 0,78 | 0,017268238 |
| miR.558 + miR.609 + miR.603 + miR.519b + miR.26a + miR.29a + miR.518c | 0,78 | 0,02295382 |
| miR.609 + miR.220aa + miR.130a + miR.29a + miR.376a* + miR.518c + miR.639 | 0,78 | 0,045718265 |
| miR.558 + miR.609 + miR.603 + miR.519b + miR.130a + miR.376a* + miR.518c | 0,78 | 0,018681653 |
| miR.609 + miR.603 + miR.520f + miR.376a* + miR.518c + miR.639 + miR.338 | 0,78 | 0,079513918 |
| miR.558 + miR.609 + miR.220aa + miR.130a + miR.376a* + miR.518c + miR.338 | 0,78 | 0,04606652 |
| miR.558 + miR.609 + miR.603 + miR.362 + miR.369.3p + miR.376a* + miR.518c | 0,78 | 0,02760726 |
| miR.609 + miR.520f + miR.29a + miR.369.3p + miR.376a* + miR.494 + miR.338 | 0,78 | 0,108065437 |
| miR.609 + miR.519b + miR.29a + miR.362 + miR.369.3p + miR.376a* + miR.518c | 0,78 | 0,151827667 |
| miR.558 + miR.609 + miR.603 + miR.130a + miR.376a* + miR.494 + miR.518c | 0,78 | 0,017785064 |
| miR.558 + miR.609 + miR.519b + miR.369.3p + miR.376a* + miR.518c + miR.639 | 0,78 | 0,142879297 |
| miR.609 + miR.603 + miR.520f + miR.220aa + miR.376a* + miR.518c + miR.639 | 0,78 | 0,032965168 |
| miR.558 + miR.609 + miR.603 + miR.130a + miR.26a + miR.29a + miR.518c | 0,78 | 0,011673792 |
| miR.558 + miR.609 + miR.220aa + miR.130a + miR.376a* + miR.494 + miR.518c | 0,78 | 0,018551299 |
| miR.558 + miR.609 + miR.220aa + miR.130a + miR.362 + miR.376a* + miR.518c | 0,78 | 0,016995329 |
| miR.558 + miR.609 + miR.369.3p + miR.376a* + miR.518c + miR.639 + miR.660 | 0,78 | 0,128633443 |
| miR.558 + miR.609 + miR.603 + miR.26a + miR.29a + miR.494 + miR.518c | 0,78 | 0,017647124 |
| miR.558 + miR.609 + miR.603 + miR.369.3p + miR.376a* + miR.518c + miR.338 | 0,78 | 0,055527369 |
| miR.609 + miR.603 + miR.520f + miR.29a + miR.376a* + miR.494 + miR.338 | 0,78 | 0,110738092 |
| miR.609 + miR.29a + miR.362 + miR.369.3p + miR.376a* + miR.518c + miR.660 | 0,78 | 0,289420411 |
| miR.558 + miR.609 + miR.520f + miR.26a + miR.29a + miR.362 + miR.518c | 0,78 | 0,014683101 |
| miR.558 + miR.609 + miR.220aa + miR.519b + miR.130a + miR.376a* + miR.518c | 0,78 | 0,035167937 |
| miR.558 + miR.609 + miR.603 + miR.130a + miR.29a + miR.518c + miR.660 | 0,78 | 0,004039961 |
| miR.558 + miR.609 + miR.130a + miR.29a + miR.518c + miR.639 + miR.660 | 0,78 | 0,006978861 |
| miR.558 + miR.609 + miR.220aa + miR.130a + miR.376a* + miR.518c + miR.660 | 0,78 | 0,030526538 |
| miR.558 + miR.609 + miR.603 + miR.520f + miR.518c + miR.639 + miR.660 | 0,78 | 0,098028023 |
| miR.558 + miR.609 + miR.603 + miR.220aa + miR.376a* + miR.518c + miR.338 | 0,78 | 0,017517995 |
| miR.558 + miR.609 + miR.520f + miR.130a + miR.376a* + miR.639 + miR.338 | 0,78 | 0,232178322 |
| miR.609 + miR.519b + miR.29a + miR.369.3p + miR.376a* + miR.494 + miR.518c | 0,78 | 0,07723018 |
| miR.609 + miR.520f + miR.220aa + miR.130a + miR.369.3p + miR.376a* + miR.518c | 0,78 | 0,023212457 |
| miR.558 + miR.609 + miR.220aa + miR.130a + miR.26a + miR.29a + miR.518c | 0,78 | 0,007519246 |
| miR.558 + miR.609 + miR.520f + miR.26a + miR.29a + miR.518c + miR.338 | 0,78 | 0,014007881 |
| miR.609 + miR.603 + miR.362 + miR.369.3p + miR.376a* + miR.518c + miR.639 | 0,78 | 0,018224599 |
| miR.558 + miR.609 + miR.220aa + miR.26a + miR.29a + miR.369.3p + miR.518c | 0,78 | 0,010640788 |
| miR.609 + miR.603 + miR.520f + miR.26a + miR.376a* + miR.518c + miR.639 | 0,78 | 0,040581193 |
| miR.609 + miR.603 + miR.369.3p + miR.376a* + miR.518c + miR.639 + miR.338 | 0,78 | 0,014921049 |
| miR.558 + miR.609 + miR.220aa + miR.376a* + miR.518c + miR.639 + miR.338 | 0,78 | 0,078540457 |
| miR.558 + miR.609 + miR.26a + miR.29a + miR.362 + miR.518c + miR.338 | 0,78 | 0,017131629 |
| miR.558 + miR.609 + miR.130a + miR.29a + miR.362 + miR.518c + miR.660 | 0,78 | 0,000338646 |
| miR.558 + miR.609 + miR.520f + miR.220aa + miR.26a + miR.29a + miR.518c | 0,78 | 0,011161417 |
| miR.558 + miR.609 + miR.603 + miR.520f + miR.220aa + miR.376a* + miR.518c | 0,78 | 0,026649299 |
| miR.558 + miR.609 + miR.603 + miR.220aa + miR.362 + miR.376a* + miR.518c | 0,78 | 0,013961948 |
| miR.558 + miR.609 + miR.603 + miR.520f + miR.130a + miR.376a* + miR.338 | 0,78 | 0,246690869 |
| miR.558 + miR.609 + miR.603 + miR.220aa + miR.376a* + miR.518c + miR.660 | 0,78 | 0,013150272 |
| miR.558 + miR.609 + miR.220aa + miR.376a* + miR.494 + miR.518c + miR.639 | 0,78 | 0,092408184 |
| miR.558 + miR.609 + miR.220aa + miR.519b + miR.26a + miR.29a + miR.518c | 0,78 | 0,013614183 |
| miR.558 + miR.609 + miR.603 + miR.220aa + miR.26a + miR.376a* + miR.518c | 0,78 | 0,01128397 |
| miR.609 + miR.603 + miR.520f + miR.130a + miR.26a + miR.376a* + miR.518c | 0,78 | 0,010920085 |
| miR.558 + miR.609 + miR.130a + miR.29a + miR.518c + miR.660 + miR.338 | 0,78 | 0,000125877 |
| miR.558 + miR.609 + miR.520f + miR.26a + miR.29a + miR.518c + miR.639 | 0,78 | 0,018969429 |
| miR.558 + miR.609 + miR.520f + miR.130a + miR.26a + miR.29a + miR.518c | 0,78 | 0,011746875 |
| miR.558 + miR.609 + miR.130a + miR.362 + miR.369.3p + miR.376a* + miR.518c | 0,78 | 0,037309484 |
| miR.558 + miR.609 + miR.220aa + miR.26a + miR.29a + miR.494 + miR.518c | 0,78 | 0,007507676 |
| miR.558 + miR.609 + miR.520f + miR.26a + miR.29a + miR.369.3p + miR.518c | 0,78 | 0,014342151 |
| miR.558 + miR.609 + miR.603 + miR.220aa + miR.376a* + miR.494 + miR.518c | 0,78 | 0,017097182 |
| miR.609 + miR.603 + miR.220aa + miR.376a* + miR.494 + miR.518c + miR.639 | 0,78 | 0,03325381 |
| miR.609 + miR.603 + miR.130a + miR.362 + miR.369.3p + miR.376a* + miR.518c | 0,78 | 0,009392167 |
| miR.558 + miR.609 + miR.29a + miR.362 + miR.369.3p + miR.376a* + miR.494 | 0,78 | 0,048829978 |
| miR.558 + miR.609 + miR.29a + miR.362 + miR.369.3p + miR.518c + miR.660 | 0,78 | 0,004400146 |
| miR.558 + miR.609 + miR.369.3p + miR.376a* + miR.494 + miR.518c + miR.639 | 0,78 | 0,163352928 |
| miR.558 + miR.609 + miR.220aa + miR.376a* + miR.518c + miR.639 + miR.660 | 0,78 | 0,103008562 |
| miR.558 + miR.609 + miR.220aa + miR.26a + miR.29a + miR.518c + miR.639 | 0,78 | 0,006804615 |
| miR.558 + miR.609 + miR.603 + miR.29a + miR.518c + miR.660 + miR.338 | 0,78 | 0,00223336 |
| miR.558 + miR.609 + miR.520f + miR.26a + miR.29a + miR.494 + miR.518c | 0,78 | 0,010475226 |
| miR.558 + miR.609 + miR.603 + miR.519b + miR.369.3p + miR.376a* + miR.518c | 0,78 | 0,035598786 |
| miR.558 + miR.609 + miR.220aa + miR.26a + miR.376a* + miR.518c + miR.639 | 0,78 | 0,083481811 |
| miR.558 + miR.609 + miR.26a + miR.29a + miR.369.3p + miR.518c + miR.338 | 0,78 | 0,014576318 |
| miR.558 + miR.609 + miR.603 + miR.520f + miR.369.3p + miR.376a* + miR.518c | 0,78 | 0,03197051 |
| miR.558 + miR.609 + miR.130a + miR.26a + miR.29a + miR.362 + miR.518c | 0,78 | 0,010372529 |
| miR.609 + miR.520f + miR.220aa + miR.130a + miR.376a* + miR.518c + miR.639 | 0,78 | 0,02410423 |
| miR.609 + miR.520f + miR.26a + miR.29a + miR.376a* + miR.494 + miR.338 | 0,78 | 0,056627184 |
| miR.558 + miR.609 + miR.603 + miR.520f + miR.519b + miR.29a + miR.518c | 0,78 | 0,048193832 |
| miR.558 + miR.609 + miR.603 + miR.29a + miR.362 + miR.518c + miR.660 | 0,78 | 0,004829453 |
| miR.609 + miR.603 + miR.520f + miR.519b + miR.130a + miR.376a* + miR.518c | 0,78 | 0,006763588 |
| miR.609 + miR.603 + miR.520f + miR.130a + miR.362 + miR.376a* + miR.518c | 0,78 | 0,005724349 |
| miR.558 + miR.609 + miR.220aa + miR.362 + miR.376a* + miR.518c + miR.639 | 0,78 | 0,065406188 |
| miR.609 + miR.603 + miR.520f + miR.130a + miR.376a* + miR.494 + miR.518c | 0,78 | 0,006763588 |
| miR.609 + miR.603 + miR.369.3p + miR.376a* + miR.494 + miR.518c + miR.639 | 0,78 | 0,020815213 |
| miR.609 + miR.603 + miR.520f + miR.130a + miR.376a* + miR.518c + miR.660 | 0,78 | 0,00649936 |
| miR.558 + miR.609 + miR.130a + miR.26a + miR.376a* + miR.518c + miR.639 | 0,78 | 0,115075349 |
| miR.609 + miR.603 + miR.130a + miR.369.3p + miR.376a* + miR.518c + miR.338 | 0,78 | 0,008293336 |
| miR.558 + miR.609 + miR.220aa + miR.519b + miR.376a* + miR.518c + miR.639 | 0,78 | 0,066085896 |
| miR.609 + miR.519b + miR.130a + miR.29a + miR.369.3p + miR.376a* + miR.518c | 0,78 | 0,033545182 |
| miR.609 + miR.603 + miR.520f + miR.376a* + miR.494 + miR.518c + miR.639 | 0,78 | 0,043081915 |
| miR.609 + miR.520f + miR.29a + miR.376a* + miR.494 + miR.639 + miR.338 | 0,78 | 0,105883144 |
| miR.558 + miR.609 + miR.603 + miR.369.3p + miR.376a* + miR.518c + miR.660 | 0,78 | 0,030516934 |
| miR.558 + miR.609 + miR.520f + miR.519b + miR.26a + miR.29a + miR.518c | 0,78 | 0,02547074 |
| miR.609 + miR.520f + miR.29a + miR.376a* + miR.494 + miR.660 + miR.338 | 0,78 | 0,1747453 |
| miR.609 + miR.519b + miR.29a + miR.369.3p + miR.376a* + miR.518c + miR.660 | 0,78 | 0,04021298 |
| miR.558 + miR.609 + miR.130a + miR.369.3p + miR.376a* + miR.518c + miR.338 | 0,78 | 0,079708523 |
| miR.609 + miR.603 + miR.520f + miR.29a + miR.369.3p + miR.376a* + miR.494 | 0,78 | 0,077941718 |
| miR.558 + miR.609 + miR.603 + miR.29a + miR.362 + miR.369.3p + miR.376a* | 0,78 | 0,027716639 |
| miR.558 + miR.609 + miR.603 + miR.520f + miR.130a + miR.29a + miR.518c | 0,78 | 0,021815158 |
| miR.558 + miR.609 + miR.520f + miR.519b + miR.130a + miR.29a + miR.518c | 0,78 | 0,02772778 |
| miR.558 + miR.609 + miR.26a + miR.29a + miR.362 + miR.494 + miR.518c | 0,78 | 0,009084869 |
| miR.609 + miR.519b + miR.29a + miR.369.3p + miR.376a* + miR.518c + miR.338 | 0,78 | 0,071009407 |
| miR.558 + miR.609 + miR.130a + miR.26a + miR.29a + miR.369.3p + miR.518c | 0,78 | 0,006328575 |
| miR.558 + miR.609 + miR.520f + miR.29a + miR.362 + miR.518c + miR.338 | 0,78 | 0,014421096 |
| miR.558 + miR.609 + miR.130a + miR.376a* + miR.518c + miR.639 + miR.338 | 0,78 | 0,108959907 |
| miR.558 + miR.609 + miR.603 + miR.520f + miR.29a + miR.362 + miR.518c | 0,78 | 0,028510686 |
| miR.558 + miR.609 + miR.26a + miR.29a + miR.369.3p + miR.376a* + miR.494 | 0,78 | 0,066158718 |
| miR.558 + miR.609 + miR.519b + miR.26a + miR.29a + miR.362 + miR.518c | 0,78 | 0,008922316 |
| miR.558 + miR.609 + miR.520f + miR.130a + miR.29a + miR.362 + miR.518c | 0,78 | 0,016751707 |
| miR.558 + miR.609 + miR.603 + miR.520f + miR.26a + miR.376a* + miR.518c | 0,78 | 0,016754078 |
| miR.558 + miR.609 + miR.130a + miR.26a + miR.29a + miR.518c + miR.639 | 0,78 | 0,009978795 |
| miR.609 + miR.29a + miR.369.3p + miR.376a* + miR.494 + miR.518c + miR.639 | 0,78 | 0,025322603 |
| miR.558 + miR.609 + miR.520f + miR.220aa + miR.519b + miR.29a + miR.518c | 0,78 | 0,05345257 |
| miR.558 + miR.609 + miR.520f + miR.130a + miR.29a + miR.518c + miR.338 | 0,78 | 0,016788844 |
| miR.558 + miR.609 + miR.520f + miR.519b + miR.29a + miR.362 + miR.518c | 0,78 | 0,037161741 |
| miR.558 + miR.609 + miR.220aa + miR.29a + miR.369.3p + miR.518c + miR.660 | 0,78 | 0,002872986 |
| miR.558 + miR.609 + miR.26a + miR.29a + miR.369.3p + miR.518c + miR.639 | 0,78 | 0,012857981 |
| miR.558 + miR.609 + miR.520f + miR.130a + miR.29a + miR.518c + miR.639 | 0,78 | 0,020261972 |
| miR.558 + miR.609 + miR.26a + miR.29a + miR.362 + miR.369.3p + miR.376a* | 0,78 | 0,035205989 |
| miR.558 + miR.609 + miR.26a + miR.29a + miR.362 + miR.369.3p + miR.518c | 0,78 | 0,009225922 |
| miR.558 + miR.609 + miR.26a + miR.29a + miR.369.3p + miR.494 + miR.518c | 0,78 | 0,008130434 |
| miR.558 + miR.609 + miR.603 + miR.29a + miR.369.3p + miR.376a* + miR.494 | 0,78 | 0,012928909 |
| miR.609 + miR.603 + miR.519b + miR.369.3p + miR.376a* + miR.518c + miR.639 | 0,78 | 0,0083191 |
| miR.558 + miR.609 + miR.520f + miR.519b + miR.29a + miR.518c + miR.338 | 0,78 | 0,024292032 |
| miR.558 + miR.609 + miR.220aa + miR.26a + miR.29a + miR.369.3p + miR.376a* | 0,78 | 0,022371809 |
| miR.558 + miR.609 + miR.130a + miR.362 + miR.376a* + miR.518c + miR.639 | 0,78 | 0,034122028 |
| miR.558 + miR.609 + miR.130a + miR.26a + miR.29a + miR.518c + miR.338 | 0,78 | 0,01379703 |
| miR.558 + miR.609 + miR.603 + miR.520f + miR.220aa + miR.29a + miR.518c | 0,78 | 0,02836698 |
| miR.558 + miR.609 + miR.520f + miR.362 + miR.518c + miR.639 + miR.660 | 0,78 | 0,100024182 |
| miR.558 + miR.609 + miR.603 + miR.520f + miR.29a + miR.518c + miR.338 | 0,78 | 0,016907051 |
| miR.558 + miR.609 + miR.26a + miR.29a + miR.518c + miR.639 + miR.338 | 0,78 | 0,013084907 |
| miR.558 + miR.609 + miR.520f + miR.29a + miR.362 + miR.494 + miR.518c | 0,78 | 0,011779808 |
| miR.558 + miR.609 + miR.130a + miR.29a + miR.369.3p + miR.518c + miR.660 | 0,78 | 0,000728457 |
| miR.558 + miR.609 + miR.519b + miR.26a + miR.29a + miR.369.3p + miR.518c | 0,78 | 0,008975345 |
| miR.609 + miR.603 + miR.130a + miR.26a + miR.369.3p + miR.376a* + miR.518c | 0,78 | 0,006730499 |
| miR.558 + miR.609 + miR.520f + miR.220aa + miR.29a + miR.518c + miR.338 | 0,78 | 0,010603664 |
| miR.558 + miR.609 + miR.220aa + miR.29a + miR.362 + miR.369.3p + miR.376a* | 0,78 | 0,021540479 |
| miR.558 + miR.609 + miR.603 + miR.520f + miR.29a + miR.494 + miR.518c | 0,78 | 0,020102222 |
| miR.558 + miR.609 + miR.520f + miR.29a + miR.518c + miR.639 + miR.338 | 0,78 | 0,017021124 |
| miR.609 + miR.520f + miR.220aa + miR.130a + miR.376a* + miR.518c + miR.338 | 0,78 | 0,04349407 |
| miR.558 + miR.609 + miR.130a + miR.29a + miR.494 + miR.518c + miR.660 | 0,78 | 0,001064546 |
| miR.558 + miR.609 + miR.520f + miR.29a + miR.362 + miR.518c + miR.639 | 0,78 | 0,015233262 |
| miR.558 + miR.609 + miR.520f + miR.130a + miR.26a + miR.376a* + miR.338 | 0,78 | 0,243410022 |
| miR.609 + miR.520f + miR.29a + miR.362 + miR.376a* + miR.494 + miR.660 | 0,78 | 0,113681762 |
| miR.558 + miR.609 + miR.603 + miR.26a + miR.369.3p + miR.376a* + miR.518c | 0,78 | 0,04241887 |
| miR.558 + miR.609 + miR.519b + miR.130a + miR.29a + miR.518c + miR.660 | 0,78 | 0,000490446 |
| miR.558 + miR.609 + miR.130a + miR.26a + miR.369.3p + miR.376a* + miR.518c | 0,78 | 0,058994582 |
| miR.558 + miR.609 + miR.520f + miR.29a + miR.494 + miR.518c + miR.338 | 0,78 | 0,01513134 |
| miR.609 + miR.520f + miR.220aa + miR.29a + miR.376a* + miR.494 + miR.338 | 0,78 | 0,063917787 |
| miR.558 + miR.609 + miR.603 + miR.220aa + miR.29a + miR.518c + miR.338 | 0,78 | 0,007654893 |
| miR.558 + miR.609 + miR.603 + miR.520f + miR.376a* + miR.518c + miR.338 | 0,78 | 0,090850124 |
| miR.558 + miR.609 + miR.220aa + miR.519b + miR.29a + miR.518c + miR.660 | 0,78 | 0,004246216 |
| miR.558 + miR.609 + miR.519b + miR.130a + miR.376a* + miR.518c + miR.639 | 0,78 | 0,05677794 |
| miR.558 + miR.609 + miR.520f + miR.220aa + miR.29a + miR.362 + miR.518c | 0,78 | 0,018548067 |
| miR.609 + miR.29a + miR.362 + miR.369.3p + miR.376a* + miR.494 + miR.518c | 0,78 | 0,074783771 |
| miR.558 + miR.609 + miR.520f + miR.29a + miR.369.3p + miR.518c + miR.338 | 0,78 | 0,012831826 |
| miR.558 + miR.609 + miR.603 + miR.369.3p + miR.376a* + miR.494 + miR.518c | 0,78 | 0,053291295 |

**Table 8: clusters of 8 miRNAs**

| **Cluster of 8 miRs** | **c_tau level** | **pv compared to top signature miR.558 + miR.609 + miR.603 + miR.220a + miR.29a + miR.369.3p+ miR.376a *+ miR.518c** |
|---|---|---|
| miR.558 + miR.609 + miR.603 + miR.220a + miR.29a + miR.369.3p + miR.376a* + miR.518c | 0,81 | NaN |
| miR.558 + miR.609 + miR.603 + miR.220a + miR.26a + miR.29a + miR.376a* + miR.518c | 0,81 | 0,268588527 |
| miR.558 + miR.609 + miR.220a + miR.26a + miR.29a + miR.369.3p + miR.376a* + miR.518c | 0,81 | 0,213114289 |
| miR.558 + miR.609 + miR.220a + miR.29a + miR.369.3p + miR.376a* + miR.518c + miR.660 | 0,81 | 0,182815818 |
| miR.558 + miR.609 + miR.603 + miR.220a + miR.519b + miR.29a + miR.376a* + miR.518c | 0,81 | 0,289192622 |
| miR.558 + miR.609 + miR.520f + miR.220a + miR.29a + miR.369.3p + miR.376a* + miR.518c | 0,81 | 0,361357455 |
| miR.558 + miR.609 + miR.603 + miR.220a + miR.29a + miR.362 + miR.376a* + miR.518c | 0,81 | 0,077393148 |
| miR.558 + miR.609 + miR.603 + miR.520f + miR.220a + miR.29a + miR.376a* + miR.518c | 0,81 | 0,122807573 |
| miR.558 + miR.609 + miR.603 + miR.520f + miR.29a + miR.369.3p + miR.376a* + miR.518c | 0,81 | 0,272458791 |
| miR.558 + miR.609 + miR.603 + miR.26a + miR.29a + miR.369.3p + miR.376a* + miR.518c | 0,81 | 0,431946111 |
| miR.558 + miR.609 + miR.520f + miR.29a + miR.369.3p + miR.376a* + miR.518c + miR.338 | 0,81 | 0,412120313 |
| miR.558 + miR.609 + miR.520f + miR.220a + miR.29a + miR.362 + miR.376a* + miR.518c | 0,8 | 0,44685223 |
| miR.558 + miR.609 + miR.603 + miR.220a + miR.29a + miR.376a* + miR.518c + miR.660 | 0,8 | 0,055136323 |
| miR.558 + miR.609 + miR.520f + miR.220a + miR.29a + miR.376a* + miR.518c + miR.660 | 0,8 | 0,313798592 |
| miR.558 + miR.609 + miR.520f + miR.29a + miR.376a* + miR.518c + miR.660 + miR.338 | 0,8 | 0,489039597 |
| miR.558 + miR.609 + miR.520f + miR.29a + miR.369.3p + miR.376a* + miR.518c + miR.660 | 0,8 | 0,393346155 |
| miR.558 + miR.609 + miR.520f + miR.220a + miR.29a + miR.376a* + miR.518c + miR.338 | 0,8 | 0,482514973 |
| miR.558 + miR.609 + miR.220a + miR.130a + miR.29a + miR.369.3p + miR.376a* + miR.518c | 0,8 | 0,228451591 |
| miR.558 + miR.609 + miR.220a + miR.26a + miR.29a + miR.362 + miR.376a* + miR.518c | 0,8 | 0,350771488 |
| miR.558 + miR.609 + miR.220a + miR.26a + miR.29a + miR.376a* + miR.494 + miR.518c | 0,8 | 0,323451407 |
| miR.558 + miR.609 + miR.220a + miR.29a + miR.362 + miR.369.3p + miR.376a* + miR.518c | 0,8 | 0,371229727 |
| miR.558 + miR.609 + miR.220a + miR.29a + miR.369.3p + miR.376a* + miR.494 + miR.518c | 0,8 | 0,406008638 |
| miR.558 + miR.609 + miR.220a + miR.519b + miR.29a + miR.369.3p + miR.376a* + miR.518c | 0,8 | 0,292171386 |
| miR.558 + miR.609 + miR.220a + miR.29a + miR.369.3p + miR.376a* + miR.518c + miR.639 | 0,8 | 0,367581417 |
| miR.558 + miR.609 + miR.220a + miR.29a + miR.369.3p + miR.376a* + miR.518c + miR.338 | 0,8 | 0,231792794 |
| miR.558 + miR.609 + miR.520f + miR.220a + miR.29a + miR.376a* + miR.494 + miR.518c | 0,8 | 0,29026386 |
| miR.558 + miR.609 + miR.520f + miR.29a + miR.369.3p + miR.376a* + miR.518c + miR.639 | 0,8 | 0,294789553 |
| miR.558 + miR.609 + miR.220a + miR.26a + miR.29a + miR.376a* + miR.518c + miR.660 | 0,8 | 0,293352698 |
| miR.558 + miR.609 + miR.520f + miR.220a + miR.26a + miR.29a + miR.376a* + miR.518c | 0,8 | 0,414989303 |
| miR.558 + miR.609 + miR.603 + miR.220a + miR.29a + miR.376a* + miR.518c + miR.639 | 0,8 | 0,018528237 |
| miR.558 + miR.609 + miR.603 + miR.520f + miR.29a + miR.376a* + miR.518c + miR.338 | 0,8 | 0,319545984 |
| miR.558 + miR.609 + miR.520f + miR.29a + miR.369.3p + miR.376a* + miR.494 + miR.518c | 0,8 | 0,286586866 |
| miR.558 + miR.609 + miR.603 + miR.220a + miR.130a + miR.29a + miR.376a* + miR.518c | 0,8 | 0,019571018 |
| miR.558 + miR.609 + miR.603 + miR.220a + miR.29a + miR.376a* + miR.518c + miR.338 | 0,8 | 0,031011931 |
| miR.558 + miR.609 + miR.603 + miR.520f + miR.29a + miR.376a* + miR.494 + miR.518c | 0,8 | 0,119310203 |
| miR.558 + miR.609 + miR.603 + miR.520f + miR.29a + miR.362 + miR.376a* + miR.518c | 0,8 | 0,220773607 |
| miR.558 + miR.609 + miR.603 + miR.520f + miR.29a + miR.376a* + miR.518c + miR.639 | 0,8 | 0,111018106 |
| miR.558 + miR.609 + miR.520f + miR.26a + miR.29a + miR.369.3p + miR.376a* + miR.518c | 0,8 | 0,323312997 |
| miR.558 + miR.609 + miR.520f + miR.220a + miR.519b + miR.29a + miR.376a* + miR.518c | 0,8 | 0,459439323 |
| miR.558 + miR.609 + miR.520f + miR.220a + miR.130a + miR.29a + miR.376a* + miR.518c | 0,8 | 0,317028691 |
| miR.558 + miR.609 + miR.520f + miR.220a + miR.29a + miR.376a* + miR.518c + miR.639 | 0,8 | 0,313525665 |
| miR.609 + miR.603 + miR.220a + miR.29a + miR.369.3p + miR.376a* + miR.518c + miR.660 | 0,8 | 0,150538532 |
| miR.558 + miR.609 + miR.520f + miR.29a + miR.376a* + miR.494 + miR.518c + miR.338 | 0,8 | 0,2666813 |
| miR.558 + miR.609 + miR.603 + miR.520f + miR.29a + miR.376a* + miR.518c + miR.660 | 0,8 | 0,166396868 |
| miR.558 + miR.609 + miR.520f + miR.26a + miR.29a + miR.376a* + miR.518c + miR.660 | 0,8 | 0,192635079 |
| miR.558 + miR.609 + miR.520f + miR.29a + miR.376a* + miR.518c + miR.639 + miR.660 | 0,8 | 0,326979926 |
| miR.558 + miR.609 + miR.603 + miR.519b + miR.29a + miR.369.3p + miR.376a* + miR.518c | 0,8 | 0,184541446 |
| miR.558 + miR.609 + miR.520f + miR.29a + miR.362 + miR.376a* + miR.518c + miR.660 | 0,8 | 0,20424416 |
| miR.558 + miR.609 + miR.520f + miR.29a + miR.376a* + miR.518c + miR.639 + miR.338 | 0,8 | 0,312699347 |
| miR.558 + miR.609 + miR.603 + miR.29a + miR.369.3p + miR.376a* + miR.518c + miR.639 | 0,8 | 0,18500349 |
| miR.558 + miR.609 + miR.603 + miR.29a + miR.369.3p + miR.376a* + miR.518c + miR.660 | 0,8 | 0,121068728 |
| miR.558 + miR.609 + miR.520f + miR.29a + miR.362 + miR.369.3p + miR.376a* + miR.518c | 0,8 | 0,289544573 |
| miR.558 + miR.609 + miR.520f + miR.29a + miR.362 + miR.376a* + miR.518c + miR.338 | 0,8 | 0,496530929 |
| miR.558 + miR.609 + miR.603 + miR.220a + miR.29a + miR.376a* + miR.494 + miR.518c | 0,8 | 0,00891388 |
| miR.558 + miR.609 + miR.603 + miR.29a + miR.362 + miR.369.3p + miR.376a* + miR.518c | 0,8 | 0,200931672 |
| miR.558 + miR.609 + miR.220a + miR.26a + miR.29a + miR.376a* + miR.518c + miR.338 | 0,8 | 0,361017321 |
| miR.558 + miR.609 + miR.520f + miR.519b + miR.29a + miR.376a* + miR.518c + miR.660 | 0,8 | 0,206909861 |
| miR.558 + miR.609 + miR.603 + miR.130a + miR.29a + miR.369.3p + miR.376a* + miR.518c | 0,8 | 0,148949042 |
| miR.558 + miR.609 + miR.520f + miR.519b + miR.29a + miR.369.3p + miR.376a* + miR.518c | 0,8 | 0,300806891 |
| miR.558 + miR.609 + miR.520f + miR.130a + miR.29a + miR.369.3p + miR.376a* + miR.518c | 0,8 | 0,262991213 |
| miR.558 + miR.609 + miR.520f + miR.29a + miR.362 + miR.376a* + miR.518c + miR.639 | 0,8 | 0,303472164 |
| miR.558 + miR.609 + miR.220a + miR.130a + miR.29a + miR.362 + miR.376a* + miR.518c | 0,8 | 0,263995365 |
| miR.558 + miR.609 + miR.520f + miR.29a + miR.376a* + miR.494 + miR.518c + miR.660 | 0,8 | 0,240538444 |
| miR.558 + miR.609 + miR.220a + miR.519b + miR.29a + miR.362 + miR.376a* + miR.518c | 0,8 | 0,287179997 |
| miR.558 + miR.609 + miR.220a + miR.130a + miR.26a + miR.29a + miR.376a* + miR.518c | 0,8 | 0,252584893 |
| miR.558 + miR.609 + miR.220a + miR.26a + miR.29a + miR.376a* + miR.518c + miR.639 | 0,8 | 0,2300802 |
| miR.558 + miR.609 + miR.220a + miR.519b + miR.26a + miR.29a + miR.376a* + miR.518c | 0,8 | 0,212034229 |
| miR.558 + miR.609 + miR.603 + miR.520f + miR.519b + miR.29a + miR.376a* + miR.518c | 0,8 | 0,150034774 |
| miR.558 + miR.609 + miR.220a + miR.29a + miR.362 + miR.376a* + miR.518c + miR.338 | 0,8 | 0,18806259 |
| miR.558 + miR.609 + miR.520f + miR.29a + miR.376a* + miR.494 + miR.518c + miR.639 | 0,8 | 0,16791359 |
| miR.558 + miR.609 + miR.220a + miR.29a + miR.362 + miR.376a* + miR.518c + miR.639 | 0,8 | 0,187103112 |
| miR.558 + miR.609 + miR.603 + miR.520f + miR.130a + miR.29a + miR.376a* + miR.518c | 0,8 | 0,078398975 |
| miR.558 + miR.609 + miR.603 + miR.520f + miR.26a + miR.29a + miR.376a* + miR.518c | 0,8 | 0,140471281 |
| miR.558 + miR.609 + miR.520f + miR.29a + miR.362 + miR.376a* + miR.494 + miR.518c | 0,8 | 0,329819355 |
| miR.558 + miR.609 + miR.26a + miR.29a + miR.369.3p + miR.376a* + miR.518c + miR.338 | 0,8 | 0,449625169 |
| miR.558 + miR.609 + miR.603 + miR.29a + miR.369.3p + miR.376a* + miR.518c + miR.338 | 0,8 | 0,335759964 |
| miR.558 + miR.609 + miR.220a + miR.29a + miR.376a* + miR.494 + miR.518c + miR.660 | 0,8 | 0,267344726 |
| miR.558 + miR.609 + miR.520f + miR.130a + miR.29a + miR.376a* + miR.518c + miR.338 | 0,8 | 0,308053158 |
| miR.558 + miR.609 + miR.26a + miR.29a + miR.369.3p + miR.376a* + miR.494 + miR.518c | 0,8 | 0,207927956 |
| miR.558 + miR.609 + miR.26a + miR.29a + miR.369.3p + miR.376a* + miR.518c + miR.639 | 0,8 | 0,186741599 |
| miR.558 + miR.609 + miR.220a + miR.519b + miR.29a + miR.376a* + miR.518c + miR.660 | 0,8 | 0,254116495 |
| miR.558 + miR.609 + miR.603 + miR.29a + miR.369.3p + miR.376a* + miR.494 + miR.518c | 0,8 | 0,171531707 |
| miR.558 + miR.609 + miR.220a + miR.29a + miR.376a* + miR.518c + miR.639 + miR.660 | 0,8 | 0,248205674 |
| miR.558 + miR.609 + miR.220a + miR.130a + miR.29a + miR.376a* + miR.518c + miR.660 | 0,8 | 0,2530454 |
| miR.558 + miR.609 + miR.520f + miR.26a + miR.29a + miR.376a* + miR.518c + miR.338 | 0,8 | 0,37924681 |
| miR.558 + miR.609 + miR.520f + miR.519b + miR.29a + miR.376a* + miR.518c + miR.338 | 0,8 | 0,454251801 |
| miR.558 + miR.609 + miR.220a + miR.29a + miR.362 + miR.376a* + miR.518c + miR.660 | 0,8 | 0,214624308 |
| miR.558 + miR.609 + miR.220a + miR.29a + miR.376a* + miR.518c + miR.660 + miR.338 | 0,8 | 0,245944937 |
| miR.558 + miR.609 + miR.220a + miR.130a + miR.29a + miR.376a* + miR.494 + miR.518c | 0,8 | 0,256722473 |
| miR.609 + miR.603 + miR.220a + miR.26a + miR.29a + miR.369.3p + miR.376a* + miR.518c | 0,8 | 0,185061761 |
| miR.558 + miR.609 + miR.603 + miR.26a + miR.29a + miR.376a* + miR.518c + miR.639 | 0,8 | 0,211280496 |
| miR.558 + miR.609 + miR.520f + miR.130a + miR.29a + miR.376a* + miR.518c + miR.639 | 0,8 | 0,176391392 |
| miR.609 + miR.603 + miR.220a + miR.29a + miR.369.3p + miR.376a* + miR.494 + miR.518c | 0,8 | 0,261711358 |
| miR.558 + miR.609 + miR.520f + miR.519b + miR.29a + miR.376a* + miR.494 + miR.518c | 0,8 | 0,239641338 |
| miR.558 + miR.609 + miR.220a + miR.519b + miR.130a + miR.29a + miR.376a* + miR.518c | 0,8 | 0,266135049 |
| miR.558 + miR.609 + miR.520f + miR.130a + miR.29a + miR.376a* + miR.518c + miR.660 | 0,8 | 0,198874815 |
| miR.558 + miR.609 + miR.220a + miR.130a + miR.29a + miR.376a* + miR.518c + miR.338 | 0,8 | 0,238065786 |
| miR.558 + miR.609 + miR.520f + miR.130a + miR.29a + miR.362 + miR.376a* + miR.518c | 0,8 | 0,295159139 |
| miR.558 + miR.609 + miR.603 + miR.26a + miR.29a + miR.376a* + miR.494 + miR.518c | 0,8 | 0,12140431 |
| miR.558 + miR.609 + miR.520f + miR.519b + miR.29a + miR.362 + miR.376a* + miR.518c | 0,8 | 0,320182911 |
| miR.558 + miR.609 + miR.220a + miR.519b + miR.29a + miR.376a* + miR.518c + miR.639 | 0,8 | 0,279758171 |
| miR.558 + miR.609 + miR.220a + miR.130a + miR.29a + miR.376a* + miR.518c + miR.639 | 0,8 | 0,249205161 |
| miR.558 + miR.609 + miR.520f + miR.26a + miR.29a + miR.362 + miR.376a* + miR.518c | 0,8 | 0,300314786 |
| miR.558 + miR.609 + miR.220a + miR.519b + miR.29a + miR.376a* + miR.518c + miR.338 | 0,8 | 0,340189429 |
| miR.558 + miR.609 + miR.520f + miR.26a + miR.29a + miR.376a* + miR.518c + miR.639 | 0,8 | 0,200668383 |
| miR.558 + miR.609 + miR.520f + miR.130a + miR.29a + miR.376a* + miR.494 + miR.518c | 0,8 | 0,225833391 |
| miR.558 + miR.609 + miR.26a + miR.29a + miR.369.3p + miR.376a* + miR.518c + miR.660 | 0,8 | 0,268694626 |
| miR.558 + miR.609 + miR.520f + miR.26a + miR.29a + miR.376a* + miR.494 + miR.518c | 0,8 | 0,221197278 |
| miR.558 + miR.609 + miR.519b + miR.26a + miR.29a + miR.369.3p + miR.376a* + miR.518c | 0,8 | 0,238847644 |
| miR.558 + miR.609 + miR.220a + miR.29a + miR.376a* + miR.518c + miR.639 + miR.338 | 0,8 | 0,196005317 |
| miR.558 + miR.609 + miR.520f + miR.519b + miR.29a + miR.376a* + miR.518c + miR.639 | 0,8 | 0,19084843 |
| miR.558 + miR.609 + miR.220a + miR.29a + miR.376a* + miR.494 + miR.518c + miR.639 | 0,8 | 0,203579685 |
| miR.609 + miR.603 + miR.520f + miR.220a + miR.29a + miR.369.3p + miR.376a* + miR.518c | 0,8 | 0,228865741 |
| miR.558 + miR.609 + miR.26a + miR.29a + miR.362 + miR.369.3p + miR.376a* + miR.518c | 0,8 | 0,202072601 |
| miR.558 + miR.609 + miR.220a + miR.29a + miR.376a* + miR.494 + miR.518c + miR.338 | 0,8 | 0,175352497 |
| miR.558 + miR.609 + miR.603 + miR.26a + miR.29a + miR.376a* + miR.518c + miR.338 | 0,8 | 0,199674652 |
| miR.558 + miR.609 + miR.29a + miR.362 + miR.369.3p + miR.376a* + miR.518c + miR.660 | 0,8 | 0,420367935 |
| miR.558 + miR.609 + miR.130a + miR.26a + miR.29a + miR.369.3p + miR.376a* + miR.518c | 0,8 | 0,328716887 |
| miR.609 + miR.603 + miR.220a + miR.519b + miR.29a + miR.369.3p + miR.376a* + miR.518c | 0,8 | 0,271684254 |
| miR.558 + miR.609 + miR.520f + miR.130a + miR.26a + miR.29a + miR.376a* + miR.518c | 0,8 | 0,237214954 |
| miR.609 + miR.603 + miR.220a + miR.29a + miR.362 + miR.369.3p + miR.376a* + miR.518c | 0,8 | 0,087793914 |
| miR.609 + miR.603 + miR.220a + miR.29a + miR.369.3p + miR.376a* + miR.518c + miR.639 | 0,8 | 0,177717277 |
| miR.558 + miR.609 + miR.130a + miR.29a + miR.369.3p + miR.376a* + miR.518c + miR.639 | 0,8 | 0,165100031 |
| miR.558 + miR.609 + miR.520f + miR.519b + miR.130a + miR.29a + miR.376a* + miR.518c | 0,8 | 0,236748396 |
| miR.558 + miR.609 + miR.520f + miR.519b + miR.26a + miR.29a + miR.376a* + miR.518c | 0,8 | 0,243362019 |
| miR.609 + miR.603 + miR.220a + miR.29a + miR.369.3p + miR.376a* + miR.518c + miR.338 | 0,8 | 0,056920249 |
| miR.558 + miR.609 + miR.130a + miR.29a + miR.369.3p + miR.376a* + miR.494 + miR.518c | 0,8 | 0,138873294 |
| miR.558 + miR.609 + miR.220a + miR.29a + miR.362 + miR.376a* + miR.494 + miR.518c | 0,8 | 0,261772468 |
| miR.558 + miR.609 + miR.220a + miR.519b + miR.29a + miR.376a* + miR.494 + miR.518c | 0,8 | 0,277820043 |
| miR.558 + miR.609 + miR.603 + miR.26a + miR.29a + miR.362 + miR.376a* + miR.518c | 0,8 | 0,20891331 |
| miR.558 + miR.609 + miR.603 + miR.29a + miR.376a* + miR.518c + miR.639 + miR.660 | 0,8 | 0,135181446 |
| miR.609 + miR.603 + miR.220a + miR.130a + miR.29a + miR.369.3p + miR.376a* + miR.518c | 0,8 | 0,128158679 |
| miR.558 + miR.609 + miR.603 + miR.130a + miR.29a + miR.376a* + miR.494 + miR.518c | 0,8 | 0,056880534 |
| miR.558 + miR.609 + miR.603 + miR.130a + miR.26a + miR.29a + miR.376a* + miR.518c | 0,8 | 0,123855549 |
| miR.558 + miR.609 + miR.130a + miR.29a + miR.376a* + miR.494 + miR.518c + miR.639 | 0,8 | 0,118227692 |
| miR.558 + miR.609 + miR.603 + miR.26a + miR.29a + miR.376a* + miR.518c + miR.660 | 0,8 | 0,154474098 |
| miR.558 + miR.609 + miR.603 + miR.130a + miR.29a + miR.376a* + miR.518c + miR.639 | 0,8 | 0,062202459 |
| miR.558 + miR.609 + miR.603 + miR.519b + miR.26a + miR.29a + miR.376a* + miR.518c | 0,8 | 0,174325893 |
| miR.558 + miR.609 + miR.603 + miR.29a + miR.376a* + miR.494 + miR.518c + miR.639 | 0,8 | 0,034840802 |
| miR.558 + miR.609 + miR.29a + miR.369.3p + miR.376a* + miR.518c + miR.639+miR.338 | 0,8 | 0,280813829 |
| miR.558 + miR.609 + miR.26a + miR.29a + miR.376a* + miR.518c + miR.639 + miR.338 | 0,8 | 0,1616683 |
| miR.558 + miR.609 + miR.603 + miR.29a + miR.376a* + miR.518c + miR.639 + miR.338 | 0,8 | 0,056465371 |
| miR.558 + miR.609 + miR.603 + miR.29a + miR.362 + miR.376a* + miR.518c + miR.639 | 0,8 | 0,097576451 |
| miR.558 + miR.609 + miR.130a + miR.29a + miR.369.3p + miR.376a* + miR.518c + miR.338 | 0,8 | 0,195306685 |
| miR.609 + miR.603 + miR.520f + miR.220a + miR.29a + miR.362 + miR.376a* + miR.518c | 0,8 | 0,298103073 |
| miR.558 + miR.609 + miR.26a + miR.29a + miR.362 + miR.376a* + miR.518c + miR.639 | 0,8 | 0,11359095 |
| miR.558 + miR.609 + miR.29a + miR.369.3p + miR.376a* + miR.518c + miR.639 + miR.660 | 0,8 | 0,138043804 |
| miR.558 + miR.609 + miR.519b + miR.26a + miR.29a + miR.376a* + miR.518c + miR.639 | 0,8 | 0,097424408 |
| miR.558 + miR.609 + miR.29a + miR.369.3p + miR.376a* + miR.494 + miR.518c + miR.639 | 0,8 | 0,1200447 73 |
| miR.609 + miR.603 + miR.520f + miR.29a + miR.369.3p + miR.376a* + miR.518c + miR.338 | 0,8 | 0,465334345 |
| miR.558 + miR.609 + miR.603 + miR.519b + miR.29a + miR.376a* + miR.518c + miR.639 | 0,8 | 0,121362751 |
| miR.609 + miR.603 + miR.520f + miR.29a + miR.369.3p + miR.376a* + miR.494 + miR.518c | 0,8 | 0,137329361 |
| miR.558 + miR.609 + miR.603 + miR.29a + miR.376a* + miR.494 + miR.518c + miR.660 | 0,8 | 0,022947512 |
| miR.558 + miR.609 + miR.130a + miR.29a + miR.369.3p + miR.376a* + miR.518c + miR.660 | 0,8 | 0,127014266 |
| miR.558 + miR.609 + miR.603 + miR.519b + miR.29a + miR.376a* + miR.494 + miR.518c | 0,8 | 0,235217454 |
| miR.558 + miR.609 + miR.130a + miR.26a + miR.29a + miR.376a* + miR.518c + miR.639 | 0,8 | 0,068427243 |
| miR.558 + miR.609 + miR.603 + miR.519b + miR.29a + miR.362 + miR.376a* + miR.518c | 0,8 | 0,109812067 |
| miR.558 + miR.609 + miR.603 + miR.29a + miR.376a* + miR.518c + miR.660 + miR.338 | 0,8 | 0,028202923 |
| miR.558 + miR.609 + miR.519b + miR.29a + miR.369.3p + miR.376a* + miR.518c + miR.660 | 0,8 | 0,082284699 |
| miR.558 + miR.609 + miR.130a + miR.29a + miR.362 + miR.369.3p + miR.376a* + miR.518c | 0,8 | 0,120164496 |
| miR.558 + miR.609 + miR.29a + miR.362 + miR.369.3p + miR.376a* + miR.518c + miR.639 | 0,8 | 0,186922436 |
| miR.558 + miR.609 + miR.603 + miR.130a + miR.29a + miR.362 + miR.376a* + miR.518c | 0,8 | 0,029305309 |
| miR.558 + miR.609 + miR.603 + miR.29a + miR.362 + miR.376a* + miR.518c + miR.338 | 0,8 | 0,031276981 |
| miR.558 + miR.609 + miR.603 + miR.519b + miR.29a + miR.376a* + miR.518c + miR.338 | 0,8 | 0,140168957 |
| miR.558 + miR.609 + miR.519b + miR.29a + miR.369.3p + miR.376a* + miR.518c + miR.639 | 0,8 | 0,104607901 |
| miR.558 + miR.609 + miR.519b + miR.130a + miR.29a + miR.369.3p + miR.376a* + miR.518c | 0,8 | 0,069192656 |
| miR.558 + miR.609 + miR.603 + miR.519b + miR.29a + miR.376a* + miR.518c + miR.660 | 0,8 | 0,085445288 |
| miR.609 + miR.603 + miR.520f + miR.220a + miR.29a + miR.376a* + miR.494 + miR.518c | 0,8 | 0,178980602 |
| miR.558 + miR.609 + miR.603 + miR.130a + miR.29a + miR.376a* + miR.518c + miR.338 | 0,8 | 0,015359888 |
| miR.558 + miR.609 + miR.603 + miR.29a + miR.362 + miR.376a* + miR.518c + miR.660 | 0,8 | 0,015195308 |
| miR.609 + miR.520f + miR.220a + miR.29a + miR.369.3p + miR.376a* + miR.518c + miR.660 | 0,8 | 0,435898894 |
| miR.609 + miR.603 + miR.520f + miR.220a + miR.29a + miR.376a* + miR.518c + miR.660 | 0,8 | 0,25544504 |
| miR.609 + miR.603 + miR.220a + miR.519b + miR.29a + miR.376a* + miR.494 + miR.518c | 0,8 | 0,079935193 |
| miR.558 + miR.609 + miR.603 + miR.130a + miR.29a + miR.376a* + miR.518c + miR.660 | 0,8 | 0,026025415 |
| miR.558 + miR.609 + miR.603 + miR.29a + miR.376a* + miR.494 + miR.518c + miR.338 | 0,8 | 0,035055003 |
| miR.558 + miR.609 + miR.603 + miR.519b + miR.130a + miR.29a + miR.376a* + miR.518c | 0,8 | 0,089871509 |
| miR.558 + miR.609 + miR.519b + miR.29a + miR.362 + miR.369.3p + miR.376a* + miR.518c | 0,8 | 0,103014709 |
| miR.609 + miR.603 + miR.220a + miR.26a + miR.29a + miR.376a* + miR.494 + miR.518c | 0,8 | 0,067719009 |
| miR.558 + miR.609 + miR.130a + miR.29a + miR.376a* + miR.518c + miR.639 + miR.660 | 0,8 | 0,143256166 |
| miR.558 + miR.609 + miR.29a + miR.369.3p + miR.376a* + miR.518c + miR.660 + miR.338 | 0,8 | 0,180360693 |
| miR.558 + miR.609 + miR.29a + miR.369.3p + miR.376a* + miR.494 + miR.518c + miR.338 | 0,8 | 0,146286413 |
| miR.558 + miR.609 + miR.29a + miR.369.3p + miR.376a* + miR.494 + miR.518c + miR.660 | 0,8 | 0,094223238 |
| miR.609 + miR.603 + miR.220a + miR.519b + miR.29a + miR.362 + miR.376a* + miR.518c | 0,8 | 0,213858057 |
| miR.558 + miR.609 + miR.26a + miR.29a + miR.376a* + miR.518c + miR.639 + miR.660 | 0,8 | 0,086522612 |
| miR.609 + miR.603 + miR.220a + miR.29a + miR.376a* + miR.494 + miR.518c + miR.660 | 0,8 | 0,119327352 |
| miR.558 + miR.609 + miR.29a + miR.362 + miR.369.3p + miR.376a* + miR.518c + miR.338 | 0,8 | 0,266383734 |
| miR.558 + miR.609 + miR.26a + miR.29a + miR.376a* + miR.494 + miR.518c + miR.639 | 0,8 | 0,072177566 |
| miR.609 + miR.603 + miR.520f + miR.29a + miR.369.3p + miR.376a* + miR.518c + miR.639 | 0,8 | 0,172400609 |
| miR.558 + miR.609 + miR.130a + miR.26a + miR.29a + miR.376a* + miR.494 + miR.518c | 0,8 | 0,075112277 |
| miR.558 + miR.609 + miR.29a + miR.362 + miR.369.3p + miR.376a* + miR.494 + miR.518c | 0,8 | 0,094654013 |
| miR.609 + miR.603 + miR.520f + miR.220a + miR.130a + miR.29a + miR.376a* + miR.518c | 0,8 | 0,119123492 |
| miR.609 + miR.520f + miR.220a + miR.29a + miR.376a* + miR.494 + miR.518c + miR.660 | 0,8 | 0,264617006 |
| miR.558 + miR.609 + miR.26a + miR.29a + miR.376a* + miR.494 + miR.518c + miR.338 | 0,8 | 0,133540571 |
| miR.558 + miR.609 + miR.519b + miR.29a + miR.369.3p + miR.376a* + miR.494 + miR.518c | 0,8 | 0,080724013 |
| miR.558 + miR.609 + miR.519b + miR.29a + miR.369.3p + miR.376a* + miR.518c + miR.338 | 0,8 | 0,154390529 |
| miR.609 + miR.520f + miR.220a + miR.29a + miR.369.3p + miR.376a* + miR.494 + miR.518c | 0,8 | 0,396584072 |
| miR.609 + miR.603 + miR.520f + miR.29a + miR.376a* + miR.494 + miR.518c + miR.338 | 0,8 | 0,142937902 |
| miR.609 + miR.603 + miR.220a + miR.26a + miR.29a + miR.362 + miR.376a* + miR.518c | 0,8 | 0,1707397 |
| miR.558 + miR.609 + miR.26a + miR.29a + miR.376a* + miR.494 + miR.518c + miR.660 | 0,8 | 0,036014826 |
| miR.609 + miR.603 + miR.520f + miR.130a + miR.29a + miR.369.3p + miR.376a* + miR.518c | 0,8 | 0,104606059 |
| miR.609 + miR.603 + miR.520f + miR.220a + miR.519b + miR.29a + miR.376a* + miR.518c | 0,8 | 0,186283275 |
| miR.558 + miR.609 + miR.603 + miR.29a + miR.362 + miR.376a* + miR.494 + miR.518c | 0,8 | 0,046802641 |
| miR.609 + miR.603 + miR.520f + miR.29a + miR.376a* + miR.494 + miR.518c + miR.639 | 0,8 | 0,071257355 |
| miR.609 + miR.603 + miR.520f + miR.220a + miR.26a + miR.29a + miR.376a* + miR.518c | 0,8 | 0,232483239 |
| miR.558 + miR.609 + miR.26a + miR.29a + miR.362 + miR.376a* + miR.494 + miR.518c | 0,8 | 0,064134024 |
| miR.558 + miR.609 + miR.130a + miR.26a + miR.29a + miR.376a* + miR.518c + miR.338 | 0,8 | 0,110791155 |
| miR.558 + miR.609 + miR.519b + miR.26a + miR.29a + miR.376a* + miR.518c + miR.338 | 0,8 | 0,093287896 |
| miR.609 + miR.603 + miR.520f + miR.29a + miR.369.3p + miR.376a* + miR.518c + miR.660 | 0,8 | 0,134407093 |
| miR.558 + miR.609 + miR.603 + miR.220a + miR.369.3p + miR.376a* + miR.518c + miR.639 | 0,8 | 0,162259653 |
| miR.558 + miR.609 + miR.26a + miR.29a + miR.362 + miR.376a* + miR.518c + miR.338 | 0,8 | 0,133489174 |
| miR.558 + miR.609 + miR.519b + miR.26a + miR.29a + miR.376a + miR.494 + miR.518c | 0,8 | 0,078055879 |
| miR.609 + miR.220a + miR.29a + miR.362 + miR.369.3p + miR.376a* + miR.518c + miR.660 | 0,8 | 0,328685687 |
| miR.609 + miR.603 + miR.220a + miR.29a + miR.362 + miR.376a* + miR.518c + miR.639 | 0,8 | 0,107395171 |
| miR.609 + miR.603 + miR.220a + miR.519b + miR.26a + miR.29a + miR.376a* + miR.518c | 0,8 | 0,094737666 |
| miR.609 + miR.603 + miR.220a + miR.519b + miR.29a + miR.376a* + miR.518c + miR.660 | 0,8 | 0,212758102 |
| miR.609 + miR.603 + miR.220a + miR.26a + miR.29a + miR.376a* + miR.518c + miR.639 | 0,8 | 0,053619139 |
| miR.558 + miR.609 + miR.130a + miR.29a + miR.362 + miR.376a* + miR.518c + miR.639 | 0,8 | 0,101029379 |
| miR.609 + miR.603 + miR.220a + miR.29a + miR.376a* + miR.518c + miR.639 + miR.660 | 0,8 | 0,074083874 |
| miR.609 + miR.603 + miR.220a + miR.29a + miR.362 + miR.376a* + miR.518c + miR.338 | 0,8 | 0,095069147 |
| miR.609 + miR.520f + miR.220a + miR.29a + miR.362 + miR.376a* + miR.494 + miR.518c | 0,8 | 0,303762525 |
| miR.609 + miR.603 + miR.520f + miR.29a + miR.376a* + miR.494 + miR.518c + miR.660 | 0,8 | 0,123670087 |
| miR.609 + miR.603 + miR.220a + miR.519b + miR.29a + miR.376a* + miR.518c + miR.639 | 0,8 | 0,094014764 |
| miR.558 + miR.609 + miR.130a + miR.29a + miR.376a* + miR.494 + miR.518c + miR.660 | 0,8 | 0,045727689 |
| miR.609 + miR.603 + miR.520f + miR.26a + miR.29a + miR.369.3p + miR.376a* + miR.518c | 0,8 | 0,197866833 |
| miR.609 + miR.603 + miR.220a + miR.29a + miR.362 + miR.376a* + miR.518c + miR.660 | 0,8 | 0,117066493 |
| miR.609 + miR.603 + miR.520f + miR.29a + miR.376a* + miR.518c + miR.639 + miR.338 | 0,8 | 0,166778331 |
| miR.609 + miR.603 + miR.520f + miR.519b + miR.29a + miR.369.3p + miR.376a* + miR.518c | 0,8 | 0,125139861 |
| miR.558 + miR.609 + miR.130a + miR.29a + miR.376a* + miR.518c + miR.639 + miR.338 | 0,8 | 0,089186105 |
| miR.609 + miR.603 + miR.520f + miR.220a + miR.29a + miR.376a* + miR.518c + miR.639 | 0,8 | 0,160389649 |
| miR.558 + miR.609 + miR.519b + miR.130a + miR.29a + miR.376a* + miR.494 + miR.518c | 0,8 | 0,084954918 |
| miR.609 + miR.603 + miR.220a + miR.26a + miR.29a + miR.376a* + miR.518c + miR.338 | 0,8 | 0,102695035 |
| miR.609 + miR.603 + miR.520f + miR.29a + miR.362 + miR.376a* + miR.494 + miR.518c | 0,8 | 0,110204651 |
| miR.558 + miR.609 + miR.603 + miR.520f + miR.376a* + miR.518c + miR.639 + miR.338 | 0,8 | 0,284136432 |
| miR.609 + miR.603 + miR.520f + miR.220a + miR.29a + miR.376a* + miR.518c + miR.338 | 0,8 | 0,463417504 |
| miR.558 + miR.609 + miR.130a + miR.29a + miR.376a* + miR.494 + miR.518c + miR.338 | 0,8 | 0,131382553 |
| miR.609 + miR.603 + miR.520f + miR.519b + miR.29a + miR.376a* + miR.494 + miR.518c | 0,8 | 0,079519627 |
| miR.558 + miR.609 + miR.130a + miR.29a + miR.362 + miR.376a* + miR.494 + miR.518c | 0,8 | 0,099330475 |
| miR.609 + miR.603 + miR.220a + miR.130a + miR.26a + miR.29a + miR.376a* + miR.518c | 0,8 | 0,140026047 |
| miR.609 + miR.603 + miR.220a + miR.26a + miR.29a + miR.376a* + miR.518c + miR.660 | 0,8 | 0,121181666 |
| miR.609 + miR.603 + miR.29a + miR.369.3p + miR.376a* + miR.494 + miR.518c + miR.639 | 0,8 | 0,045503448 |
| miR.609 + miR.603 + miR.220a + miR.130a + miR.29a + miR.376a* + miR.518c + miR.660 | 0,8 | 0,204975548 |
| miR.558 + miR.609 + miR.519b + miR.26a + miR.29a + miR.376a* + miR.518c + miR.660 | 0,8 | 0,047513606 |
| miR.558 + miR.609 + miR.26a + miR.29a + miR.376a* + miR.518c + miR.660 + miR.338 | 0,8 | 0,112203655 |
| miR.558 + miR.609 + miR.519b + miR.130a + miR.29a + miR.376a* + miR.518c + miR.639 | 0,8 | 0,079495498 |
| miR.609 + miR.603 + miR.520f + miR.29a + miR.362 + miR.369.3p + miR.376a* + miR.518c | 0,8 | 0,175008729 |
| miR.609 + miR.603 + miR.220a + miR.29a + miR.376a* + miR.518c + miR.660 + miR.338 | 0,8 | 0,12574749 |
| miR.609 + miR.603 + miR.220a + miR.519b + miR.130a + miR.29a + miR.376a* + miR.518c | 0,8 | 0,129863439 |
| miR.558 + miR.609 + miR.603 + miR.520f + miR.369.3p + miR.376a* + miR.518c + miR.639 | 0,8 | 0,262358671 |
| miR.609 + miR.603 + miR.520f + miR.26a + miR.29a + miR.376a* + miR.494 + miR.518c | 0,8 | 0,07637776 |
| miR.558 + miR.609 + miR.519b + miR.26a + miR.29a + miR.362 + miR.376a* + miR.518c | 0,8 | 0,072637868 |
| miR.609 + miR.603 + miR.520f + miR.130a + miR.29a + miR.376a* + miR.494 + miR.518c | 0,8 | 0,075484443 |
| miR.609 + miR.520f + miR.220a + miR.519b + miR.29a + miR.369.3p + miR.376a* + miR.518c | 0,8 | 0,449651468 |
| miR.609 + miR.603 + miR.26a + miR.29a + miR.369.3p + miR.376a* + miR.494 + miR.518c | 0,8 | 0,059350142 |
| miR.558 + miR.609 + miR.603 + miR.220a + miR.130a + miR.369.3p + miR.376a* + miR.518c | 0,8 | 0,047306499 |
| miR.609 + miR.520f + miR.220a + miR.130a + miR.29a + miR.369.3p + miR.376a* + miR.518c | 0,8 | 0,351618123 |
| miR.609 + miR.603 + miR.220a + miR.130a + miR.29a + miR.362 + miR.376a* + miR.518c | 0,8 | 0,158567797 |
| miR.558 + miR.609 + miR.519b + miR.130a + miR.26a + miR.29a + miR.376a* + miR.518c | 0,8 | 0,049965452 |
| miR.558 + miR.609 + miR.520f + miR.29a + miR.369.3p + miR.376a* + miR.494 + miR.338 | 0,8 | 0,187377223 |
| miR.558 + miR.609 + miR.130a + miR.26a + miR.29a + miR.362 + miR.376a* + miR.518c | 0,8 | 0,066922956 |
| miR.558 + miR.609 + miR.26a + miR.29a + miR.362 + miR.376a* + miR.518c + miR.660 | 0,8 | 0,029053191 |
| miR.558 + miR.609 + miR.130a + miR.26a + miR.29a + miR.376a* + miR.518c + miR.660 | 0,8 | 0,034497604 |
| miR.558 + miR.609 + miR.603 + miR.520f + miR.130a + miR.376a* + miR.518c + miR.338 | 0,8 | 0,16955606 |
| miR.558 + miR.609 + miR.29a + miR.362 + miR.376a* + miR.518c + miR.639 + miR.660 | 0,8 | 0,063517482 |
| miR.558 + miR.609 + miR.520f + miR.29a + miR.369.3p + miR.376a* + miR.660 + miR.338 | 0,8 | 0,386278992 |
| miR.609 + miR.603 + miR.29a + miR.369.3p + miR.376a* + miR.518c + miR.639 + miR.338 | 0,8 | 0,148250881 |
| miR.609 + miR.603 + miR.26a + miR.29a + miR.369.3p + miR.376a* + miR.518c + miR.639 | 0,8 | 0,040619957 |
| miR.558 + miR.609 + miR.603 + miR.520f + miR.130a + miR.376a* + miR.518c + miR.639 | 0,8 | 0,117617632 |
| miR.609 + miR.220a + miR.29a + miR.369.3p + miR.376a* + miR.494 + miR.518c + miR.660 | 0,8 | 0,227245906 |
| miR.609 + miR.603 + miR.520f + miR.130a + miR.29a + miR.376a* + miR.518c + miR.338 | 0,8 | 0,214616822 |
| miR.609 + miR.520f + miR.220a + miR.130a + miR.29a + miR.376a* + miR.518c + miR.660 | 0,8 | 0,217974393 |
| miR.609 + miR.603 + miR.220a + miR.519b + miR.29a + miR.376a* + miR.518c + miR.338 | 0,8 | 0,106536586 |
| miR.558 + miR.609 + miR.29a + miR.376a* + miR.494 + miR.518c + miR.639 + miR.660 | 0,8 | 0,047867864 |
| miR.558 + miR.609 + miR.29a + miR.362 + miR.376a* + miR.518c + miR.639 + miR.338 | 0,79 | 0,104359161 |
| miR.609 + miR.520f + miR.220a + miR.29a + miR.376a* + miR.494 + miR.518c + miR.338 | 0,79 | 0,279861289 |
| miR.609 + miR.520f + miR.220a + miR.29a + miR.362 + miR.369.3p + miR.376a* + miR.518c | 0,79 | 0,465378209 |
| miR.609 + miR.603 + miR.220a + miR.29a + miR.376a* + miR.494 + miR.518c + miR.639 | 0,79 | 0,014065978 |
| miR.609 + miR.520f + miR.220a + miR.29a + miR.369.3p + miR.376a* + miR.518c + miR.338 | 0,79 | 0,464336891 |
| miR.609 + miR.603 + miR.220a + miR.29a + miR.362 + miR.376a* + miR.494 + miR.518c | 0,79 | 0,070687312 |
| miR.609 + miR.520f + miR.220a + miR.130a + miR.29a + miR.376a* + miR.494 + miR.518c | 0,79 | 0,183461255 |
| miR.609 + miR.603 + miR.520f + miR.29a + miR.376a* + miR.518c + miR.639 + miR.660 | 0,79 | 0,057213186 |
| miR.558 + miR.609 + miR.29a + miR.362 + miR.376a* + miR.494 + miR.518c + miR.660 | 0,79 | 0,027088602 |
| miR.609 + miR.603 + miR.29a + miR.369.3p + miR.376a* + miR.494 + miR.518c + miR.338 | 0,79 | 0,102702923 |
| miR.558 + miR.609 + miR.603 + miR.520f + miR.220a + miR.376a* + miR.518c + miR.639 | 0,79 | 0,155884459 |
| miR.609 + miR.603 + miR.29a + miR.362 + miR.369.3p + miR.376a* + miR.518c + miR.660 | 0,79 | 0,239347088 |
| miR.609 + miR.603 + miR.220a + miR.130a + miR.29a + miR.376a* + miR.494 + miR.518c | 0,79 | 0,018685042 |
| miR.609 + miR.520f + miR.220a + miR.26a + miR.29a + miR.369.3p + miR.376a* + miR.518c | 0,79 | 0,411255671 |
| miR.558 + miR.609 + miR.519b + miR.29a + miR.376a* + miR.518c + miR.639 + miR.338 | 0,79 | 0,063912679 |
| miR.558 + miR.609 + miR.520f + miR.130a + miR.376a* + miR.518c + miR.639 + miR.338 | 0,79 | 0,256645515 |
| miR.609 + miR.603 + miR.520f + miR.130a + miR.29a + miR.376a* + miR.518c + miR.639 | 0,79 | 0,037498021 |
| miR.609 + miR.220a + miR.26a + miR.29a + miR.369.3p + miR.376a* + miR.494 + miR.518c | 0,79 | 0,316840421 |
| miR.558 + miR.609 + miR.29a + miR.376a* + miR.494 + miR.518c + miR.639 + miR.338 | 0,79 | 0,061686596 |
| miR.558 + miR.609 + miR.603 + miR.369.3p + miR.376a* + miR.518c + miR.639 + miR.338 | 0,79 | 0,218566902 |
| miR.558 + miR.609 + miR.519b + miR.29a + miR.362 + miR.376a* + miR.518c + miR.639 | 0,79 | 0,056849978 |
| miR.609 + miR.520f + miR.220a + miR.29a + miR.376a* + miR.494 + miR.518c + miR.639 | 0,79 | 0,170298444 |
| miR.609 + miR.520f + miR.220a + miR.29a + miR.369.3p + miR.376a* + miR.518c + miR.639 | 0,79 | 0,465899598 |
| miR.609 + miR.603 + miR.520f + miR.29a + miR.362 + miR.376a* + miR.518c + miR.639 | 0,79 | 0,070826007 |
| miR.609 + miR.520f + miR.220a + miR.519b + miR.29a + miR.376a* + miR.494 + miR.518c | 0,79 | 0,204368669 |
| miR.609 + miR.603 + miR.220a + miR.29a + miR.376a* + miR.494 + miR.518c + miR.338 | 0,79 | 0,034996557 |
| miR.609 + miR.520f + miR.220a + miR.26a + miR.29a + miR.376a* + miR.494 + miR.518c | 0,79 | 0,225911103 |
| miR.609 + miR.220a + miR.519b + miR.29a + miR.369.3p + miR.376a* + miR.518c + miR.660 | 0,79 | 0,328982051 |
| miR.558 + miR.609 + miR.520f + miR.130a + miR.369.3p + miR.376a* + miR.518c + miR.338 | 0,79 | 0,256583654 |
| miR.609 + miR.603 + miR.220a + miR.29a + miR.376a* + miR.518c + miR.639 + miR.338 | 0,79 | 0,025434948 |
| miR.609 + miR.520f + miR.220a + miR.130a + miR.29a + miR.362 + miR.376a* + miR.518c | 0,79 | 0,279953483 |
| miR.609 + miR.520f + miR.29a + miR.369.3p + miR.376a* + miR.494 + miR.518c + miR.338 | 0,79 | 0,189088526 |
| miR.558 + miR.609 + miR.603 + miR.520f + miR.130a + miR.369.3p + miR.376a* + miR.518c | 0,79 | 0,064198399 |
| miR.558 + miR.609 + miR.520f + miR.29a + miR.369.3p + miR.376a* + miR.639 + miR.338 | 0,79 | 0,278238064 |
| miR.609 + miR.603 + miR.26a + miR.29a + miR.369.3p + miR.376a* + miR.518c + miR.338 | 0,79 | 0,341314163 |
| miR.558 + miR.609 + miR.519b + miR.29a + miR.376a* + miR.494 + miR.518c + miR.639 | 0,79 | 0,040660192 |
| miR.609 + miR.603 + miR.220a + miR.130a + miR.29a + miR.376a* + miR.518c + miR.338 | 0,79 | 0,141246547 |
| miR.558 + miR.609 + miR.29a + miR.376a* + miR.518c + miR.639 + miR.660 + miR.338 | 0,79 | 0,134976955 |
| miR.558 + miR.609 + miR.29a + miR.362 + miR.376a* + miR.494 + miR.518c + miR.639 | 0,79 | 0,030368201 |
| miR.558 + miR.609 + miR.603 + miR.130a + miR.369.3p + miR.376a* + miR.518c + miR.639 | 0,79 | 0,163503941 |
| miR.609 + miR.603 + miR.220a + miR.130a + miR.29a + miR.376a* + miR.518c + miR.639 | 0,79 | 0,062054808 |
| miR.609 + miR.520f + miR.29a + miR.376a* + miR.494 + miR.518c + miR.660 + miR.338 | 0,79 | 0,231117069 |
| miR.558 + miR.609 + miR.603 + miR.26a + miR.369.3p + miR.376a* + miR.518c + miR.639 | 0,79 | 0,247509787 |
| miR.609 + miR.520f + miR.220a + miR.26a + miR.29a + miR.376a* + miR.518c + miR.660 | 0,79 | 0,246924086 |
| miR.558 + miR.609 + miR.520f + miR.369.3p + miR.376a* + miR.518c + miR.639 + miR.338 | 0,79 | 0,312469759 |
| miR.609 + miR.603 + miR.520f + miR.130a + miR.29a + miR.362 + miR.376a* + miR.518c | 0,79 | 0,048265991 |
| miR.558 + miR.609 + miR.519b + miR.29a + miR.376a* + miR.518c + miR.639 + miR.660 | 0,79 | 0,061297534 |
| miR.558 + miR.609 + miR.130a + miR.29a + miR.362 + miR.376a* + miR.518c + miR.338 | 0,79 | 0,056270086 |
| miR.558 + miR.609 + miR.520f + miR.130a + miR.29a + miR.369.3p + miR.376a* + miR.338 | 0,79 | 0,258763326 |
| miR.558 + miR.609 + miR.29a + miR.362 + miR.376a* + miR.518c + miR.660 + miR.338 | 0,79 | 0,025551434 |
| miR.609 + miR.603 + miR.29a + miR.362 + miR.369.3p + miR.376a* + miR.494 + miR.518c | 0,79 | 0,120634276 |
| miR.609 + miR.220a + miR.26a + miR.29a + miR.369.3p + miR.376a* + miR.518c + miR.660 | 0,79 | 0,457598987 |
| miR.609 + miR.603 + miR.520f + miR.130a + miR.29a + miR.376a* + miR.518c + miR.660 | 0,79 | 0,056117071 |
| miR.558 + miR.609 + miR.519b + miR.29a + miR.362 + miR.376a* + miR.518c + miR.660 | 0,79 | 0,019781247 |
| miR.609 + miR.603 + miR.520f + miR.519b + miR.29a + miR.376a* + miR.518c + miR.639 | 0,79 | 0,042751085 |
| miR.609 + miR.603 + miR.130a + miR.29a + miR.369.3p + miR.376a* + miR.494 + miR.518c | 0,79 | 0,042899006 |
| miR.558 + miR.609 + miR.603 + miR.520f + miR.220a + miR.130a + miR.376a* + miR.518c | 0,79 | 0,029829614 |
| miR.558 + miR.609 + miR.130a + miR.29a + miR.376a* + miR.518c + miR.660 + miR.338 | 0,79 | 0,042703307 |
| miR.609 + miR.603 + miR.520f + miR.29a + miR.362 + miR.376a* + miR.518c + miR.338 | 0,79 | 0,33965037 |
| miR.558 + miR.609 + miR.519b + miR.29a + miR.376a* + miR.518c + miR.660 + miR.338 | 0,79 | 0,043604576 |
| miR.609 + miR.603 + miR.519b + miR.29a + miR.369.3p + miR.376a* + miR.494 + miR.518c | 0,79 | 0,044786066 |
| miR.558 + miR.609 + miR.603 + miR.362 + miR.369.3p + miR.376a* + miR.518c + miR.639 | 0,79 | 0,139841484 |
| miR.558 + miR.609 + miR.520f + miR.519b + miR.29a + miR.376a* + miR.494 + miR.338 | 0,79 | 0,252724832 |
| miR.609 + miR.603 + miR.520f + miR.26a + miR.29a + miR.376a* + miR.518c + miR.338 | 0,79 | 0,250206184 |
| miR.558 + miR.609 + miR.519b + miR.29a + miR.362 + miR.376a* + miR.518c + miR.338 | 0,79 | 0,036084696 |
| miR.558 + miR.609 + miR.130a + miR.29a + miR.362 + miR.376a* + miR.518c + miR.660 | 0,79 | 0,027794919 |
| miR.609 + miR.603 + miR.519b + miR.29a + miR.369.3p + miR.376a* + miR.518c + miR.639 | 0,79 | 0,030735393 |
| miR.609 + miR.220a + miR.519b + miR.26a + miR.29a + miR.369.3p + miR.376a* + miR.518c | 0,79 | 0,471614629 |
| miR.558 + miR.609 + miR.519b + miR.130a + miR.29a + miR.376a* + miR.518c + miR.660 | 0,79 | 0,036027337 |
| miR.609 + miR.603 + miR.29a + miR.369.3p + miR.376a* + miR.518c + miR.639 + miR.660 | 0,79 | 0,047357012 |
| miR.609 + miR.603 + miR.29a + miR.362 + miR.369.3p + miR.376a* + miR.518c + miR.338 | 0,79 | 0,31970622 |
| miR.558 + miR.609 + miR.519b + miR.130a + miR.29a + miR.362 + miR.376a* + miR.518c | 0,79 | 0,043132144 |
| miR.609 + miR.603 + miR.520f + miR.26a + miR.29a + miR.376a* + miR.518c + miR.639 | 0,79 | 0,044595943 |
| miR.609 + miR.603 + miR.520f + miR.29a + miR.376a* + miR.518c + miR.660 + miR.338 | 0,79 | 0,281114861 |
| miR.558 + miR.609 + miR.519b + miR.130a + miR.29a + miR.376a* + miR.518c + miR.338 | 0,79 | 0,038646208 |
| miR.558 + miR.609 + miR.29a + miR.362 + miR.376a* + miR.494 + miR.518c + miR.338 | 0,79 | 0,083279685 |
| miR.558 + miR.609 + miR.603 + miR.369.3p + miR.376a* + miR.518c + miR.639 + miR.660 | 0,79 | 0,231557189 |
| miR.609 + miR.520f + miR.29a + miR.369.3p + miR.376a* + miR.494 + miR.518c + miR.660 | 0,79 | 0,114451385 |
| miR.558 + miR.609 + miR.603 + miR.220a + miR.26a + miR.29a + miR.518c + miR.660 | 0,79 | 0,031755053 |
| miR.609 + miR.603 + miR.130a + miR.29a + miR.369.3p + miR.376a* + miR.518c + miR.639 | 0,79 | 0,035254339 |
| miR.609 + miR.603 + miR.519b + miR.26a + miR.29a + miR.369.3p + miR.376a* + miR.518c | 0,79 | 0,056844182 |
| miR.609 + miR.603 + miR.520f + miR.130a + miR.26a + miR.29a + miR.376a* + miR.518c | 0,79 | 0,041982334 |
| miR.609 + miR.520f + miR.220a + miR.26a + miR.29a + miR.362 + miR.376a* + miR.518c | 0,79 | 0,40011922 |
| miR.558 + miR.609 + miR.519b + miR.29a + miR.376a* + miR.494 + miR.518c + miR.338 | 0,79 | 0,08561037 |
| miR.609 + miR.220a + miR.130a + miR.29a + miR.369.3p + miR.376a* + miR.518c + miR.660 | 0,79 | 0,29937593 |
| miR.609 + miR.603 + miR.26a + miR.29a + miR.362 + miR.369.3p + miR.376a* + miR.518c | 0,79 | 0,135631786 |
| miR.609 + miR.603 + miR.29a + miR.362 + miR.369.3p + miR.376a* + miR.518c + miR.639 | 0,79 | 0,090600075 |
| miR.558 + miR.609 + miR.603 + miR.520f + miR.29a + miR.369.3p + miR.376a* + miR.338 | 0,79 | 0,153718607 |
| miR.609 + miR.603 + miR.26a + miR.29a + miR.369.3p + miR.376a* + miR.518c + miR.660 | 0,79 | 0,043162093 |
| miR.558 + miR.609 + miR.520f + miR.29a + miR.376a* + miR.494 + miR.639 + miR.338 | 0,79 | 0,14820154 |
| miR.609 + miR.520f + miR.29a + miR.369.3p + miR.376a* + miR.494 + miR.518c + miR.639 | 0,79 | 0,095739415 |
| miR.609 + miR.603 + miR.29a + miR.369.3p + miR.376a* + miR.494 + miR.518c + miR.660 | 0,79 | 0,040154197 |
| miR.609 + miR.603 + miR.520f + miR.519b + miR.130a + miR.29a + miR.376a* + miR.518c | 0,79 | 0,044739482 |
| miR.609 + miR.520f + miR.29a + miR.376a* + miR.494 + miR.518c + miR.639 + miR.660 | 0,79 | 0,119345831 |
| miR.609 + miR.603 + miR.130a + miR.26a + miR.29a + miR.369.3p + miR.376a* + miR.518c | 0,79 | 0,031585691 |
| miR.558 + miR.609 + miR.519b + miR.29a + miR.362 + miR.376a* + miR.494 + miR.518c | 0,79 | 0,06162303 |
| miR.558 + miR.609 + miR.603 + miR.220a + miR.130a + miR.376a* + miR.518c + miR.639 | 0,79 | 0,047240347 |
| miR.558 + miR.609 + miR.603 + miR.519b + miR.369.3p + miR.376a* + miR.518c + miR.639 | 0,79 | 0,237565849 |
| miR.609 + miR.520f + miR.220a + miR.29a + miR.376a* + miR.518c + miR.660 + miR.338 | 0,79 | 0,340001773 |
| miR.609 + miR.603 + miR.520f + miR.26a + miR.29a + miR.362 + miR.376a* + miR.518c | 0,79 | 0,090374906 |
| miR.609 + miR.220a + miR.29a + miR.369.3p + miR.376a* + miR.518c + miR.639 + miR.660 | 0,79 | 0,397342617 |
| miR.558 + miR.609 + miR.520f + miR.519b + miR.29a + miR.369.3p + miR.376a* + miR.338 | 0,79 | 0,309152891 |
| miR.609 + miR.220a + miR.29a + miR.369.3p + miR.376a* + miR.518c + miR.660 + miR.338 | 0,79 | 0,380220712 |
| miR.609 + miR.520f + miR.220a + miR.519b + miR.29a + miR.376a* + miR.518c + miR.660 | 0,79 | 0,152635729 |
| miR.558 + miR.609 + miR.520f + miR.29a + miR.376a* + miR.494 + miR.660 + miR.338 | 0,79 | 0,301429516 |
| miR.609 + miR.520f + miR.220a + miR.29a + miR.376a* + miR.518c + miR.639 + miR.660 | 0,79 | 0,182807676 |
| miR.558 + miR.609 + miR.520f + miR.220a + miR.29a + miR.369.3p + miR.376a* + miR.338 | 0,79 | 0,185479846 |
| miR.558 + miR.609 + miR.520f + miR.26a + miR.29a + miR.369.3p + miR.376a* + miR.338 | 0,79 | 0,197426817 |
| miR.558 + miR.609 + miR.603 + miR.220a + miR.519b + miR.130a + miR.376a* + miR.518c | 0,79 | 0,07127935 |
| miR.609 + miR.603 + miR.29a + miR.369.3p + miR.376a* + miR.518c + miR.660 + miR.338 | 0,79 | 0,208196677 |
| miR.609 + miR.520f + miR.220a + miR.519b + miR.29a + miR.362 + miR.376a* + miR.518c | 0,79 | 0,359853699 |
| miR.558 + miR.609 + miR.29a + miR.376a* + miR.494 + miR.518c + miR.660 + miR.338 | 0,79 | 0,065888161 |
| miR.609 + miR.520f + miR.29a + miR.369.3p + miR.376a* + miR.518c + miR.660 + miR.338 | 0,79 | 0,340812349 |
| miR.609 + miR.520f + miR.519b + miR.29a + miR.369.3p + miR.376a* + miR.518c + miR.639 | 0,79 | 0,089042496 |
| miR.558 + miR.609 + miR.520f + miR.29a + miR.362 + miR.369.3p + miR.376a* + miR.660 | 0,79 | 0,294570752 |
| miR.558 + miR.609 + miR.603 + miR.520f + miR.26a + miR.376a* + miR.518c + miR.639 | 0,79 | 0,129760043 |
| miR.558 + miR.609 + miR.519b + miR.29a + miR.376a* + miR.494 + miR.518c + miR.660 | 0,79 | 0,042861313 |
| miR.609 + miR.603 + miR.519b + miR.29a + miR.362 + miR.369.3p + miR.376a* + miR.518c | 0,79 | 0,152183224 |
| miR.558 + miR.609 + miR.520f + miR.220a + miR.130a + miR.376a* + miR.518c + miR.338 | 0,79 | 0,201695924 |
| miR.609 + miR.520f + miR.220a + miR.29a + miR.362 + miR.376a* + miR.518c + miR.660 | 0,79 | 0,208199176 |
| miR.609 + miR.520f + miR.220a + miR.130a + miR.29a + miR.376a* + miR.518c + miR.338 | 0,79 | 0,280012662 |
| miR.609 + miR.603 + miR.520f + miR.220a + miR.130a + miR.369.3p + miR.376a* + miR.518c | 0,79 | 0,059917474 |
| miR.558 + miR.609 + miR.603 + miR.220a + miR.519b + miR.369.3p + miR.376a* + miR.518c | 0,79 | 0,066670565 |
| miR.558 + miR.609 + miR.520f + miR.29a + miR.362 + miR.369.3p + miR.376a* + miR.338 | 0,79 | 0,182667564 |
| miR.609 + miR.520f + miR.220a + miR.519b + miR.130a + miR.29a + miR.376a* + miR.518c | 0,79 | 0,18231478 |
| miR.609 + miR.520f + miR.220a + miR.29a + miR.362 + miR.376a* + miR.518c + miR.639 | 0,79 | 0,298952941 |
| miR.609 + miR.520f + miR.220a + miR.29a + miR.362 + miR.376a* + miR.518c + miR.338 | 0,79 | 0,379786737 |
| miR.609 + miR.520f + miR.26a + miR.29a + miR.376a* + miR.494 + miR.518c + miR.660 | 0,79 | 0,125015225 |
| miR.558 + miR.609 + miR.603 + miR.520f + miR.29a + miR.376a* + miR.494 + miR.338 | 0,79 | 0,088730013 |
| miR.558 + miR.609 + miR.520f + miR.130a + miR.369.3p + miR.376a* + miR.518c + miR.639 | 0,79 | 0,237171356 |
| miR.609 + miR.603 + miR.520f + miR.130a + miR.376a* + miR.518c + miR.639 + miR.338 | 0,79 | 0,09389186 |
| miR.609 + miR.603 + miR.520f + miR.519b + miR.29a + miR.376a* + miR.518c + miR.338 | 0,79 | 0,284157594 |
| miR.609 + miR.520f + miR.519b + miR.29a + miR.369.3p + miR.376a* + miR.518c + miR.338 | 0,79 | 0,252322389 |
| miR.609 + miR.520f + miR.519b + miR.29a + miR.369.3p + miR.376a* + miR.494 + miR.518c | 0,79 | 0,066610658 |
| miR.558 + miR.609 + miR.520f + miR.29a + miR.362 + miR.518c + miR.639 + miR.660 | 0,79 | 0,065060289 |
| miR.609 + miR.220a + miR.130a + miR.26a + miR.29a + miR.369.3p + miR.376a* + miR.518c | 0,79 | 0,367250572 |
| miR.609 + miR.603 + miR.519b + miR.29a + miR.369.3p + miR.376a* + miR.518c + miR.660 | 0,79 | 0,034723432 |
| miR.558 + miR.609 + miR.520f + miR.220a + miR.130a + miR.369.3p + miR.376a* + miR.518c | 0,79 | 0,090632124 |
| miR.609 + miR.603 + miR.220a + miR.130a + miR.369.3p + miR.376a* + miR.518c + miR.639 | 0,79 | 0,050903247 |
| miR.609 + miR.520f + miR.519b + miR.29a + miR.369.3p + miR.376a* + miR.518c + miR.660 | 0,79 | 0,057529682 |
| miR.609 + miR.520f + miR.220a + miR.130a + miR.26a + miR.29a + miR.376a* + miR.518c | 0,79 | 0,165732822 |
| miR.609 + miR.603 + miR.520f + miR.26a + miR.29a + miR.376a* + miR.518c + miR.660 | 0,79 | 0,059939163 |
| miR.558 + miR.609 + miR.520f + miR.29a + miR.362 + miR.376a* + miR.494 + miR.338 | 0,79 | 0,146847775 |
| miR.609 + miR.520f + miR.29a + miR.362 + miR.376a* + miR.494 + miR.518c + miR.338 | 0,79 | 0,183593874 |
| miR.609 + miR.220a + miR.519b + miR.29a + miR.369.3p + miR.376a* + miR.494 + miR.518c | 0,79 | 0,431925176 |
| miR.558 + miR.609 + miR.520f + miR.26a + miR.29a + miR.376a* + miR.494 + miR.338 | 0,79 | 0,111381504 |
| miR.609 + miR.603 + miR.520f + miR.519b + miR.29a + miR.362 + miR.376a* + miR.518c | 0,79 | 0,098953257 |
| miR.609 + miR.520f + miR.220a + miR.130a + miR.29a + miR.376a* + miR.518c + miR.639 | 0,79 | 0,174486634 |
| miR.558 + miR.609 + miR.520f + miR.220a + miR.130a + miR.376a* + miR.518c + miR.639 | 0,79 | 0,12723752 |
| miR.558 + miR.609 + miR.603 + miR.369.3p + miR.376a* + miR.494 + miR.518c + miR.639 | 0,79 | 0,25168833 |
| miR.558 + miR.609 + miR.520f + miR.130a + miR.29a + miR.376a* + miR.494 + miR.338 | 0,79 | 0,117744264 |
| miR.609 + miR.603 + miR.130a + miR.29a + miR.369.3p + miR.376a* + miR.518c + miR.338 | 0,79 | 0,117534401 |
| miR.558 + miR.609 + miR.520f + miR.376a* + miR.518c + miR.639 + miR.660 + miR.338 | 0,79 | 0,395905603 |
| miR.609 + miR.220a + miR.519b + miR.29a + miR.369.3p + miR.376a* + miR.518c + miR.639 | 0,79 | 0,496016522 |
| miR.558 + miR.609 + miR.520f + miR.220a + miR.29a + miR.376a* + miR.494 + miR.338 | 0,79 | 0,114259054 |
| miR.609 + miR.220a + miR.519b + miR.29a + miR.369.3p + miR.376a* + miR.518c + miR.338 | 0,79 | 0,383941349 |
| miR.609 + miR.220a + miR.26a + miR.29a + miR.369.3p + miR.376a* + miR.518c + miR.639 | 0,79 | 0,433126729 |
| miR.609 + miR.603 + miR.520f + miR.29a + miR.362 + miR.376a* + miR.518c + miR.660 | 0,79 | 0,060237243 |
| miR.609 + miR.603 + miR.220a + miR.130a + miR.26a + miR.369.3p + miR.376a* + miR.518c | 0,79 | 0,039528655 |
| miR.558 + miR.609 + miR.520f + miR.220a + miR.369.3p + miR.376a* + miR.518c + miR.639 | 0,79 | 0,166040329 |
| miR.609 + miR.603 + miR.130a + miR.29a + miR.362 + miR.369.3p + miR.376a* + miR.518c | 0,79 | 0,136175349 |
| miR.609 + miR.603 + miR.519b + miR.29a + miR.369.3p + miR.376a* + miR.518c + miR.338 | 0,79 | 0,197410181 |
| miR.609 + miR.220a + miR.26a + miR.29a + miR.369.3p + miR.376a* + miR.518c + miR.338 | 0,79 | 0,409586732 |
| miR.558 + miR.609 + miR.220a + miR.26a + miR.29a + miR.518c + miR.660 + miR.338 | 0,79 | 0,030944515 |
| miR.558 + miR.609 + miR.220a + miR.26a + miR.29a + miR.362 + miR.518c + miR.660 | 0,79 | 0,027952168 |
| miR.558 + miR.609 + miR.520f + miR.29a + miR.362 + miR.376a* + miR.494 + miR.660 | 0,79 | 0,124411527 |
| miR.609 + miR.520f + miR.26a + miR.29a + miR.369.3p + miR.376a* + miR.494 + miR.518c | 0,79 | 0,080840583 |
| miR.609 + miR.603 + miR.520f + miR.519b + miR.26a + miR.29a + miR.376a* + miR.518c | 0,79 | 0,058848822 |
| miR.609 + miR.220a + miR.519b + miR.29a + miR.362 + miR.369.3p + miR.376a* + miR.518c | 0,79 | 0,464343227 |
| miR.558 + miR.609 + miR.520f + miR.220a + miR.26a + miR.29a + miR.518c + miR.660 | 0,79 | 0,031137959 |
| miR.558 + miR.609 + miR.520f + miR.26a + miR.29a + miR.518c + miR.639 + miR.660 | 0,79 | 0,046148103 |
| miR.558 + miR.609 + miR.520f + miR.220a + miR.130a + miR.376a* + miR.518c + miR.660 | 0,79 | 0,054357431 |
| miR.609 + miR.520f + miR.26a + miR.29a + miR.376a* + miR.494 + miR.518c + miR.338 | 0,79 | 0,132907257 |
| miR.609 + miR.603 + miR.519b + miR.130a + miR.29a + miR.369.3p + miR.376a* + miR.518c | 0,79 | 0,034127363 |
| miR.558 + miR.609 + miR.520f + miR.29a + miR.369.3p + miR.376a* + miR.494 + miR.660 | 0,79 | 0,162971938 |
| miR.558 + miR.609 + miR.603 + miR.130a + miR.26a + miR.369.3p + miR.376a* + miR.518c | 0,79 | 0,100931545 |
| miR.558 + miR.609 + miR.520f + miR.220a + miR.29a + miR.362 + miR.518c + miR.660 | 0,79 | 0,056476805 |
| miR.558 + miR.609 + miR.603 + miR.130a + miR.369.3p + miR.376a* + miR.518c + miR.338 | 0,79 | 0,063090031 |
| miR.609 + miR.603 + miR.130a + miR.29a + miR.369.3p + miR.376a* + miR.518c + miR.660 | 0,79 | 0,033305231 |
| miR.609 + miR.603 + miR.26a + miR.29a + miR.376a* + miR.494 + miR.518c + miR.639 | 0,79 | 0,010451913 |
| miR.609 + miR.603 + miR.29a + miR.376a* + miR.494 + miR.518c + miR.639 + miR.338 | 0,79 | 0,02489557 |
| miR.609 + miR.603 + miR.130a + miR.29a + miR.376a* + miR.494 + miR.518c + miR.639 | 0,79 | 0,013238823 |
| miR.609 + miR.603 + miR.520f + miR.519b + miR.29a + miR.376a* + miR.518c + miR.660 | 0,79 | 0,056573042 |
| miR.558 + miR.609 + miR.520f + miR.29a + miR.362 + miR.369.3p + miR.518c + miR.660 | 0,79 | 0,032593838 |
| miR.609 + miR.520f + miR.130a + miR.29a + miR.369.3p + miR.376a* + miR.494 + miR.518c | 0,79 | 0,094907421 |
| miR.609 + miR.520f + miR.29a + miR.362 + miR.376a* + miR.494 + miR.518c + miR.660 | 0,79 | 0,071419167 |
| miR.558 + miR.609 + miR.603 + miR.520f + miR.29a + miR.369.3p + miR.376a* + miR.494 | 0,79 | 0,085192814 |
| miR.609 + miR.603 + miR.519b + miR.29a + miR.376a* + miR.494 + miR.518c + miR.639 | 0,79 | 0,007619869 |
| miR.609 + miR.520f + miR.519b + miR.29a + miR.376a* + miR.494 + miR.518c + miR.660 | 0,79 | 0,077672648 |
| miR.609 + miR.520f + miR.519b + miR.29a + miR.376a* + miR.494 + miR.518c + miR.639 | 0,79 | 0,051971571 |
| miR.558 + miR.609 + miR.603 + miR.520f + miR.519b + miR.130a + miR.376a* + miR.518c | 0,79 | 0,040154426 |
| miR.609 + miR.220a + miR.130a + miR.29a + miR.369.3p + miR.376a* + miR.494 + miR.518c | 0,79 | 0,330151427 |
| miR.558 + miR.609 + miR.603 + miR.130a + miR.362 + miR.369.3p + miR.376a* + miR.518c | 0,79 | 0,043811715 |
| miR.609 + miR.520f + miR.29a + miR.362 + miR.376a* + miR.494 + miR.518c + miR.639 | 0,79 | 0,114744713 |
| miR.558 + miR.609 + miR.520f + miR.130a + miR.29a + miR.518c + miR.639 + miR.660 | 0,79 | 0,078551074 |
| miR.558 + miR.609 + miR.603 + miR.520f + miR.376a* + miR.518c + miR.639 + miR.660 | 0,79 | 0,322852283 |
| miR.558 + miR.609 + miR.520f + miR.29a + miR.376a* + miR.639 + miR.660 + miR.338 | 0,79 | 0,387825763 |
| miR.609 + miR.520f + miR.519b + miR.29a + miR.376a* + miR.494 + miR.518c + miR.338 | 0,79 | 0,073800921 |
| miR.609 + miR.603 + miR.520f + miR.130a + miR.369.3p + miR.376a* + miR.518c + miR.639 | 0,79 | 0,077297966 |
| miR.609 + miR.603 + miR.29a + miR.376a* + miR.494 + miR.518c + miR.639 + miR.660 | 0,79 | 0,00710432 |
| miR.558 + miR.609 + miR.603 + miR.220a + miR.376a* + miR.518c + miR.639 + miR.338 | 0,79 | 0,070383796 |
| miR.609 + miR.520f + miR.519b + miR.130a + miR.29a + miR.369.3p + miR.376a* + miR.518c | 0,79 | 0,087421804 |
| miR.609 + miR.603 + miR.520f + miR.130a + miR.369.3p + miR.376a* + miR.518c + miR.338 | 0,79 | 0,184327837 |
| miR.558 + miR.609 + miR.520f + miR.519b + miR.29a + miR.369.3p + miR.376a* + miR.494 | 0,79 | 0,128429369 |
| miR.558 + miR.609 + miR.520f + miR.130a + miR.376a* + miR.518c + miR.660 + miR.338 | 0,79 | 0,181948423 |
| miR.558 + miR.609 + miR.220a + miR.130a + miR.26a + miR.29a + miR.518c + miR.660 | 0,79 | 0,017902511 |
| miR.558 + miR.609 + miR.603 + miR.220a + miR.519b + miR.376a* + miR.518c + miR.639 | 0,79 | 0,085472418 |
| miR.558 + miR.609 + miR.603 + miR.520f + miR.130a + miR.376a* + miR.494 + miR.518c | 0,79 | 0,022805475 |
| miR.558 + miR.609 + miR.520f + miR.130a + miR.376a* + miR.494 + miR.518c + miR.338 | 0,79 | 0,144650307 |
| miR.558 + miR.609 + miR.603 + miR.520f + miR.29a + miR.362 + miR.518c + miR.660 | 0,79 | 0,042956133 |
| miR.609 + miR.520f + miR.220a + miR.26a + miR.29a + miR.376a* + miR.518c + miR.338 | 0,79 | 0,283619468 |
| miR.558 + miR.609 + miR.603 + miR.520f + miR.26a + miR.29a + miR.518c + miR.660 | 0,79 | 0,031415816 |
| miR.609 + miR.220a + miR.29a + miR.369.3p + miR.376a* + miR.494 + miR.518c + miR.639 | 0,79 | 0,362071428 |
| miR.558 + miR.609 + miR.603 + miR.520f + miR.376a* + miR.494 + miR.518c + miR.639 | 0,79 | 0,213697254 |
| miR.558 + miR.609 + miR.603 + miR.520f + miR.130a + miR.376a* + miR.518c + miR.660 | 0,79 | 0,031045783 |
| miR.609 + miR.520f + miR.220a + miR.519b + miR.29a + miR.376a* + miR.518c + miR.338 | 0,79 | 0,258225028 |
| miR.558 + miR.609 + miR.603 + miR.520f + miR.130a + miR.362 + miR.376a* + miR.518c | 0,79 | 0,02212113 |
| miR.558 + miR.609 + miR.520f + miR.519b + miR.130a + miR.376a* + miR.518c + miR.338 | 0,79 | 0,170310922 |
| miR.558 + miR.609 + miR.603 + miR.520f + miR.130a + miR.26a + miR.376a* + miR.518c | 0,79 | 0,024719357 |
| miR.609 + miR.520f + miR.220a + miR.519b + miR.26a + miR.29a + miR.376a* + miR.518c | 0,79 | 0,204155936 |
| miR.558 + miR.609 + miR.603 + miR.520f + miR.29a + miR.518c + miR.639 + miR.660 | 0,79 | 0,071267072 |
| miR.609 + miR.520f + miR.26a + miR.29a + miR.376a* + miR.494 + miR.518c + miR.639 | 0,79 | 0,104625293 |
| miR.609 + miR.603 + miR.29a + miR.362 + miR.376a* + miR.494 + miR.518c + miR.639 | 0,79 | 0,009163906 |
| miR.609 + miR.520f + miR.29a + miR.362 + miR.369.3p + miR.376a* + miR.494 + miR.518c | 0,79 | 0,073238708 |
| miR.558 + miR.609 + miR.520f + miR.369.3p + miR.376a* + miR.518c + miR.639 + miR.660 | 0,79 | 0,223363007 |
| miR.558 + miR.609 + miR.520f + miR.130a + miR.26a + miR.376a* + miR.518c + miR.639 | 0,79 | 0,090110171 |
| miR.558 + miR.609 + miR.520f + miR.130a + miR.376a* + miR.518c + miR.639 + miR.660 | 0,79 | 0,208790148 |
| miR.609 + miR.220a + miR.29a + miR.369.3p + miR.376a* + miR.494 + miR.518c + miR.338 | 0,79 | 0,309376449 |

**Table 9: clusters of 9 miRNAs**

| **Cluster of 9 miRs** | **c_tau level** | **pv compared to top signature miR.558 + miR.609 + miR.603 + miR.220a + miR.26a + miR.29a + miR.369.3p + miR.376a* + miR.518c** |
|---|---|---|
| miR.558 + miR.609 + miR.603 + miR.220a + miR.26a + miR.29a + miR.369.3p + miR.376a* + miR.518c | 0,81 | NaN |
| miR.558 + miR.609 + miR.603 + miR.220a + miR.29a + miR.369.3p + miR.376a* + miR.518c + miR.660 | 0,81 | 0,405184155 |
| miR.558 + miR.609 + miR.603 + miR.220a + miR.519b + miR.29a + miR.369.3p + miR.376a* + miR.518c | 0,81 | 0,246448719 |
| miR.558 + miR.609 + miR.603 + miR.520f + miR.220a + miR.29a + miR.369.3p + miR.376a* + miR.518c | 0,81 | 0,196840946 |
| miR.558 + miR.609 + miR.520f + miR.220a + miR.29a + miR.369.3p + miR.376a* + miR.518c + miR.660 | 0,81 | 0,329909732 |
| miR.558 + miR.609 + miR.220a + miR.26a + miR.29a + miR.369.3p + miR.376a* + miR.518c + miR.660 | 0,81 | 0,128466451 |
| miR.558 + miR.609 + miR.603 + miR.220a + miR.130a + miR.29a + miR.369.3p + miR.376a* + miR.518c | 0,81 | 0,116317947 |
| miR.558 + miR.609 + miR.603 + miR.220a + miR.519b + miR.29a + miR.362 + miR.376a* + miR.518c | 0,81 | 0,447554582 |
| miR.558 + miR.609 + miR.603 + miR.220a + miR.26a + miR.29a + miR.362 + miR.376a* + miR.518c | 0,81 | 0,059893333 |
| miR.558 + miR.609 + miR.603 + miR.520f + miR.29a + miR.369.3p + miR.376a* + miR.518c + miR.338 | 0,81 | 0,463029319 |
| miR.558 + miR.609 + miR.603 + miR.220a + miR.26a + miR.29a + miR.376a* + miR.518c + miR.660 | 0,81 | 0,039588187 |
| miR.558 + miR.609 + miR.603 + miR.220a + miR.26a + miR.29a + miR.376a* + miR.518c + miR.639 | 0,81 | 0,012535164 |
| miR.558 + miR.609 + miR.520f + miR.220a + miR.29a + miR.369.3p + miR.376a* + miR.518c + miR.338 | 0,81 | 0,400683559 |
| miR.558 + miR.609 + miR.220a + miR.29a + miR.362 + miR.369.3p + miR.376a* + miR.518c + miR.660 | 0,81 | 0,311774714 |
| miR.558 + miR.609 + miR.220a + miR.26a + miR.29a + miR.369.3p + miR.376a* + miR.518c + miR.338 | 0,81 | 0,110070757 |
| miR.558 + miR.609 + miR.603 + miR.220a + miR.519b + miR.26a + miR.29a + miR.376a* + miR.518c | 0,81 | 0,139809243 |
| miR.558 + miR.609 + miR.603 + miR.220a + miR.29a + miR.362 + miR.369.3p + miR.376a* + miR.518c | 0,81 | 0,190392203 |
| miR.558 + miR.609 + miR.603 + miR.220a + miR.29a + miR.369.3p + miR.376a* + miR.518c + miR.639 | 0,81 | 0,081427064 |
| miR.558 + miR.609 + miR.603 + miR.220a + miR.26a + miR.29a + miR.376a* + miR.518c + miR.338 | 0,81 | 0,048680066 |
| miR.558 + miR.609 + miR.603 + miR.220a + miR.29a + miR.369.3p + miR.376a* + miR.518c + miR.338 | 0,81 | 0,186226373 |
| miR.558 + miR.609 + miR.603 + miR.520f + miR.220a + miR.29a + miR.362 + miR.376a* + miR.518c | 0,81 | 0,09905613 |
| miR.558 + miR.609 + miR.603 + miR.220a + miR.29a + miR.369.3p + miR.376a* + miR.494 + miR.518c | 0,81 | 0,130611427 |
| miR.558 + miR.609 + miR.603 + miR.220a + miR.26a + miR.29a + miR.376a* + miR.494 + miR.518c | 0,81 | 0,005338207 |
| miR.558 + miR.609 + miR.220a + miR.26a + miR.29a + miR.369.3p + miR.376a* + miR.494 + miR.518c | 0,81 | 0,320717135 |
| miR.558 + miR.609 + miR.520f + miR.29a + miR.369.3p + miR.376a* + miR.518c + miR.660 + miR.338 | 0,81 | 0,42529961 |
| miR.558 + miR.609 + miR.520f + miR.220a + miR.26a + miR.29a + miR.369.3p + miR.376a* + miR.518c | 0,81 | 0,334919585 |
| miR.558 + miR.609 + miR.603 + miR.520f + miR.29a + miR.369.3p + miR.376a* + miR.518c + miR.639 | 0,81 | 0,067091161 |
| miR.558 + miR.609 + miR.603 + miR.220a + miR.130a + miR.26a + miR.29a + miR.376a* + miR.518c | 0,81 | 0,014626567 |
| miR.558 + miR.609 + miR.520f + miR.220a + miR.29a + miR.362 + miR.369.3p + miR.376a* + miR.518c | 0,81 | 0,495987113 |
| miR.558 + miR.609 + miR.220a + miR.26a + miR.29a + miR.362 + miR.369.3p + miR.376a* + miR.518c | 0,81 | 0,221787351 |
| miR.558 + miR.609 + miR.603 + miR.520f + miR.220a + miR.29a + miR.376a* + miR.518c + miR.660 | 0,81 | 0,099997395 |
| miR.558 + miR.609 + miR.220a + miR.26a + miR.29a + miR.369.3p + miR.376a* + miR.518c + miR.639 | 0,81 | 0,329506061 |
| miR.558 + miR.609 + miR.603 + miR.220a + miR.519b + miR.29a + miR.376a* + miR.494 + miR.518c | 0,81 | 0,324863522 |
| miR.558 + miR.609 + miR.603 + miR.520f + miR.220a + miR.29a + miR.376a* + miR.518c + miR.338 | 0,81 | 0,127330549 |
| miR.558 + miR.609 + miR.603 + miR.520f + miR.29a + miR.369.3p + miR.376a* + miR.518c + miR.660 | 0,81 | 0,204887351 |
| miR.558 + miR.609 + miR.220a + miR.29a + miR.369.3p + miR.376a* + miR.494 + miR.518c + miR.660 | 0,81 | 0,427310852 |
| miR.558 + miR.609 + miR.603 + miR.26a + miR.29a + miR.369.3p + miR.376a* + miR.518c + miR.639 | 0,81 | 0,232508147 |
| miR.558 + miR.609 + miR.603 + miR.26a + miR.29a + miR.369.3p + miR.376a* + miR.518c + miR.338 | 0,81 | 0,426798872 |
| miR.558 + miR.609 + miR.603 + miR.520f + miR.220a + miR.26a + miR.29a + miR.376a* + miR.518c | 0,81 | 0,015632232 |
| miR.558 + miR.609 + miR.220a + miR.29a + miR.369.3p + miR.376a* + miR.518c + miR.639 + miR.660 | 0,81 | 0,406477895 |
| miR.558 + miR.609 + miR.603 + miR.520f + miR.220a + miR.519b + miR.29a + miR.376a* + miR.518c | 0,81 | 0,107863762 |
| miR.558 + miR.609 + miR.220a + miR.519b + miR.29a + miR.369.3p + miR.376a* + miR.518c + miR.660 | 0,81 | 0,378455134 |
| miR.558 + miR.609 + miR.520f + miR.220a + miR.29a + miR.376a* + miR.518c + miR.660 + miR.338 | 0,81 | 0,354575192 |
| miR.558 + miR.609 + miR.220a + miR.29a + miR.369.3p + miR.376a* + miR.518c + miR.660 + miR.338 | 0,81 | 0,380394727 |
| miR.558 + miR.609 + miR.520f + miR.220a + miR.29a + miR.369.3p + miR.376a* + miR.494 + miR.518c | 0,81 | 0,399172056 |
| miR.558 + miR.609 + miR.520f + miR.220a + miR.29a + miR.376a* + miR.494 + miR.518c + miR.660 | 0,81 | 0,207190665 |
| miR.558 + miR.609 + miR.220a + miR.519b + miR.26a + miR.29a + miR.369.3p + miR.376a* + miR.518c | 0,81 | 0,296070608 |
| miR.558 + miR.609 + miR.520f + miR.220a + miR.29a + miR.369.3p + miR.376a* + miR.518c + miR.639 | 0,81 | 0,409887674 |
| miR.558 + miR.609 + miR.603 + miR.520f + miR.26a + miR.29a + miR.369.3p + miR.376a* + miR.518c | 0,81 | 0,065157593 |
| miR.558 + miR.609 + miR.603 + miR.220a + miR.519b + miR.29a + miR.376a* + miR.518c + miR.338 | 0,81 | 0,379025909 |
| miR.558 + miR.609 + miR.220a + miR.130a + miR.26a + miR.29a + miR.369.3p + miR.376a* + miR.518c | 0,81 | 0,237552871 |
| miR.558 + miR.609 + miR.603 + miR.520f + miR.220a + miR.29a + miR.376a* + miR.518c + miR.639 | 0,81 | 0,024425329 |
| miR.558 + miR.609 + miR.520f + miR.29a + miR.369.3p + miR.376a* + miR.518c + miR.639 + miR.338 | 0,81 | 0,413149685 |
| miR.558 + miR.609 + miR.220a + miR.130a + miR.29a + miR.369.3p + miR.376a* + miR.518c + miR.660 | 0,81 | 0,481305199 |
| miR.558 + miR.609 + miR.603 + miR.220a + miR.519b + miR.29a + miR.376a* + miR.518c + miR.639 | 0,81 | 0,259947051 |
| miR.558 + miR.609 + miR.520f + miR.29a + miR.376a* + miR.518c + miR.639 + miR.660 + miR.338 | 0,81 | 0,460534976 |
| miR.558 + miR.609 + miR.603 + miR.220a + miR.519b + miR.130a + miR.29a + miR.376a* + miR.518c | 0,81 | 0,24708209 |
| miR.558 + miR.609 + miR.603 + miR.26a + miR.29a + miR.369.3p + miR.376a* + miR.494 + miR.518c | 0,81 | 0,257662026 |
| miR.558 + miR.609 + miR.603 + miR.220a + miR.519b + miR.29a + miR.376a* + miR.518c + miR.660 | 0,81 | 0,424422464 |
| miR.558 + miR.609 + miR.520f + miR.220a + miR.130a + miR.29a + miR.369.3p + miR.376a* + miR.518c | 0,81 | 0,479425443 |
| miR.558 + miR.609 + miR.603 + miR.220a + miR.29a + miR.362 + miR.376a* + miR.518c + miR.639 | 0,81 | 0,13589269 |
| miR.558 + miR.609 + miR.520f + miR.29a + miR.376a* + miR.494 + miR.518c + miR.660 + miR.338 | 0,81 | 0,36967044 |
| miR.558 + miR.609 + miR.603 + miR.520f + miR.29a + miR.369.3p + miR.376a* + miR.494 + miR.518c | 0,81 | 0,089395994 |
| miR.558 + miR.609 + miR.520f + miR.220a + miR.519b + miR.29a + miR.369.3p + miR.376a* + miR.518c | 0,81 | 0,298435504 |
| miR.558 + miR.609 + miR.603 + miR.520f + miR.220a + miR.29a + miR.376a* + miR.494 + miR.518c | 0,81 | 0,010543759 |
| miR.558 + miR.609 + miR.520f + miR.220a + miR.29a + miR.362 + miR.376a* + miR.518c + miR.338 | 0,81 | 0,480715861 |
| miR.558 + miR.609 + miR.603 + miR.220a + miR.130a + miR.29a + miR.362 + miR.376a* + miR.518c | 0,81 | 0,081844216 |
| miR.558 + miR.609 + miR.520f + miR.29a + miR.369.3p + miR.376a* + miR.494 + miR.518c + miR.338 | 0,81 | 0,342503816 |
| miR.558 + miR.609 + miR.603 + miR.520f + miR.29a + miR.362 + miR.369.3p + miR.376a* + miR.518c | 0,81 | 0,098699779 |
| miR.558 + miR.609 + miR.603 + miR.220a + miR.29a + miR.362 + miR.376a* + miR.518c + miR.338 | 0,81 | 0,164711094 |
| miR.558 + miR.609 + miR.603 + miR.26a + miR.29a + miR.369.3p + miR.376a* + miR.518c + miR.660 | 0,81 | 0,218296942 |
| miR.558 + miR.609 + miR.603 + miR.520f + miR.130a + miR.29a + miR.369.3p + miR.376a* + miR.518c | 0,81 | 0,092864134 |
| miR.558 + miR.609 + miR.603 + miR.520f + miR.519b + miR.29a + miR.369.3p + miR.376a* + miR.518c | 0,81 | 0,114699091 |
| miR.558 + miR.609 + miR.520f + miR.220a + miR.29a + miR.376a* + miR.518c + miR.639 + miR.660 | 0,81 | 0,303649269 |
| miR.558 + miR.609 + miR.220a + miR.130a + miR.29a + miR.369.3p + miR.376a* + miR.518c + miR.639 | 0,81 | 0,297064743 |
| miR.558 + miR.609 + miR.603 + miR.520f + miR.29a + miR.376a* + miR.518c + miR.639 + miR.338 | 0,81 | 0,1815712 |
| miR.558 + miR.609 + miR.520f + miR.220a + miR.29a + miR.362 + miR.376a* + miR.494 + miR.518c | 0,81 | 0,21918321 |
| miR.558 + miR.609 + miR.520f + miR.220a + miR.29a + miR.362 + miR.376a* + miR.518c + miR.639 | 0,81 | 0,263170677 |
| miR.558 + miR.609 + miR.220a + miR.26a + miR.29a + miR.362 + miR.376a* + miR.494 + miR.518c | 0,81 | 0,249212767 |
| miR.558 + miR.609 + miR.520f + miR.26a + miR.29a + miR.369.3p + miR.376a* + miR.518c + miR.338 | 0,81 | 0,488093002 |
| miR.558 + miR.609 + miR.603 + miR.130a + miR.26a + miR.29a + miR.369.3p + miR.376a* + miR.518c | 0,81 | 0,174142711 |
| miR.558 + miR.609 + miR.520f + miR.220a + miR.519b + miR.29a + miR.362 + miR.376a* + miR.518c | 0,81 | 0,425943522 |
| miR.558 + miR.609 + miR.603 + miR.220a + miR.29a + miR.376a* + miR.518c + miR.639 + miR.660 | 0,81 | 0,08633331 |
| miR.558 + miR.609 + miR.603 + miR.519b + miR.26a + miR.29a + miR.369.3p + miR.376a* + miR.518c | 0,81 | 0,136399791 |
| miR.558 + miR.609 + miR.220a + miR.519b + miR.130a + miR.29a + miR.369.3p + miR.376a* + miR.518c | 0,81 | 0,368636506 |
| miR.558 + miR.609 + miR.603 + miR.26a + miR.29a + miR.362 + miR.369.3p + miR.376a* + miR.518c | 0,81 | 0,216977335 |
| miR.558 + miR.609 + miR.520f + miR.130a + miR.29a + miR.369.3p + miR.376a* + miR.518c + miR.338 | 0,81 | 0,35581821 |
| miR.558 + miR.609 + miR.520f + miR.29a + miR.369.3p + miR.376a* + miR.518c + miR.639 + miR.660 | 0,81 | 0,363334394 |
| miR.558 + miR.609 + miR.520f + miR.220a + miR.519b + miR.29a + miR.376a* + miR.518c + miR.660 | 0,81 | 0,264417081 |
| miR.558 + miR.609 + miR.603 + miR.130a + miR.29a + miR.369.3p + miR.376a* + miR.518c + miR.639 | 0,81 | 0,10961265 |
| miR.558 + miR.609 + miR.520f + miR.29a + miR.362 + miR.369.3p + miR.376a* + miR.518c + miR.338 | 0,81 | 0,348273131 |
| miR.558 + miR.609 + miR.603 + miR.520f + miR.220a + miR.130a + miR.29a + miR.376a* + miR.518c | 0,8 | 0,020094622 |
| miR.558 + miR.609 + miR.520f + miR.220a + miR.26a + miR.29a + miR.376a* + miR.518c + miR.660 | 0,8 | 0,162238347 |
| miR.558 + miR.609 + miR.520f + miR.29a + miR.369.3p + miR.376a* + miR.494 + miR.518c + miR.660 | 0,8 | 0,224746043 |
| miR.558 + miR.609 + miR.520f + miR.220a + miR.26a + miR.29a + miR.362 + miR.376a* + miR.518c | 0,8 | 0,232798065 |
| miR.558 + miR.609 + miR.603 + miR.220a + miR.130a + miR.29a + miR.376a* + miR.518c + miR.660 | 0,8 | 0,048798642 |
| miR.558 + miR.609 + miR.520f + miR.220a + miR.130a + miR.29a + miR.362 + miR.376a* + miR.518c | 0,8 | 0,264567693 |
| miR.558 + miR.609 + miR.603 + miR.220a + miR.29a + miR.376a* + miR.518c + miR.660 + miR.338 | 0,8 | 0,100789797 |
| miR.558 + miR.609 + miR.520f + miR.220a + miR.29a + miR.362 + miR.376a* + miR.518c + miR.660 | 0,8 | 0,204969048 |
| miR.558 + miR.609 + miR.520f + miR.220a + miR.29a + miR.376a* + miR.494 + miR.518c + miR.338 | 0,8 | 0,224810798 |
| miR.558 + miR.609 + miR.603 + miR.220a + miR.29a + miR.362 + miR.376a* + miR.518c + miR.660 | 0,8 | 0,075776502 |
| miR.558 + miR.609 + miR.603 + miR.520f + miR.29a + miR.362 + miR.376a* + miR.518c + miR.639 | 0,8 | 0,092813236 |
| miR.558 + miR.609 + miR.220a + miR.130a + miR.29a + miR.369.3p + miR.376a* + miR.494 + miR.518c | 0,8 | 0,296308912 |
| miR.558 + miR.609 + miR.520f + miR.220a + miR.130a + miR.29a + miR.376a* + miR.518c + miR.660 | 0,8 | 0,195599244 |
| miR.558 + miR.609 + miR.520f + miR.519b + miR.29a + miR.369.3p + miR.376a* + miR.518c + miR.338 | 0,8 | 0,479936725 |
| miR.558 + miR.609 + miR.603 + miR.520f + miR.29a + miR.376a* + miR.494 + miR.518c + miR.338 | 0,8 | 0,211200269 |
| miR.558 + miR.609 + miR.520f + miR.29a + miR.362 + miR.376a* + miR.518c + miR.660 + miR.338 | 0,8 | 0,327412429 |
| miR.558 + miR.609 + miR.603 + miR.520f + miR.29a + miR.362 + miR.376a* + miR.518c + miR.338 | 0,8 | 0,331718628 |
| miR.558 + miR.609 + miR.220a + miR.26a + miR.29a + miR.376a* + miR.494 + miR.518c + miR.660 | 0,8 | 0,215177023 |
| miR.558 + miR.609 + miR.520f + miR.26a + miR.29a + miR.376a* + miR.518c + miR.660 + miR.338 | 0,8 | 0,342947707 |
| miR.558 + miR.609 + miR.603 + miR.520f + miR.29a + miR.376a* + miR.518c + miR.639 + miR.660 | 0,8 | 0,215973331 |
| miR.558 + miR.609 + miR.603 + miR.520f + miR.29a + miR.376a* + miR.494 + miR.518c + miR.639 | 0,8 | 0,016870957 |
| miR.558 + miR.609 + miR.520f + miR.29a + miR.369.3p + miR.376a* + miR.494 + miR.518c + miR.639 | 0,8 | 0,115598303 |
| miR.558 + miR.609 + miR.520f + miR.220a + miR.519b + miR.29a + miR.376a* + miR.494 + miR.518c | 0,8 | 0,232468645 |
| miR.558 + miR.609 + miR.603 + miR.520f + miR.29a + miR.376a* + miR.518c + miR.660 + miR.338 | 0,8 | 0,373285911 |
| miR.558 + miR.609 + miR.603 + miR.220a + miR.29a + miR.376a* + miR.494 + miR.518c + miR.660 | 0,8 | 0,104374757 |
| miR.558 + miR.609 + miR.220a + miR.519b + miR.26a + miR.29a + miR.376a* + miR.494 + miR.518c | 0,8 | 0,279417062 |
| miR.558 + miR.609 + miR.603 + miR.520f + miR.519b + miR.29a + miR.376a* + miR.494 + miR.518c | 0,8 | 0,062938142 |
| miR.558 + miR.609 + miR.520f + miR.220a + miR.29a + miR.376a* + miR.518c + miR.639 + miR.338 | 0,8 | 0,236017156 |
| miR.558 + miR.609 + miR.220a + miR.26a + miR.29a + miR.376a* + miR.494 + miR.518c + miR.338 | 0,8 | 0,221173558 |
| miR.558 + miR.609 + miR.220a + miR.519b + miR.26a + miR.29a + miR.362 + miR.376a* + miR.518c | 0,8 | 0,252819696 |
| miR.558 + miR.609 + miR.520f + miR.29a + miR.362 + miR.369.3p + miR.376a* + miR.518c + miR.660 | 0,8 | 0,285434425 |
| miR.558 + miR.609 + miR.603 + miR.29a + miR.362 + miR.369.3p + miR.376a* + miR.518c + miR.660 | 0,8 | 0,259589577 |
| miR.558 + miR.609 + miR.603 + miR.220a + miR.130a + miR.29a + miR.376a* + miR.494 + miR.518c | 0,8 | 0,006847963 |
| miR.558 + miR.609 + miR.520f + miR.220a + miR.519b + miR.29a + miR.376a* + miR.518c + miR.338 | 0,8 | 0,390803298 |
| miR.558 + miR.609 + miR.520f + miR.220a + miR.130a + miR.29a + miR.376a* + miR.518c + miR.338 | 0,8 | 0,308429664 |
| miR.558 + miR.609 + miR.520f + miR.130a + miR.29a + miR.376a* + miR.518c + miR.639 + miR.660 | 0,8 | 0,172455922 |
| miR.558 + miR.609 + miR.520f + miR.519b + miR.29a + miR.376a* + miR.518c + miR.660 + miR.338 | 0,8 | 0,351658298 |
| miR.558 + miR.609 + miR.220a + miR.26a + miR.29a + miR.362 + miR.376a* + miR.518c + miR.338 | 0,8 | 0,35269588 |
| miR.558 + miR.609 + miR.220a + miR.26a + miR.29a + miR.362 + miR.376a* + miR.518c + miR.639 | 0,8 | 0,170694315 |
| miR.558 + miR.609 + miR.220a + miR.130a + miR.29a + miR.369.3p + miR.376a* + miR.518c + miR.338 | 0,8 | 0,373269862 |
| miR.558 + miR.609 + miR.220a + miR.130a + miR.26a + miR.29a + miR.376a* + miR.494 + miR.518c | 0,8 | 0,20149379 |
| miR.558 + miR.609 + miR.220a + miR.519b + miR.29a + miR.362 + miR.369.3p + miR.376a* + miR.518c | 0,8 | 0,394859734 |
| miR.558 + miR.609 + miR.603 + miR.520f + miR.29a + miR.362 + miR.376a* + miR.494 + miR.518c | 0,8 | 0,078778091 |
| miR.558 + miR.609 + miR.603 + miR.29a + miR.369.3p + miR.376a* + miR.518c + miR.639 + miR.338 | 0,8 | 0,166924142 |
| miR.558 + miR.609 + miR.220a + miR.130a + miR.29a + miR.362 + miR.369.3p + miR.376a* + miR.518c | 0,8 | 0,237585098 |
| miR.558 + miR.609 + miR.520f + miR.220a + miR.26a + miR.29a + miR.376a* + miR.518c + miR.338 | 0,8 | 0,306992027 |
| miR.558 + miR.609 + miR.520f + miR.26a + miR.29a + miR.369.3p + miR.376a* + miR.518c + miR.660 | 0,8 | 0,184596775 |
| miR.558 + miR.609 + miR.520f + miR.26a + miR.29a + miR.369.3p + miR.376a* + miR.518c + miR.639 | 0,8 | 0,127457221 |
| miR.558 + miR.609 + miR.520f + miR.29a + miR.362 + miR.376a* + miR.518c + miR.639 + miR.338 | 0,8 | 0,314682404 |
| miR.558 + miR.609 + miR.220a + miR.130a + miR.26a + miR.29a + miR.362 + miR.376a* + miR.518c | 0,8 | 0,196230158 |
| miR.558 + miR.609 + miR.220a + miR.26a + miR.29a + miR.376a* + miR.518c + miR.639 + miR.660 | 0,8 | 0,246176262 |
| miR.609 + miR.603 + miR.520f + miR.220a + miR.29a + miR.369.3p + miR.376a* + miR.518c + miR.660 | 0,8 | 0,221039373 |
| miR.558 + miR.609 + miR.603 + miR.520f + miR.130a + miR.29a + miR.376a* + miR.518c + miR.338 | 0,8 | 0,25365842 |
| miR.558 + miR.609 + miR.220a + miR.26a + miR.29a + miR.376a* + miR.494 + miR.518c + miR.639 | 0,8 | 0,170983241 |
| miR.609 + miR.603 + miR.220a + miR.29a + miR.369.3p + miR.376a* + miR.494 + miR.518c + miR.660 | 0,8 | 0,474796754 |
| miR.558 + miR.609 + miR.520f + miR.29a + miR.376a* + miR.494 + miR.518c + miR.639 + miR.338 | 0,8 | 0,157646369 |
| miR.558 + miR.609 + miR.220a + miR.29a + miR.362 + miR.369.3p + miR.376a* + miR.518c + miR.639 | 0,8 | 0,175504569 |
| miR.558 + miR.609 + miR.520f + miR.220a + miR.130a + miR.29a + miR.376a* + miR.494 + miR.518c | 0,8 | 0,078147535 |
| miR.558 + miR.609 + miR.220a + miR.29a + miR.362 + miR.369.3p + miR.376a* + miR.494 + miR.518c | 0,8 | 0,190649471 |
| miR.558 + miR.609 + miR.520f + miR.220a + miR.26a + miR.29a + miR.376a* + miR.494 + miR.518c | 0,8 | 0,117757749 |
| miR.558 + miR.609 + miR.520f + miR.220a + miR.29a + miR.376a* + miR.494 + miR.518c + miR.639 | 0,8 | 0,071425949 |
| miR.609 + miR.603 + miR.220a + miR.29a + miR.362 + miR.369.3p + miR.376a* + miR.518c + miR.660 | 0,8 | 0,48849136 |
| miR.558 + miR.609 + miR.520f + miR.29a + miR.362 + miR.369.3p + miR.376a* + miR.518c + miR.639 | 0,8 | 0,131590258 |
| miR.558 + miR.609 + miR.603 + miR.520f + miR.26a + miR.29a + miR.376a* + miR.518c + miR.639 | 0,8 | 0,013949842 |
| miR.558 + miR.609 + miR.603 + miR.520f + miR.29a + miR.376a* + miR.494 + miR.518c + miR.660 | 0,8 | 0,074032881 |
| miR.558 + miR.609 + miR.520f + miR.130a + miR.29a + miR.369.3p + miR.376a* + miR.518c + miR.660 | 0,8 | 0,184603818 |
| miR.558 + miR.609 + miR.520f + miR.29a + miR.376a* + miR.494 + miR.518c + miR.639 + miR.660 | 0,8 | 0,189111019 |
| miR.558 + miR.609 + miR.220a + miR.519b + miR.29a + miR.369.3p + miR.376a* + miR.518c + miR.639 | 0,8 | 0,365639771 |
| miR.558 + miR.609 + miR.520f + miR.130a + miR.29a + miR.376a* + miR.494 + miR.518c + miR.660 | 0,8 | 0,079497418 |
| miR.558 + miR.609 + miR.220a + miR.26a + miR.29a + miR.376a* + miR.518c + miR.660 + miR.338 | 0,8 | 0,288432683 |
| miR.558 + miR.609 + miR.220a + miR.519b + miR.29a + miR.369.3p + miR.376a* + miR.518c + miR.338 | 0,8 | 0,391496807 |
| miR.558 + miR.609 + miR.520f + miR.220a + miR.26a + miR.29a + miR.376a* + miR.518c + miR.639 | 0,8 | 0,061782416 |
| miR.558 + miR.609 + miR.603 + miR.220a + miR.29a + miR.362 + miR.376a* + miR.494 + miR.518c | 0,8 | 0,121198281 |
| miR.558 + miR.609 + miR.220a + miR.29a + miR.362 + miR.369.3p + miR.376a* + miR.518c + miR.338 | 0,8 | 0,222233599 |
| miR.558 + miR.609 + miR.220a + miR.26a + miR.29a + miR.362 + miR.376a* + miR.518c + miR.660 | 0,8 | 0,188586623 |
| miR.558 + miR.609 + miR.220a + miR.519b + miR.26a + miR.29a + miR.376a* + miR.518c + miR.660 | 0,8 | 0,226750233 |
| miR.558 + miR.609 + miR.520f + miR.220a + miR.130a + miR.26a + miR.29a + miR.376a* + miR.518c | 0,8 | 0,098943942 |
| miR.558 + miR.609 + miR.603 + miR.520f + miR.130a + miR.29a + miR.376a* + miR.518c + miR.639 | 0,8 | 0,017256413 |
| miR.558 + miR.609 + miR.220a + miR.130a + miR.26a + miR.29a + miR.376a* + miR.518c + miR.660 | 0,8 | 0,176732056 |
| miR.558 + miR.609 + miR.520f + miR.26a + miR.29a + miR.369.3p + miR.376a* + miR.494 + miR.518c | 0,8 | 0,153093676 |
| miR.558 + miR.609 + miR.603 + miR.130a + miR.29a + miR.369.3p + miR.376a* + miR.494 + miR.518c | 0,8 | 0,074838999 |
| miR.558 + miR.609 + miR.603 + miR.520f + miR.519b + miR.29a + miR.376a* + miR.518c + miR.639 | 0,8 | 0,017218361 |
| miR.558 + miR.609 + miR.520f + miR.220a + miR.519b + miR.26a + miR.29a + miR.376a* + miR.518c | 0,8 | 0,151358087 |
| miR.558 + miR.609 + miR.603 + miR.520f + miR.519b + miR.29a + miR.362 + miR.376a* + miR.518c | 0,8 | 0,080317105 |
| miR.558 + miR.609 + miR.520f + miR.130a + miR.29a + miR.369.3p + miR.376a* + miR.518c + miR.639 | 0,8 | 0,134521813 |
| miR.558 + miR.609 + miR.520f + miR.519b + miR.29a + miR.369.3p + miR.376a* + miR.518c + miR.639 | 0,8 | 0,144018839 |
| miR.558 + miR.609 + miR.520f + miR.29a + miR.362 + miR.376a* + miR.494 + miR.518c + miR.338 | 0,8 | 0,328546416 |
| miR.558 + miR.609 + miR.603 + miR.220a + miR.29a + miR.376a* + miR.494 + miR.518c + miR.639 | 0,8 | 0,019253644 |
| miR.558 + miR.609 + miR.520f + miR.29a + miR.362 + miR.369.3p + miR.376a* + miR.494 + miR.518c | 0,8 | 0,106348993 |
| miR.558 + miR.609 + miR.603 + miR.520f + miR.26a + miR.29a + miR.376a* + miR.518c + miR.338 | 0,8 | 0,151958651 |
| miR.558 + miR.609 + miR.520f + miR.519b + miR.29a + miR.369.3p + miR.376a* + miR.518c + miR.660 | 0,8 | 0,182822237 |
| miR.558 + miR.609 + miR.520f + miR.130a + miR.29a + miR.376a* + miR.494 + miR.518c + miR.639 | 0,8 | 0,055727295 |
| miR.558 + miR.609 + miR.520f + miR.220a + miR.130a + miR.29a + miR.376a* + miR.518c + miR.639 | 0,8 | 0,086087625 |
| miR.558 + miR.609 + miR.603 + miR.220a + miR.29a + miR.376a* + miR.518c + miR.639 + miR.338 | 0,8 | 0,012546125 |
| miR.558 + miR.609 + miR.220a + miR.26a + miR.29a + miR.376a* + miR.518c + miR.639 + miR.338 | 0,8 | 0,185309344 |
| miR.558 + miR.609 + miR.130a + miR.26a + miR.29a + miR.369.3p + miR.376a* + miR.518c + miR.639 | 0,8 | 0,122208698 |
| miR.609 + miR.603 + miR.220a + miR.26a + miR.29a + miR.369.3p + miR.376a* + miR.494 + miR.518c | 0,8 | 0,427593084 |
| miR.558 + miR.609 + miR.603 + miR.220a + miR.130a + miR.29a + miR.376a* + miR.518c + miR.639 | 0,8 | 0,011082239 |
| miR.558 + miR.609 + miR.520f + miR.220a + miR.519b + miR.130a + miR.29a + miR.376a* + miR.518c | 0,8 | 0,136140854 |
| miR.558 + miR.609 + miR.603 + miR.29a + miR.369.3p + miR.376a* + miR.518c + miR.660 + miR.338 | 0,8 | 0,16699956 |
| miR.558 + miR.609 + miR.26a + miR.29a + miR.369.3p + miR.376a* + miR.518c + miR.639 + miR.338 | 0,8 | 0,429224314 |
| miR.558 + miR.609 + miR.603 + miR.520f + miR.130a + miR.29a + miR.362 + miR.376a* + miR.518c | 0,8 | 0,060663582 |
| miR.558 + miR.609 + miR.603 + miR.520f + miR.519b + miR.29a + miR.376a* + miR.518c + miR.338 | 0,8 | 0,265640636 |
| miR.558 + miR.609 + miR.603 + miR.220a + miR.130a + miR.29a + miR.376a* + miR.518c + miR.338 | 0,8 | 0,018996692 |
| miR.558 + miR.609 + miR.520f + miR.29a + miR.362 + miR.376a* + miR.494 + miR.518c + miR.639 | 0,8 | 0,115205009 |
| miR.558 + miR.609 + miR.603 + miR.26a + miR.29a + miR.376a* + miR.518c + miR.639 + miR.338 | 0,8 | 0,198914508 |
| miR.558 + miR.609 + miR.603 + miR.520f + miR.130a + miR.29a + miR.376a* + miR.494 + miR.518c | 0,8 | 0,032853655 |
| miR.558 + miR.609 + miR.603 + miR.220a + miR.29a + miR.376a* + miR.494 + miR.518c + miR.338 | 0,8 | 0,020442168 |
| miR.558 + miR.609 + miR.603 + miR.29a + miR.369.3p + miR.376a* + miR.518c + miR.639 + miR.660 | 0,8 | 0,296530549 |
| miR.558 + miR.609 + miR.220a + miR.519b + miR.29a + miR.369.3p + miR.376a* + miR.494 + miR.518c | 0,8 | 0,389441123 |
| miR.558 + miR.609 + miR.520f + miR.130a + miR.29a + miR.369.3p + miR.376a* + miR.494 + miR.518c | 0,8 | 0,136097883 |
| miR.609 + miR.603 + miR.220a + miR.26a + miR.29a + miR.369.3p + miR.376a* + miR.518c + miR.660 | 0,8 | 0,253503881 |
| miR.558 + miR.609 + miR.603 + miR.520f + miR.26a + miR.29a + miR.376a* + miR.494 + miR.518c | 0,8 | 0,029060967 |
| miR.558 + miR.609 + miR.220a + miR.519b + miR.130a + miR.29a + miR.376a* + miR.494 + miR.518c | 0,8 | 0,15282261 |
| miR.558 + miR.609 + miR.220a + miR.130a + miR.29a + miR.376a* + miR.494 + miR.518c + miR.660 | 0,8 | 0,124333642 |
| miR.558 + miR.609 + miR.520f + miR.519b + miR.29a + miR.369.3p + miR.376a* + miR.494 + miR.518c | 0,8 | 0,191950259 |
| miR.558 + miR.609 + miR.130a + miR.26a + miR.29a + miR.369.3p + miR.376a* + miR.494 + miR.518c | 0,8 | 0,069718 |
| miR.558 + miR.609 + miR.603 + miR.29a + miR.362 + miR.369.3p + miR.376a* + miR.518c + miR.338 | 0,8 | 0,119114353 |
| miR.558 + miR.609 + miR.603 + miR.130a + miR.29a + miR.369.3p + miR.376a* + miR.518c + miR.338 | 0,8 | 0,119451203 |
| miR.558 + miR.609 + miR.603 + miR.29a + miR.369.3p + miR.376a* + miR.494 + miR.518c + miR.639 | 0,8 | 0,102353955 |
| miR.609 + miR.603 + miR.220a + miR.519b + miR.29a + miR.369.3p + miR.376a* + miR.518c + miR.660 | 0,8 | 0,271375564 |
| miR.558 + miR.609 + miR.520f + miR.519b + miR.29a + miR.376a* + miR.494 + miR.518c + miR.338 | 0,8 | 0,224800326 |
| miR.558 + miR.609 + miR.603 + miR.29a + miR.369.3p + miR.376a* + miR.494 + miR.518c + miR.660 | 0,8 | 0,08274244 |
| miR.558 + miR.609 + miR.520f + miR.130a + miR.29a + miR.376a* + miR.518c + miR.660 + miR.338 | 0,8 | 0,363852303 |
| miR.558 + miR.609 + miR.603 + miR.520f + miR.26a + miR.29a + miR.362 + miR.376a* + miR.518c | 0,8 | 0,050927502 |
| miR.558 + miR.609 + miR.603 + miR.520f + miR.130a + miR.29a + miR.376a* + miR.518c + miR.660 | 0,8 | 0,092661585 |
| miR.558 + miR.609 + miR.520f + miR.130a + miR.29a + miR.362 + miR.376a* + miR.518c + miR.338 | 0,8 | 0,35349821 |
| miR.558 + miR.609 + miR.26a + miR.29a + miR.369.3p + miR.376a* + miR.494 + miR.518c + miR.338 | 0,8 | 0,373724854 |
| miR.558 + miR.609 + miR.520f + miR.26a + miR.29a + miR.362 + miR.369.3p + miR.376a* + miR.518c | 0,8 | 0,103714601 |
| miR.558 + miR.609 + miR.520f + miR.220a + miR.519b + miR.29a + miR.376a* + miR.518c + miR.639 | 0,8 | 0,111859043 |
| miR.609 + miR.603 + miR.220a + miR.29a + miR.369.3p + miR.376a* + miR.518c + miR.639 + miR.660 | 0,8 | 0,472103206 |
| miR.558 + miR.609 + miR.603 + miR.130a + miR.29a + miR.369.3p + miR.376a* + miR.518c + miR.660 | 0,8 | 0,085614049 |
| miR.558 + miR.609 + miR.603 + miR.519b + miR.29a + miR.369.3p + miR.376a* + miR.518c + miR.660 | 0,8 | 0,203232586 |
| miR.558 + miR.609 + miR.603 + miR.520f + miR.519b + miR.29a + miR.376a* + miR.518c + miR.660 | 0,8 | 0,081620747 |
| miR.558 + miR.609 + miR.603 + miR.519b + miR.29a + miR.369.3p + miR.376a* + miR.518c + miR.639 | 0,8 | 0,130881195 |
| miR.558 + miR.609 + miR.603 + miR.519b + miR.29a + miR.362 + miR.369.3p + miR.376a* + miR.518c | 0,8 | 0,096326216 |
| miR.558 + miR.609 + miR.220a + miR.29a + miR.369.3p + miR.376a* + miR.494 + miR.518c + miR.639 | 0,8 | 0,172144231 |
| miR.558 + miR.609 + miR.220a + miR.519b + miR.130a + miR.29a + miR.362 + miR.376a* + miR.518c | 0,8 | 0,249366138 |
| miR.558 + miR.609 + miR.603 + miR.520f + miR.29a + miR.362 + miR.376a* + miR.518c + miR.660 | 0,8 | 0,095128794 |
| miR.558 + miR.609 + miR.520f + miR.130a + miR.29a + miR.376a* + miR.494 + miR.518c + miR.338 | 0,8 | 0,209393423 |
| miR.558 + miR.609 + miR.220a + miR.29a + miR.369.3p + miR.376a* + miR.494 + miR.518c + miR.338 | 0,8 | 0,227799703 |
| miR.558 + miR.609 + miR.603 + miR.29a + miR.362 + miR.369.3p + miR.376a* + miR.518c + miR.639 | 0,8 | 0,065582373 |
| miR.558 + miR.609 + miR.520f+ miR.519b + miR.29a + miR.376a* + miR.494 + miR.518c + miR.660 | 0,8 | 0,078551699 |
| miR.558 + miR.609 + miR.520f + miR.26a + miR.29a + miR.376a* + miR.494 + miR.518c + miR.338 | 0,8 | 0,239015696 |
| miR.558 + miR.609 + miR.520f + miR.519b + miR.29a + miR.376a* + miR.518c + miR.639 + miR.338 | 0,8 | 0,213830836 |
| miR.558 + miR.609 + miR.603 + miR.520f + miR.26a + miR.29a + miR.376a* + miR.518c + miR.660 | 0,8 | 0,085213256 |
| miR.558 + miR.609 + miR.520f + miR.519b + miR.26a + miR.29a + miR.369.3p + miR.376a* + miR.518c | 0,8 | 0,133535929 |
| miR.558 + miR.609 + miR.520f + miR.519b + miR.130a + miR.29a + miR.376a* + miR.494 + miR.518c | 0,8 | 0,101755017 |
| miR.558 + miR.609 + miR.520f + miR.26a + miR.29a + miR.362 + miR.376a* + miR.518c + miR.660 | 0,8 | 0,067179551 |
| miR.558 + miR.609 + miR.520f + miR.130a + miR.26a + miR.29a + miR.369.3p + miR.376a* + miR.518c | 0,8 | 0,106466987 |
| miR.558 + miR.609 + miR.520f + miR.519b + miR.29a + miR.376a* + miR.518c + miR.639 + miR.660 | 0,8 | 0,162912065 |
| miR.558 + miR.609 + miR.520f + miR.130a + miR.29a + miR.376a* + miR.518c + miR.639 + miR.338 | 0,8 | 0,180518269 |
| miR.558 + miR.609 + miR.220a + miR.29a + miR.369.3p + miR.376a* + miR.518c + miR.639 + miR.338 | 0,8 | 0,229237386 |
| miR.558 + miR.609 + miR.520f + miR.26a + miR.29a + miR.376a* + miR.518c + miR.639 + miR.338 | 0,8 | 0,17032701 |
| miR.558 + miR.609 + miR.520f + miR.29a + miR.362 + miR.376a* + miR.518c + miR.639 + miR.660 | 0,8 | 0,160777596 |
| miR.558 + miR.609 + miR.520f + miR.26a + miR.29a + miR.376a* + miR.518c + miR.639 + miR.660 | 0,8 | 0,171614654 |
| miR.558 + miR.609 + miR.520f + miR.519b + miR.29a + miR.362 + miR.376a* + miR.518c + miR.338 | 0,8 | 0,428704603 |
| miR.558 + miR.609 + miR.26a + miR.29a + miR.369.3p + miR.376a* + miR.494 + miR.518c + miR.639 | 0,8 | 0,104096385 |
| miR.558 + miR.609 + miR.220a + miR.130a + miR.29a + miR.362 + miR.376a* + miR.494 + miR.518c | 0,8 | 0,13332866 |
| miR.558 + miR.609 + miR.220a + miR.130a + miR.26a + miR.29a + miR.376a* + miR.518c + miR.338 | 0,8 | 0,273608191 |
| miR.558 + miR.609 + miR.603 + miR.130a + miR.29a + miR.376a* + miR.494 + miR.518c + miR.639 | 0,8 | 0,024497224 |
| miR.558 + miR.609 + miR.220a + miR.519b + miR.29a + miR.376a* + miR.494 + miR.518c + miR.660 | 0,8 | 0,206194156 |
| miR.558 + miR.609 + miR.603 + miR.130a + miR.29a + miR.362 + miR.369.3p + miR.376a* + miR.518c | 0,8 | 0,042217641 |
| miR.558 + miR.609 + miR.603 + miR.519b + miR.29a + miR.369.3p + miR.376a* + miR.494 + miR.518c | 0,8 | 0,196342391 |
| miR.558 + miR.609 + miR.220a + miR.130a + miR.29a + miR.362 + miR.376a* + miR.518c + miR.639 | 0,8 | 0,144684567 |
| miR.558 + miR.609 + miR.220a + miR.519b + miR.26a + miR.29a + miR.376a* + miR.518c + miR.338 | 0,8 | 0,26724475 |
| miR.558 + miR.609 + miR.26a + miR.29a + miR.369.3p + miR.376a* + miR.518c + miR.639 + miR.660 | 0,8 | 0,143259995 |
| miR.558 + miR.609 + miR.520f + miR.519b + miR.29a + miR.362 + miR.376a* + miR.518c + miR.660 | 0,8 | 0,07658739 |
| miR.558 + miR.609 + miR.520f + miR.519b + miR.130a + miR.29a + miR.376a* + miR.518c + miR.660 | 0,8 | 0,07674067 |
| miR.558 + miR.609 + miR.520f + miR.519b + miR.29a + miR.362 + miR.369.3p + miR.376a* + miR.518c | 0,8 | 0,099325768 |
| miR.558 + miR.609 + miR.520f + miR.26a + miR.29a + miR.362 + miR.376a* + miR.518c + miR.338 | 0,8 | 0,371461895 |
| miR.558 + miR.609 + miR.520f + miR.130a + miR.29a + miR.362 + miR.369.3p + miR.376a* + miR.518c | 0,8 | 0,074703987 |
| miR.558 + miR.609 + miR.603 + miR.26a + miR.29a + miR.376a* + miR.494 + miR.518c + miR.639 | 0,8 | 0,05069194 |
| miR.558 + miR.609 + miR.29a + miR.362 + miR.369.3p + miR.376a* + miR.518c + miR.660 + miR.338 | 0,8 | 0,365122071 |
| miR.558 + miR.609 + miR.603 + miR.519b + miR.130a + miR.29a + miR.369.3p + miR.376a* + miR.518c | 0,8 | 0,129465375 |
| miR.558 + miR.609 + miR.220a + miR.130a + miR.29a + miR.362 + miR.376a* + miR.518c + miR.338 | 0,8 | 0,150124142 |
| miR.558 + miR.609 + miR.603 + miR.520f + miR.519b + miR.130a + miR.29a + miR.376a* + miR.518c | 0,8 | 0,024607047 |
| miR.558 + miR.609 + miR.520f + miR.29a + miR.362 + miR.376a* + miR.494 + miR.518c + miR.660 | 0,8 | 0,063271826 |
| miR.558 + miR.609 + miR.520f + miR.26a + miR.29a + miR.376a* + miR.494 + miR.518c + miR.660 | 0,8 | 0,064058318 |
| miR.558 + miR.609 + miR.520f + miR.130a + miR.29a + miR.362 + miR.376a* + miR.518c + miR.639 | 0,8 | 0,126667018 |
| miR.609 + miR.603 + miR.220a + miR.29a + miR.369.3p + miR.376a* + miR.518c + miR.660 + miR.338 | 0,8 | 0,332766418 |
| miR.558 + miR.609 + miR.220a + miR.519b + miR.29a + miR.362 + miR.376a* + miR.518c + miR.639 | 0,8 | 0,280490895 |
| miR.558 + miR.609 + miR.520f + miR.26a + miR.29a + miR.362 + miR.376a* + miR.518c + miR.639 | 0,8 | 0,100263046 |
| miR.558 + miR.609 + miR.519b + miR.26a + miR.29a + miR.369.3p + miR.376a* + miR.518c + miR.639 | 0,8 | 0,07995757 |
| miR.558 + miR.609 + miR.220a + miR.519b + miR.29a + miR.362 + miR.376a* + miR.518c + miR.338 | 0,8 | 0,329238753 |
| miR.558 + miR.609 + miR.220a + miR.130a + miR.26a + miR.29a + miR.376a* + miR.518c + miR.639 | 0,8 | 0,129710832 |
| miR.558 + miR.609 + miR.520f + miR.519b + miR.130a + miR.29a + miR.369.3p + miR.376a* + miR.518c | 0,8 | 0,134355095 |
| miR.558 + miR.609 + miR.220a + miR.519b + miR.26a + miR.29a + miR.376a* + miR.518c + miR.639 | 0,8 | 0,251972101 |
| miR.558 + miR.609 + miR.220a + miR.519b + miR.130a + miR.26a + miR.29a + miR.376a* + miR.518c | 0,8 | 0,257965806 |
| miR.558 + miR.609 + miR.603 + miR.520f + miR.519b + miR.26a + miR.29a + miR.376a* + miR.518c | 0,8 | 0,021827111 |
| miR.558 + miR.609 + miR.520f + miR.519b + miR.29a + miR.362 + miR.376a* + miR.518c + miR.639 | 0,8 | 0,100596699 |
| miR.558 + miR.609 + miR.520f + miR.519b + miR.29a + miR.362 + miR.376a* + miR.494 + miR.518c | 0,8 | 0,165993763 |
| miR.558 + miR.609 + miR.603 + miR.519b + miR.29a + miR.369.3p + miR.376a* + miR.518c + miR.338 | 0,8 | 0,257926032 |
| miR.558 + miR.609 + miR.603 + miR.29a + miR.369.3p + miR.376a* + miR.494 + miR.518c + miR.338 | 0,8 | 0,143503023 |
| miR.609 + miR.603 + miR.520f + miR.220a + miR.29a + miR.362 + miR.369.3p + miR.376a* + miR.518c | 0,8 | 0,305273849 |
| miR.558 + miR.609 + miR.220a + miR.29a + miR.362 + miR.376a* + miR.494 + miR.518c + miR.660 | 0,8 | 0,116391653 |
| miR.558 + miR.609 + miR.26a + miR.29a + miR.369.3p + miR.376a* + miR.518c + miR.660 + miR.338 | 0,8 | 0,427902828 |
| miR.558 + miR.609 + miR.520f + miR.519b + miR.29a + miR.376a* + miR.494 + miR.518c + miR.639 | 0,8 | 0,064412715 |
| miR.558 + miR.609 + miR.26a + miR.29a + miR.369.3p + miR.376a* + miR.494 + miR.518c + miR.660 | 0,8 | 0,11552722 |
| miR.558 + miR.609 + miR.520f + miR.26a + miR.29a + miR.376a* + miR.494 + miR.518c + miR.639 | 0,8 | 0,041948152 |
| miR.558 + miR.609 + miR.520f + miR.130a + miR.29a + miR.362 + miR.376a* + miR.494 + miR.518c | 0,8 | 0,108599573 |
| miR.558 + miR.609 + miR.603 + miR.26a + miR.29a + miR.362 + miR.376a* + miR.518c + miR.639 | 0,8 | 0,18904734 |
| miR.558 + miR.609 + miR.603 + miR.520f + miR.130a + miR.26a + miR.29a + miR.376a* + miR.518c | 0,8 | 0,021148867 |
| miR.558 + miR.609 + miR.519b + miR.26a + miR.29a + miR.369.3p + miR.376a* + miR.518c + miR.338 | 0,8 | 0,38337346 |
| miR.558 + miR.609 + miR.220a + miR.29a + miR.362 + miR.376a* + miR.518c + miR.639 + miR.338 | 0,8 | 0,163203959 |
| miR.609 + miR.603 + miR.220a + miR.519b + miR.29a + miR.369.3p + miR.376a* + miR.494 + miR.518c | 0,8 | 0,463138608 |
| miR.609 + miR.603 + miR.220a + miR.130a + miR.29a + miR.369.3p + miR.376a* + miR.518c + miR.660 | 0,8 | 0,481612847 |
| miR.609 + miR.603 + miR.520f + miR.220a + miR.29a + miR.369.3p + miR.376a* + miR.494 + miR.518c | 0,8 | 0,411190909 |
| miR.558 + miR.609 + miR.220a + miR.29a + miR.376a* + miR.494 + miR.518c + miR.639 + miR.660 | 0,8 | 0,115285111 |
| miR.558 + miR.609 + miR.26a + miR.29a + miR.362 + miR.369.3p + miR.376a* + miR.518c + miR.660 | 0,8 | 0,377938106 |
| miR.558 + miR.609 + miR.520f + miR.26a + miR.29a + miR.362 + miR.376a* + miR.494 + miR.518c | 0,8 | 0,097758112 |
| miR.558 + miR.609 + miR.26a + miR.29a + miR.362 + miR.369.3p + miR.376a* + miR.518c + miR.338 | 0,8 | 0,429345055 |
| miR.558 + miR.609 + miR.603 + miR.26a + miR.29a + miR.376a* + miR.518c + miR.639 + miR.660 | 0,8 | 0,183860403 |
| miR.558 + miR.609 + miR.29a + miR.362 + miR.369.3p + miR.376a* + miR.518c + miR.639 + miR.660 | 0,8 | 0,34935503 |
| miR.558 + miR.609 + miR.603 + miR.26a + miR.29a + miR.376a* + miR.494 + miR.518c + miR.338 | 0,8 | 0,078425624 |
| miR.558 + miR.609 + miR.130a + miR.26a + miR.29a + miR.369.3p + miR.376a* + miR.518c + miR.338 | 0,8 | 0,398793295 |
| miR.558 + miR.609 + miR.220a + miR.130a + miR.29a + miR.376a* + miR.494 + miR.518c + miR.639 | 0,8 | 0,025222439 |
| miR.609 + miR.603 + miR.520f + miR.220a + miR.26a + miR.29a + miR.369.3p + miR.376a* + miR.518c | 0,8 | 0,268929606 |
| miR.558 + miR.609 + miR.220a + miR.130a + miR.29a + miR.376a* + miR.518c + miR.639 + miR.660 | 0,8 | 0,111832869 |
| miR.558 + miR.609 + miR.220a + miR.29a + miR.376a* + miR.494 + miR.518c + miR.660 + miR.338 | 0,8 | 0,175735685 |
| miR.558 + miR.609 + miR.520f + miR.130a + miR.26a + miR.29a + miR.376a* + miR.518c + miR.338 | 0,8 | 0,199409176 |
| miR.609 + miR.603 + miR.520f + miR.220a + miR.519b + miR.29a + miR.369.3p + miR.376a* + miR.518c | 0,8 | 0,442508019 |
| miR.609 + miR.603 + miR.220a + miR.130a + miR.26a + miR.29a + miR.369.3p + miR.376a* + miR.518c | 0,8 | 0,427623952 |
| miR.558 + miR.609 + miR.603 + miR.29a + miR.362 + miR.369.3p + miR.376a* + miR.494 + miR.518c | 0,8 | 0,053765849 |
| miR.558 + miR.609 + miR.519b + miR.26a + miR.29a + miR.369.3p + miR.376a* + miR.494 + miR.518c | 0,8 | 0,071596829 |
| miR.558 + miR.609 + miR.220a + miR.519b + miR.130a + miR.29a + miR.376a* + miR.518c + miR.660 | 0,8 | 0,155742136 |
| miR.558 + miR.609 + miR.220a + miR.519b + miR.29a + miR.376a* + miR.518c + miR.639 + miR.660 | 0,8 | 0,12950482 |
| miR.609 + miR.603 + miR.220a + miR.26a + miR.29a + miR.369.3p + miR.376a* + miR.518c + miR.639 | 0,8 | 0,471126345 |
| miR.609 + miR.603 + miR.220a + miR.26a + miR.29a + miR.369.3p + miR.376a* + miR.518c + miR.338 | 0,8 | 0,174744472 |
| miR.558 + miR.609 + miR.220a + miR.519b + miR.29a + miR.362 + miR.376a* + miR.518c + miR.660 | 0,8 | 0,136241522 |
| miR.558 + miR.609 + miR.26a + miR.29a + miR.362 + miR.369.3p + miR.376a* + miR.494 + miR.518c | 0,8 | 0,084003226 |
| miR.558 + miR.609 + miR.520f + miR.519b + miR.130a + miR.29a + miR.376a* + miR.518c + miR.338 | 0,8 | 0,269413661 |
| miR.609 + miR.603 + miR.520f + miR.220a + miR.519b + miR.29a + miR.376a* + miR.494 + miR.518c | 0,8 | 0,158062 |
| miR.609 + miR.603 + miR.220a + miR.519b + miR.26a + miR.29a + miR.369.3p + miR.376a* + miR.518c | 0,8 | 0,422983257 |
| miR.609 + miR.603 + miR.520f + miR.220a + miR.130a + miR.29a + miR.369.3p + miR.376a* + miR.518c | 0,8 | 0,426997797 |
| miR.609 + miR.603 + miR.220a + miR.26a + miR.29a + miR.362 + miR.369.3p + miR.376a* + miR.518c | 0,8 | 0,323308493 |
| miR.558 + miR.609 + miR.220a + miR.519b + miR.29a + miR.376a* + miR.518c + miR.660 + miR.338 | 0,8 | 0,170761026 |
| miR.558 + miR.609 + miR.520f + miR.519b + miR.26a + miR.29a + miR.376a* + miR.518c + miR.338 | 0,8 | 0,28683872 |
| miR.558 + miR.609 + miR.220a + miR.130a + miR.29a + miR.362 + miR.376a* + miR.518c + miR.660 | 0,8 | 0,077463871 |
| miR.558 + miR.609 + miR.220a + miR.29a + miR.362 + miR.376a* + miR.518c + miR.639 + miR.660 | 0,8 | 0,084212395 |
| miR.558 + miR.609 + miR.220a + miR.29a + miR.362 + miR.376a* + miR.518c + miR.660 + miR.338 | 0,8 | 0,110346752 |
| miR.558 + miR.609 + miR.220a + miR.29a + miR.376a* + miR.518c + miR.639 + miR.660 + miR.338 | 0,8 | 0,123021301 |
| miR.558 + miR.609 + miR.26a + miR.29a + miR.362 + miR.369.3p + miR.376a* + miR.518c + miR.639 | 0,8 | 0,141080519 |
| miR.558 + miR.609 + miR.220a + miR.130a + miR.29a + miR.376a* + miR.494 + miR.518c + miR.338 | 0,8 | 0,020979497 |
| miR.558 + miR.609 + miR.519b + miR.26a + miR.29a + miR.369.3p + miR.376a* + miR.518c + miR.660 | 0,8 | 0,12421106 |
| miR.558 + miR.609 + miR.603 + miR.519b + miR.26a + miR.29a + miR.376a* + miR.518c + miR.639 | 0,8 | 0,103141448 |
| miR.558 + miR.609 + miR.130a + miR.29a + miR.369.3p + miR.376a* + miR.518c + miR.639 + miR.338 | 0,8 | 0,113194072 |
| miR.558 + miR.609 + miR.220a + miR.130a + miR.29a + miR.376a* + miR.518c + miR.660 + miR.338 | 0,8 | 0,100544208 |
| miR.558 + miR.609 + miR.603 + miR.130a + miR.26a + miR.29a + miR.376a* + miR.494 + miR.518c | 0,8 | 0,052344062 |
| miR.609 + miR.603 + miR.220a + miR.29a + miR.362 + miR.369.3p + miR.376a* + miR.494 + miR.518c | 0,8 | 0,350274623 |
| miR.558 + miR.609 + miR.130a + miR.29a + miR.369.3p + miR.376a* + miR.494 + miR.518c + miR.639 | 0,8 | 0,13106931 |
| miR.558 + miR.609 + miR.603 + miR.130a + miR.26a + miR.29a + miR.376a* + miR.518c + miR.639 | 0,8 | 0,049287003 |
| miR.558 + miR.609 + miR.520f + miR.130a + miR.26a + miR.29a + miR.376a* + miR.518c + miR.639 | 0,8 | 0,058279819 |
| miR.558 + miR.609 + miR.520f + miR.519b + miR.26a + miR.29a + miR.376a* + miR.494 + miR.518c | 0,8 | 0,066098363 |
| miR.558 + miR.609 + miR.130a + miR.26a + miR.29a + miR.376a* + miR.494 + miR.518c + miR.639 | 0,8 | 0,065283681 |
| miR.558 + miR.609 + miR.603 + miR.519b + miR.26a + miR.29a + miR.376a* + miR.494 + miR.518c | 0,8 | 0,184657667 |
| miR.558 + miR.609 + miR.520f + miR.519b + miR.130a + miR.29a + miR.376a* + miR.518c + miR.639 | 0,8 | 0,043721789 |
| miR.558 + miR.609 + miR.220a + miR.519b + miR.130a + miR.29a + miR.376a* + miR.518c + miR.639 | 0,8 | 0,079817418 |
| miR.558 + miR.609 + miR.220a + miR.519b + miR.130a + miR.29a + miR.376a* + miR.518c + miR.338 | 0,8 | 0,089405155 |
| miR.558 + miR.609 + miR.520f + miR.130a + miR.29a + miR.362 + miR.376a* + miR.518c + miR.660 | 0,8 | 0,080171246 |
| miR.558 + miR.609 + miR.520f + miR.130a + miR.26a + miR.29a + miR.376a* + miR.518c + miR.660 | 0,8 | 0,068256211 |
| miR.558 + miR.609 + miR.29a + miR.362 + miR.369.3p + miR.376a* + miR.494 + miR.518c + miR.660 | 0,8 | 0,196601967 |
| miR.558 + miR.609 + miR.520f + miR.519b + miR.26a + miR.29a + miR.362 + miR.376a* + miR.518c | 0,8 | 0,088997057 |
| miR.558 + miR.609 + miR.220a + miR.519b + miR.29a + miR.376a* + miR.518c + miR.639+miR.338 | 0,8 | 0,105010951 |
| miR.558 + miR.609 + miR.603 + miR.519b + miR.130a + miR.29a + miR.376a* + miR.494 + miR.518c | 0,8 | 0,097981396 |
| miR.558 + miR.609 + miR.220a + miR.130a + miR.29a + miR.376a* + miR.518c + miR.639+miR.338 | 0,8 | 0,022940199 |
| miR.558 + miR.609 + miR.520f + miR.519b + miR.26a + miR.29a + miR.376a* + miR.518c + miR.660 | 0,8 | 0,084782852 |
| miR.558 + miR.609 + miR.520f + miR.130a + miR.26a + miR.29a + miR.362 + miR.376a* + miR.518c | 0,8 | 0,076049991 |
| miR.558 + miR.609 + miR.520f + miR.519b + miR.130a + miR.29a + miR.362 + miR.376a* + miR.518c | 0,8 | 0,099497198 |
| miR.558 + miR.609 + miR.520f + miR.130a + miR.26a + miR.29a + miR.376a* + miR.494 + miR.518c | 0,8 | 0,060483414 |
| miR.558 + miR.609 + miR.220a + miR.519b + miR.29a + miR.376a* + miR.494 + miR.518c + miR.639 | 0,8 | 0,12618368 |
| miR.609 + miR.603 + miR.220a + miR.519b + miR.130a + miR.29a + miR.369.3p + miR.376a* + miR.518c | 0,8 | 0,487950583 |
| miR.609 + miR.603 + miR.520f + miR.220a + miR.29a + miR.369.3p + miR.376a* + miR.518c + miR.338 | 0,8 | 0,213794953 |
| miR.609 + miR.603 + miR.520f + miR.220a + miR.29a + miR.376a* + miR.494 + miR.518c + miR.660 | 0,8 | 0,138062234 |
| miR.558 + miR.609 + miR.520f + miR.519b + miR.26a + miR.29a + miR.376a* + miR.518c + miR.639 | 0,8 | 0,034901673 |
| miR.609 + miR.603 + miR.520f + miR.220a + miR.29a + miR.369.3p + miR.376a* + miR.518c + miR.639 | 0,8 | 0,478297632 |
| miR.558 + miR.609 + miR.603 + miR.26a + miR.29a + miR.362 + miR.376a* + miR.518c + miR.338 | 0,8 | 0,150637982 |
| miR.558 + miR.609 + miR.519b + miR.29a + miR.362 + miR.369.3p + miR.376a* + miR.518c + miR.660 | 0,8 | 0,212509268 |
| miR.558 + miR.609 + miR.519b + miR.26a + miR.29a + miR.362 + miR.369.3p + miR.376a* + miR.518c | 0,8 | 0,119732414 |
| miR.609 + miR.603 + miR.220a + miR.519b + miR.29a + miR.376a* + miR.494 + miR.518c + miR.660 | 0,8 | 0,26408102 |
| miR.609 + miR.520f + miR.220a + miR.29a + miR.369.3p + miR.376a* + miR.494 + miR.518c + miR.660 | 0,8 | 0,382526012 |
| miR.558 + miR.609 + miR.220a + miR.519b + miR.29a + miR.362 + miR.376a* + miR.494 + miR.518c | 0,8 | 0,267305514 |
| miR.558 + miR.609 + miR.603 + miR.130a + miR.26a + miR.29a + miR.376a* + miR.518c + miR.338 | 0,8 | 0,077106553 |
| miR.558 + miR.609 + miR.603 + miR.26a + miR.29a + miR.362 + miR.376a* + miR.494 + miR.518c | 0,8 | 0,063217109 |
| miR.558 + miR.609 + miR.130a + miR.29a + miR.369.3p + miR.376a* + miR.518c + miR.639 + miR.660 | 0,8 | 0,227075835 |
| miR.558 + miR.609 + miR.603 + miR.26a + miR.29a + miR.376a* + miR.494 + miR.518c + miR.660 | 0,8 | 0,041314211 |
| miR.609 + miR.603 + miR.520f + miR.29a + miR.369.3p + miR.376a* + miR.494 + miR.518c + miR.338 | 0,8 | 0,345531422 |
| miR.558 + miR.609 + miR.130a + miR.26a + miR.29a + miR.369.3p + miR.376a* + miR.518c + miR.660 | 0,8 | 0,114196672 |
| miR.558 + miR.609 + miR.220a + miR.29a + miR.362 + miR.376a* + miR.494 + miR.518c + miR.639 | 0,8 | 0,141067233 |
| miR.609 + miR.603 + miR.220a + miR.29a + miR.369.3p + miR.376a* + miR.494 + miR.518c + miR.639 | 0,8 | 0,315892469 |
| miR.558 + miR.609 + miR.220a + miR.29a + miR.362 + miR.376a* + miR.494 + miR.518c + miR.338 | 0,8 | 0,164020547 |
| miR.558 + miR.609 + miR.603 + miR.130a + miR.29a + miR.376a* + miR.518c + miR.639+miR.338 | 0,8 | 0,006479141 |
| miR.558 + miR.609 + miR.519b + miR.130a + miR.26a + miR.29a + miR.369.3p + miR.376a* + miR.518c | 0,8 | 0,077117054 |
| miR.558 + miR.609 + miR.220a + miR.29a + miR.376a* + miR.494 + miR.518c + miR.639 + miR.338 | 0,8 | 0,035534543 |
| miR.609 + miR.603 + miR.220a + miR.29a + miR.369.3p + miR.376a* + miR.494 + miR.518c + miR.338 | 0,8 | 0,415251922 |
| miR.609 + miR.603 + miR.520f + miR.29a + miR.369.3p + miR.376a* + miR.518c + miR.639 + miR.338 | 0,8 | 0,319667202 |
| miR.558 + miR.609 + miR.130a + miR.29a + miR.362 + miR.369.3p + miR.376a* + miR.518c + miR.660 | 0,8 | 0,264912863 |
| miR.609 + miR.603 + miR.520f + miR.220a + miR.29a + miR.362 + miR.376a* + miR.494 + miR.518c | 0,8 | 0,087304781 |
| miR.609 + miR.603 + miR.220a + miR.519b + miR.26a + miR.29a + miR.376a* + miR.494 + miR.518c | 0,8 | 0,034587309 |
| miR.609 + miR.603 + miR.220a + miR.519b + miR.29a + miR.362 + miR.369.3p + miR.376a* + miR.518c | 0,8 | 0,480285293 |
| miR.609 + miR.603 + miR.220a + miR.519b + miR.29a + miR.369.3p + miR.376a* + miR.518c + miR.338 | 0,8 | 0,433362225 |
| miR.558 + miR.609 + miR.520f + miR.29a + miR.362 + miR.369.3p + miR.376a* + miR.660 + miR.338 | 0,8 | 0,290697116 |
| miR.558 + miR.609 + miR.603 + miR.519b + miR.26a + miR.29a + miR.376a* + miR.518c + miR.338 | 0,8 | 0,139822779 |
| miR.609 + miR.603 + miR.220a + miR.130a + miR.29a + miR.369.3p + miR.376a* + miR.494 + miR.518c | 0,8 | 0,306145794 |
| miR.609 + miR.603 + miR.220a + miR.519b + miR.29a + miR.369.3p + miR.376a* + miR.518c + miR.639 | 0,8 | 0,457628132 |
| miR.558 + miR.609 + miR.130a + miR.26a + miR.29a + miR.362 + miR.369.3p + miR.376a* + miR.518c | 0,8 | 0,103179358 |
| miR.609 + miR.603 + miR.220a + miR.29a + miR.369.3p + miR.376a* + miR.518c + miR.639 + miR.338 | 0,8 | 0,495998135 |
| miR.558 + miR.609 + miR.603 + miR.519b + miR.29a + miR.376a* + miR.494 + miR.518c + miR.639 | 0,8 | 0,034868758 |
| miR.558 + miR.609 + miR.130a + miR.29a + miR.376a* + miR.494 + miR.518c + miR.639 + miR.660 | 0,8 | 0,158089758 |
| miR.558 + miR.609 + miR.130a + miR.29a + miR.369.3p + miR.376a* + miR.494 + miR.518c + miR.660 | 0,8 | 0,114688875 |
| miR.558 + miR.609 + miR.130a + miR.29a + miR.369.3p + miR.376a* + miR.494 + miR.518c + miR.338 | 0,8 | 0,171828811 |
| miR.558 + miR.609 + miR.130a + miR.29a + miR.362 + miR.369.3p + miR.376a* + miR.518c + miR.639 | 0,8 | 0,090864947 |
| miR.609 + miR.603 + miR.220a + miR.519b + miR.29a + miR.362 + miR.376a* + miR.494 + miR.518c | 0,8 | 0,181494289 |
| miR.609 + miR.603 + miR.220a + miR.519b + miR.130a + miR.29a + miR.376a* + miR.494 + miR.518c | 0,8 | 0,051961949 |
| miR.609 + miR.603 + miR.220a + miR.29a + miR.362 + miR.369.3p + miR.376a* + miR.518c + miR.639 | 0,8 | 0,414565334 |
| miR.609 + miR.603 + miR.520f + miR.220a + miR.130a + miR.29a + miR.376a* + miR.518c + miR.660 | 0,8 | 0,127139137 |
| miR.558 + miR.609 + miR.519b + miR.130a + miR.29a + miR.369.3p + miR.376a* + miR.518c + miR.639 | 0,8 | 0,073997354 |
| miR.558 + miR.609 + miR.130a + miR.26a + miR.29a + miR.362 + miR.376a* + miR.518c + miR.639 | 0,8 | 0,106245043 |
| miR.558 + miR.609 + miR.520f + miR.519b + miR.130a + miR.26a + miR.29a + miR.376a* + miR.518c | 0,8 | 0,020564717 |
| miR.609 + miR.603 + miR.220a + miR.29a + miR.362 + miR.369.3p + miR.376a* + miR.518c + miR.338 | 0,8 | 0,457160688 |
| miR.558 + miR.609 + miR.603 + miR.130a + miR.26a + miR.29a + miR.362 + miR.376a* + miR.518c | 0,8 | 0,028720054 |
| miR.558 + miR.609 + miR.220a + miR.519b + miR.29a + miR.376a* + miR.494 + miR.518c + miR.338 | 0,8 | 0,135275975 |
| miR.558 + miR.609 + miR.603 + miR.130a + miR.29a + miR.362 + miR.376a* + miR.518c + miR.639 | 0,8 | 0,035358736 |
| miR.558 + miR.609 + miR.130a + miR.26a + miR.29a + miR.376a* + miR.518c + miR.639 + miR.660 | 0,8 | 0,117954732 |
| miR.558 + miR.609 + miR.130a + miR.29a + miR.362 + miR.376a* + miR.494 + miR.518c + miR.639 | 0,8 | 0,095810638 |
| miR.558 + miR.609 + miR.130a + miR.29a + miR.362 + miR.369.3p + miR.376a* + miR.494 + miR.518c | 0,8 | 0,129757872 |
| miR.558 + miR.609 + miR.603 + miR.519b + miR.26a + miR.29a + miR.362 + miR.376a* + miR.518c | 0,8 | 0,094782244 |
| miR.558 + miR.609 + miR.603 + miR.130a + miR.29a + miR.376a* + miR.518c + miR.639 + miR.660 | 0,8 | 0,066510885 |
| miR.558 + miR.609 + miR.519b + miR.130a + miR.29a + miR.369.3p + miR.376a* + miR.494 + miR.518c | 0,8 | 0,068273345 |
| miR.558 + miR.609 + miR.603 + miR.26a + miR.29a + miR.376a* + miR.518c + miR.660 + miR.338 | 0,8 | 0,101713117 |
| miR.558 + miR.609 + miR.603 + miR.519b + miR.130a + miR.26a + miR.29a + miR.376a* + miR.518c | 0,8 | 0,035453664 |
| miR.609 + miR.603 + miR.220a + miR.130a + miR.29a + miR.362 + miR.369.3p + miR.376a* + miR.518c | 0,8 | 0,37009407 |
| miR.558 + miR.609 + miR.603 + miR.130a + miR.26a + miR.29a + miR.376a* + miR.518c + miR.660 | 0,8 | 0,021782704 |
| miR.558 + miR.609 + miR.603 + miR.519b + miR.29a + miR.376a* + miR.518c + miR.639 + miR.660 | 0,8 | 0,039816049 |
| miR.558 + miR.609 + miR.603 + miR.130a + miR.29a + miR.362 + miR.376a* + miR.494 + miR.518c | 0,8 | 0,011144508 |
| miR.609 + miR.603 + miR.220a + miR.130a + miR.29a + miR.369.3p + miR.376a* + miR.518c + miR.338 | 0,8 | 0,44092978 |
| miR.609 + miR.603 + miR.520f + miR.29a + miR.369.3p + miR.376a* + miR.494 + miR.518c + miR.660 | 0,8 | 0,137581827 |
| miR.558 + miR.609 + miR.603 + miR.130a + miR.29a + miR.376a* + miR.494 + miR.518c + miR.338 | 0,8 | 0,009263095 |
| miR.558 + miR.609 + miR.603 + miR.130a + miR.29a + miR.376a* + miR.494 + miR.518c + miR.660 | 0,8 | 0,010895069 |
| miR.558 + miR.609 + miR.603 + miR.29a + miR.376a* + miR.494 + miR.518c + miR.639+miR.338 | 0,8 | 0,017277545 |
| miR.558 + miR.609 + miR.603 + miR.29a + miR.362 + miR.376a* + miR.518c + miR.639 + miR.660 | 0,8 | 0,032467834 |
| miR.609 + miR.520f + miR.220a + miR.29a + miR.362 + miR.369.3p + miR.376a* + miR.518c + miR.660 | 0,8 | 0,392375462 |
| miR.558 + miR.609 + miR.603 + miR.29a + miR.376a* + miR.494 + miR.518c + miR.639 + miR.660 | 0,8 | 0,038240115 |
| miR.558 + miR.609 + miR.130a + miR.29a + miR.376a* + miR.494 + miR.518c + miR.639 + miR.338 | 0,8 | 0,09606346 |
| miR.558 + miR.609 + miR.603 + miR.26a + miR.29a + miR.362 + miR.376a* + miR.518c + miR.660 | 0,8 | 0,022539566 |
| miR.609 + miR.603 + miR.220a + miR.130a + miR.29a + miR.369.3p + miR.376a* + miR.518c + miR.639 | 0,8 | 0,399140134 |
| miR.609 + miR.603 + miR.520f + miR.220a + miR.29a + miR.362 + miR.376a* + miR.518c + miR.639 | 0,8 | 0,144676791 |
| miR.609 + miR.603 + miR.520f + miR.29a + miR.369.3p + miR.376a* + miR.518c + miR.660 + miR.338 | 0,8 | 0,393283621 |
| miR.558 + miR.609 + miR.519b + miR.26a + miR.29a + miR.376a* + miR.518c + miR.639+miR.338 | 0,8 | 0,108718229 |
| miR.558 + miR.609 + miR.603 + miR.519b + miR.26a + miR.29a + miR.376a* + miR.518c + miR.660 | 0,8 | 0,078605663 |
| miR.558 + miR.609 + miR.26a + miR.29a + miR.376a* + miR.518c + miR.639 + miR.660 + miR.338 | 0,8 | 0,20368957 |
| miR.558 + miR.609 + miR.26a + miR.29a + miR.362 + miR.376a* + miR.518c + miR.639+miR.338 | 0,8 | 0,213832161 |
| miR.558 + miR.609 + miR.29a + miR.369.3p + miR.376a* + miR.518c + miR.639 + miR.660 + miR.338 | 0,8 | 0,244691095 |
| miR.609 + miR.520f + miR.220a + miR.130a + miR.29a + miR.369.3p + miR.376a* + miR.518c + miR.660 | 0,8 | 0,360236492 |
| miR.558 + miR.609 + miR.603 + miR.520f + miR.130a + miR.376a* + miR.518c + miR.639 + miR.338 | 0,8 | 0,142353629 |
| miR.558 + miR.609 + miR.519b + miR.130a + miR.29a + miR.376a* + miR.494 + miR.518c + miR.639 | 0,8 | 0,052844987 |
| miR.558 + miR.609 + miR.603 + miR.519b + miR.130a + miR.29a + miR.376a* + miR.518c + miR.639 | 0,8 | 0,029623938 |
| miR.558 + miR.609 + miR.603 + miR.29a + miR.362 + miR.376a* + miR.494 + miR.518c + miR.639 | 0,8 | 0,017294487 |
| miR.558 + miR.609 + miR.603 + miR.29a + miR.362 + miR.376a* + miR.518c + miR.639 + miR.338 | 0,8 | 0,024834852 |
| miR.558 + miR.609 + miR.29a + miR.369.3p + miR.376a* + miR.494 + miR.518c + miR.639 + miR.660 | 0,8 | 0,155807164 |
| miR.609 + miR.603 + miR.520f + miR.220a + miR.519b + miR.29a + miR.362 + miR.376a* + miR.518c | 0,8 | 0,238139694 |
| miR.558 + miR.609 + miR.603 + miR.520f + miR.369.3p + miR.376a* + miR.518c + miR.639 + miR.338 | 0,8 | 0,207790313 |
| miR.609 + miR.603 + miR.520f + miR.29a + miR.369.3p + miR.376a* + miR.494 + miR.518c + miR.639 | 0,8 | 0,116866676 |
| miR.558 + miR.609 + miR.519b + miR.29a + miR.369.3p + miR.376a* + miR.518c + miR.639 + miR.338 | 0,8 | 0,140009465 |
| miR.558 + miR.609 + miR.130a + miR.29a + miR.369.3p + miR.376a* + miR.518c + miR.660 + miR.338 | 0,8 | 0,133472241 |
| miR.558 + miR.609 + miR.130a + miR.26a + miR.29a + miR.376a* + miR.518c + miR.639 + miR.338 | 0,8 | 0,115608628 |
| miR.558 + miR.609 + miR.29a + miR.369.3p + miR.376a* + miR.494 + miR.518c + miR.639 + miR.338 | 0,8 | 0,126671162 |
| miR.558 + miR.609 + miR.603 + miR.29a + miR.376a* + miR.518c + miR.639 + miR.660 + miR.338 | 0,8 | 0,057914406 |
| miR.558 + miR.609 + miR.130a + miR.29a + miR.362 + miR.369.3p + miR.376a* + miR.518c + miR.338 | 0,8 | 0,132734107 |
| miR.609 + miR.603 + miR.520f + miR.29a + miR.369.3p + miR.376a* + miR.518c + miR.639 + miR.660 | 0,8 | 0,166407828 |
| miR.558 + miR.609 + miR.519b + miR.26a + miR.29a + miR.362 + miR.376a* + miR.518c + miR.639 | 0,8 | 0,066227121 |
| miR.558 + miR.609 + miR.519b + miR.29a + miR.369.3p + miR.376a* + miR.518c + miR.639 + miR.660 | 0,8 | 0,171844452 |
| miR.609 + miR.603 + miR.520f + miR.220a + miR.29a + miR.376a* + miR.518c + miR.639 + miR.660 | 0,8 | 0,091869834 |
| miR.558 + miR.609 + miR.603 + miR.519b + miR.29a + miR.376a* + miR.494 + miR.518c + miR.338 | 0,8 | 0,086101572 |
| miR.609 + miR.220a + miR.29a + miR.362 + miR.369.3p + miR.376a* + miR.494 + miR.518c + miR.660 | 0,8 | 0,349722804 |
| miR.609 + miR.603 + miR.520f + miR.130a + miR.29a + miR.369.3p + miR.376a* + miR.518c + miR.338 | 0,8 | 0,432397172 |
| miR.609 + miR.603 + miR.520f + miR.220a + miR.130a + miR.29a + miR.362 + miR.376a* + miR.518c | 0,8 | 0,128742377 |
| miR.558 + miR.609 + miR.603 + miR.519b + miR.29a + miR.376a* + miR.518c + miR.639 + miR.338 | 0,8 | 0,022446774 |
| miR.558 + miR.609 + miR.29a + miR.362 + miR.369.3p + miR.376a* + miR.518c + miR.639 + miR.338 | 0,8 | 0,149018493 |
| miR.609 + miR.603 + miR.220a + miR.26a + miR.29a + miR.376a* + miR.494 + miR.518c + miR.660 | 0,8 | 0,104536552 |
| miR.609 + miR.603 + miR.520f + miR.519b + miR.29a + miR.369.3p + miR.376a* + miR.494 + miR.518c | 0,8 | 0,110908764 |
| miR.558 + miR.609 + miR.519b + miR.130a + miR.26a + miR.29a + miR.376a* + miR.494 + miR.518c | 0,8 | 0,064289596 |
| miR.558 + miR.609 + miR.603 + miR.519b + miR.29a + miR.362 + miR.376a* + miR.518c + miR.639 | 0,8 | 0,04786143 |
| miR.609 + miR.603 + miR.520f + miR.220a + miR.29a + miR.376a* + miR.494 + miR.518c + miR.639 | 0,8 | 0,026086832 |
| miR.558 + miR.609 + miR.603 + miR.520f + miR.130a + miR.369.3p + miR.376a* + miR.518c + miR.338 | 0,8 | 0,202312429 |
| miR.609 + miR.520f + miR.220a + miR.519b + miR.29a + miR.369.3p + miR.376a* + miR.518c + miR.660 | 0,8 | 0,432735808 |
| miR.558 + miR.609 + miR.603 + miR.520f + miR.130a + miR.369.3p + miR.376a* + miR.518c + miR.639 | 0,8 | 0,15793484 |
| miR.558 + miR.609 + miR.519b + miR.26a + miR.29a + miR.376a* + miR.518c + miR.639 + miR.660 | 0,8 | 0,08087559 |
| miR.558 + miR.609 + miR.29a + miR.362 + miR.369.3p + miR.376a* + miR.494 + miR.518c + miR.639 | 0,8 | 0,092391206 |
| miR.609 + miR.603 + miR.520f + miR.26a + miR.29a + miR.369.3p + miR.376a* + miR.518c + miR.338 | 0,8 | 0,479385389 |
| miR.558 + miR.609 + miR.26a + miR.29a + miR.376a* + miR.494 + miR.518c + miR.639 + miR.660 | 0,8 | 0,063910988 |
| miR.558 + miR.609 + miR.519b + miR.130a + miR.26a + miR.29a + miR.376a* + miR.518c + miR.639 | 0,8 | 0,03309098 |
| miR.558 + miR.609 + miR.519b + miR.29a + miR.369.3p + miR.376a* + miR.494 + miR.518c + miR.639 | 0,8 | 0,082814397 |
| miR.609 + miR.603 + miR.520f + miR.220a + miR.29a + miR.376a* + miR.494 + miR.518c + miR.338 | 0,8 | 0,085588816 |
| miR.558 + miR.609 + miR.603 + miR.29a + miR.362 + miR.376a* + miR.494 + miR.518c + miR.660 | 0,8 | 0,018293817 |
| miR.609 + miR.603 + miR.520f + miR.220a + miR.130a + miR.29a + miR.376a* + miR.494 + miR.518c | 0,8 | 0,042337528 |
| miR.558 + miR.609 + miR.130a + miR.26a + miR.29a + miR.376a* + miR.494 + miR.518c + miR.660 | 0,8 | 0,058870396 |
| miR.609 + miR.603 + miR.520f + miR.130a + miR.29a + miR.369.3p + miR.376a* + miR.494 + miR.518c | 0,8 | 0,13154698 |
| miR.609 + miR.603 + miR.520f + miR.519b + miR.29a + miR.369.3p + miR.376a* + miR.518c + miR.338 | 0,8 | 0,430628151 |
| miR.609 + miR.603 + miR.220a + miR.26a + miR.29a + miR.362 + miR.376a* + miR.494 + miR.518c | 0,8 | 0,049252717 |
| miR.558 + miR.609 + miR.519b + miR.130a + miR.29a + miR.369.3p + miR.376a* + miR.518c + miR.338 | 0,8 | 0,120103833 |
| miR.609 + miR.603 + miR.520f + miR.29a + miR.376a* + miR.494 + miR.518c + miR.639 + miR.660 | 0,8 | 0,067806989 |
| miR.609 + miR.603 + miR.520f + miR.220a + miR.519b + miR.29a + miR.376a* + miR.518c + miR.660 | 0,8 | 0,221127196 |
| miR.609 + miR.603 + miR.520f + miR.220a + miR.29a + miR.376a* + miR.518c + miR.660 + miR.338 | 0,8 | 0,302752216 |
| miR.609 + miR.603 + miR.520f + miR.26a + miR.29a + miR.369.3p + miR.376a* + miR.494 + miR.518c | 0,8 | 0,132855449 |
| miR.609 + miR.520f + miR.220a + miR.29a + miR.369.3p + miR.376a* + miR.518c + miR.639 + miR.660 | 0,8 | 0,450491072 |
| miR.609 + miR.603 + miR.520f + miR.130a + miR.29a + miR.369.3p + miR.376a* + miR.518c + miR.639 | 0,8 | 0,084150772 |
| miR.558 + miR.609 + miR.29a + miR.369.3p + miR.376a* + miR.494 + miR.518c + miR.660 + miR.338 | 0,8 | 0,165261001 |
| miR.558 + miR.609 + miR.603 + miR.519b + miR.29a + miR.376a* + miR.518c + miR.660 + miR.338 | 0,8 | 0,030694635 |

**Table 10: clusters of 10 miRNAs**

| **Cluster of 10 miRs** | **c_tau level** | **pv compared to top signature miR.558 + miR.609 + miR.603 + miR.220a + miR.26a + miR.29a + miR.369.3p + miR.376a* + miR.518c + miR.660** |
|---|---|---|
| miR.558 + miR.609 + miR.603 + miR.220a + miR.26a + miR.29a + miR.369.3p + miR.376a* + miR.518c + miR.660 | 0,81 | NaN |
| miR.558 + miR.609 + miR.603 + miR.220a + miR.29a + miR.362 + miR.369.3p + miR.376a* + miR.518c + miR.660 | 0,81 | 0,242090457 |
| miR.558 + miR.609 + miR.603 + miR.520f + miR.220a + miR.29a + miR.369.3p + miR.376a* + miR.518c + miR.660 | 0,81 | 0,305884533 |
| miR.558 + miR.609 + miR.603 + miR.220a + miR.26a + miR.29a + miR.369.3p + miR.376a* + miR.518c + miR.338 | 0,81 | 0,496600392 |
| miR.558 + miR.609 + miR.603 + miR.220a + miR.26a + miR.29a + miR.362 + miR.369.3p + miR.376a* + miR.518c | 0,81 | 0,176882281 |
| miR.558 + miR.609 + miR.603 + miR.220a + miR.26a + miR.29a + miR.369.3p + miR.376a* + miR.518c + miR.639 | 0,81 | 0,209905826 |
| miR.558 + miR.609 + miR.603 + miR.220a + miR.26a + miR.29a + miR.369.3p + miR.376a* + miR.494 + miR.518c | 0,81 | 0,177508558 |
| miR.558 + miR.609 + miR.603 + miR.220a + miR.29a + miR.369.3p + miR.376a* + miR.518c + miR.639 + miR.660 | 0,81 | 0,13465496 |
| miR.558 + miR.609 + miR.603 + miR.220a + miR.519b + miR.29a + miR.369.3p + miR.376a* + miR.518c + miR.660 | 0,81 | 0,297307498 |
| miR.558 + miR.609 + miR.603 + miR.220a + miR.130a + miR.26a + miR.29a + miR.369.3p + miR.376a* + miR.518c | 0,81 | 0,223947224 |
| miR.558 + miR.609 + miR.603 + miR.520f + miR.220a + miR.26a + miR.29a + miR.369.3p + miR.376a* + miR.518c | 0,81 | 0,21718402 |
| miR.558 + miR.609 + miR.603 + miR.220a + miR.29a + miR.369.3p + miR.376a* + miR.494 + miR.518c + miR.660 | 0,81 | 0,157448882 |
| miR.558 + miR.609 + miR.603 + miR.220a + miR.29a + miR.369.3p + miR.376a* + miR.518c + miR.660 + miR.338 | 0,81 | 0,269392482 |
| miR.558 + miR.609 + miR.520f + miR.220a + miR.29a + miR.369.3p + miR.376a* + miR.518c + miR.660 + miR.338 | 0,81 | 0,382825458 |
| miR.558 + miR.609 + miR.603 + miR.220a + miR.130a + miR.29a + miR.369.3p + miR.376a* + miR.518c + miR.660 | 0,81 | 0,141825603 |
| miR.558 + miR.609 + miR.603 + miR.520f + miR.220a + miR.29a + miR.362 + miR.369.3p + miR.376a* + miR.518c | 0,81 | 0,070542282 |
| miR.558 + miR.609 + miR.520f + miR.29a + miR.362 + miR.369.3p + miR.376a* + miR.518c + miR.660 + miR.338 | 0,81 | 0,292740124 |
| miR.558 + miR.609 + miR.603 + miR.220a + miR.519b + miR.26a + miR.29a + miR.369.3p + miR.376a* + miR.518c | 0,81 | 0,384087979 |
| miR.558 + miR.609 + miR.603 + miR.220a + miR.519b + miR.29a + miR.362 + miR.369.3p + miR.376a* + miR.518c | 0,81 | 0,308180061 |
| miR.558 + miR.609 + miR.520f + miR.220a + miR.29a + miR.369.3p + miR.376a* + miR.518c + miR.639 + miR.660 | 0,81 | 0,37505175 |
| miR.558 + miR.609 + miR.603 + miR.220a + miR.519b + miR.130a + miR.29a + miR.369.3p + miR.376a* + miR.518c | 0,81 | 0,277532588 |
| miR.558 + miR.609 + miR.603 + miR.520f + miR.29a + miR.369.3p + miR.376a* + miR.518c + miR.639 + miR.338 | 0,81 | 0,146358228 |
| miR.558 + miR.609 + miR.520f + miR.220a + miR.29a + miR.369.3p + miR.376a* + miR.494 + miR.518c + miR.660 | 0,81 | 0,472431482 |
| miR.558 + miR.609 + miR.520f + miR.220a + miR.26a + miR.29a + miR.369.3p + miR.376a* + miR.518c + miR.660 | 0,81 | 0,295395022 |
| miR.558 + miR.609 + miR.603 + miR.520f + miR.220a + miR.519b + miR.29a + miR.369.3p + miR.376a* + miR.518c | 0,81 | 0,256455357 |
| miR.558 + miR.609 + miR.220a + miR.26a + miR.29a + miR.369.3p + miR.376a* + miR.494 + miR.518c + miR.660 | 0,81 | 0,307836339 |
| miR.558 + miR.609 + miR.520f + miR.29a + miR.369.3p + miR.376a* + miR.518c + miR.639 + miR.660 + miR.338 | 0,81 | 0,458941312 |
| miR.558 + miR.609 + miR.220a + miR.26a + miR.29a + miR.369.3p + miR.376a* + miR.518c + miR.660 + miR.338 | 0,81 | 0,08413771 |
| miR.558 + miR.609 + miR.603 + miR.220a + miR.519b + miR.29a + miR.369.3p + miR.376a* + miR.494 + miR.518c | 0,81 | 0,425031749 |
| miR.558 + miR.609 + miR.603 + miR.520f + miR.220a + miR.130a + miR.29a + miR.369.3p + miR.376a* + miR.518c | 0,81 | 0,073664811 |
| miR.558 + miR.609 + miR.520f + miR.220a + miR.29a + miR.362 + miR.369.3p + miR.376a* + miR.518c + miR.660 | 0,81 | 0,327162696 |
| miR.558 + miR.609 + miR.603 + miR.520f + miR.29a + miR.369.3p + miR.376a* + miR.518c + miR.660 + miR.338 | 0,81 | 0,415701525 |
| miR.558 + miR.609 + miR.220a + miR.26a + miR.29a + miR.369.3p + miR.376a* + miR.518c + miR.639 + miR.660 | 0,81 | 0,351475224 |
| miR.558 + miR.609 + miR.603 + miR.220a + miR.519b + miR.130a + miR.29a + miR.376a* + miR.494 + miR.518c | 0,81 | 0,072059664 |
| miR.558 + miR.609 + miR.520f + miR.220a + miR.130a + miR.29a + miR.369.3p + miR.376a* + miR.518c + miR.660 | 0,81 | 0,440507773 |
| miR.558 + miR.609 + miR.603 + miR.520f + miR.220a + miR.29a + miR.369.3p + miR.376a* + miR.518c + miR.338 | 0,81 | 0,269521529 |
| miR.558 + miR.609 + miR.520f + miR.220a + miR.519b + miR.29a + miR.369.3p + miR.376a* + miR.518c + miR.660 | 0,81 | 0,298687934 |
| miR.558 + miR.609 + miR.603 + miR.220a + miR.26a + miR.29a + miR.362 + miR.376a* + miR.494 + miR.518c | 0,81 | 0,004441999 |
| miR.558 + miR.609 + miR.220a + miR.26a + miR.29a + miR.362 + miR.369.3p + miR.376a* + miR.518c + miR.660 | 0,81 | 0,141098979 |
| miR.558 + miR.609 + miR.603 + miR.520f + miR.29a + miR.369.3p + miR.376a* + miR.518c + miR.639 + miR.660 | 0,81 | 0,063444211 |
| miR.558 + miR.609 + miR.603 + miR.220a + miR.130a + miR.29a + miR.369.3p + miR.376a* + miR.494 + miR.518c | 0,81 | 0,054972939 |
| miR.558 + miR.609 + miR.603 + miR.220a + miR.519b + miR.26a + miR.29a + miR.362 + miR.376a* + miR.518c | 0,81 | 0,064183216 |
| miR.558 + miR.609 + miR.603 + miR.220a + miR.519b + miR.29a + miR.369.3p + miR.376a* + miR.518c + miR.639 | 0,81 | 0,292553142 |
| miR.558 + miR.609 + miR.603 + miR.26a + miR.29a + miR.369.3p + miR.376a* + miR.518c + miR.639 + miR.338 | 0,81 | 0,378239996 |
| miR.558 + miR.609 + miR.603 + miR.520f + miR.220a + miR.519b + miR.29a + miR.376a* + miR.494 + miR.518c | 0,81 | 0,037056936 |
| miR.558 + miR.609 + miR.603 + miR.220a + miR.130a + miR.29a + miR.362 + miR.369.3p + miR.376a* + miR.518c | 0,81 | 0,050284986 |
| miR.558 + miR.609 + miR.220a + miR.29a + miR.362 + miR.369.3p + miR.376a* + miR.518c + miR.660 + miR.338 | 0,81 | 0,476759421 |
| miR.558 + miR.609 + miR.603 + miR.220a + miR.130a + miR.29a + miR.369.3p + miR.376a* + miR.518c + miR.639 | 0,81 | 0,017873439 |
| miR.558 + miR.609 + miR.603 + miR.520f + miR.29a + miR.369.3p + miR.376a* + miR.494 + miR.518c + miR.338 | 0,81 | 0,23623766 |
| miR.558 + miR.609 + miR.603 + miR.520f + miR.220a + miR.519b + miR.29a + miR.362 + miR.376a* + miR.518c | 0,81 | 0,114412749 |
| miR.558 + miR.609 + miR.603 + miR.520f + miR.26a + miR.29a + miR.369.3p + miR.376a* + miR.518c + miR.338 | 0,81 | 0,315070621 |
| miR.558 + miR.609 + miR.220a + miR.519b + miR.26a + miR.29a + miR.369.3p + miR.376a* + miR.518c + miR.660 | 0,81 | 0,228951442 |
| miR.558 + miR.609 + miR.520f + miR.220a + miR.26a + miR.29a + miR.369.3p + miR.376a* + miR.518c + miR.338 | 0,81 | 0,426409297 |
| miR.558 + miR.609 + miR.603 + miR.220a + miR.519b + miR.26a + miR.29a + miR.376a* + miR.494 + miR.518c | 0,81 | 0,04004517 |
| miR.558 + miR.609 + miR.603 + miR.220a + miR.26a + miR.29a + miR.376a* + miR.494 + miR.518c + miR.660 | 0,81 | 0,001870538 |
| miR.558 + miR.609 + miR.603 + miR.220a + miR.26a + miR.29a + miR.362 + miR.376a* + miR.518c + miR.639 | 0,81 | 0,006986126 |
| miR.558 + miR.609 + miR.603 + miR.520f + miR.130a + miR.29a + miR.369.3p + miR.376a* + miR.518c + miR.338 | 0,81 | 0,304421744 |
| miR.558 + miR.609 + miR.220a + miR.130a + miR.26a + miR.29a + miR.369.3p + miR.376a* + miR.518c + miR.660 | 0,81 | 0,216822953 |
| miR.558 + miR.609 + miR.603 + miR.220a + miR.519b + miR.29a + miR.362 + miR.376a* + miR.494 + miR.518c | 0,81 | 0,108357368 |
| miR.558 + miR.609 + miR.603 + miR.520f + miR.220a + miR.29a + miR.369.3p + miR.376a* + miR.518c + miR.639 | 0,81 | 0,03215356 |
| miR.558 + miR.609 + miR.603 + miR.520f + miR.519b + miR.29a + miR.369.3p + miR.376a* + miR.518c + miR.338 | 0,81 | 0,391842764 |
| miR.558 + miR.609 + miR.603 + miR.220a + miR.130a + miR.26a + miR.29a + miR.362 + miR.376a* + miR.518c | 0,81 | 0,003073458 |
| miR.558 + miR.609 + miR.520f + miR.220a + miR.29a + miR.362 + miR.369.3p + miR.376a* + miR.518c + miR.338 | 0,81 | 0,487671446 |
| miR.558 + miR.609 + miR.603 + miR.520f + miR.220a + miR.26a + miR.29a + miR.362 + miR.376a* + miR.518c | 0,81 | 0,005624385 |
| miR.558 + miR.609 + miR.220a + miR.130a + miR.29a + miR.362 + miR.369.3p + miR.376a* + miR.518c + miR.660 | 0,81 | 0,489205609 |
| miR.558 + miR.609 + miR.603 + miR.220a + miR.26a + miR.29a + miR.362 + miR.376a* + miR.518c + miR.338 | 0,81 | 0,032725563 |
| miR.558 + miR.609 + miR.603 + miR.520f + miR.220a + miR.29a + miR.369.3p + miR.376a* + miR.494 + miR.518c | 0,81 | 0,051081681 |
| miR.558 + miR.609 + miR.603 + miR.520f + miR.220a + miR.519b + miR.29a + miR.376a* + miR.518c + miR.660 | 0,81 | 0,063867955 |
| miR.558 + miR.609 + miR.520f + miR.29a + miR.369.3p + miR.376a* + miR.494 + miR.518c + miR.660 + miR.338 | 0,81 | 0,352231088 |
| miR.558 + miR.609 + miR.603 + miR.220a + miR.519b + miR.130a + miR.29a + miR.362 + miR.376a* + miR.518c | 0,81 | 0,091683373 |
| miR.558 + miR.609 + miR.603 + miR.520f + miR.220a + miR.29a + miR.376a* + miR.494 + miR.518c + miR.660 | 0,81 | 0,019374327 |
| miR.558 + miR.609 + miR.603 + miR.220a + miR.26a + miR.29a + miR.376a* + miR.494 + miR.518c + miR.639 | 0,81 | 0,000472352 |
| miR.558 + miR.609 + miR.220a + miR.130a + miR.29a + miR.369.3p + miR.376a* + miR.518c + miR.639 + miR.660 | 0,81 | 0,337869581 |
| miR.558 + miR.609 + miR.603 + miR.220a + miR.26a + miR.29a + miR.376a* + miR.494 + miR.518c + miR.338 | 0,81 | 0,007843322 |
| miR.558 + miR.609 + miR.603 + miR.220a + miR.519b + miR.26a + miR.29a + miR.376a* + miR.518c + miR.660 | 0,81 | 0,066267481 |
| miR.558 + miR.609 + miR.220a + miR.26a + miR.29a + miR.369.3p + miR.376a* + miR.494 + miR.518c + miR.338 | 0,81 | 0,333854732 |
| miR.558 + miR.609 + miR.603 + miR.220a + miR.519b + miR.29a + miR.362 + miR.376a* + miR.518c + miR.338 | 0,81 | 0,156363682 |
| miR.558 + miR.609 + miR.603 + miR.220a + miR.26a + miR.29a + miR.376a* + miR.518c + miR.639 + miR.660 | 0,81 | 0,001727364 |
| miR.558 + miR.609 + miR.603 + miR.220a + miR.519b + miR.26a + miR.29a + miR.376a* + miR.518c + miR.338 | 0,81 | 0,01944327 |
| miR.558 + miR.609 + miR.603 + miR.520f + miR.220a + miR.26a + miR.29a + miR.376a* + miR.518c + miR.660 | 0,81 | 0,013873658 |
| miR.558 + miR.609 + miR.603 + miR.220a + miR.519b + miR.29a + miR.376a* + miR.494 + miR.518c + miR.660 | 0,81 | 0,099460898 |
| miR.558 + miR.609 + miR.603 + miR.220a + miR.519b + miR.29a + miR.362 + miR.376a* + miR.518c + miR.639 | 0,81 | 0,131557674 |
| miR.558 + miR.609 + miR.603 + miR.520f + miR.29a + miR.362 + miR.369.3p + miR.376a* + miR.518c + miR.338 | 0,81 | 0,304698544 |
| miR.558 + miR.609 + miR.220a + miR.29a + miR.362 + miR.369.3p + miR.376a* + miR.494 + miR.518c + miR.660 | 0,81 | 0,464408364 |
| miR.558 + miR.609 + miR.520f + miR.220a + miR.29a + miR.369.3p + miR.376a* + miR.494 + miR.518c + miR.338 | 0,81 | 0,448232041 |
| miR.558 + miR.609 + miR.520f + miR.29a + miR.376a* + miR.494 + miR.518c + miR.639 + miR.660 + miR.338 | 0,81 | 0,230587013 |
| miR.558 + miR.609 + miR.603 + miR.520f + miR.220a + miR.29a + miR.376a* + miR.518c + miR.660 + miR.338 | 0,81 | 0,193908206 |
| miR.558 + miR.609 + miR.220a + miR.29a + miR.362 + miR.369.3p + miR.376a* + miR.518c + miR.639 + miR.660 | 0,81 | 0,475902061 |
| miR.558 + miR.609 + miR.603 + miR.520f + miR.220a + miR.29a + miR.362 + miR.376a* + miR.518c + miR.639 | 0,81 | 0,008531382 |
| miR.558 + miR.609 + miR.520f + miR.220a + miR.29a + miR.369.3p + miR.376a* + miR.518c + miR.639 + miR.338 | 0,81 | 0,276900513 |
| miR.558 + miR.609 + miR.603 + miR.220a + miR.26a + miR.29a + miR.362 + miR.376a* + miR.518c + miR.660 | 0,81 | 0,002224319 |
| miR.558 + miR.609 + miR.603 + miR.220a + miR.26a + miR.29a + miR.376a* + miR.518c + miR.639 + miR.338 | 0,81 | 0,002954162 |
| miR.558 + miR.609 + miR.603 + miR.220a + miR.130a + miR.26a + miR.29a + miR.376a* + miR.518c + miR.338 | 0,81 | 0,006050105 |
| miR.558 + miR.609 + miR.220a + miR.26a + miR.29a + miR.369.3p + miR.376a* + miR.518c + miR.639 + miR.338 | 0,81 | 0,458263005 |
| miR.558 + miR.609 + miR.603 + miR.520f + miR.220a + miR.29a + miR.362 + miR.376a* + miR.518c + miR.338 | 0,81 | 0,099135563 |
| miR.558 + miR.609 + miR.603 + miR.220a + miR.519b + miR.29a + miR.369.3p + miR.376a* + miR.518c + miR.338 | 0,81 | 0,364096223 |
| miR.558 + miR.609 + miR.520f + miR.220a + miR.29a + miR.376a* + miR.518c + miR.639 + miR.660 + miR.338 | 0,81 | 0,359569056 |
| miR.558 + miR.609 + miR.603 + miR.520f + miR.220a + miR.29a + miR.362 + miR.376a* + miR.494 + miR.518c | 0,81 | 0,00430911 |
| miR.558 + miR.609 + miR.603 + miR.220a + miR.130a + miR.26a + miR.29a + miR.376a* + miR.518c + miR.660 | 0,81 | 0,00188 |
| miR.558 + miR.609 + miR.603 + miR.520f + miR.26a + miR.29a + miR.369.3p + miR.376a* + miR.518c + miR.639 | 0,81 | 0,073848885 |
| miR.558 + miR.609 + miR.603 + miR.520f + miR.220a + miR.29a + miR.376a* + miR.518c + miR.639 + miR.660 | 0,81 | 0,051720828 |
| miR.558 + miR.609 + miR.603 + miR.220a + miR.29a + miR.362 + miR.369.3p + miR.376a* + miR.518c + miR.639 | 0,81 | 0,016477914 |
| miR.558 + miR.609 + miR.220a + miR.519b + miR.130a + miR.29a + miR.369.3p + miR.376a* + miR.518c + miR.660 | 0,81 | 0,286223061 |
| miR.558 + miR.609 + miR.520f + miR.220a + miR.26a + miR.29a + miR.369.3p + miR.376a* + miR.494 + miR.518c | 0,81 | 0,419018364 |
| miR.558 + miR.609 + miR.520f + miR.220a + miR.130a + miR.29a + miR.369.3p + miR.376a* + miR.518c + miR.338 | 0,81 | 0,393139492 |
| miR.558 + miR.609 + miR.220a + miR.26a + miR.29a + miR.362 + miR.369.3p + miR.376a* + miR.518c + miR.338 | 0,81 | 0,355492006 |
| miR.558 + miR.609 + miR.603 + miR.220a + miR.29a + miR.362 + miR.369.3p + miR.376a* + miR.494 + miR.518c | 0,81 | 0,04568601 |
| miR.558 + miR.609 + miR.520f + miR.220a + miR.26a + miR.29a + miR.362 + miR.369.3p + miR.376a* + miR.518c | 0,81 | 0,32893203 |
| miR.558 + miR.609 + miR.603 + miR.220a + miR.26a + miR.29a + miR.376a* + miR.518c + miR.660 + miR.338 | 0,81 | 0,038842008 |
| miR.558 + miR.609 + miR.603 + miR.220a + miR.519b + miR.29a + miR.362 + miR.376a* + miR.518c + miR.660 | 0,81 | 0,149408518 |
| miR.558 + miR.609 + miR.603 + miR.220a + miR.519b + miR.130a + miR.26a + miR.29a + miR.376a* + miR.518c | 0,81 | 0,010456894 |
| miR.558 + miR.609 + miR.603 + miR.220a + miR.29a + miR.362 + miR.369.3p + miR.376a* + miR.518c + miR.338 | 0,81 | 0,095445321 |
| miR.558 + miR.609 + miR.603 + miR.220a + miR.29a + miR.369.3p + miR.376a* + miR.494 + miR.518c + miR.338 | 0,81 | 0,076569129 |
| miR.558 + miR.609 + miR.603 + miR.220a + miR.29a + miR.369.3p + miR.376a* + miR.494 + miR.518c + miR.639 | 0,81 | 0,015134717 |
| miR.558 + miR.609 + miR.603 + miR.220a + miR.29a + miR.369.3p + miR.376a* + miR.518c + miR.639 + miR.338 | 0,81 | 0,058817566 |
| miR.558 + miR.609 + miR.220a + miR.26a + miR.29a + miR.362 + miR.369.3p + miR.376a* + miR.494 + miR.518c | 0,81 | 0,487975487 |
| miR.558 + miR.609 + miR.520f + miR.220a + miR.29a + miR.376a* + miR.494 + miR.518c + miR.660 + miR.338 | 0,81 | 0,257463392 |
| miR.558 + miR.609 + miR.220a + miR.130a + miR.29a + miR.369.3p + miR.376a* + miR.494 + miR.518c + miR.660 | 0,81 | 0,308349348 |
| miR.558 + miR.609 + miR.220a + miR.26a + miR.29a + miR.369.3p + miR.376a* + miR.494 + miR.518c + miR.639 | 0,81 | 0,471868104 |
| miR.558 + miR.609 + miR.220a + miR.130a + miR.26a + miR.29a + miR.369.3p + miR.376a* + miR.518c + miR.338 | 0,81 | 0,362016475 |
| miR.558 + miR.609 + miR.520f + miR.130a + miR.29a + miR.369.3p + miR.376a* + miR.518c + miR.660 + miR.338 | 0,81 | 0,313361348 |
| miR.558 + miR.609 + miR.603 + miR.520f + miR.29a + miR.369.3p + miR.376a* + miR.494 + miR.518c + miR.660 | 0,81 | 0,071084446 |
| miR.558 + miR.609 + miR.603 + miR.520f + miR.220a + miR.519b + miR.29a + miR.376a* + miR.518c + miR.338 | 0,81 | 0,089838817 |
| miR.558 + miR.609 + miR.520f + miR.130a + miR.29a + miR.369.3p + miR.376a* + miR.518c + miR.639 + miR.660 | 0,81 | 0,116329817 |
| miR.558 + miR.609 + miR.520f + miR.220a + miR.29a + miR.362 + miR.369.3p + miR.376a* + miR.494 + miR.518c | 0,81 | 0,252782252 |
| miR.558 + miR.609 + miR.603 + miR.26a + miR.29a + miR.369.3p + miR.376a* + miR.494 + miR.518c + miR.338 | 0,81 | 0,450726322 |
| miR.558 + miR.609 + miR.520f + miR.220a + miR.26a + miR.29a + miR.369.3p + miR.376a* + miR.518c + miR.639 | 0,81 | 0,283689117 |
| miR.558 + miR.609 + miR.603 + miR.26a + miR.29a + miR.369.3p + miR.376a* + miR.494 + miR.518c + miR.639 | 0,81 | 0,212059129 |
| miR.558 + miR.609 + miR.603 + miR.520f + miR.220a + miR.130a + miR.29a + miR.362 + miR.376a* + miR.518c | 0,81 | 0,007645967 |
| miR.558 + miR.609 + miR.520f + miR.26a + miR.29a + miR.369.3p + miR.376a* + miR.518c + miR.660 + miR.338 | 0,81 | 0,402354453 |
| miR.558 + miR.609 + miR.220a + miR.519b + miR.29a + miR.362 + miR.369.3p + miR.376a* + miR.518c + miR.660 | 0,81 | 0,469190746 |
| miR.558 + miR.609 + miR.520f + miR.220a + miR.29a + miR.362 + miR.369.3p + miR.376a* + miR.518c + miR.639 | 0,81 | 0,240452421 |
| miR.558 + miR.609 + miR.603 + miR.26a + miR.29a + miR.369.3p + miR.376a* + miR.518c + miR.639 + miR.660 | 0,81 | 0,231762576 |
| miR.558 + miR.609 + miR.220a + miR.519b + miR.26a + miR.29a + miR.369.3p + miR.376a* + miR.518c + miR.338 | 0,81 | 0,384843321 |
| miR.558 + miR.609 + miR.220a + miR.519b + miR.26a + miR.29a + miR.369.3p + miR.376a* + miR.494 + miR.518c | 0,81 | 0,449405178 |
| miR.558 + miR.609 + miR.603 + miR.130a + miR.26a + miR.29a + miR.369.3p + miR.376a* + miR.518c + miR.639 | 0,81 | 0,199361251 |
| miR.558 + miR.609 + miR.520f + miR.220a + miR.519b + miR.29a + miR.376a* + miR.494 + miR.518c + miR.660 | 0,81 | 0,210817077 |
| miR.558 + miR.609 + miR.603 + miR.220a + miR.130a + miR.26a + miR.29a + miR.376a* + miR.494 + miR.518c | 0,81 | 0,001027791 |
| miR.558 + miR.609 + miR.603 + miR.520f + miR.29a + miR.369.3p + miR.376a* + miR.494 + miR.518c + miR.639 | 0,81 | 0,044394297 |
| miR.558 + miR.609 + miR.603 + miR.520f + miR.29a + miR.362 + miR.369.3p + miR.376a* + miR.518c + miR.639 | 0,81 | 0,039881187 |
| miR.558 + miR.609 + miR.220a + miR.519b + miR.29a + miR.369.3p + miR.376a* + miR.494 + miR.518c + miR.660 | 0,81 | 0,326020377 |
| miR.558 + miR.609 + miR.520f + miR.519b + miR.29a + miR.369.3p + miR.376a* + miR.518c + miR.660 + miR.338 | 0,81 | 0,42508147 |
| miR.558 + miR.609 + miR.603 + miR.520f + miR.29a + miR.362 + miR.376a* + miR.518c + miR.639 + miR.338 | 0,81 | 0,094439382 |
| miR.558 + miR.609 + miR.220a + miR.26a + miR.29a + miR.362 + miR.369.3p + miR.376a* + miR.518c + miR.639 | 0,81 | 0,394201952 |
| miR.558 + miR.609 + miR.603 + miR.520f + miR.29a + miR.376a* + miR.518c + miR.639 + miR.660 + miR.338 | 0,81 | 0,204337264 |
| miR.558 + miR.609 + miR.603 + miR.220a + miR.130a + miR.29a + miR.369.3p + miR.376a* + miR.518c + miR.338 | 0,81 | 0,104079509 |
| miR.558 + miR.609 + miR.220a + miR.130a + miR.26a + miR.29a + miR.369.3p + miR.376a* + miR.518c + miR.639 | 0,81 | 0,322168393 |
| miR.558 + miR.609 + miR.603 + miR.520f + miR.220a + miR.29a + miR.362 + miR.376a* + miR.518c + miR.660 | 0,81 | 0,020820914 |
| miR.558 + miR.609 + miR.220a + miR.130a + miR.26a + miR.29a + miR.369.3p + miR.376a* + miR.494 + miR.518c | 0,81 | 0,471599647 |
| miR.558 + miR.609 + miR.520f + miR.220a + miR.519b + miR.29a + miR.369.3p + miR.376a* + miR.518c + miR.338 | 0,81 | 0,496153956 |
| miR.558 + miR.609 + miR.520f + miR.220a + miR.130a + miR.29a + miR.362 + miR.369.3p + miR.376a* + miR.518c | 0,81 | 0,263908247 |
| miR.558 + miR.609 + miR.603 + miR.520f + miR.220a + miR.130a + miR.29a + miR.376a* + miR.518c + miR.660 | 0,81 | 0,022160449 |
| miR.558 + miR.609 + miR.603 + miR.220a + miR.130a + miR.26a + miR.29a + miR.376a* + miR.518c + miR.639 | 0,81 | 0,00070945 |
| miR.558 + miR.609 + miR.520f + miR.220a + miR.29a + miR.376a* + miR.494 + miR.518c + miR.639 + miR.660 | 0,81 | 0,15161367 |
| miR.558 + miR.609 + miR.603 + miR.520f + miR.519b + miR.29a + miR.369.3p + miR.376a* + miR.518c + miR.639 | 0,81 | 0,069421065 |
| miR.558 + miR.609 + miR.603 + miR.26a + miR.29a + miR.369.3p + miR.376a* + miR.518c + miR.660 + miR.338 | 0,81 | 0,480125378 |
| miR.558 + miR.609 + miR.520f + miR.220a + miR.519b + miR.29a + miR.362 + miR.369.3p + miR.376a* + miR.518c | 0,81 | 0,306260109 |
| miR.558 + miR.609 + miR.520f + miR.26a + miR.29a + miR.369.3p + miR.376a* + miR.518c + miR.639 + miR.338 | 0,81 | 0,303184497 |
| miR.558 + miR.609 + miR.603 + miR.520f + miR.220a + miR.26a + miR.29a + miR.376a* + miR.518c + miR.639 | 0,81 | 0,001824984 |
| miR.558 + miR.609 + miR.603 + miR.220a + miR.519b + miR.29a + miR.376a* + miR.518c + miR.660 + miR.338 | 0,81 | 0,189955258 |
| miR.558 + miR.609 + miR.603 + miR.520f + miR.220a + miR.29a + miR.376a* + miR.494 + miR.518c + miR.338 | 0,81 | 0,035803136 |
| miR.558 + miR.609 + miR.603 + miR.520f + miR.220a + miR.26a + miR.29a + miR.376a* + miR.494 + miR.518c | 0,81 | 0,001336434 |
| miR.558 + miR.609 + miR.603 + miR.520f + miR.220a + miR.26a + miR.29a + miR.376a* + miR.518c + miR.338 | 0,81 | 0,025600957 |
| miR.558 + miR.609 + miR.520f + miR.29a + miR.369.3p + miR.376a* + miR.494 + miR.518c + miR.639 + miR.338 | 0,81 | 0,202237208 |
| miR.558 + miR.609 + miR.520f + miR.220a + miR.130a + miR.26a + miR.29a + miR.369.3p + miR.376a* + miR.518c | 0,81 | 0,347964318 |
| miR.558 + miR.609 + miR.603 + miR.520f + miR.519b + miR.29a + miR.369.3p + miR.376a* + miR.494 + miR.518c | 0,81 | 0,108055295 |
| miR.558 + miR.609 + miR.603 + miR.520f + miR.26a + miR.29a + miR.369.3p + miR.376a* + miR.518c + miR.660 | 0,81 | 0,041141359 |
| miR.558 + miR.609 + miR.603 + miR.130a + miR.29a + miR.369.3p + miR.376a* + miR.518c + miR.639 + miR.338 | 0,81 | 0,111738164 |
| miR.558 + miR.609 + miR.603 + miR.520f + miR.130a + miR.29a + miR.369.3p + miR.376a* + miR.518c + miR.639 | 0,81 | 0,044132835 |
| miR.558 + miR.609 + miR.603 + miR.520f + miR.26a + miR.29a + miR.369.3p + miR.376a* + miR.494 + miR.518c | 0,81 | 0,096065859 |
| miR.558 + miR.609 + miR.520f + miR.130a + miR.29a + miR.376a* + miR.518c + miR.639 + miR.660 + miR.338 | 0,81 | 0,234279085 |
| miR.558 + miR.609 + miR.603 + miR.220a + miR.519b + miR.29a + miR.376a* + miR.494 + miR.518c + miR.639 | 0,81 | 0,033482371 |
| miR.558 + miR.609 + miR.520f + miR.130a + miR.29a + miR.369.3p + miR.376a* + miR.494 + miR.518c + miR.660 | 0,81 | 0,080162974 |
| miR.558 + miR.609 + miR.520f + miR.220a + miR.519b + miR.29a + miR.362 + miR.376a* + miR.494 + miR.518c | 0,81 | 0,287359831 |
| miR.558 + miR.609 + miR.520f + miR.519b + miR.29a + miR.376a* + miR.494 + miR.518c + miR.660 + miR.338 | 0,81 | 0,186070849 |
| miR.558 + miR.609 + miR.220a + miR.29a + miR.369.3p + miR.376a* + miR.494 + miR.518c + miR.660 + miR.338 | 0,81 | 0,252498532 |
| miR.558 + miR.609 + miR.603 + miR.520f + miR.220a + miR.519b + miR.26a + miR.29a + miR.376a* + miR.518c | 0,81 | 0,008098556 |
| miR.558 + miR.609 + miR.520f + miR.220a + miR.519b + miR.26a + miR.29a + miR.369.3p + miR.376a* + miR.518c | 0,81 | 0,385959252 |
| miR.558 + miR.609 + miR.603 + miR.520f + miR.220a + miR.130a + miR.26a + miR.29a + miR.376a* + miR.518c | 0,81 | 0,002504812 |
| miR.558 + miR.609 + miR.220a + miR.519b + miR.26a + miR.29a + miR.362 + miR.369.3p + miR.376a* + miR.518c | 0,81 | 0,382855731 |
| miR.558 + miR.609 + miR.520f + miR.220a + miR.130a + miR.29a + miR.369.3p + miR.376a* + miR.494 + miR.518c | 0,81 | 0,246307193 |
| miR.558 + miR.609 + miR.220a + miR.519b + miR.130a + miR.26a + miR.29a + miR.369.3p + miR.376a* + miR.518c | 0,81 | 0,359670745 |
| miR.558 + miR.609 + miR.520f + miR.130a + miR.29a + miR.376a* + miR.494 + miR.518c + miR.639 + miR.660 | 0,81 | 0,082870159 |
| miR.558 + miR.609 + miR.220a + miR.29a + miR.369.3p + miR.376a* + miR.494 + miR.518c + miR.639 + miR.660 | 0,81 | 0,23614188 |
| miR.558 + miR.609 + miR.603 + miR.520f + miR.29a + miR.362 + miR.369.3p + miR.376a* + miR.518c + miR.660 | 0,81 | 0,215991569 |
| miR.558 + miR.609 + miR.603 + miR.520f + miR.130a + miR.29a + miR.369.3p + miR.376a* + miR.518c + miR.660 | 0,81 | 0,060077038 |
| miR.558 + miR.609 + miR.520f + miR.220a + miR.29a + miR.369.3p + miR.376a* + miR.494 + miR.518c + miR.639 | 0,81 | 0,171419098 |
| miR.558 + miR.609 + miR.603 + miR.520f + miR.29a + miR.376a* + miR.494 + miR.518c + miR.639 + miR.338 | 0,81 | 0,031423091 |
| miR.558 + miR.609 + miR.603 + miR.26a + miR.29a + miR.362 + miR.369.3p + miR.376a* + miR.518c + miR.338 | 0,81 | 0,473666173 |
| miR.558 + miR.609 + miR.603 + miR.130a + miR.26a + miR.29a + miR.369.3p + miR.376a* + miR.518c + miR.338 | 0,81 | 0,450664408 |
| miR.558 + miR.609 + miR.220a + miR.29a + miR.369.3p + miR.376a* + miR.518c + miR.639 + miR.660 + miR.338 | 0,81 | 0,316633041 |
| miR.558 + miR.609 + miR.220a + miR.519b + miR.29a + miR.369.3p + miR.376a* + miR.518c + miR.639 + miR.660 | 0,81 | 0,31322339 |
| miR.558 + miR.609 + miR.220a + miR.519b + miR.29a + miR.369.3p + miR.376a* + miR.518c + miR.660 + miR.338 | 0,81 | 0,407468907 |
| miR.558 + miR.609 + miR.603 + miR.26a + miR.29a + miR.369.3p + miR.376a* + miR.494 + miR.518c + miR.660 | 0,81 | 0,254326465 |
| miR.558 + miR.609 + miR.603 + miR.520f + miR.220a + miR.519b + miR.29a + miR.376a* + miR.518c + miR.639 | 0,81 | 0,01008574 |
| miR.558 + miR.609 + miR.220a + miR.130a + miR.26a + miR.29a + miR.362 + miR.369.3p + miR.376a* + miR.518c | 0,81 | 0,358881729 |
| miR.558 + miR.609 + miR.520f + miR.29a + miR.362 + miR.369.3p + miR.376a* + miR.518c + miR.639 + miR.338 | 0,81 | 0,229324595 |
| miR.558 + miR.609 + miR.603 + miR.520f + miR.519b + miR.29a + miR.376a* + miR.494 + miR.518c + miR.338 | 0,81 | 0,158335392 |
| miR.558 + miR.609 + miR.603 + miR.520f + miR.220a + miR.519b + miR.130a + miR.29a + miR.376a* + miR.518c | 0,81 | 0,011335269 |
| miR.558 + miR.609 + miR.603 + miR.519b + miR.26a + miR.29a + miR.369.3p + miR.376a* + miR.518c + miR.338 | 0,81 | 0,480661116 |
| miR.558 + miR.609 + miR.520f + miR.220a + miR.519b + miR.29a + miR.369.3p + miR.376a* + miR.494 + miR.518c | 0,81 | 0,356384924 |
| miR.558 + miR.609 + miR.520f + miR.220a + miR.26a + miR.29a + miR.376a* + miR.518c + miR.660 + miR.338 | 0,81 | 0,279741056 |
| miR.558 + miR.609 + miR.603 + miR.220a + miR.519b + miR.26a + miR.29a + miR.376a* + miR.518c + miR.639 | 0,81 | 0,009120381 |
| miR.558 + miR.609 + miR.520f + miR.220a + miR.130a + miR.29a + miR.376a* + miR.518c + miR.660 + miR.338 | 0,81 | 0,27193668 |
| miR.558 + miR.609 + miR.603 + miR.519b + miR.26a + miR.29a + miR.369.3p + miR.376a* + miR.518c + miR.639 | 0,81 | 0,205050527 |
| miR.558 + miR.609 + miR.603 + miR.220a + miR.130a + miR.29a + miR.376a* + miR.494 + miR.518c + miR.639 | 0,81 | 0,000348129 |
| miR.558 + miR.609 + miR.220a + miR.519b + miR.26a + miR.29a + miR.369.3p + miR.376a* + miR.518c + miR.639 | 0,81 | 0,388486849 |
| miR.558 + miR.609 + miR.520f + miR.220a + miR.29a + miR.362 + miR.376a* + miR.518c + miR.660 + miR.338 | 0,81 | 0,320250759 |
| miR.558 + miR.609 + miR.220a + miR.130a + miR.29a + miR.369.3p + miR.376a* + miR.518c + miR.660 + miR.338 | 0,81 | 0,480642513 |
| miR.558 + miR.609 + miR.520f + miR.220a + miR.519b + miR.29a + miR.362 + miR.376a* + miR.518c + miR.338 | 0,81 | 0,376650107 |
| miR.558 + miR.609 + miR.520f + miR.220a + miR.29a + miR.362 + miR.376a* + miR.494 + miR.518c + miR.338 | 0,81 | 0,281795082 |
| miR.558 + miR.609 + miR.603 + miR.29a + miR.362 + miR.369.3p + miR.376a* + miR.518c + miR.660 + miR.338 | 0,81 | 0,273430203 |
| miR.558 + miR.609 + miR.520f + miR.220a + miR.519b + miR.29a + miR.376a* + miR.518c + miR.660 + miR.338 | 0,81 | 0,45696 1795 |
| miR.558 + miR.609 + miR.603 + miR.26a + miR.29a + miR.362 + miR.369.3p + miR.376a* + miR.518c + miR.639 | 0,81 | 0,200255509 |
| miR.558 + miR.609 + miR.520f + miR.220a + miR.29a + miR.362 + miR.376a* + miR.494 + miR.518c + miR.660 | 0,81 | 0,08991085 |
| miR.558 + miR.609 + miR.603 + miR.520f + miR.130a + miR.26a + miR.29a + miR.369.3p + miR.376a* + miR.518c | 0,81 | 0,059566171 |
| miR.558 + miR.609 + miR.603 + miR.29a + miR.362 + miR.369.3p + miR.376a* + miR.518c + miR.639 + miR.660 | 0,81 | 0,264461638 |
| miR.558 + miR.609 + miR.603 + miR.220a + miR.519b + miR.29a + miR.376a* + miR.518c + miR.639 + miR.338 | 0,81 | 0,023753572 |
| miR.558 + miR.609 + miR.603 + miR.130a + miR.29a + miR.369.3p + miR.376a* + miR.494 + miR.518c + miR.639 | 0,81 | 0,073592704 |
| miR.558 + miR.609 + miR.520f + miR.220a + miR.26a + miR.29a + miR.376a* + miR.494 + miR.518c + miR.660 | 0,81 | 0,085052412 |
| miR.558 + miR.609 + miR.520f + miR.220a + miR.29a + miR.362 + miR.376a* + miR.518c + miR.639 + miR.338 | 0,81 | 0,185195833 |
| miR.558 + miR.609 + miR.603 + miR.220a + miR.519b + miR.130a + miR.29a + miR.376a* + miR.518c + miR.338 | 0,81 | 0,020120546 |
| miR.558 + miR.609 + miR.603 + miR.520f + miR.220a + miR.130a + miR.29a + miR.376a* + miR.518c + miR.639 | 0,81 | 0,000981724 |
| miR.558 + miR.609 + miR.603 + miR.220a + miR.130a + miR.29a + miR.362 + miR.376a* + miR.518c + miR.639 | 0,81 | 0,00504257 |
| miR.558 + miR.609 + miR.520f + miR.220a + miR.130a + miR.29a + miR.376a* + miR.494 + miR.518c + miR.660 | 0,81 | 0,073850466 |
| miR.558 + miR.609 + miR.520f + miR.29a + miR.362 + miR.376a* + miR.518c + miR.639 + miR.660 + miR.338 | 0,81 | 0,263320303 |
| miR.558 + miR.609 + miR.520f + miR.130a + miR.29a + miR.369.3p + miR.376a* + miR.494 + miR.518c + miR.639 | 0,81 | 0,108101911 |
| miR.558 + miR.609 + miR.603 + miR.220a + miR.519b + miR.130a + miR.29a + miR.376a* + miR.518c + miR.639 | 0,81 | 0,011210467 |
| miR.558 + miR.609 + miR.520f + miR.26a + miR.29a + miR.376a* + miR.518c + miR.639 + miR.660 + miR.338 | 0,81 | 0,222430836 |
| miR.558 + miR.609 + miR.520f + miR.519b + miR.29a + miR.376a* + miR.518c + miR.639 + miR.660 + miR.338 | 0,81 | 0,244405577 |
| miR.558 + miR.609 + miR.520f + miR.29a + miR.362 + miR.369.3p + miR.376a* + miR.518c + miR.639 + miR.660 | 0,81 | 0,248454565 |
| miR.558 + miR.609 + miR.603 + miR.26a + miR.29a + miR.362 + miR.369.3p + miR.376a* + miR.518c + miR.660 | 0,81 | 0,382633637 |
| miR.558 + miR.609 + miR.520f + miR.130a + miR.29a + miR.369.3p + miR.376a* + miR.518c + miR.639 + miR.338 | 0,81 | 0,236366899 |
| miR.558 + miR.609 + miR.603 + miR.520f + miR.519b + miR.29a + miR.369.3p + miR.376a* + miR.518c + miR.660 | 0,81 | 0,053852408 |
| miR.558 + miR.609 + miR.603 + miR.520f + miR.26a + miR.29a + miR.362 + miR.369.3p + miR.376a* + miR.518c | 0,81 | 0,079708776 |
| miR.558 + miR.609 + miR.603 + miR.520f + miR.130a + miR.29a + miR.369.3p + miR.376a* + miR.494 + miR.518c | 0,81 | 0,065575769 |
| miR.558 + miR.609 + miR.603 + miR.220a + miR.130a + miR.29a + miR.362 + miR.376a* + miR.494 + miR.518c | 0,81 | 0,001381817 |
| miR.558 + miR.609 + miR.520f + miR.220a + miR.130a + miR.29a + miR.369.3p + miR.376a* + miR.518c + miR.639 | 0,81 | 0,137192282 |
| miR.558 + miR.609 + miR.603 + miR.220a + miR.519b + miR.130a + miR.29a + miR.376a* + miR.518c + miR.660 | 0,81 | 0,107219978 |
| miR.558 + miR.609 + miR.220a + miR.519b + miR.130a + miR.29a + miR.369.3p + miR.376a* + miR.494 + miR.518c | 0,81 | 0,324848373 |
| miR.558 + miR.609 + miR.603 + miR.220a + miR.29a + miR.376a* + miR.518c + miR.639 + miR.660 + miR.338 | 0,81 | 0,015660171 |
| miR.558 + miR.609 + miR.520f + miR.26a + miR.29a + miR.369.3p + miR.376a* + miR.494 + miR.518c + miR.338 | 0,81 | 0,322097373 |
| miR.558 + miR.609 + miR.603 + miR.26a + miR.29a + miR.362 + miR.369.3p + miR.376a* + miR.494 + miR.518c | 0,81 | 0,307177464 |
| miR.558 + miR.609 + miR.520f + miR.220a + miR.29a + miR.362 + miR.376a* + miR.494 + miR.518c + miR.639 | 0,81 | 0,065097 |
| miR.558 + miR.609 + miR.520f + miR.26a + miR.29a + miR.376a* + miR.494 + miR.518c + miR.660 + miR.338 | 0,81 | 0,232021177 |
| miR.558 + miR.609 + miR.603 + miR.220a + miR.519b + miR.29a + miR.376a* + miR.518c + miR.639 + miR.660 | 0,81 | 0,133199324 |
| miR.558 + miR.609 + miR.603 + miR.130a + miR.26a + miR.29a + miR.369.3p + miR.376a* + miR.494 + miR.518c | 0,81 | 0,292852361 |
| miR.558 + miR.609 + miR.603 + miR.220a + miR.29a + miR.362 + miR.376a* + miR.518c + miR.639 + miR.338 | 0,81 | 0,005024632 |
| miR.558 + miR.609 + miR.603 + miR.520f + miR.29a + miR.362 + miR.369.3p + miR.376a* + miR.494 + miR.518c | 0,81 | 0,060906889 |
| miR.558 + miR.609 + miR.520f + miR.29a + miR.362 + miR.376a* + miR.494 + miR.518c + miR.660 + miR.338 | 0,81 | 0,159914432 |
| miR.558 + miR.609 + miR.520f + miR.130a + miR.29a + miR.376a* + miR.494 + miR.518c + miR.660 + miR.338 | 0,81 | 0,209755519 |
| miR.558 + miR.609 + miR.603 + miR.520f + miR.220a + miR.130a + miR.29a + miR.376a* + miR.494 + miR.518c | 0,81 | 0,002621169 |
| miR.558 + miR.609 + miR.220a + miR.26a + miR.29a + miR.376a* + miR.494 + miR.518c + miR.639 + miR.660 | 0,81 | 0,097978868 |
| miR.558 + miR.609 + miR.520f + miR.220a + miR.519b + miR.29a + miR.369.3p + miR.376a* + miR.518c + miR.639 | 0,81 | 0,263790625 |
| miR.609 + miR.603 + miR.520f + miR.220a + miR.29a + miR.369.3p + miR.376a* + miR.494 + miR.518c + miR.660 | 0,81 | 0,40479337 |
| miR.558 + miR.609 + miR.520f + miR.519b + miR.29a + miR.369.3p + miR.376a* + miR.494 + miR.518c + miR.338 | 0,81 | 0,35591504 |
| miR.558 + miR.609 + miR.603 + miR.520f + miR.29a + miR.376a* + miR.494 + miR.518c + miR.660 + miR.338 | 0,81 | 0,235368241 |
| miR.558 + miR.609 + miR.520f + miR.519b + miR.29a + miR.369.3p + miR.376a* + miR.518c + miR.639 + miR.660 | 0,81 | 0,080957183 |
| miR.558 + miR.609 + miR.520f + miR.29a + miR.369.3p + miR.376a* + miR.494 + miR.518c + miR.639 + miR.660 | 0,81 | 0,099097856 |
| miR.609 + miR.603 + miR.220a + miR.519b + miR.29a + miR.369.3p + miR.376a* + miR.494 + miR.518c + miR.660 | 0,81 | 0,39848711 |
| miR.558 + miR.609 + miR.603 + miR.519b + miR.26a + miR.29a + miR.369.3p + miR.376a* + miR.494 + miR.518c | 0,81 | 0,275215499 |
| miR.558 + miR.609 + miR.603 + miR.520f + miR.29a + miR.376a* + miR.494 + miR.518c + miR.639 + miR.660 | 0,81 | 0,02392215 |
| miR.558 + miR.609 + miR.520f + miR.220a + miR.26a + miR.29a + miR.362 + miR.376a* + miR.518c + miR.338 | 0,81 | 0,262963178 |
| miR.558 + miR.609 + miR.603 + miR.520f + miR.29a + miR.362 + miR.376a* + miR.494 + miR.518c + miR.338 | 0,81 | 0,155122084 |
| miR.558 + miR.609 + miR.520f + miR.29a + miR.362 + miR.369.3p + miR.376a* + miR.494 + miR.518c + miR.338 | 0,81 | 0,25689938 |
| miR.558 + miR.609 + miR.520f + miR.220a + miR.130a + miR.29a + miR.362 + miR.376a* + miR.518c + miR.338 | 0,81 | 0,334384744 |
| miR.558 + miR.609 + miR.603 + miR.220a + miR.29a + miR.376a* + miR.494 + miR.518c + miR.639 + miR.660 | 0,81 | 0,015683767 |
| miR.558 + miR.609 + miR.603 + miR.520f + miR.519b + miR.26a + miR.29a + miR.369.3p + miR.376a* + miR.518c | 0,81 | 0,124969108 |
| miR.558 + miR.609 + miR.520f + miR.130a + miR.29a + miR.369.3p + miR.376a* + miR.494 + miR.518c + miR.338 | 0,81 | 0,28283847 |
| miR.558 + miR.609 + miR.26a + miR.29a + miR.362 + miR.369.3p + miR.376a* + miR.518c + miR.660 + miR.338 | 0,81 | 0,329265895 |
| miR.558 + miR.609 + miR.520f + miR.220a + miR.519b + miR.130a + miR.29a + miR.369.3p + miR.376a* + miR.518c | 0,81 | 0,287853034 |
| miR.558 + miR.609 + miR.220a + miR.130a + miR.29a + miR.369.3p + miR.376a* + miR.518c + miR.639 + miR.338 | 0,81 | 0,20598416 |
| miR.558 + miR.609 + miR.603 + miR.220a + miR.130a + miR.29a + miR.376a* + miR.494 + miR.518c + miR.660 | 0,81 | 0,002888413 |
| miR.558 + miR.609 + miR.603 + miR.130a + miR.29a + miR.369.3p + miR.376a* + miR.518c + miR.639 + miR.660 | 0,81 | 0,073738899 |
| miR.558 + miR.609 + miR.220a + miR.130a + miR.26a + miR.29a + miR.376a* + miR.494 + miR.518c + miR.660 | 0,81 | 0,138336111 |
| miR.558 + miR.609 + miR.520f + miR.519b + miR.29a + miR.369.3p + miR.376a* + miR.518c + miR.639 + miR.338 | 0,81 | 0,263983098 |
| miR.558 + miR.609 + miR.520f + miR.220a + miR.29a + miR.362 + miR.376a* + miR.518c + miR.639 + miR.660 | 0,81 | 0,1472095 |
| miR.558 + miR.609 + miR.603 + miR.520f + miR.130a + miR.29a + miR.362 + miR.369.3p + miR.376a* + miR.518c | 0,81 | 0,05517958 |
| miR.558 + miR.609 + miR.603 + miR.519b + miR.26a + miR.29a + miR.369.3p + miR.376a* + miR.518c + miR.660 | 0,81 | 0,175306949 |
| miR.558 + miR.609 + miR.603 + miR.520f + miR.519b + miR.29a + miR.362 + miR.369.3p + miR.376a* + miR.518c | 0,81 | 0,064756459 |
| miR.558 + miR.609 + miR.603 + miR.220a + miR.130a + miR.29a + miR.362 + miR.376a* + miR.518c + miR.338 | 0,81 | 0,006001262 |
| miR.558 + miR.609 + miR.130a + miR.29a + miR.362 + miR.369.3p + miR.376a* + miR.518c + miR.639 + miR.660 | 0,81 | 0,252405915 |
| miR.558 + miR.609 + miR.220a + miR.130a + miR.29a + miR.362 + miR.369.3p + miR.376a* + miR.518c + miR.639 | 0,81 | 0,147640619 |
| miR.558 + miR.609 + miR.520f + miR.130a + miR.29a + miR.362 + miR.369.3p + miR.376a* + miR.518c + miR.660 | 0,81 | 0,160650467 |
| miR.558 + miR.609 + miR.603 + miR.520f + miR.29a + miR.362 + miR.376a* + miR.494 + miR.518c + miR.639 | 0,81 | 0,018157199 |
| miR.558 + miR.609 + miR.603 + miR.520f + miR.519b + miR.29a + miR.376a* + miR.518c + miR.639 + miR.338 | 0,81 | 0,0746739 |
| miR.558 + miR.609 + miR.603 + miR.130a + miR.26a + miR.29a + miR.369.3p + miR.376a* + miR.518c + miR.660 | 0,81 | 0,176148762 |
| miR.558 + miR.609 + miR.603 + miR.520f + miR.130a + miR.29a + miR.376a* + miR.518c + miR.639 + miR.338 | 0,81 | 0,027896681 |
| miR.558 + miR.609 + miR.520f + miR.220a + miR.519b + miR.29a + miR.376a* + miR.518c + miR.639 + miR.660 | 0,81 | 0,110719455 |
| miR.609 + miR.603 + miR.220a + miR.29a + miR.362 + miR.369.3p + miR.376a* + miR.494 + miR.518c + miR.660 | 0,81 | 0,447289899 |
| miR.558 + miR.609 + miR.220a + miR.26a + miR.29a + miR.362 + miR.376a* + miR.494 + miR.518c + miR.639 | 0,81 | 0,130242993 |
| miR.558 + miR.609 + miR.520f + miR.220a + miR.130a + miR.29a + miR.362 + miR.376a* + miR.494 + miR.518c | 0,81 | 0,141157911 |
| miR.558 + miR.609 + miR.520f + miR.220a + miR.26a + miR.29a + miR.376a* + miR.518c + miR.639 + miR.660 | 0,81 | 0,122398943 |
| miR.558 + miR.609 + miR.520f + miR.220a + miR.26a + miR.29a + miR.362 + miR.376a* + miR.494 + miR.518c | 0,81 | 0,092904362 |
| miR.558 + miR.609 + miR.220a + miR.519b + miR.130a + miR.29a + miR.369.3p + miR.376a* + miR.518c + miR.338 | 0,81 | 0,414797687 |
| miR.558 + miR.609 + miR.603 + miR.520f + miR.519b + miR.130a + miR.29a + miR.369.3p + miR.376a* + miR.518c | 0,81 | 0,074887432 |
| miR.558 + miR.609 + miR.520f + miR.130a + miR.26a + miR.29a + miR.369.3p + miR.376a* + miR.518c + miR.660 | 0,81 | 0,062741407 |
| miR.558 + miR.609 + miR.520f + miR.220a + miR.519b + miR.29a + miR.362 + miR.376a* + miR.518c + miR.639 | 0,81 | 0,175724939 |
| miR.558 + miR.609 + miR.603 + miR.520f + miR.26a + miR.29a + miR.376a* + miR.518c + miR.639 + miR.338 | 0,81 | 0,023148698 |
| miR.558 + miR.609 + miR.520f + miR.130a + miR.29a + miR.376a* + miR.494 + miR.518c + miR.639 + miR.338 | 0,81 | 0,050371527 |
| miR.558 + miR.609 + miR.220a + miR.519b + miR.130a + miR.29a + miR.362 + miR.369.3p + miR.376a* + miR.518c | 0,81 | 0,34228867 |
| miR.558 + miR.609 + miR.520f + miR.220a + miR.519b + miR.26a + miR.29a + miR.362 + miR.376a* + miR.518c | 0,81 | 0,211983257 |
| miR.558 + miR.609 + miR.520f + miR.220a + miR.29a + miR.376a* + miR.494 + miR.518c + miR.639 + miR.338 | 0,81 | 0,068578966 |
| miR.558 + miR.609 + miR.520f + miR.26a + miR.29a + miR.369.3p + miR.376a* + miR.518c + miR.639 + miR.660 | 0,81 | 0,095372662 |
| miR.558 + miR.609 + miR.520f + miR.220a + miR.130a + miR.29a + miR.376a* + miR.518c + miR.639 + miR.660 | 0,81 | 0,137704734 |
| miR.558 + miR.609 + miR.220a + miR.26a + miR.29a + miR.362 + miR.376a* + miR.494 + miR.518c + miR.338 | 0,81 | 0,235173704 |
| miR.558 + miR.609 + miR.520f + miR.220a + miR.519b + miR.29a + miR.362 + miR.376a* + miR.518c + miR.660 | 0,81 | 0,128233558 |
| miR.558 + miR.609 + miR.520f + miR.26a + miR.29a + miR.362 + miR.369.3p + miR.376a* + miR.518c + miR.338 | 0,81 | 0,337022388 |
| miR.558 + miR.609 + miR.520f + miR.220a + miR.519b + miR.130a + miR.29a + miR.362 + miR.376a* + miR.518c | 0,81 | 0,208433596 |
| miR.609 + miR.603 + miR.220a + miR.26a + miR.29a + miR.369.3p + miR.376a* + miR.494 + miR.518c + miR.660 | 0,81 | 0,4191519 |
| miR.558 + miR.609 + miR.603 + miR.520f + miR.220a + miR.29a + miR.376a* + miR.518c + miR.639 + miR.338 | 0,81 | 0,028388447 |
| miR.558 + miR.609 + miR.220a + miR.130a + miR.26a + miR.29a + miR.362 + miR.376a* + miR.494 + miR.518c | 0,81 | 0,210834278 |
| miR.558 + miR.609 + miR.220a + miR.519b + miR.130a + miR.29a + miR.369.3p + miR.376a* + miR.518c + miR.639 | 0,81 | 0,212381828 |
| miR.558 + miR.609 + miR.520f + miR.130a + miR.29a + miR.362 + miR.376a* + miR.494 + miR.518c + miR.639 | 0,81 | 0,050364553 |
| miR.558 + miR.609 + miR.520f + miR.220a + miR.26a + miR.29a + miR.362 + miR.376a* + miR.518c + miR.639 | 0,81 | 0,059177851 |
| miR.558 + miR.609 + miR.130a + miR.26a + miR.29a + miR.369.3p + miR.376a* + miR.494 + miR.518c + miR.639 | 0,81 | 0,12648103 |
| miR.558 + miR.609 + miR.603 + miR.220a + miR.29a + miR.362 + miR.376a* + miR.518c + miR.639 + miR.660 | 0,81 | 0,008465347 |
| miR.609 + miR.603 + miR.520f + miR.220a + miR.130a + miR.29a + miR.369.3p + miR.376a* + miR.518c + miR.660 | 0,81 | 0,420460443 |
| miR.558 + miR.609 + miR.520f + miR.29a + miR.362 + miR.369.3p + miR.376a* + miR.494 + miR.518c + miR.660 | 0,81 | 0,163184669 |
| miR.558 + miR.609 + miR.603 + miR.520f + miR.519b + miR.29a + miR.362 + miR.376a* + miR.494 + miR.518c | 0,81 | 0,039497864 |
| miR.558 + miR.609 + miR.520f + miR.220a + miR.519b + miR.29a + miR.376a* + miR.494 + miR.518c + miR.338 | 0,81 | 0,18456671 |
| miR.558 + miR.609 + miR.520f + miR.220a + miR.519b + miR.26a + miR.29a + miR.376a* + miR.518c + miR.660 | 0,81 | 0,082198902 |
| miR.558 + miR.609 + miR.520f + miR.130a + miR.29a + miR.362 + miR.369.3p + miR.376a* + miR.518c + miR.338 | 0,81 | 0,290246432 |
| miR.558 + miR.609 + miR.520f + miR.220a + miR.130a + miR.29a + miR.362 + miR.376a* + miR.518c + miR.639 | 0,81 | 0,103543937 |
| miR.558 + miR.609 + miR.603 + miR.519b + miR.130a + miR.29a + miR.369.3p + miR.376a* + miR.494 + miR.518c | 0,81 | 0,289155068 |
| miR.558 + miR.609 + miR.603 + miR.520f + miR.130a + miR.29a + miR.362 + miR.376a* + miR.518c + miR.338 | 0,81 | 0,195929136 |
| miR.558 + miR.609 + miR.520f + miR.220a + miR.519b + miR.130a + miR.29a + miR.376a* + miR.518c + miR.660 | 0,81 | 0,087787187 |
| miR.558 + miR.609 + miR.603 + miR.519b + miR.130a + miR.26a + miR.29a + miR.369.3p + miR.376a* + miR.518c | 0,81 | 0,229457517 |
| miR.558 + miR.609 + miR.603 + miR.220a + miR.130a + miR.29a + miR.376a* + miR.518c + miR.639 + miR.660 | 0,81 | 0,007679135 |
| miR.558 + miR.609 + miR.520f + miR.220a + miR.26a + miR.29a + miR.376a* + miR.494 + miR.518c + miR.338 | 0,81 | 0,111046985 |
| miR.558 + miR.609 + miR.520f + miR.130a + miR.26a + miR.29a + miR.369.3p + miR.376a* + miR.518c + miR.338 | 0,81 | 0,331151321 |
| miR.558 + miR.609 + miR.603 + miR.130a + miR.26a + miR.29a + miR.362 + miR.369.3p + miR.376a* + miR.518c | 0,81 | 0,280057814 |
| miR.558 + miR.609 + miR.520f + miR.130a + miR.26a + miR.29a + miR.369.3p + miR.376a* + miR.518c + miR.639 | 0,81 | 0,123952603 |
| miR.558 + miR.609 + miR.603 + miR.130a + miR.29a + miR.362 + miR.369.3p + miR.376a* + miR.518c + miR.639 | 0,8 | 0,05897204 |
| miR.558 + miR.609 + miR.603 + miR.220a + miR.29a + miR.376a* + miR.494 + miR.518c + miR.660 + miR.338 | 0,8 | 0,031541686 |
| miR.558 + miR.609 + miR.603 + miR.520f + miR.220a + miR.29a + miR.376a* + miR.494 + miR.518c + miR.639 | 0,8 | 0,001099932 |
| miR.558 + miR.609 + miR.220a + miR.519b + miR.26a + miR.29a + miR.362 + miR.376a* + miR.494 + miR.518c | 0,8 | 0,190198659 |
| miR.558 + miR.609 + miR.220a + miR.130a + miR.29a + miR.362 + miR.369.3p + miR.376a* + miR.494 + miR.518c | 0,8 | 0,171463275 |
| miR.558 + miR.609 + miR.220a + miR.130a + miR.29a + miR.369.3p + miR.376a* + miR.494 + miR.518c + miR.639 | 0,8 | 0,202808687 |
| miR.558 + miR.609 + miR.603 + miR.519b + miR.26a + miR.29a + miR.362 + miR.369.3p + miR.376a* + miR.518c | 0,8 | 0,261471368 |
| miR.558 + miR.609 + miR.220a + miR.519b + miR.26a + miR.29a + miR.376a* + miR.494 + miR.518c + miR.660 | 0,8 | 0,214274152 |
| miR.558 + miR.609 + miR.520f + miR.220a + miR.26a + miR.29a + miR.362 + miR.376a* + miR.518c + miR.660 | 0,8 | 0,088071967 |
| miR.558 + miR.609 + miR.603 + miR.520f + miR.519b + miR.29a + miR.376a* + miR.494 + miR.518c + miR.639 | 0,8 | 0,021161889 |
| miR.558 + miR.609 + miR.603 + miR.520f + miR.130a + miR.29a + miR.376a* + miR.518c + miR.660 + miR.338 | 0,8 | 0,261272665 |
| miR.558 + miR.609 + miR.520f + miR.220a + miR.130a + miR.26a + miR.29a + miR.376a* + miR.518c + miR.660 | 0,8 | 0,062487763 |
| miR.558 + miR.609 + miR.220a + miR.130a + miR.29a + miR.362 + miR.369.3p + miR.376a* + miR.518c + miR.338 | 0,8 | 0,234837862 |
| miR.558 + miR.609 + miR.520f + miR.29a + miR.362 + miR.376a* + miR.494 + miR.518c + miR.639 + miR.338 | 0,8 | 0,09822688 |
| miR.609 + miR.603 + miR.520f + miR.220a + miR.29a + miR.362 + miR.369.3p + miR.376a* + miR.518c + miR.660 | 0,8 | 0,292431465 |
| miR.558 + miR.609 + miR.220a + miR.130a + miR.29a + miR.369.3p + miR.376a* + miR.494 + miR.518c + miR.338 | 0,8 | 0,252903768 |
| miR.558 + miR.609 + miR.520f + miR.220a + miR.130a + miR.26a + miR.29a + miR.362 + miR.376a* + miR.518c | 0,8 | 0,099774745 |
| miR.558 + miR.609 + miR.603 + miR.520f + miR.26a + miR.29a + miR.376a* + miR.494 + miR.518c + miR.338 | 0,8 | 0,041986961 |
| miR.558 + miR.609 + miR.520f + miR.220a + miR.130a + miR.29a + miR.376a* + miR.494 + miR.518c + miR.338 | 0,8 | 0,170273859 |
| miR.558 + miR.609 + miR.603 + miR.520f + miR.220a + miR.130a + miR.29a + miR.376a* + miR.518c + miR.338 | 0,8 | 0,053892411 |
| miR.558 + miR.609 + miR.220a + miR.519b + miR.130a + miR.26a + miR.29a + miR.376a* + miR.494 + miR.518c | 0,8 | 0,089278645 |
| miR.609 + miR.603 + miR.220a + miR.130a + miR.26a + miR.29a + miR.369.3p + miR.376a* + miR.518c + miR.660 | 0,8 | 0,478869728 |
| miR.558 + miR.609 + miR.520f + miR.519b + miR.130a + miR.29a + miR.369.3p + miR.376a* + miR.518c + miR.338 | 0,8 | 0,322922403 |
| miR.558 + miR.609 + miR.520f + miR.26a + miR.29a + miR.369.3p + miR.376a* + miR.494 + miR.518c + miR.639 | 0,8 | 0,121240501 |
| miR.558 + miR.609 + miR.520f + miR.220a + miR.130a + miR.29a + miR.362 + miR.376a* + miR.518c + miR.660 | 0,8 | 0,078987383 |
| miR.558 + miR.609 + miR.603 + miR.220a + miR.29a + miR.362 + miR.376a* + miR.518c + miR.660 + miR.338 | 0,8 | 0,024503351 |
| miR.558 + miR.609 + miR.520f + miR.130a + miR.26a + miR.29a + miR.369.3p + miR.376a* + miR.494 + miR.518c | 0,8 | 0,085308895 |
| miR.558 + miR.609 + miR.603 + miR.520f + miR.130a + miR.29a + miR.376a* + miR.494 + miR.518c + miR.338 | 0,8 | 0,059234491 |
| miR.558 + miR.609 + miR.603 + miR.519b + miR.130a + miR.29a + miR.369.3p + miR.376a* + miR.518c + miR.639 | 0,8 | 0,104002014 |
| miR.558 + miR.609 + miR.520f + miR.519b + miR.130a + miR.29a + miR.369.3p + miR.376a* + miR.494 + miR.518c | 0,8 | 0,147864848 |
| miR.558 + miR.609 + miR.603 + miR.220a + miR.130a + miR.29a + miR.362 + miR.376a* + miR.518c + miR.660 | 0,8 | 0,008460968 |
| miR.558 + miR.609 + miR.603 + miR.520f + miR.26a + miR.29a + miR.362 + miR.376a* + miR.518c + miR.639 | 0,8 | 0,011457893 |
| miR.558 + miR.609 + miR.220a + miR.519b + miR.130a + miR.29a + miR.376a* + miR.494 + miR.518c + miR.660 | 0,8 | 0,112733405 |
| miR.558 + miR.609 + miR.220a + miR.26a + miR.29a + miR.376a* + miR.494 + miR.518c + miR.660 + miR.338 | 0,8 | 0,252848414 |
| miR.609 + miR.603 + miR.220a + miR.29a + miR.362 + miR.369.3p + miR.376a* + miR.518c + miR.639 + miR.660 | 0,8 | 0,480306281 |
| miR.558 + miR.609 + miR.520f + miR.519b + miR.29a + miR.362 + miR.369.3p + miR.376a* + miR.518c + miR.338 | 0,8 | 0,343418956 |
| miR.558 + miR.609 + miR.603 + miR.220a + miR.130a + miR.29a + miR.376a* + miR.518c + miR.660 + miR.338 | 0,8 | 0,020350316 |
| miR.558 + miR.609 + miR.520f + miR.519b + miR.26a + miR.29a + miR.369.3p + miR.376a* + miR.518c + miR.338 | 0,8 | 0,396120627 |
| miR.558 + miR.609 + miR.603 + miR.520f + miR.519b + miR.29a + miR.362 + miR.376a* + miR.518c + miR.338 | 0,8 | 0,183284615 |
| miR.558 + miR.609 + miR.220a + miR.519b + miR.130a + miR.29a + miR.362 + miR.376a* + miR.494 + miR.518c | 0,8 | 0,095177228 |
| miR.558 + miR.609 + miR.520f + miR.26a + miR.29a + miR.369.3p + miR.376a* + miR.494 + miR.518c + miR.660 | 0,8 | 0,077009346 |
| miR.609 + miR.603 + miR.520f + miR.220a + miR.29a + miR.369.3p + miR.376a* + miR.518c + miR.660 + miR.338 | 0,8 | 0,230976952 |
| miR.558 + miR.609 + miR.603 + miR.520f + miR.130a + miR.29a + miR.362 + miR.376a* + miR.518c + miR.639 | 0,8 | 0,013924943 |
| miR.558 + miR.609 + miR.520f + miR.220a + miR.519b + miR.130a + miR.29a + miR.376a* + miR.494 + miR.518c | 0,8 | 0,142359538 |
| miR.558 + miR.609 + miR.603 + miR.520f + miR.519b + miR.29a + miR.362 + miR.376a* + miR.518c + miR.639 | 0,8 | 0,015721883 |
| miR.558 + miR.609 + miR.130a + miR.29a + miR.369.3p + miR.376a* + miR.494 + miR.518c + miR.639 + miR.660 | 0,8 | 0,124896992 |
| miR.558 + miR.609 + miR.603 + miR.29a + miR.362 + miR.369.3p + miR.376a* + miR.494 + miR.518c + miR.660 | 0,8 | 0,134276714 |
| miR.609 + miR.603 + miR.220a + miR.519b + miR.26a + miR.29a + miR.369.3p + miR.376a* + miR.494 + miR.518c | 0,8 | 0,47236884 |
| miR.558 + miR.609 + miR.220a + miR.519b + miR.26a + miR.29a + miR.376a* + miR.494 + miR.518c + miR.338 | 0,8 | 0,119641425 |
| miR.558 + miR.609 + miR.520f + miR.26a + miR.29a + miR.362 + miR.376a* + miR.518c + miR.660 + miR.338 | 0,8 | 0,257438553 |
| miR.609 + miR.603 + miR.520f + miR.220a + miR.29a + miR.369.3p + miR.376a^{*} + miR.518c + miR.639 + miR.660 | 0,8 | 0,328956297 |
| miR.558 + miR.609 + miR.603 + miR.520f + miR.26a + miR.29a + miR.362 + miR.376a* + miR.518c + miR.338 | 0,8 | 0,136991518 |
| miR.609 + miR.603 + miR.220a + miR.29a + miR.369.3p + miR.376a* + miR.494 + miR.518c + miR.639 + miR.660 | 0,8 | 0,313470786 |
| miR.558 + miR.609 + miR.603 + miR.520f + miR.130a + miR.29a + miR.376a* + miR.494 + miR.518c + miR.639 | 0,8 | 0,008372877 |
| miR.558 + miR.609 + miR.520f + miR.220a + miR.26a + miR.29a + miR.376a* + miR.518c + miR.639 + miR.338 | 0,8 | 0,059321615 |
| miR.558 + miR.609 + miR.603 + miR.520f + miR.29a + miR.362 + miR.376a* + miR.518c + miR.639 + miR.660 | 0,8 | 0,030320104 |
| miR.558 + miR.609 + miR.220a + miR.519b + miR.29a + miR.369.3p + miR.376a* + miR.494 + miR.518c + miR.639 | 0,8 | 0,321233184 |
| miR.558 + miR.609 + miR.220a + miR.26a + miR.29a + miR.362 + miR.376a* + miR.494 + miR.518c + miR.660 | 0,8 | 0,130349807 |
| miR.558 + miR.609 + miR.130a + miR.26a + miR.29a + miR.369.3p + miR.376a* + miR.518c + miR.639 + miR.660 | 0,8 | 0,09332473 |
| miR.558 + miR.609 + miR.603 + miR.520f + miR.26a + miR.29a + miR.376a* + miR.518c + miR.639 + miR.660 | 0,8 | 0,018658566 |
| miR.558 + miR.609 + miR.603 + miR.220a + miR.29a + miR.362 + miR.376a* + miR.494 + miR.518c + miR.660 | 0,8 | 0,010783662 |
| miR.558 + miR.609 + miR.520f + miR.29a + miR.362 + miR.369.3p + miR.376a* + miR.494 + miR.518c + miR.639 | 0,8 | 0,064170212 |
| miR.558 + miR.609 + miR.603 + miR.520f + miR.26a + miR.29a + miR.376a* + miR.494 + miR.518c + miR.639 | 0,8 | 0,005965356 |
| miR.558 + miR.609 + miR.520f + miR.519b + miR.29a + miR.369.3p + miR.376a* + miR.494 + miR.518c + miR.660 | 0,8 | 0,101150324 |
| miR.558 + miR.609 + miR.520f + miR.220a + miR.519b + miR.29a + miR.376a* + miR.494 + miR.518c + miR.639 | 0,8 | 0,047708118 |
| miR.558 + miR.609 + miR.520f + miR.220a + miR.519b + miR.26a + miR.29a + miR.376a* + miR.494 + miR.518c | 0,8 | 0,067717943 |
| miR.558 + miR.609 + miR.603 + miR.520f + miR.519b + miR.130a + miR.29a + miR.376a* + miR.494 + miR.518c | 0,8 | 0,037239584 |
| miR.558 + miR.609 + miR.603 + miR.520f + miR.519b + miR.29a + miR.376a* + miR.518c + miR.660 + miR.338 | 0,8 | 0,250456953 |
| miR.558 + miR.609 + miR.603 + miR.520f + miR.130a + miR.29a + miR.376a* + miR.518c + miR.639 + miR.660 | 0,8 | 0,027696717 |
| miR.558 + miR.609 + miR.603 + miR.520f + miR.29a + miR.362 + miR.376a* + miR.518c + miR.660 + miR.338 | 0,8 | 0,297239077 |
| miR.558 + miR.609 + miR.603 + miR.520f + miR.26a + miR.29a + miR.376a* + miR.518c + miR.660 + miR.338 | 0,8 | 0,296430343 |
| miR.558 + miR.609 + miR.220a + miR.519b + miR.26a + miR.29a + miR.376a* + miR.494 + miR.518c + miR.639 | 0,8 | 0,081806724 |
| miR.558 + miR.609 + miR.603 + miR.520f + miR.519b + miR.26a + miR.29a + miR.376a* + miR.494 + miR.518c | 0,8 | 0,021534962 |
| miR.558 + miR.609 + miR.520f + miR.519b + miR.26a + miR.29a + miR.376a* + miR.518c + miR.660 + miR.338 | 0,8 | 0,314668653 |
| miR.558 + miR.609 + miR.603 + miR.520f + miR.519b + miR.29a + miR.376a* + miR.518c + miR.639 + miR.660 | 0,8 | 0,024442943 |
| miR.558 + miR.609 + miR.520f + miR.26a + miR.29a + miR.362 + miR.369.3p + miR.376a* + miR.518c + miR.660 | 0,8 | 0,161361511 |
| miR.558 + miR.609 + miR.603 + miR.520f + miR.519b + miR.29a + miR.376a* + miR.494 + miR.518c + miR.660 | 0,8 | 0,022472817 |
| miR.609 + miR.603 + miR.220a + miR.29a + miR.362 + miR.369.3p + miR.376a* + miR.518c + miR.660 + miR.338 | 0,8 | 0,418900132 |
| miR.558 + miR.609 + miR.220a + miR.130a + miR.26a + miR.29a + miR.376a* + miR.494 + miR.518c + miR.338 | 0,8 | 0,079389399 |
| miR.558 + miR.609 + miR.220a + miR.519b + miR.26a + miR.29a + miR.362 + miR.376a* + miR.518c + miR.639 | 0,8 | 0,182886545 |
| miR.558 + miR.609 + miR.220a + miR.130a + miR.26a + miR.29a + miR.376a* + miR.494 + miR.518c + miR.639 | 0,8 | 0,048278412 |
| miR.558 + miR.609 + miR.220a + miR.26a + miR.29a + miR.376a* + miR.494 + miR.518c + miR.639 + miR.338 | 0,8 | 0,096115174 |
| miR.558 + miR.609 + miR.520f + miR.519b + miR.29a + miR.362 + miR.369.3p + miR.376a* + miR.518c + miR.660 | 0,8 | 0,106707357 |
| miR.609 + miR.603 + miR.220a + miR.519b + miR.130a + miR.29a + miR.369.3p + miR.376a* + miR.494 + miR.518c | 0,8 | 0,361619292 |
| miR.558 + miR.609 + miR.603 + miR.220a + miR.519b + miR.29a + miR.376a* + miR.494 + miR.518c + miR.338 | 0,8 | 0,040619312 |
| miR.558 + miR.609 + miR.220a + miR.519b + miR.26a + miR.29a + miR.362 + miR.376a* + miR.518c + miR.338 | 0,8 | 0,225550488 |
| miR.558 + miR.609 + miR.520f + miR.220a + miR.519b + miR.29a + miR.376a* + miR.518c + miR.639 + miR.338 | 0,8 | 0,104143064 |
| miR.558 + miR.609 + miR.520f + miR.519b + miR.29a + miR.369.3p + miR.376a* + miR.494 + miR.518c + miR.639 | 0,8 | 0,084569552 |
| miR.558 + miR.609 + miR.220a + miR.26a + miR.29a + miR.362 + miR.376a* + miR.518c + miR.639 + miR.338 | 0,8 | 0,226878972 |
| miR.558 + miR.609 + miR.520f + miR.220a + miR.130a + miR.29a + miR.376a* + miR.518c + miR.639 + miR.338 | 0,8 | 0,076866019 |
| miR.558 + miR.609 + miR.520f + miR.220a + miR.519b + miR.130a + miR.29a + miR.376a* + miR.518c + miR.338 | 0,8 | 0,128513794 |
| miR.558 + miR.609 + miR.520f + miR.220a + miR.26a + miR.29a + miR.376a* + miR.494 + miR.518c + miR.639 | 0,8 | 0,019413714 |
| miR.558 + miR.609 + miR.603 + miR.130a + miR.29a + miR.369.3p + miR.376a* + miR.494 + miR.518c + miR.338 | 0,8 | 0,094248495 |
| miR.558 + miR.609 + miR.520f + miR.29a + miR.362 + miR.369.3p + miR.376a* + miR.494 + miR.660 + miR.338 | 0,8 | 0,454067465 |
| miR.558 + miR.609 + miR.520f + miR.220a + miR.130a + miR.26a + miR.29a + miR.376a* + miR.518c + miR.338 | 0,8 | 0,11857397 |
| miR.558 + miR.609 + miR.520f + miR.519b + miR.29a + miR.362 + miR.376a* + miR.518c + miR.660 + miR.338 | 0,8 | 0,267168221 |
| miR.558 + miR.609 + miR.603 + miR.519b + miR.29a + miR.369.3p + miR.376a* + miR.518c + miR.639 + miR.338 | 0,8 | 0,135096255 |
| miR.609 + miR.603 + miR.220a + miR.26a + miR.29a + miR.362 + miR.369.3p + miR.376a* + miR.518c + miR.660 | 0,8 | 0,449112306 |
| miR.558 + miR.609 + miR.520f + miR.519b + miR.130a + miR.29a + miR.376a* + miR.518c + miR.639 + miR.660 | 0,8 | 0,035307844 |
| miR.558 + miR.609 + miR.520f + miR.130a + miR.26a + miR.29a + miR.376a* + miR.518c + miR.639 + miR.660 | 0,8 | 0,035307844 |
| miR.558 + miR.609 + miR.603 + miR.519b + miR.29a + miR.362 + miR.369.3p + miR.376a* + miR.518c + miR.660 | 0,8 | 0,113283862 |
| miR.558 + miR.609 + miR.520f + miR.130a + miR.29a + miR.362 + miR.376a* + miR.518c + miR.639 + miR.660 | 0,8 | 0,035307844 |
| miR.558 + miR.609 + miR.220a + miR.26a + miR.29a + miR.376a* + miR.518c + miR.639 + miR.660 + miR.338 | 0,8 | 0,178404706 |
| miR.558 + miR.609 + miR.220a + miR.130a + miR.29a + miR.376a* + miR.494 + miR.518c + miR.639 + miR.660 | 0,8 | 0,061816912 |
| miR.558 + miR.609 + miR.520f + miR.519b + miR.130a + miR.29a + miR.376a* + miR.494 + miR.518c + miR.338 | 0,8 | 0,119518264 |
| miR.558 + miR.609 + miR.520f + miR.26a + miR.29a + miR.362 + miR.376a* + miR.518c + miR.639 + miR.338 | 0,8 | 0,118487619 |
| miR.558 + miR.609 + miR.520f + miR.220a + miR.519b + miR.26a + miR.29a + miR.376a* + miR.518c + miR.338 | 0,8 | 0,105949407 |
| miR.558 + miR.609 + miR.520f + miR.26a + miR.29a + miR.362 + miR.369.3p + miR.376a* + miR.518c + miR.639 | 0,8 | 0,117792797 |
| miR.558 + miR.609 + miR.603 + miR.520f + miR.130a + miR.26a + miR.29a + miR.376a*+miR.518c+miR.338 | 0,8 | 0,042590319 |
| miR.558 + miR.609 + miR.603 + miR.220a + miR.130a + miR.29a + miR.376a* + miR.494 + miR.518c + miR.338 | 0,8 | 0,000354017 |
| miR.558 + miR.609 + miR.519b + miR.130a + miR.26a + miR.29a + miR.369.3p + miR.376a* + miR.518c + miR.639 | 0,8 | 0,102098744 |
| miR.558 + miR.609 + miR.520f + miR.220a + miR.130a + miR.29a + miR.376a* + miR.494 + miR.518c + miR.639 | 0,8 | 0,030243424 |
| miR.558 + miR.609 + miR.220a + miR.130a + miR.26a + miR.29a + miR.362 + miR.376a* + miR.518c + miR.338 | 0,8 | 0,234142517 |
| miR.558 + miR.609 + miR.220a + miR.26a + miR.29a + miR.362 + miR.376a* + miR.518c + miR.639 + miR.660 | 0,8 | 0,101833869 |
| miR.558 + miR.609 + miR.220a + miR.519b + miR.29a + miR.362 + miR.369.3p + miR.376a* + miR.518c + miR.639 | 0,8 | 0,338633389 |
| miR.558 + miR.609 + miR.130a + miR.26a + miR.29a + miR.369.3p + miR.376a* + miR.518c + miR.639 + miR.338 | 0,8 | 0,165168227 |
| miR.558 + miR.609 + miR.603 + miR.29a + miR.369.3p + miR.376a* + miR.518c + miR.639 + miR.660 + miR.338 | 0,8 | 0,124131098 |
| miR.558 + miR.609 + miR.520f + miR.519b + miR.26a + miR.29a + miR.369.3p + miR.376a* + miR.518c + miR.639 | 0,8 | 0,119651463 |
| miR.558 + miR.609 + miR.603 + miR.130a + miR.29a + miR.369.3p + miR.376a* + miR.494 + miR.518c + miR.660 | 0,8 | 0,044916097 |
| miR.558 + miR.609 + miR.520f + miR.26a + miR.29a + miR.376a* + miR.494 + miR.518c + miR.639 + miR.338 | 0,8 | 0,050187896 |
| miR.558 + miR.609 + miR.220a + miR.130a + miR.26a + miR.29a + miR.362 + miR.376a* + miR.518c + miR.639 | 0,8 | 0,176800046 |
| miR.558 + miR.609 + miR.220a + miR.519b + miR.130a + miR.26a + miR.29a + miR.362 + miR.376a* + miR.518c | 0,8 | 0,182652059 |
| miR.558 + miR.609 + miR.520f + miR.519b + miR.130a + miR.29a + miR.376a* + miR.494 + miR.518c + miR.660 | 0,8 | 0,043934059 |
| miR.558 + miR.609 + miR.603 + miR.130a + miR.29a + miR.362 + miR.369.3p + miR.376a* + miR.518c + miR.660 | 0,8 | 0,17812419 |
| miR.609 + miR.603 + miR.520f + miR.220a + miR.519b + miR.29a + miR.369.3p + miR.376a* + miR.518c + miR.660 | 0,8 | 0,388360738 |
| miR.558 + miR.609 + miR.520f + miR.29a + miR.362 + miR.376a* + miR.494 + miR.518c + miR.639 + miR.660 | 0,8 | 0,031409716 |
| miR.558 + miR.609 + miR.520f + miR.519b + miR.29a + miR.376a* + miR.494 + miR.518c + miR.639 + miR.338 | 0,8 | 0,08377831 |
| miR.558 + miR.609 + miR.520f + miR.220a + miR.130a + miR.26a + miR.29a + miR.376a* + miR.494 + miR.518c | 0,8 | 0,033215535 |
| miR.558 + miR.609 + miR.603 + miR.520f + miR.130a + miR.29a + miR.362 + miR.376a* + miR.494 + miR.518c | 0,8 | 0,012219591 |
| miR.558 + miR.609 + miR.520f + miR.130a + miR.29a + miR.362 + miR.376a* + miR.518c + miR.639 + miR.338 | 0,8 | 0,107574466 |
| miR.558 + miR.609 + miR.520f + miR.519b + miR.29a + miR.362 + miR.376a* + miR.518c + miR.639 + miR.338 | 0,8 | 0,17090723 |
| miR.609 + miR.603 + miR.520f + miR.220a + miR.26a + miR.29a + miR.369.3p + miR.376a* + miR.518c + miR.660 | 0,8 | 0,323412626 |
| miR.558 + miR.609 + miR.603 + miR.520f + miR.519b + miR.130a + miR.29a + miR.376a* + miR.518c + miR.338 | 0,8 | 0,097680191 |
| miR.558 + miR.609 + miR.220a + miR.519b + miR.26a + miR.29a + miR.376a* + miR.518c + miR.639 + miR.660 | 0,8 | 0,096384101 |
| miR.558 + miR.609 + miR.519b + miR.26a + miR.29a + miR.369.3p + miR.376a* + miR.518c + miR.639 + miR.338 | 0,8 | 0,262497855 |
| miR.558 + miR.609 + miR.603 + miR.520f + miR.26a + miR.29a + miR.362 + miR.376a* + miR.494 + miR.518c | 0,8 | 0,010450007 |
| miR.558 + miR.609 + miR.520f + miR.130a + miR.29a + miR.362 + miR.376a* + miR.494 + miR.518c + miR.660 | 0,8 | 0,029097308 |
| miR.558 + miR.609 + miR.603 + miR.29a + miR.369.3p + miR.376a* + miR.494 + miR.518c + miR.639 + miR.338 | 0,8 | 0,06745849 |
| miR.609 + miR.603 + miR.220a + miR.130a + miR.29a + miR.369.3p + miR.376a* + miR.494 + miR.518c + miR.660 | 0,8 | 0,283883726 |
| miR.558 + miR.609 + miR.603 + miR.520f + miR.29a + miR.362 + miR.376a* + miR.494 + miR.518c + miR.660 | 0,8 | 0,017728004 |
| miR.558 + miR.609 + miR.26a + miR.29a + miR.362 + miR.369.3p + miR.376a* + miR.518c + miR.639 + miR.660 | 0,8 | 0,273769934 |
| miR.558 + miR.609 + miR.603 + miR.520f + miR.130a + miR.26a + miR.29a + miR.376a* + miR.518c + miR.639 | 0,8 | 0,002350801 |
| miR.558 + miR.609 + miR.220a + miR.519b + miR.26a + miR.29a + miR.376a* + miR.518c + miR.660 + miR.338 | 0,8 | 0,246318434 |
| miR.558 + miR.609 + miR.520f + miR.519b + miR.130a + miR.29a + miR.376a^{*} + miR.494 + miR.518c + miR.639 | 0,8 | 0,056916651 |
| miR.558 + miR.609 + miR.520f + miR.26a + miR.29a + miR.376a* + miR.494 + miR.518c + miR.639 + miR.660 | 0,8 | 0,040606025 |
| miR.558 + miR.609 + miR.220a + miR.26a + miR.29a + miR.362 + miR.376a* + miR.518c + miR.660 + miR.338 | 0,8 | 0,255488044 |
| miR.558 + miR.609 + miR.220a + miR.519b + miR.26a + miR.29a + miR.362 + miR.376a* + miR.518c + miR.660 | 0,8 | 0,233109969 |
| miR.558 + miR.609 + miR.220a + miR.130a + miR.26a + miR.29a + miR.376a* + miR.518c + miR.639 + miR.660 | 0,8 | 0,110347189 |
| miR.558 + miR.609 + miR.520f + miR.130a + miR.26a + miR.29a + miR.376a* + miR.494 + miR.518c + miR.660 | 0,8 | 0,027243459 |
| miR.558 + miR.609 + miR.220a + miR.130a + miR.26a + miR.29a + miR.376a* + miR.518c + miR.660 + miR.338 | 0,8 | 0,241113713 |
| miR.558 + miR.609 + miR.603 + miR.520f + miR.130a + miR.29a + miR.376a* + miR.494 + miR.518c + miR.660 | 0,8 | 0,014144873 |
| miR.558 + miR.609 + miR.220a + miR.519b + miR.29a + miR.362 + miR.369.3p + miR.376a* + miR.518c + miR.338 | 0,8 | 0,296740788 |
| miR.558 + miR.609 + miR.130a + miR.29a + miR.362 + miR.369.3p + miR.376a* + miR.494 + miR.518c + miR.660 | 0,8 | 0,194761578 |
| miR.558 + miR.609 + miR.520f + miR.220a + miR.130a + miR.26a + miR.29a + miR.376a* + miR.518c + miR.639 | 0,8 | 0,01661889 |
| miR.558 + miR.609 + miR.220a + miR.519b + miR.29a + miR.369.3p + miR.376a* + miR.518c + miR.639 + miR.338 | 0,8 | 0,30150057 |
| miR.558 + miR.609 + miR.520f + miR.519b + miR.130a + miR.29a + miR.362 + miR.376a* + miR.494 + miR.518c | 0,8 | 0,075560534 |
| miR.558 + miR.609 + miR.603 + miR.520f + miR.26a + miR.29a + miR.376a* + miR.494 + miR.518c + miR.660 | 0,8 | 0,015720296 |
| miR.558 + miR.609 + miR.520f + miR.130a + miR.29a + miR.362 + miR.369.3p + miR.376a* + miR.518c + miR.639 | 0,8 | 0,067265192 |
| miR.558 + miR.609 + miR.520f + miR.519b + miR.29a + miR.362 + miR.376a* + miR.494 + miR.518c + miR.338 | 0,8 | 0,262933746 |
| miR.558 + miR.609 + miR.520f + miR.519b + miR.29a + miR.362 + miR.369.3p + miR.376a* + miR.518c + miR.639 | 0,8 | 0,072646365 |
| miR.558 + miR.609 + miR.26a + miR.29a + miR.362 + miR.369.3p + miR.376a* + miR.494 + miR.518c + miR.660 | 0,8 | 0,233858196 |
| miR.609 + miR.603 + miR.220a + miR.26a + miR.29a + miR.369.3p + miR.376a* + miR.518c + miR.639 + miR.660 | 0,8 | 0,351462549 |
| miR.609 + miR.603 + miR.220a + miR.519b + miR.29a + miR.362 + miR.369.3p + miR.376a* + miR.518c + miR.660 | 0,8 | 0,408604619 |
| miR.558 + miR.609 + miR.603 + miR.520f + miR.519b + miR.26a + miR.29a + miR.376a* + miR.518c + miR.639 | 0,8 | 0,007457304 |
| miR.558 + miR.609 + miR.220a + miR.130a + miR.26a + miR.29a + miR.362 + miR.376a* + miR.518c+miR.660 | 0,8 | 0,176968562 |
| miR.558 + miR.609 + miR.520f + miR.220a + miR.519b + miR.26a + miR.29a + miR.376a*+miR.518c+miR.639 | 0,8 | 0,041621787 |
| miR.558 + miR.609 + miR.520f + miR.130a + miR.26a + miR.29a + miR.376a* + miR.494 + miR.518c + miR.639 | 0,8 | 0,036583587 |

**Table 11: clusters of 11 miRNAs**

| **Cluster of 11 miRs** | **c_tau level** | **pv compared to top signature miR.558+ miR.609+ miR.520f+ miR.29a+ miR.362+ miR.369.3p + miR.376a* + miR.518c + miR.639+ miR.660+ miR.338** |
|---|---|---|
| miR.558 + miR.609 + miR.520f + miR.29a + miR.362 + miR.369.3p + miR.376a* + miR.518c + miR.639 + miR.660 + miR.338 | 0,81 | NaN |
| miR.558 + miR.609 + miR.603 + miR.220a + miR.26a + miR.29a + miR.369.3p + miR.376a* + miR.518c + miR.660 + miR.338 | 0,81 | 0,157246016 |
| miR.558 + miR.609 + miR.603 + miR.220a + miR.26a + miR.29a + miR.369.3p + miR.376a* + miR.518c + miR.639 + miR.660 | 0,81 | 0,171856579 |
| miR.558 + miR.609 + miR.520f + miR.220a + miR.29a + miR.362 + miR.369.3p + miR.376a* + miR.518c + miR.660 + miR.338 | 0,81 | 0,496362599 |
| miR.558 + miR.609 + miR.603 + miR.220a + miR.26a + miR.29a + miR.369.3p + miR.376a* + miR.494 + miR.518c + miR.660 | 0,81 | 0,146004635 |
| miR.558 + miR.609 + miR.603 + miR.520f + miR.220a + miR.29a + miR.369.3p + miR.376a* + miR.518c + miR.660 + miR.338 | 0,81 | 0,091948512 |
| miR.558 + miR.609 + miR.603 + miR.520f + miR.220a + miR.26a + miR.29a + miR.369.3p + miR.376a* + miR.518c + miR.660 | 0,81 | 0,181963139 |
| miR.558 + miR.609 + miR.603 + miR.220a + miR.130a + miR.26a + miR.29a + miR.369.3p + miR.376a* + miR.518c + miR.660 | 0,81 | 0,165405632 |
| miR.558 + miR.609 + miR.603 + miR.520f + miR.220a + miR.29a + miR.369.3p + miR.376a* + miR.518c + miR.639 + miR.660 | 0,81 | 0,092406785 |
| miR.558 + miR.609 + miR.603 + miR.220a + miR.26a + miR.29a + miR.362 + miR.369.3p + miR.376a* + miR.518c + miR.660 | 0,81 | 0,329353441 |
| miR.558 + miR.609 + miR.603 + miR.220a + miR.519b + miR.26a + miR.29a + miR.369.3p + miR.376a* + miR.518c + miR.660 | 0,81 | 0,213883456 |
| miR.558 + miR.609 + miR.603 + miR.520f + miR.220a + miR.29a + miR.362 + miR.369.3p + miR.376a* + miR.518c + miR.660 | 0,81 | 0,150894928 |
| miR.558 + miR.609 + miR.603 + miR.220a + miR.519b + miR.29a + miR.369.3p + miR.376a* + miR.494 + miR.518c + miR.660 | 0,81 | 0,16646344 |
| miR.558 + miR.609 + miR.603 + miR.220a + miR.26a + miR.29a + miR.369.3p + miR.376a* + miR.518c + miR.639 + miR.338 | 0,81 | 0,055438935 |
| miR.558 + miR.609 + miR.603 + miR.220a + miR.26a + miR.29a + miR.369.3p + miR.376a* + miR.494 + miR.518c + miR.338 | 0,81 | 0,067433109 |
| miR.558 + miR.609 + miR.603 + miR.220a + miR.29a + miR.362 + miR.369.3p + miR.376a* + miR.494 + miR.518c + miR.660 | 0,81 | 0,208951906 |
| miR.558 + miR.609 + miR.603 + miR.520f + miR.220a + miR.29a + miR.369.3p + miR.376a* + miR.494 + miR.518c + miR.660 | 0,81 | 0,094187974 |
| miR.558 + miR.609 + miR.603 + miR.220a + miR.519b + miR.26a + miR.29a + miR.369.3p + miR.376a* + miR.494 + miR.518c | 0,81 | 0,106889517 |
| miR.558 + miR.609 + miR.603 + miR.520f + miR.220a + miR.519b + miR.29a + miR.369.3p + miR.376a* + miR.518c + miR.660 | 0,81 | 0,167163633 |
| miR.558 + miR.609 + miR.520f + miR.26a + miR.29a + miR.362 + miR.369.3p + miR.376a* + miR.518c + miR.660 + miR.338 | 0,81 | 0,274816579 |
| miR.558 + miR.609 + miR.603 + miR.220a + miR.26a + miR.29a + miR.362 + miR.369.3p + miR.376a* + miR.518c + miR.338 | 0,81 | 0,085174637 |
| miR.558 + miR.609 + miR.603 + miR.520f + miR.29a + miR.362 + miR.369.3p + miR.376a* + miR.518c + miR.660 + miR.338 | 0,81 | 0,199200667 |
| miR.558 + miR.609 + miR.520f + miR.220a + miR.29a + miR.369.3p + miR.376a* + miR.518c + miR.639 + miR.660 + miR.338 | 0,81 | 0,121073665 |
| miR.558 + miR.609 + miR.603 + miR.520f + miR.29a + miR.369.3p + miR.376a* + miR.518c + miR.639 + miR.660 + miR.338 | 0,81 | 0,010508988 |
| miR.558 + miR.609 + miR.603 + miR.220a + miR.130a + miR.29a + miR.362 + miR.369.3p + miR.376a* + miR.518c + miR.660 | 0,81 | 0,254433993 |
| miR.558 + miR.609 + miR.603 + miR.220a + miR.29a + miR.362 + miR.369.3p + miR.376a* + miR.518c + miR.639 + miR.660 | 0,81 | 0,191974648 |
| miR.558 + miR.609 + miR.603 + miR.520f + miR.220a + miR.26a + miR.29a + miR.369.3p + miR.376a* + miR.518c + miR.338 | 0,81 | 0,059765494 |
| miR.558 + miR.609 + miR.603 + miR.220a + miR.29a + miR.362 + miR.369.3p + miR.376a* + miR.518c + miR.660 + miR.338 | 0,81 | 0,411235756 |
| miR.558 + miR.609 + miR.603 + miR.220a + miR.130a + miR.26a + miR.29a + miR.369.3p + miR.376a* + miR.518c + miR.338 | 0,81 | 0,07159897 |
| miR.558 + miR.609 + miR.603 + miR.520f + miR.220a + miR.130a + miR.29a + miR.369.3p + miR.376a* + miR.518c + miR.660 | 0,81 | 0,098482424 |
| miR.558 + miR.609 + miR.603 + miR.220a + miR.519b + miR.130a + miR.29a + miR.369.3p + miR.376a* + miR.494 + miR.518c | 0,81 | 0,128718078 |
| miR.558 + miR.609 + miR.603 + miR.220a + miR.26a + miR.29a + miR.362 + miR.369.3p + miR.376a* + miR.518c + miR.639 | 0,81 | 0,080417628 |
| miR.558 + miR.609 + miR.603 + miR.220a + miR.26a + miR.29a + miR.362 + miR.369.3p + miR.376a* + miR.494 + miR.518c | 0,81 | 0,05140153 |
| miR.558 + miR.609 + miR.603 + miR.220a + miR.519b + miR.29a + miR.362 + miR.369.3p + miR.376a* + miR.518c + miR.660 | 0,81 | 0,227723131 |
| miR.558 + miR.609 + miR.603 + miR.220a + miR.519b + miR.130a + miR.29a + miR.369.3p + miR.376a* + miR.518c + miR.660 | 0,81 | 0,178842795 |
| miR.558 + miR.609 + miR.603 + miR.220a + miR.519b + miR.26a + miR.29a + miR.362 + miR.369.3p + miR.376a* + miR.518c | 0,81 | 0,13587915 |
| miR.558 + miR.609 + miR.603 + miR.220a + miR.519b + miR.130a + miR.29a + miR.362 + miR.376a* + miR.494 + miR.518c | 0,81 | 0,090644921 |
| miR.558 + miR.609 + miR.603 + miR.520f + miR.220a + miR.26a + miR.29a + miR.362 + miR.369.3p + miR.376a* + miR.518c | 0,81 | 0,066839667 |
| miR.558 + miR.609 + miR.520f + miR.220a + miR.29a + miR.369.3p + miR.376a* + miR.494 + miR.518c + miR.660 + miR.338 | 0,81 | 0,105045113 |
| miR.558 + miR.609 + miR.603 + miR.220a + miR.130a + miR.26a + miR.29a + miR.362 + miR.369.3p + miR.376a* + miR.518c | 0,81 | 0,066839667 |
| miR.558 + miR.609 + miR.603 + miR.220a + miR.26a + miR.29a + miR.369.3p + miR.376a* + miR.494 + miR.518c + miR.639 | 0,81 | 0,049902204 |
| miR.558 + miR.609 + miR.603 + miR.520f + miR.220a + miR.519b + miR.29a + miR.369.3p + miR.376a* + miR.494 + miR.518c | 0,81 | 0,089387076 |
| miR.558 + miR.609 + miR.603 + miR.220a + miR.130a + miR.26a + miR.29a + miR.369.3p + miR.376a* + miR.518c + miR.639 | 0,81 | 0,066355389 |
| miR.558 + miR.609 + miR.603 + miR.220a + miR.130a + miR.26a + miR.29a + miR.369.3p + miR.376a* + miR.494 + miR.518c | 0,81 | 0,045991693 |
| miR.558 + miR.609 + miR.520f + miR.29a + miR.362 + miR.369.3p + miR.376a* + miR.494 + miR.518c + miR.660 + miR.338 | 0,81 | 0,272054339 |
| miR.558 + miR.609 + miR.603 + miR.220a + miR.29a + miR.369.3p + miR.376a* + miR.494 + miR.518c + miR.639 + miR.660 | 0,81 | 0,131669372 |
| miR.558 + miR.609 + miR.603 + miR.520f + miR.220a + miR.26a + miR.29a + miR.369.3p + miR.376a* + miR.518c + miR.639 | 0,81 | 0,0583122 |
| miR.558 + miR.609 + miR.603 + miR.220a + miR.29a + miR.369.3p + miR.376a* + miR.518c + miR.639 + miR.660 + miR.338 | 0,81 | 0,18247775 |
| miR.558 + miR.609 + miR.603 + miR.220a + miR.519b + miR.26a + miR.29a + miR.369.3p + miR.376a* + miR.518c + miR.338 | 0,81 | 0,109488784 |
| miR.558 + miR.609 + miR.603 + miR.220a + miR.519b + miR.29a + miR.362 + miR.369.3p + miR.376a* + miR.494 + miR.518c | 0,81 | 0,079074586 |
| miR.558 + miR.609 + miR.603 + miR.520f + miR.220a + miR.29a + miR.362 + miR.369.3p + miR.376a* + miR.518c + miR.338 | 0,81 | 0,028631288 |
| miR.558 + miR.609 + miR.603 + miR.220a + miR.29a + miR.369.3p + miR.376a* + miR.494 + miR.518c + miR.660 + miR.338 | 0,81 | 0,235907465 |
| miR.558 + miR.609 + miR.603 + miR.220a + miR.130a + miR.29a + miR.369.3p + miR.376a* + miR.494 + miR.518c + miR.660 | 0,81 | 0,131461341 |
| miR.558 + miR.609 + miR.603 + miR.220a + miR.130a + miR.29a + miR.369.3p + miR.376a* + miR.518c + miR.639 + miR.660 | 0,81 | 0,147748075 |
| miR.558 + miR.609 + miR.603 + miR.520f + miR.220a + miR.26a + miR.29a + miR.369.3p + miR.376a* + miR.494 + miR.518c | 0,81 | 0,045216308 |
| miR.558 + miR.609 + miR.603 + miR.220a + miR.519b + miR.29a + miR.369.3p + miR.376a* + miR.518c + miR.639 + miR.660 | 0,81 | 0,220170147 |
| miR.558 + miR.609 + miR.603 + miR.220a + miR.519b + miR.29a + miR.369.3p + miR.376a* + miR.518c + miR.660 + miR.338 | 0,81 | 0,169875898 |
| miR.558 + miR.609 + miR.520f + miR.130a + miR.29a + miR.369.3p + miR.376a* + miR.518c + miR.639 + miR.660 + miR.338 | 0,81 | 0,128601523 |
| miR.558 + miR.609 + miR.220a + miR.26a + miR.29a + miR.362 + miR.369.3p + miR.376a* + miR.518c + miR.660 + miR.338 | 0,81 | 0,311444877 |
| miR.558 + miR.609 + miR.603 + miR.520f + miR.220a + miR.130a + miR.26a + miR.29a + miR.369.3p + miR.376a* + miR.518c | 0,81 | 0,066414344 |
| miR.558 + miR.609 + miR.520f + miR.220a + miR.26a + miR.29a + miR.369.3p + miR.376a* + miR.518c + miR.660 + miR.338 | 0,81 | 0,155513204 |
| miR.558 + miR.609 + miR.520f + miR.220a + miR.519b + miR.29a + miR.369.3p + miR.376a* + miR.518c + miR.660 + miR.338 | 0,81 | 0,214374038 |
| miR.558 + miR.609 + miR.520f + miR.220a + miR.130a + miR.29a + miR.369.3p + miR.376a* + miR.518c + miR.660 + miR.338 | 0,81 | 0,106900261 |
| miR.558 + miR.609 + miR.603 + miR.220a + miR.519b + miR.130a + miR.26a + miR.29a + miR.369.3p + miR.376a* + miR.518c | 0,81 | 0,119541359 |
| miR.558 + miR.609 + miR.603 + miR.520f + miR.220a + miR.519b + miR.29a + miR.369.3p + miR.376a* + miR.518c + miR.338 | 0,81 | 0,044448471 |
| miR.558 + miR.609 + miR.603 + miR.220a + miR.519b + miR.29a + miR.369.3p + miR.376a* + miR.494 + miR.518c + miR.639 | 0,81 | 0,080955667 |
| miR.558 + miR.609 + miR.603 + miR.520f + miR.220a + miR.519b + miR.29a + miR.362 + miR.376a* + miR.494 + miR.518c | 0,81 | 0,088147267 |
| miR.558 + miR.609 + miR.603 + miR.220a + miR.130a + miR.29a + miR.369.3p + miR.376a* + miR.518c + miR.660 + miR.338 | 0,81 | 0,210223613 |
| miR.558 + miR.609 + miR.603 + miR.220a + miR.519b + miR.130a + miR.29a + miR.362 + miR.369.3p + miR.376a* + miR.518c | 0,81 | 0,075058128 |
| miR.558 + miR.609 + miR.603 + miR.520f + miR.220a + miR.519b + miR.29a + miR.362 + miR.369.3p + miR.376a* + miR.518c | 0,81 | 0,116764211 |
| miR.558 + miR.609 + miR.603 + miR.520f + miR.220a + miR.29a + miR.362 + miR.369.3p + miR.376a* + miR.518c + miR.639 | 0,81 | 0,031559751 |
| miR.558 + miR.609 + miR.520f + miR.130a + miR.29a + miR.362 + miR.369.3p + miR.376a* + miR.518c + miR.660 + miR.338 | 0,81 | 0,300762925 |
| miR.558 + miR.609 + miR.520f + miR.220a + miR.29a + miR.369.3p + miR.376a* + miR.494 + miR.518c + miR.639 + miR.660 | 0,81 | 0,054662395 |
| miR.558 + miR.609 + miR.603 + miR.520f + miR.26a + miR.29a + miR.369.3p + miR.376a* + miR.518c + miR.639 + miR.338 | 0,81 | 0,029028283 |
| miR.558 + miR.609 + miR.520f + miR.220a + miR.26a + miR.29a + miR.369.3p + miR.376a* + miR.518c + miR.639 + miR.660 | 0,81 | 0,107383644 |
| miR.558 + miR.609 + miR.520f + miR.29a + miR.369.3p + miR.376a* + miR.494 + miR.518c + miR.639 + miR.660 + miR.338 | 0,81 | 0,081054444 |
| miR.558 + miR.609 + miR.603 + miR.520f + miR.220a + miR.29a + miR.362 + miR.369.3p + miR.376a* + miR.494 + miR.518c | 0,81 | 0,029649922 |
| miR.558 + miR.609 + miR.603 + miR.220a + miR.519b + miR.130a + miR.29a + miR.376a* + miR.494 + miR.518c + miR.660 | 0,81 | 0,080293447 |
| miR.558 + miR.609 + miR.220a + miR.26a + miR.29a + miR.369.3p + miR.376a* + miR.494 + miR.518c + miR.660 + miR.338 | 0,81 | 0,203556658 |
| miR.558 + miR.609 + miR.520f + miR.220a + miR.29a + miR.362 + miR.369.3p + miR.376a* + miR.518c + miR.639 + miR.660 | 0,81 | 0,119155844 |
| miR.558 + miR.609 + miR.603 + miR.520f + miR.220a + miR.130a + miR.29a + miR.362 + miR.369.3p + miR.376a* + miR.518c | 0,81 | 0,03916983 |
| miR.558 + miR.609 + miR.603 + miR.220a + miR.519b + miR.26a + miR.29a + miR.369.3p + miR.376a* + miR.518c + miR.639 | 0,81 | 0,118944722 |
| miR.558 + miR.609 + miR.603 + miR.520f + miR.220a + miR.519b + miR.26a + miR.29a + miR.369.3p + miR.376a* + miR.518c | 0,81 | 0,131184379 |
| miR.558 + miR.609 + miR.520f + miR.220a + miR.26a + miR.29a + miR.369.3p + miR.376a* + miR.494 + miR.518c + miR.660 | 0,81 | 0,152524201 |
| miR.558 + miR.609 + miR.603 + miR.220a + miR.519b + miR.130a + miR.29a + miR.369.3p + miR.376a* + miR.518c + miR.338 | 0,81 | 0,05479335 |
| miR.558 + miR.609 + miR.603 + miR.520f + miR.29a + miR.362 + miR.369.3p + miR.376a* + miR.518c + miR.639 + miR.338 | 0,81 | 0,023807176 |
| miR.558 + miR.609 + miR.520f + miR.519b + miR.29a + miR.362 + miR.369.3p + miR.376a* + miR.518c + miR.660 + miR.338 | 0,81 | 0,287009844 |
| miR.558 + miR.609 + miR.603 + miR.520f + miR.29a + miR.369.3p + miR.376a* + miR.494 + miR.518c + miR.660 + miR.338 | 0,81 | 0,022241593 |
| miR.558 + miR.609 + miR.603 + miR.220a + miR.519b + miR.29a + miR.362 + miR.369.3p + miR.376a* + miR.518c + miR.338 | 0,81 | 0,06891838 |
| miR.558 + miR.609 + miR.220a + miR.26a + miR.29a + miR.362 + miR.369.3p + miR.376a* + miR.494 + miR.518c + miR.660 | 0,81 | 0,202559842 |
| miR.558 + miR.609 + miR.603 + miR.26a + miR.29a + miR.362 + miR.369.3p + miR.376a* + miR.518c + miR.660 + miR.338 | 0,81 | 0,129538185 |
| miR.558 + miR.609 + miR.220a + miR.26a + miR.29a + miR.369.3p + miR.376a* + miR.518c + miR.639 + miR.660 + miR.338 | 0,81 | 0,206612529 |
| miR.558 + miR.609 + miR.520f + miR.220a + miR.29a + miR.362 + miR.369.3p + miR.376a* + miR.494 + miR.518c + miR.660 | 0,81 | 0,176200458 |
| miR.558 + miR.609 + miR.603 + miR.220a + miR.519b + miR.29a + miR.362 + miR.369.3p + miR.376a* + miR.518c + miR.639 | 0,81 | 0,078489254 |
| miR.558 + miR.609 + miR.520f + miR.220a + miR.26a + miR.29a + miR.362 + miR.369.3p + miR.376a* + miR.518c + miR.660 | 0,81 | 0,301062138 |
| miR.558 + miR.609 + miR.603 + miR.220a + miR.519b + miR.130a + miR.29a + miR.369.3p + miR.376a* + miR.518c + miR.639 | 0,81 | 0,067902456 |
| miR.558 + miR.609 + miR.603 + miR.220a + miR.519b + miR.130a + miR.26a + miR.29a + miR.376a* + miR.494 + miR.518c | 0,81 | 0,062014197 |
| miR.558 + miR.609 + miR.220a + miR.26a + miR.29a + miR.369.3p + miR.376a* + miR.494 + miR.518c + miR.639 + miR.660 | 0,81 | 0,21512478 |
| miR.558 + miR.609 + miR.520f + miR.220a + miR.130a + miR.29a + miR.369.3p + miR.376a* + miR.518c + miR.639 + miR.660 | 0,81 | 0,09004539 |
| miR.558 + miR.609 + miR.603 + miR.520f + miR.220a + miR.29a + miR.369.3p + miR.376a* + miR.518c + miR.639 + miR.338 | 0,81 | 0,015083939 |
| miR.558 + miR.609 + miR.520f + miR.220a + miR.130a + miR.29a + miR.369.3p + miR.376a* + miR.494 + miR.518c + miR.660 | 0,81 | 0,110624948 |
| miR.558 + miR.609 + miR.603 + miR.520f + miR.220a + miR.519b + miR.29a + miR.376a* + miR.494 + miR.518c + miR.660 | 0,81 | 0,095230232 |
| miR.558 + miR.609 + miR.603 + miR.220a + miR.519b + miR.26a + miR.29a + miR.362 + miR.376a* + miR.494 + miR.518c | 0,81 | 0,101333194 |
| miR.558 + miR.609 + miR.603 + miR.520f + miR.519b + miR.29a + miR.369.3p + miR.376a* + miR.494 + miR.518c + miR.338 | 0,81 | 0,031978532 |
| miR.558 + miR.609 + miR.603 + miR.220a + miR.130a + miR.29a + miR.362 + miR.369.3p + miR.376a* + miR.518c + miR.639 | 0,81 | 0,022672081 |
| miR.558 + miR.609 + miR.220a + miR.519b + miR.26a + miR.29a + miR.369.3p + miR.376a* + miR.494 + miR.518c + miR.660 | 0,81 | 0,294181385 |
| miR.558 + miR.609 + miR.603 + miR.520f + miR.519b + miR.29a + miR.369.3p + miR.376a* + miR.518c + miR.660 + miR.338 | 0,81 | 0,042711014 |
| miR.558 + miR.609 + miR.603 + miR.220a + miR.26a + miR.29a + miR.362 + miR.376a* + miR.518c + miR.639 + miR.338 | 0,81 | 0,061977272 |
| miR.558 + miR.609 + miR.603 + miR.520f + miR.130a + miR.29a + miR.369.3p + miR.376a* + miR.518c + miR.660 + miR.338 | 0,81 | 0,02204482 |
| miR.558 + miR.609 + miR.603 + miR.520f + miR.220a + miR.130a + miR.29a + miR.369.3p + miR.376a* + miR.518c + miR.639 | 0,81 | 0,023116393 |
| miR.558 + miR.609 + miR.603 + miR.520f + miR.130a + miR.29a + miR.369.3p + miR.376a* + miR.518c + miR.639 + miR.338 | 0,81 | 0,027568859 |
| miR.558 + miR.609 + miR.603 + miR.520f + miR.220a + miR.130a + miR.29a + miR.369.3p + miR.376a* + miR.494 + miR.518c | 0,81 | 0,024877954 |
| miR.558 + miR.609 + miR.520f + miR.26a + miR.29a + miR.369.3p + miR.376a* + miR.518c + miR.639 + miR.660 + miR.338 | 0,81 | 0,120221476 |
| miR.558 + miR.609 + miR.603 + miR.520f + miR.220a + miR.519b + miR.29a + miR.369.3p + miR.376a* + miR.518c + miR.639 | 0,81 | 0,074303321 |
| miR.558 + miR.609 + miR.603 + miR.520f + miR.29a + miR.369.3p + miR.376a* + miR.494 + miR.518c + miR.639 + miR.660 | 0,81 | 0,004682371 |
| miR.558 + miR.609 + miR.520f + miR.220a + miR.130a + miR.29a + miR.362 + miR.369.3p + miR.376a* + miR.518c + miR.660 | 0,81 | 0,235944522 |
| miR.558 + miR.609 + miR.520f + miR.130a + miR.29a + miR.369.3p + miR.376a* + miR.494 + miR.518c + miR.639 + miR.660 | 0,81 | 0,04833469 |
| miR.558 + miR.609 + miR.220a + miR.130a + miR.29a + miR.362 + miR.369.3p + miR.376a* + miR.494 + miR.518c + miR.660 | 0,81 | 0,172749789 |
| miR.558 + miR.609 + miR.520f + miR.519b + miR.29a + miR.369.3p + miR.376a* + miR.518c + miR.639 + miR.660 + miR.338 | 0,81 | 0,082595715 |
| miR.558 + miR.609 + miR.130a + miR.29a + miR.362 + miR.369.3p + miR.376a* + miR.494 + miR.518c + miR.639 + miR.660 | 0,81 | 0,116592883 |
| miR.558 + miR.609 + miR.603 + miR.26a + miR.29a + miR.369.3p + miR.376a* + miR.518c + miR.639 + miR.660 + miR.338 | 0,81 | 0,128889488 |
| miR.558 + miR.609 + miR.603 + miR.520f + miR.519b + miR.29a + miR.369.3p + miR.376a* + miR.518c + miR.639 + miR.338 | 0,81 | 0,039806387 |
| miR.558 + miR.609 + miR.520f + miR.220a + miR.519b + miR.29a + miR.369.3p + miR.376a* + miR.494 + miR.518c + miR.660 | 0,81 | 0,179763739 |
| miR.558 + miR.609 + miR.520f + miR.220a + miR.130a + miR.26a + miR.29a + miR.369.3p + miR.376a* + miR.518c + miR.660 | 0,81 | 0,191779006 |
| miR.558 + miR.609 + miR.603 + miR.220a + miR.519b + miR.26a + miR.29a + miR.376a* + miR.494 + miR.518c + miR.660 | 0,81 | 0,084003019 |
| miR.558 + miR.609 + miR.603 + miR.520f + miR.220a + miR.519b + miR.29a + miR.376a* + miR.494 + miR.518c + miR.338 | 0,81 | 0,027080309 |
| miR.558 + miR.609 + miR.603 + miR.130a + miR.26a + miR.29a + miR.369.3p + miR.376a* + miR.518c + miR.639 + miR.338 | 0,81 | 0,103986664 |
| miR.558 + miR.609 + miR.603 + miR.520f + miR.220a + miR.519b + miR.26a + miR.29a + miR.362 + miR.376a* + miR.518c | 0,81 | 0,107758422 |
| miR.558 + miR.609 + miR.220a + miR.519b + miR.26a + miR.29a + miR.369.3p + miR.376a* + miR.518c + miR.660 + miR.338 | 0,81 | 0,236412998 |
| miR.558 + miR.609 + miR.603 + miR.220a + miR.26a + miR.29a + miR.362 + miR.376a* + miR.494 + miR.518c + miR.639 | 0,81 | 0,046229114 |
| miR.558 + miR.609 + miR.603 + miR.220a + miR.130a + miR.29a + miR.369.3p + miR.376a* + miR.494 + miR.518c + miR.639 | 0,81 | 0,018181423 |
| miR.558 + miR.609 + miR.220a + miR.130a + miR.26a + miR.29a + miR.369.3p + miR.376a* + miR.518c + miR.660 + miR.338 | 0,81 | 0,180331179 |
| miR.558 + miR.609 + miR.603 + miR.520f + miR.26a + miR.29a + miR.369.3p + miR.376a* + miR.518c + miR.660 + miR.338 | 0,81 | 0,029370985 |
| miR.558 + miR.609 + miR.220a + miR.26a + miR.29a + miR.362 + miR.369.3p + miR.376a* + miR.518c + miR.639 + miR.660 | 0,81 | 0,287302613 |
| miR.558 + miR.609 + miR.603 + miR.520f + miR.26a + miR.29a + miR.369.3p + miR.376a* + miR.494 + miR.518c + miR.338 | 0,81 | 0,034711155 |
| miR.558 + miR.609 + miR.520f + miR.220a + miR.519b + miR.29a + miR.362 + miR.369.3p + miR.376a* + miR.518c + miR.660 | 0,81 | 0,277262363 |
| miR.558 + miR.609 + miR.220a + miR.130a + miR.26a + miR.29a + miR.369.3p + miR.376a* + miR.494 + miR.518c + miR.660 | 0,81 | 0,167285229 |
| miR.558 + miR.609 + miR.603 + miR.520f + miR.220a + miR.519b + miR.29a + miR.362 + miR.376a* + miR.518c + miR.338 | 0,81 | 0,061056014 |
| miR.558 + miR.609 + miR.520f + miR.220a + miR.519b + miR.29a + miR.369.3p + miR.376a* + miR.518c + miR.639 + miR.660 | 0,81 | 0,119596033 |
| miR.558 + miR.609 + miR.220a + miR.29a + miR.362 + miR.369.3p + miR.376a* + miR.518c + miR.639 + miR.660 + miR.338 | 0,81 | 0,260947722 |
| miR.558 + miR.609 + miR.603 + miR.520f + miR.220a + miR.29a + miR.369.3p + miR.376a* + miR.494 + miR.518c + miR.338 | 0,81 | 0,016688397 |
| miR.558 + miR.609 + miR.603 + miR.220a + miR.519b + miR.130a + miR.29a + miR.376a* + miR.494 + miR.518c + miR.338 | 0,81 | 0,046262483 |
| miR.558 + miR.609 + miR.603 + miR.520f + miR.220a + miR.519b + miR.130a + miR.29a + miR.369.3p + miR.376a* + miR.518c | 0,81 | 0,084016227 |
| miR.558 + miR.609 + miR.603 + miR.520f + miR.220a + miR.519b + miR.130a + miR.29a + miR.362 + miR.376a* + miR.518c | 0,81 | 0,115517656 |
| miR.558 + miR.609 + miR.603 + miR.220a + miR.130a + miR.29a + miR.362 + miR.369.3p + miR.376a* + miR.494 + miR.518c | 0,81 | 0,021317512 |
| miR.558 + miR.609 + miR.603 + miR.29a + miR.362 + miR.369.3p + miR.376a* + miR.518c + miR.639 + miR.660 + miR.338 | 0,81 | 0,061171041 |
| miR.558 + miR.609 + miR.603 + miR.220a + miR.519b + miR.26a + miR.29a + miR.362 + miR.376a* + miR.518c + miR.338 | 0,81 | 0,069080501 |
| miR.558 + miR.609 + miR.603 + miR.220a + miR.519b + miR.130a + miR.29a + miR.376a* + miR.494 + miR.518c + miR.639 | 0,81 | 0,052200567 |
| miR.558 + miR.609 + miR.603 + miR.520f + miR.220a + miR.519b + miR.26a + miR.29a + miR.376a* + miR.494 + miR.518c | 0,81 | 0,048373812 |
| miR.558 + miR.609 + miR.603 + miR.520f + miR.220a + miR.130a + miR.29a + miR.369.3p + miR.376a* + miR.518c + miR.338 | 0,81 | 0,020099179 |
| miR.558 + miR.609 + miR.603 + miR.520f + miR.220a + miR.29a + miR.376a* + miR.518c + miR.639 + miR.660 + miR.338 | 0,81 | 0,031295958 |
| miR.558 + miR.609 + miR.603 + miR.220a + miR.130a + miR.26a + miR.29a + miR.362 + miR.376a* + miR.494 + miR.518c | 0,81 | 0,052787732 |
| miR.558 + miR.609 + miR.220a + miR.519b + miR.26a + miR.29a + miR.369.3p + miR.376a* + miR.518c + miR.639 + miR.660 | 0,81 | 0,263389576 |
| miR.558 + miR.609 + miR.603 + miR.220a + miR.26a + miR.29a + miR.362 + miR.376a* + miR.494 + miR.518c + miR.338 | 0,81 | 0,04631865 |
| miR.558 + miR.609 + miR.520f + miR.220a + miR.519b + miR.26a + miR.29a + miR.369.3p + miR.376a* + miR.518c + miR.660 | 0,81 | 0,241232516 |
| miR.558 + miR.609 + miR.520f + miR.220a + miR.26a + miR.29a + miR.362 + miR.369.3p + miR.376a* + miR.518c + miR.338 | 0,81 | 0,048374898 |
| miR.558 + miR.609 + miR.220a + miR.130a + miR.29a + miR.362 + miR.369.3p + miR.376a* + miR.518c + miR.660 + miR.338 | 0,81 | 0,273236304 |
| miR.558 + miR.609 + miR.603 + miR.520f + miR.220a + miR.29a + miR.362 + miR.376a* + miR.518c + miR.639 + miR.338 | 0,81 | 0,020061474 |
| miR.558 + miR.609 + miR.603 + miR.520f + miR.220a + miR.519b + miR.130a + miR.29a + miR.376a* + miR.494 + miR.518c | 0,81 | 0,05988177 |
| miR.558 + miR.609 + miR.520f + miR.220a + miR.29a + miR.376a* + miR.494 + miR.518c + miR.639 + miR.660 + miR.338 | 0,81 | 0,028607081 |
| miR.558 + miR.609 + miR.603 + miR.520f + miR.220a + miR.29a + miR.376a* + miR.494 + miR.518c + miR.639 + miR.660 | 0,81 | 0,071271615 |
| miR.558 + miR.609 + miR.603 + miR.520f + miR.29a + miR.369.3p + miR.376a* + miR.494 + miR.518c + miR.639 + miR.338 | 0,81 | 0,014376236 |
| miR.558 + miR.609 + miR.220a + miR.29a + miR.362 + miR.369.3p + miR.376a* + miR.494 + miR.518c + miR.660 + miR.338 | 0,81 | 0,235462188 |
| miR.558 + miR.609 + miR.603 + miR.520f + miR.26a + miR.29a + miR.369.3p + miR.376a* + miR.518c + miR.639 + miR.660 | 0,81 | 0,012140255 |
| miR.558 + miR.609 + miR.520f + miR.220a + miR.26a + miR.29a + miR.369.3p + miR.376a* + miR.494 + miR.518c + miR.338 | 0,81 | 0,043902695 |
| miR.558 + miR.609 + miR.603 + miR.220a + miR.130a + miR.29a + miR.369.3p + miR.376a* + miR.494 + miR.518c + miR.338 | 0,81 | 0,025637666 |
| miR.558 + miR.609 + miR.603 + miR.520f + miR.220a + miR.26a + miR.29a + miR.362 + miR.376a* + miR.518c + miR.639 | 0,81 | 0,046912851 |
| miR.558 + miR.609 + miR.603 + miR.520f + miR.220a + miR.519b + miR.29a + miR.362 + miR.376a* + miR.518c + miR.660 | 0,81 | 0,121982862 |
| miR.558 + miR.609 + miR.603 + miR.220a + miR.26a + miR.29a + miR.376a* + miR.494 + miR.518c + miR.639 + miR.660 | 0,81 | 0,050429994 |
| miR.558 + miR.609 + miR.520f + miR.130a + miR.29a + miR.376a* + miR.494 + miR.518c + miR.639 + miR.660 + miR.338 | 0,81 | 0,031247547 |
| miR.558 + miR.609 + miR.603 + miR.520f + miR.220a + miR.29a + miR.376a* + miR.494 + miR.518c + miR.660 + miR.338 | 0,81 | 0,03715542 |
| miR.558 + miR.609 + miR.603 + miR.520f + miR.29a + miR.362 + miR.369.3p + miR.376a* + miR.518c + miR.639 + miR.660 | 0,81 | 0,002566694 |
| miR.558 + miR.609 + miR.220a + miR.519b + miR.26a + miR.29a + miR.362 + miR.369.3p + miR.376a* + miR.518c + miR.660 | 0,81 | 0,285636001 |
| miR.558 + miR.609 + miR.603 + miR.220a + miR.519b + miR.130a + miR.26a + miR.29a + miR.362 + miR.376a* + miR.518c | 0,81 | 0,090123865 |
| miR.558 + miR.609 + miR.603 + miR.520f + miR.130a + miR.26a + miR.29a + miR.369.3p + miR.376a* + miR.518c + miR.338 | 0,81 | 0,029075047 |
| miR.558 + miR.609 + miR.520f + miR.220a + miR.26a + miR.29a + miR.369.3p + miR.376a* + miR.518c + miR.639 + miR.338 | 0,81 | 0,038639888 |
| miR.558 + miR.609 + miR.603 + miR.220a + miR.519b + miR.26a + miR.29a + miR.376a* + miR.494 + miR.518c + miR.338 | 0,81 | 0,056499614 |
| miR.558 + miR.609 + miR.603 + miR.220a + miR.519b + miR.29a + miR.369.3p + miR.376a* + miR.494 + miR.518c + miR.338 | 0,81 | 0,063066277 |
| miR.558 + miR.609 + miR.603 + miR.220a + miR.519b + miR.26a + miR.29a + miR.362 + miR.376a* + miR.518c + miR.639 | 0,81 | 0,095366934 |
| miR.558 + miR.609 + miR.603 + miR.220a + miR.130a + miR.29a + miR.362 + miR.369.3p + miR.376a* + miR.518c + miR.338 | 0,81 | 0,021725703 |
| miR.558 + miR.609 + miR.520f + miR.519b + miR.29a + miR.369.3p + miR.376a* + miR.494 + miR.518c + miR.660 + miR.338 | 0,81 | 0,222321539 |
| miR.558 + miR.609 + miR.520f + miR.220a + miR.29a + miR.362 + miR.369.3p + miR.376a* + miR.518c + miR.639 + miR.338 | 0,81 | 0,016462533 |
| miR.558 + miR.609 + miR.603 + miR.520f + miR.130a + miR.29a + miR.369.3p + miR.376a* + miR.494 + miR.518c + miR.338 | 0,81 | 0,019295261 |
| miR.558 + miR.609 + miR.220a + miR.130a + miR.26a + miR.29a + miR.362 + miR.369.3p + miR.376a* + miR.518c + miR.660 | 0,81 | 0,304747856 |
| miR.558 + miR.609 + miR.603 + miR.520f + miR.220a + miR.519b + miR.29a + miR.376a* + miR.494 + miR.518c + miR.639 | 0,81 | 0,042315388 |
| miR.558 + miR.609 + miR.520f + miR.220a + miR.519b + miR.29a + miR.376a* + miR.494 + miR.518c + miR.660 + miR.338 | 0,81 | 0,165603354 |
| miR.558 + miR.609 + miR.603 + miR.520f + miR.29a + miR.362 + miR.369.3p + miR.376a* + miR.494 + miR.518c + miR.338 | 0,81 | 0,017937353 |
| miR.558 + miR.609 + miR.220a + miR.519b + miR.130a + miR.29a + miR.369.3p + miR.376a* + miR.494 + miR.518c + miR.660 | 0,81 | 0,227912696 |
| miR.558 + miR.609 + miR.520f + miR.29a + miR.362 + miR.376a* + miR.494 + miR.518c + miR.639 + miR.660 + miR.338 | 0,81 | 0,013107368 |
| miR.558 + miR.609 + miR.220a + miR.130a + miR.29a + miR.362 + miR.369.3p + miR.376a* + miR.518c + miR.639 + miR.660 | 0,81 | 0,277978719 |
| miR.558 + miR.609 + miR.603 + miR.520f + miR.220a + miR.519b + miR.29a + miR.362 + miR.376a* + miR.518c + miR.639 | 0,81 | 0,098132347 |
| miR.558 + miR.609 + miR.220a + miR.130a + miR.29a + miR.369.3p + miR.376a* + miR.518c + miR.639 + miR.660 + miR.338 | 0,81 | 0,165580534 |
| miR.558 + miR.609 + miR.603 + miR.26a + miR.29a + miR.362 + miR.369.3p + miR.376a* + miR.518c + miR.639 + miR.338 | 0,81 | 0,097560492 |
| miR.558 + miR.609 + miR.220a + miR.130a + miR.26a + miR.29a + miR.369.3p + miR.376a* + miR.518c + miR.639 + miR.660 | 0,81 | 0,269457271 |
| miR.558 + miR.609 + miR.520f + miR.220a + miR.29a + miR.362 + miR.369.3p + miR.376a* + miR.494 + miR.518c + miR.338 | 0,81 | 0,018148135 |
| miR.558 + miR.609 + miR.603 + miR.519b + miR.26a + miR.29a + miR.369.3p + miR.376a* + miR.518c + miR.639 + miR.338 | 0,81 | 0,077165642 |
| miR.558 + miR.609 + miR.603 + miR.26a + miR.29a + miR.369.3p + miR.376a* + miR.494 + miR.518c + miR.639 + miR.338 | 0,81 | 0,071608284 |
| miR.558 + miR.609 + miR.220a + miR.519b + miR.29a + miR.362 + miR.369.3p + miR.376a* + miR.518c + miR.660 + miR.338 | 0,81 | 0,269155184 |
| miR.558 + miR.609 + miR.520f + miR.220a + miR.519b + miR.130a + miR.29a + miR.369.3p + miR.376a* + miR.518c + miR.660 | 0,81 | 0,216868617 |
| miR.558 + miR.609 + miR.603 + miR.220a + miR.26a + miR.29a + miR.376a* + miR.494 + miR.518c + miR.660 + miR.338 | 0,81 | 0,060593535 |
| miR.558 + miR.609 + miR.603 + miR.520f + miR.220a + miR.26a + miR.29a + miR.376a* + miR.518c + miR.639 + miR.660 | 0,81 | 0,074448852 |
| miR.558 + miR.609 + miR.603 + miR.520f + miR.130a + miR.29a + miR.369.3p + miR.376a* + miR.518c + miR.639 + miR.660 | 0,81 | 0,019785352 |
| miR.558 + miR.609 + miR.603 + miR.520f + miR.220a + miR.519b + miR.29a + miR.376a* + miR.518c + miR.639 + miR.660 | 0,81 | 0,112686787 |
| miR.558 + miR.609 + miR.603 + miR.26a + miR.29a + miR.362 + miR.369.3p + miR.376a* + miR.518c + miR.639 + miR.660 | 0,81 | 0,039634756 |
| miR.558 + miR.609 + miR.603 + miR.520f + miR.26a + miR.29a + miR.362 + miR.369.3p + miR.376a* + miR.518c + miR.338 | 0,81 | 0,026101501 |
| miR.558 + miR.609 + miR.603 + miR.220a + miR.519b + miR.26a + miR.29a + miR.362 + miR.376a* + miR.518c + miR.660 | 0,81 | 0,113390363 |
| miR.558 + miR.609 + miR.603 + miR.520f + miR.29a + miR.376a* + miR.494 + miR.518c + miR.639 + miR.660 + miR.338 | 0,81 | 0,012312031 |
| miR.558 + miR.609 + miR.603 + miR.520f + miR.220a + miR.29a + miR.362 + miR.376a* + miR.494 + miR.518c + miR.639 | 0,81 | 0,018411513 |
| miR.558 + miR.609 + miR.520f + miR.220a + miR.29a + miR.369.3p + miR.376a* + miR.494 + miR.518c + miR.639 + miR.338 | 0,81 | 0,019031544 |
| miR.558 + miR.609 + miR.603 + miR.520f + miR.220a + miR.29a + miR.369.3p + miR.376a* + miR.494 + miR.518c + miR.639 | 0,81 | 0,009341948 |
| miR.558 + miR.609 + miR.603 + miR.220a + miR.519b + miR.26a + miR.29a + miR.376a* + miR.518c + miR.660 + miR.338 | 0,81 | 0,09326183 |
| miR.558 + miR.609 + miR.603 + miR.220a + miR.26a + miR.29a + miR.376a* + miR.494 + miR.518c + miR.639 + miR.338 | 0,81 | 0,017499963 |
| miR.558 + miR.609 + miR.603 + miR.520f + miR.130a + miR.29a + miR.362 + miR.369.3p + miR.376a* + miR.518c + miR.338 | 0,81 | 0,012000363 |
| miR.558 + miR.609 + miR.603 + miR.520f + miR.220a + miR.519b + miR.26a + miR.29a + miR.376a* + miR.518c + miR.660 | 0,81 | 0,137890968 |
| miR.558 + miR.609 + miR.603 + miR.520f + miR.519b + miR.130a + miR.29a + miR.369.3p + miR.376a* + miR.518c + miR.338 | 0,81 | 0,0306986 |
| miR.558 + miR.609 + miR.220a + miR.26a + miR.29a + miR.369.3p + miR.376a* + miR.494 + miR.518c + miR.639 + miR.338 | 0,81 | 0,057323871 |
| miR.558 + miR.609 + miR.603 + miR.220a + miR.130a + miR.26a + miR.29a + miR.376a* + miR.494 + miR.518c + miR.639 | 0,81 | 0,011173161 |
| miR.558 + miR.609 + miR.603 + miR.520f + miR.220a + miR.26a + miR.29a + miR.376a* + miR.494 + miR.518c + miR.660 | 0,81 | 0,051301049 |
| miR.558 + miR.609 + miR.603 + miR.520f + miR.220a + miR.130a + miR.29a + miR.376a* + miR.518c + miR.639 + miR.660 | 0,81 | 0,056009001 |
| miR.558 + miR.609 + miR.603 + miR.220a + miR.130a + miR.26a + miR.29a + miR.376a* + miR.494 + miR.518c + miR.660 | 0,81 | 0,05768124 |
| miR.558 + miR.609 + miR.520f + miR.220a + miR.130a + miR.26a + miR.29a + miR.369.3p + miR.376a* + miR.518c + miR.338 | 0,81 | 0,043360833 |
| miR.558 + miR.609 + miR.603 + miR.520f + miR.220a + miR.29a + miR.362 + miR.376a* + miR.494 + miR.518c + miR.338 | 0,81 | 0,016421895 |
| miR.558 + miR.609 + miR.603 + miR.220a + miR.26a + miR.29a + miR.362 + miR.376a* + miR.494 + miR.518c + miR.660 | 0,81 | 0,072490939 |
| miR.558 + miR.609 + miR.220a + miR.130a + miR.26a + miR.29a + miR.369.3p + miR.376a* + miR.518c + miR.639 + miR.338 | 0,81 | 0,065646277 |
| miR.558 + miR.609 + miR.603 + miR.220a + miR.519b + miR.26a + miR.29a + miR.376a* + miR.494 + miR.518c + miR.639 | 0,81 | 0,046523365 |
| miR.558 + miR.609 + miR.220a + miR.130a + miR.29a + miR.369.3p + miR.376a* + miR.494 + miR.518c + miR.639 + miR.660 | 0,81 | 0,111273576 |
| miR.558 + miR.609 + miR.603 + miR.220a + miR.519b + miR.29a + miR.362 + miR.376a* + miR.494 + miR.518c + miR.639 | 0,81 | 0,097485823 |
| miR.558 + miR.609 + miR.603 + miR.220a + miR.130a + miR.26a + miR.29a + miR.362 + miR.376a* + miR.518c + miR.639 | 0,81 | 0,052467669 |
| miR.558 + miR.609 + miR.603 + miR.520f + miR.519b + miR.26a + miR.29a + miR.369.3p + miR.376a* + miR.518c + miR.338 | 0,81 | 0,0378875 |
| miR.558 + miR.609 + miR.520f + miR.220a + miR.519b + miR.29a + miR.369.3p + miR.376a* + miR.494 + miR.518c + miR.338 | 0,81 | 0,089804686 |
| miR.558 + miR.609 + miR.603 + miR.220a + miR.130a + miR.26a + miR.29a + miR.362 + miR.376a* + miR.518c + miR.338 | 0,81 | 0,056385248 |
| miR.558 + miR.609 + miR.603 + miR.520f + miR.220a + miR.519b + miR.130a + miR.29a + miR.376a* + miR.518c + miR.660 | 0,81 | 0,132753572 |
| miR.558 + miR.609 + miR.603 + miR.130a + miR.26a + miR.29a + miR.369.3p + miR.376a* + miR.494 + miR.518c + miR.639 | 0,81 | 0,054725285 |
| miR.558 + miR.609 + miR.220a + miR.29a + miR.362 + miR.369.3p + miR.376a* + miR.494 + miR.518c + miR.639 + miR.660 | 0,81 | 0,153042349 |
| miR.558 + miR.609 + miR.220a + miR.519b + miR.26a + miR.29a + miR.369.3p + miR.376a* + miR.494 + miR.518c + miR.338 | 0,81 | 0,080942299 |
| miR.558 + miR.609 + miR.603 + miR.520f + miR.220a + miR.519b + miR.29a + miR.376a* + miR.518c + miR.660 + miR.338 | 0,81 | 0,060178915 |
| miR.558 + miR.609 + miR.603 + miR.26a + miR.29a + miR.369.3p + miR.376a* + miR.494 + miR.518c + miR.639 + miR.660 | 0,81 | 0,062862694 |
| miR.558 + miR.609 + miR.603 + miR.520f + miR.519b + miR.29a + miR.362 + miR.369.3p + miR.376a* + miR.518c + miR.338 | 0,81 | 0,038116876 |
| miR.558 + miR.609 + miR.520f + miR.220a + miR.130a + miR.29a + miR.362 + miR.369.3p + miR.376a* + miR.518c + miR.338 | 0,81 | 0,01928441 |
| miR.558 + miR.609 + miR.220a + miR.130a + miR.26a + miR.29a + miR.369.3p + miR.376a* + miR.494 + miR.518c + miR.338 | 0,81 | 0,069434014 |
| miR.558 + miR.609 + miR.520f + miR.26a + miR.29a + miR.369.3p + miR.376a* + miR.494 + miR.518c + miR.660 + miR.338 | 0,81 | 0,093282086 |
| miR.558 + miR.609 + miR.603 + miR.520f + miR.220a + miR.26a + miR.29a + miR.362 + miR.376a* + miR.518c + miR.660 | 0,81 | 0,075718044 |
| miR.558 + miR.609 + miR.603 + miR.520f + miR.220a + miR.26a + miR.29a + miR.362 + miR.376a* + miR.518c + miR.338 | 0,81 | 0,029713564 |
| miR.558 + miR.609 + miR.603 + miR.520f + miR.220a + miR.130a + miR.29a + miR.376a* + miR.518c + miR.660 + miR.338 | 0,81 | 0,041172667 |
| miR.558 + miR.609 + miR.520f + miR.220a + miR.26a + miR.29a + miR.362 + miR.369.3p + miR.376a* + miR.494 + miR.518c | 0,81 | 0,051887657 |
| miR.558 + miR.609 + miR.603 + miR.520f + miR.26a + miR.29a + miR.369.3p + miR.376a* + miR.494 + miR.518c + miR.639 | 0,81 | 0,019119189 |
| miR.558 + miR.609 + miR.520f + miR.130a + miR.29a + miR.369.3p + miR.376a* + miR.494 + miR.518c + miR.660 + miR.338 | 0,81 | 0,092761698 |
| miR.558 + miR.609 + miR.603 + miR.130a + miR.29a + miR.362 + miR.369.3p + miR.376a* + miR.518c + miR.639 + miR.660 | 0,81 | 0,107461489 |
| miR.558 + miR.609 + miR.603 + miR.220a + miR.26a + miR.29a + miR.376a* + miR.518c + miR.639 + miR.660 + miR.338 | 0,81 | 0,079419293 |
| miR.558 + miR.609 + miR.603 + miR.220a + miR.519b + miR.130a + miR.26a + miR.29a + miR.376a* + miR.518c + miR.338 | 0,81 | 0,0313775 |
| miR.558 + miR.609 + miR.603 + miR.220a + miR.519b + miR.29a + miR.362 + miR.376a* + miR.494 + miR.518c + miR.660 | 0,81 | 0,100913603 |
| miR.558 + miR.609 + miR.603 + miR.520f + miR.220a + miR.130a + miR.29a + miR.376a* + miR.494 + miR.518c + miR.660 | 0,81 | 0,049787804 |
| miR.558 + miR.609 + miR.603 + miR.220a + miR.130a + miR.29a + miR.369.3p + miR.376a* + miR.518c + miR.639 + miR.338 | 0,81 | 0,027100903 |
| miR.558 + miR.609 + miR.220a + miR.26a + miR.29a + miR.362 + miR.369.3p + miR.376a* + miR.494 + miR.518c + miR.338 | 0,81 | 0,048225115 |
| miR.558 + miR.609 + miR.603 + miR.220a + miR.130a + miR.26a + miR.29a + miR.376a* + miR.494 + miR.518c + miR.338 | 0,81 | 0,019214948 |
| miR.558 + miR.609 + miR.603 + miR.220a + miR.519b + miR.130a + miR.26a + miR.29a + miR.376a* + miR.518c + miR.660 | 0,81 | 0,118081012 |
| miR.558 + miR.609 + miR.603 + miR.520f + miR.220a + miR.29a + miR.362 + miR.376a* + miR.494 + miR.518c + miR.660 | 0,81 | 0,042256704 |
| miR.558 + miR.609 + miR.603 + miR.220a + miR.519b + miR.26a + miR.29a + miR.376a* + miR.518c + miR.639 + miR.660 | 0,81 | 0,11382439 |
| miR.558 + miR.609 + miR.603 + miR.220a + miR.130a + miR.26a + miR.29a + miR.376a* + miR.518c + miR.639 + miR.660 | 0,81 | 0,086310468 |
| miR.558 + miR.609 + miR.220a + miR.519b + miR.130a + miR.26a + miR.29a + miR.369.3p + miR.376a* + miR.518c + miR.660 | 0,81 | 0,294569844 |
| miR.558 + miR.609 + miR.603 + miR.220a + miR.519b + miR.26a + miR.29a + miR.376a* + miR.518c + miR.639 + miR.338 | 0,81 | 0,03381292 |
| miR.558 + miR.609 + miR.603 + miR.520f + miR.220a + miR.26a + miR.29a + miR.376a* + miR.518c + miR.660 + miR.338 | 0,81 | 0,045403754 |
| miR.558 + miR.609 + miR.603 + miR.520f + miR.220a + miR.130a + miR.29a + miR.362 + miR.376a* + miR.518c + miR.338 | 0,81 | 0,013739647 |
| miR.558 + miR.609 + miR.603 + miR.220a + miR.519b + miR.130a + miR.29a + miR.362 + miR.376a* + miR.518c + miR.639 | 0,81 | 0,075407389 |
| miR.558 + miR.609 + miR.520f + miR.220a + miR.26a + miR.29a + miR.362 + miR.369.3p + miR.376a* + miR.518c + miR.639 | 0,81 | 0,053593204 |
| miR.558 + miR.609 + miR.603 + miR.220a + miR.519b + miR.29a + miR.376a* + miR.494 + miR.518c + miR.639 + miR.660 | 0,81 | 0,085050933 |
| miR.558 + miR.609 + miR.603 + miR.220a + miR.519b + miR.130a + miR.29a + miR.362 + miR.376a* + miR.518c + miR.338 | 0,81 | 0,051576947 |
| miR.558 + miR.609 + miR.220a + miR.519b + miR.29a + miR.362 + miR.369.3p + miR.376a* + miR.494 + miR.518c + miR.660 | 0,81 | 0,151790369 |
| miR.558 + miR.609 + miR.603 + miR.520f + miR.519b + miR.29a + miR.369.3p + miR.376a* + miR.518c + miR.639 + miR.660 | 0,81 | 0,014192635 |
| miR.558 + miR.609 + miR.520f + miR.220a + miR.130a + miR.29a + miR.369.3p + miR.376a* + miR.494 + miR.518c + miR.338 | 0,81 | 0,012472117 |
| miR.558 + miR.609 + miR.603 + miR.220a + miR.130a + miR.26a + miR.29a + miR.376a* + miR.518c + miR.660 + miR.338 | 0,81 | 0,076540686 |
| miR.558 + miR.609 + miR.220a + miR.130a + miR.29a + miR.369.3p + miR.376a* + miR.494 + miR.518c + miR.660 + miR.338 | 0,81 | 0,067014402 |
| miR.558 + miR.609 + miR.603 + miR.130a + miR.29a + miR.369.3p + miR.376a* + miR.494 + miR.518c + miR.639 + miR.660 | 0,81 | 0,086421408 |
| miR.558 + miR.609 + miR.603 + miR.520f + miR.220a + miR.29a + miR.362 + miR.376a* + miR.518c + miR.660 + miR.338 | 0,81 | 0,041981926 |
| miR.558 + miR.609 + miR.603 + miR.220a + miR.519b + miR.29a + miR.376a* + miR.494 + miR.518c + miR.660 + miR.338 | 0,81 | 0,083692371 |
| miR.558 + miR.609 + miR.603 + miR.220a + miR.519b + miR.29a + miR.362 + miR.376a* + miR.518c + miR.639 + miR.338 | 0,81 | 0,04976216 |
| miR.558 + miR.609 + miR.603 + miR.520f + miR.220a + miR.130a + miR.29a + miR.362 + miR.376a* + miR.518c + miR.639 | 0,81 | 0,037063648 |
| miR.558 + miR.609 + miR.520f + miR.220a + miR.26a + miR.29a + miR.369.3p + miR.376a* + miR.494 + miR.518c + miR.639 | 0,81 | 0,039214085 |
| miR.558 + miR.609 + miR.603 + miR.220a + miR.26a + miR.29a + miR.362 + miR.376a* + miR.518c + miR.639 + miR.660 | 0,81 | 0,087085895 |
| miR.558 + miR.609 + miR.603 + miR.130a + miR.26a + miR.29a + miR.369.3p + miR.376a* + miR.518c + miR.639 + miR.660 | 0,81 | 0,081616764 |
| miR.558 + miR.609 + miR.220a + miR.519b + miR.130a + miR.26a + miR.29a + miR.369.3p + miR.376a* + miR.518c + miR.338 | 0,81 | 0,085988815 |
| miR.558 + miR.609 + miR.603 + miR.519b + miR.130a + miR.29a + miR.369.3p + miR.376a* + miR.494 + miR.518c + miR.639 | 0,81 | 0,092592731 |
| miR.558 + miR.609 + miR.220a + miR.519b + miR.130a + miR.29a + miR.369.3p + miR.376a* + miR.518c + miR.639 + miR.660 | 0,81 | 0,201261968 |
| miR.558 + miR.609 + miR.603 + miR.220a + miR.130a + miR.26a + miR.29a + miR.376a* + miR.518c + miR.639 + miR.338 | 0,81 | 0,022175169 |
| miR.558 + miR.609 + miR.603 + miR.520f + miR.220a + miR.26a + miR.29a + miR.362 + miR.376a* + miR.494 + miR.518c | 0,81 | 0,035996796 |
| miR.558 + miR.609 + miR.220a + miR.519b + miR.29a + miR.362 + miR.369.3p + miR.376a* + miR.518c + miR.639 + miR.660 | 0,81 | 0,186763379 |
| miR.558 + miR.609 + miR.603 + miR.520f + miR.220a + miR.26a + miR.29a + miR.376a* + miR.518c + miR.639 + miR.338 | 0,81 | 0,01721356 |
| miR.558 + miR.609 + miR.220a + miR.26a + miR.29a + miR.362 + miR.369.3p + miR.376a* + miR.518c + miR.639 + miR.338 | 0,81 | 0,064255573 |
| miR.558 + miR.609 + miR.603 + miR.26a + miR.29a + miR.369.3p + miR.376a* + miR.494 + miR.518c + miR.660 + miR.338 | 0,81 | 0,04049375 |
| miR.558 + miR.609 + miR.520f + miR.220a + miR.29a + miR.362 + miR.376a* + miR.518c + miR.639 + miR.660 + miR.338 | 0,81 | 0,022946312 |
| miR.558 + miR.609 + miR.603 + miR.220a + miR.29a + miR.362 + miR.369.3p + miR.376a* + miR.494 + miR.518c + miR.639 | 0,81 | 0,015549792 |
| miR.558 + miR.609 + miR.603 + miR.220a + miR.26a + miR.29a + miR.362 + miR.376a* + miR.518c + miR.660 + miR.338 | 0,81 | 0,077055176 |
| miR.558 + miR.609 + miR.603 + miR.220a + miR.130a + miR.26a + miR.29a + miR.362 + miR.376a* + miR.518c + miR.660 | 0,81 | 0,095416401 |
| miR.558 + miR.609 + miR.520f + miR.220a + miR.29a + miR.362 + miR.376a* + miR.494 + miR.518c + miR.660 + miR.338 | 0,81 | 0,025050582 |
| miR.558 + miR.609 + miR.603 + miR.520f + miR.220a + miR.29a + miR.362 + miR.376a* + miR.518c + miR.639 + miR.660 | 0,81 | 0,07243504 |
| miR.558 + miR.609 + miR.603 + miR.520f + miR.220a + miR.130a + miR.29a + miR.362 + miR.376a* + miR.494 + miR.518c | 0,81 | 0,019927203 |
| miR.558 + miR.609 + miR.603 + miR.520f + miR.29a + miR.362 + miR.369.3p + miR.376a* + miR.494 + miR.518c + miR.660 | 0,81 | 0,012455695 |
| miR.558 + miR.609 + miR.603 + miR.220a + miR.519b + miR.130a + miR.29a + miR.362 + miR.376a* + miR.518c + miR.660 | 0,81 | 0,075968748 |
| miR.558 + miR.609 + miR.520f + miR.220a + miR.26a + miR.29a + miR.376a* + miR.518c + miR.639 + miR.660 + miR.338 | 0,81 | 0,03433846 |
| miR.558 + miR.609 + miR.603 + miR.220a + miR.130a + miR.29a + miR.376a* + miR.494 + miR.518c + miR.639 + miR.660 | 0,81 | 0,062226162 |
| miR.558 + miR.609 + miR.520f + miR.220a + miR.519b + miR.26a + miR.29a + miR.369.3p + miR.376a* + miR.518c + miR.338 | 0,81 | 0,093529858 |
| miR.558 + miR.609 + miR.220a + miR.519b + miR.130a + miR.29a + miR.369.3p + miR.376a* + miR.518c + miR.660 + miR.338 | 0,81 | 0,198581619 |
| miR.558 + miR.609 + miR.603 + miR.26a + miR.29a + miR.362 + miR.369.3p + miR.376a* + miR.494 + miR.518c + miR.660 | 0,81 | 0,049827665 |
| miR.558 + miR.609 + miR.603 + miR.520f + miR.29a + miR.362 + miR.376a* + miR.494 + miR.518c + miR.639 + miR.338 | 0,81 | 0,007905548 |
| miR.558 + miR.609 + miR.603 + miR.220a + miR.29a + miR.362 + miR.369.3p + miR.376a* + miR.494 + miR.518c + miR.338 | 0,81 | 0,022692148 |
| miR.558 + miR.609 + miR.603 + miR.220a + miR.519b + miR.29a + miR.369.3p + miR.376a* + miR.518c + miR.639 + miR.338 | 0,81 | 0,067244444 |
| miR.558 + miR.609 + miR.220a + miR.519b + miR.26a + miR.29a + miR.362 + miR.369.3p + miR.376a* + miR.494 + miR.518c | 0,81 | 0,117542114 |
| miR.558 + miR.609 + miR.130a + miR.29a + miR.362 + miR.369.3p + miR.376a* + miR.518c + miR.639 + miR.660 + miR.338 | 0,81 | 0,220689648 |
| miR.558 + miR.609 + miR.603 + miR.520f + miR.26a + miR.29a + miR.369.3p + miR.376a* + miR.494 + miR.518c + miR.660 | 0,81 | 0,016007002 |
| miR.558 + miR.609 + miR.603 + miR.520f + miR.26a + miR.29a + miR.362 + miR.369.3p + miR.376a* + miR.518c + miR.639 | 0,81 | 0,022021555 |
| miR.558 + miR.609 + miR.130a + miR.26a + miR.29a + miR.369.3p + miR.376a* + miR.494 + miR.518c + miR.639 + miR.660 | 0,81 | 0,132901754 |
| miR.558 + miR.609 + miR.220a + miR.26a + miR.29a + miR.362 + miR.369.3p + miR.376a* + miR.494 + miR.518c + miR.639 | 0,81 | 0,069800398 |
| miR.558 + miR.609 + miR.603 + miR.520f + miR.130a + miR.29a + miR.369.3p + miR.376a* + miR.494 + miR.518c + miR.639 | 0,81 | 0,012340108 |
| miR.558 + miR.609 + miR.520f + miR.220a + miR.29a + miR.362 + miR.369.3p + miR.376a* + miR.494 + miR.518c + miR.639 | 0,81 | 0,015361333 |
| miR.558 + miR.609 + miR.520f + miR.220a + miR.130a + miR.29a + miR.369.3p + miR.376a* + miR.518c + miR.639 + miR.338 | 0,81 | 0,025699046 |
| miR.558 + miR.609 + miR.520f + miR.220a + miR.130a + miR.29a + miR.376a* + miR.518c + miR.639 + miR.660 + miR.338 | 0,81 | 0,030405254 |
| miR.558 + miR.609 + miR.603 + miR.520f + miR.519b + miR.29a + miR.376a* + miR.494 + miR.518c + miR.639 + miR.338 | 0,81 | 0,020540108 |
| miR.558 + miR.609 + miR.520f + miR.220a + miR.26a + miR.29a + miR.376a* + miR.494 + miR.518c + miR.660 + miR.338 | 0,81 | 0,042497847 |
| miR.558 + miR.609 + miR.603 + miR.220a + miR.29a + miR.362 + miR.369.3p + miR.376a* + miR.518c + miR.639 + miR.338 | 0,81 | 0,016978118 |
| miR.558 + miR.609 + miR.603 + miR.520f + miR.220a + miR.519b + miR.29a + miR.376a* + miR.518c + miR.639 + miR.338 | 0,81 | 0,01481517 |
| miR.558 + miR.609 + miR.520f + miR.130a + miR.26a + miR.29a + miR.369.3p + miR.376a* + miR.518c + miR.660 + miR.338 | 0,81 | 0,124051098 |
| miR.558 + miR.609 + miR.520f + miR.220a + miR.519b + miR.29a + miR.362 + miR.369.3p + miR.376a* + miR.518c + miR.338 | 0,81 | 0,078314353 |
| miR.558 + miR.609 + miR.603 + miR.520f + miR.220a + miR.519b + miR.130a + miR.29a + miR.376a* + miR.518c + miR.338 | 0,81 | 0,021390948 |
| miR.558 + miR.609 + miR.603 + miR.520f + miR.519b + miR.29a + miR.362 + miR.376a* + miR.518c + miR.639 + miR.338 | 0,81 | 0,025591113 |
| miR.558 + miR.609 + miR.603 + miR.220a + miR.519b + miR.29a + miR.362 + miR.376a* + miR.518c + miR.660 + miR.338 | 0,81 | 0,06634884 |
| miR.558 + miR.609 + miR.603 + miR.520f + miR.220a + miR.519b + miR.26a + miR.29a + miR.376a* + miR.518c + miR.338 | 0,81 | 0,024426349 |
| miR.558 + miR.609 + miR.520f + miR.519b + miR.130a + miR.29a + miR.369.3p + miR.376a* + miR.518c + miR.639 + miR.660 | 0,81 | 0,050286249 |
| miR.558 + miR.609 + miR.520f + miR.220a + miR.519b + miR.29a + miR.362 + miR.369.3p + miR.376a* + miR.494 + miR.518c | 0,81 | 0,112318628 |
| miR.558 + miR.609 + miR.603 + miR.220a + miR.519b + miR.29a + miR.362 + miR.376a* + miR.518c + miR.639 + miR.660 | 0,81 | 0,075811398 |
| miR.558 + miR.609 + miR.519b + miR.130a + miR.29a + miR.362 + miR.369.3p + miR.376a* + miR.518c + miR.639 + miR.660 | 0,81 | 0,09718383 |
| miR.558 + miR.609 + miR.520f + miR.26a + miR.29a + miR.369.3p + miR.376a* + miR.494 + miR.518c + miR.639 + miR.338 | 0,81 | 0,098814329 |
| miR.558 + miR.609 + miR.603 + miR.520f + miR.130a + miR.26a + miR.29a + miR.369.3p + miR.376a* + miR.518c + miR.639 | 0,81 | 0,035025936 |
| miR.558 + miR.609 + miR.603 + miR.520f + miR.519b + miR.29a + miR.376a* + miR.494 + miR.518c + miR.660 + miR.338 | 0,81 | 0,022883571 |
| miR.558 + miR.609 + miR.603 + miR.26a + miR.29a + miR.362 + miR.369.3p + miR.376a* + miR.494 + miR.518c + miR.338 | 0,81 | 0,040336229 |
| miR.558 + miR.609 + miR.603 + miR.519b + miR.26a + miR.29a + miR.369.3p + miR.376a* + miR.494 + miR.518c + miR.338 | 0,81 | 0,078455313 |
| miR.558 + miR.609 + miR.220a + miR.519b + miR.26a + miR.29a + miR.369.3p + miR.376a* + miR.518c + miR.639 + miR.338 | 0,81 | 0,106678031 |
| miR.558 + miR.609 + miR.603 + miR.519b + miR.26a + miR.29a + miR.369.3p + miR.376a* + miR.494 + miR.518c + miR.639 | 0,81 | 0,047800307 |
| miR.558 + miR.609 + miR.220a + miR.130a + miR.26a + miR.29a + miR.369.3p + miR.376a* + miR.494 + miR.518c + miR.639 | 0,81 | 0,056661572 |
| miR.558 + miR.609 + miR.220a + miR.130a + miR.26a + miR.29a + miR.362 + miR.369.3p + miR.376a* + miR.518c + miR.338 | 0,81 | 0,06114084 |
| miR.558 + miR.609 + miR.520f + miR.220a + miR.519b + miR.26a + miR.29a + miR.362 + miR.369.3p + miR.376a* + miR.518c | 0,81 | 0,104305147 |
| miR.558 + miR.609 + miR.603 + miR.520f + miR.29a + miR.362 + miR.369.3p + miR.376a* + miR.494 + miR.518c + miR.639 | 0,81 | 0,006938993 |
| miR.558 + miR.609 + miR.520f + miR.220a + miR.519b + miR.29a + miR.376a* + miR.494 + miR.518c + miR.639 + miR.660 | 0,81 | 0,075863803 |
| miR.558 + miR.609 + miR.520f + miR.130a + miR.29a + miR.362 + miR.369.3p + miR.376a* + miR.518c + miR.639 + miR.660 | 0,81 | 0,042787163 |
| miR.558 + miR.609 + miR.520f + miR.220a + miR.130a + miR.29a + miR.362 + miR.369.3p + miR.376a* + miR.494 + miR.518c | 0,81 | 0,026105257 |
| miR.558 + miR.609 + miR.220a + miR.130a + miR.26a + miR.29a + miR.362 + miR.369.3p + miR.376a* + miR.518c + miR.639 | 0,81 | 0,073602386 |
| miR.558 + miR.609 + miR.603 + miR.520f + miR.220a + miR.130a + miR.26a + miR.29a + miR.376a* + miR.518c + miR.660 | 0,81 | 0,057573822 |
| miR.558 + miR.609 + miR.520f + miR.220a + miR.519b + miR.29a + miR.362 + miR.376a* + miR.494 + miR.518c + miR.338 | 0,81 | 0,156587474 |
| miR.558 + miR.609 + miR.603 + miR.520f + miR.220a + miR.130a + miR.26a + miR.29a + miR.362 + miR.376a* + miR.518c | 0,81 | 0,041293271 |
| miR.558 + miR.609 + miR.520f + miR.220a + miR.130a + miR.29a + miR.376a* + miR.494 + miR.518c + miR.660 + miR.338 | 0,81 | 0,035990165 |
| miR.558 + miR.609 + miR.520f + miR.220a + miR.130a + miR.26a + miR.29a + miR.362 + miR.369.3p + miR.376a* + miR.518c | 0,81 | 0,061490013 |
| miR.558 + miR.609 + miR.520f + miR.519b + miR.130a + miR.29a + miR.369.3p + miR.376a* + miR.518c + miR.660 + miR.338 | 0,81 | 0,161684369 |
| miR.558 + miR.609 + miR.220a + miR.519b + miR.26a + miR.29a + miR.362 + miR.369.3p + miR.376a* + miR.518c + miR.338 | 0,81 | 0,086573213 |
| miR.558 + miR.609 + miR.520f + miR.29a + miR.362 + miR.369.3p + miR.376a* + miR.494 + miR.518c + miR.639 + miR.660 | 0,81 | 0,005060017 |
| miR.558 + miR.609 + miR.220a + miR.519b + miR.130a + miR.26a + miR.29a + miR.369.3p + miR.376a* + miR.494 + miR.518c | 0,81 | 0,129172239 |
| miR.558 + miR.609 + miR.603 + miR.520f + miR.220a + miR.26a + miR.29a + miR.376a* + miR.494 + miR.518c + miR.338 | 0,81 | 0,009723753 |
| miR.558 + miR.609 + miR.520f + miR.220a + miR.130a + miR.26a + miR.29a + miR.369.3p + miR.376a* + miR.494 + miR.518c | 0,81 | 0,046743094 |
| miR.558 + miR.609 + miR.26a + miR.29a + miR.362 + miR.369.3p + miR.376a* + miR.494 + miR.518c + miR.660 + miR.338 | 0,81 | 0,296286447 |
| miR.558 + miR.609 + miR.603 + miR.520f + miR.519b + miR.29a + miR.362 + miR.376a* + miR.494 + miR.518c + miR.338 | 0,81 | 0,036308954 |
| miR.558 + miR.609 + miR.603 + miR.520f + miR.519b + miR.26a + miR.29a + miR.369.3p + miR.376a* + miR.518c + miR.639 | 0,81 | 0,02993632 |
| miR.558 + miR.609 + miR.520f + miR.220a + miR.519b + miR.130a + miR.29a + miR.376a* + miR.494 + miR.518c + miR.660 | 0,81 | 0,099339586 |
| miR.558 + miR.609 + miR.520f + miR.220a + miR.519b + miR.26a + miR.29a + miR.376a* + miR.494 + miR.518c + miR.660 | 0,81 | 0,104036996 |
| miR.558 + miR.609 + miR.520f + miR.220a + miR.519b + miR.29a + miR.376a* + miR.518c + miR.639 + miR.660 + miR.338 | 0,81 | 0,052831722 |
| miR.558 + miR.609 + miR.603 + miR.519b + miR.26a + miR.29a + miR.369.3p + miR.376a* + miR.518c + miR.639 + miR.660 | 0,81 | 0,049674536 |
| miR.558 + miR.609 + miR.220a + miR.519b + miR.26a + miR.29a + miR.369.3p + miR.376a* + miR.494 + miR.518c + miR.639 | 0,81 | 0,11045933 |
| miR.558 + miR.609 + miR.220a + miR.130a + miR.26a + miR.29a + miR.362 + miR.369.3p + miR.376a* + miR.494 + miR.518c | 0,81 | 0,056192034 |
| miR.558 + miR.609 + miR.520f + miR.220a + miR.519b + miR.29a + miR.362 + miR.376a* + miR.494 + miR.518c + miR.660 | 0,81 | 0,112225925 |
| miR.558 + miR.609 + miR.603 + miR.520f + miR.519b + miR.29a + miR.369.3p + miR.376a* + miR.494 + miR.518c + miR.639 | 0,81 | 0,023455678 |
| miR.558 + miR.609 + miR.603 + miR.130a + miR.29a + miR.369.3p + miR.376a* + miR.494 + miR.518c + miR.639 + miR.338 | 0,81 | 0,063600125 |
| miR.558 + miR.609 + miR.603 + miR.520f + miR.519b + miR.29a + miR.369.3p + miR.376a* + miR.494 + miR.518c + miR.660 | 0,81 | 0,025545841 |
| miR.558 + miR.609 + miR.603 + miR.520f + miR.130a + miR.29a + miR.369.3p + miR.376a* + miR.494 + miR.518c + miR.660 | 0,81 | 0,011931387 |
| miR.558 + miR.609 + miR.520f + miR.29a + miR.362 + miR.369.3p + miR.376a* + miR.494 + miR.639 + miR.660 + miR.338 | 0,81 | 0,416173783 |
| miR.558 + miR.609 + miR.520f + miR.220a + miR.130a + miR.29a + miR.376a* + miR.494 + miR.518c + miR.639 + miR.660 | 0,81 | 0,040495396 |
| miR.558 + miR.609 + miR.603 + miR.26a + miR.29a + miR.362 + miR.369.3p + miR.376a* + miR.494 + miR.518c + miR.639 | 0,81 | 0,038626744 |
| miR.558 + miR.609 + miR.603 + miR.520f + miR.26a + miR.29a + miR.362 + miR.369.3p + miR.376a* + miR.518c + miR.660 | 0,81 | 0,023366486 |
| miR.558 + miR.609 + miR.520f + miR.130a + miR.29a + miR.362 + miR.369.3p + miR.376a* + miR.494 + miR.518c + miR.660 | 0,81 | 0,040028487 |
| miR.558 + miR.609 + miR.603 + miR.130a + miR.26a + miR.29a + miR.369.3p + miR.376a* + miR.494 + miR.518c + miR.338 | 0,81 | 0,029667186 |
| miR.558 + miR.609 + miR.520f + miR.130a + miR.26a + miR.29a + miR.369.3p + miR.376a* + miR.518c + miR.639 + miR.660 | 0,81 | 0,088773445 |
| miR.558 + miR.609 + miR.220a + miR.519b + miR.29a + miR.369.3p + miR.376a* + miR.494 + miR.518c + miR.660 + miR.338 | 0,81 | 0,143729223 |
| miR.558 + miR.609 + miR.220a + miR.519b + miR.29a + miR.369.3p + miR.376a* + miR.494 + miR.518c + miR.639 + miR.660 | 0,81 | 0,144697773 |
| miR.558 + miR.609 + miR.520f + miR.130a + miR.29a + miR.369.3p + miR.376a* + miR.494 + miR.518c + miR.639 + miR.338 | 0,81 | 0,027816587 |
| miR.558 + miR.609 + miR.520f + miR.519b + miR.26a + miR.29a + miR.369.3p + miR.376a* + miR.518c + miR.660 + miR.338 | 0,81 | 0,154113792 |
| miR.558 + miR.609 + miR.520f + miR.220a + miR.519b + miR.130a + miR.29a + miR.362 + miR.376a* + miR.494 + miR.518c | 0,81 | 0,153084048 |
| miR.558 + miR.609 + miR.520f + miR.220a + miR.519b + miR.29a + miR.369.3p + miR.376a* + miR.518c + miR.639 + miR.338 | 0,81 | 0,063432761 |
| miR.558 + miR.609 + miR.603 + miR.520f + miR.26a + miR.29a + miR.362 + miR.376a* + miR.518c + miR.639 + miR.338 | 0,81 | 0,016097375 |
| miR.558 + miR.609 + miR.220a + miR.519b + miR.130a + miR.29a + miR.362 + miR.369.3p + miR.376a* + miR.518c + miR.660 | 0,81 | 0,184820007 |
| miR.558 + miR.609 + miR.603 + miR.130a + miR.26a + miR.29a + miR.369.3p + miR.376a* + miR.518c + miR.660 + miR.338 | 0,81 | 0,057914614 |
| miR.558 + miR.609 + miR.520f + miR.220a + miR.519b + miR.130a + miR.29a + miR.369.3p + miR.376a* + miR.494 + miR.518c | 0,81 | 0,095816035 |
| miR.558 + miR.609 + miR.603 + miR.519b + miR.130a + miR.26a + miR.29a + miR.369.3p + miR.376a* + miR.518c + miR.639 | 0,81 | 0,060645707 |
| miR.609 + miR.603 + miR.520f + miR.220a + miR.519b + miR.29a + miR.369.3p + miR.376a* + miR.494 + miR.518c + miR.660 | 0,81 | 0,099724155 |
| miR.558 + miR.609 + miR.520f + miR.220a + miR.519b + miR.26a + miR.29a + miR.369.3p + miR.376a* + miR.494 + miR.518c | 0,81 | 0,102782071 |
| miR.558 + miR.609 + miR.220a + miR.519b + miR.130a + miR.26a + miR.29a + miR.362 + miR.369.3p + miR.376a* + miR.518c | 0,81 | 0,123426492 |
| miR.558 + miR.609 + miR.603 + miR.520f + miR.220a + miR.130a + miR.29a + miR.362 + miR.376a* + miR.518c + miR.660 | 0,81 | 0,051362561 |
| miR.558 + miR.609 + miR.520f + miR.220a + miR.130a + miR.29a + miR.362 + miR.369.3p + miR.376a* + miR.518c + miR.639 | 0,81 | 0,02196976 |
| miR.558 + miR.609 + miR.603 + miR.520f + miR.519b + miR.29a + miR.362 + miR.369.3p + miR.376a* + miR.518c + miR.639 | 0,81 | 0,023890343 |
| miR.558 + miR.609 + miR.520f + miR.220a + miR.29a + miR.362 + miR.376a* + miR.494 + miR.518c + miR.639 + miR.338 | 0,81 | 0,018338552 |
| miR.558 + miR.609 + miR.520f + miR.29a + miR.362 + miR.369.3p + miR.376a* + miR.494 + miR.518c + miR.639 + miR.338 | 0,81 | 0,019993663 |
| miR.558 + miR.609 + miR.603 + miR.130a + miR.26a + miR.29a + miR.362 + miR.369.3p + miR.376a* + miR.518c + miR.639 | 0,81 | 0,059898262 |
| miR.558 + miR.609 + miR.603 + miR.520f + miR.26a + miR.29a + miR.376a* + miR.518c + miR.639 + miR.660 + miR.338 | 0,81 | 0,014163526 |
| miR.558 + miR.609 + miR.603 + miR.520f + miR.519b + miR.26a + miR.29a + miR.369.3p + miR.376a* + miR.494 + miR.518c | 0,81 | 0,038010751 |
| miR.609 + miR.603 + miR.520f + miR.220a + miR.29a + miR.369.3p + miR.376a* + miR.494 + miR.518c + miR.639 + miR.660 | 0,81 | 0,038549994 |
| miR.558 + miR.609 + miR.520f + miR.220a + miR.26a + miR.29a + miR.376a* + miR.494 + miR.518c + miR.639 + miR.660 | 0,81 | 0,044172366 |
| miR.558 + miR.609 + miR.520f + miR.220a + miR.29a + miR.362 + miR.376a* + miR.494 + miR.518c + miR.639 + miR.660 | 0,81 | 0,032531163 |
| miR.558 + miR.609 + miR.603 + miR.520f + miR.29a + miR.362 + miR.376a* + miR.518c + miR.639 + miR.660 + miR.338 | 0,81 | 0,014691323 |
| miR.558 + miR.609 + miR.520f + miR.519b + miR.29a + miR.362 + miR.369.3p + miR.376a* + miR.518c + miR.639 + miR.660 | 0,81 | 0,001373045 |
| miR.558 + miR.609 + miR.520f + miR.220a + miR.130a + miR.26a + miR.29a + miR.369.3p + miR.376a* + miR.518c + miR.639 | 0,81 | 0,042499172 |
| miR.558 + miR.609 + miR.603 + miR.520f + miR.220a + miR.26a + miR.29a + miR.376a* + miR.494 + miR.518c + miR.639 | 0,81 | 0,015092493 |
| miR.558 + miR.609 + miR.603 + miR.220a + miR.519b + miR.130a + miR.29a + miR.376a* + miR.518c + miR.660 + miR.338 | 0,81 | 0,069813694 |
| miR.558 + miR.609 + miR.520f + miR.220a + miR.130a + miR.29a + miR.369.3p + miR.376a* + miR.494 + miR.518c + miR.639 | 0,81 | 0,015873606 |
| miR.558 + miR.609 + miR.520f + miR.26a + miR.29a + miR.362 + miR.369.3p + miR.376a* + miR.518c + miR.639 + miR.338 | 0,81 | 0,070490723 |
| miR.558 + miR.609 + miR.603 + miR.29a + miR.362 + miR.369.3p + miR.376a* + miR.494 + miR.518c + miR.660 + miR.338 | 0,81 | 0,094213424 |
| miR.558 + miR.609 + miR.603 + miR.520f + miR.220a + miR.130a + miR.26a + miR.29a + miR.376a* + miR.518c + miR.338 | 0,81 | 0,011896494 |
| miR.558 + miR.609 + miR.520f + miR.519b + miR.29a + miR.362 + miR.376a* + miR.494 + miR.518c + miR.660 + miR.338 | 0,81 | 0,108902057 |
| miR.558 + miR.609 + miR.603 + miR.520f + miR.519b + miR.130a + miR.29a + miR.369.3p + miR.376a* + miR.494 + miR.518c | 0,81 | 0,043412609 |
| miR.558 + miR.609 + miR.603 + miR.520f + miR.130a + miR.29a + miR.362 + miR.369.3p + miR.376a* + miR.518c + miR.639 | 0,81 | 0,017273326 |
| miR.558 + miR.609 + miR.603 + miR.520f + miR.130a + miR.29a + miR.362 + miR.376a* + miR.518c + miR.639 + miR.338 | 0,81 | 0,017606101 |
| miR.558 + miR.609 + miR.520f + miR.220a + miR.519b + miR.29a + miR.362 + miR.369.3p + miR.376a* + miR.518c + miR.639 | 0,81 | 0,088455078 |
| miR.558 + miR.609 + miR.220a + miR.29a + miR.369.3p + miR.376a* + miR.494 + miR.518c + miR.639 + miR.660 + miR.338 | 0,81 | 0,106187002 |
| miR.558 + miR.609 + miR.520f + miR.220a + miR.519b + miR.29a + miR.362 + miR.376a* + miR.494 + miR.518c + miR.639 | 0,81 | 0,114745014 |
| miR.558 + miR.609 + miR.603 + miR.220a + miR.519b + miR.29a + miR.376a* + miR.518c + miR.639 + miR.660 + miR.338 | 0,81 | 0,061048784 |
| miR.558 + miR.609 + miR.603 + miR.520f + miR.130a + miR.29a + miR.376a* + miR.518c + miR.639 + miR.660 + miR.338 | 0,81 | 0,014998606 |
| miR.558 + miR.609 + miR.603 + miR.520f + miR.220a + miR.130a + miR.26a + miR.29a + miR.376a* + miR.518c + miR.639 | 0,81 | 0,018647205 |
| miR.558 + miR.609 + miR.603 + miR.520f + miR.519b + miR.29a + miR.376a* + miR.518c + miR.639 + miR.660 + miR.338 | 0,81 | 0,021039622 |
| miR.558 + miR.609 + miR.520f + miR.220a + miR.519b + miR.130a + miR.29a + miR.369.3p + miR.376a* + miR.518c + miR.338 | 0,81 | 0,059254397 |
| miR.558 + miR.609 + miR.26a + miR.29a + miR.362 + miR.369.3p + miR.376a* + miR.518c + miR.639 + miR.660 + miR.338 | 0,81 | 0,260696485 |
| miR.609 + miR.603 + miR.220a + miR.26a + miR.29a + miR.362 + miR.369.3p + miR.376a* + miR.494 + miR.518c + miR.660 | 0,81 | 0,182700703 |
| miR.558 + miR.609 + miR.520f + miR.220a + miR.519b + miR.26a + miR.29a + miR.369.3p + miR.376a* + miR.518c + miR.639 | 0,81 | 0,077060389 |
| miR.558 + miR.609 + miR.520f + miR.519b + miR.29a + miR.369.3p + miR.376a* + miR.494 + miR.518c + miR.639 + miR.338 | 0,81 | 0,066005251 |
| miR.558 + miR.609 + miR.603 + miR.520f + miR.220a + miR.519b + miR.130a + miR.26a + miR.29a + miR.376a* + miR.518c | 0,81 | 0,056802355 |
| miR.558 + miR.609 + miR.603 + miR.130a + miR.29a + miR.369.3p + miR.376a* + miR.518c + miR.639 + miR.660 + miR.338 | 0,81 | 0,130785723 |
| miR.558 + miR.609 + miR.603 + miR.220a + miR.130a + miR.29a + miR.362 + miR.376a* + miR.494 + miR.518c + miR.639 | 0,81 | 0,02207973 |
| miR.558 + miR.609 + miR.603 + miR.519b + miR.26a + miR.29a + miR.369.3p + miR.376a* + miR.518c + miR.660 + miR.338 | 0,81 | 0,05751366 |
| miR.558 + miR.609 + miR.520f + miR.130a + miR.29a + miR.362 + miR.376a* + miR.494 + miR.518c + miR.639 + miR.338 | 0,81 | 0,035704573 |
| miR.558 + miR.609 + miR.603 + miR.520f + miR.26a + miR.29a + miR.376a* + miR.494 + miR.518c + miR.639 + miR.338 | 0,81 | 0,007665705 |
| miR.558 + miR.609 + miR.220a + miR.519b + miR.130a + miR.26a + miR.29a + miR.369.3p + miR.376a* + miR.518c + miR.639 | 0,81 | 0,10393488 |
| miR.609 + miR.603 + miR.520f + miR.220a + miR.29a + miR.362 + miR.369.3p + miR.376a* + miR.494 + miR.518c + miR.660 | 0,81 | 0,169286523 |
| miR.558 + miR.609 + miR.603 + miR.520f + miR.519b + miR.29a + miR.362 + miR.369.3p + miR.376a* + miR.494 + miR.518c | 0,81 | 0,038996754 |
| miR.558 + miR.609 + miR.520f + miR.130a + miR.26a + miR.29a + miR.376a* + miR.518c + miR.639 + miR.660 + miR.338 | 0,81 | 0,023260381 |
| miR.558 + miR.609 + miR.603 + miR.520f + miR.130a + miR.26a + miR.29a + miR.369.3p + miR.376a* + miR.518c + miR.660 | 0,81 | 0,018767456 |
| miR.558 + miR.609 + miR.603 + miR.220a + miR.519b + miR.29a + miR.362 + miR.376a* + miR.494 + miR.518c + miR.338 | 0,81 | 0,090567038 |
| miR.558 + miR.609 + miR.220a + miR.519b + miR.26a + miR.29a + miR.362 + miR.369.3p + miR.376a* + miR.518c + miR.639 | 0,81 | 0,107298349 |
| miR.558 + miR.609 + miR.603 + miR.520f + miR.130a + miR.29a + miR.376a* + miR.494 + miR.518c + miR.639 + miR.338 | 0,81 | 0,00823629 |
| miR.558 + miR.609 + miR.130a + miR.26a + miR.29a + miR.369.3p + miR.376a* + miR.494 + miR.518c + miR.639 + miR.338 | 0,81 | 0,151347347 |
| miR.558 + miR.609 + miR.520f + miR.130a + miR.29a + miR.362 + miR.376a* + miR.494 + miR.518c + miR.639 + miR.660 | 0,81 | 0,012970067 |
| miR.558 + miR.609 + miR.603 + miR.520f + miR.220a + miR.130a + miR.26a + miR.29a + miR.376a* + miR.494 + miR.518c | 0,81 | 0,010543674 |
| miR.558 + miR.609 + miR.520f + miR.220a + miR.519b + miR.26a + miR.29a + miR.362 + miR.376a* + miR.494 + miR.518c | 0,81 | 0,121473899 |
| miR.609 + miR.603 + miR.220a + miR.519b + miR.26a + miR.29a + miR.369.3p + miR.376a* + miR.494 + miR.518c + miR.660 | 0,81 | 0,095758197 |
| miR.558 + miR.609 + miR.519b + miR.26a + miR.29a + miR.362 + miR.369.3p + miR.376a* + miR.518c + miR.660 + miR.338 | 0,81 | 0,193220916 |
| miR.558 + miR.609 + miR.520f + miR.130a + miR.26a + miR.29a + miR.369.3p + miR.376a* + miR.494 + miR.518c + miR.660 | 0,81 | 0,046186278 |
| miR.558 + miR.609 + miR.520f + miR.519b + miR.130a + miR.29a + miR.376a* + miR.494 + miR.518c + miR.660 + miR.338 | 0,81 | 0,182429686 |
| miR.558 + miR.609 + miR.520f + miR.130a + miR.26a + miR.29a + miR.369.3p + miR.376a* + miR.494 + miR.518c + miR.639 | 0,81 | 0,058565236 |
| miR.609 + miR.603 + miR.220a + miR.29a + miR.362 + miR.369.3p + miR.376a* + miR.494 + miR.518c + miR.639 + miR.660 | 0,81 | 0,25083681 |
| miR.558 + miR.609 + miR.603 + miR.130a + miR.26a + miR.29a + miR.362 + miR.369.3p + miR.376a* + miR.518c + miR.338 | 0,81 | 0,035361638 |
| miR.558 + miR.609 + miR.520f + miR.519b + miR.29a + miR.376a* + miR.494 + miR.518c + miR.639 + miR.660 + miR.338 | 0,81 | 0,050680595 |
| miR.558 + miR.609 + miR.603 + miR.520f + miR.130a + miR.26a + miR.29a + miR.369.3p + miR.376a* + miR.494 + miR.518c | 0,81 | 0,021069965 |
| miR.558 + miR.609 + miR.520f + miR.519b + miR.130a + miR.29a + miR.376a* + miR.494 + miR.518c + miR.639 + miR.660 | 0,81 | 0,031941045 |
| miR.558 + miR.609 + miR.603 + miR.520f + miR.220a + miR.519b + miR.26a + miR.29a + miR.376a* + miR.518c + miR.639 | 0,81 | 0,049963026 |
| miR.558 + miR.609 + miR.220a + miR.519b + miR.29a + miR.369.3p + miR.376a* + miR.518c + miR.639 + miR.660 + miR.338 | 0,81 | 0,141750039 |
| miR.558 + miR.609 + miR.520f + miR.220a + miR.519b + miR.130a + miR.29a + miR.362 + miR.369.3p + miR.376a* + miR.518c | 0,81 | 0,096622842 |
| miR.558 + miR.609 + miR.603 + miR.29a + miR.362 + miR.369.3p + miR.376a* + miR.494 + miR.518c + miR.639 + miR.660 | 0,81 | 0,034245163 |
| miR.558 + miR.609 + miR.603 + miR.520f + miR.26a + miR.29a + miR.362 + miR.369.3p + miR.376a* + miR.494 + miR.518c | 0,81 | 0,014757351 |
| miR.558 + miR.609 + miR.603 + miR.519b + miR.130a + miR.29a + miR.369.3p + miR.376a* + miR.518c + miR.639 + miR.338 | 0,81 | 0,083844351 |
| miR.558 + miR.609 + miR.520f + miR.220a + miR.26a + miR.29a + miR.362 + miR.376a* + miR.494 + miR.518c + miR.338 | 0,81 | 0,028961257 |
| miR.558 + miR.609 + miR.603 + miR.519b + miR.130a + miR.26a + miR.29a + miR.369.3p + miR.376a* + miR.494 + miR.518c | 0,81 | 0,049473938 |
| miR.558 + miR.609 + miR.603 + miR.520f + miR.519b + miR.130a + miR.29a + miR.369.3p + miR.376a* + miR.518c + miR.639 | 0,81 | 0,045356413 |
| miR.558 + miR.609 + miR.520f + miR.130a + miR.26a + miR.29a + miR.376a* + miR.494 + miR.518c + miR.639 + miR.660 | 0,81 | 0,01361162 |
| miR.609 + miR.603 + miR.520f + miR.220a + miR.29a + miR.362 + miR.369.3p + miR.376a* + miR.518c + miR.660 + miR.338 | 0,81 | 0,365700497 |
| miR.558 + miR.609 + miR.603 + miR.520f + miR.519b + miR.29a + miR.362 + miR.369.3p + miR.376a* + miR.518c + miR.660 | 0,81 | 0,026497458 |
| miR.558 + miR.609 + miR.520f + miR.26a + miR.29a + miR.369.3p + miR.376a* + miR.494 + miR.518c + miR.639 + miR.660 | 0,81 | 0,040835548 |
| miR.558 + miR.609 + miR.520f + miR.220a + miR.130a + miR.26a + miR.29a + miR.376a* + miR.518c + miR.660 + miR.338 | 0,81 | 0,037096068 |
| miR.558 + miR.609 + miR.603 + miR.220a + miR.519b + miR.130a + miR.26a + miR.29a + miR.376a* + miR.518c + miR.639 | 0,81 | 0,044256523 |
| miR.558 + miR.609 + miR.520f + miR.220a + miR.26a + miR.29a + miR.362 + miR.376a* + miR.518c + miR.639 + miR.338 | 0,81 | 0,040345533 |
| miR.558 + miR.609 + miR.603 + miR.519b + miR.130a + miR.26a + miR.29a + miR.369.3p + miR.376a* + miR.518c + miR.338 | 0,81 | 0,0479813 |
| miR.609 + miR.603 + miR.220a + miR.519b + miR.29a + miR.362 + miR.369.3p + miR.376a* + miR.494 + miR.518c + miR.660 | 0,81 | 0,178950875 |
| miR.558 + miR.609 + miR.603 + miR.130a + miR.29a + miR.362 + miR.369.3p + miR.376a* + miR.518c + miR.639 + miR.338 | 0,81 | 0,067925379 |
| miR.558 + miR.609 + miR.520f + miR.220a + miR.519b + miR.29a + miR.362 + miR.376a* + miR.518c + miR.639 + miR.338 | 0,81 | 0,066226981 |
| miR.558 + miR.609 + miR.603 + miR.520f + miR.519b + miR.26a + miR.29a + miR.376a* + miR.494 + miR.518c + miR.338 | 0,81 | 0,027930109 |
| miR.558 + miR.609 + miR.520f + miR.130a + miR.26a + miR.29a + miR.369.3p + miR.376a* + miR.518c + miR.639 + miR.338 | 0,81 | 0,083554906 |
| miR.609 + miR.603 + miR.520f + miR.220a + miR.29a + miR.369.3p + miR.376a* + miR.494 + miR.518c + miR.660 + miR.338 | 0,81 | 0,120543505 |
| miR.558 + miR.609 + miR.603 + miR.520f + miR.362 + miR.369.3p + miR.376a* + miR.518c + miR.639 + miR.660 + miR.338 | 0,81 | 0,158481203 |
| miR.558 + miR.609 + miR.520f + miR.220a + miR.519b + miR.130a + miR.26a + miR.29a + miR.369.3p + miR.376a* + miR.518c | 0,81 | 0,092280989 |
| miR.558 + miR.609 + miR.603 + miR.520f + miR.220a + miR.519b + miR.130a + miR.29a + miR.376a* + miR.518c + miR.639 | 0,81 | 0,051565527 |
| miR.558 + miR.609 + miR.520f + miR.220a + miR.519b + miR.130a + miR.29a + miR.376a* + miR.518c + miR.660 + miR.338 | 0,81 | 0,068063887 |
| miR.558 + miR.609 + miR.603 + miR.130a + miR.26a + miR.29a + miR.369.3p + miR.376a* + miR.494 + miR.518c + miR.660 | 0,81 | 0,04079765 |
| miR.558 + miR.609 + miR.520f + miR.220a + miR.519b + miR.29a + miR.369.3p + miR.376a* + miR.494 + miR.518c + miR.639 | 0,81 | 0,067714172 |
| miR.558 + miR.609 + miR.520f + miR.220a + miR.26a + miR.29a + miR.362 + miR.376a* + miR.518c + miR.660 + miR.338 | 0,81 | 0,034211345 |
| miR.558 + miR.609 + miR.220a + miR.519b + miR.130a + miR.26a + miR.29a + miR.376a* + miR.494 + miR.518c + miR.660 | 0,81 | 0,118927367 |
| miR.558 + miR.609 + miR.520f + miR.220a + miR.130a + miR.29a + miR.362 + miR.376a* + miR.518c + miR.660 + miR.338 | 0,81 | 0,033067311 |
| miR.558 + miR.609 + miR.603 + miR.519b + miR.29a + miR.362 + miR.369.3p + miR.376a* + miR.518c + miR.660 + miR.338 | 0,81 | 0,070848434 |
| miR.558 + miR.609 + miR.603 + miR.520f + miR.130a + miR.29a + miR.362 + miR.369.3p + miR.376a* + miR.518c + miR.660 | 0,81 | 0,022320448 |
| miR.558 + miR.609 + miR.130a + miR.26a + miR.29a + miR.362 + miR.369.3p + miR.376a* + miR.518c + miR.639 + miR.660 | 0,81 | 0,12240663 |
| miR.558 + miR.609 + miR.130a + miR.26a + miR.29a + miR.369.3p + miR.376a* + miR.518c + miR.639 + miR.660 + miR.338 | 0,81 | 0,190655218 |
| miR.558 + miR.609 + miR.603 + miR.520f + miR.519b + miR.130a + miR.29a + miR.376a* + miR.494 + miR.518c + miR.338 | 0,81 | 0,033349431 |
| miR.558 + miR.609 + miR.520f + miR.220a + miR.130a + miR.29a + miR.362 + miR.376a* + miR.494 + miR.518c + miR.338 | 0,81 | 0,01826168 |
| miR.558 + miR.609 + miR.220a + miR.519b + miR.130a + miR.26a + miR.29a + miR.362 + miR.376a* + miR.494 + miR.518c | 0,81 | 0,146018166 |
| miR.558 + miR.609 + miR.603 + miR.520f + miR.519b + miR.26a + miR.29a + miR.369.3p + miR.376a* + miR.518c + miR.660 | 0,81 | 0,035714228 |
| miR.558 + miR.609 + miR.603 + miR.220a + miR.130a + miR.29a + miR.376a* + miR.494 + miR.518c + miR.639 + miR.338 | 0,81 | 0,008513863 |
| miR.558 + miR.609 + miR.603 + miR.519b + miR.29a + miR.362 + miR.369.3p + miR.376a* + miR.518c + miR.639 + miR.660 | 0,81 | 0,03394552 |
| miR.558 + miR.609 + miR.603 + miR.130a + miR.29a + miR.362 + miR.369.3p + miR.376a* + miR.518c + miR.660 + miR.338 | 0,81 | 0,078037577 |
| miR.558 + miR.609 + miR.603 + miR.520f + miR.220a + miR.29a + miR.376a* + miR.494 + miR.518c + miR.639 + miR.338 | 0,81 | 0,004743588 |
| miR.558 + miR.609 + miR.520f + miR.220a + miR.519b + miR.26a + miR.29a + miR.376a* + miR.518c + miR.660 + miR.338 | 0,81 | 0,059889168 |

**Table 12: clusters of 12 miRNAs**

| **Cluster of 12 miRs** | **c_tau level** | **pv compared to top signature miR.558+ miR.609+ miR.520f + miR.220a + miR.29a+ miR.362+ miR.369.3p + miR.376a* + miR.518c + miR.639 + miR.660 + miR.338** |
|---|---|---|
| miR.558 + miR.609 + miR.520f + miR.220a + miR.29a + miR.362 + miR.369.3p + miR.376a^{*} + miR.518c + miR.639 + miR.660 + miR.338 | 0,81 | NaN |
| miR.558 + miR.609 + miR.603 + miR.520f + miR.29a + miR.362 + miR.369.3p + miR.376a* + miR.518c + miR.639 + miR.660 + miR.338 | 0,81 | 0,0025 92812 |
| miR.558 + miR.609 + miR.520f + miR.130a + miR.29a + miR.362 + miR.369.3p + miR.376a* + miR.518c + miR.639 + miR.660 + miR.338 | 0,81 | 0,488531084 |
| miR.558 + miR.609 + miR.520f + miR.29a + miR.362 + miR.369.3p + miR.376a* + miR.494 + miR.518c + miR.639 + miR.660 + miR.338 | 0,81 | 0,336902282 |
| miR.558 + miR.609 + miR.603 + miR.520f + miR.220a + miR.29a + miR.369.3p + miR.376a* + miR.518c + miR.639 + miR.660 + miR.338 | 0,81 | 0,02007668 |
| miR.558 + miR.609 + miR.603 + miR.220a + miR.26a + miR.29a + miR.362 + miR.369.3p + miR.376a* + miR.518c + miR.660 + miR.338 | 0,81 | 0,177098203 |
| miR.558 + miR.609 + miR.603 + miR.520f + miR.220a + miR.29a + miR.362 + miR.369.3p + miR.376a* + miR.518c + miR.660 + miR.338 | 0,81 | 0,189132192 |
| miR.558 + miR.609 + miR.520f + miR.519b + miR.29a + miR.362 + miR.369.3p + miR.376a* + miR.518c + miR.639 + miR.660 + miR.338 | 0,81 | 0,326944202 |
| miR.558 + miR.609 + miR.603 + miR.220a + miR.26a + miR.29a + miR.369.3p + miR.376a* + miR.518c + miR.639 + miR.660 + miR.338 | 0,81 | 0,124009861 |
| miR.558 + miR.609 + miR.603 + miR.220a + miR.519b + miR.26a + miR.29a + miR.369.3p + miR.376a* + miR.494 + miR.518c + miR.660 | 0,81 | 0,141408293 |
| miR.558 + miR.609 + miR.603 + miR.220a + miR.26a + miR.29a + miR.362 + miR.369.3p + miR.376a* + miR.518c + miR.639 + miR.660 | 0,81 | 0,126183915 |
| miR.558 + miR.609 + miR.603 + miR.520f + miR.220a + miR.26a + miR.29a + miR.369.3p + miR.376a* + miR.518c + miR.660 + miR.338 | 0,81 | 0,050609288 |
| miR.558 + miR.609 + miR.603 + miR.220a + miR.26a + miR.29a + miR.369.3p + miR.376a* + miR.494 + miR.518c + miR.660 + miR.338 | 0,81 | 0,065026461 |
| miR.558 + miR.609 + miR.603 + miR.220a + miR.26a + miR.29a + miR.369.3p + miR.376a* + miR.494 + miR.518c + miR.639 + miR.660 | 0,81 | 0,058482956 |
| miR.558 + miR.609 + miR.520f + miR.26a + miR.29a + miR.362 + miR.369.3p + miR.376a* + miR.518c + miR.639 + miR.660 + miR.338 | 0,81 | 0,412377362 |
| miR.558 + miR.609 + miR.603 + miR.220a + miR.26a + miR.29a + miR.362 + miR.369.3p + miR.376a* + miR.494 + miR.518c + miR.660 | 0,81 | 0,107094527 |
| miR.558 + miR.609 + miR.603 + miR.520f + miR.220a + miR.519b + miR.29a + miR.369.3p + miR.376a* + miR.518c + miR.660 + miR.338 | 0,81 | 0,06586111 |
| miR.558 + miR.609 + miR.520f + miR.220a + miR.29a + miR.362 + miR.369.3p + miR.376a* + miR.494 + miR.518c + miR.660 + miR.338 | 0,81 | 0,242438064 |
| miR.558 + miR.609 + miR.603 + miR.520f + miR.220a + miR.26a + miR.29a + miR.369.3p + miR.376a* + miR.518c + miR.639 + miR.660 | 0,81 | 0,081782138 |
| miR.558 + miR.609 + miR.603 + miR.520f + miR.220a + miR.29a + miR.369.3p + miR.376a* + miR.494 + miR.518c + miR.660 + miR.338 | 0,81 | 0,027361624 |
| miR.558 + miR.609 + miR.603 + miR.220a + miR.519b + miR.130a + miR.29a + miR.369.3p + miR.376a* + miR.494 + miR.518c + miR.660 | 0,81 | 0,15418724 |
| miR.558 + miR.609 + miR.603 + miR.520f + miR.220a + miR.519b + miR.29a + miR.369.3p + miR.376a* + miR.494 + miR.518c + miR.660 | 0,81 | 0,083490286 |
| miR.558 + miR.609 + miR.603 + miR.220a + miR.130a + miR.26a + miR.29a + miR.369.3p + miR.376a* + miR.518c + miR.660 + miR.338 | 0,81 | 0,06569385 |
| miR.558 + miR.609 + miR.520f + miR.220a + miR.130a + miR.29a + miR.362 + miR.369.3p + miR.376a* + miR.518c + miR.660 + miR.338 | 0,81 | 0,201067691 |
| miR.558 + miR.609 + miR.603 + miR.220a + miR.130a + miR.26a + miR.29a + miR.369.3p + miR.376a* + miR.494 + miR.518c + miR.660 | 0,81 | 0,078405114 |
| miR.558 + miR.609 + miR.603 + miR.520f + miR.220a + miR.26a + miR.29a + miR.369.3p + miR.376a* + miR.494 + miR.518c + miR.660 | 0,81 | 0,061031771 |
| miR.558 + miR.609 + miR.520f + miR.220a + miR.519b + miR.29a + miR.362 + miR.369.3p + miR.376a* + miR.518c + miR.660 + miR.338 | 0,81 | 0,215506409 |
| miR.558 + miR.609 + miR.603 + miR.520f + miR.220a + miR.130a + miR.29a + miR.369.3p + miR.376a* + miR.518c + miR.660 + miR.338 | 0,81 | 0,032147086 |
| miR.558 + miR.609 + miR.520f + miR.220a + miR.26a + miR.29a + miR.362 + miR.369.3p + miR.376a* + miR.518c + miR.660 + miR.338 | 0,81 | 0,210865186 |
| miR.558 + miR.609 + miR.603 + miR.220a + miR.519b + miR.26a + miR.29a + miR.362 + miR.369.3p + miR.376a* + miR.518c + miR.660 | 0,81 | 0,182910244 |
| miR.558 + miR.609 + miR.603 + miR.220a + miR.130a + miR.26a + miR.29a + miR.369.3p + miR.376a* + miR.518c + miR.639 + miR.660 | 0,81 | 0,114604304 |
| miR.558 + miR.609 + miR.130a + miR.29a + miR.362 + miR.369.3p + miR.376a* + miR.494 + miR.518c + miR.639 + miR.660 + miR.338 | 0,81 | 0,146794034 |
| miR.558 + miR.609 + miR.603 + miR.520f + miR.220a + miR.130a + miR.26a + miR.29a + miR.369.3p + miR.376a* + miR.518c + miR.660 | 0,81 | 0,076886713 |
| miR.558 + miR.609 + miR.603 + miR.520f + miR.220a + miR.29a + miR.369.3p + miR.376a* + miR.494 + miR.518c + miR.639 + miR.660 | 0,81 | 0,015969629 |
| miR.558 + miR.609 + miR.603 + miR.220a + miR.519b + miR.26a + miR.29a + miR.369.3p + miR.376a* + miR.494 + miR.518c + miR.338 | 0,81 | 0,09854142 |
| miR.558 + miR.609 + miR.603 + miR.220a + miR.519b + miR.26a + miR.29a + miR.369.3p + miR.376a* + miR.518c + miR.660 + miR.338 | 0,81 | 0,079364664 |
| miR.558 + miR.609 + miR.603 + miR.520f + miR.220a + miR.29a + miR.362 + miR.369.3p + miR.376a* + miR.518c + miR.639 + miR.660 | 0,81 | 0,034275923 |
| miR.558 + miR.609 + miR.603 + miR.220a + miR.29a + miR.362 + miR.369.3p + miR.376a* + miR.518c + miR.639 + miR.660 + miR.338 | 0,81 | 0,202746634 |
| miR.558 + miR.609 + miR.603 + miR.26a + miR.29a + miR.362 + miR.369.3p + miR.376a* + miR.518c + miR.639 + miR.660 + miR.338 | 0,81 | 0,155445223 |
| miR.558 + miR.609 + miR.603 + miR.520f + miR.220a + miR.26a + miR.29a + miR.362 + miR.369.3p + miR.376a* + miR.518c + miR.660 | 0,81 | 0,101826296 |
| miR.558 + miR.609 + miR.520f + miR.130a + miR.29a + miR.362 + miR.369.3p + miR.376a* + miR.494 + miR.518c + miR.660 + miR.338 | 0,81 | 0,268917868 |
| miR.558 + miR.609 + miR.520f + miR.26a + miR.29a + miR.362 + miR.369.3p + miR.376a* + miR.494 + miR.518c + miR.660 + miR.338 | 0,81 | 0,326230383 |
| miR.558 + miR.609 + miR.603 + miR.220a + miR.130a + miR.26a + miR.29a + miR.362 + miR.369.3p + miR.376a* + miR.518c + miR.660 | 0,81 | 0,112631263 |
| miR.558 + miR.609 + miR.603 + miR.220a + miR.519b + miR.29a + miR.362 + miR.369.3p + miR.376a* + miR.494 + miR.518c + miR.660 | 0,81 | 0,108348697 |
| miR.558 + miR.609 + miR.603 + miR.220a + miR.519b + miR.26a + miR.29a + miR.362 + miR.369.3p + miR.376a* + miR.494 + miR.518c | 0,81 | 0,108883642 |
| miR.558 + miR.609 + miR.603 + miR.220a + miR.130a + miR.29a + miR.362 + miR.369.3p + miR.376a* + miR.494 + miR.518c + miR.660 | 0,81 | 0,062616733 |
| miR.558 + miR.609 + miR.603 + miR.520f + miR.220a + miR.29a + miR.362 + miR.369.3p + miR.376a* + miR.494 + miR.518c + miR.660 | 0,81 | 0,029376529 |
| miR.558 + miR.609 + miR.603 + miR.220a + miR.519b + miR.130a + miR.26a + miR.29a + miR.369.3p + miR.376a* + miR.494 + miR.518c | 0,81 | 0,109712201 |
| miR.558 + miR.609 + miR.520f + miR.220a + miR.29a + miR.369.3p + miR.376a* + miR.494 + miR.518c + miR.639 + miR.660 + miR.338 | 0,81 | 0,057529773 |
| miR.558 + miR.609 + miR.603 + miR.220a + miR.519b + miR.26a + miR.29a + miR.369.3p + miR.376a* + miR.518c + miR.639 + miR.660 | 0,81 | 0,156489391 |
| miR.558 + miR.609 + miR.603 + miR.220a + miR.519b + miR.29a + miR.369.3p + miR.376a* + miR.494 + miR.518c + miR.660 + miR.338 | 0,81 | 0,129743304 |
| miR.558 + miR.609 + miR.603 + miR.220a + miR.26a + miR.29a + miR.362 + miR.369.3p + miR.376a* + miR.518c + miR.639 + miR.338 | 0,81 | 0,055914072 |
| miR.558 + miR.609 + miR.603 + miR.520f + miR.29a + miR.369.3p + miR.376a* + miR.494 + miR.518c + miR.639 + miR.660 + miR.338 | 0,81 | 0,015640054 |
| miR.558 + miR.609 + miR.603 + miR.520f + miR.220a + miR.519b + miR.26a + miR.29a + miR.369.3p + miR.376a* + miR.518c + miR.660 | 0,81 | 0,126250646 |
| miR.558 + miR.609 + miR.603 + miR.220a + miR.26a + miR.29a + miR.369.3p + miR.376a* + miR.494 + miR.518c + miR.639 + miR.338 | 0,81 | 0,05527903 |
| miR.558 + miR.609 + miR.220a + miR.26a + miR.29a + miR.362 + miR.369.3p + miR.376a* + miR.518c + miR.639 + miR.660 + miR.338 | 0,81 | 0,371855165 |
| miR.558 + miR.609 + miR.603 + miR.520f + miR.220a + miR.26a + miR.29a + miR.369.3p + miR.376a* + miR.518c + miR.639 + miR.338 | 0,81 | 0,038508599 |
| miR.558 + miR.609 + miR.603 + miR.220a + miR.26a + miR.29a + miR.362 + miR.369.3p + miR.376a* + miR.494 + miR.518c + miR.338 | 0,81 | 0,039809222 |
| miR.558 + miR.609 + miR.603 + miR.520f + miR.220a + miR.519b + miR.29a + miR.369.3p + miR.376a* + miR.518c + miR.639 + miR.660 | 0,81 | 0,100539927 |
| miR.558 + miR.609 + miR.603 + miR.520f + miR.220a + miR.130a + miR.29a + miR.369.3p + miR.376a* + miR.518c + miR.639 + miR.660 | 0,81 | 0,078439518 |
| miR.558 + miR.609 + miR.603 + miR.220a + miR.29a + miR.362 + miR.369.3p + miR.376a* + miR.494 + miR.518c + miR.639 + miR.660 | 0,81 | 0,103874716 |
| miR.558 + miR.609 + miR.603 + miR.220a + miR.519b + miR.130a + miR.26a + miR.29a + miR.369.3p + miR.376a* + miR.518c + miR.660 | 0,81 | 0,139958843 |
| miR.558 + miR.609 + miR.603 + miR.520f + miR.220a + miR.26a + miR.29a + miR.362 + miR.369.3p + miR.376a* + miR.518c + miR.338 | 0,81 | 0,035585025 |
| miR.558 + miR.609 + miR.603 + miR.220a + miR.519b + miR.29a + miR.369.3p + miR.376a* + miR.494 + miR.518c + miR.639 + miR.660 | 0,81 | 0,083709205 |
| miR.558 + miR.609 + miR.603 + miR.520f + miR.130a + miR.29a + miR.362 + miR.369.3p + miR.376a* + miR.518c + miR.660 + miR.338 | 0,81 | 0,178470673 |
| miR.558 + miR.609 + miR.603 + miR.520f + miR.220a + miR.519b + miR.29a + miR.362 + miR.369.3p + miR.376a* + miR.494 + miR.518c | 0,81 | 0,078231414 |
| miR.558 + miR.609 + miR.603 + miR.520f + miR.29a + miR.362 + miR.369.3p + miR.376a* + miR.494 + miR.518c + miR.660 + miR.338 | 0,81 | 0,148540156 |
| miR.558 + miR.609 + miR.603 + miR.220a + miR.130a + miR.26a + miR.29a + miR.369.3p + miR.376a* + miR.518c + miR.639 + miR.338 | 0,81 | 0,052870822 |
| miR.558 + miR.609 + miR.603 + miR.220a + miR.519b + miR.130a + miR.29a + miR.369.3p + miR.376a* + miR.494 + miR.518c + miR.338 | 0,81 | 0,102197194 |
| miR.558 + miR.609 + miR.603 + miR.220a + miR.519b + miR.130a + miR.26a + miR.29a + miR.362 + miR.376a^{*} + miR.494 + miR.518c | 0,81 | 0,083184758 |
| miR.558 + miR.609 + miR.603 + miR.220a + miR.130a + miR.26a + miR.29a + miR.369.3p + miR.376a* + miR.494 + miR.518c + miR.338 | 0,81 | 0,039965868 |
| miR.558 + miR.609 + miR.603 + miR.220a + miR.519b + miR.130a + miR.29a + miR.362 + miR.369.3p + miR.376a* + miR.494 + miR.518c | 0,81 | 0,087852848 |
| miR.558 + miR.609 + miR.603 + miR.520f + miR.220a + miR.130a + miR.29a + miR.362 + miR.369.3p + miR.376a* + miR.518c + miR.660 | 0,81 | 0,048687321 |
| miR.558 + miR.609 + miR.603 + miR.220a + miR.130a + miR.29a + miR.362 + miR.369.3p + miR.376a* + miR.518c + miR.639 + miR.660 | 0,81 | 0,166015604 |
| miR.558 + miR.609 + miR.520f + miR.220a + miR.519b + miR.29a + miR.369.3p + miR.376a* + miR.494 + miR.518c + miR.660 + miR.338 | 0,81 | 0,198580988 |
| miR.558 + miR.609 + miR.603 + miR.520f + miR.220a + miR.26a + miR.29a + miR.369.3p + miR.376a* + miR.494 + miR.518c + miR.338 | 0,81 | 0,030327668 |
| miR.558 + miR.609 + miR.603 + miR.220a + miR.519b + miR.29a + miR.362 + miR.369.3p + miR.376a* + miR.518c + miR.660 + miR.338 | 0,81 | 0,202028658 |
| miR.558 + miR.609 + miR.603 + miR.520f + miR.26a + miR.29a + miR.369.3p + miR.376a* + miR.518c + miR.639 + miR.660 + miR.338 | 0,81 | 0,057304326 |
| miR.558 + miR.609 + miR.603 + miR.520f + miR.220a + miR.130a + miR.29a + miR.369.3p + miR.376a* + miR.494 + miR.518c + miR.660 | 0,81 | 0,026982462 |
| miR.558 + miR.609 + miR.603 + miR.520f + miR.26a + miR.29a + miR.362 + miR.369.3p + miR.376a* + miR.518c + miR.660 + miR.338 | 0,81 | 0,116492472 |
| miR.558 + miR.609 + miR.603 + miR.220a + miR.130a + miR.26a + miR.29a + miR.362 + miR.369.3p + miR.376a* + miR.518c + miR.338 | 0,81 | 0,046219772 |
| miR.558 + miR.609 + miR.603 + miR.220a + miR.519b + miR.26a + miR.29a + miR.369.3p + miR.376a* + miR.494 + miR.518c + miR.639 | 0,81 | 0,104033211 |
| miR.558 + miR.609 + miR.520f + miR.220a + miR.26a + miR.29a + miR.369.3p + miR.376a* + miR.518c + miR.639 + miR.660 + miR.338 | 0,81 | 0,170976258 |
| miR.558 + miR.609 + miR.603 + miR.520f + miR.220a + miR.519b + miR.26a + miR.29a + miR.369.3p + miR.376a* + miR.494 + miR.518c | 0,81 | 0,10934352 |
| miR.558 + miR.609 + miR.603 + miR.220a + miR.29a + miR.362 + miR.369.3p + miR.376a* + miR.494 + miR.518c + miR.660 + miR.338 | 0,81 | 0,127306475 |
| miR.558 + miR.609 + miR.603 + miR.220a + miR.130a + miR.29a + miR.369.3p + miR.376a* + miR.494 + miR.518c + miR.639 + miR.660 | 0,81 | 0,059034471 |
| miR.558 + miR.609 + miR.603 + miR.520f + miR.220a + miR.519b + miR.29a + miR.362 + miR.369.3p + miR.376a* + miR.518c + miR.660 | 0,81 | 0,08903709 |
| miR.558 + miR.609 + miR.603 + miR.520f + miR.519b + miR.29a + miR.369.3p + miR.376a* + miR.518c + miR.639 + miR.660 + miR.338 | 0,81 | 0,031648071 |
| miR.558 + miR.609 + miR.603 + miR.220a + miR.130a + miR.29a + miR.362 + miR.369.3p + miR.376a* + miR.518c + miR.660 + miR.338 | 0,81 | 0,199677494 |
| miR.558 + miR.609 + miR.603 + miR.220a + miR.130a + miR.29a + miR.369.3p + miR.376a* + miR.518c + miR.639 + miR.660 + miR.338 | 0,81 | 0,138829141 |
| miR.558 + miR.609 + miR.520f + miR.220a + miR.519b + miR.29a + miR.369.3p + miR.376a* + miR.518c + miR.639 + miR.660 + miR.338 | 0,81 | 0,117717175 |
| miR.558 + miR.609 + miR.603 + miR.220a + miR.26a + miR.29a + miR.362 + miR.369.3p + miR.376a^{*} + miR.494 + miR.518c + miR.639 | 0,81 | 0,039564709 |
| miR.558 + miR.609 + miR.603 + miR.520f + miR.220a + miR.130a + miR.26a + miR.29a + miR.369.3p + miR.376a* + miR.518c + miR.338 | 0,81 | 0,036582714 |
| miR.558 + miR.609 + miR.520f + miR.130a + miR.29a + miR.369.3p + miR.376a* + miR.494 + miR.518c + miR.639 + miR.660 + miR.338 | 0,81 | 0,154022559 |
| miR.558 + miR.609 + miR.603 + miR.520f + miR.220a + miR.519b + miR.130a + miR.29a + miR.369.3p + miR.376a* + miR.518c + miR.660 | 0,81 | 0,104834008 |
| miR.558 + miR.609 + miR.520f + miR.519b + miR.26a + miR.29a + miR.362 + miR.369.3p + miR.376a* + miR.518c + miR.660 + miR.338 | 0,81 | 0,357884375 |
| miR.558 + miR.609 + miR.603 + miR.520f + miR.220a + miR.519b + miR.130a + miR.29a + miR.369.3p + miR.376a* + miR.494 + miR.518c | 0,81 | 0,096898134 |
| miR.558 + miR.609 + miR.220a + miR.26a + miR.29a + miR.362 + miR.369.3p + miR.376a* + miR.494 + miR.518c + miR.660 + miR.338 | 0,81 | 0,220933014 |
| miR.558 + miR.609 + miR.603 + miR.520f + miR.519b + miR.29a + miR.362 + miR.369.3p + miR.376a* + miR.518c + miR.660 + miR.338 | 0,81 | 0,125736984 |
| miR.558 + miR.609 + miR.603 + miR.220a + miR.519b + miR.29a + miR.362 + miR.369.3p + miR.376a* + miR.518c + miR.639 + miR.660 | 0,81 | 0,106875564 |
| miR.558 + miR.609 + miR.603 + miR.130a + miR.29a + miR.362 + miR.369.3p + miR.376a* + miR.518c + miR.639 + miR.660 + miR.338 | 0,81 | 0,133554228 |
| miR.558 + miR.609 + miR.603 + miR.220a + miR.130a + miR.26a + miR.29a + miR.362 + miR.369.3p + miR.376a* + miR.518c + miR.639 | 0,81 | 0,039682886 |
| miR.558 + miR.609 + miR.603 + miR.220a + miR.130a + miR.26a + miR.29a + miR.362 + miR.369.3p + miR.376a* + miR.494 + miR.518c | 0,81 | 0,041285397 |
| miR.558 + miR.609 + miR.603 + miR.220a + miR.519b + miR.130a + miR.29a + miR.369.3p + miR.376a* + miR.494 + miR.518c + miR.639 | 0,81 | 0,084614591 |
| miR.558 + miR.609 + miR.603 + miR.520f + miR.220a + miR.26a + miR.29a + miR.362 + miR.369.3p + miR.376a* + miR.518c + miR.639 | 0,81 | 0,0293337 |
| miR.558 + miR.609 + miR.603 + miR.220a + miR.519b + miR.130a + miR.29a + miR.369.3p + miR.376a* + miR.518c + miR.639 + miR.660 | 0,81 | 0,087459624 |
| miR.558 + miR.609 + miR.603 + miR.520f + miR.130a + miR.29a + miR.369.3p + miR.376a* + miR.518c + miR.639 + miR.660 + miR.338 | 0,81 | 0,021747995 |
| miR.558 + miR.609 + miR.603 + miR.520f + miR.220a + miR.519b + miR.26a + miR.29a + miR.362 + miR.369.3p + miR.376a* + miR.518c | 0,81 | 0,094564237 |
| miR.558 + miR.609 + miR.603 + miR.520f + miR.220a + miR.26a + miR.29a + miR.362 + miR.369.3p + miR.376a* + miR.494 + miR.518c | 0,81 | 0,037494818 |
| miR.558 + miR.609 + miR.520f + miR.220a + miR.130a + miR.29a + miR.369.3p + miR.376a* + miR.518c + miR.639 + miR.660 + miR.338 | 0,81 | 0,068668665 |
| miR.558 + miR.609 + miR.603 + miR.220a + miR.130a + miR.26a + miR.29a + miR.369.3p + miR.376a* + miR.494 + miR.518c + miR.639 | 0,81 | 0,041958545 |
| miR.558 + miR.609 + miR.603 + miR.220a + miR.519b + miR.26a + miR.29a + miR.362 + miR.369.3p + miR.376a* + miR.518c + miR.639 | 0,81 | 0,086356836 |
| miR.558 + miR.609 + miR.603 + miR.520f + miR.220a + miR.519b + miR.26a + miR.29a + miR.369.3p + miR.376a* + miR.518c + miR.338 | 0,81 | 0,082559044 |
| miR.558 + miR.609 + miR.603 + miR.520f + miR.220a + miR.519b + miR.29a + miR.362 + miR.369.3p + miR.376a* + miR.518c + miR.338 | 0,81 | 0,039798152 |
| miR.558 + miR.609 + miR.603 + miR.220a + miR.519b + miR.130a + miR.29a + miR.362 + miR.376a* + miR.494 + miR.518c + miR.639 | 0,81 | 0,104217685 |
| miR.558 + miR.609 + miR.520f + miR.220a + miR.26a + miR.29a + miR.369.3p + miR.376a* + miR.494 + miR.518c + miR.660 + miR.338 | 0,81 | 0,069197434 |
| miR.558 + miR.609 + miR.220a + miR.130a + miR.29a + miR.362 + miR.369.3p + miR.376a* + miR.518c + miR.639 + miR.660 + miR.338 | 0,81 | 0,258902111 |
| miR.558 + miR.609 + miR.603 + miR.520f + miR.519b + miR.29a + miR.369.3p + miR.376a^{*} + miR.494 + miR.518c + miR.660 + miR.338 | 0,81 | 0,065111946 |
| miR.558 + miR.609 + miR.603 + miR.520f + miR.220a + miR.130a + miR.26a + miR.29a + miR.362 + miR.369.3p + miR.376a* + miR.518c | 0,81 | 0,045420644 |
| miR.558 + miR.609 + miR.603 + miR.220a + miR.519b + miR.130a + miR.29a + miR.362 + miR.376a* + miR.494 + miR.518c + miR.338 | 0,81 | 0,066336168 |
| miR.558 + miR.609 + miR.603 + miR.520f + miR.220a + miR.519b + miR.29a + miR.369.3p + miR.376a* + miR.494 + miR.518c + miR.338 | 0,81 | 0,053178985 |
| miR.558 + miR.609 + miR.603 + miR.520f + miR.220a + miR.519b + miR.29a + miR.376a* + miR.494 + miR.518c + miR.660 + miR.338 | 0,81 | 0,01952814 |
| miR.558 + miR.609 + miR.603 + miR.26a + miR.29a + miR.362 + miR.369.3p + miR.376a* + miR.494 + miR.518c + miR.660 + miR.338 | 0,81 | 0,129352779 |
| miR.558 + miR.609 + miR.603 + miR.220a + miR.29a + miR.369.3p + miR.376a* + miR.494 + miR.518c + miR.639 + miR.660 + miR.338 | 0,81 | 0,082167223 |
| miR.558 + miR.609 + miR.603 + miR.220a + miR.130a + miR.29a + miR.369.3p + miR.376a* + miR.494 + miR.518c + miR.660 + miR.338 | 0,81 | 0,063721406 |
| miR.558 + miR.609 + miR.603 + miR.220a + miR.519b + miR.130a + miR.26a + miR.29a + miR.362 + miR.369.3p + miR.376a* + miR.518c | 0,81 | 0,090634553 |
| miR.558 + miR.609 + miR.520f + miR.519b + miR.29a + miR.362 + miR.369.3p + miR.376a* + miR.494 + miR.518c + miR.660 + miR.338 | 0,81 | 0,355684696 |
| miR.558 + miR.609 + miR.603 + miR.220a + miR.519b + miR.130a + miR.29a + miR.369.3p + miR.376a* + miR.518c + miR.660 + miR.338 | 0,81 | 0,07409765 |
| miR.558 + miR.609 + miR.603 + miR.220a + miR.519b + miR.26a + miR.29a + miR.362 + miR.369.3p + miR.376a* + miR.518c + miR.338 | 0,81 | 0,076790974 |
| miR.558 + miR.609 + miR.603 + miR.220a + miR.519b + miR.130a + miR.29a + miR.362 + miR.369.3p + miR.376a* + miR.518c + miR.660 | 0,81 | 0,116971097 |
| miR.558 + miR.609 + miR.603 + miR.520f + miR.220a + miR.26a + miR.29a + miR.369.3p + miR.376a* + miR.494 + miR.518c + miR.639 | 0,81 | 0,04606939 |
| miR.558 + miR.609 + miR.603 + miR.520f + miR.220a + miR.519b + miR.130a + miR.29a + miR.362 + miR.376a* + miR.494 + miR.518c | 0,81 | 0,050118311 |
| miR.558 + miR.609 + miR.603 + miR.520f + miR.220a + miR.29a + miR.362 + miR.369.3p + miR.376a* + miR.494 + miR.518c + miR.338 | 0,81 | 0,019786653 |
| miR.558 + miR.609 + miR.603 + miR.520f + miR.220a + miR.519b + miR.29a + miR.362 + miR.376a* + miR.494 + miR.518c + miR.338 | 0,81 | 0,02708335 |
| miR.558 + miR.609 + miR.603 + miR.520f + miR.220a + miR.519b + miR.29a + miR.369.3p + miR.376a* + miR.494 + miR.518c + miR.639 | 0,81 | 0,071022896 |
| miR.558 + miR.609 + miR.520f + miR.220a + miR.130a + miR.29a + miR.369.3p + miR.376a* + miR.494 + miR.518c + miR.660 + miR.338 | 0,81 | 0,053347364 |
| miR.558 + miR.609 + miR.603 + miR.520f + miR.220a + miR.130a + miR.26a + miR.29a + miR.369.3p + miR.376a* + miR.518c + miR.639 | 0,81 | 0,045323492 |
| miR.558 + miR.609 + miR.603 + miR.220a + miR.519b + miR.130a + miR.26a + miR.29a + miR.376a* + miR.494 + miR.518c + miR.660 | 0,81 | 0,080182822 |
| miR.558 + miR.609 + miR.130a + miR.26a + miR.29a + miR.362 + miR.369.3p + miR.376a* + miR.518c + miR.639 + miR.660 + miR.338 | 0,81 | 0,283076839 |
| miR.558 + miR.609 + miR.603 + miR.220a + miR.519b + miR.26a + miR.29a + miR.369.3p + miR.376a* + miR.518c + miR.639 + miR.338 | 0,81 | 0,082612745 |
| miR.558 + miR.609 + miR.603 + miR.520f + miR.26a + miR.29a + miR.369.3p + miR.376a* + miR.494 + miR.518c + miR.639 + miR.338 | 0,81 | 0,051257339 |
| miR.558 + miR.609 + miR.603 + miR.520f + miR.220a + miR.130a + miR.26a + miR.29a + miR.369.3p + miR.376a* + miR.494 + miR.518c | 0,81 | 0,041750078 |
| miR.558 + miR.609 + miR.519b + miR.26a + miR.29a + miR.362 + miR.369.3p + miR.376a* + miR.518c + miR.639 + miR.660 + miR.338 | 0,81 | 0,209219184 |
| miR.558 + miR.609 + miR.603 + miR.220a + miR.519b + miR.29a + miR.362 + miR.369.3p + miR.376a* + miR.494 + miR.518c + miR.639 | 0,81 | 0,054616283 |
| miR.558 + miR.609 + miR.603 + miR.220a + miR.519b + miR.130a + miR.26a + miR.29a + miR.369.3p + miR.376a* + miR.518c + miR.338 | 0,81 | 0,077981211 |
| miR.558 + miR.609 + miR.520f + miR.220a + miR.130a + miR.29a + miR.369.3p + miR.376a* + miR.494 + miR.518c + miR.639 + miR.660 | 0,81 | 0,027596958 |
| miR.558 + miR.609 + miR.520f + miR.220a + miR.130a + miR.26a + miR.29a + miR.369.3p + miR.376a* + miR.518c + miR.660 + miR.338 | 0,81 | 0,09511014 |
| miR.558 + miR.609 + miR.220a + miR.130a + miR.29a + miR.362 + miR.369.3p + miR.376a* + miR.494 + miR.518c + miR.639 + miR.660 | 0,81 | 0,117596861 |
| miR.558 + miR.609 + miR.520f + miR.220a + miR.29a + miR.362 + miR.369.3p + miR.376a* + miR.494 + miR.518c + miR.639 + miR.660 | 0,81 | 0,00942296 |
| miR.558 + miR.609 + miR.603 + miR.220a + miR.519b + miR.130a + miR.26a + miR.29a + miR.376a* + miR.494 + miR.518c + miR.338 | 0,81 | 0,044567153 |
| miR.558 + miR.609 + miR.603 + miR.220a + miR.519b + miR.29a + miR.369.3p + miR.376a* + miR.518c + miR.639 + miR.660 + miR.338 | 0,81 | 0,059754431 |
| miR.558 + miR.609 + miR.220a + miR.26a + miR.29a + miR.369.3p + miR.376a* + miR.494 + miR.518c + miR.639 + miR.660 + miR.338 | 0,81 | 0,177500681 |
| miR.558 + miR.609 + miR.520f + miR.130a + miR.26a + miR.29a + miR.369.3p + miR.376a* + miR.518c + miR.639 + miR.660 + miR.338 | 0,81 | 0,20128664 |
| miR.558 + miR.609 + miR.603 + miR.220a + miR.519b + miR.26a + miR.29a + miR.362 + miR.376a* + miR.494 + miR.518c + miR.338 | 0,81 | 0,04197064 |
| miR.558 + miR.609 + miR.519b + miR.130a + miR.29a + miR.362 + miR.369.3p + miR.376a* + miR.518c + miR.639 + miR.660 + miR.338 | 0,81 | 0,106046306 |
| miR.558 + miR.609 + miR.220a + miR.130a + miR.29a + miR.362 + miR.369.3p + miR.376a^{*} + miR.494 + miR.518c + miR.660 + miR.338 | 0,81 | 0,139816579 |
| miR.558 + miR.609 + miR.520f + miR.220a + miR.26a + miR.29a + miR.362 + miR.369.3p + miR.376a* + miR.518c + miR.639 + miR.660 | 0,81 | 0,056852964 |
| miR.558 + miR.609 + miR.520f + miR.519b + miR.130a + miR.29a + miR.369.3p + miR.376a* + miR.518c + miR.639 + miR.660 + miR.338 | 0,81 | 0,175255101 |
| miR.558 + miR.609 + miR.603 + miR.520f + miR.220a + miR.519b + miR.130a + miR.29a + miR.369.3p + miR.376a* + miR.518c + miR.338 | 0,81 | 0,050875026 |
| miR.558 + miR.609 + miR.603 + miR.26a + miR.29a + miR.369.3p + miR.376a* + miR.494 + miR.518c + miR.639 + miR.660 + miR.338 | 0,81 | 0,106355626 |
| miR.558 + miR.609 + miR.520f + miR.220a + miR.26a + miR.29a + miR.369.3p + miR.376a* + miR.494 + miR.518c + miR.639 + miR.660 | 0,81 | 0,080751308 |
| miR.558 + miR.609 + miR.603 + miR.520f + miR.220a + miR.519b + miR.130a + miR.29a + miR.362 + miR.369.3p + miR.376a* + miR.518c | 0,81 | 0,06798211 |
| miR.558 + miR.609 + miR.220a + miR.519b + miR.26a + miR.29a + miR.362 + miR.369.3p + miR.376a* + miR.518c + miR.660 + miR.338 | 0,81 | 0,259415946 |
| miR.558 + miR.609 + miR.520f + miR.130a + miR.29a + miR.362 + miR.369.3p + miR.376a* + miR.494 + miR.518c + miR.639 + miR.660 | 0,81 | 0,039528118 |
| miR.558 + miR.609 + miR.520f + miR.220a + miR.519b + miR.26a + miR.29a + miR.369.3p + miR.376a* + miR.518c + miR.660 + miR.338 | 0,81 | 0,186351378 |
| miR.558 + miR.609 + miR.603 + miR.520f + miR.220a + miR.519b + miR.26a + miR.29a + miR.362 + miR.376a* + miR.494 + miR.518c | 0,81 | 0,049974354 |
| miR.558 + miR.609 + miR.603 + miR.220a + miR.519b + miR.130a + miR.26a + miR.29a + miR.369.3p + miR.376a^{*} + miR.518c + miR.639 | 0,81 | 0,092144157 |
| miR.558 + miR.609 + miR.603 + miR.220a + miR.519b + miR.130a + miR.26a + miR.29a + miR.376a^{*} + miR.494 + miR.518c + miR.639 | 0,81 | 0,045242646 |
| miR.558 + miR.609 + miR.520f + miR.220a + miR.26a + miR.29a + miR.362 + miR.369.3p + miR.376a* + miR.494 + miR.518c + miR.660 | 0,81 | 0,08969417 |
| miR.558 + miR.609 + miR.130a + miR.26a + miR.29a + miR.362 + miR.369.3p + miR.376a* + miR.494 + miR.518c + miR.639 + miR.660 | 0,81 | 0,060981205 |
| miR.558 + miR.609 + miR.603 + miR.220a + miR.519b + miR.130a + miR.29a + miR.362 + miR.376a* + miR.494 + miR.518c + miR.660 | 0,81 | 0,080916323 |
| miR.558 + miR.609 + miR.520f + miR.220a + miR.519b + miR.130a + miR.29a + miR.369.3p + miR.376a* + miR.518c + miR.660 + miR.338 | 0,81 | 0,14154825 |
| miR.558 + miR.609 + miR.603 + miR.220a + miR.519b + miR.130a + miR.29a + miR.362 + miR.369.3p + miR.376a* + miR.518c + miR.338 | 0,81 | 0,03597143 |
| miR.558 + miR.609 + miR.603 + miR.520f + miR.220a + miR.519b + miR.29a + miR.362 + miR.376a* + miR.494 + miR.518c + miR.639 | 0,81 | 0,040098116 |
| miR.558 + miR.609 + miR.603 + miR.520f + miR.220a + miR.29a + miR.376a* + miR.494 + miR.518c + miR.639 + miR.660 + miR.338 | 0,81 | 0,005981727 |
| miR.558 + miR.609 + miR.603 + miR.520f + miR.220a + miR.519b + miR.29a + miR.362 + miR.369.3p + miR.376a* + miR.518c + miR.639 | 0,81 | 0,057462334 |
| miR.558 + miR.609 + miR.220a + miR.26a + miR.29a + miR.362 + miR.369.3p + miR.376a* + miR.494 + miR.518c + miR.639 + miR.660 | 0,81 | 0,105476242 |
| miR.558 + miR.609 + miR.603 + miR.220a + miR.519b + miR.130a + miR.29a + miR.362 + miR.369.3p + miR.376a* + miR.518c + miR.639 | 0,81 | 0,053864735 |
| miR.558 + miR.609 + miR.603 + miR.220a + miR.519b + miR.130a + miR.29a + miR.376a* + miR.494 + miR.518c + miR.660 + miR.338 | 0,81 | 0,054775303 |
| miR.558 + miR.609 + miR.603 + miR.520f + miR.220a + miR.130a + miR.29a + miR.362 + miR.369.3p + miR.376a* + miR.518c + miR.639 | 0,81 | 0,022055344 |
| miR.558 + miR.609 + miR.220a + miR.130a + miR.26a + miR.29a + miR.362 + miR.369.3p + miR.376a^{*} + miR.518c + miR.660 + miR.338 | 0,81 | 0,334796332 |
| miR.558 + miR.609 + miR.220a + miR.130a + miR.26a + miR.29a + miR.369.3p + miR.376a* + miR.518c + miR.639 + miR.660 + miR.338 | 0,81 | 0,17540391 |
| miR.558 + miR.609 + miR.603 + miR.130a + miR.29a + miR.362 + miR.369.3p + miR.376a* + miR.494 + miR.518c + miR.639 + miR.660 | 0,81 | 0,08334602 |
| miR.558 + miR.609 + miR.603 + miR.520f + miR.220a + miR.29a + miR.362 + miR.369.3p + miR.376a* + miR.518c + miR.639 + miR.338 | 0,81 | 0,015001384 |
| miR.558 + miR.609 + miR.520f + miR.519b + miR.29a + miR.369.3p + miR.376a* + miR.494 + miR.518c + miR.639 + miR.660 + miR.338 | 0,81 | 0,123206228 |
| miR.558 + miR.609 + miR.603 + miR.520f + miR.220a + miR.130a + miR.29a + miR.362 + miR.369.3p + miR.376a* + miR.494 + miR.518c | 0,81 | 0,022472817 |
| miR.558 + miR.609 + miR.603 + miR.220a + miR.519b + miR.130a + miR.29a + miR.376a* + miR.494 + miR.518c + miR.639 + miR.660 | 0,81 | 0,049741064 |
| miR.558 + miR.609 + miR.603 + miR.520f + miR.26a + miR.29a + miR.362 + miR.369.3p + miR.376a* + miR.518c + miR.639 + miR.338 | 0,81 | 0,063997541 |
| miR.558 + miR.609 + miR.520f + miR.220a + miR.519b + miR.29a + miR.362 + miR.369.3p + miR.376a* + miR.494 + miR.518c + miR.660 | 0,81 | 0,102740231 |
| miR.558 + miR.609 + miR.520f + miR.130a + miR.26a + miR.29a + miR.362 + miR.369.3p + miR.376a* + miR.518c + miR.660 + miR.338 | 0,81 | 0,389791642 |
| miR.558 + miR.609 + miR.603 + miR.520f + miR.130a + miR.26a + miR.29a + miR.369.3p + miR.376a* + miR.518c + miR.639 + miR.338 | 0,81 | 0,060724377 |
| miR.558 + miR.609 + miR.603 + miR.130a + miR.26a + miR.29a + miR.369.3p + miR.376a* + miR.494 + miR.518c + miR.639 + miR.338 | 0,81 | 0,129006594 |
| miR.558 + miR.609 + miR.520f + miR.519b + miR.130a + miR.29a + miR.362 + miR.369.3p + miR.376a* + miR.518c + miR.660 + miR.338 | 0,81 | 0,358017579 |
| miR.558 + miR.609 + miR.520f + miR.26a + miR.29a + miR.369.3p + miR.376a* + miR.494 + miR.518c + miR.639 + miR.660 + miR.338 | 0,81 | 0,159221004 |
| miR.558 + miR.609 + miR.603 + miR.520f + miR.220a + miR.519b + miR.130a + miR.29a + miR.376a* + miR.494 + miR.518c + miR.660 | 0,81 | 0,054894571 |
| miR.558 + miR.609 + miR.520f + miR.519b + miR.130a + miR.29a + miR.369.3p + miR.376a* + miR.494 + miR.518c + miR.660 + miR.338 | 0,81 | 0,210899702 |
| miR.558 + miR.609 + miR.220a + miR.130a + miR.26a + miR.29a + miR.369.3p + miR.376a* + miR.494 + miR.518c + miR.660 + miR.338 | 0,81 | 0,106786642 |
| miR.558 + miR.609 + miR.603 + miR.520f + miR.130a + miR.29a + miR.369.3p + miR.376a* + miR.494 + miR.518c + miR.639 + miR.338 | 0,81 | 0,021848275 |
| miR.558 + miR.609 + miR.603 + miR.130a + miR.26a + miR.29a + miR.369.3p + miR.376a* + miR.518c + miR.639 + miR.660 + miR.338 | 0,81 | 0,163430507 |
| miR.558 + miR.609 + miR.220a + miR.130a + miR.26a + miR.29a + miR.369.3p + miR.376a* + miR.494 + miR.518c + miR.639 + miR.660 | 0,81 | 0,136259581 |
| miR.558 + miR.609 + miR.603 + miR.520f + miR.220a + miR.519b + miR.26a + miR.29a + miR.369.3p + miR.376a* + miR.518c + miR.639 | 0,81 | 0,088088935 |
| miR.558 + miR.609 + miR.603 + miR.520f + miR.130a + miR.29a + miR.369.3p + miR.376a* + miR.494 + miR.518c + miR.660 + miR.338 | 0,81 | 0,0240627 |
| miR.558 + miR.609 + miR.603 + miR.520f + miR.26a + miR.29a + miR.369.3p + miR.376a* + miR.494 + miR.518c + miR.660 + miR.338 | 0,81 | 0,034936273 |
| miR.558 + miR.609 + miR.603 + miR.220a + miR.519b + miR.130a + miR.29a + miR.369.3p + miR.376a* + miR.518c + miR.639 + miR.338 | 0,81 | 0,037656318 |
| miR.558 + miR.609 + miR.520f + miR.220a + miR.519b + miR.26a + miR.29a + miR.369.3p + miR.376a* + miR.494 + miR.518c + miR.660 | 0,81 | 0,172233835 |
| miR.558 + miR.609 + miR.603 + miR.220a + miR.26a + miR.29a + miR.376a* + miR.494 + miR.518c + miR.639 + miR.660 + miR.338 | 0,81 | 0,022139774 |
| miR.558 + miR.609 + miR.520f + miR.220a + miR.519b + miR.29a + miR.362 + miR.369.3p + miR.376a* + miR.518c + miR.639 + miR.660 | 0,81 | 0,02836117 |
| miR.558 + miR.609 + miR.26a + miR.29a + miR.362 + miR.369.3p + miR.376a* + miR.494 + miR.518c + miR.639 + miR.660 + miR.338 | 0,81 | 0,153774809 |
| miR.558 + miR.609 + miR.603 + miR.520f + miR.220a + miR.130a + miR.29a + miR.369.3p + miR.376a* + miR.494 + miR.518c + miR.639 | 0,81 | 0,017899036 |
| miR.558 + miR.609 + miR.603 + miR.520f + miR.220a + miR.519b + miR.130a + miR.26a + miR.29a + miR.369.3p + miR.376a* + miR.518c | 0,81 | 0,096257059 |
| miR.558 + miR.609 + miR.603 + miR.520f + miR.130a + miR.29a + miR.369.3p + miR.376a* + miR.494 + miR.518c + miR.639 + miR.660 | 0,81 | 0,017465393 |
| miR.558 + miR.609 + miR.603 + miR.520f + miR.220a + miR.29a + miR.369.3p + miR.376a* + miR.494 + miR.518c + miR.639 + miR.338 | 0,81 | 0,015137463 |
| miR.558 + miR.609 + miR.603 + miR.520f + miR.220a + miR.519b + miR.29a + miR.362 + miR.376a* + miR.494 + miR.518c + miR.660 | 0,81 | 0,055639673 |
| miR.558 + miR.609 + miR.603 + miR.520f + miR.519b + miR.130a + miR.29a + miR.369.3p + miR.376a* + miR.518c + miR.660 + miR.338 | 0,81 | 0,071805459 |
| miR.558 + miR.609 + miR.603 + miR.520f + miR.220a + miR.519b + miR.29a + miR.376a* + miR.494 + miR.518c + miR.639 + miR.660 | 0,81 | 0,034546593 |
| miR.558 + miR.609 + miR.603 + miR.519b + miR.26a + miR.29a + miR.362 + miR.369.3p + miR.376a* + miR.518c + miR.660 + miR.338 | 0,81 | 0,130017303 |
| miR.558 + miR.609 + miR.520f + miR.220a + miR.130a + miR.26a + miR.29a + miR.369.3p + miR.376a* + miR.494 + miR.518c + miR.660 | 0,81 | 0,087415784 |
| miR.558 + miR.609 + miR.603 + miR.220a + miR.130a + miR.26a + miR.29a + miR.376a* + miR.494 + miR.518c + miR.639 + miR.660 | 0,81 | 0,018542927 |
| miR.558 + miR.609 + miR.603 + miR.520f + miR.519b + miR.130a + miR.29a + miR.369.3p + miR.376a* + miR.494 + miR.518c + miR.338 | 0,81 | 0,067861605 |
| miR.558 + miR.609 + miR.520f + miR.220a + miR.130a + miR.26a + miR.29a + miR.369.3p + miR.376a* + miR.518c + miR.639 + miR.660 | 0,81 | 0,120120739 |
| miR.558 + miR.609 + miR.220a + miR.519b + miR.26a + miR.29a + miR.369.3p + miR.376a* + miR.518c + miR.639 + miR.660 + miR.338 | 0,81 | 0,194489944 |
| miR.558 + miR.609 + miR.603 + miR.220a + miR.519b + miR.29a + miR.362 + miR.369.3p + miR.376a* + miR.518c + miR.639 + miR.338 | 0,81 | 0,033896627 |
| miR.558 + miR.609 + miR.603 + miR.520f + miR.220a + miR.519b + miR.26a + miR.29a + miR.376a* + miR.494 + miR.518c + miR.660 | 0,81 | 0,043033016 |
| miR.558 + miR.609 + miR.603 + miR.130a + miR.26a + miR.29a + miR.362 + miR.369.3p + miR.376a* + miR.518c + miR.660 + miR.338 | 0,81 | 0,12068531 |
| miR.558 + miR.609 + miR.603 + miR.26a + miR.29a + miR.362 + miR.369.3p + miR.376a* + miR.494 + miR.518c + miR.639 + miR.660 | 0,81 | 0,031281313 |
| miR.558 + miR.609 + miR.520f + miR.220a + miR.519b + miR.130a + miR.29a + miR.369.3p + miR.376a* + miR.494 + miR.518c + miR.660 | 0,81 | 0,168682121 |
| miR.558 + miR.609 + miR.603 + miR.520f + miR.130a + miR.29a + miR.362 + miR.369.3p + miR.376a* + miR.518c + miR.639 + miR.338 | 0,81 | 0,025570568 |
| miR.558 + miR.609 + miR.603 + miR.520f + miR.519b + miR.29a + miR.369.3p + miR.376a* + miR.494 + miR.518c + miR.639 + miR.338 | 0,81 | 0,076088828 |
| miR.558 + miR.609 + miR.520f + miR.220a + miR.130a + miR.29a + miR.362 + miR.369.3p + miR.376a* + miR.494 + miR.518c + miR.660 | 0,81 | 0,059145395 |
| miR.558 + miR.609 + miR.220a + miR.130a + miR.26a + miR.29a + miR.362 + miR.369.3p + miR.376a* + miR.494 + miR.518c + miR.660 | 0,81 | 0,162066088 |
| miR.558 + miR.609 + miR.220a + miR.519b + miR.26a + miR.29a + miR.362 + miR.369.3p + miR.376a* + miR.494 + miR.518c + miR.660 | 0,81 | 0,232841234 |
| miR.558 + miR.609 + miR.220a + miR.519b + miR.26a + miR.29a + miR.369.3p + miR.376a* + miR.494 + miR.518c + miR.639 + miR.660 | 0,81 | 0,231961563 |
| miR.558 + miR.609 + miR.220a + miR.519b + miR.130a + miR.26a + miR.29a + miR.369.3p + miR.376a* + miR.494 + miR.518c + miR.660 | 0,81 | 0,169081995 |
| miR.558 + miR.609 + miR.603 + miR.520f + miR.519b + miR.26a + miR.29a + miR.369.3p + miR.376a* + miR.494 + miR.518c + miR.338 | 0,81 | 0,081746985 |
| miR.558 + miR.609 + miR.603 + miR.520f + miR.519b + miR.26a + miR.29a + miR.369.3p + miR.376a* + miR.518c + miR.639 + miR.338 | 0,81 | 0,06810275 |
| miR.558 + miR.609 + miR.603 + miR.520f + miR.130a + miR.26a + miR.29a + miR.369.3p + miR.376a* + miR.518c + miR.660 + miR.338 | 0,81 | 0,046108142 |
| miR.558 + miR.609 + miR.520f + miR.220a + miR.130a + miR.26a + miR.29a + miR.362 + miR.369.3p + miR.376a* + miR.518c + miR.660 | 0,81 | 0,152570284 |
| miR.558 + miR.609 + miR.220a + miR.519b + miR.26a + miR.29a + miR.369.3p + miR.376a* + miR.494 + miR.518c + miR.660 + miR.338 | 0,81 | 0,179884667 |
| miR.558 + miR.609 + miR.520f + miR.220a + miR.519b + miR.26a + miR.29a + miR.362 + miR.369.3p + miR.376a* + miR.518c + miR.660 | 0,81 | 0,193116825 |
| miR.558 + miR.609 + miR.603 + miR.220a + miR.519b + miR.29a + miR.369.3p + miR.376a* + miR.494 + miR.518c + miR.639 + miR.338 | 0,81 | 0,066058661 |
| miR.558 + miR.609 + miR.603 + miR.520f + miR.519b + miR.29a + miR.362 + miR.369.3p + miR.376a* + miR.494 + miR.518c + miR.338 | 0,81 | 0,059933189 |
| miR.558 + miR.609 + miR.603 + miR.220a + miR.519b + miR.26a + miR.29a + miR.376a* + miR.494 + miR.518c + miR.660 + miR.338 | 0,81 | 0,051471896 |
| miR.558 + miR.609 + miR.603 + miR.520f + miR.220a + miR.130a + miR.29a + miR.369.3p + miR.376a* + miR.518c + miR.639 + miR.338 | 0,81 | 0,014365012 |
| miR.558 + miR.609 + miR.603 + miR.220a + miR.519b + miR.26a + miR.29a + miR.362 + miR.376a* + miR.494 + miR.518c + miR.639 | 0,81 | 0,04706569 |
| miR.558 + miR.609 + miR.603 + miR.520f + miR.519b + miR.26a + miR.29a + miR.369.3p + miR.376a* + miR.518c + miR.660 + miR.338 | 0,81 | 0,06573151 |
| miR.558 + miR.609 + miR.520f + miR.220a + miR.519b + miR.29a + miR.369.3p + miR.376a* + miR.494 + miR.518c + miR.639 + miR.660 | 0,81 | 0,119343204 |
| miR.558 + miR.609 + miR.220a + miR.519b + miR.26a + miR.29a + miR.362 + miR.369.3p + miR.376a* + miR.518c + miR.639 + miR.660 | 0,81 | 0,109932735 |
| miR.558 + miR.609 + miR.603 + miR.220a + miR.519b + miR.26a + miR.29a + miR.362 + miR.376a* + miR.494 + miR.518c + miR.660 | 0,81 | 0,053887773 |
| miR.558 + miR.609 + miR.603 + miR.220a + miR.130a + miR.29a + miR.362 + miR.369.3p + miR.376a* + miR.494 + miR.518c + miR.639 | 0,81 | 0,013894687 |
| miR.558 + miR.609 + miR.520f + miR.220a + miR.130a + miR.29a + miR.362 + miR.369.3p + miR.376a* + miR.518c + miR.639 + miR.660 | 0,81 | 0,062249234 |
| miR.558 + miR.609 + miR.603 + miR.520f + miR.220a + miR.130a + miR.29a + miR.362 + miR.369.3p + miR.376a* + miR.518c + miR.338 | 0,81 | 0,022192148 |
| miR.558 + miR.609 + miR.603 + miR.220a + miR.26a + miR.29a + miR.362 + miR.376a* + miR.494 + miR.518c + miR.639 + miR.338 | 0,81 | 0,021787739 |
| miR.558 + miR.609 + miR.603 + miR.520f + miR.519b + miR.29a + miR.362 + miR.369.3p + miR.376a* + miR.518c + miR.639 + miR.338 | 0,81 | 0,058085464 |
| miR.558 + miR.609 + miR.603 + miR.520f + miR.220a + miR.519b + miR.29a + miR.362 + miR.376a* + miR.518c + miR.639 + miR.338 | 0,81 | 0,021492634 |
| miR.558 + miR.609 + miR.220a + miR.519b + miR.130a + miR.29a + miR.362 + miR.369.3p + miR.376a* + miR.494 + miR.518c + miR.660 | 0,81 | 0,166324239 |
| miR.558 + miR.609 + miR.603 + miR.220a + miR.130a + miR.26a + miR.29a + miR.362 + miR.376a* + miR.518c + miR.639 + miR.338 | 0,81 | 0,024702824 |
| miR.558 + miR.609 + miR.603 + miR.29a + miR.362 + miR.369.3p + miR.376a* + miR.494 + miR.518c + miR.639 + miR.660 + miR.338 | 0,81 | 0,047390942 |
| miR.558 + miR.609 + miR.603 + miR.130a + miR.26a + miR.29a + miR.369.3p + miR.376a* + miR.494 + miR.518c + miR.639 + miR.660 | 0,81 | 0,093783858 |
| miR.558 + miR.609 + miR.520f + miR.519b + miR.26a + miR.29a + miR.369.3p + miR.376a* + miR.518c + miR.639 + miR.660 + miR.338 | 0,81 | 0,18947222 |
| miR.558 + miR.609 + miR.603 + miR.520f + miR.29a + miR.362 + miR.369.3p + miR.376a* + miR.494 + miR.518c + miR.639 + miR.660 | 0,81 | 0,002840781 |
| miR.558 + miR.609 + miR.603 + miR.519b + miR.26a + miR.29a + miR.369.3p + miR.376a* + miR.518c + miR.639 + miR.660 + miR.338 | 0,81 | 0,191880906 |
| miR.558 + miR.609 + miR.603 + miR.520f + miR.26a + miR.29a + miR.369.3p + miR.376a* + miR.494 + miR.518c + miR.639 + miR.660 | 0,81 | 0,019834271 |
| miR.558 + miR.609 + miR.603 + miR.520f + miR.26a + miR.29a + miR.362 + miR.369.3p + miR.376a* + miR.518c + miR.639 + miR.660 | 0,81 | 0,006494839 |
| miR.558 + miR.609 + miR.603 + miR.520f + miR.220a + miR.519b + miR.29a + miR.369.3p + miR.376a* + miR.518c + miR.639 + miR.338 | 0,81 | 0,04153001 |
| miR.558 + miR.609 + miR.220a + miR.29a + miR.362 + miR.369.3p + miR.376a* + miR.494 + miR.518c + miR.639 + miR.660 + miR.338 | 0,81 | 0,106905315 |
| miR.558 + miR.609 + miR.603 + miR.220a + miR.130a + miR.26a + miR.29a + miR.362 + miR.376a* + miR.494 + miR.518c + miR.639 | 0,81 | 0,021948764 |
| miR.558 + miR.609 + miR.603 + miR.520f + miR.29a + miR.362 + miR.369.3p + miR.376a* + miR.494 + miR.518c + miR.639 + miR.338 | 0,81 | 0,014711722 |
| miR.558 + miR.609 + miR.603 + miR.520f + miR.220a + miR.519b + miR.26a + miR.29a + miR.362 + miR.376a* + miR.518c + miR.639 | 0,81 | 0,063714707 |
| miR.558 + miR.609 + miR.520f + miR.130a + miR.29a + miR.362 + miR.376a* + miR.494 + miR.518c + miR.639 + miR.660 + miR.338 | 0,81 | 0,017104441 |
| miR.558 + miR.609 + miR.520f + miR.130a + miR.26a + miR.29a + miR.369.3p + miR.376a* + miR.494 + miR.518c + miR.639 + miR.660 | 0,81 | 0,141167705 |
| miR.558 + miR.609 + miR.519b + miR.130a + miR.29a + miR.362 + miR.369.3p + miR.376a* + miR.494 + miR.518c + miR.639 + miR.660 | 0,81 | 0,074241908 |
| miR.558 + miR.609 + miR.603 + miR.520f + miR.220a + miR.519b + miR.130a + miR.29a + miR.362 + miR.376a* + miR.518c + miR.338 | 0,81 | 0,025818186 |
| miR.558 + miR.609 + miR.130a + miR.26a + miR.29a + miR.362 + miR.369.3p + miR.376a* + miR.494 + miR.518c + miR.660 + miR.338 | 0,81 | 0,290922579 |
| miR.558 + miR.609 + miR.603 + miR.520f + miR.220a + miR.519b + miR.130a + miR.29a + miR.376a* + miR.494 + miR.518c + miR.338 | 0,81 | 0,030305215 |
| miR.558 + miR.609 + miR.520f + miR.220a + miR.519b + miR.130a + miR.29a + miR.369.3p + miR.376a* + miR.518c + miR.639 + miR.660 | 0,81 | 0,111648116 |
| miR.558 + miR.609 + miR.603 + miR.220a + miR.519b + miR.26a + miR.29a + miR.376a* + miR.494 + miR.518c + miR.639 + miR.660 | 0,81 | 0,042901913 |
| miR.558 + miR.609 + miR.220a + miR.519b + miR.29a + miR.362 + miR.369.3p + miR.376a* + miR.518c + miR.639 + miR.660 + miR.338 | 0,81 | 0,112734324 |
| miR.558 + miR.609 + miR.520f + miR.220a + miR.26a + miR.29a + miR.362 + miR.369.3p + miR.376a* + miR.494 + miR.518c + miR.338 | 0,81 | 0,06594461 |
| miR.558 + miR.609 + miR.520f + miR.220a + miR.519b + miR.26a + miR.29a + miR.369.3p + miR.376a* + miR.518c + miR.639 + miR.660 | 0,81 | 0,120222111 |
| miR.558 + miR.609 + miR.520f + miR.220a + miR.29a + miR.362 + miR.376a* + miR.494 + miR.518c + miR.639 + miR.660 + miR.338 | 0,81 | 0,006393723 |
| miR.558 + miR.609 + miR.520f + miR.220a + miR.130a + miR.29a + miR.376a* + miR.494 + miR.518c + miR.639 + miR.660 + miR.338 | 0,81 | 0,018470788 |
| miR.558 + miR.609 + miR.603 + miR.520f + miR.220a + miR.519b + miR.29a + miR.376a* + miR.494 + miR.518c + miR.639 + miR.338 | 0,81 | 0,019428291 |
| miR.558 + miR.609 + miR.603 + miR.520f + miR.220a + miR.130a + miR.29a + miR.376a* + miR.494 + miR.518c + miR.639 + miR.660 | 0,81 | 0,01301409 |
| miR.558 + miR.609 + miR.603 + miR.520f + miR.220a + miR.26a + miR.29a + miR.362 + miR.376a* + miR.518c + miR.639 + miR.338 | 0,81 | 0,013746926 |
| miR.558 + miR.609 + miR.603 + miR.520f + miR.220a + miR.519b + miR.130a + miR.26a + miR.29a + miR.362 + miR.376a* + miR.518c | 0,81 | 0,074476753 |
| miR.558 + miR.609 + miR.603 + miR.520f + miR.220a + miR.519b + miR.26a + miR.29a + miR.362 + miR.376a* + miR.518c + miR.338 | 0,81 | 0,032386546 |
| miR.558 + miR.609 + miR.603 + miR.520f + miR.220a + miR.519b + miR.26a + miR.29a + miR.376a* + miR.494 + miR.518c + miR.338 | 0,81 | 0,030468916 |
| miR.558 + miR.609 + miR.520f + miR.220a + miR.26a + miR.29a + miR.369.3p + miR.376a* + miR.494 + miR.518c + miR.639 + miR.338 | 0,81 | 0,079528316 |
| miR.558 + miR.609 + miR.603 + miR.520f + miR.220a + miR.519b + miR.130a + miR.29a + miR.362 + miR.376a* + miR.518c + miR.639 | 0,81 | 0,063065268 |
| miR.558 + miR.609 + miR.220a + miR.519b + miR.130a + miR.29a + miR.362 + miR.369.3p + miR.376a* + miR.518c + miR.639 + miR.660 | 0,81 | 0,18351129 |
| miR.609 + miR.603 + miR.520f + miR.29a + miR.362 + miR.369.3p + miR.376a* + miR.494 + miR.518c + miR.639 + miR.660 + miR.338 | 0,81 | 0,265674832 |
| miR.558 + miR.609 + miR.603 + miR.520f + miR.220a + miR.26a + miR.29a + miR.376a* + miR.494 + miR.518c + miR.639 + miR.660 | 0,81 | 0,01544211 |
| miR.558 + miR.609 + miR.603 + miR.519b + miR.130a + miR.26a + miR.29a + miR.369.3p + miR.376a* + miR.518c + miR.639 + miR.338 | 0,81 | 0,124208435 |
| miR.558 + miR.609 + miR.603 + miR.519b + miR.26a + miR.29a + miR.362 + miR.369.3p + miR.376a* + miR.518c + miR.639 + miR.660 | 0,81 | 0,045304156 |
| miR.558 + miR.609 + miR.603 + miR.520f + miR.220a + miR.26a + miR.29a + miR.376a* + miR.494 + miR.518c + miR.660 + miR.338 | 0,81 | 0,026751435 |
| miR.558 + miR.609 + miR.603 + miR.520f + miR.519b + miR.130a + miR.29a + miR.369.3p + miR.376a* + miR.518c + miR.639 + miR.338 | 0,81 | 0,055350253 |
| miR.558 + miR.609 + miR.520f + miR.220a + miR.519b + miR.29a + miR.376a* + miR.494 + miR.518c + miR.639 + miR.660 + miR.338 | 0,81 | 0,082900428 |
| miR.558 + miR.609 + miR.603 + miR.520f + miR.220a + miR.29a + miR.362 + miR.369.3p + miR.376a* + miR.494 + miR.518c + miR.639 | 0,81 | 0,012420108 |
| miR.558 + miR.609 + miR.603 + miR.220a + miR.519b + miR.130a + miR.29a + miR.376a* + miR.494 + miR.518c + miR.639 + miR.338 | 0,81 | 0,028845391 |
| miR.558 + miR.609 + miR.603 + miR.520f + miR.220a + miR.130a + miR.29a + miR.369.3p + miR.376a* + miR.494 + miR.518c + miR.338 | 0,81 | 0,021174594 |
| miR.558 + miR.609 + miR.220a + miR.519b + miR.130a + miR.26a + miR.29a + miR.369.3p + miR.376a* + miR.518c + miR.639 + miR.660 | 0,81 | 0,176861305 |
| miR.558 + miR.609 + miR.603 + miR.519b + miR.130a + miR.29a + miR.369.3p + miR.376a* + miR.494 + miR.518c + miR.639 + miR.338 | 0,81 | 0,124043279 |
| miR.558 + miR.609 + miR.603 + miR.520f + miR.130a + miR.26a + miR.29a + miR.369.3p + miR.376a* + miR.494 + miR.518c + miR.338 | 0,81 | 0,029378212 |
| miR.558 + miR.609 + miR.603 + miR.520f + miR.220a + miR.26a + miR.29a + miR.376a* + miR.518c + miR.639 + miR.660 + miR.338 | 0,81 | 0,011053221 |
| miR.558 + miR.609 + miR.603 + miR.520f + miR.220a + miR.519b + miR.130a + miR.26a + miR.29a + miR.376a* + miR.494 + miR.518c | 0,81 | 0,043274351 |
| miR.558 + miR.609 + miR.519b + miR.130a + miR.26a + miR.29a + miR.362 + miR.369.3p + miR.376a* + miR.518c + miR.639 + miR.660 | 0,81 | 0,074341835 |
| miR.558 + miR.609 + miR.603 + miR.220a + miR.130a + miR.29a + miR.362 + miR.369.3p + miR.376a* + miR.494 + miR.518c + miR.338 | 0,81 | 0,016252215 |
| miR.558 + miR.609 + miR.603 + miR.520f + miR.220a + miR.519b + miR.130a + miR.29a + miR.369.3p + miR.376a* + miR.518c + miR.639 | 0,81 | 0,058328568 |
| miR.558 + miR.609 + miR.520f + miR.220a + miR.26a + miR.29a + miR.362 + miR.369.3p + miR.376a* + miR.518c + miR.639 + miR.338 | 0,81 | 0,071939318 |
| miR.558 + miR.609 + miR.603 + miR.519b + miR.29a + miR.362 + miR.369.3p + miR.376a* + miR.518c + miR.639 + miR.660 + miR.338 | 0,81 | 0,051451185 |
| miR.558 + miR.609 + miR.603 + miR.520f + miR.26a + miR.29a + miR.362 + miR.369.3p + miR.376a* + miR.494 + miR.518c + miR.338 | 0,81 | 0,025888854 |
| miR.558 + miR.609 + miR.603 + miR.520f + miR.220a + miR.519b + miR.26a + miR.29a + miR.362 + miR.376a* + miR.518c + miR.660 | 0,81 | 0,069083284 |
| miR.558 + miR.609 + miR.603 + miR.220a + miR.519b + miR.26a + miR.29a + miR.362 + miR.376a* + miR.518c + miR.639 + miR.338 | 0,81 | 0,042037969 |
| miR.558 + miR.609 + miR.220a + miR.519b + miR.130a + miR.29a + miR.362 + miR.369.3p + miR.376a* + miR.518c + miR.660 + miR.338 | 0,81 | 0,174025913 |
| miR.558 + miR.609 + miR.603 + miR.520f + miR.519b + miR.29a + miR.376a* + miR.494 + miR.518c + miR.639 + miR.660 + miR.338 | 0,81 | 0,015928597 |
| miR.558 + miR.609 + miR.603 + miR.220a + miR.519b + miR.130a + miR.26a + miR.29a + miR.362 + miR.376a* + miR.518c + miR.338 | 0,81 | 0,042037969 |
| miR.558 + miR.609 + miR.603 + miR.130a + miR.29a + miR.369.3p + miR.376a* + miR.494 + miR.518c + miR.639 + miR.660 + miR.338 | 0,81 | 0,080313155 |
| miR.558 + miR.609 + miR.220a + miR.130a + miR.26a + miR.29a + miR.362 + miR.369.3p + miR.376a* + miR.518c + miR.639 + miR.660 | 0,81 | 0,229477513 |
| miR.558 + miR.609 + miR.603 + miR.520f + miR.220a + miR.130a + miR.29a + miR.376a* + miR.518c + miR.639 + miR.660 + miR.338 | 0,81 | 0,008487668 |
| miR.558 + miR.609 + miR.520f + miR.220a + miR.29a + miR.362 + miR.369.3p + miR.376a* + miR.494 + miR.518c + miR.639 + miR.338 | 0,81 | 0,017076293 |
| miR.558 + miR.609 + miR.603 + miR.520f + miR.220a + miR.519b + miR.130a + miR.29a + miR.376a* + miR.494 + miR.518c + miR.639 | 0,81 | 0,039708588 |
| miR.558 + miR.609 + miR.220a + miR.519b + miR.130a + miR.26a + miR.29a + miR.369.3p + miR.376a* + miR.518c + miR.660 + miR.338 | 0,81 | 0,13385948 |
| miR.558 + miR.609 + miR.220a + miR.519b + miR.130a + miR.29a + miR.369.3p + miR.376a* + miR.494 + miR.518c + miR.639 + miR.660 | 0,81 | 0,17654863 |
| miR.558 + miR.609 + miR.520f + miR.220a + miR.26a + miR.29a + miR.376a* + miR.494 + miR.518c + miR.639 + miR.660 + miR.338 | 0,81 | 0,01373943 |
| miR.558 + miR.609 + miR.130a + miR.26a + miR.29a + miR.369.3p + miR.376a* + miR.494 + miR.518c + miR.639 + miR.660 + miR.338 | 0,81 | 0,136137339 |
| miR.558 + miR.609 + miR.520f + miR.220a + miR.130a + miR.26a + miR.29a + miR.362 + miR.369.3p + miR.376a* + miR.518c + miR.338 | 0,81 | 0,059078533 |
| miR.558 + miR.609 + miR.520f + miR.130a + miR.26a + miR.29a + miR.369.3p + miR.376a* + miR.494 + miR.518c + miR.639 + miR.338 | 0,81 | 0,150656465 |
| miR.558 + miR.609 + miR.520f + miR.220a + miR.519b + miR.130a + miR.29a + miR.362 + miR.369.3p + miR.376a* + miR.518c + miR.660 | 0,81 | 0,194891454 |
| miR.558 + miR.609 + miR.603 + miR.220a + miR.130a + miR.26a + miR.29a + miR.362 + miR.376a* + miR.494 + miR.518c + miR.338 | 0,81 | 0,036858595 |
| miR.609 + miR.603 + miR.520f + miR.220a + miR.29a + miR.362 + miR.369.3p + miR.376a* + miR.518c + miR.639 + miR.660 + miR.338 | 0,81 | 0,28878047 |
| miR.558 + miR.609 + miR.603 + miR.520f + miR.220a + miR.519b + miR.29a + miR.362 + miR.376a* + miR.518c + miR.639 + miR.660 | 0,81 | 0,063699233 |
| miR.558 + miR.609 + miR.603 + miR.520f + miR.220a + miR.29a + miR.362 + miR.376a* + miR.518c + miR.639 + miR.660 + miR.338 | 0,81 | 0,01008843 |
| miR.558 + miR.609 + miR.603 + miR.520f + miR.220a + miR.130a + miR.29a + miR.362 + miR.376a* + miR.518c + miR.639 + miR.338 | 0,81 | 0,011132006 |
| miR.558 + miR.609 + miR.520f + miR.220a + miR.519b + miR.26a + miR.29a + miR.369.3p + miR.376a* + miR.494 + miR.518c + miR.338 | 0,81 | 0,108702048 |
| miR.558 + miR.609 + miR.220a + miR.519b + miR.29a + miR.362 + miR.369.3p + miR.376a* + miR.494 + miR.518c + miR.660 + miR.338 | 0,81 | 0,135216814 |
| miR.558 + miR.609 + miR.603 + miR.130a + miR.26a + miR.29a + miR.362 + miR.369.3p + miR.376a* + miR.518c + miR.639 + miR.660 | 0,81 | 0,060174887 |
| miR.558 + miR.609 + miR.603 + miR.520f + miR.220a + miR.130a + miR.29a + miR.376a* + miR.494 + miR.518c + miR.660 + miR.338 | 0,81 | 0,012907975 |
| miR.558 + miR.609 + miR.603 + miR.520f + miR.220a + miR.519b + miR.26a + miR.29a + miR.376a* + miR.494 + miR.518c + miR.639 | 0,81 | 0,035766004 |
| miR.558 + miR.609 + miR.603 + miR.519b + miR.130a + miR.26a + miR.29a + miR.369.3p + miR.376a* + miR.494 + miR.518c + miR.639 | 0,81 | 0,12980033 |
| miR.558 + miR.609 + miR.520f + miR.220a + miR.519b + miR.29a + miR.362 + miR.369.3p + miR.376a* + miR.494 + miR.518c + miR.338 | 0,81 | 0,110800804 |
| miR.558 + miR.609 + miR.603 + miR.520f + miR.220a + miR.29a + miR.362 + miR.376a* + miR.494 + miR.518c + miR.639 + miR.660 | 0,81 | 0,012330843 |
| miR.558 + miR.609 + miR.603 + miR.520f + miR.220a + miR.29a + miR.362 + miR.376a* + miR.494 + miR.518c + miR.660 + miR.338 | 0,81 | 0,019484589 |
| miR.558 + miR.609 + miR.603 + miR.520f + miR.220a + miR.519b + miR.26a + miR.29a + miR.376a* + miR.518c + miR.660 + miR.338 | 0,81 | 0,031636068 |
| miR.558 + miR.609 + miR.520f + miR.130a + miR.26a + miR.29a + miR.376a* + miR.494 + miR.518c + miR.639 + miR.660 + miR.338 | 0,81 | 0,03241325 |
| miR.558 + miR.609 + miR.220a + miR.130a + miR.29a + miR.369.3p + miR.376a* + miR.494 + miR.518c + miR.639 + miR.660 + miR.338 | 0,81 | 0,142320328 |
| miR.558 + miR.609 + miR.520f + miR.519b + miR.29a + miR.362 + miR.376a* + miR.494 + miR.518c + miR.639 + miR.660 + miR.338 | 0,81 | 0,029351233 |
| miR.558 + miR.609 + miR.603 + miR.520f + miR.220a + miR.130a + miR.26a + miR.29a + miR.362 + miR.376a* + miR.518c + miR.639 | 0,81 | 0,015167093 |
| miR.558 + miR.609 + miR.603 + miR.520f + miR.220a + miR.26a + miR.29a + miR.362 + miR.376a* + miR.494 + miR.518c + miR.338 | 0,81 | 0,016209377 |
| miR.558 + miR.609 + miR.603 + miR.520f + miR.519b + miR.29a + miR.362 + miR.369.3p + miR.376a* + miR.518c + miR.639 + miR.660 | 0,81 | 0,001469839 |
| miR.558 + miR.609 + miR.220a + miR.519b + miR.130a + miR.29a + miR.369.3p + miR.376a* + miR.494 + miR.518c + miR.660 + miR.338 | 0,81 | 0,121695094 |
| miR.558 + miR.609 + miR.603 + miR.520f + miR.220a + miR.362 + miR.369.3p + miR.376a* + miR.518c + miR.639 + miR.660 + miR.338 | 0,81 | 0,137494535 |
| miR.558 + miR.609 + miR.603 + miR.520f + miR.220a + miR.519b + miR.130a + miR.29a + miR.362 + miR.376a* + miR.518c + miR.660 | 0,81 | 0,065356793 |
| miR.558 + miR.609 + miR.603 + miR.520f + miR.220a + miR.26a + miR.29a + miR.362 + miR.376a* + miR.494 + miR.518c + miR.639 | 0,81 | 0,011836102 |
| miR.558 + miR.609 + miR.603 + miR.519b + miR.130a + miR.29a + miR.362 + miR.369.3p + miR.376a* + miR.518c + miR.639 + miR.660 | 0,81 | 0,068256135 |
| miR.558 + miR.609 + miR.520f + miR.519b + miR.130a + miR.29a + miR.369.3p + miR.376a* + miR.494 + miR.518c + miR.639 + miR.660 | 0,81 | 0,145298664 |
| miR.558 + miR.609 + miR.603 + miR.220a + miR.26a + miR.29a + miR.362 + miR.376a* + miR.494 + miR.518c + miR.639 + miR.660 | 0,81 | 0,022531502 |
| miR.558 + miR.609 + miR.520f + miR.519b + miR.130a + miR.29a + miR.376a* + miR.494 + miR.518c + miR.639 + miR.660 + miR.338 | 0,81 | 0,092287149 |
| miR.558 + miR.609 + miR.520f + miR.220a + miR.130a + miR.26a + miR.29a + miR.369.3p + miR.376a* + miR.494 + miR.518c + miR.338 | 0,81 | 0,062383315 |
| miR.558 + miR.609 + miR.520f + miR.220a + miR.130a + miR.29a + miR.369.3p + miR.376a* + miR.494 + miR.518c + miR.639 + miR.338 | 0,81 | 0,018346144 |
| miR.558 + miR.609 + miR.603 + miR.220a + miR.519b + miR.26a + miR.29a + miR.362 + miR.376a* + miR.518c + miR.660 + miR.338 | 0,81 | 0,046966269 |
| miR.609 + miR.603 + miR.520f + miR.220a + miR.29a + miR.362 + miR.369.3p + miR.376a* + miR.494 + miR.518c + miR.660 + miR.338 | 0,81 | 0,459369718 |
| miR.558 + miR.609 + miR.603 + miR.520f + miR.29a + miR.362 + miR.376a* + miR.494 + miR.518c + miR.639 + miR.660 + miR.338 | 0,81 | 0,00453373 |
| miR.558 + miR.609 + miR.603 + miR.220a + miR.519b + miR.130a + miR.26a + miR.29a + miR.362 + miR.376a* + miR.518c + miR.639 | 0,81 | 0,059953662 |
| miR.558 + miR.609 + miR.603 + miR.520f + miR.519b + miR.130a + miR.26a + miR.29a + miR.369.3p + miR.376a* + miR.518c + miR.338 | 0,81 | 0,046046847 |
| miR.558 + miR.609 + miR.603 + miR.220a + miR.519b + miR.29a + miR.362 + miR.369.3p + miR.376a* + miR.494 + miR.518c + miR.338 | 0,81 | 0,060805084 |
| miR.558 + miR.609 + miR.603 + miR.519b + miR.130a + miR.29a + miR.369.3p + miR.376a* + miR.494 + miR.518c + miR.639 + miR.660 | 0,81 | 0,084343115 |
| miR.558 + miR.609 + miR.603 + miR.520f + miR.519b + miR.130a + miR.29a + miR.369.3p + miR.376a* + miR.494 + miR.518c + miR.639 | 0,81 | 0,055157305 |
| miR.558 + miR.609 + miR.603 + miR.220a + miR.519b + miR.26a + miR.29a + miR.376a* + miR.494 + miR.518c + miR.639 + miR.338 | 0,81 | 0,041939507 |
| miR.558 + miR.609 + miR.520f + miR.220a + miR.519b + miR.29a + miR.362 + miR.376a* + miR.494 + miR.518c + miR.660 + miR.338 | 0,81 | 0,131488636 |
| miR.558 + miR.609 + miR.603 + miR.520f + miR.130a + miR.29a + miR.362 + miR.369.3p + miR.376a* + miR.494 + miR.518c + miR.338 | 0,81 | 0,015459265 |
| miR.558 + miR.609 + miR.603 + miR.520f + miR.130a + miR.26a + miR.29a + miR.369.3p + miR.376a* + miR.518c + miR.639 + miR.660 | 0,81 | 0,042859862 |
| miR.558 + miR.609 + miR.520f + miR.220a + miR.130a + miR.26a + miR.29a + miR.369.3p + miR.376a* + miR.518c + miR.639 + miR.338 | 0,81 | 0,065284809 |
| miR.558 + miR.609 + miR.520f + miR.26a + miR.29a + miR.362 + miR.376a* + miR.494 + miR.518c + miR.639 + miR.660 + miR.338 | 0,81 | 0,008497078 |
| miR.558 + miR.609 + miR.520f + miR.519b + miR.26a + miR.29a + miR.369.3p + miR.376a* + miR.494 + miR.518c + miR.660 + miR.338 | 0,81 | 0,197398853 |
| miR.558 + miR.609 + miR.603 + miR.520f + miR.220a + miR.519b + miR.29a + miR.376a* + miR.518c + miR.639 + miR.660 + miR.338 | 0,81 | 0,022481601 |
| miR.558 + miR.609 + miR.603 + miR.520f + miR.130a + miR.26a + miR.29a + miR.362 + miR.369.3p + miR.376a* + miR.518c + miR.338 | 0,81 | 0,02748033 |
| miR.558 + miR.609 + miR.603 + miR.520f + miR.220a + miR.130a + miR.29a + miR.362 + miR.376a* + miR.494 + miR.518c + miR.639 | 0,81 | 0,014566377 |
| miR.558 + miR.609 + miR.220a + miR.130a + miR.26a + miR.29a + miR.369.3p + miR.376a* + miR.494 + miR.518c + miR.639 + miR.338 | 0,81 | 0,160453303 |
| miR.609 + miR.603 + miR.520f + miR.220a + miR.519b + miR.29a + miR.369.3p + miR.376a* + miR.494 + miR.518c + miR.660 + miR.338 | 0,81 | 0,073323357 |
| miR.558 + miR.609 + miR.603 + miR.220a + miR.519b + miR.130a + miR.26a + miR.29a + miR.376a* + miR.518c + miR.660 + miR.338 | 0,81 | 0,033961652 |
| miR.558 + miR.609 + miR.520f + miR.220a + miR.519b + miR.130a + miR.29a + miR.376a* + miR.494 + miR.518c + miR.660 + miR.338 | 0,81 | 0,151794101 |
| miR.558 + miR.609 + miR.603 + miR.220a + miR.130a + miR.26a + miR.29a + miR.376a* + miR.494 + miR.518c + miR.639 + miR.338 | 0,81 | 0,011945282 |
| miR.558 + miR.609 + miR.520f + miR.220a + miR.519b + miR.26a + miR.29a + miR.376a* + miR.494 + miR.518c + miR.660 + miR.338 | 0,81 | 0,122443803 |
| miR.558 + miR.609 + miR.603 + miR.520f + miR.220a + miR.26a + miR.29a + miR.362 + miR.376a* + miR.518c + miR.639 + miR.660 | 0,81 | 0,02084666 |
| miR.558 + miR.609 + miR.603 + miR.26a + miR.29a + miR.362 + miR.369.3p + miR.376a* + miR.494 + miR.518c + miR.639 + miR.338 | 0,81 | 0,1059354 |
| miR.558 + miR.609 + miR.603 + miR.520f + miR.519b + miR.29a + miR.369.3p + miR.376a* + miR.494 + miR.518c + miR.639 + miR.660 | 0,81 | 0,017721516 |
| miR.558 + miR.609 + miR.520f + miR.220a + miR.519b + miR.130a + miR.26a + miR.29a + miR.369.3p + miR.376a* + miR.518c + miR.660 | 0,81 | 0,145828771 |
| miR.558 + miR.609 + miR.603 + miR.220a + miR.130a + miR.26a + miR.29a + miR.376a* + miR.494 + miR.518c + miR.660 + miR.338 | 0,81 | 0,025582821 |
| miR.558 + miR.609 + miR.603 + miR.520f + miR.220a + miR.130a + miR.26a + miR.29a + miR.362 + miR.376a* + miR.518c + miR.338 | 0,81 | 0,019232568 |
| miR.558 + miR.609 + miR.520f + miR.220a + miR.130a + miR.29a + miR.362 + miR.369.3p + miR.376a* + miR.494 + miR.518c + miR.338 | 0,81 | 0,012884269 |
| miR.558 + miR.609 + miR.220a + miR.519b + miR.130a + miR.26a + miR.29a + miR.362 + miR.369.3p + miR.376a* + miR.518c + miR.660 | 0,81 | 0,208274507 |
| miR.558 + miR.609 + miR.603 + miR.520f + miR.130a + miR.26a + miR.29a + miR.369.3p + miR.376a* + miR.494 + miR.518c + miR.639 | 0,81 | 0,046115075 |
| miR.558 + miR.609 + miR.603 + miR.220a + miR.519b + miR.26a + miR.29a + miR.376a* + miR.518c + miR.639 + miR.660 + miR.338 | 0,81 | 0,031320253 |
| miR.609 + miR.603 + miR.520f + miR.220a + miR.29a + miR.362 + miR.369.3p + miR.376a* + miR.494 + miR.518c + miR.639 + miR.660 | 0,81 | 0,050804025 |
| miR.558 + miR.609 + miR.603 + miR.520f + miR.130a + miR.29a + miR.376a* + miR.494 + miR.518c + miR.639 + miR.660 + miR.338 | 0,81 | 0,010407148 |
| miR.558 + miR.609 + miR.603 + miR.130a + miR.26a + miR.29a + miR.362 + miR.369.3p + miR.376a* + miR.518c + miR.639 + miR.338 | 0,81 | 0,169599323 |
| miR.558 + miR.609 + miR.603 + miR.520f + miR.26a + miR.29a + miR.376a* + miR.494 + miR.518c + miR.639 + miR.660 + miR.338 | 0,81 | 0,007266293 |
| miR.558 + miR.609 + miR.220a + miR.519b + miR.130a + miR.26a + miR.29a + miR.369.3p + miR.376a* + miR.494 + miR.518c + miR.338 | 0,81 | 0,102180906 |
| miR.558 + miR.609 + miR.603 + miR.520f + miR.220a + miR.519b + miR.26a + miR.29a + miR.376a* + miR.518c + miR.639 + miR.660 | 0,81 | 0,056788287 |
| miR.558 + miR.609 + miR.520f + miR.220a + miR.519b + miR.26a + miR.29a + miR.362 + miR.369.3p + miR.376a* + miR.494 + miR.518c | 0,81 | 0,091913791 |
| miR.558 + miR.609 + miR.603 + miR.220a + miR.519b + miR.26a + miR.29a + miR.362 + miR.376a* + miR.518c + miR.639 + miR.660 | 0,81 | 0,065859447 |
| miR.558 + miR.609 + miR.520f + miR.220a + miR.26a + miR.29a + miR.362 + miR.369.3p + miR.376a* + miR.494 + miR.518c + miR.639 | 0,81 | 0,037019274 |
| miR.558 + miR.609 + miR.603 + miR.520f + miR.220a + miR.130a + miR.26a + miR.29a + miR.376a* + miR.518c + miR.660 + miR.338 | 0,81 | 0,012673271 |
| miR.558 + miR.609 + miR.520f + miR.220a + miR.519b + miR.130a + miR.29a + miR.369.3p + miR.376a* + miR.494 + miR.518c + miR.338 | 0,81 | 0,084575545 |
| miR.558 + miR.609 + miR.603 + miR.220a + miR.26a + miR.29a + miR.362 + miR.376a* + miR.494 + miR.518c + miR.660 + miR.338 | 0,81 | 0,024314979 |
| miR.558 + miR.609 + miR.603 + miR.520f + miR.220a + miR.519b + miR.130a + miR.29a + miR.376a* + miR.518c + miR.639 + miR.660 | 0,81 | 0,053034712 |
| miR.558 + miR.609 + miR.520f + miR.220a + miR.519b + miR.130a + miR.29a + miR.362 + miR.376a* + miR.494 + miR.518c + miR.338 | 0,81 | 0,105246147 |
| miR.558 + miR.609 + miR.603 + miR.520f + miR.220a + miR.519b + miR.130a + miR.26a + miR.29a + miR.376a* + miR.518c + miR.660 | 0,81 | 0,055184389 |
| miR.558 + miR.609 + miR.603 + miR.520f + miR.220a + miR.26a + miR.29a + miR.362 + miR.376a* + miR.494 + miR.518c + miR.660 | 0,81 | 0,021839451 |
| miR.558 + miR.609 + miR.603 + miR.520f + miR.220a + miR.130a + miR.26a + miR.29a + miR.376a* + miR.518c + miR.639 + miR.660 | 0,81 | 0,013455972 |
| miR.558 + miR.609 + miR.220a + miR.519b + miR.26a + miR.29a + miR.369.3p + miR.376a* + miR.494 + miR.518c + miR.639 + miR.338 | 0,81 | 0,107983349 |
| miR.558 + miR.609 + miR.603 + miR.520f + miR.519b + miR.130a + miR.29a + miR.362 + miR.369.3p + miR.376a* + miR.518c + miR.338 | 0,81 | 0,036866114 |
| miR.558 + miR.609 + miR.603 + miR.520f + miR.220a + miR.130a + miR.26a + miR.29a + miR.376a* + miR.494 + miR.518c + miR.660 | 0,81 | 0,018768361 |
| miR.558 + miR.609 + miR.520f + miR.220a + miR.519b + miR.29a + miR.369.3p + miR.376a* + miR.494 + miR.518c + miR.639 + miR.338 | 0,81 | 0,06726191 |
| miR.558 + miR.609 + miR.603 + miR.220a + miR.519b + miR.130a + miR.26a + miR.29a + miR.362 + miR.376a* + miR.518c + miR.660 | 0,81 | 0,065863144 |
| miR.558 + miR.609 + miR.220a + miR.130a + miR.26a + miR.29a + miR.362 + miR.369.3p + miR.376a* + miR.518c + miR.639 + miR.338 | 0,81 | 0,073562011 |
| miR.558 + miR.609 + miR.520f + miR.220a + miR.519b + miR.29a + miR.362 + miR.376a* + miR.494 + miR.518c + miR.639 + miR.338 | 0,81 | 0,082843943 |
| miR.558 + miR.609 + miR.220a + miR.519b + miR.29a + miR.362 + miR.369.3p + miR.376a* + miR.494 + miR.518c + miR.639 + miR.660 | 0,81 | 0,096588718 |
| miR.558 + miR.609 + miR.220a + miR.26a + miR.29a + miR.362 + miR.369.3p + miR.376a* + miR.494 + miR.518c + miR.639 + miR.338 | 0,81 | 0,08833119 |
| miR.558 + miR.609 + miR.603 + miR.520f + miR.220a + miR.519b + miR.130a + miR.29a + miR.376a* + miR.518c + miR.660 + miR.338 | 0,81 | 0,020873253 |
| miR.558 + miR.609 + miR.603 + miR.520f + miR.519b + miR.26a + miR.29a + miR.362 + miR.369.3p + miR.376a* + miR.518c + miR.338 | 0,81 | 0,05918306 |
| miR.609 + miR.603 + miR.220a + miR.26a + miR.29a + miR.362 + miR.369.3p + miR.376a* + miR.494 + miR.518c + miR.639 + miR.660 | 0,81 | 0,088589459 |
| miR.558 + miR.609 + miR.603 + miR.520f + miR.26a + miR.29a + miR.362 + miR.369.3p + miR.376a* + miR.494 + miR.518c + miR.660 | 0,81 | 0,015296836 |
| miR.558 + miR.609 + miR.603 + miR.220a + miR.130a + miR.29a + miR.369.3p + miR.376a* + miR.494 + miR.518c + miR.639 + miR.338 | 0,81 | 0,029482207 |
| miR.558 + miR.609 + miR.603 + miR.519b + miR.26a + miR.29a + miR.362 + miR.369.3p + miR.376a* + miR.518c + miR.639 + miR.338 | 0,81 | 0,150802321 |
| miR.558 + miR.609 + miR.603 + miR.220a + miR.130a + miR.29a + miR.362 + miR.369.3p + miR.376a* + miR.518c + miR.639 + miR.338 | 0,81 | 0,018974883 |
| miR.558 + miR.609 + miR.520f + miR.220a + miR.130a + miR.29a + miR.362 + miR.369.3p + miR.376a* + miR.518c + miR.639 + miR.338 | 0,81 | 0,023453589 |
| miR.558 + miR.609 + miR.520f + miR.220a + miR.519b + miR.26a + miR.29a + miR.362 + miR.369.3p + miR.376a* + miR.518c + miR.338 | 0,81 | 0,091880685 |
| miR.558 + miR.609 + miR.603 + miR.520f + miR.220a + miR.130a + miR.29a + miR.362 + miR.376a* + miR.494 + miR.518c + miR.338 | 0,81 | 0,008155178 |
| miR.558 + miR.609 + miR.603 + miR.220a + miR.130a + miR.26a + miR.29a + miR.362 + miR.376a* + miR.494 + miR.518c + miR.660 | 0,81 | 0,028393429 |
| miR.558 + miR.609 + miR.220a + miR.519b + miR.26a + miR.29a + miR.362 + miR.369.3p + miR.376a* + miR.494 + miR.518c + miR.338 | 0,81 | 0,106742132 |
| miR.558 + miR.609 + miR.603 + miR.220a + miR.130a + miR.26a + miR.29a + miR.376a* + miR.518c + miR.639 + miR.660 + miR.338 | 0,81 | 0,023293723 |
| miR.558 + miR.609 + miR.220a + miR.519b + miR.130a + miR.29a + miR.369.3p + miR.376a* + miR.518c + miR.639 + miR.660 + miR.338 | 0,81 | 0,160898556 |
| miR.558 + miR.609 + miR.603 + miR.519b + miR.26a + miR.29a + miR.369.3p + miR.376a* + miR.494 + miR.518c + miR.639 + miR.660 | 0,81 | 0,104359824 |
| miR.558 + miR.609 + miR.520f + miR.519b + miR.130a + miR.29a + miR.369.3p + miR.376a* + miR.494 + miR.518c + miR.639 + miR.338 | 0,81 | 0,150080424 |
| miR.558 + miR.609 + miR.603 + miR.220a + miR.519b + miR.29a + miR.362 + miR.376a* + miR.494 + miR.518c + miR.639 + miR.660 | 0,81 | 0,040242063 |
| miR.558 + miR.609 + miR.603 + miR.520f + miR.220a + miR.519b + miR.29a + miR.362 + miR.376a* + miR.518c + miR.660 + miR.338 | 0,81 | 0,033843126 |
| miR.558 + miR.609 + miR.220a + miR.130a + miR.26a + miR.29a + miR.362 + miR.369.3p + miR.376a* + miR.494 + miR.518c + miR.338 | 0,81 | 0,08263367 |
| miR.558 + miR.609 + miR.603 + miR.520f + miR.130a + miR.29a + miR.362 + miR.369.3p + miR.376a* + miR.518c + miR.639 + miR.660 | 0,81 | 0,009075302 |
| miR.558 + miR.609 + miR.603 + miR.130a + miR.26a + miR.29a + miR.362 + miR.369.3p + miR.376a* + miR.494 + miR.518c + miR.639 | 0,81 | 0,077297432 |
| miR.558 + miR.609 + miR.603 + miR.220a + miR.26a + miR.29a + miR.362 + miR.376a* + miR.518c + miR.639 + miR.660 + miR.338 | 0,81 | 0,026020114 |
| miR.558 + miR.609 + miR.520f + miR.130a + miR.26a + miR.29a + miR.369.3p + miR.376a* + miR.494 + miR.518c + miR.660 + miR.338 | 0,81 | 0,139163048 |
| miR.558 + miR.609 + miR.603 + miR.519b + miR.26a + miR.29a + miR.369.3p + miR.376a* + miR.494 + miR.518c + miR.639 + miR.338 | 0,81 | 0,107080588 |
| miR.558 + miR.609 + miR.520f + miR.519b + miR.130a + miR.29a + miR.362 + miR.369.3p + miR.376a* + miR.518c + miR.639 + miR.660 | 0,81 | 0,011583671 |
| miR.558 + miR.609 + miR.603 + miR.520f + miR.220a + miR.130a + miR.29a + miR.362 + miR.376a* + miR.518c + miR.660 + miR.338 | 0,81 | 0,008695689 |
| miR.558 + miR.609 + miR.603 + miR.220a + miR.519b + miR.29a + miR.362 + miR.376a* + miR.494 + miR.518c + miR.639 + miR.338 | 0,81 | 0,034361831 |
| miR.558 + miR.609 + miR.603 + miR.520f + miR.26a + miR.29a + miR.362 + miR.369.3p + miR.376a* + miR.494 + miR.518c + miR.639 | 0,81 | 0,058897126 |
| miR.558 + miR.609 + miR.220a + miR.519b + miR.130a + miR.26a + miR.29a + miR.369.3p + miR.376a* + miR.518c + miR.639 + miR.338 | 0,81 | 0,068326703 |
| miR.558 + miR.609 + miR.603 + miR.220a + miR.519b + miR.130a + miR.26a + miR.29a + miR.376a* + miR.518c + miR.639 + miR.338 | 0,81 | 0,024465061 |
| miR.558 + miR.609 + miR.603 + miR.520f + miR.519b + miR.26a + miR.29a + miR.369.3p + miR.376a* + miR.518c + miR.639 + miR.660 | 0,81 | 0,034232877 |
| miR.558 + miR.609 + miR.603 + miR.220a + miR.130a + miR.26a + miR.29a + miR.362 + miR.376a* + miR.518c + miR.660 + miR.338 | 0,81 | 0,028182389 |
| miR.558 + miR.609 + miR.603 + miR.520f + miR.220a + miR.26a + miR.29a + miR.362 + miR.376a* + miR.518c + miR.660 + miR.338 | 0,81 | 0,032196505 |
| miR.558 + miR.609 + miR.603 + miR.520f + miR.220a + miR.29a + miR.362 + miR.376a* + miR.494 + miR.518c + miR.639 + miR.338 | 0,81 | 0,010431776 |
| miR.558 + miR.609 + miR.520f + miR.220a + miR.130a + miR.26a + miR.29a + miR.362 + miR.369.3p + miR.376a* + miR.494 + miR.518c | 0,81 | 0,044108403 |
| miR.558 + miR.609 + miR.603 + miR.520f + miR.220a + miR.130a + miR.29a + miR.362 + miR.376a* + miR.494 + miR.518c + miR.660 | 0,81 | 0,01425374 |
| miR.558 + miR.609 + miR.603 + miR.519b + miR.130a + miR.26a + miR.29a + miR.369.3p + miR.376a* + miR.518c + miR.639 + miR.660 | 0,81 | 0,099730158 |
| miR.558 + miR.609 + miR.603 + miR.220a + miR.519b + miR.130a + miR.26a + miR.29a + miR.376a* + miR.518c + miR.639 + miR.660 | 0,81 | 0,051801339 |
| miR.558 + miR.609 + miR.520f + miR.220a + miR.519b + miR.130a + miR.29a + miR.362 + miR.369.3p + miR.376a* + miR.494 + miR.518c | 0,81 | 0,114057659 |
| miR.558 + miR.609 + miR.603 + miR.520f + miR.519b + miR.130a + miR.29a + miR.369.3p + miR.376a* + miR.518c + miR.639 + miR.660 | 0,81 | 0,036275635 |
| miR.558 + miR.609 + miR.603 + miR.520f + miR.519b + miR.29a + miR.362 + miR.369.3p + miR.376a* + miR.494 + miR.518c + miR.660 | 0,81 | 0,015514056 |
| miR.558 + miR.609 + miR.603 + miR.520f + miR.130a + miR.29a + miR.362 + miR.376a* + miR.494 + miR.518c + miR.639 + miR.338 | 0,81 | 0,013000463 |
| miR.558 + miR.609 + miR.220a + miR.519b + miR.130a + miR.26a + miR.29a + miR.362 + miR.369.3p + miR.376a* + miR.518c + miR.338 | 0,81 | 0,097029557 |
| miR.558 + miR.609 + miR.520f + miR.519b + miR.26a + miR.29a + miR.362 + miR.369.3p + miR.376a* + miR.518c + miR.639 + miR.660 | 0,81 | 0,009252758 |
| miR.558 + miR.609 + miR.603 + miR.520f + miR.519b + miR.130a + miR.29a + miR.376a* + miR.494 + miR.518c + miR.639 + miR.338 | 0,81 | 0,049176377 |
| miR.558 + miR.609 + miR.603 + miR.220a + miR.519b + miR.29a + miR.362 + miR.376a* + miR.494 + miR.518c + miR.660 + miR.338 | 0,81 | 0,028930724 |
| miR.558 + miR.609 + miR.603 + miR.520f + miR.220a + miR.130a + miR.26a + miR.29a + miR.362 + miR.376a* + miR.494 + miR.518c | 0,81 | 0,016122044 |
| miR.609 + miR.603 + miR.520f + miR.220a + miR.519b + miR.29a + miR.362 + miR.369.3p + miR.376a* + miR.494 + miR.518c + miR.660 | 0,81 | 0,081627784 |
| miR.558 + miR.609 + miR.520f + miR.26a + miR.29a + miR.362 + miR.369.3p + miR.376a* + miR.494 + miR.518c + miR.639 + miR.660 | 0,81 | 0,028677451 |
| miR.558 + miR.609 + miR.520f + miR.220a + miR.519b + miR.130a + miR.29a + miR.376a* + miR.494 + miR.518c + miR.639 + miR.660 | 0,81 | 0,046268632 |
| miR.558 + miR.609 + miR.520f + miR.220a + miR.519b + miR.26a + miR.29a + miR.369.3p + miR.376a* + miR.518c + miR.639 + miR.338 | 0,81 | 0,083714545 |
| miR.558 + miR.609 + miR.603 + miR.520f + miR.26a + miR.29a + miR.362 + miR.376a* + miR.494 + miR.518c + miR.639 + miR.338 | 0,81 | 0,014706726 |
| miR.558 + miR.609 + miR.603 + miR.520f + miR.220a + miR.519b + miR.130a + miR.26a + miR.29a + miR.376a* + miR.518c + miR.338 | 0,81 | 0,016802873 |
| miR.558 + miR.609 + miR.520f + miR.130a + miR.29a + miR.362 + miR.369.3p + miR.376a* + miR.494 + miR.639 + miR.660 + miR.338 | 0,81 | 0,326811823 |
| miR.558 + miR.609 + miR.603 + miR.520f + miR.130a + miR.29a + miR.362 + miR.369.3p + miR.376a* + miR.494 + miR.518c + miR.639 | 0,81 | 0,017844597 |
| miR.558 + miR.609 + miR.603 + miR.220a + miR.519b + miR.29a + miR.376a* + miR.494 + miR.518c + miR.639 + miR.660 + miR.338 | 0,81 | 0,022385025 |
| miR.558 + miR.609 + miR.520f + miR.220a + miR.130a + miR.26a + miR.29a + miR.369.3p + miR.376a* + miR.494 + miR.518c + miR.639 | 0,81 | 0,040945691 |
| miR.558 + miR.609 + miR.603 + miR.220a + miR.519b + miR.130a + miR.29a + miR.362 + miR.376a* + miR.518c + miR.639 + miR.338 | 0,81 | 0,028113842 |
| miR.558 + miR.609 + miR.603 + miR.220a + miR.130a + miR.26a + miR.29a + miR.362 + miR.376a* + miR.518c + miR.639 + miR.660 | 0,81 | 0,025666095 |
| miR.558 + miR.609 + miR.520f + miR.220a + miR.130a + miR.29a + miR.362 + miR.369.3p + miR.376a* + miR.494 + miR.518c + miR.639 | 0,81 | 0,015069342 |
| miR.558 + miR.609 + miR.603 + miR.519b + miR.130a + miR.26a + miR.29a + miR.369.3p + miR.376a* + miR.494 + miR.518c + miR.338 | 0,81 | 0,134016351 |
| miR.558 + miR.609 + miR.519b + miR.130a + miR.26a + miR.29a + miR.369.3p + miR.376a* + miR.518c + miR.639 + miR.660 + miR.338 | 0,81 | 0,264229673 |
| miR.558 + miR.609 + miR.603 + miR.519b + miR.26a + miR.29a + miR.362 + miR.369.3p + miR.376a* + miR.494 + miR.518c + miR.660 | 0,81 | 0,048621112 |
| miR.558 + miR.609 + miR.520f + miR.220a + miR.26a + miR.29a + miR.362 + miR.376a* + miR.494 + miR.518c + miR.660 + miR.338 | 0,81 | 0,024900079 |
| miR.558 + miR.609 + miR.603 + miR.520f + miR.220a + miR.519b + miR.130a + miR.29a + miR.376a* + miR.518c + miR.639 + miR.338 | 0,81 | 0,012540071 |
| miR.558 + miR.609 + miR.520f + miR.220a + miR.26a + miR.29a + miR.362 + miR.376a* + miR.518c + miR.639 + miR.660 + miR.338 | 0,81 | 0,014852528 |
| miR.609 + miR.603 + miR.220a + miR.519b + miR.26a + miR.29a + miR.362 + miR.369.3p + miR.376a* + miR.494 + miR.518c + miR.660 | 0,81 | 0,117838095 |
| miR.558 + miR.609 + miR.603 + miR.520f + miR.220a + miR.519b + miR.26a + miR.29a + miR.376a* + miR.518c + miR.639 + miR.338 | 0,81 | 0,019448369 |
| miR.558 + miR.609 + miR.520f + miR.220a + miR.519b + miR.29a + miR.362 + miR.369.3p + miR.376a* + miR.518c + miR.639 + miR.338 | 0,81 | 0,056690145 |
| miR.558 + miR.609 + miR.603 + miR.520f + miR.519b + miR.26a + miR.29a + miR.369.3p + miR.376a* + miR.494 + miR.518c + miR.660 | 0,81 | 0,057877174 |
| miR.558 + miR.609 + miR.603 + miR.220a + miR.130a + miR.29a + miR.362 + miR.376a* + miR.494 + miR.518c + miR.639 + miR.660 | 0,81 | 0,016410248 |
| miR.558 + miR.609 + miR.520f + miR.220a + miR.130a + miR.29a + miR.362 + miR.376a* + miR.494 + miR.518c + miR.660 + miR.338 | 0,81 | 0,026981964 |
| miR.558 + miR.609 + miR.603 + miR.520f + miR.130a + miR.362 + miR.369.3p + miR.376a* + miR.518c + miR.639 + miR.660 + miR.338 | 0,81 | 0,099572501 |
| miR.558 + miR.609 + miR.220a + miR.519b + miR.130a + miR.26a + miR.29a + miR.362 + miR.369.3p + miR.376a* + miR.494 + miR.518c | 0,81 | 0,113619614 |
| miR.558 + miR.609 + miR.520f + miR.220a + miR.130a + miR.29a + miR.362 + miR.376a* + miR.518c + miR.639 + miR.660 + miR.338 | 0,81 | 0,013267938 |
| miR.558 + miR.609 + miR.603 + miR.520f + miR.220a + miR.130a + miR.26a + miR.29a + miR.376a* + miR.494 + miR.518c + miR.639 | 0,81 | 0,012488873 |
| miR.558 + miR.609 + miR.603 + miR.130a + miR.26a + miR.29a + miR.369.3p + miR.376a* + miR.494 + miR.518c + miR.660 + miR.338 | 0,81 | 0,082804278 |
| miR.558 + miR.609 + miR.603 + miR.519b + miR.26a + miR.29a + miR.369.3p + miR.376a* + miR.494 + miR.518c + miR.660 + miR.338 | 0,81 | 0,107391331 |
| miR.558 + miR.609 + miR.220a + miR.130a + miR.26a + miR.29a + miR.362 + miR.369.3p + miR.376a* + miR.494 + miR.518c + miR.639 | 0,81 | 0,070220752 |
| miR.558 + miR.609 + miR.603 + miR.519b + miR.26a + miR.29a + miR.362 + miR.369.3p + miR.376a* + miR.494 + miR.518c + miR.338 | 0,81 | 0,112781487 |
| miR.558 + miR.609 + miR.519b + miR.26a + miR.29a + miR.362 + miR.369.3p + miR.376a* + miR.494 + miR.518c + miR.660 + miR.338 | 0,81 | 0,285336004 |

**Table 13: clusters of 13 miRNAs**

| **Cluster of 13 miRs** | **c_tau level** | **pv compared to top signature miR.558 + miR.609 + miR.520f + miR.130a + miR.29a + miR.362 + miR.369.3p + miR.376a* + miR.494 + miR.518c + miR.639 + miR.660 + miR.338** |
|---|---|---|
| miR.558 + miR.609 + miR.520f + miR.130a + miR.29a + miR.362 + miR.369.3p + miR.376a* + miR.494 + miR.518c + miR.639 + miR.660 + miR.338 | 0,82 | NaN |
| miR.558 + miR.609 + miR.603 + miR.520f + miR.220a + miR.29a + miR.362 + miR.369.3p + miR.376a* + miR.518c + miR.639 + miR.660 + miR.338 | 0,81 | 0,472941439 |
| miR.558 + miR.609 + miR.520f + miR.519b + miR.26a + miR.29a + miR.362 + miR.369.3p + miR.376a* + miR.518c + miR.639 + miR.660 + miR.338 | 0,81 | 0,445059025 |
| miR.558 + miR.609 + miR.520f + miR.220a + miR.29a + miR.362 + miR.369.3p + miR.376a* + miR.494 + miR.518c + miR.639 + miR.660 + miR.338 | 0,81 | 0,464030389 |
| miR.558 + miR.609 + miR.603 + miR.520f + miR.29a + miR.362 + miR.369.3p + miR.376a* + miR.494 + miR.518c + miR.639 + miR.660 + miR.338 | 0,81 | 0,272060897 |
| miR.558 + miR.609 + miR.603 + miR.220a + miR.26a + miR.29a + miR.362 + miR.369.3p + miR.376a* + miR.518c + miR.639 + miR.660 + miR.338 | 0,81 | 0,363975285 |
| miR.558 + miR.609 + miR.520f + miR.220a + miR.26a + miR.29a + miR.362 + miR.369.3p + miR.376a* + miR.518c + miR.639 + miR.660 + miR.338 | 0,81 | 0,447323743 |
| miR.558 + miR.609 + miR.520f + miR.519b + miR.130a + miR.29a + miR.362 + miR.369.3p + miR.376a* + miR.518c + miR.639 + miR.660 + miR.338 | 0,81 | 0,366518399 |
| miR.558 + miR.609 + miR.520f + miR.220a + miR.519b + miR.29a + miR.362 + miR.369.3p + miR.376a* + miR.518c + miR.639 + miR.660 + miR.338 | 0,81 | 0,314675009 |
| miR.558 + miR.609 + miR.603 + miR.520f + miR.26a + miR.29a + miR.362 + miR.369.3p + miR.376a* + miR.518c + miR.639 + miR.660 + miR.338 | 0,81 | 0,20443385 |
| miR.558 + miR.609 + miR.520f + miR.220a + miR.130a + miR.29a + miR.362 + miR.369.3p + miR.376a* + miR.518c + miR.639 + miR.660 + miR.338 | 0,81 | 0,424259484 |
| miR.558 + miR.609 + miR.603 + miR.520f + miR.130a + miR.29a + miR.362 + miR.369.3p + miR.376a* + miR.518c + miR.639 + miR.660 + miR.338 | 0,81 | 0,268558591 |
| miR.558 + miR.609 + miR.603 + miR.520f + miR.220a + miR.519b + miR.29a + miR.369.3p + miR.376a* + miR.494 + miR.518c + miR.660 + miR.338 | 0,81 | 0,193645248 |
| miR.558 + miR.609 + miR.603 + miR.520f + miR.519b + miR.29a + miR.362 + miR.369.3p + miR.376a* + miR.518c + miR.639 + miR.660 + miR.338 | 0,81 | 0,408551927 |
| miR.558 + miR.609 + miR.603 + miR.520f + miR.220a + miR.26a + miR.29a + miR.369.3p + miR.376a* + miR.518c + miR.639 + miR.660 + miR.338 | 0,81 | 0,164812906 |
| miR.558 + miR.609 + miR.520f + miR.130a + miR.26a + miR.29a + miR.362 + miR.369.3p + miR.376a* + miR.518c + miR.639 + miR.660 + miR.338 | 0,81 | 0,28390477 |
| miR.558 + miR.609 + miR.130a + miR.26a + miR.29a + miR.362 + miR.369.3p + miR.376a* + miR.494 + miR.518c + miR.639 + miR.660 + miR.338 | 0,81 | 0,287064031 |
| miR.558 + miR.609 + miR.603 + miR.220a + miR.26a + miR.29a + miR.362 + miR.369.3p + miR.376a* + miR.494 + miR.518c + miR.660 + miR.338 | 0,81 | 0,377718286 |
| miR.558 + miR.609 + miR.520f + miR.26a + miR.29a + miR.362 + miR.369.3p + miR.376a* + miR.494 + miR.518c + miR.639 + miR.660 + miR.338 | 0,81 | 0,495493491 |
| miR.558 + miR.609 + miR.603 + miR.220a + miR.26a + miR.29a + miR.362 + miR.369.3p + miR.376a* + miR.494 + miR.518c + miR.639 + miR.660 | 0,81 | 0,299726626 |
| miR.558 + miR.609 + miR.603 + miR.220a + miR.26a + miR.29a + miR.369.3p + miR.376a* + miR.494 + miR.518c + miR.639 + miR.660 + miR.338 | 0,81 | 0,129683152 |
| miR.558 + miR.609 + miR.603 + miR.520f + miR.220a + miR.519b + miR.29a + miR.362 + miR.369.3p + miR.376a* + miR.518c + miR.660 + miR.338 | 0,81 | 0,393237863 |
| miR.558 + miR.609 + miR.603 + miR.520f + miR.220a + miR.29a + miR.369.3p + miR.376a* + miR.494 + miR.518c + miR.639 + miR.660 + miR.338 | 0,81 | 0,09061259 |
| miR.558 + miR.609 + miR.603 + miR.520f + miR.220a + miR.29a + miR.362 + miR.369.3p + miR.376a* + miR.494 + miR.518c + miR.660 + miR.338 | 0,81 | 0,374257772 |
| miR.558 + miR.609 + miR.520f + miR.519b + miR.29a + miR.362 + miR.369.3p + miR.376a* + miR.494 + miR.518c + miR.639 + miR.660 + miR.338 | 0,81 | 0,428506317 |
| miR.558 + miR.609 + miR.603 + miR.520f + miR.220a + miR.26a + miR.29a + miR.362 + miR.369.3p + miR.376a* + miR.518c + miR.660 + miR.338 | 0,81 | 0,378482633 |
| miR.558 + miR.609 + miR.603 + miR.520f + miR.220a + miR.130a + miR.29a + miR.369.3p + miR.376a* + miR.518c + miR.639 + miR.660 + miR.338 | 0,81 | 0,154696043 |
| miR.558 + miR.609 + miR.603 + miR.220a + miR.130a + miR.29a + miR.362 + miR.369.3p + miR.376a* + miR.518c + miR.639 + miR.660 + miR.338 | 0,81 | 0,461459422 |
| miR.558 + miR.609 + miR.603 + miR.220a + miR.519b + miR.26a + miR.29a + miR.369.3p + miR.376a* + miR.494 + miR.518c + miR.660 + miR.338 | 0,81 | 0,131097854 |
| miR.558 + miR.609 + miR.603 + miR.520f + miR.220a + miR.26a + miR.29a + miR.369.3p + miR.376a* + miR.494 + miR.518c + miR.660 + miR.338 | 0,81 | 0,120540301 |
| miR.558 + miR.609 + miR.603 + miR.220a + miR.519b + miR.26a + miR.29a + miR.362 + miR.369.3p + miR.376a* + miR.494 + miR.518c + miR.660 | 0,81 | 0,23188802 |
| miR.558 + miR.609 + miR.603 + miR.220a + miR.519b + miR.26a + miR.29a + miR.362 + miR.369.3p + miR.376a* + miR.518c + miR.660 + miR.338 | 0,81 | 0,338325475 |
| miR.558 + miR.609 + miR.603 + miR.220a + miR.519b + miR.130a + miR.29a + miR.369.3p + miR.376a* + miR.494 + miR.518c + miR.660 + miR.338 | 0,81 | 0,115593707 |
| miR.558 + miR.609 + miR.603 + miR.220a + miR.130a + miR.26a + miR.29a + miR.362 + miR.369.3p + miR.376a* + miR.518c + miR.660 + miR.338 | 0,81 | 0,357504945 |
| miR.558 + miR.609 + miR.603 + miR.520f + miR.220a + miR.130a + miR.29a + miR.362 + miR.369.3p + miR.376a* + miR.518c + miR.660 + miR.338 | 0,81 | 0,404005572 |
| miR.558 + miR.609 + miR.603 + miR.26a + miR.29a + miR.362 + miR.369.3p + miR.376a* + miR.494 + miR.518c + miR.639 + miR.660 + miR.338 | 0,81 | 0,096770394 |
| miR.558 + miR.609 + miR.520f + miR.220a + miR.26a + miR.29a + miR.362 + miR.369.3p + miR.376a* + miR.494 + miR.518c + miR.660 + miR.338 | 0,81 | 0,447201342 |
| miR.558 + miR.609 + miR.603 + miR.520f + miR.220a + miR.519b + miR.29a + miR.369.3p + miR.376a* + miR.518c + miR.639 + miR.660 + miR.338 | 0,81 | 0,214286829 |
| miR.558 + miR.609 + miR.603 + miR.130a + miR.29a + miR.362 + miR.369.3p + miR.376a* + miR.494 + miR.518c + miR.639 + miR.660 + miR.338 | 0,81 | 0,298233807 |
| miR.558 + miR.609 + miR.603 + miR.520f + miR.220a + miR.519b + miR.26a + miR.29a + miR.369.3p + miR.376a* + miR.494 + miR.518c + miR.660 | 0,81 | 0,180517781 |
| miR.558 + miR.609 + miR.603 + miR.520f + miR.220a + miR.26a + miR.29a + miR.362 + miR.369.3p + miR.376a* + miR.518c + miR.639 + miR.660 | 0,81 | 0,280699357 |
| miR.558 + miR.609 + miR.603 + miR.220a + miR.519b + miR.26a + miR.29a + miR.369.3p + miR.376a* + miR.494 + miR.518c + miR.639 + miR.660 | 0,81 | 0,182193834 |
| miR.558 + miR.609 + miR.603 + miR.520f + miR.220a + miR.130a + miR.26a + miR.29a + miR.369.3p + miR.376a* + miR.518c + miR.660 + miR.338 | 0,81 | 0,137041633 |
| miR.558 + miR.609 + miR.520f + miR.220a + miR.130a + miR.29a + miR.362 + miR.369.3p + miR.376a* + miR.494 + miR.518c + miR.660 + miR.338 | 0,81 | 0,417142274 |
| miR.558 + miR.609 + miR.603 + miR.220a + miR.130a + miR.26a + miR.29a + miR.369.3p + miR.376a* + miR.518c + miR.639 + miR.660 + miR.338 | 0,81 | 0,199437025 |
| miR.558 + miR.609 + miR.603 + miR.220a + miR.130a + miR.26a + miR.29a + miR.369.3p + miR.376a* + miR.494 + miR.518c + miR.639 + miR.660 | 0,81 | 0,181981186 |
| miR.558 + miR.609 + miR.603 + miR.220a + miR.130a + miR.26a + miR.29a + miR.369.3p + miR.376a* + miR.494 + miR.518c + miR.660 + miR.338 | 0,81 | 0,113204377 |
| miR.558 + miR.609 + miR.603 + miR.220a + miR.519b + miR.130a + miR.26a + miR.29a + miR.369.3p + miR.376a* + miR.494 + miR.518c + miR.660 | 0,81 | 0,191134215 |
| miR.558 + miR.609 + miR.603 + miR.520f + miR.220a + miR.519b + miR.26a + miR.29a + miR.369.3p + miR.376a* + miR.518c + miR.660 + miR.338 | 0,81 | 0,204627596 |
| miR.558 + miR.609 + miR.603 + miR.220a + miR.130a + miR.29a + miR.362 + miR.369.3p + miR.376a* + miR.494 + miR.518c + miR.639 + miR.660 | 0,81 | 0,197575929 |
| miR.558 + miR.609 + miR.603 + miR.220a + miR.519b + miR.26a + miR.29a + miR.362 + miR.369.3p + miR.376a* + miR.518c + miR.639 + miR.660 | 0,81 | 0,21718402 |
| miR.558 + miR.609 + miR.520f + miR.220a + miR.519b + miR.29a + miR.362 + miR.369.3p + miR.376a* + miR.494 + miR.518c + miR.660 + miR.338 | 0,81 | 0,409045122 |
| miR.558 + miR.609 + miR.603 + miR.520f + miR.220a + miR.26a + miR.29a + miR.369.3p + miR.376a* + miR.494 + miR.518c + miR.639 + miR.660 | 0,81 | 0,119776607 |
| miR.558 + miR.609 + miR.603 + miR.220a + miR.519b + miR.130a + miR.29a + miR.369.3p + miR.376a* + miR.494 + miR.518c + miR.639 + miR.660 | 0,81 | 0,151514703 |
| miR.558 + miR.609 + miR.603 + miR.519b + miR.26a + miR.29a + miR.362 + miR.369.3p + miR.376a* + miR.518c + miR.639 + miR.660 + miR.338 | 0,81 | 0,172712142 |
| miR.558 + miR.609 + miR.603 + miR.130a + miR.26a + miR.29a + miR.362 + miR.369.3p + miR.376a* + miR.518c + miR.639 + miR.660 + miR.338 | 0,81 | 0,186993533 |
| miR.558 + miR.609 + miR.603 + miR.520f + miR.220a + miR.26a + miR.29a + miR.362 + miR.369.3p + miR.376a* + miR.494 + miR.518c + miR.660 | 0,81 | 0,190463659 |
| miR.558 + miR.609 + miR.603 + miR.520f + miR.220a + miR.519b + miR.130a + miR.29a + miR.369.3p + miR.376a* + miR.518c + miR.660 + miR.338 | 0,81 | 0,233359488 |
| miR.558 + miR.609 + miR.603 + miR.520f + miR.220a + miR.519b + miR.29a + miR.362 + miR.369.3p + miR.376a* + miR.494 + miR.518c + miR.660 | 0,81 | 0,234529745 |
| miR.558 + miR.609 + miR.603 + miR.220a + miR.519b + miR.130a + miR.29a + miR.362 + miR.369.3p + miR.376a* + miR.494 + miR.518c + miR.660 | 0,81 | 0,175576547 |
| miR.558 + miR.609 + miR.603 + miR.520f + miR.220a + miR.519b + miR.29a + miR.369.3p + miR.376a* + miR.494 + miR.518c + miR.639 + miR.660 | 0,81 | 0,196246439 |
| miR.558 + miR.609 + miR.220a + miR.130a + miR.29a + miR.362 + miR.369.3p + miR.376a* + miR.494 + miR.518c + miR.639 + miR.660 + miR.338 | 0,81 | 0,387358587 |
| miR.558 + miR.609 + miR.603 + miR.220a + miR.519b + miR.130a + miR.26a + miR.29a + miR.369.3p + miR.376a* + miR.494 + miR.518c + miR.338 | 0,81 | 0,097397814 |
| miR.558 + miR.609 + miR.603 + miR.520f + miR.220a + miR.29a + miR.362 + miR.369.3p + miR.376a* + miR.494 + miR.518c + miR.639 + miR.660 | 0,81 | 0,128333944 |
| miR.558 + miR.609 + miR.603 + miR.220a + miR.130a + miR.26a + miR.29a + miR.362 + miR.369.3p + miR.376a* + miR.494 + miR.518c + miR.660 | 0,81 | 0,232043214 |
| miR.558 + miR.609 + miR.603 + miR.520f + miR.220a + miR.519b + miR.130a + miR.29a + miR.369.3p + miR.376a* + miR.494 + miR.518c + miR.660 | 0,81 | 0,162771652 |
| miR.558 + miR.609 + miR.220a + miR.26a + miR.29a + miR.362 + miR.369.3p + miR.376a* + miR.494 + miR.518c + miR.639 + miR.660 + miR.338 | 0,81 | 0,444413996 |
| miR.558 + miR.609 + miR.603 + miR.520f + miR.220a + miR.519b + miR.26a + miR.29a + miR.369.3p + miR.376a* + miR.494 + miR.518c + miR.338 | 0,81 | 0,099259954 |
| miR.558 + miR.609 + miR.603 + miR.520f + miR.220a + miR.130a + miR.29a + miR.369.3p + miR.376a* + miR.494 + miR.518c + miR.660 + miR.338 | 0,81 | 0,117335604 |
| miR.558 + miR.609 + miR.603 + miR.520f + miR.220a + miR.130a + miR.29a + miR.369.3p + miR.376a* + miR.494 + miR.518c + miR.639 + miR.660 | 0,81 | 0,090046396 |
| miR.558 + miR.609 + miR.603 + miR.220a + miR.130a + miR.26a + miR.29a + miR.362 + miR.369.3p + miR.376a* + miR.518c + miR.639 + miR.660 | 0,81 | 0,353748036 |
| miR.558 + miR.609 + miR.603 + miR.220a + miR.519b + miR.26a + miR.29a + miR.362 + miR.369.3p + miR.376a* + miR.494 + miR.518c + miR.338 | 0,81 | 0,114205406 |
| miR.558 + miR.609 + miR.603 + miR.520f + miR.220a + miR.519b + miR.26a + miR.29a + miR.362 + miR.369.3p + miR.376a* + miR.518c + miR.660 | 0,81 | 0,246124567 |
| miR.558 + miR.609 + miR.519b + miR.130a + miR.26a + miR.29a + miR.362 + miR.369.3p + miR.376a* + miR.518c + miR.639 + miR.660 + miR.338 | 0,81 | 0,388923604 |
| miR.558 + miR.609 + miR.603 + miR.520f + miR.220a + miR.519b + miR.29a + miR.362 + miR.369.3p + miR.376a* + miR.494 + miR.518c + miR.338 | 0,81 | 0,10603463 |
| miR.558 + miR.609 + miR.603 + miR.520f + miR.220a + miR.130a + miR.26a + miR.29a + miR.369.3p + miR.376a* + miR.494 + miR.518c + miR.660 | 0,81 | 0,15167349 |
| miR.558 + miR.609 + miR.520f + miR.220a + miR.519b + miR.130a + miR.29a + miR.362 + miR.369.3p + miR.376a* + miR.518c + miR.660 + miR.338 | 0,81 | 0,483032004 |
| miR.558 + miR.609 + miR.520f + miR.220a + miR.519b + miR.26a + miR.29a + miR.362 + miR.369.3p + miR.376a* + miR.518c + miR.660 + miR.338 | 0,81 | 0,483412918 |
| miR.558 + miR.609 + miR.603 + miR.220a + miR.519b + miR.26a + miR.29a + miR.369.3p + miR.376a* + miR.518c + miR.639 + miR.660 + miR.338 | 0,81 | 0,178112812 |
| miR.558 + miR.609 + miR.603 + miR.220a + miR.519b + miR.130a + miR.26a + miR.29a + miR.362 + miR.369.3p + miR.376a* + miR.494 + miR.518c | 0,81 | 0,182035102 |
| miR.558 + miR.609 + miR.520f + miR.220a + miR.130a + miR.26a + miR.29a + miR.362 + miR.369.3p + miR.376a* + miR.518c + miR.660 + miR.338 | 0,81 | 0,496622557 |
| miR.558 + miR.609 + miR.603 + miR.520f + miR.220a + miR.130a + miR.26a + miR.29a + miR.369.3p + miR.376a* + miR.518c + miR.639 + miR.660 | 0,81 | 0,172344989 |
| miR.558 + miR.609 + miR.220a + miR.519b + miR.26a + miR.29a + miR.362 + miR.369.3p + miR.376a* + miR.518c + miR.639 + miR.660 + miR.338 | 0,81 | 0,42999729 |
| miR.558 + miR.609 + miR.603 + miR.520f + miR.519b + miR.29a + miR.369.3p + miR.376a* + miR.494 + miR.518c + miR.639 + miR.660 + miR.338 | 0,81 | 0,063892553 |
| miR.558 + miR.609 + miR.603 + miR.220a + miR.130a + miR.29a + miR.362 + miR.369.3p + miR.376a* + miR.494 + miR.518c + miR.660 + miR.338 | 0,81 | 0,348489444 |
| miR.558 + miR.609 + miR.603 + miR.220a + miR.519b + miR.29a + miR.362 + miR.369.3p + miR.376a* + miR.494 + miR.518c + miR.660 + miR.338 | 0,81 | 0,22534063 |
| miR.558 + miR.609 + miR.519b + miR.130a + miR.29a + miR.362 + miR.369.3p + miR.376a* + miR.494 + miR.518c + miR.639 + miR.660 + miR.338 | 0,81 | 0,172555113 |
| miR.558 + miR.609 + miR.603 + miR.520f + miR.220a + miR.519b + miR.130a + miR.29a + miR.369.3p + miR.376a* + miR.494 + miR.518c + miR.338 | 0,81 | 0,06035927 |
| miR.558 + miR.609 + miR.603 + miR.220a + miR.519b + miR.26a + miR.29a + miR.369.3p + miR.376a* + miR.494 + miR.518c + miR.639 + miR.338 | 0,81 | 0,10391966 |
| miR.558 + miR.609 + miR.603 + miR.520f + miR.130a + miR.29a + miR.369.3p + miR.376a* + miR.494 + miR.518c + miR.639 + miR.660 + miR.338 | 0,81 | 0,021685409 |
| miR.558 + miR.609 + miR.520f + miR.519b + miR.130a + miR.29a + miR.362 + miR.369.3p + miR.376a* + miR.494 + miR.518c + miR.660 + miR.338 | 0,81 | 0,436072031 |
| miR.558 + miR.609 + miR.520f + miR.220a + miR.130a + miR.29a + miR.369.3p + miR.376a* + miR.494 + miR.518c + miR.639 + miR.660 + miR.338 | 0,81 | 0,090304402 |
| miR.558 + miR.609 + miR.520f + miR.220a + miR.519b + miR.29a + miR.369.3p + miR.376a* + miR.494 + miR.518c + miR.639 + miR.660 + miR.338 | 0,81 | 0,262140064 |
| miR.558 + miR.609 + miR.603 + miR.220a + miR.519b + miR.130a + miR.26a + miR.29a + miR.369.3p + miR.376a* + miR.518c + miR.660 + miR.338 | 0,81 | 0,16719454 |
| miR.558 + miR.609 + miR.603 + miR.220a + miR.26a + miR.29a + miR.362 + miR.369.3p + miR.376a* + miR.494 + miR.518c + miR.639 + miR.338 | 0,81 | 0,051418316 |
| miR.558 + miR.609 + miR.603 + miR.520f + miR.220a + miR.26a + miR.29a + miR.362 + miR.369.3p + miR.376a* + miR.518c + miR.639 + miR.338 | 0,81 | 0,051492307 |
| miR.558 + miR.609 + miR.603 + miR.220a + miR.519b + miR.130a + miR.26a + miR.29a + miR.362 + miR.369.3p + miR.376a* + miR.518c + miR.660 | 0,81 | 0,236074713 |
| miR.558 + miR.609 + miR.603 + miR.520f + miR.220a + miR.130a + miR.26a + miR.29a + miR.362 + miR.369.3p + miR.376a* + miR.518c + miR.660 | 0,81 | 0,236869513 |
| miR.558 + miR.609 + miR.603 + miR.220a + miR.519b + miR.29a + miR.362 + miR.369.3p + miR.376a* + miR.494 + miR.518c + miR.639 + miR.660 | 0,81 | 0,190449602 |
| miR.558 + miR.609 + miR.603 + miR.520f + miR.26a + miR.29a + miR.369.3p + miR.376a* + miR.494 + miR.518c + miR.639 + miR.660 + miR.338 | 0,81 | 0,022397978 |
| miR.558 + miR.609 + miR.603 + miR.520f + miR.220a + miR.519b + miR.26a + miR.29a + miR.362 + miR.369.3p + miR.376a* + miR.494 + miR.518c | 0,81 | 0,123919493 |
| miR.558 + miR.609 + miR.520f + miR.130a + miR.26a + miR.29a + miR.362 + miR.369.3p + miR.376a* + miR.494 + miR.518c + miR.660 + miR.338 | 0,81 | 0,356299555 |
| miR.558 + miR.609 + miR.603 + miR.520f + miR.220a + miR.519b + miR.26a + miR.29a + miR.369.3p + miR.376a* + miR.518c + miR.639 + miR.660 | 0,81 | 0,207873921 |
| miR.558 + miR.609 + miR.603 + miR.520f + miR.220a + miR.130a + miR.29a + miR.362 + miR.369.3p + miR.376a* + miR.494 + miR.518c + miR.660 | 0,81 | 0,178581156 |
| miR.558 + miR.609 + miR.603 + miR.520f + miR.26a + miR.29a + miR.362 + miR.369.3p + miR.376a* + miR.494 + miR.518c + miR.660 + miR.338 | 0,81 | 0,238459179 |
| miR.558 + miR.609 + miR.603 + miR.520f + miR.519b + miR.29a + miR.362 + miR.369.3p + miR.376a* + miR.494 + miR.518c + miR.660 + miR.338 | 0,81 | 0,377482029 |
| miR.558 + miR.609 + miR.603 + miR.520f + miR.220a + miR.26a + miR.29a + miR.369.3p + miR.376a* + miR.494 + miR.518c + miR.639 + miR.338 | 0,81 | 0,052528182 |
| miR.558 + miR.609 + miR.603 + miR.220a + miR.519b + miR.29a + miR.362 + miR.369.3p + miR.376a* + miR.518c + miR.639 + miR.660 + miR.338 | 0,81 | 0,305502078 |
| miR.558 + miR.609 + miR.520f + miR.220a + miR.26a + miR.29a + miR.369.3p + miR.376a* + miR.494 + miR.518c + miR.639 + miR.660 + miR.338 | 0,81 | 0,158073054 |
| miR.558 + miR.609 + miR.603 + miR.220a + miR.130a + miR.26a + miR.29a + miR.369.3p + miR.376a* + miR.494 + miR.518c + miR.639 + miR.338 | 0,81 | 0,042969607 |
| miR.558 + miR.609 + miR.603 + miR.520f + miR.220a + miR.519b + miR.130a + miR.29a + miR.362 + miR.369.3p + miR.376a* + miR.494 + miR.518c | 0,81 | 0,135163353 |
| miR.558 + miR.609 + miR.520f + miR.519b + miR.26a + miR.29a + miR.362 + miR.369.3p + miR.376a* + miR.494 + miR.518c + miR.660 + miR.338 | 0,81 | 0,326826099 |
| miR.558 + miR.609 + miR.603 + miR.220a + miR.519b + miR.26a + miR.29a + miR.362 + miR.369.3p + miR.376a* + miR.494 + miR.518c + miR.639 | 0,81 | 0,125139629 |
| miR.558 + miR.609 + miR.603 + miR.220a + miR.519b + miR.130a + miR.26a + miR.29a + miR.369.3p + miR.376a* + miR.494 + miR.518c + miR.639 | 0,81 | 0,156284326 |
| miR.558 + miR.609 + miR.220a + miR.130a + miR.26a + miR.29a + miR.362 + miR.369.3p + miR.376a* + miR.518c + miR.639 + miR.660 + miR.338 | 0,81 | 0,450514876 |
| miR.558 + miR.609 + miR.603 + miR.520f + miR.220a + miR.519b + miR.29a + miR.362 + miR.369.3p + miR.376a* + miR.518c + miR.639 + miR.660 | 0,81 | 0,180818629 |
| miR.558 + miR.609 + miR.603 + miR.520f + miR.220a + miR.26a + miR.29a + miR.362 + miR.369.3p + miR.376a* + miR.494 + miR.518c + miR.338 | 0,81 | 0,035595123 |
| miR.558 + miR.609 + miR.603 + miR.220a + miR.519b + miR.130a + miR.29a + miR.362 + miR.369.3p + miR.376a* + miR.494 + miR.518c + miR.338 | 0,81 | 0,088341056 |
| miR.558 + miR.609 + miR.603 + miR.220a + miR.519b + miR.29a + miR.369.3p + miR.376a* + miR.494 + miR.518c + miR.639 + miR.660 + miR.338 | 0,81 | 0,104019261 |
| miR.558 + miR.609 + miR.603 + miR.520f + miR.220a + miR.519b + miR.130a + miR.26a + miR.29a + miR.369.3p + miR.376a* + miR.494 + miR.518c | 0,81 | 0,136023326 |
| miR.558 + miR.609 + miR.603 + miR.220a + miR.519b + miR.130a + miR.26a + miR.29a + miR.362 + miR.376a* + miR.494 + miR.518c + miR.338 | 0,81 | 0,060958369 |
| miR.558 + miR.609 + miR.603 + miR.130a + miR.26a + miR.29a + miR.362 + miR.369.3p + miR.376a* + miR.494 + miR.518c + miR.639 + miR.660 | 0,81 | 0,041496487 |
| miR.558 + miR.609 + miR.603 + miR.220a + miR.130a + miR.26a + miR.29a + miR.362 + miR.369.3p + miR.376a* + miR.518c + miR.639 + miR.338 | 0,81 | 0,053206862 |
| miR.558 + miR.609 + miR.603 + miR.220a + miR.130a + miR.29a + miR.369.3p + miR.376a* + miR.494 + miR.518c + miR.639 + miR.660 + miR.338 | 0,81 | 0,132410273 |
| miR.558 + miR.609 + miR.603 + miR.520f + miR.130a + miR.26a + miR.29a + miR.362 + miR.369.3p + miR.376a* + miR.518c + miR.660 + miR.338 | 0,81 | 0,168300313 |
| miR.558 + miR.609 + miR.603 + miR.220a + miR.130a + miR.26a + miR.29a + miR.362 + miR.369.3p + miR.376a* + miR.494 + miR.518c + miR.338 | 0,81 | 0,042876004 |
| miR.558 + miR.609 + miR.603 + miR.520f + miR.220a + miR.130a + miR.26a + miR.29a + miR.369.3p + miR.376a* + miR.518c + miR.639 + miR.338 | 0,81 | 0,049783643 |
| miR.558 + miR.609 + miR.603 + miR.520f + miR.220a + miR.130a + miR.26a + miR.29a + miR.362 + miR.369.3p + miR.376a* + miR.518c + miR.338 | 0,81 | 0,039514334 |
| miR.558 + miR.609 + miR.603 + miR.220a + miR.519b + miR.130a + miR.26a + miR.29a + miR.369.3p + miR.376a* + miR.518c + miR.639 + miR.660 | 0,81 | 0,217189728 |
| miR.558 + miR.609 + miR.603 + miR.520f + miR.130a + miR.29a + miR.362 + miR.369.3p + miR.376a* + miR.494 + miR.518c + miR.639 + miR.660 | 0,81 | 0,006068116 |
| miR.558 + miR.609 + miR.520f + miR.519b + miR.130a + miR.29a + miR.369.3p + miR.376a* + miR.494 + miR.518c + miR.639 + miR.660 + miR.338 | 0,81 | 0,120770018 |
| miR.558 + miR.609 + miR.603 + miR.520f + miR.220a + miR.130a + miR.26a + miR.29a + miR.369.3p + miR.376a* + miR.494 + miR.518c + miR.338 | 0,81 | 0,037361085 |
| miR.558 + miR.609 + miR.603 + miR.220a + miR.519b + miR.130a + miR.29a + miR.369.3p + miR.376a* + miR.494 + miR.518c + miR.639 + miR.338 | 0,81 | 0,063650582 |
| miR.558 + miR.609 + miR.603 + miR.220a + miR.519b + miR.130a + miR.26a + miR.29a + miR.362 + miR.376a* + miR.494 + miR.518c + miR.639 | 0,81 | 0,082538779 |
| miR.558 + miR.609 + miR.603 + miR.220a + miR.519b + miR.130a + miR.26a + miR.29a + miR.362 + miR.376a* + miR.494 + miR.518c + miR.660 | 0,81 | 0,111258667 |
| miR.558 + miR.609 + miR.603 + miR.520f + miR.220a + miR.130a + miR.29a + miR.362 + miR.369.3p + miR.376a* + miR.518c + miR.639 + miR.660 | 0,81 | 0,146308564 |
| miR.558 + miR.609 + miR.603 + miR.520f + miR.220a + miR.519b + miR.130a + miR.26a + miR.29a + miR.369.3p + miR.376a* + miR.518c + miR.660 | 0,81 | 0,217635541 |
| miR.558 + miR.609 + miR.603 + miR.520f + miR.220a + miR.519b + miR.29a + miR.362 + miR.369.3p + miR.376a* + miR.494 + miR.518c + miR.639 | 0,81 | 0,124531345 |
| miR.558 + miR.609 + miR.603 + miR.220a + miR.130a + miR.26a + miR.29a + miR.362 + miR.369.3p + miR.376a* + miR.494 + miR.518c + miR.639 | 0,81 | 0,070738674 |
| miR.558 + miR.609 + miR.603 + miR.220a + miR.519b + miR.130a + miR.29a + miR.362 + miR.369.3p + miR.376a* + miR.494 + miR.518c + miR.639 | 0,81 | 0,104649693 |
| miR.558 + miR.609 + miR.603 + miR.520f + miR.130a + miR.29a + miR.362 + miR.369.3p + miR.376a* + miR.494 + miR.518c + miR.660 + miR.338 | 0,81 | 0,32638794 |
| miR.558 + miR.609 + miR.603 + miR.220a + miR.519b + miR.130a + miR.29a + miR.362 + miR.369.3p + miR.376a* + miR.518c + miR.639 + miR.660 | 0,81 | 0,237519424 |
| miR.558 + miR.609 + miR.603 + miR.220a + miR.519b + miR.130a + miR.29a + miR.369.3p + miR.376a* + miR.518c + miR.639 + miR.660 + miR.338 | 0,81 | 0,16109912 |
| miR.558 + miR.609 + miR.603 + miR.520f + miR.519b + miR.26a + miR.29a + miR.362 + miR.369.3p + miR.376a* + miR.518c + miR.660 + miR.338 | 0,81 | 0,144140479 |
| miR.558 + miR.609 + miR.220a + miR.130a + miR.26a + miR.29a + miR.362 + miR.369.3p + miR.376a* + miR.494 + miR.518c + miR.660 + miR.338 | 0,81 | 0,373888812 |
| miR.558 + miR.609 + miR.520f + miR.220a + miR.519b + miR.26a + miR.29a + miR.369.3p + miR.376a* + miR.494 + miR.518c + miR.660 + miR.338 | 0,81 | 0,22144321 |
| miR.558 + miR.609 + miR.603 + miR.520f + miR.519b + miR.26a + miR.29a + miR.369.3p + miR.376a* + miR.518c + miR.639 + miR.660 + miR.338 | 0,81 | 0,050223589 |
| miR.558 + miR.609 + miR.520f + miR.220a + miR.519b + miR.130a + miR.29a + miR.369.3p + miR.376a* + miR.494 + miR.518c + miR.660 + miR.338 | 0,81 | 0,228984452 |
| miR.558 + miR.609 + miR.520f + miR.220a + miR.519b + miR.26a + miR.29a + miR.369.3p + miR.376a* + miR.518c + miR.639 + miR.660 + miR.338 | 0,81 | 0,202590909 |
| miR.558 + miR.609 + miR.603 + miR.520f + miR.220a + miR.519b + miR.130a + miR.29a + miR.369.3p + miR.376a* + miR.518c + miR.639 + miR.660 | 0,81 | 0,198405441 |
| miR.558 + miR.609 + miR.603 + miR.520f + miR.220a + miR.519b + miR.26a + miR.29a + miR.369.3p + miR.376a* + miR.494 + miR.518c + miR.639 | 0,81 | 0,107884424 |
| miR.558 + miR.609 + miR.603 + miR.220a + miR.519b + miR.130a + miR.29a + miR.362 + miR.369.3p + miR.376a* + miR.518c + miR.660 + miR.338 | 0,81 | 0,399603495 |
| miR.558 + miR.609 + miR.520f + miR.130a + miR.26a + miR.29a + miR.369.3p + miR.376a* + miR.494 + miR.518c + miR.639 + miR.660 + miR.338 | 0,81 | 0,151648953 |
| miR.558 + miR.609 + miR.603 + miR.519b + miR.130a + miR.29a + miR.362 + miR.369.3p + miR.376a* + miR.518c + miR.639 + miR.660 + miR.338 | 0,81 | 0,146726442 |
| miR.558 + miR.609 + miR.603 + miR.520f + miR.220a + miR.519b + miR.26a + miR.29a + miR.362 + miR.369.3p + miR.376a* + miR.518c + miR.338 | 0,81 | 0,116312649 |
| miR.558 + miR.609 + miR.603 + miR.220a + miR.519b + miR.130a + miR.26a + miR.29a + miR.362 + miR.369.3p + miR.376a* + miR.518c + miR.639 | 0,81 | 0,111737823 |
| miR.558 + miR.609 + miR.603 + miR.520f + miR.519b + miR.130a + miR.29a + miR.362 + miR.369.3p + miR.376a* + miR.518c + miR.660 + miR.338 | 0,81 | 0,217527688 |
| miR.558 + miR.609 + miR.603 + miR.130a + miR.26a + miR.29a + miR.369.3p + miR.376a* + miR.494 + miR.518c + miR.639 + miR.660 + miR.338 | 0,81 | 0,137020658 |
| miR.558 + miR.609 + miR.603 + miR.220a + miR.29a + miR.362 + miR.369.3p + miR.376a* + miR.494 + miR.518c + miR.639 + miR.660 + miR.338 | 0,81 | 0,287344575 |
| miR.558 + miR.609 + miR.603 + miR.520f + miR.220a + miR.26a + miR.29a + miR.362 + miR.369.3p + miR.376a* + miR.494 + miR.518c + miR.639 | 0,81 | 0,076865744 |
| miR.558 + miR.609 + miR.603 + miR.520f + miR.220a + miR.519b + miR.130a + miR.29a + miR.369.3p + miR.376a* + miR.494 + miR.518c + miR.639 | 0,81 | 0,095765593 |
| miR.558 + miR.609 + miR.603 + miR.520f + miR.220a + miR.519b + miR.130a + miR.29a + miR.362 + miR.369.3p + miR.376a* + miR.518c + miR.660 | 0,81 | 0,233473801 |
| miR.558 + miR.609 + miR.220a + miR.519b + miR.26a + miR.29a + miR.362 + miR.369.3p + miR.376a* + miR.494 + miR.518c + miR.660 + miR.338 | 0,81 | 0,439970485 |
| miR.558 + miR.609 + miR.603 + miR.520f + miR.130a + miR.26a + miR.29a + miR.369.3p + miR.376a* + miR.518c + miR.639 + miR.660 + miR.338 | 0,81 | 0,0322276 |
| miR.558 + miR.609 + miR.603 + miR.520f + miR.220a + miR.130a + miR.26a + miR.29a + miR.362 + miR.369.3p + miR.376a* + miR.518c + miR.639 | 0,81 | 0,091894888 |
| miR.558 + miR.609 + miR.520f + miR.220a + miR.130a + miR.26a + miR.29a + miR.369.3p + miR.376a* + miR.518c + miR.639 + miR.660 + miR.338 | 0,81 | 0,168935619 |
| miR.558 + miR.609 + miR.603 + miR.520f + miR.220a + miR.130a + miR.26a + miR.29a + miR.362 + miR.369.3p + miR.376a* + miR.494 + miR.518c | 0,81 | 0,062361826 |
| miR.558 + miR.609 + miR.603 + miR.520f + miR.519b + miR.130a + miR.29a + miR.369.3p + miR.376a* + miR.518c + miR.639 + miR.660 + miR.338 | 0,81 | 0,057639073 |
| miR.558 + miR.609 + miR.220a + miR.519b + miR.130a + miR.29a + miR.362 + miR.369.3p + miR.376a* + miR.518c + miR.639 + miR.660 + miR.338 | 0,81 | 0,442563861 |
| miR.558 + miR.609 + miR.603 + miR.520f + miR.220a + miR.519b + miR.26a + miR.29a + miR.362 + miR.369.3p + miR.376a* + miR.518c + miR.639 | 0,81 | 0,142333583 |
| miR.558 + miR.609 + miR.603 + miR.520f + miR.220a + miR.519b + miR.26a + miR.29a + miR.369.3p + miR.376a* + miR.518c + miR.639 + miR.338 | 0,81 | 0,083427866 |
| miR.558 + miR.609 + miR.519b + miR.26a + miR.29a + miR.362 + miR.369.3p + miR.376a* + miR.494 + miR.518c + miR.639 + miR.660 + miR.338 | 0,81 | 0,225943814 |
| miR.558 + miR.609 + miR.220a + miR.130a + miR.26a + miR.29a + miR.362 + miR.369.3p + miR.376a* + miR.494 + miR.518c + miR.639 + miR.660 | 0,81 | 0,220355412 |
| miR.558 + miR.609 + miR.603 + miR.520f + miR.220a + miR.519b + miR.29a + miR.376a* + miR.494 + miR.518c + miR.639 + miR.660 + miR.338 | 0,81 | 0,062678291 |
| miR.558 + miR.609 + miR.603 + miR.520f + miR.220a + miR.519b + miR.29a + miR.362 + miR.369.3p + miR.376a* + miR.518c + miR.639 + miR.338 | 0,81 | 0,096458624 |
| miR.558 + miR.609 + miR.520f + miR.220a + miR.26a + miR.29a + miR.362 + miR.369.3p + miR.376a* + miR.494 + miR.518c + miR.639 + miR.660 | 0,81 | 0,133522478 |
| miR.558 + miR.609 + miR.603 + miR.520f + miR.220a + miR.130a + miR.26a + miR.29a + miR.369.3p + miR.376a* + miR.494 + miR.518c + miR.639 | 0,81 | 0,057173785 |
| miR.558 + miR.609 + miR.603 + miR.520f + miR.220a + miR.519b + miR.130a + miR.26a + miR.29a + miR.369.3p + miR.376a* + miR.518c + miR.338 | 0,81 | 0,091936433 |
| miR.558 + miR.609 + miR.520f + miR.220a + miR.130a + miR.29a + miR.362 + miR.369.3p + miR.376a* + miR.494 + miR.518c + miR.639 + miR.660 | 0,81 | 0,137126068 |
| miR.558 + miR.609 + miR.520f + miR.220a + miR.519b + miR.130a + miR.29a + miR.369.3p + miR.376a* + miR.518c + miR.639 + miR.660 + miR.338 | 0,81 | 0,170485549 |
| miR.558 + miR.609 + miR.220a + miR.130a + miR.26a + miR.29a + miR.369.3p + miR.376a* + miR.494 + miR.518c + miR.639 + miR.660 + miR.338 | 0,81 | 0,209177085 |
| miR.558 + miR.609 + miR.603 + miR.520f + miR.220a + miR.519b + miR.130a + miR.26a + miR.29a + miR.362 + miR.369.3p + miR.376a* + miR.518c | 0,81 | 0,140365434 |
| miR.558 + miR.609 + miR.603 + miR.520f + miR.220a + miR.519b + miR.130a + miR.29a + miR.362 + miR.376a* + miR.494 + miR.518c + miR.338 | 0,81 | 0,053398377 |
| miR.558 + miR.609 + miR.603 + miR.520f + miR.220a + miR.519b + miR.130a + miR.29a + miR.376a* + miR.494 + miR.518c + miR.660 + miR.338 | 0,81 | 0,084042868 |
| miR.558 + miR.609 + miR.603 + miR.520f + miR.220a + miR.519b + miR.29a + miR.369.3p + miR.376a* + miR.494 + miR.518c + miR.639 + miR.338 | 0,81 | 0,083550012 |
| miR.558 + miR.609 + miR.603 + miR.520f + miR.220a + miR.519b + miR.130a + miR.29a + miR.362 + miR.369.3p + miR.376a* + miR.518c + miR.338 | 0,81 | 0,0905644 |
| miR.558 + miR.609 + miR.603 + miR.520f + miR.519b + miR.26a + miR.29a + miR.369.3p + miR.376a* + miR.494 + miR.518c + miR.660 + miR.338 | 0,81 | 0,095416778 |
| miR.558 + miR.609 + miR.603 + miR.220a + miR.519b + miR.130a + miR.29a + miR.362 + miR.376a* + miR.494 + miR.518c + miR.639 + miR.338 | 0,81 | 0,056415696 |
| miR.558 + miR.609 + miR.603 + miR.520f + miR.519b + miR.130a + miR.29a + miR.369.3p + miR.376a* + miR.494 + miR.518c + miR.660 + miR.338 | 0,81 | 0,075606555 |
| miR.558 + miR.609 + miR.603 + miR.519b + miR.26a + miR.29a + miR.362 + miR.369.3p + miR.376a* + miR.494 + miR.518c + miR.660 + miR.338 | 0,81 | 0,149873902 |
| miR.558 + miR.609 + miR.603 + miR.220a + miR.519b + miR.130a + miR.29a + miR.362 + miR.376a* + miR.494 + miR.518c + miR.639 + miR.660 | 0,81 | 0,10234866 |
| miR.558 + miR.609 + miR.603 + miR.220a + miR.519b + miR.26a + miR.29a + miR.362 + miR.369.3p + miR.376a* + miR.518c + miR.639 + miR.338 | 0,81 | 0,1095917 |
| miR.558 + miR.609 + miR.603 + miR.520f + miR.220a + miR.519b + miR.29a + miR.362 + miR.376a* + miR.494 + miR.518c + miR.660 + miR.338 | 0,81 | 0,105958858 |
| miR.558 + miR.609 + miR.603 + miR.520f + miR.220a + miR.519b + miR.130a + miR.29a + miR.362 + miR.376a* + miR.494 + miR.518c + miR.639 | 0,81 | 0,097183864 |
| miR.558 + miR.609 + miR.603 + miR.130a + miR.26a + miR.29a + miR.362 + miR.369.3p + miR.376a* + miR.494 + miR.518c + miR.660 + miR.338 | 0,81 | 0,094975348 |
| miR.558 + miR.609 + miR.603 + miR.220a + miR.519b + miR.130a + miR.26a + miR.29a + miR.376a* + miR.494 + miR.518c + miR.660 + miR.338 | 0,81 | 0,067932326 |
| miR.558 + miR.609 + miR.603 + miR.520f + miR.220a + miR.519b + miR.26a + miR.29a + miR.376a* + miR.494 + miR.518c + miR.660 + miR.338 | 0,81 | 0,10344806 |
| miR.558 + miR.609 + miR.603 + miR.520f + miR.220a + miR.519b + miR.130a + miR.26a + miR.29a + miR.362 + miR.376a* + miR.494 + miR.518c | 0,81 | 0,097108796 |
| miR.558 + miR.609 + miR.603 + miR.520f + miR.130a + miR.26a + miR.29a + miR.369.3p + miR.376a* + miR.494 + miR.518c + miR.639 + miR.338 | 0,81 | 0,034401585 |
| miR.558 + miR.609 + miR.603 + miR.520f + miR.519b + miR.130a + miR.29a + miR.369.3p + miR.376a* + miR.494 + miR.518c + miR.639 + miR.338 | 0,81 | 0,054079815 |
| miR.558 + miR.609 + miR.520f + miR.220a + miR.130a + miR.26a + miR.29a + miR.369.3p + miR.376a* + miR.494 + miR.518c + miR.660 + miR.338 | 0,81 | 0,106293041 |
| miR.558 + miR.609 + miR.603 + miR.520f + miR.220a + miR.519b + miR.29a + miR.362 + miR.376a* + miR.494 + miR.518c + miR.639 + miR.338 | 0,81 | 0,068104962 |
| miR.558 + miR.609 + miR.603 + miR.220a + miR.519b + miR.130a + miR.26a + miR.29a + miR.376a* + miR.494 + miR.518c + miR.639 + miR.660 | 0,81 | 0,109350789 |
| miR.558 + miR.609 + miR.603 + miR.220a + miR.519b + miR.130a + miR.26a + miR.29a + miR.362 + miR.369.3p + miR.376a* + miR.518c + miR.338 | 0,81 | 0,115746824 |
| miR.558 + miR.609 + miR.603 + miR.520f + miR.220a + miR.130a + miR.29a + miR.362 + miR.369.3p + miR.376a* + miR.494 + miR.518c + miR.338 | 0,81 | 0,042546222 |
| miR.558 + miR.609 + miR.603 + miR.520f + miR.220a + miR.519b + miR.26a + miR.29a + miR.362 + miR.376a* + miR.494 + miR.518c + miR.338 | 0,81 | 0,077476199 |
| miR.558 + miR.609 + miR.603 + miR.519b + miR.130a + miR.26a + miR.29a + miR.369.3p + miR.376a* + miR.494 + miR.518c + miR.639 + miR.338 | 0,81 | 0,093186559 |
| miR.558 + miR.609 + miR.603 + miR.520f + miR.220a + miR.519b + miR.130a + miR.29a + miR.362 + miR.376a* + miR.494 + miR.518c + miR.660 | 0,81 | 0,151147002 |
| miR.558 + miR.609 + miR.220a + miR.519b + miR.130a + miR.29a + miR.362 + miR.369.3p + miR.376a* + miR.494 + miR.518c + miR.660 + miR.338 | 0,81 | 0,27313387 |
| miR.558 + miR.609 + miR.603 + miR.220a + miR.519b + miR.130a + miR.26a + miR.29a + miR.376a* + miR.494 + miR.518c + miR.639 + miR.338 | 0,81 | 0,031402705 |
| miR.558 + miR.609 + miR.603 + miR.520f + miR.220a + miR.519b + miR.130a + miR.29a + miR.376a* + miR.494 + miR.518c + miR.639 + miR.660 | 0,81 | 0,108309166 |
| miR.558 + miR.609 + miR.520f + miR.220a + miR.130a + miR.26a + miR.29a + miR.369.3p + miR.376a* + miR.494 + miR.518c + miR.639 + miR.660 | 0,81 | 0,108296254 |
| miR.558 + miR.609 + miR.603 + miR.520f + miR.26a + miR.29a + miR.362 + miR.369.3p + miR.376a* + miR.494 + miR.518c + miR.639 + miR.338 | 0,81 | 0,026737371 |
| miR.558 + miR.609 + miR.603 + miR.220a + miR.519b + miR.130a + miR.26a + miR.29a + miR.369.3p + miR.376a* + miR.518c + miR.639 + miR.338 | 0,81 | 0,122408948 |
| miR.558 + miR.609 + miR.520f + miR.519b + miR.130a + miR.26a + miR.29a + miR.369.3p + miR.376a^{*} + miR.518c + miR.639 + miR.660 + miR.338 | 0,81 | 0,10651772 |
| miR.558 + miR.609 + miR.520f + miR.130a + miR.26a + miR.29a + miR.362 + miR.369.3p + miR.376a^{*} + miR.494 + miR.518c + miR.639 + miR.660 | 0,81 | 0,034075449 |
| miR.558 + miR.609 + miR.520f + miR.220a + miR.519b + miR.26a + miR.29a + miR.362 + miR.369.3p + miR.376a* + miR.518c + miR.639 + miR.660 | 0,81 | 0,204425213 |
| miR.558 + miR.609 + miR.603 + miR.220a + miR.519b + miR.26a + miR.29a + miR.362 + miR.376a* + miR.494 + miR.518c + miR.639 + miR.338 | 0,81 | 0,092015336 |
| miR.558 + miR.609 + miR.603 + miR.520f + miR.220a + miR.519b + miR.130a + miR.29a + miR.369.3p + miR.376a* + miR.518c + miR.639 + miR.338 | 0,81 | 0,064440424 |
| miR.558 + miR.609 + miR.603 + miR.520f + miR.220a + miR.130a + miR.29a + miR.362 + miR.369.3p + miR.376a* + miR.494 + miR.518c + miR.639 | 0,81 | 0,046519555 |
| miR.558 + miR.609 + miR.520f + miR.220a + miR.519b + miR.29a + miR.362 + miR.369.3p + miR.376a* + miR.494 + miR.518c + miR.639 + miR.660 | 0,81 | 0,187256281 |
| miR.558 + miR.609 + miR.603 + miR.520f + miR.220a + miR.519b + miR.130a + miR.29a + miR.362 + miR.369.3p + miR.376a^{*} + miR.518c + miR.639 | 0,81 | 0,100998799 |
| miR.558 + miR.609 + miR.520f + miR.220a + miR.519b + miR.26a + miR.29a + miR.362 + miR.369.3p + miR.376a^{*} + miR.494 + miR.518c + miR.660 | 0,81 | 0,226326011 |
| miR.558 + miR.609 + miR.520f + miR.519b + miR.130a + miR.29a + miR.362 + miR.369.3p + miR.376a^{*} + miR.494 + miR.518c + miR.639 + miR.660 | 0,81 | 0,009030937 |
| miR.558 + miR.609 + miR.603 + miR.520f + miR.220a + miR.29a + miR.362 + miR.369.3p + miR.376a^{*} + miR.494 + miR.518c + miR.639 + miR.338 | 0,81 | 0,049864978 |
| miR.558 + miR.609 + miR.519b + miR.130a + miR.26a + miR.29a + miR.362 + miR.369.3p + miR.376a* + miR.494 + miR.518c + miR.639 + miR.660 | 0,81 | 0,066783621 |
| miR.558 + miR.609 + miR.603 + miR.520f + miR.220a + miR.519b + miR.130a + miR.26a + miR.29a + miR.369.3p + miR.376a^{*} + miR.518c + miR.639 | 0,81 | 0,118813626 |
| miR.558 + miR.609 + miR.603 + miR.520f + miR.220a + miR.29a + miR.362 + miR.376a* + miR.494 + miR.518c + miR.639 + miR.660 + miR.338 | 0,81 | 0,030176993 |
| miR.558 + miR.609 + miR.603 + miR.520f + miR.220a + miR.519b + miR.130a + miR.26a + miR.29a + miR.376a* + miR.494 + miR.518c + miR.660 | 0,81 | 0,123637498 |
| miR.558 + miR.609 + miR.520f + miR.220a + miR.519b + miR.130a + miR.26a + miR.29a + miR.369.3p + miR.376a* + miR.518c + miR.660 + miR.338 | 0,81 | 0,192757297 |
| miR.558 + miR.609 + miR.603 + miR.220a + miR.519b + miR.130a + miR.29a + miR.362 + miR.376a* + miR.494 + miR.518c + miR.660 + miR.338 | 0,81 | 0,062264897 |
| miR.558 + miR.609 + miR.520f + miR.519b + miR.130a + miR.26a + miR.29a + miR.362 + miR.369.3p + miR.376a* + miR.518c + miR.660 + miR.338 | 0,81 | 0,31004701 |
| miR.558 + miR.609 + miR.603 + miR.520f + miR.220a + miR.519b + miR.26a + miR.29a + miR.362 + miR.376a^{*} + miR.494 + miR.518c + miR.639 | 0,81 | 0,130242102 |
| miR.558 + miR.609 + miR.603 + miR.520f + miR.220a + miR.26a + miR.29a + miR.376a* + miR.494 + miR.518c + miR.639 + miR.660 + miR.338 | 0,81 | 0,029732961 |
| miR.558 + miR.609 + miR.520f + miR.220a + miR.130a + miR.26a + miR.29a + miR.362 + miR.369.3p + miR.376a* + miR.494 + miR.518c + miR.660 | 0,81 | 0,241293383 |
| miR.558 + miR.609 + miR.603 + miR.520f + miR.220a + miR.130a + miR.29a + miR.376a* + miR.494 + miR.518c + miR.639 + miR.660 + miR.338 | 0,81 | 0,026158188 |
| miR.558 + miR.609 + miR.603 + miR.220a + miR.519b + miR.130a + miR.29a + miR.376a* + miR.494 + miR.518c + miR.639 + miR.660 + miR.338 | 0,81 | 0,054322724 |
| miR.558 + miR.609 + miR.603 + miR.220a + miR.519b + miR.130a + miR.29a + miR.362 + miR.369.3p + miR.376a* + miR.518c + miR.639 + miR.338 | 0,81 | 0,097413097 |
| miR.558 + miR.609 + miR.603 + miR.520f + miR.130a + miR.29a + miR.362 + miR.369.3p + miR.376a* + miR.494 + miR.518c + miR.639 + miR.338 | 0,81 | 0,023024307 |
| miR.558 + miR.609 + miR.220a + miR.519b + miR.26a + miR.29a + miR.362 + miR.369.3p + miR.376a* + miR.494 + miR.518c + miR.639 + miR.660 | 0,81 | 0,200981447 |
| miR.558 + miR.609 + miR.220a + miR.519b + miR.130a + miR.29a + miR.362 + miR.369.3p + miR.376a* + miR.494 + miR.518c + miR.639 + miR.660 | 0,81 | 0,189218997 |
| miR.558 + miR.609 + miR.220a + miR.519b + miR.130a + miR.26a + miR.29a + miR.362 + miR.369.3p + miR.376a* + miR.518c + miR.660 + miR.338 | 0,81 | 0,371816229 |
| miR.558 + miR.609 + miR.220a + miR.519b + miR.130a + miR.26a + miR.29a + miR.369.3p + miR.376a* + miR.494 + miR.518c + miR.639 + miR.660 | 0,81 | 0,257892045 |
| miR.558 + miR.609 + miR.603 + miR.220a + miR.130a + miR.26a + miR.29a + miR.376a^{*} + miR.494 + miR.518c + miR.639 + miR.660 + miR.338 | 0,81 | 0,048311512 |
| miR.558 + miR.609 + miR.220a + miR.519b + miR.130a + miR.26a + miR.29a + miR.369.3p + miR.376a^{*} + miR.518c + miR.639 + miR.660 + miR.338 | 0,81 | 0,223974146 |
| miR.558 + miR.609 + miR.603 + miR.519b + miR.130a + miR.29a + miR.369.3p + miR.376a* + miR.494 + miR.518c + miR.639 + miR.660 + miR.338 | 0,81 | 0,104633759 |
| miR.558 + miR.609 + miR.520f + miR.220a + miR.519b + miR.130a + miR.29a + miR.362 + miR.369.3p + miR.376a^{*} + miR.494 + miR.518c + miR.660 | 0,81 | 0,31524463 |
| miR.558 + miR.609 + miR.520f + miR.519b + miR.26a + miR.29a + miR.369.3p + miR.376a^{*} + miR.494 + miR.518c + miR.639 + miR.660 + miR.338 | 0,81 | 0,169572806 |
| miR.558 + miR.609 + miR.520f + miR.220a + miR.519b + miR.26a + miR.29a + miR.369.3p + miR.376a* + miR.494 + miR.518c + miR.639 + miR.660 | 0,81 | 0,150095078 |
| miR.558 + miR.609 + miR.603 + miR.520f + miR.220a + miR.130a + miR.29a + miR.362 + miR.369.3p + miR.376a* + miR.518c + miR.639 + miR.338 | 0,81 | 0,052605777 |
| miR.558 + miR.609 + miR.603 + miR.520f + miR.130a + miR.26a + miR.29a + miR.362 + miR.369.3p + miR.376a* + miR.518c + miR.639 + miR.338 | 0,81 | 0,031102589 |
| miR.558 + miR.609 + miR.603 + miR.519b + miR.130a + miR.26a + miR.29a + miR.369.3p + miR.376a* + miR.518c + miR.639 + miR.660 + miR.338 | 0,81 | 0,071281412 |
| miR.558 + miR.609 + miR.220a + miR.519b + miR.26a + miR.29a + miR.369.3p + miR.376a* + miR.494 + miR.518c + miR.639 + miR.660 + miR.338 | 0,81 | 0,232704663 |
| miR.558 + miR.609 + miR.603 + miR.130a + miR.26a + miR.29a + miR.362 + miR.369.3p + miR.376a* + miR.494 + miR.518c + miR.639 + miR.338 | 0,81 | 0,088718228 |
| miR.558 + miR.609 + miR.603 + miR.520f + miR.220a + miR.130a + miR.29a + miR.369.3p + miR.376a* + miR.494 + miR.518c + miR.639 + miR.338 | 0,81 | 0,033031201 |
| miR.609 + miR.603 + miR.520f + miR.220a + miR.29a + miR.362 + miR.369.3p + miR.376a* + miR.494 + miR.518c + miR.639 + miR.660 + miR.338 | 0,81 | 0,386426673 |
| miR.558 + miR.609 + miR.520f + miR.519b + miR.130a + miR.26a + miR.29a + miR.369.3p + miR.376a* + miR.494 + miR.518c + miR.660 + miR.338 | 0,81 | 0,25269539 |
| miR.558 + miR.609 + miR.603 + miR.520f + miR.130a + miR.26a + miR.29a + miR.369.3p + miR.376a* + miR.494 + miR.518c + miR.660 + miR.338 | 0,81 | 0,072920527 |
| miR.558 + miR.609 + miR.603 + miR.520f + miR.220a + miR.519b + miR.29a + miR.362 + miR.376a* + miR.494 + miR.518c + miR.639 + miR.660 | 0,81 | 0,121094502 |
| miR.558 + miR.609 + miR.520f + miR.220a + miR.130a + miR.26a + miR.29a + miR.362 + miR.369.3p + miR.376a* + miR.518c + miR.639 + miR.660 | 0,81 | 0,242402407 |
| miR.558 + miR.609 + miR.603 + miR.520f + miR.26a + miR.29a + miR.362 + miR.369.3p + miR.376a* + miR.494 + miR.518c + miR.639 + miR.660 | 0,81 | 0,015891226 |
| miR.558 + miR.609 + miR.603 + miR.220a + miR.26a + miR.29a + miR.362 + miR.376a* + miR.494 + miR.518c + miR.639 + miR.660 + miR.338 | 0,81 | 0,054315882 |
| miR.558 + miR.609 + miR.603 + miR.520f + miR.130a + miR.26a + miR.29a + miR.369.3p + miR.376a* + miR.494 + miR.518c + miR.639 + miR.660 | 0,81 | 0,039648106 |
| miR.558 + miR.609 + miR.603 + miR.519b + miR.130a + miR.26a + miR.29a + miR.362 + miR.369.3p + miR.376a* + miR.518c + miR.660 + miR.338 | 0,81 | 0,09555985 |
| miR.558 + miR.609 + miR.603 + miR.519b + miR.26a + miR.29a + miR.362 + miR.369.3p + miR.376a* + miR.494 + miR.518c + miR.639 + miR.660 | 0,81 | 0,022074144 |
| miR.558 + miR.609 + miR.220a + miR.519b + miR.130a + miR.26a + miR.29a + miR.369.3p + miR.376a* + miR.494 + miR.518c + miR.660 + miR.338 | 0,81 | 0,254826732 |
| miR.558 + miR.609 + miR.603 + miR.520f + miR.519b + miR.26a + miR.29a + miR.369.3p + miR.376a* + miR.494 + miR.518c + miR.639 + miR.338 | 0,81 | 0,056003775 |
| miR.558 + miR.609 + miR.220a + miR.519b + miR.130a + miR.26a + miR.29a + miR.362 + miR.369.3p + miR.376a* + miR.494 + miR.518c + miR.660 | 0,81 | 0,345123625 |
| miR.558 + miR.609 + miR.520f + miR.220a + miR.519b + miR.130a + miR.26a + miR.29a + miR.369.3p + miR.376a* + miR.494 + miR.518c + miR.660 | 0,81 | 0,244119723 |
| miR.558 + miR.609 + miR.603 + miR.520f + miR.220a + miR.519b + miR.26a + miR.29a + miR.376a* + miR.494 + miR.518c + miR.639 + miR.660 | 0,81 | 0,117388442 |
| miR.558 + miR.609 + miR.603 + miR.520f + miR.220a + miR.519b + miR.26a + miR.29a + miR.362 + miR.376a* + miR.494 + miR.518c + miR.660 | 0,81 | 0,144644235 |
| miR.558 + miR.609 + miR.603 + miR.519b + miR.130a + miR.26a + miR.29a + miR.369.3p + miR.376a* + miR.494 + miR.518c + miR.639 + miR.660 | 0,81 | 0,097466046 |
| miR.558 + miR.609 + miR.603 + miR.520f + miR.519b + miR.130a + miR.26a + miR.29a + miR.369.3p + miR.376a* + miR.518c + miR.660 + miR.338 | 0,81 | 0,120253234 |
| miR.558 + miR.609 + miR.603 + miR.520f + miR.519b + miR.130a + miR.26a + miR.29a + miR.369.3p + miR.376a* + miR.494 + miR.518c + miR.338 | 0,81 | 0,049964592 |
| miR.558 + miR.609 + miR.520f + miR.220a + miR.519b + miR.130a + miR.29a + miR.369.3p + miR.376a* + miR.494 + miR.518c + miR.639 + miR.660 | 0,81 | 0,175789263 |
| miR.558 + miR.609 + miR.603 + miR.220a + miR.519b + miR.26a + miR.29a + miR.362 + miR.376a* + miR.494 + miR.518c + miR.660 + miR.338 | 0,81 | 0,102398037 |
| miR.558 + miR.609 + miR.603 + miR.520f + miR.220a + miR.26a + miR.29a + miR.362 + miR.376a* + miR.494 + miR.518c + miR.639 + miR.338 | 0,81 | 0,015203128 |
| miR.558 + miR.609 + miR.603 + miR.520f + miR.519b + miR.29a + miR.362 + miR.369.3p + miR.376a* + miR.494 + miR.518c + miR.639 + miR.338 | 0,81 | 0,045984998 |
| miR.558 + miR.609 + miR.603 + miR.520f + miR.519b + miR.130a + miR.29a + miR.362 + miR.369.3p + miR.376a* + miR.494 + miR.518c + miR.338 | 0,81 | 0,045712288 |
| miR.558 + miR.609 + miR.603 + miR.220a + miR.130a + miR.26a + miR.29a + miR.362 + miR.376a* + miR.494 + miR.518c + miR.639 + miR.660 | 0,81 | 0,046429079 |
| miR.558 + miR.609 + miR.603 + miR.220a + miR.519b + miR.29a + miR.362 + miR.369.3p + miR.376a* + miR.494 + miR.518c + miR.639 + miR.338 | 0,81 | 0,090865721 |
| miR.558 + miR.609 + miR.603 + miR.220a + miR.130a + miR.29a + miR.362 + miR.369.3p + miR.376a* + miR.494 + miR.518c + miR.639 + miR.338 | 0,81 | 0,02698096 |
| miR.558 + miR.609 + miR.603 + miR.520f + miR.519b + miR.130a + miR.29a + miR.369.3p + miR.376a* + miR.494 + miR.518c + miR.639 + miR.660 | 0,81 | 0,05172435 |
| miR.558 + miR.609 + miR.603 + miR.220a + miR.130a + miR.26a + miR.29a + miR.362 + miR.376a* + miR.494 + miR.518c + miR.639 + miR.338 | 0,81 | 0,033364716 |
| miR.558 + miR.609 + miR.603 + miR.520f + miR.519b + miR.26a + miR.29a + miR.362 + miR.369.3p + miR.376a* + miR.518c + miR.639 + miR.338 | 0,81 | 0,04386086 |
| miR.558 + miR.609 + miR.603 + miR.520f + miR.519b + miR.26a + miR.29a + miR.362 + miR.369.3p + miR.376a* + miR.494 + miR.518c + miR.338 | 0,81 | 0,057183959 |
| miR.558 + miR.609 + miR.520f + miR.220a + miR.26a + miR.29a + miR.362 + miR.369.3p + miR.376a* + miR.494 + miR.518c + miR.639 + miR.338 | 0,81 | 0,045132674 |
| miR.558 + miR.609 + miR.603 + miR.520f + miR.519b + miR.26a + miR.29a + miR.362 + miR.369.3p + miR.376a* + miR.518c + miR.639 + miR.660 | 0,81 | 0,018395196 |
| miR.609 + miR.603 + miR.520f + miR.220a + miR.519b + miR.29a + miR.362 + miR.369.3p + miR.376a* + miR.494 + miR.518c + miR.660 + miR.338 | 0,81 | 0,459736758 |
| miR.558 + miR.609 + miR.603 + miR.220a + miR.519b + miR.26a + miR.29a + miR.376a* + miR.494 + miR.518c + miR.639 + miR.660 + miR.338 | 0,81 | 0,088404933 |
| miR.558 + miR.609 + miR.603 + miR.520f + miR.220a + miR.519b + miR.26a + miR.29a + miR.362 + miR.376a* + miR.518c + miR.639 + miR.338 | 0,81 | 0,080411041 |
| miR.558 + miR.609 + miR.520f + miR.220a + miR.130a + miR.26a + miR.29a + miR.369.3p + miR.376a* + miR.494 + miR.518c + miR.639 + miR.338 | 0,81 | 0,045221056 |
| miR.558 + miR.609 + miR.603 + miR.520f + miR.519b + miR.130a + miR.26a + miR.29a + miR.369.3p + miR.376a* + miR.518c + miR.639 + miR.338 | 0,81 | 0,042179126 |
| miR.558 + miR.609 + miR.520f + miR.220a + miR.130a + miR.29a + miR.362 + miR.376a* + miR.494 + miR.518c + miR.639 + miR.660 + miR.338 | 0,81 | 0,010997913 |
| miR.558 + miR.609 + miR.603 + miR.520f + miR.220a + miR.519b + miR.130a + miR.29a + miR.376a* + miR.494 + miR.518c + miR.639 + miR.338 | 0,81 | 0,018417286 |
| miR.609 + miR.603 + miR.220a + miR.26a + miR.29a + miR.362 + miR.369.3p + miR.376a* + miR.494 + miR.518c + miR.639 + miR.660 + miR.338 | 0,81 | 0,361025124 |
| miR.558 + miR.609 + miR.603 + miR.220a + miR.519b + miR.26a + miR.29a + miR.362 + miR.376a* + miR.494 + miR.518c + miR.639 + miR.660 | 0,81 | 0,117927725 |
| miR.558 + miR.609 + miR.603 + miR.520f + miR.220a + miR.519b + miR.130a + miR.29a + miR.362 + miR.376a* + miR.518c + miR.639 + miR.338 | 0,81 | 0,067829062 |
| miR.558 + miR.609 + miR.603 + miR.520f + miR.220a + miR.519b + miR.130a + miR.26a + miR.29a + miR.362 + miR.376a* + miR.518c + miR.338 | 0,81 | 0,092831003 |
| miR.558 + miR.609 + miR.520f + miR.220a + miR.519b + miR.130a + miR.26a + miR.29a + miR.369.3p + miR.376a* + miR.518c + miR.639 + miR.660 | 0,81 | 0,18155177 |
| miR.558 + miR.609 + miR.520f + miR.220a + miR.519b + miR.130a + miR.29a + miR.362 + miR.369.3p + miR.376a* + miR.518c + miR.639 + miR.660 | 0,81 | 0,241729871 |
| miR.558 + miR.609 + miR.603 + miR.520f + miR.220a + miR.130a + miR.26a + miR.29a + miR.376a* + miR.494 + miR.518c + miR.639 + miR.660 | 0,81 | 0,050900165 |
| miR.558 + miR.609 + miR.520f + miR.220a + miR.519b + miR.130a + miR.26a + miR.29a + miR.362 + miR.369.3p + miR.376a* + miR.518c + miR.660 | 0,81 | 0,262366457 |
| miR.558 + miR.609 + miR.603 + miR.519b + miR.130a + miR.29a + miR.362 + miR.369.3p + miR.376a* + miR.494 + miR.518c + miR.639 + miR.660 | 0,81 | 0,079200578 |
| miR.558 + miR.609 + miR.220a + miR.519b + miR.29a + miR.362 + miR.369.3p + miR.376a* + miR.494 + miR.518c + miR.639 + miR.660 + miR.338 | 0,81 | 0,225050342 |
| miR.558 + miR.609 + miR.603 + miR.520f + miR.220a + miR.130a + miR.29a + miR.362 + miR.376a* + miR.494 + miR.518c + miR.639 + miR.338 | 0,81 | 0,013924727 |
| miR.558 + miR.609 + miR.603 + miR.520f + miR.220a + miR.519b + miR.130a + miR.26a + miR.29a + miR.376a* + miR.494 + miR.518c + miR.338 | 0,81 | 0,026721962 |
| miR.558 + miR.609 + miR.603 + miR.519b + miR.29a + miR.362 + miR.369.3p + miR.376a* + miR.494 + miR.518c + miR.639 + miR.660 + miR.338 | 0,81 | 0,04508522 |
| miR.558 + miR.609 + miR.520f + miR.519b + miR.130a + miR.26a + miR.29a + miR.369.3p + miR.376a* + miR.494 + miR.518c + miR.639 + miR.660 | 0,81 | 0,088505592 |
| miR.558 + miR.609 + miR.603 + miR.520f + miR.220a + miR.519b + miR.26a + miR.29a + miR.376a* + miR.494 + miR.518c + miR.639 + miR.338 | 0,81 | 0,031154613 |
| miR.558 + miR.609 + miR.603 + miR.520f + miR.519b + miR.29a + miR.362 + miR.369.3p + miR.376a* + miR.494 + miR.518c + miR.639 + miR.660 | 0,81 | 0,005118879 |
| miR.558 + miR.609 + miR.520f + miR.220a + miR.519b + miR.130a + miR.29a + miR.376a* + miR.494 + miR.518c + miR.639 + miR.660 + miR.338 | 0,81 | 0,069586412 |
| miR.558 + miR.609 + miR.520f + miR.220a + miR.519b + miR.29a + miR.362 + miR.376a* + miR.494 + miR.518c + miR.639 + miR.660 + miR.338 | 0,81 | 0,067533792 |
| miR.558 + miR.609 + miR.603 + miR.520f + miR.220a + miR.519b + miR.130a + miR.26a + miR.29a + miR.362 + miR.376a* + miR.518c + miR.639 | 0,81 | 0,095777108 |
| miR.558 + miR.609 + miR.220a + miR.519b + miR.130a + miR.26a + miR.29a + miR.362 + miR.369.3p + miR.376a* + miR.518c + miR.639 + miR.660 | 0,81 | 0,300787646 |
| miR.609 + miR.603 + miR.520f + miR.130a + miR.29a + miR.362 + miR.369.3p + miR.376a* + miR.494 + miR.518c + miR.639 + miR.660 + miR.338 | 0,81 | 0,40673594 |
| miR.558 + miR.609 + miR.603 + miR.520f + miR.519b + miR.130a + miR.29a + miR.362 + miR.369.3p + miR.376a* + miR.518c + miR.639 + miR.338 | 0,81 | 0,048782077 |
| miR.558 + miR.609 + miR.520f + miR.130a + miR.26a + miR.29a + miR.362 + miR.376a* + miR.494 + miR.518c + miR.639 + miR.660 + miR.338 | 0,81 | 0,010597746 |
| miR.558 + miR.609 + miR.520f + miR.220a + miR.130a + miR.26a + miR.29a + miR.362 + miR.369.3p + miR.376a* + miR.494 + miR.518c + miR.338 | 0,81 | 0,034718922 |
| miR.558 + miR.609 + miR.603 + miR.520f + miR.220a + miR.130a + miR.26a + miR.29a + miR.362 + miR.376a* + miR.518c + miR.639 + miR.338 | 0,81 | 0,018119977 |
| miR.609 + miR.603 + miR.520f + miR.519b + miR.29a + miR.362 + miR.369.3p + miR.376a* + miR.494 + miR.518c + miR.639 + miR.660 + miR.338 | 0,81 | 0,385849057 |
| miR.558 + miR.609 + miR.520f + miR.220a + miR.26a + miR.29a + miR.362 + miR.376a + miR.494 + miR.518c + miR.639 + miR.660 + miR.338 | 0,81 | 0,02249848 |
| miR.558 + miR.609 + miR.520f + miR.220a + miR.130a + miR.26a + miR.29a + miR.376a* + miR.494 + miR.518c + miR.639 + miR.660 + miR.338 | 0,81 | 0,016477681 |
| miR.558 + miR.609 + miR.220a + miR.519b + miR.130a + miR.29a + miR.369.3p + miR.376a* + miR.494 + miR.518c + miR.639 + miR.660 + miR.338 | 0,81 | 0,244670126 |
| miR.558 + miR.609 + miR.603 + miR.520f + miR.220a + miR.130a + miR.29a + miR.362 + miR.376a* + miR.494 + miR.518c + miR.639 + miR.660 | 0,81 | 0,046165598 |
| miR.558 + miR.609 + miR.603 + miR.520f + miR.220a + miR.130a + miR.26a + miR.29a + miR.376a* + miR.494 + miR.518c + miR.660 + miR.338 | 0,81 | 0,05165049 |
| miR.558 + miR.609 + miR.520f + miR.220a + miR.519b + miR.26a + miR.29a + miR.362 + miR.369.3p + miR.376a* + miR.494 + miR.518c + miR.338 | 0,81 | 0,075443189 |
| miR.558 + miR.609 + miR.519b + miR.130a + miR.26a + miR.29a + miR.362 + miR.369.3p + miR.376a* + miR.494 + miR.518c + miR.660 + miR.338 | 0,81 | 0,30874575 |
| miR.558 + miR.609 + miR.520f + miR.220a + miR.519b + miR.26a + miR.29a + miR.376a* + miR.494 + miR.518c + miR.639 + miR.660 + miR.338 | 0,81 | 0,073319372 |
| miR.558 + miR.609 + miR.520f + miR.519b + miR.130a + miR.29a + miR.362 + miR.376a* + miR.494 + miR.518c + miR.639 + miR.660 + miR.338 | 0,81 | 0,042740219 |
| miR.609 + miR.603 + miR.520f + miR.26a + miR.29a + miR.362 + miR.369.3p + miR.376a* + miR.494 + miR.518c + miR.639 + miR.660 + miR.338 | 0,81 | 0,471600557 |
| miR.558 + miR.609 + miR.603 + miR.520f + miR.220a + miR.26a + miR.29a + miR.362 + miR.376a* + miR.494 + miR.518c + miR.660 + miR.338 | 0,81 | 0,050276267 |
| miR.558 + miR.609 + miR.603 + miR.520f + miR.220a + miR.519b + miR.130a + miR.26a + miR.29a + miR.376a* + miR.494 + miR.518c + miR.639 | 0,81 | 0,055150859 |
| miR.558 + miR.609 + miR.603 + miR.520f + miR.220a + miR.130a + miR.26a + miR.29a + miR.376a* + miR.518c + miR.639 + miR.660 + miR.338 | 0,81 | 0,033222739 |
| miR.609 + miR.603 + miR.520f + miR.220a + miR.519b + miR.29a + miR.362 + miR.369.3p + miR.376a^{*} + miR.518c + miR.639 + miR.660 + miR.338 | 0,81 | 0,38615574 |
| miR.558 + miR.609 + miR.603 + miR.520f + miR.519b + miR.130a + miR.362 + miR.369.3p + miR.376a* + miR.518c + miR.639 + miR.660 + miR.338 | 0,81 | 0,132875689 |
| miR.558 + miR.609 + miR.520f + miR.220a + miR.130a + miR.29a + miR.362 + miR.369.3p + miR.376a* + miR.494 + miR.518c + miR.639 + miR.338 | 0,81 | 0,032927583 |
| miR.558 + miR.609 + miR.603 + miR.520f + miR.220a + miR.26a + miR.29a + miR.362 + miR.376a* + miR.494 + miR.518c + miR.639 + miR.660 | 0,81 | 0,048454383 |
| miR.558 + miR.609 + miR.603 + miR.520f + miR.519b + miR.29a + miR.362 + miR.376a* + miR.494 + miR.518c + miR.639 + miR.660 + miR.338 | 0,81 | 0,01610058 |
| miR.558 + miR.609 + miR.603 + miR.520f + miR.220a + miR.519b + miR.26a + miR.29a + miR.362 + miR.376a* + miR.518c + miR.639 + miR.660 | 0,81 | 0,128991474 |
| miR.558 + miR.609 + miR.603 + miR.520f + miR.519b + miR.130a + miR.29a + miR.376a* + miR.494 + miR.518c + miR.639 + miR.660 + miR.338 | 0,81 | 0,023807371 |
| miR.558 + miR.609 + miR.603 + miR.520f + miR.220a + miR.130a + miR.362 + miR.369.3p + miR.376a* + miR.518c + miR.639 + miR.660 + miR.338 | 0,81 | 0,192196845 |
| miR.609 + miR.603 + miR.520f + miR.220a + miR.519b + miR.29a + miR.369.3p + miR.376a* + miR.494 + miR.518c + miR.639 + miR.660 + miR.338 | 0,81 | 0,128030782 |
| miR.558 + miR.609 + miR.603 + miR.520f + miR.220a + miR.26a + miR.29a + miR.362 + miR.376a* + miR.518c + miR.639 + miR.660 + miR.338 | 0,81 | 0,03202411 |
| miR.558 + miR.609 + miR.603 + miR.520f + miR.519b + miR.130a + miR.29a + miR.362 + miR.376a* + miR.494 + miR.518c + miR.639 + miR.338 | 0,81 | 0,034099937 |
| miR.558 + miR.609 + miR.520f + miR.220a + miR.519b + miR.26a + miR.29a + miR.369.3p + miR.376a* + miR.494 + miR.518c + miR.639 + miR.338 | 0,81 | 0,079452257 |
| miR.558 + miR.609 + miR.603 + miR.520f + miR.519b + miR.26a + miR.29a + miR.369.3p + miR.376a* + miR.494 + miR.518c + miR.639 + miR.660 | 0,81 | 0,027326002 |
| miR.558 + miR.609 + miR.603 + miR.520f + miR.220a + miR.362 + miR.369.3p + miR.376a* + miR.494 + miR.518c + miR.639 + miR.660 + miR.338 | 0,81 | 0,114463692 |
| miR.558 + miR.609 + miR.603 + miR.519b + miR.130a + miR.26a + miR.29a + miR.362 + miR.369.3p + miR.376a* + miR.518c + miR.639 + miR.660 | 0,81 | 0,059308071 |
| miR.558 + miR.609 + miR.603 + miR.220a + miR.519b + miR.130a + miR.26a + miR.29a + miR.362 + miR.376a* + miR.518c + miR.639 + miR.338 | 0,81 | 0,089768465 |
| miR.558 + miR.609 + miR.603 + miR.520f + miR.519b + miR.130a + miR.26a + miR.29a + miR.369.3p + miR.376a* + miR.494 + miR.518c + miR.639 | 0,81 | 0,052541728 |
| miR.558 + miR.609 + miR.520f + miR.130a + miR.26a + miR.29a + miR.362 + miR.369.3p + miR.376a* + miR.494 + miR.518c + miR.639 + miR.338 | 0,81 | 0,075210281 |
| miR.558 + miR.609 + miR.520f + miR.220a + miR.130a + miR.26a + miR.29a + miR.362 + miR.369.3p + miR.376a* + miR.518c + miR.639 + miR.338 | 0,81 | 0,061056171 |
| miR.558 + miR.609 + miR.603 + miR.519b + miR.130a + miR.29a + miR.362 + miR.369.3p + miR.376a* + miR.494 + miR.518c + miR.660 + miR.338 | 0,81 | 0,11034628 |
| miR.558 + miR.609 + miR.603 + miR.520f + miR.220a + miR.130a + miR.29a + miR.362 + miR.376a* + miR.518c + miR.639 + miR.660 + miR.338 | 0,81 | 0,036030433 |
| miR.558 + miR.609 + miR.603 + miR.520f + miR.220a + miR.519b + miR.130a + miR.29a + miR.362 + miR.376a* + miR.518c + miR.639 + miR.660 | 0,81 | 0,10661972 |
| miR.558 + miR.609 + miR.603 + miR.519b + miR.130a + miR.26a + miR.29a + miR.362 + miR.369.3p + miR.376a* + miR.518c + miR.639 + miR.338 | 0,81 | 0,081410858 |
| miR.558 + miR.609 + miR.603 + miR.520f + miR.220a + miR.519b + miR.130a + miR.26a + miR.29a + miR.362 + miR.376a* + miR.518c + miR.660 | 0,81 | 0,134906596 |
| miR.558 + miR.609 + miR.520f + miR.220a + miR.519b + miR.29a + miR.362 + miR.369.3p + miR.376a* + miR.494 + miR.518c + miR.639 + miR.338 | 0,81 | 0,104596229 |
| miR.558 + miR.609 + miR.603 + miR.520f + miR.220a + miR.130a + miR.29a + miR.362 + miR.376a* + miR.494 + miR.518c + miR.660 + miR.338 | 0,81 | 0,049421668 |
| miR.558 + miR.609 + miR.519b + miR.130a + miR.26a + miR.29a + miR.369.3p + miR.376a* + miR.494 + miR.518c + miR.639 + miR.660 + miR.338 | 0,81 | 0,212933566 |
| miR.558 + miR.609 + miR.603 + miR.520f + miR.130a + miR.26a + miR.362 + miR.369.3p + miR.376a* + miR.518c + miR.639 + miR.660 + miR.338 | 0,81 | 0,197008094 |
| miR.558 + miR.609 + miR.603 + miR.520f + miR.130a + miR.26a + miR.29a + miR.362 + miR.369.3p + miR.376a* + miR.494 + miR.518c + miR.338 | 0,81 | 0,031984849 |
| miR.609 + miR.603 + miR.520f + miR.220a + miR.130a + miR.29a + miR.362 + miR.369.3p + miR.376a* + miR.494 + miR.518c + miR.660 + miR.338 | 0,81 | 0,403062744 |
| miR.609 + miR.520f + miR.220a + miR.519b + miR.29a + miR.362 + miR.369.3p + miR.376a* + miR.494 + miR.518c + miR.639 + miR.660 + miR.338 | 0,81 | 0,320775769 |
| miR.558 + miR.609 + miR.520f + miR.519b + miR.130a + miR.26a + miR.29a + miR.362 + miR.369.3p + miR.376a* + miR.518c + miR.639 + miR.660 | 0,81 | 0,020618254 |
| miR.558 + miR.609 + miR.520f + miR.220a + miR.519b + miR.130a + miR.29a + miR.362 + miR.369.3p + miR.376a* + miR.494 + miR.518c + miR.338 | 0,81 | 0,094797523 |
| miR.558 + miR.609 + miR.603 + miR.519b + miR.130a + miR.29a + miR.362 + miR.369.3p + miR.376a* + miR.494 + miR.518c + miR.639 + miR.338 | 0,81 | 0,076381383 |
| miR.558 + miR.609 + miR.220a + miR.519b + miR.130a + miR.26a + miR.29a + miR.369.3p + miR.376a* + miR.494 + miR.518c + miR.639 + miR.338 | 0,81 | 0,066389273 |
| miR.558 + miR.609 + miR.520f + miR.220a + miR.519b + miR.130a + miR.26a + miR.29a + miR.369.3p + miR.376a* + miR.494 + miR.518c + miR.338 | 0,81 | 0,066096791 |
| miR.558 + miR.609 + miR.603 + miR.519b + miR.26a + miR.29a + miR.369.3p + miR.376a* + miR.494 + miR.518c + miR.639 + miR.660 + miR.338 | 0,81 | 0,067564173 |
| miR.558 + miR.609 + miR.603 + miR.220a + miR.519b + miR.26a + miR.29a + miR.362 + miR.376a* + miR.518c + miR.639 + miR.660 + miR.338 | 0,81 | 0,083413289 |
| miR.558 + miR.609 + miR.603 + miR.520f + miR.220a + miR.26a + miR.362 + miR.369.3p + miR.376a* + miR.518c + miR.639 + miR.660 + miR.338 | 0,81 | 0,155299782 |
| miR.558 + miR.609 + miR.603 + miR.519b + miR.130a + miR.26a + miR.29a + miR.362 + miR.369.3p + miR.376a* + miR.494 + miR.518c + miR.639 | 0,81 | 0,077741167 |
| miR.558 + miR.609 + miR.603 + miR.520f + miR.220a + miR.130a + miR.26a + miR.29a + miR.362 + miR.376a* + miR.494 + miR.518c + miR.338 | 0,81 | 0,021219669 |
| miR.558 + miR.609 + miR.603 + miR.520f + miR.130a + miR.26a + miR.29a + miR.362 + miR.369.3p + miR.376a* + miR.518c + miR.639 + miR.660 | 0,81 | 0,053951448 |
| miR.609 + miR.603 + miR.520f + miR.220a + miR.26a + miR.29a + miR.362 + miR.369.3p + miR.376a* + miR.494 + miR.518c + miR.660 + miR.338 | 0,81 | 0,443982258 |
| miR.558 + miR.609 + miR.603 + miR.520f + miR.220a + miR.519b + miR.362 + miR.369.3p + miR.376a* + miR.518c + miR.639 + miR.660 + miR.338 | 0,81 | 0,11173385 |
| miR.558 + miR.609 + miR.603 + miR.220a + miR.519b + miR.130a + miR.26a + miR.29a + miR.362 + miR.376a* + miR.518c + miR.660 + miR.338 | 0,81 | 0,072404804 |
| miR.558 + miR.609 + miR.603 + miR.520f + miR.220a + miR.130a + miR.26a + miR.29a + miR.362 + miR.376a* + miR.494 + miR.518c + miR.639 | 0,81 | 0,026378889 |
| miR.558 + miR.609 + miR.603 + miR.520f + miR.220a + miR.519b + miR.29a + miR.362 + miR.376a* + miR.518c + miR.639 + miR.660 + miR.338 | 0,81 | 0,077778662 |
| miR.558 + miR.609 + miR.603 + miR.520f + miR.220a + miR.519b + miR.130a + miR.29a + miR.376a* + miR.518c + miR.639 + miR.660 + miR.338 | 0,81 | 0,103228828 |
| miR.558 + miR.609 + miR.603 + miR.520f + miR.519b + miR.130a + miR.29a + miR.362 + miR.369.3p + miR.376a* + miR.494 + miR.518c + miR.639 | 0,81 | 0,05694836 |
| miR.558 + miR.609 + miR.520f + miR.220a + miR.519b + miR.26a + miR.29a + miR.362 + miR.376a* + miR.494 + miR.518c + miR.660 + miR.338 | 0,81 | 0,114359219 |
| miR.558 + miR.609 + miR.603 + miR.520f + miR.130a + miR.29a + miR.362 + miR.376a* + miR.494 + miR.518c + miR.639 + miR.660 + miR.338 | 0,81 | 0,010084958 |
| miR.558 + miR.609 + miR.603 + miR.520f + miR.26a + miR.29a + miR.362 + miR.376a* + miR.494 + miR.518c + miR.639 + miR.660 + miR.338 | 0,81 | 0,009887988 |
| miR.558 + miR.609 + miR.220a + miR.519b + miR.130a + miR.26a + miR.29a + miR.362 + miR.369.3p + miR.376a* + miR.494 + miR.518c + miR.338 | 0,81 | 0,089903015 |
| miR.558 + miR.609 + miR.520f + miR.220a + miR.519b + miR.130a + miR.29a + miR.362 + miR.376a* + miR.494 + miR.518c + miR.660 + miR.338 | 0,81 | 0,150029262 |
| miR.558 + miR.609 + miR.520f + miR.519b + miR.130a + miR.26a + miR.29a + miR.369.3p + miR.376a* + miR.494 + miR.518c + miR.639 + miR.338 | 0,81 | 0,088836809 |
| miR.558 + miR.609 + miR.603 + miR.520f + miR.519b + miR.130a + miR.26a + miR.29a + miR.362 + miR.369.3p + miR.376a* + miR.518c + miR.338 | 0,81 | 0,059390274 |
| miR.609 + miR.603 + miR.520f + miR.220a + miR.519b + miR.29a + miR.362 + miR.369.3p + miR.376a* + miR.494 + miR.518c + miR.639 + miR.660 | 0,81 | 0,13064767 |
| miR.558 + miR.609 + miR.603 + miR.520f + miR.519b + miR.26a + miR.29a + miR.376a* + miR.494 + miR.518c + miR.639 + miR.660 + miR.338 | 0,81 | 0,023953942 |
| miR.558 + miR.609 + miR.520f + miR.220a + miR.519b + miR.130a + miR.26a + miR.29a + miR.376a* + miR.494 + miR.518c + miR.660 + miR.338 | 0,81 | 0,157618381 |
| miR.558 + miR.609 + miR.603 + miR.220a + miR.519b + miR.130a + miR.26a + miR.29a + miR.376a* + miR.518c + miR.639 + miR.660 + miR.338 | 0,81 | 0,069866539 |
| miR.558 + miR.609 + miR.603 + miR.520f + miR.220a + miR.519b + miR.26a + miR.29a + miR.376a* + miR.518c + miR.639 + miR.660 + miR.338 | 0,81 | 0,090434072 |
| miR.558 + miR.609 + miR.220a + miR.130a + miR.26a + miR.29a + miR.362 + miR.369.3p + miR.376a* + miR.494 + miR.518c + miR.639 + miR.338 | 0,81 | 0,058237304 |
| miR.609 + miR.603 + miR.520f + miR.220a + miR.26a + miR.29a + miR.362 + miR.369.3p + miR.376a* + miR.494 + miR.518c + miR.639 + miR.660 | 0,81 | 0,099984841 |
| miR.558 + miR.609 + miR.520f + miR.220a + miR.519b + miR.130a + miR.29a + miR.362 + miR.376a* + miR.494 + miR.518c + miR.639 + miR.338 | 0,81 | 0,092166906 |
| miR.558 + miR.609 + miR.603 + miR.520f + miR.220a + miR.130a + miR.26a + miR.29a + miR.362 + miR.376a* + miR.518c + miR.639 + miR.660 | 0,81 | 0,07279787 |
| miR.609 + miR.603 + miR.520f + miR.220a + miR.519b + miR.26a + miR.29a + miR.369.3p + miR.376a* + miR.494 + miR.518c + miR.660 + miR.338 | 0,81 | 0,12642831 |
| miR.558 + miR.609 + miR.520f + miR.220a + miR.519b + miR.130a + miR.29a + miR.369.3p + miR.376a* + miR.494 + miR.518c + miR.639 + miR.338 | 0,81 | 0,064322743 |
| miR.558 + miR.609 + miR.603 + miR.520f + miR.130a + miR.26a + miR.29a + miR.362 + miR.369.3p + miR.376a* + miR.494 + miR.518c + miR.639 | 0,81 | 0,033837657 |
| miR.609 + miR.603 + miR.520f + miR.220a + miR.519b + miR.130a + miR.29a + miR.369.3p + miR.376a* + miR.494 + miR.518c + miR.660 + miR.338 | 0,81 | 0,148315589 |
| miR.558 + miR.609 + miR.603 + miR.520f + miR.130a + miR.26a + miR.29a + miR.376a* + miR.494 + miR.518c + miR.639 + miR.660 + miR.338 | 0,81 | 0,014773319 |
| miR.558 + miR.609 + miR.520f + miR.220a + miR.519b + miR.26a + miR.29a + miR.362 + miR.369.3p + miR.376a* + miR.494 + miR.518c + miR.639 | 0,81 | 0,132856533 |
| miR.558 + miR.609 + miR.603 + miR.220a + miR.130a + miR.26a + miR.29a + miR.362 + miR.376a* + miR.494 + miR.518c + miR.660 + miR.338 | 0,81 | 0,044475068 |
| miR.558 + miR.609 + miR.520f + miR.220a + miR.130a + miR.26a + miR.29a + miR.362 + miR.369.3p + miR.376a* + miR.494 + miR.518c + miR.639 | 0,81 | 0,054075693 |
| miR.558 + miR.609 + miR.603 + miR.520f + miR.220a + miR.130a + miR.26a + miR.29a + miR.362 + miR.376a* + miR.518c + miR.660 + miR.338 | 0,81 | 0,043628889 |
| miR.609 + miR.603 + miR.520f + miR.220a + miR.130a + miR.29a + miR.362 + miR.369.3p + miR.376a* + miR.494 + miR.518c + miR.639 + miR.660 | 0,81 | 0,069759092 |
| miR.609 + miR.603 + miR.220a + miR.519b + miR.26a + miR.29a + miR.362 + miR.369.3p + miR.376a* + miR.494 + miR.518c + miR.639 + miR.660 | 0,81 | 0,194638112 |
| miR.558 + miR.609 + miR.520f + miR.519b + miR.130a + miR.29a + miR.362 + miR.369.3p + miR.376a* + miR.494 + miR.518c + miR.639 + miR.338 | 0,81 | 0,064801749 |
| miR.558 + miR.609 + miR.520f + miR.519b + miR.130a + miR.26a + miR.29a + miR.376a* + miR.494 + miR.518c + miR.639 + miR.660 + miR.338 | 0,81 | 0,072759393 |
| miR.558 + miR.609 + miR.603 + miR.520f + miR.220a + miR.519b + miR.130a + miR.26a + miR.29a + miR.376a* + miR.518c + miR.639 + miR.660 | 0,81 | 0,109795784 |
| miR.558 + miR.609 + miR.520f + miR.220a + miR.519b + miR.26a + miR.29a + miR.362 + miR.376a* + miR.494 + miR.518c + miR.639 + miR.338 | 0,81 | 0,129823773 |
| miR.558 + miR.609 + miR.520f + miR.220a + miR.519b + miR.130a + miR.26a + miR.29a + miR.362 + miR.369.3p + miR.376a* + miR.494 + miR.518c | 0,81 | 0,139084816 |
| miR.558 + miR.609 + miR.603 + miR.520f + miR.220a + miR.130a + miR.26a + miR.29a + miR.376a* + miR.494 + miR.518c + miR.639 + miR.338 | 0,81 | 0,011172234 |
| miR.558 + miR.609 + miR.220a + miR.519b + miR.26a + miR.29a + miR.362 + miR.369.3p + miR.376a* + miR.494 + miR.518c + miR.639 + miR.338 | 0,81 | 0,050520222 |
| miR.558 + miR.609 + miR.520f + miR.220a + miR.519b + miR.130a + miR.26a + miR.29a + miR.362 + miR.376a* + miR.494 + miR.518c + miR.338 | 0,81 | 0,14375355 |
| miR.558 + miR.609 + miR.603 + miR.520f + miR.519b + miR.130a + miR.26a + miR.29a + miR.369.3p + miR.376a* + miR.518c + miR.639 + miR.660 | 0,81 | 0,05862995 |
| miR.558 + miR.609 + miR.520f + miR.220a + miR.519b + miR.26a + miR.29a + miR.362 + miR.369.3p + miR.376a* + miR.518c + miR.639 + miR.338 | 0,81 | 0,102879027 |
| miR.558 + miR.609 + miR.603 + miR.520f + miR.220a + miR.519b + miR.130a + miR.29a + miR.362 + miR.376a* + miR.518c + miR.660 + miR.338 | 0,81 | 0,10448496 |
| miR.558 + miR.609 + miR.603 + miR.520f + miR.220a + miR.130a + miR.26a + miR.29a + miR.362 + miR.376a* + miR.494 + miR.518c + miR.660 | 0,81 | 0,056900306 |
| miR.558 + miR.609 + miR.603 + miR.220a + miR.519b + miR.130a + miR.26a + miR.29a + miR.362 + miR.376a* + miR.518c + miR.639 + miR.660 | 0,81 | 0,105228026 |
| miR.558 + miR.609 + miR.603 + miR.520f + miR.220a + miR.519b + miR.130a + miR.26a + miR.29a + miR.376a* + miR.518c + miR.660 + miR.338 | 0,81 | 0,102851595 |
| miR.609 + miR.603 + miR.220a + miR.130a + miR.26a + miR.29a + miR.362 + miR.369.3p + miR.376a* + miR.494 + miR.518c + miR.639 + miR.660 | 0,81 | 0,156983946 |
| miR.558 + miR.609 + miR.520f + miR.519b + miR.26a + miR.29a + miR.362 + miR.369.3p + miR.376a* + miR.494 + miR.518c + miR.639 + miR.660 | 0,81 | 0,011731119 |
| miR.558 + miR.609 + miR.603 + miR.520f + miR.519b + miR.130a + miR.29a + miR.362 + miR.369.3p + miR.376a* + miR.518c + miR.639 + miR.660 | 0,81 | 0,014291383 |
| miR.609 + miR.603 + miR.520f + miR.220a + miR.130a + miR.29a + miR.362 + miR.369.3p + miR.376a* + miR.518c + miR.639 + miR.660 + miR.338 | 0,81 | 0,363021363 |
| miR.558 + miR.609 + miR.520f + miR.220a + miR.519b + miR.130a + miR.26a + miR.29a + miR.362 + miR.369.3p + miR.376a* + miR.518c + miR.338 | 0,81 | 0,084537667 |
| miR.609 + miR.603 + miR.220a + miR.519b + miR.26a + miR.29a + miR.362 + miR.369.3p + miR.376a* + miR.494 + miR.518c + miR.660 + miR.338 | 0,81 | 0,419295859 |
| miR.558 + miR.609 + miR.603 + miR.519b + miR.26a + miR.29a + miR.362 + miR.369.3p + miR.376a* + miR.494 + miR.518c + miR.639 + miR.338 | 0,81 | 0,056449177 |
| miR.558 + miR.609 + miR.603 + miR.520f + miR.220a + miR.519b + miR.26a + miR.29a + miR.362 + miR.376a* + miR.518c + miR.660 + miR.338 | 0,81 | 0,105855453 |
| miR.558 + miR.609 + miR.520f + miR.220a + miR.130a + miR.29a + miR.362 + miR.369.3p + miR.376a* + miR.494 + miR.639 + miR.660 + miR.338 | 0,81 | 0,296783286 |
| miR.558 + miR.609 + miR.603 + miR.220a + miR.130a + miR.26a + miR.29a + miR.362 + miR.376a* + miR.518c + miR.639 + miR.660 + miR.338 | 0,81 | 0,049002286 |
| miR.558 + miR.609 + miR.603 + miR.520f + miR.519b + miR.26a + miR.29a + miR.362 + miR.369.3p + miR.376a* + miR.494 + miR.518c + miR.660 | 0,81 | 0,054974724 |
| miR.558 + miR.609 + miR.220a + miR.519b + miR.130a + miR.26a + miR.29a + miR.362 + miR.369.3p + miR.376a* + miR.518c + miR.639 + miR.338 | 0,81 | 0,07702746 |
| miR.558 + miR.609 + miR.520f + miR.220a + miR.519b + miR.130a + miR.29a + miR.362 + miR.369.3p + miR.376a* + miR.494 + miR.518c + miR.639 | 0,81 | 0,124257893 |
| miR.558 + miR.609 + miR.603 + miR.520f + miR.130a + miR.29a + miR.362 + miR.369.3p + miR.376a* + miR.494 + miR.639 + miR.660 + miR.338 | 0,81 | 0,317759423 |
| miR.609 + miR.603 + miR.520f + miR.220a + miR.519b + miR.26a + miR.29a + miR.362 + miR.369.3p + miR.376a* + miR.494 + miR.518c + miR.660 | 0,81 | 0,195976087 |
| miR.558 + miR.609 + miR.603 + miR.520f + miR.130a + miR.26a + miR.29a + miR.362 + miR.376a* + miR.494 + miR.518c + miR.639 + miR.338 | 0,81 | 0,018509603 |
| miR.609 + miR.603 + miR.520f + miR.220a + miR.519b + miR.130a + miR.29a + miR.362 + miR.369.3p + miR.376a* + miR.494 + miR.518c + miR.660 | 0,81 | 0,176526046 |
| miR.558 + miR.609 + miR.520f + miR.519b + miR.130a + miR.29a + miR.362 + miR.369.3p + miR.376a* + miR.494 + miR.639 + miR.660 + miR.338 | 0,81 | 0,299979233 |
| miR.558 + miR.609 + miR.603 + miR.220a + miR.519b + miR.29a + miR.362 + miR.376a* + miR.494 + miR.518c + miR.639 + miR.660 + miR.338 | 0,81 | 0,081031334 |
| miR.558 + miR.609 + miR.603 + miR.520f + miR.220a + miR.519b + miR.130a + miR.26a + miR.29a + miR.376a* + miR.518c + miR.639 + miR.338 | 0,81 | 0,037621692 |
| miR.558 + miR.609 + miR.603 + miR.519b + miR.130a + miR.26a + miR.29a + miR.362 + miR.369.3p + miR.376a* + miR.494 + miR.518c + miR.338 | 0,81 | 0,055365894 |
| miR.558 + miR.609 + miR.220a + miR.519b + miR.130a + miR.26a + miR.29a + miR.362 + miR.369.3p + miR.376a* + miR.494 + miR.518c + miR.639 | 0,81 | 0,113968637 |
| miR.558 + miR.609 + miR.603 + miR.520f + miR.130a + miR.26a + miR.29a + miR.362 + miR.369.3p + miR.376a* + miR.494 + miR.518c + miR.660 | 0,81 | 0,03505868 |
| miR.558 + miR.609 + miR.603 + miR.519b + miR.130a + miR.26a + miR.29a + miR.369.3p + miR.376a* + miR.494 + miR.518c + miR.660 + miR.338 | 0,81 | 0,050652216 |
| miR.558 + miR.609 + miR.520f + miR.130a + miR.26a + miR.29a + miR.362 + miR.369.3p + miR.376a* + miR.494 + miR.639 + miR.660 + miR.338 | 0,81 | 0,315190456 |
| miR.558 + miR.609 + miR.520f + miR.220a + miR.519b + miR.130a + miR.29a + miR.362 + miR.376a* + miR.494 + miR.518c + miR.639 + miR.660 | 0,81 | 0,114933733 |
| miR.558 + miR.609 + miR.520f + miR.220a + miR.519b + miR.130a + miR.26a + miR.29a + miR.369.3p + miR.376a* + miR.518c + miR.639 + miR.338 | 0,81 | 0,099823206 |
| miR.558 + miR.609 + miR.520f + miR.220a + miR.519b + miR.130a + miR.26a + miR.29a + miR.376a* + miR.494 + miR.518c + miR.639 + miR.660 | 0,81 | 0,115593279 |
| miR.558 + miR.609 + miR.603 + miR.520f + miR.519b + miR.130a + miR.29a + miR.362 + miR.369.3p + miR.376a* + miR.494 + miR.518c + miR.660 | 0,81 | 0,054685768 |
| miR.558 + miR.609 + miR.603 + miR.520f + miR.26a + miR.362 + miR.369.3p + miR.376a* + miR.494 + miR.518c + miR.639 + miR.660 + miR.338 | 0,81 | 0,14649747 |
| miR.558 + miR.609 + miR.603 + miR.220a + miR.130a + miR.29a + miR.362 + miR.376a* + miR.494 + miR.518c + miR.639 + miR.660 + miR.338 | 0,81 | 0,042425785 |
| miR.558 + miR.609 + miR.603 + miR.520f + miR.519b + miR.130a + miR.26a + miR.29a + miR.376a* + miR.494 + miR.518c + miR.639 + miR.338 | 0,81 | 0,023002691 |
| miR.609 + miR.603 + miR.220a + miR.130a + miR.26a + miR.29a + miR.362 + miR.369.3p + miR.376a* + miR.494 + miR.518c + miR.660 + miR.338 | 0,81 | 0,391306838 |
| miR.558 + miR.609 + miR.520f + miR.220a + miR.130a + miR.26a + miR.29a + miR.362 + miR.376a* + miR.494 + miR.518c + miR.660 + miR.338 | 0,81 | 0,043584453 |
| miR.609 + miR.520f + miR.220a + miR.26a + miR.29a + miR.362 + miR.369.3p + miR.376a* + miR.494 + miR.518c + miR.639 + miR.660 + miR.338 | 0,81 | 0,253779002 |
| miR.558 + miR.609 + miR.520f + miR.220a + miR.130a + miR.26a + miR.29a + miR.362 + miR.376a* + miR.518c + miR.639 + miR.660 + miR.338 | 0,81 | 0,023286823 |
| miR.558 + miR.609 + miR.603 + miR.520f + miR.130a + miR.362 + miR.369.3p + miR.376a* + miR.494 + miR.518c + miR.639 + miR.660 + miR.338 | 0,81 | 0,124610367 |
| miR.609 + miR.603 + miR.220a + miR.519b + miR.130a + miR.26a + miR.29a + miR.362 + miR.369.3p + miR.376a* + miR.494 + miR.518c + miR.660 | 0,81 | 0,201195729 |
| miR.609 + miR.603 + miR.520f + miR.220a + miR.26a + miR.29a + miR.362 + miR.369.3p + miR.376a* + miR.518c + miR.639 + miR.660 + miR.338 | 0,81 | 0,347221247 |
| miR.558 + miR.609 + miR.603 + miR.520f + miR.220a + miR.29a + miR.362 + miR.369.3p + miR.376a* + miR.494 + miR.639 + miR.660 + miR.338 | 0,81 | 0,281132438 |
| miR.558 + miR.609 + miR.520f + miR.220a + miR.519b + miR.130a + miR.26a + miR.29a + miR.369.3p + miR.376a* + miR.494 + miR.518c + miR.639 | 0,81 | 0,083637503 |
| miR.609 + miR.520f + miR.220a + miR.130a + miR.29a + miR.362 + miR.369.3p + miR.376a* + miR.494 + miR.518c + miR.639 + miR.660 + miR.338 | 0,81 | 0,291545586 |
| miR.558 + miR.609 + miR.520f + miR.519b + miR.130a + miR.26a + miR.29a + miR.362 + miR.376a* + miR.494 + miR.518c + miR.639 + miR.338 | 0,81 | 0,073905315 |
| miR.558 + miR.609 + miR.520f + miR.220a + miR.519b + miR.130a + miR.29a + miR.362 + miR.369.3p + miR.376a* + miR.518c + miR.639 + miR.338 | 0,81 | 0,087195074 |
| miR.558 + miR.609 + miR.603 + miR.520f + miR.519b + miR.26a + miR.29a + miR.362 + miR.369.3p + miR.376a* + miR.494 + miR.518c + miR.639 | 0,81 | 0,043064954 |
| miR.609 + miR.603 + miR.520f + miR.519b + miR.130a + miR.29a + miR.362 + miR.369.3p + miR.376a* + miR.518c + miR.639 + miR.660 + miR.338 | 0,81 | 0,478390968 |
| miR.558 + miR.609 + miR.603 + miR.520f + miR.519b + miR.130a + miR.26a + miR.29a + miR.369.3p + miR.376a* + miR.494 + miR.518c + miR.660 | 0,81 | 0,061718871 |
| miR.558 + miR.609 + miR.520f + miR.220a + miR.519b + miR.130a + miR.26a + miR.29a + miR.362 + miR.376a* + miR.494 + miR.518c + miR.639 | 0,81 | 0,107551914 |
| miR.558 + miR.609 + miR.603 + miR.520f + miR.519b + miR.130a + miR.29a + miR.362 + miR.376a* + miR.494 + miR.518c + miR.660 + miR.338 | 0,81 | 0,071885439 |
| miR.558 + miR.609 + miR.603 + miR.520f + miR.26a + miR.29a + miR.362 + miR.369.3p + miR.376a* + miR.494 + miR.639 + miR.660 + miR.338 | 0,81 | 0,367884507 |
| miR.558 + miR.609 + miR.520f + miR.220a + miR.26a + miR.29a + miR.362 + miR.369.3p + miR.376a* + miR.494 + miR.639 + miR.660 + miR.338 | 0,81 | 0,295403231 |
| miR.558 + miR.609 + miR.520f + miR.220a + miR.519b + miR.130a + miR.362 + miR.369.3p + miR.376a* + miR.518c + miR.639 + miR.660 + miR.338 | 0,81 | 0,129392208 |
| miR.558 + miR.609 + miR.603 + miR.220a + miR.519b + miR.130a + miR.29a + miR.362 + miR.376a* + miR.518c + miR.639 + miR.660 + miR.338 | 0,81 | 0,079518032 |
| miR.558 + miR.609 + miR.603 + miR.520f + miR.519b + miR.29a + miR.362 + miR.369.3p + miR.376a* + miR.494 + miR.639 + miR.660 + miR.338 | 0,81 | 0,328184604 |
| miR.558 + miR.609 + miR.603 + miR.520f + miR.519b + miR.26a + miR.29a + miR.362 + miR.376a* + miR.494 + miR.518c + miR.639 + miR.338 | 0,81 | 0,048286108 |
| miR.558 + miR.609 + miR.603 + miR.520f + miR.519b + miR.130a + miR.26a + miR.29a + miR.362 + miR.376a* + miR.518c + miR.639 + miR.338 | 0,81 | 0,047126318 |
| miR.609 + miR.603 + miR.220a + miR.519b + miR.130a + miR.29a + miR.362 + miR.369.3p + miR.376a* + miR.494 + miR.518c + miR.639 + miR.660 | 0,81 | 0,197656575 |
| miR.558 + miR.609 + miR.520f + miR.220a + miR.519b + miR.130a + miR.26a + miR.29a + miR.362 + miR.376a* + miR.494 + miR.518c + miR.660 | 0,81 | 0,203443026 |
| miR.609 + miR.603 + miR.220a + miR.519b + miR.26a + miR.29a + miR.369.3p + miR.376a* + miR.494 + miR.518c + miR.639 + miR.660 + miR.338 | 0,81 | 0,113225883 |
| miR.558 + miR.609 + miR.520f + miR.220a + miR.519b + miR.26a + miR.29a + miR.362 + miR.376a* + miR.494 + miR.518c + miR.639 + miR.660 | 0,81 | 0,115644894 |
| miR.558 + miR.609 + miR.603 + miR.519b + miR.130a + miR.26a + miR.29a + miR.362 + miR.369.3p + miR.376a* + miR.494 + miR.518c + miR.660 | 0,81 | 0,092194271 |
| miR.609 + miR.603 + miR.220a + miR.519b + miR.130a + miR.29a + miR.362 + miR.369.3p + miR.376a* + miR.494 + miR.518c + miR.660 + miR.338 | 0,81 | 0,358832089 |
| miR.558 + miR.609 + miR.603 + miR.520f + miR.519b + miR.130a + miR.26a + miR.29a + miR.362 + miR.376a* + miR.494 + miR.518c + miR.338 | 0,81 | 0,033923581 |
| miR.558 + miR.609 + miR.520f + miR.220a + miR.130a + miR.26a + miR.29a + miR.362 + miR.376a* + miR.494 + miR.518c + miR.639 + miR.338 | 0,81 | 0,036992373 |
| miR.558 + miR.609 + miR.520f + miR.220a + miR.519b + miR.29a + miR.362 + miR.369.3p + miR.376a* + miR.494 + miR.639 + miR.660 + miR.338 | 0,81 | 0,284745688 |
| miR.558 + miR.609 + miR.603 + miR.520f + miR.519b + miR.26a + miR.362 + miR.369.3p + miR.376a* + miR.518c + miR.639 + miR.660 + miR.338 | 0,81 | 0,124693966 |
| miR.558 + miR.609 + miR.520f + miR.220a + miR.130a + miR.26a + miR.29a + miR.362 + miR.376a* + miR.494 + miR.518c + miR.639 + miR.660 | 0,81 | 0,044327285 |
| miR.609 + miR.603 + miR.520f + miR.220a + miR.519b + miR.130a + miR.29a + miR.369.3p + miR.376a* + miR.494 + miR.518c + miR.639 + miR.660 | 0,81 | 0,109366469 |
| miR.558 + miR.609 + miR.520f + miR.519b + miR.130a + miR.26a + miR.29a + miR.362 + miR.369.3p + miR.376a* + miR.494 + miR.518c + miR.660 | 0,81 | 0,096025553 |
| miR.558 + miR.609 + miR.520f + miR.220a + miR.519b + miR.26a + miR.29a + miR.362 + miR.376a* + miR.518c + miR.639 + miR.660 + miR.338 | 0,81 | 0,039254335 |
| miR.558 + miR.609 + miR.603 + miR.520f + miR.519b + miR.362 + miR.369.3p + miR.376a* + miR.494 + miR.518c + miR.639 + miR.660 + miR.338 | 0,81 | 0,135571984 |
| miR.558 + miR.609 + miR.603 + miR.520f + miR.519b + miR.26a + miR.29a + miR.362 + miR.376a* + miR.494 + miR.518c + miR.660 + miR.338 | 0,81 | 0,054449119 |
| miR.609 + miR.603 + miR.520f + miR.220a + miR.519b + miR.26a + miR.29a + miR.369.3p + miR.376a* + miR.494 + miR.518c + miR.639 + miR.660 | 0,81 | 0,10526367 |
| miR.558 + miR.609 + miR.520f + miR.220a + miR.519b + miR.130a + miR.26a + miR.29a + miR.362 + miR.369.3p + miR.376a* + miR.518c + miR.639 | 0,81 | 0,110763733 |
| miR.558 + miR.609 + miR.603 + miR.520f + miR.519b + miR.130a + miR.26a + miR.29a + miR.376a* + miR.494 + miR.518c + miR.660 + miR.338 | 0,81 | 0,051269285 |
| miR.558 + miR.609 + miR.520f + miR.220a + miR.519b + miR.130a + miR.29a + miR.362 + miR.376a* + miR.518c + miR.639 + miR.660 + miR.338 | 0,81 | 0,04037021 |

**Table 14: clusters of 14 miRNAs**

| **Cluster of 14 miRs** | **c_tau level** | **pv compared to top signature miR.558 + miR.609 + miR.603 + miR.520f + miR.130a + miR.29a + miR.362 + miR.369.3p + miR.376a* + miR.494 + miR.518c + miR.639 + miR.660 + miR.338** |
|---|---|---|
| miR.558 + miR.609 + miR.603 + miR.520f + miR.130a + miR.29a + miR.362 + miR.369.3p + miR.376a* + miR.494 + miR.518c + miR.639 + miR.660 + miR.338 | 0,82 | NaN |
| miR.558 + miR.609 + miR.603 + miR.130a + miR.26a + miR.29a + miR.362 + miR.369.3p + miR.376a* + miR.494 + miR.518c + miR.639 + miR.660 + miR.338 | 0,82 | 0,301591691 |
| miR.558 + miR.609 + miR.520f + miR.130a + miR.26a + miR.29a + miR.362 + miR.369.3p + miR.376a* + miR.494 + miR.518c + miR.639 + miR.660 + miR.338 | 0,82 | 0,165411135 |
| miR.558 + miR.609 + miR.520f + miR.519b + miR.130a + miR.29a + miR.362 + miR.369.3p + miR.376a* + miR.494 + miR.518c + miR.639 + miR.660 + miR.338 | 0,82 | 0,025209783 |
| miR.558 + miR.609 + miR.520f + miR.220a + miR.130a + miR.29a + miR.362 + miR.369.3p + miR.376a* + miR.494 + miR.518c + miR.639 + miR.660 + miR.338 | 0,82 | 0,003221038 |
| miR.558 + miR.609 + miR.603 + miR.520f + miR.220a + miR.26a + miR.29a + miR.362 + miR.369.3p + miR.376a* + miR.518c + miR.639 + miR.660 + miR.338 | 0,82 | 0,389830678 |
| miR.558 + miR.609 + miR.603 + miR.520f + miR.220a + miR.29a + miR.362 + miR.369.3p + miR.376a* + miR.494 + miR.518c + miR.639 + miR.660 + miR.338 | 0,82 | 0,496123065 |
| miR.558 + miR.609 + miR.603 + miR.220a + miR.26a + miR.29a + miR.362 + miR.369.3p + miR.376a* + miR.494 + miR.518c + miR.639 + miR.660 + miR.338 | 0,81 | 0,444774953 |
| miR.558 + miR.609 + miR.520f + miR.220a + miR.26a + miR.29a + miR.362 + miR.369.3p + miR.376a* + miR.494 + miR.518c + miR.639 + miR.660 + miR.338 | 0,81 | 0,104413155 |
| miR.558 + miR.609 + miR.603 + miR.520f + miR.220a + miR.519b + miR.29a + miR.362 + miR.369.3p + miR.376a* + miR.518c + miR.639 + miR.660 + miR.338 | 0,81 | 0,435667582 |
| miR.558 + miR.609 + miR.603 + miR.520f + miR.220a + miR.130a + miR.29a + miR.362 + miR.369.3p + miR.376a* + miR.518c + miR.639 + miR.660 + miR.338 | 0,81 | 0,48987011 |
| miR.558 + miR.609 + miR.520f + miR.519b + miR.26a + miR.29a + miR.362 + miR.369.3p + miR.376a* + miR.494 + miR.518c + miR.639 + miR.660 + miR.338 | 0,81 | 0,265407378 |
| miR.558 + miR.609 + miR.520f + miR.519b + miR.130a + miR.26a + miR.29a + miR.362 + miR.369.3p + miR.376a* + miR.518c + miR.639 + miR.660 + miR.338 | 0,81 | 0,207501296 |
| miR.558 + miR.609 + miR.603 + miR.520f + miR.26a + miR.29a + miR.362 + miR.369.3p + miR.376a* + miR.494 + miR.518c + miR.639 + miR.660 + miR.338 | 0,81 | 0,418811391 |
| miR.558 + miR.609 + miR.520f + miR.220a + miR.519b + miR.29a + miR.362 + miR.369.3p + miR.376a* + miR.494 + miR.518c + miR.639 + miR.660 + miR.338 | 0,81 | 0,073133149 |
| miR.558 + miR.609 + miR.520f + miR.220a + miR.519b + miR.26a + miR.29a + miR.362 + miR.369.3p + miR.376a* + miR.518c + miR.639 + miR.660 + miR.338 | 0,81 | 0,128223285 |
| miR.558 + miR.609 + miR.603 + miR.520f + miR.519b + miR.29a + miR.362 + miR.369.3p + miR.376a* + miR.494 + miR.518c + miR.639 + miR.660 + miR.338 | 0,81 | 0,495657306 |
| miR.558 + miR.609 + miR.603 + miR.220a + miR.130a + miR.26a + miR.29a + miR.362 + miR.369.3p + miR.376a* + miR.518c + miR.639 + miR.660 + miR.338 | 0,81 | 0,429185148 |
| miR.558 + miR.609 + miR.603 + miR.520f + miR.519b + miR.26a + miR.29a + miR.362 + miR.369.3p + miR.376a* + miR.518c + miR.639 + miR.660 + miR.338 | 0,81 | 0,304888846 |
| miR.558 + miR.609 + miR.603 + miR.220a + miR.519b + miR.26a + miR.29a + miR.362 + miR.369.3p + miR.376a* + miR.518c + miR.639 + miR.660 + miR.338 | 0,81 | 0,426980358 |
| miR.558 + miR.609 + miR.603 + miR.220a + miR.130a + miR.29a + miR.362 + miR.369.3p + miR.376a* + miR.494 + miR.518c + miR.639 + miR.660 + miR.338 | 0,81 | 0,489476287 |
| miR.558 + miR.609 + miR.603 + miR.520f + miR.220a + miR.519b + miR.29a + miR.369.3p + miR.376a* + miR.494 + miR.518c + miR.639 + miR.660 + miR.338 | 0,81 | 0,221998549 |
| miR.558 + miR.609 + miR.603 + miR.520f + miR.130a + miR.26a + miR.29a + miR.362 + miR.369.3p + miR.376a* + miR.518c + miR.639 + miR.660 + miR.338 | 0,81 | 0,398743697 |
| miR.558 + miR.609 + miR.603 + miR.520f + miR.220a + miR.519b + miR.130a + miR.29a + miR.369.3p + miR.376a* + miR.494 + miR.518c + miR.660 + miR.338 | 0,81 | 0,168300629 |
| miR.558 + miR.609 + miR.520f + miR.220a + miR.130a + miR.26a + miR.29a + miR.362 + miR.369.3p + miR.376a* + miR.518c + miR.639 + miR.660 + miR.338 | 0,81 | 0,229918161 |
| miR.558 + miR.609 + miR.603 + miR.520f + miR.220a + miR.519b + miR.26a + miR.29a + miR.369.3p + miR.376a* + miR.494 + miR.518c + miR.660 + miR.338 | 0,81 | 0,199585294 |
| miR.558 + miR.609 + miR.520f + miR.220a + miR.519b + miR.130a + miR.29a + miR.362 + miR.369.3p + miR.376a* + miR.518c + miR.639 + miR.660 + miR.338 | 0,81 | 0,11973122 |
| miR.558 + miR.609 + miR.603 + miR.220a + miR.519b + miR.26a + miR.29a + miR.362 + miR.369.3p + miR.376a* + miR.494 + miR.518c + miR.660 + miR.338 | 0,81 | 0,3394704 |
| miR.558 + miR.609 + miR.603 + miR.520f + miR.220a + miR.130a + miR.29a + miR.369.3p + miR.376a* + miR.494 + miR.518c + miR.639 + miR.660 + miR.338 | 0,81 | 0,071730683 |
| miR.558 + miR.609 + miR.603 + miR.520f + miR.220a + miR.519b + miR.29a + miR.362 + miR.369.3p + miR.376a* + miR.494 + miR.518c + miR.660 + miR.338 | 0,81 | 0,48233317 |
| miR.558 + miR.609 + miR.220a + miR.130a + miR.26a + miR.29a + miR.362 + miR.369.3p + miR.376a* + miR.494 + miR.518c + miR.639 + miR.660 + miR.338 | 0,81 | 0,430921212 |
| miR.558 + miR.609 + miR.603 + miR.520f + miR.519b + miR.130a + miR.29a + miR.362 + miR.369.3p + miR.376a* + miR.518c + miR.639 + miR.660 + miR.338 | 0,81 | 0,489277602 |
| miR.558 + miR.609 + miR.519b + miR.130a + miR.26a + miR.29a + miR.362 + miR.369.3p + miR.376a* + miR.494 + miR.518c + miR.639 + miR.660 + miR.338 | 0,81 | 0,292052047 |
| miR.558 + miR.609 + miR.603 + miR.520f + miR.220a + miR.26a + miR.29a + miR.362 + miR.369.3p + miR.376a* + miR.494 + miR.518c + miR.660 + miR.338 | 0,81 | 0,419601156 |
| miR.558 + miR.609 + miR.603 + miR.220a + miR.519b + miR.130a + miR.26a + miR.29a + miR.369.3p + miR.376a* + miR.494 + miR.518c + miR.660 + miR.338 | 0,81 | 0,211626212 |
| miR.558 + miR.609 + miR.603 + miR.220a + miR.130a + miR.26a + miR.29a + miR.369.3p + miR.376a* + miR.494 + miR.518c + miR.639 + miR.660 + miR.338 | 0,81 | 0,152572763 |
| miR.558 + miR.609 + miR.603 + miR.520f + miR.220a + miR.26a + miR.29a + miR.369.3p + miR.376a* + miR.494 + miR.518c + miR.639 + miR.660 + miR.338 | 0,81 | 0,112197842 |
| miR.558 + miR.609 + miR.603 + miR.220a + miR.519b + miR.130a + miR.29a + miR.362 + miR.369.3p + miR.376a* + miR.494 + miR.518c + miR.660 + miR.338 | 0,81 | 0,24638012 |
| miR.558 + miR.609 + miR.603 + miR.520f + miR.220a + miR.130a + miR.26a + miR.29a + miR.369.3p + miR.376a* + miR.518c + miR.639 + miR.660 + miR.338 | 0,81 | 0,155399238 |
| miR.558 + miR.609 + miR.603 + miR.220a + miR.130a + miR.26a + miR.29a + miR.362 + miR.369.3p + miR.376a* + miR.494 + miR.518c + miR.660 + miR.338 | 0,81 | 0,346202758 |
| miR.558 + miR.609 + miR.603 + miR.520f + miR.220a + miR.519b + miR.26a + miR.29a + miR.362 + miR.369.3p + miR.376a* + miR.518c + miR.660 + miR.338 | 0,81 | 0,413635986 |
| miR.558 + miR.609 + miR.603 + miR.220a + miR.519b + miR.26a + miR.29a + miR.362 + miR.369.3p + miR.376a* + miR.494 + miR.518c + miR.639 + miR.660 | 0,81 | 0,310443044 |
| miR.558 + miR.609 + miR.603 + miR.220a + miR.130a + miR.26a + miR.29a + miR.362 + miR.369.3p + miR.376a* + miR.494 + miR.518c + miR.639 + miR.660 | 0,81 | 0,277978248 |
| miR.558 + miR.609 + miR.603 + miR.520f + miR.220a + miR.26a + miR.29a + miR.362 + miR.369.3p + miR.376a* + miR.494 + miR.518c + miR.639 + miR.660 | 0,81 | 0,272404322 |
| miR.558 + miR.609 + miR.603 + miR.520f + miR.220a + miR.519b + miR.130a + miR.29a + miR.362 + miR.369.3p + miR.376a* + miR.518c + miR.660 + miR.338 | 0,81 | 0,444636774 |
| miR.558 + miR.609 + miR.603 + miR.520f + miR.220a + miR.130a + miR.26a + miR.29a + miR.362 + miR.369.3p + miR.376a* + miR.518c + miR.660 + miR.338 | 0,81 | 0,449201083 |
| miR.558 + miR.609 + miR.603 + miR.220a + miR.519b + miR.26a + miR.29a + miR.369.3p + miR.376a* + miR.494 + miR.518c + miR.639 + miR.660 + miR.338 | 0,81 | 0,1163453 |
| miR.558 + miR.609 + miR.603 + miR.220a + miR.519b + miR.130a + miR.29a + miR.369.3p + miR.376a* + miR.494 + miR.518c + miR.639 + miR.660 + miR.338 | 0,81 | 0,130939588 |
| miR.558 + miR.609 + miR.603 + miR.520f + miR.220a + miR.130a + miR.29a + miR.362 + miR.369.3p + miR.376a* + miR.494 + miR.518c + miR.660 + miR.338 | 0,81 | 0,425067482 |
| miR.558 + miR.609 + miR.603 + miR.520f + miR.220a + miR.519b + miR.26a + miR.29a + miR.362 + miR.369.3p + miR.376a* + miR.494 + miR.518c + miR.660 | 0,81 | 0,322956275 |
| miR.558 + miR.609 + miR.603 + miR.520f + miR.220a + miR.519b + miR.26a + miR.29a + miR.369.3p + miR.376a* + miR.518c + miR.639 + miR.660 + miR.338 | 0,81 | 0,200030524 |
| miR.558 + miR.609 + miR.603 + miR.520f + miR.220a + miR.130a + miR.26a + miR.29a + miR.369.3p + miR.376a* + miR.494 + miR.518c + miR.660 + miR.338 | 0,81 | 0,119283338 |
| miR.558 + miR.609 + miR.603 + miR.220a + miR.519b + miR.130a + miR.26a + miR.29a + miR.362 + miR.369.3p + miR.376a* + miR.494 + miR.518c + miR.660 | 0,81 | 0,321776587 |
| miR.558 + miR.609 + miR.603 + miR.519b + miR.26a + miR.29a + miR.362 + miR.369.3p + miR.376a* + miR.494 + miR.518c + miR.639 + miR.660 + miR.338 | 0,81 | 0,263868072 |
| miR.558 + miR.609 + miR.603 + miR.220a + miR.519b + miR.130a + miR.26a + miR.29a + miR.369.3p + miR.376a* + miR.494 + miR.518c + miR.639 + miR.660 | 0,81 | 0,240312996 |
| miR.558 + miR.609 + miR.603 + miR.519b + miR.130a + miR.29a + miR.362 + miR.369.3p + miR.376a* + miR.494 + miR.518c + miR.639 + miR.660 + miR.338 | 0,81 | 0,221884353 |
| miR.558 + miR.609 + miR.520f + miR.220a + miR.519b + miR.26a + miR.29a + miR.362 + miR.369.3p + miR.376a* + miR.494 + miR.518c + miR.660 + miR.338 | 0,81 | 0,194986273 |
| miR.558 + miR.609 + miR.603 + miR.220a + miR.519b + miR.130a + miR.26a + miR.29a + miR.362 + miR.369.3p + miR.376a* + miR.518c + miR.660 + miR.338 | 0,81 | 0,341674651 |
| miR.558 + miR.609 + miR.603 + miR.520f + miR.220a + miR.519b + miR.26a + miR.29a + miR.369.3p + miR.376a* + miR.494 + miR.518c + miR.639 + miR.660 | 0,81 | 0,237751 |
| miR.558 + miR.609 + miR.520f + miR.220a + miR.130a + miR.26a + miR.29a + miR.362 + miR.369.3p + miR.376a* + miR.494 + miR.518c + miR.660 + miR.338 | 0,81 | 0,314605021 |
| miR.558 + miR.609 + miR.603 + miR.520f + miR.220a + miR.519b + miR.130a + miR.26a + miR.29a + miR.369.3p + miR.376a* + miR.494 + miR.518c + miR.660 | 0,81 | 0,261981571 |
| miR.558 + miR.609 + miR.603 + miR.520f + miR.220a + miR.130a + miR.26a + miR.29a + miR.369.3p + miR.376a* + miR.494 + miR.518c + miR.639 + miR.660 | 0,81 | 0,179522142 |
| miR.558 + miR.609 + miR.603 + miR.520f + miR.220a + miR.519b + miR.130a + miR.29a + miR.369.3p + miR.376a* + miR.518c + miR.639 + miR.660 + miR.338 | 0,81 | 0,234449206 |
| miR.558 + miR.609 + miR.520f + miR.220a + miR.519b + miR.130a + miR.29a + miR.362 + miR.369.3p + miR.376a* + miR.494 + miR.518c + miR.660 + miR.338 | 0,81 | 0,181803011 |
| miR.558 + miR.609 + miR.603 + miR.520f + miR.220a + miR.519b + miR.130a + miR.26a + miR.29a + miR.369.3p + miR.376a* + miR.518c + miR.660 + miR.338 | 0,81 | 0,237494151 |
| miR.558 + miR.609 + miR.603 + miR.520f + miR.220a + miR.519b + miR.26a + miR.29a + miR.362 + miR.369.3p + miR.376a* + miR.518c + miR.639 + miR.660 | 0,81 | 0,279903675 |
| miR.558 + miR.609 + miR.603 + miR.220a + miR.519b + miR.130a + miR.29a + miR.362 + miR.369.3p + miR.376a* + miR.518c + miR.639 + miR.660 + miR.338 | 0,81 | 0,399037769 |
| miR.558 + miR.609 + miR.603 + miR.520f + miR.220a + miR.519b + miR.130a + miR.29a + miR.362 + miR.369.3p + miR.376a* + miR.494 + miR.518c + miR.338 | 0,81 | 0,100024204 |
| miR.558 + miR.609 + miR.603 + miR.520f + miR.220a + miR.519b + miR.29a + miR.362 + miR.369.3p + miR.376a* + miR.494 + miR.518c + miR.639 + miR.660 | 0,81 | 0,256471319 |
| miR.558 + miR.609 + miR.603 + miR.520f + miR.220a + miR.519b + miR.26a + miR.29a + miR.362 + miR.369.3p + miR.376a* + miR.494 + miR.518c + miR.338 | 0,81 | 0,113170435 |
| miR.558 + miR.609 + miR.603 + miR.220a + miR.519b + miR.130a + miR.26a + miR.29a + miR.362 + miR.369.3p + miR.376a* + miR.494 + miR.518c + miR.338 | 0,81 | 0,111280177 |
| miR.558 + miR.609 + miR.603 + miR.520f + miR.220a + miR.519b + miR.130a + miR.29a + miR.369.3p + miR.376a* + miR.494 + miR.518c + miR.639 + miR.660 | 0,81 | 0,218350361 |
| miR.558 + miR.609 + miR.220a + miR.519b + miR.26a + miR.29a + miR.362 + miR.369.3p + miR.376a* + miR.494 + miR.518c + miR.639 + miR.660 + miR.338 | 0,81 | 0,448264063 |
| miR.558 + miR.609 + miR.603 + miR.220a + miR.519b + miR.130a + miR.26a + miR.29a + miR.369.3p + miR.376a* + miR.494 + miR.518c + miR.639 + miR.338 | 0,81 | 0,102185025 |
| miR.558 + miR.609 + miR.603 + miR.520f + miR.220a + miR.519b + miR.130a + miR.26a + miR.29a + miR.369.3p + miR.376a* + miR.494 + miR.518c + miR.338 | 0,81 | 0,105466734 |
| miR.558 + miR.609 + miR.603 + miR.220a + miR.519b + miR.130a + miR.29a + miR.362 + miR.369.3p + miR.376a* + miR.494 + miR.518c + miR.639 + miR.660 | 0,81 | 0,207553067 |
| miR.558 + miR.609 + miR.603 + miR.520f + miR.220a + miR.130a + miR.29a + miR.362 + miR.369.3p + miR.376a* + miR.494 + miR.518c + miR.639 + miR.660 | 0,81 | 0,16636663 |
| miR.558 + miR.609 + miR.603 + miR.519b + miR.130a + miR.26a + miR.29a + miR.362 + miR.369.3p + miR.376a* + miR.518c + miR.639 + miR.660 + miR.338 | 0,81 | 0,444626522 |
| miR.558 + miR.609 + miR.603 + miR.220a + miR.519b + miR.29a + miR.362 + miR.369.3p + miR.376a* + miR.494 + miR.518c + miR.639 + miR.660 + miR.338 | 0,81 | 0,273438607 |
| miR.558 + miR.609 + miR.603 + miR.520f + miR.220a + miR.519b + miR.130a + miR.29a + miR.362 + miR.369.3p + miR.376a* + miR.494 + miR.518c + miR.660 | 0,81 | 0,249720873 |
| miR.558 + miR.609 + miR.603 + miR.220a + miR.519b + miR.130a + miR.26a + miR.29a + miR.362 + miR.369.3p + miR.376a* + miR.518c + miR.639 + miR.660 | 0,81 | 0,350206395 |
| miR.558 + miR.609 + miR.603 + miR.520f + miR.220a + miR.519b + miR.26a + miR.29a + miR.369.3p + miR.376a* + miR.494 + miR.518c + miR.639 + miR.338 | 0,81 | 0,096118835 |
| miR.558 + miR.609 + miR.603 + miR.520f + miR.220a + miR.130a + miR.26a + miR.29a + miR.362 + miR.369.3p + miR.376a* + miR.494 + miR.518c + miR.660 | 0,81 | 0,261276008 |
| miR.558 + miR.609 + miR.220a + miR.519b + miR.130a + miR.29a + miR.362 + miR.369.3p + miR.376a* + miR.494 + miR.518c + miR.639 + miR.660 + miR.338 | 0,81 | 0,365517569 |
| miR.558 + miR.609 + miR.603 + miR.520f + miR.220a + miR.130a + miR.26a + miR.29a + miR.362 + miR.369.3p + miR.376a* + miR.518c + miR.639 + miR.660 | 0,81 | 0,326217437 |
| miR.558 + miR.609 + miR.603 + miR.520f + miR.519b + miR.130a + miR.29a + miR.369.3p + miR.376a* + miR.494 + miR.518c + miR.639 + miR.660 + miR.338 | 0,81 | 0,137393885 |
| miR.558 + miR.609 + miR.603 + miR.520f + miR.519b + miR.26a + miR.29a + miR.369.3p + miR.376a* + miR.494 + miR.518c + miR.639 + miR.660 + miR.338 | 0,81 | 0,133512893 |
| miR.558 + miR.609 + miR.603 + miR.220a + miR.519b + miR.26a + miR.29a + miR.362 + miR.369.3p + miR.376a* + miR.494 + miR.518c + miR.639 + miR.338 | 0,81 | 0,1107797 |
| miR.558 + miR.609 + miR.220a + miR.519b + miR.130a + miR.26a + miR.29a + miR.362 + miR.369.3p + miR.376a* + miR.518c + miR.639 + miR.660 + miR.338 | 0,81 | 0,252485157 |
| miR.558 + miR.609 + miR.603 + miR.520f + miR.220a + miR.519b + miR.29a + miR.362 + miR.369.3p + miR.376a* + miR.494 + miR.518c + miR.639 + miR.338 | 0,81 | 0,113182473 |
| miR.558 + miR.609 + miR.603 + miR.520f + miR.220a + miR.26a + miR.29a + miR.362 + miR.369.3p + miR.376a* + miR.494 + miR.518c + miR.639 + miR.338 | 0,81 | 0,048805842 |
| miR.558 + miR.609 + miR.603 + miR.220a + miR.519b + miR.130a + miR.26a + miR.29a + miR.362 + miR.369.3p + miR.376a* + miR.494 + miR.518c + miR.639 | 0,81 | 0,16622317 |
| miR.558 + miR.609 + miR.603 + miR.520f + miR.220a + miR.519b + miR.130a + miR.29a + miR.369.3p + miR.376a* + miR.494 + miR.518c + miR.639 + miR.338 | 0,81 | 0,090110182 |
| miR.558 + miR.609 + miR.603 + miR.520f + miR.130a + miR.26a + miR.29a + miR.369.3p + miR.376a* + miR.494 + miR.518c + miR.639 + miR.660 + miR.338 | 0,81 | 0,182449951 |
| miR.558 + miR.609 + miR.520f + miR.220a + miR.519b + miR.130a + miR.26a + miR.29a + miR.362 + miR.369.3p + miR.376a* + miR.518c + miR.660 + miR.338 | 0,81 | 0,339667117 |
| miR.558 + miR.609 + miR.603 + miR.220a + miR.519b + miR.130a + miR.26a + miR.29a + miR.369.3p + miR.376a* + miR.518c + miR.639 + miR.660 + miR.338 | 0,81 | 0,213405578 |
| miR.558 + miR.609 + miR.603 + miR.520f + miR.220a + miR.519b + miR.130a + miR.26a + miR.29a + miR.362 + miR.369.3p + miR.376a* + miR.494 + miR.518c | 0,81 | 0,158862961 |
| miR.558 + miR.609 + miR.603 + miR.220a + miR.130a + miR.26a + miR.29a + miR.362 + miR.369.3p + miR.376a* + miR.494 + miR.518c + miR.639 + miR.338 | 0,81 | 0,03808596 |
| miR.558 + miR.609 + miR.603 + miR.520f + miR.220a + miR.519b + miR.130a + miR.26a + miR.29a + miR.362 + miR.369.3p + miR.376a* + miR.518c + miR.660 | 0,81 | 0,29861872 |
| miR.558 + miR.609 + miR.520f + miR.220a + miR.519b + miR.26a + miR.29a + miR.369.3p + miR.376a* + miR.494 + miR.518c + miR.639 + miR.660 + miR.338 | 0,81 | 0,373918099 |
| miR.558 + miR.609 + miR.603 + miR.520f + miR.220a + miR.130a + miR.26a + miR.29a + miR.369.3p + miR.376a* + miR.494 + miR.518c + miR.639 + miR.338 | 0,81 | 0,036356881 |
| miR.558 + miR.609 + miR.520f + miR.220a + miR.519b + miR.130a + miR.29a + miR.369.3p + miR.376a* + miR.494 + miR.518c + miR.639 + miR.660 + miR.338 | 0,81 | 0,299950827 |
| miR.558 + miR.609 + miR.520f + miR.519b + miR.130a + miR.26a + miR.29a + miR.362 + miR.369.3p + miR.376a* + miR.494 + miR.518c + miR.660 + miR.338 | 0,81 | 0,439230664 |
| miR.558 + miR.609 + miR.603 + miR.520f + miR.220a + miR.130a + miR.26a + miR.29a + miR.362 + miR.369.3p + miR.376a* + miR.518c + miR.639 + miR.338 | 0,81 | 0,046614267 |
| miR.558 + miR.609 + miR.603 + miR.520f + miR.519b + miR.130a + miR.29a + miR.362 + miR.369.3p + miR.376a* + miR.494 + miR.518c + miR.660 + miR.338 | 0,81 | 0,290005937 |
| miR.558 + miR.609 + miR.603 + miR.520f + miR.519b + miR.26a + miR.29a + miR.362 + miR.369.3p + miR.376a* + miR.494 + miR.518c + miR.660 + miR.338 | 0,81 | 0,327636819 |
| miR.558 + miR.609 + miR.603 + miR.520f + miR.220a + miR.519b + miR.26a + miR.29a + miR.362 + miR.369.3p + miR.376a* + miR.494 + miR.518c + miR.639 | 0,81 | 0,130968282 |
| miR.558 + miR.609 + miR.603 + miR.520f + miR.130a + miR.26a + miR.29a + miR.362 + miR.369.3p + miR.376a* + miR.494 + miR.518c + miR.660 + miR.338 | 0,81 | 0,299221673 |
| miR.558 + miR.609 + miR.603 + miR.520f + miR.220a + miR.519b + miR.130a + miR.29a + miR.362 + miR.369.3p + miR.376a* + miR.494 + miR.518c + miR.639 | 0,81 | 0,127821482 |
| miR.558 + miR.609 + miR.603 + miR.220a + miR.519b + miR.130a + miR.26a + miR.29a + miR.362 + miR.376a* + miR.494 + miR.518c + miR.639 + miR.338 | 0,81 | 0,101359364 |
| miR.558 + miR.609 + miR.603 + miR.520f + miR.220a + miR.519b + miR.130a + miR.26a + miR.29a + miR.369.3p + miR.376a* + miR.494 + miR.518c + miR.639 | 0,81 | 0,149791793 |
| miR.558 + miR.609 + miR.603 + miR.220a + miR.519b + miR.130a + miR.29a + miR.362 + miR.369.3p + miR.376a* + miR.494 + miR.518c + miR.639 + miR.338 | 0,81 | 0,09738086 |
| miR.558 + miR.609 + miR.603 + miR.520f + miR.220a + miR.130a + miR.26a + miR.29a + miR.362 + miR.369.3p + miR.376a* + miR.494 + miR.518c + miR.338 | 0,81 | 0,03168927 |
| miR.558 + miR.609 + miR.520f + miR.220a + miR.130a + miR.26a + miR.29a + miR.369.3p + miR.376a* + miR.494 + miR.518c + miR.639 + miR.660 + miR.338 | 0,81 | 0,268565804 |
| miR.558 + miR.609 + miR.603 + miR.519b + miR.130a + miR.26a + miR.29a + miR.362 + miR.369.3p + miR.376a* + miR.494 + miR.518c + miR.639 + miR.660 | 0,81 | 0,092238665 |
| miR.558 + miR.609 + miR.603 + miR.520f + miR.519b + miR.130a + miR.26a + miR.29a + miR.362 + miR.369.3p + miR.376a* + miR.518c + miR.660 + miR.338 | 0,81 | 0,281097492 |
| miR.558 + miR.609 + miR.603 + miR.519b + miR.130a + miR.26a + miR.29a + miR.369.3p + miR.376a* + miR.494 + miR.518c + miR.639 + miR.660 + miR.338 | 0,81 | 0,196888705 |
| miR.558 + miR.609 + miR.603 + miR.520f + miR.220a + miR.519b + miR.130a + miR.26a + miR.29a + miR.369.3p + miR.376a* + miR.518c + miR.639 + miR.660 | 0,81 | 0,226900096 |
| miR.558 + miR.609 + miR.603 + miR.220a + miR.519b + miR.130a + miR.26a + miR.29a + miR.376a* + miR.494 + miR.518c + miR.639 + miR.660 + miR.338 | 0,81 | 0,111933784 |
| miR.558 + miR.609 + miR.603 + miR.220a + miR.519b + miR.130a + miR.26a + miR.29a + miR.362 + miR.376a* + miR.494 + miR.518c + miR.639 + miR.660 | 0,81 | 0,14868446 |
| miR.558 + miR.609 + miR.603 + miR.520f + miR.130a + miR.26a + miR.29a + miR.362 + miR.369.3p + miR.376a* + miR.494 + miR.518c + miR.639 + miR.660 | 0,81 | 0,046427614 |
| miR.558 + miR.609 + miR.603 + miR.220a + miR.519b + miR.130a + miR.26a + miR.29a + miR.362 + miR.376a* + miR.494 + miR.518c + miR.660 + miR.338 | 0,81 | 0,104198966 |
| miR.558 + miR.609 + miR.520f + miR.519b + miR.130a + miR.26a + miR.29a + miR.369.3p + miR.376a* + miR.494 + miR.518c + miR.639 + miR.660 + miR.338 | 0,81 | 0,274345721 |
| miR.558 + miR.609 + miR.603 + miR.520f + miR.519b + miR.130a + miR.29a + miR.362 + miR.369.3p + miR.376a* + miR.494 + miR.518c + miR.639 + miR.660 | 0,81 | 0,036277883 |
| miR.558 + miR.609 + miR.603 + miR.520f + miR.220a + miR.519b + miR.130a + miR.29a + miR.362 + miR.369.3p + miR.376a* + miR.518c + miR.639 + miR.660 | 0,81 | 0,263892422 |
| miR.558 + miR.609 + miR.603 + miR.520f + miR.220a + miR.130a + miR.26a + miR.29a + miR.362 + miR.369.3p + miR.376a* + miR.494 + miR.518c + miR.639 | 0,81 | 0,07511349 |
| miR.558 + miR.609 + miR.603 + miR.520f + miR.220a + miR.519b + miR.26a + miR.29a + miR.362 + miR.369.3p + miR.376a* + miR.518c + miR.639 + miR.338 | 0,81 | 0,139866116 |
| miR.558 + miR.609 + miR.520f + miR.220a + miR.130a + miR.26a + miR.29a + miR.362 + miR.369.3p + miR.376a* + miR.494 + miR.518c + miR.639 + miR.660 | 0,81 | 0,314959385 |
| miR.558 + miR.609 + miR.220a + miR.519b + miR.130a + miR.26a + miR.29a + miR.362 + miR.369.3p + miR.376a* + miR.494 + miR.518c + miR.660 + miR.338 | 0,81 | 0,489311352 |
| miR.558 + miR.609 + miR.520f + miR.220a + miR.519b + miR.130a + miR.26a + miR.29a + miR.369.3p + miR.376a* + miR.494 + miR.518c + miR.660 + miR.338 | 0,81 | 0,41094556 |
| miR.558 + miR.609 + miR.603 + miR.520f + miR.220a + miR.519b + miR.130a + miR.26a + miR.29a + miR.362 + miR.369.3p + miR.376a* + miR.518c + miR.338 | 0,81 | 0,108427593 |
| miR.558 + miR.609 + miR.603 + miR.520f + miR.519b + miR.130a + miR.26a + miR.29a + miR.369.3p + miR.376a* + miR.518c + miR.639 + miR.660 + miR.338 | 0,81 | 0,098415273 |
| miR.558 + miR.609 + miR.603 + miR.520f + miR.220a + miR.519b + miR.130a + miR.29a + miR.376a* + miR.494 + miR.518c + miR.639 + miR.660 + miR.338 | 0,81 | 0,105732259 |
| miR.558 + miR.609 + miR.520f + miR.220a + miR.519b + miR.130a + miR.26a + miR.29a + miR.369.3p + miR.376a* + miR.518c + miR.639 + miR.660 + miR.338 | 0,81 | 0,296886249 |
| miR.609 + miR.603 + miR.520f + miR.220a + miR.519b + miR.29a + miR.362 + miR.369.3p + miR.376a* + miR.494 + miR.518c + miR.639 + miR.660 + miR.338 | 0,81 | 0,319886768 |
| miR.558 + miR.609 + miR.603 + miR.520f + miR.220a + miR.519b + miR.130a + miR.26a + miR.29a + miR.369.3p + miR.376a* + miR.518c + miR.639 + miR.338 | 0,81 | 0,121671602 |
| miR.558 + miR.609 + miR.603 + miR.520f + miR.220a + miR.519b + miR.29a + miR.362 + miR.376a* + miR.494 + miR.518c + miR.639 + miR.660 + miR.338 | 0,81 | 0,136061834 |
| miR.558 + miR.609 + miR.603 + miR.520f + miR.220a + miR.519b + miR.26a + miR.29a + miR.376a* + miR.494 + miR.518c + miR.639 + miR.660 + miR.338 | 0,81 | 0,124365975 |
| miR.558 + miR.609 + miR.220a + miR.519b + miR.130a + miR.26a + miR.29a + miR.362 + miR.369.3p + miR.376a* + miR.494 + miR.518c + miR.639 + miR.660 | 0,81 | 0,333427402 |
| miR.558 + miR.609 + miR.603 + miR.520f + miR.220a + miR.519b + miR.130a + miR.29a + miR.362 + miR.376a* + miR.494 + miR.518c + miR.639 + miR.338 | 0,81 | 0,097303 |
| miR.558 + miR.609 + miR.220a + miR.519b + miR.130a + miR.26a + miR.29a + miR.369.3p + miR.376a* + miR.494 + miR.518c + miR.639 + miR.660 + miR.338 | 0,81 | 0,312395205 |
| miR.558 + miR.609 + miR.603 + miR.519b + miR.130a + miR.26a + miR.29a + miR.362 + miR.369.3p + miR.376a* + miR.494 + miR.518c + miR.660 + miR.338 | 0,81 | 0,307052334 |
| miR.558 + miR.609 + miR.603 + miR.520f + miR.220a + miR.519b + miR.130a + miR.26a + miR.29a + miR.362 + miR.369.3p + miR.376a* + miR.518c + miR.639 | 0,81 | 0,143607967 |
| miR.558 + miR.609 + miR.603 + miR.220a + miR.519b + miR.130a + miR.29a + miR.362 + miR.376a* + miR.494 + miR.518c + miR.639 + miR.660 + miR.338 | 0,81 | 0,085158628 |
| miR.558 + miR.609 + miR.603 + miR.520f + miR.220a + miR.519b + miR.130a + miR.26a + miR.29a + miR.362 + miR.376a* + miR.494 + miR.518c + miR.338 | 0,81 | 0,087989213 |
| miR.558 + miR.609 + miR.603 + miR.520f + miR.220a + miR.519b + miR.130a + miR.29a + miR.362 + miR.369.3p + miR.376a* + miR.518c + miR.639 + miR.338 | 0,81 | 0,122044136 |
| miR.558 + miR.609 + miR.603 + miR.520f + miR.220a + miR.519b + miR.130a + miR.26a + miR.29a + miR.376a* + miR.494 + miR.518c + miR.660 + miR.338 | 0,81 | 0,148631783 |
| miR.558 + miR.609 + miR.603 + miR.520f + miR.220a + miR.519b + miR.130a + miR.29a + miR.362 + miR.376a* + miR.494 + miR.518c + miR.660 + miR.338 | 0,81 | 0,144800205 |
| miR.558 + miR.609 + miR.520f + miR.220a + miR.519b + miR.130a + miR.29a + miR.362 + miR.369.3p + miR.376a* + miR.494 + miR.518c + miR.639 + miR.660 | 0,81 | 0,336454773 |
| miR.558 + miR.609 + miR.603 + miR.520f + miR.220a + miR.519b + miR.130a + miR.26a + miR.29a + miR.362 + miR.376a* + miR.494 + miR.518c + miR.639 | 0,81 | 0,128458988 |
| miR.558 + miR.609 + miR.603 + miR.520f + miR.519b + miR.130a + miR.26a + miR.29a + miR.369.3p + miR.376a* + miR.494 + miR.518c + miR.639 + miR.338 | 0,81 | 0,065330518 |
| miR.558 + miR.609 + miR.603 + miR.520f + miR.519b + miR.130a + miR.26a + miR.29a + miR.369.3p + miR.376a* + miR.494 + miR.518c + miR.660 + miR.338 | 0,81 | 0,167923448 |
| miR.558 + miR.609 + miR.603 + miR.520f + miR.220a + miR.519b + miR.26a + miR.29a + miR.362 + miR.376a* + miR.494 + miR.518c + miR.660 + miR.338 | 0,81 | 0,208264538 |
| miR.558 + miR.609 + miR.603 + miR.520f + miR.220a + miR.519b + miR.26a + miR.29a + miR.362 + miR.376a* + miR.494 + miR.518c + miR.639 + miR.338 | 0,81 | 0,105567471 |
| miR.558 + miR.609 + miR.603 + miR.520f + miR.519b + miR.130a + miR.29a + miR.362 + miR.369.3p + miR.376a* + miR.494 + miR.518c + miR.639 + miR.338 | 0,81 | 0,069525217 |
| miR.558 + miR.609 + miR.603 + miR.220a + miR.519b + miR.130a + miR.26a + miR.29a + miR.362 + miR.369.3p + miR.376a* + miR.518c + miR.639 + miR.338 | 0,81 | 0,127177552 |
| miR.558 + miR.609 + miR.603 + miR.520f + miR.130a + miR.26a + miR.29a + miR.362 + miR.369.3p + miR.376a* + miR.494 + miR.518c + miR.639 + miR.338 | 0,81 | 0,064621923 |
| miR.558 + miR.609 + miR.603 + miR.520f + miR.220a + miR.519b + miR.130a + miR.29a + miR.362 + miR.376a* + miR.494 + miR.518c + miR.639 + miR.660 | 0,81 | 0,151849469 |
| miR.558 + miR.609 + miR.520f + miR.220a + miR.519b + miR.26a + miR.29a + miR.362 + miR.369.3p + miR.376a* + miR.494 + miR.518c + miR.639 + miR.660 | 0,81 | 0,28758458 |
| miR.558 + miR.609 + miR.603 + miR.520f + miR.220a + miR.519b + miR.130a + miR.26a + miR.29a + miR.376a* + miR.494 + miR.518c + miR.639 + miR.660 | 0,81 | 0,151317681 |
| miR.558 + miR.609 + miR.603 + miR.520f + miR.220a + miR.519b + miR.130a + miR.26a + miR.29a + miR.362 + miR.376a* + miR.494 + miR.518c + miR.660 | 0,81 | 0,172147629 |
| miR.558 + miR.609 + miR.603 + miR.520f + miR.220a + miR.26a + miR.29a + miR.362 + miR.376a* + miR.494 + miR.518c + miR.639 + miR.660 + miR.338 | 0,81 | 0,026586742 |
| miR.558 + miR.609 + miR.520f + miR.519b + miR.130a + miR.26a + miR.29a + miR.362 + miR.369.3p + miR.376a* + miR.494 + miR.518c + miR.639 + miR.660 | 0,81 | 0,094490807 |
| miR.558 + miR.609 + miR.603 + miR.520f + miR.220a + miR.130a + miR.29a + miR.362 + miR.369.3p + miR.376a* + miR.494 + miR.518c + miR.639 + miR.338 | 0,81 | 0,021233368 |
| miR.558 + miR.609 + miR.603 + miR.520f + miR.220a + miR.130a + miR.29a + miR.362 + miR.376a* + miR.494 + miR.518c + miR.639 + miR.660 + miR.338 | 0,81 | 0,013871553 |
| miR.558 + miR.609 + miR.603 + miR.520f + miR.220a + miR.130a + miR.26a + miR.29a + miR.376a* + miR.494 + miR.518c + miR.639 + miR.660 + miR.338 | 0,81 | 0,020594396 |
| miR.558 + miR.609 + miR.603 + miR.520f + miR.220a + miR.519b + miR.26a + miR.29a + miR.362 + miR.376a* + miR.494 + miR.518c + miR.639 + miR.660 | 0,81 | 0,164857512 |
| miR.558 + miR.609 + miR.603 + miR.220a + miR.130a + miR.26a + miR.29a + miR.362 + miR.376a* + miR.494 + miR.518c + miR.639 + miR.660 + miR.338 | 0,81 | 0,043424385 |
| miR.558 + miR.609 + miR.603 + miR.520f + miR.519b + miR.130a + miR.26a + miR.29a + miR.369.3p + miR.376a* + miR.494 + miR.518c + miR.639 + miR.660 | 0,81 | 0,120865418 |
| miR.558 + miR.609 + miR.520f + miR.220a + miR.519b + miR.130a + miR.26a + miR.29a + miR.369.3p + miR.376a* + miR.494 + miR.518c + miR.639 + miR.660 | 0,81 | 0,281340937 |
| miR.558 + miR.609 + miR.603 + miR.519b + miR.130a + miR.26a + miR.29a + miR.362 + miR.369.3p + miR.376a* + miR.494 + miR.518c + miR.639 + miR.338 | 0,81 | 0,15119666 |
| miR.558 + miR.609 + miR.520f + miR.220a + miR.519b + miR.130a + miR.26a + miR.29a + miR.362 + miR.369.3p + miR.376a* + miR.494 + miR.518c + miR.660 | 0,81 | 0,406136722 |
| miR.609 + miR.603 + miR.520f + miR.220a + miR.26a + miR.29a + miR.362 + miR.369.3p + miR.376a* + miR.494 + miR.518c + miR.639 + miR.660 + miR.338 | 0,81 | 0,136852887 |
| miR.558 + miR.609 + miR.520f + miR.220a + miR.519b + miR.130a + miR.26a + miR.29a + miR.362 + miR.369.3p + miR.376a* + miR.518c + miR.639 + miR.660 | 0,81 | 0,338614254 |
| miR.609 + miR.603 + miR.520f + miR.220a + miR.130a + miR.29a + miR.362 + miR.369.3p + miR.376a* + miR.494 + miR.518c + miR.639 + miR.660 + miR.338 | 0,81 | 0,14186496 |
| miR.558 + miR.609 + miR.603 + miR.520f + miR.519b + miR.26a + miR.29a + miR.362 + miR.369.3p + miR.376a* + miR.494 + miR.518c + miR.639 + miR.660 | 0,81 | 0,047922139 |
| miR.558 + miR.609 + miR.603 + miR.520f + miR.519b + miR.26a + miR.29a + miR.362 + miR.369.3p + miR.376a* + miR.494 + miR.518c + miR.639 + miR.338 | 0,81 | 0,07310338 |
| miR.558 + miR.609 + miR.603 + miR.520f + miR.220a + miR.130a + miR.26a + miR.29a + miR.362 + miR.376a* + miR.494 + miR.518c + miR.639 + miR.338 | 0,81 | 0,024917674 |
| miR.558 + miR.609 + miR.520f + miR.220a + miR.130a + miR.26a + miR.29a + miR.362 + miR.369.3p + miR.376a* + miR.494 + miR.518c + miR.639 + miR.338 | 0,81 | 0,096809607 |
| miR.558 + miR.609 + miR.603 + miR.520f + miR.519b + miR.130a + miR.26a + miR.29a + miR.362 + miR.369.3p + miR.376a* + miR.494 + miR.518c + miR.338 | 0,81 | 0,104729022 |
| miR.558 + miR.609 + miR.603 + miR.220a + miR.519b + miR.26a + miR.29a + miR.362 + miR.376a* + miR.494 + miR.518c + miR.639 + miR.660 + miR.338 | 0,81 | 0,126125163 |
| miR.558 + miR.609 + miR.603 + miR.520f + miR.220a + miR.519b + miR.130a + miR.26a + miR.29a + miR.362 + miR.376a* + miR.518c + miR.639 + miR.338 | 0,81 | 0,103732939 |
| miR.558 + miR.609 + miR.603 + miR.520f + miR.220a + miR.519b + miR.130a + miR.26a + miR.29a + miR.376a* + miR.494 + miR.518c + miR.639 + miR.338 | 0,81 | 0,038656582 |
| miR.558 + miR.609 + miR.520f + miR.220a + miR.519b + miR.130a + miR.29a + miR.362 + miR.376a* + miR.494 + miR.518c + miR.639 + miR.660 + miR.338 | 0,81 | 0,175993851 |
| miR.558 + miR.609 + miR.603 + miR.520f + miR.519b + miR.130a + miR.26a + miR.29a + miR.362 + miR.369.3p + miR.376a* + miR.518c + miR.639 + miR.338 | 0,81 | 0,063042743 |
| miR.558 + miR.609 + miR.603 + miR.520f + miR.220a + miR.130a + miR.362 + miR.369.3p + miR.376a* + miR.494 + miR.518c + miR.639 + miR.660 + miR.338 | 0,81 | 0,134146886 |
| miR.558 + miR.609 + miR.520f + miR.220a + miR.130a + miR.26a + miR.29a + miR.362 + miR.376a* + miR.494 + miR.518c + miR.639 + miR.660 + miR.338 | 0,81 | 0,06959903 |
| miR.609 + miR.603 + miR.220a + miR.130a + miR.26a + miR.29a + miR.362 + miR.369.3p + miR.376a* + miR.494 + miR.518c + miR.639 + miR.660 + miR.338 | 0,81 | 0,381927294 |
| miR.558 + miR.609 + miR.603 + miR.520f + miR.220a + miR.130a + miR.26a + miR.29a + miR.362 + miR.376a* + miR.494 + miR.518c + miR.639 + miR.660 | 0,81 | 0,042926309 |
| miR.609 + miR.603 + miR.520f + miR.220a + miR.519b + miR.130a + miR.29a + miR.362 + miR.369.3p + miR.376a* + miR.494 + miR.518c + miR.660 + miR.338 | 0,81 | 0,39643341 |
| miR.558 + miR.609 + miR.603 + miR.520f + miR.220a + miR.130a + miR.26a + miR.362 + miR.369.3p + miR.376a* + miR.518c + miR.639 + miR.660 + miR.338 | 0,81 | 0,154550367 |
| miR.558 + miR.609 + miR.520f + miR.220a + miR.519b + miR.26a + miR.29a + miR.362 + miR.376a* + miR.494 + miR.518c + miR.639 + miR.660 + miR.338 | 0,81 | 0,24815357 |
| miR.558 + miR.609 + miR.520f + miR.220a + miR.519b + miR.130a + miR.26a + miR.29a + miR.376a* + miR.494 + miR.518c + miR.639 + miR.660 + miR.338 | 0,81 | 0,17067642 |
| miR.609 + miR.603 + miR.520f + miR.220a + miR.519b + miR.26a + miR.29a + miR.362 + miR.369.3p + miR.376a* + miR.494 + miR.518c + miR.660 + miR.338 | 0,81 | 0,396187172 |
| miR.609 + miR.603 + miR.220a + miR.519b + miR.26a + miR.29a + miR.362 + miR.369.3p + miR.376a* + miR.494 + miR.518c + miR.639 + miR.660 + miR.338 | 0,81 | 0,388457399 |
| miR.558 + miR.609 + miR.603 + miR.520f + miR.220a + miR.26a + miR.362 + miR.369.3p + miR.376a* + miR.494 + miR.518c + miR.639 + miR.660 + miR.338 | 0,81 | 0,122979609 |
| miR.558 + miR.609 + miR.520f + miR.220a + miR.519b + miR.26a + miR.29a + miR.362 + miR.369.3p + miR.376a* + miR.494 + miR.518c + miR.639 + miR.338 | 0,81 | 0,156384689 |
| miR.558 + miR.609 + miR.603 + miR.520f + miR.220a + miR.519b + miR.130a + miR.362 + miR.369.3p + miR.376a* + miR.518c + miR.639 + miR.660 + miR.338 | 0,81 | 0,141322411 |
| miR.609 + miR.603 + miR.520f + miR.519b + miR.130a + miR.29a + miR.362 + miR.369.3p + miR.376a* + miR.494 + miR.518c + miR.639 + miR.660 + miR.338 | 0,81 | 0,389267118 |
| miR.609 + miR.603 + miR.520f + miR.519b + miR.26a + miR.29a + miR.362 + miR.369.3p + miR.376a* + miR.494 + miR.518c + miR.639 + miR.660 + miR.338 | 0,81 | 0,43469556 |
| miR.558 + miR.609 + miR.603 + miR.520f + miR.220a + miR.130a + miR.26a + miR.29a + miR.362 + miR.376a* + miR.494 + miR.518c + miR.660 + miR.338 | 0,81 | 0,049259581 |
| miR.609 + miR.603 + miR.520f + miR.130a + miR.26a + miR.29a + miR.362 + miR.369.3p + miR.376a* + miR.494 + miR.518c + miR.639 + miR.660 + miR.338 | 0,81 | 0,35904379 |
| miR.609 + miR.603 + miR.520f + miR.220a + miR.130a + miR.26a + miR.29a + miR.362 + miR.369.3p + miR.376a* + miR.494 + miR.518c + miR.660 + miR.338 | 0,81 | 0,448842699 |
| miR.558 + miR.609 + miR.603 + miR.520f + miR.519b + miR.130a + miR.362 + miR.369.3p + miR.376a* + miR.494 + miR.518c + miR.639 + miR.660 + miR.338 | 0,81 | 0,160209184 |
| miR.558 + miR.609 + miR.520f + miR.220a + miR.519b + miR.130a + miR.26a + miR.29a + miR.362 + miR.369.3p + miR.376a* + miR.494 + miR.518c + miR.338 | 0,81 | 0,140657418 |
| miR.558 + miR.609 + miR.603 + miR.520f + miR.519b + miR.130a + miR.29a + miR.362 + miR.376a* + miR.494 + miR.518c + miR.639 + miR.660 + miR.338 | 0,81 | 0,07626918 |
| miR.558 + miR.609 + miR.603 + miR.520f + miR.519b + miR.130a + miR.26a + miR.29a + miR.362 + miR.376a* + miR.494 + miR.518c + miR.639 + miR.338 | 0,81 | 0,076877765 |
| miR.609 + miR.603 + miR.520f + miR.220a + miR.519b + miR.130a + miR.29a + miR.369.3p + miR.376a* + miR.494 + miR.518c + miR.639 + miR.660 + miR.338 | 0,81 | 0,13790433 |
| miR.558 + miR.609 + miR.520f + miR.220a + miR.519b + miR.130a + miR.26a + miR.29a + miR.369.3p + miR.376a* + miR.494 + miR.518c + miR.639 + miR.338 | 0,81 | 0,129387255 |
| miR.558 + miR.609 + miR.603 + miR.520f + miR.220a + miR.130a + miR.26a + miR.29a + miR.362 + miR.376a* + miR.518c + miR.639 + miR.660 + miR.338 | 0,81 | 0,031372301 |
| miR.558 + miR.609 + miR.520f + miR.220a + miR.519b + miR.130a + miR.29a + miR.362 + miR.369.3p + miR.376a* + miR.494 + miR.518c + miR.639 + miR.338 | 0,81 | 0,181625827 |
| miR.558 + miR.609 + miR.603 + miR.520f + miR.220a + miR.519b + miR.130a + miR.26a + miR.29a + miR.362 + miR.376a* + miR.518c + miR.639 + miR.660 | 0,81 | 0,136519596 |
| miR.558 + miR.609 + miR.603 + miR.520f + miR.220a + miR.519b + miR.362 + miR.369.3p + miR.376a* + miR.494 + miR.518c + miR.639 + miR.660 + miR.338 | 0,81 | 0,089797649 |
| miR.558 + miR.609 + miR.603 + miR.520f + miR.519b + miR.130a + miR.26a + miR.29a + miR.362 + miR.369.3p + miR.376a* + miR.494 + miR.518c + miR.639 | 0,81 | 0,098636684 |
| miR.558 + miR.609 + miR.220a + miR.519b + miR.130a + miR.26a + miR.29a + miR.362 + miR.369.3p + miR.376a* + miR.494 + miR.518c + miR.639 + miR.338 | 0,81 | 0,110669023 |
| miR.558 + miR.609 + miR.520f + miR.519b + miR.130a + miR.26a + miR.29a + miR.362 + miR.376a* + miR.494 + miR.518c + miR.639 + miR.660 + miR.338 | 0,81 | 0,073619589 |
| miR.558 + miR.609 + miR.603 + miR.520f + miR.519b + miR.130a + miR.26a + miR.362 + miR.369.3p + miR.376a* + miR.518c + miR.639 + miR.660 + miR.338 | 0,81 | 0,183658291 |
| miR.558 + miR.609 + miR.603 + miR.520f + miR.130a + miR.26a + miR.362 + miR.369.3p + miR.376a* + miR.494 + miR.518c + miR.639 + miR.660 + miR.338 | 0,81 | 0,169680354 |
| miR.558 + miR.609 + miR.603 + miR.520f + miR.519b + miR.130a + miR.26a + miR.29a + miR.362 + miR.369.3p + miR.376a* + miR.518c + miR.639 + miR.660 | 0,81 | 0,057581379 |
| miR.558 + miR.609 + miR.603 + miR.220a + miR.519b + miR.130a + miR.26a + miR.29a + miR.362 + miR.376a* + miR.518c + miR.639 + miR.660 + miR.338 | 0,81 | 0,093708983 |
| miR.609 + miR.603 + miR.520f + miR.220a + miR.519b + miR.26a + miR.29a + miR.369.3p + miR.376a* + miR.494 + miR.518c + miR.639 + miR.660 + miR.338 | 0,81 | 0,136525761 |
| miR.558 + miR.609 + miR.603 + miR.520f + miR.220a + miR.519b + miR.130a + miR.29a + miR.362 + miR.376a* + miR.518c + miR.639 + miR.660 + miR.338 | 0,81 | 0,087714608 |
| miR.609 + miR.520f + miR.220a + miR.519b + miR.130a + miR.29a + miR.362 + miR.369.3p + miR.376a* + miR.494 + miR.518c + miR.639 + miR.660 + miR.338 | 0,81 | 0,372144025 |
| miR.558 + miR.609 + miR.520f + miR.220a + miR.519b + miR.130a + miR.26a + miR.29a + miR.362 + miR.376a* + miR.494 + miR.518c + miR.660 + miR.338 | 0,81 | 0,264678188 |
| miR.609 + miR.603 + miR.520f + miR.220a + miR.519b + miR.130a + miR.29a + miR.362 + miR.369.3p + miR.376a* + miR.494 + miR.518c + miR.639 + miR.660 | 0,81 | 0,117462912 |
| miR.558 + miR.609 + miR.520f + miR.220a + miR.519b + miR.130a + miR.26a + miR.29a + miR.362 + miR.369.3p + miR.376a* + miR.518c + miR.639 + miR.338 | 0,81 | 0,114780583 |
| miR.609 + miR.520f + miR.220a + miR.519b + miR.26a + miR.29a + miR.362 + miR.369.3p + miR.376a* + miR.494 + miR.518c + miR.639 + miR.660 + miR.338 | 0,81 | 0,316032605 |
| miR.609 + miR.603 + miR.520f + miR.220a + miR.519b + miR.26a + miR.29a + miR.362 + miR.369.3p + miR.376a* + miR.494 + miR.518c + miR.639 + miR.660 | 0,81 | 0,137540765 |
| miR.558 + miR.609 + miR.603 + miR.520f + miR.220a + miR.519b + miR.26a + miR.29a + miR.362 + miR.376a^{*} + miR.518c + miR.639 + miR.660 + miR.338 | 0,81 | 0,090969586 |
| miR.558 + miR.609 + miR.603 + miR.520f + miR.519b + miR.26a + miR.29a + miR.362 + miR.376a* + miR.494 + miR.518c + miR.639 + miR.660 + miR.338 | 0,81 | 0,042006553 |
| miR.558 + miR.609 + miR.603 + miR.520f + miR.220a + miR.519b + miR.130a + miR.26a + miR.29a + miR.376a* + miR.518c + miR.639 + miR.660 + miR.338 | 0,81 | 0,09272833 |
| miR.558 + miR.609 + miR.603 + miR.520f + miR.220a + miR.519b + miR.26a + miR.362 + miR.369.3p + miR.376a* + miR.518c + miR.639 + miR.660 + miR.338 | 0,81 | 0,108268386 |
| miR.558 + miR.609 + miR.520f + miR.519b + miR.130a + miR.26a + miR.29a + miR.362 + miR.369.3p + miR.376a* + miR.494 + miR.518c + miR.639 + miR.338 | 0,81 | 0,181426425 |
| miR.558 + miR.609 + miR.603 + miR.520f + miR.130a + miR.26a + miR.29a + miR.362 + miR.376a* + miR.494 + miR.518c + miR.639 + miR.660 + miR.338 | 0,81 | 0,01410596 |
| miR.609 + miR.603 + miR.220a + miR.519b + miR.130a + miR.29a + miR.362 + miR.369.3p + miR.376a* + miR.494 + miR.518c + miR.639 + miR.660 + miR.338 | 0,81 | 0,302330031 |
| miR.558 + miR.609 + miR.520f + miR.220a + miR.519b + miR.130a + miR.26a + miR.29a + miR.362 + miR.376a* + miR.494 + miR.518c + miR.639 + miR.338 | 0,81 | 0,169836384 |
| miR.558 + miR.609 + miR.520f + miR.220a + miR.519b + miR.130a + miR.26a + miR.29a + miR.362 + miR.369.3p + miR.376a* + miR.494 + miR.518c + miR.639 | 0,81 | 0,186796637 |
| miR.609 + miR.603 + miR.520f + miR.220a + miR.130a + miR.26a + miR.29a + miR.362 + miR.369.3p + miR.376a* + miR.494 + miR.518c + miR.639 + miR.660 | 0,81 | 0,077352304 |
| miR.609 + miR.603 + miR.520f + miR.220a + miR.519b + miR.130a + miR.26a + miR.29a + miR.369.3p + miR.376a* + miR.494 + miR.518c + miR.660 + miR.338 | 0,81 | 0,214717654 |
| miR.558 + miR.609 + miR.603 + miR.520f + miR.220a + miR.130a + miR.29a + miR.362 + miR.369.3p + miR.376a* + miR.494 + miR.639 + miR.660 + miR.338 | 0,81 | 0,261582877 |
| miR.558 + miR.609 + miR.603 + miR.520f + miR.220a + miR.519b + miR.130a + miR.26a + miR.29a + miR.362 + miR.376a* + miR.518c + miR.660 + miR.338 | 0,81 | 0,161945913 |
| miR.609 + miR.603 + miR.520f + miR.220a + miR.519b + miR.130a + miR.29a + miR.362 + miR.369.3p + miR.376a* + miR.518c + miR.639 + miR.660 + miR.338 | 0,81 | 0,291294383 |
| miR.558 + miR.609 + miR.603 + miR.520f + miR.519b + miR.130a + miR.26a + miR.29a + miR.376a* + miR.494 + miR.518c + miR.639 + miR.660 + miR.338 | 0,81 | 0,080526782 |
| miR.558 + miR.609 + miR.520f + miR.220a + miR.130a + miR.26a + miR.29a + miR.362 + miR.369.3p + miR.376a* + miR.494 + miR.639 + miR.660 + miR.338 | 0,81 | 0,248731002 |
| miR.609 + miR.603 + miR.520f + miR.220a + miR.130a + miR.26a + miR.29a + miR.362 + miR.369.3p + miR.376a* + miR.518c + miR.639 + miR.660 + miR.338 | 0,81 | 0,283710814 |
| miR.609 + miR.603 + miR.220a + miR.519b + miR.130a + miR.26a + miR.29a + miR.362 + miR.369.3p + miR.376a* + miR.494 + miR.518c + miR.660 + miR.338 | 0,81 | 0,419568865 |
| miR.609 + miR.603 + miR.220a + miR.519b + miR.130a + miR.26a + miR.29a + miR.362 + miR.369.3p + miR.376a* + miR.494 + miR.518c + miR.639 + miR.660 | 0,81 | 0,180432995 |
| miR.558 + miR.609 + miR.520f + miR.220a + miR.519b + miR.130a + miR.29a + miR.362 + miR.369.3p + miR.376a* + miR.494 + miR.639 + miR.660 + miR.338 | 0,81 | 0,243311863 |
| miR.558 + miR.609 + miR.603 + miR.520f + miR.130a + miR.26a + miR.29a + miR.362 + miR.369.3p + miR.376a* + miR.494 + miR.639 + miR.660 + miR.338 | 0,81 | 0,286216354 |
| miR.558 + miR.609 + miR.603 + miR.520f + miR.519b + miR.130a + miR.29a + miR.362 + miR.369.3p + miR.376a* + miR.494 + miR.639 + miR.660 + miR.338 | 0,81 | 0,251021462 |
| miR.558 + miR.609 + miR.603 + miR.520f + miR.519b + miR.130a + miR.26a + miR.29a + miR.362 + miR.369.3p + miR.376a* + miR.494 + miR.518c + miR.660 | 0,81 | 0,154399614 |
| miR.609 + miR.603 + miR.520f + miR.220a + miR.519b + miR.130a + miR.26a + miR.29a + miR.362 + miR.369.3p + miR.376a* + miR.494 + miR.518c + miR.660 | 0,81 | 0,180502926 |
| miR.558 + miR.609 + miR.520f + miR.519b + miR.130a + miR.26a + miR.29a + miR.362 + miR.369.3p + miR.376a* + miR.494 + miR.639 + miR.660 + miR.338 | 0,81 | 0,290119402 |
| miR.558 + miR.609 + miR.603 + miR.520f + miR.220a + miR.26a + miR.29a + miR.362 + miR.369.3p + miR.376a* + miR.494 + miR.639 + miR.660 + miR.338 | 0,81 | 0,305026907 |
| miR.558 + miR.609 + miR.520f + miR.220a + miR.519b + miR.130a + miR.26a + miR.29a + miR.362 + miR.376a* + miR.494 + miR.518c + miR.639 + miR.660 | 0,81 | 0,183012623 |
| miR.558 + miR.609 + miR.603 + miR.520f + miR.519b + miR.26a + miR.362 + miR.369.3p + miR.376a* + miR.494 + miR.518c + miR.639 + miR.660 + miR.338 | 0,81 | 0,133969406 |
| miR.609 + miR.520f + miR.220a + miR.130a + miR.26a + miR.29a + miR.362 + miR.369.3p + miR.376a* + miR.494 + miR.518c + miR.639 + miR.660 + miR.338 | 0,81 | 0,353970356 |
| miR.609 + miR.603 + miR.520f + miR.220a + miR.519b + miR.26a + miR.29a + miR.362 + miR.369.3p + miR.376a* + miR.518c + miR.639 + miR.660 + miR.338 | 0,81 | 0,320805885 |
| miR.558 + miR.609 + miR.603 + miR.520f + miR.220a + miR.519b + miR.29a + miR.362 + miR.369.3p + miR.376a* + miR.494 + miR.639 + miR.660 + miR.338 | 0,81 | 0,277875226 |
| miR.558 + miR.609 + miR.520f + miR.220a + miR.519b + miR.26a + miR.29a + miR.362 + miR.369.3p + miR.376a* + miR.494 + miR.639 + miR.660 + miR.338 | 0,81 | 0,302816376 |
| miR.558 + miR.609 + miR.520f + miR.220a + miR.519b + miR.130a + miR.362 + miR.369.3p + miR.376a* + miR.494 + miR.518c + miR.639 + miR.660 + miR.338 | 0,81 | 0,160458522 |
| miR.609 + miR.603 + miR.520f + miR.519b + miR.130a + miR.26a + miR.29a + miR.362 + miR.369.3p + miR.376a* + miR.518c + miR.639 + miR.660 + miR.338 | 0,81 | 0,436247623 |
| miR.609 + miR.603 + miR.220a + miR.519b + miR.130a + miR.26a + miR.29a + miR.369.3p + miR.376a* + miR.494 + miR.518c + miR.639 + miR.660 + miR.338 | 0,81 | 0,126768635 |
| miR.609 + miR.603 + miR.520f + miR.220a + miR.519b + miR.130a + miR.26a + miR.29a + miR.369.3p + miR.376a* + miR.494 + miR.518c + miR.639 + miR.660 | 0,81 | 0,094746111 |
| miR.558 + miR.609 + miR.603 + miR.520f + miR.519b + miR.130a + miR.26a + miR.29a + miR.362 + miR.376a* + miR.494 + miR.518c + miR.660 + miR.338 | 0,81 | 0,120212301 |
| miR.558 + miR.609 + miR.220a + miR.519b + miR.130a + miR.26a + miR.29a + miR.362 + miR.376a* + miR.494 + miR.518c + miR.639 + miR.660 + miR.338 | 0,81 | 0,13177627 |
| miR.558 + miR.609 + miR.520f + miR.220a + miR.519b + miR.130a + miR.26a + miR.29a + miR.362 + miR.376a* + miR.518c + miR.639 + miR.660 + miR.338 | 0,81 | 0,123149461 |
| miR.558 + miR.609 + miR.603 + miR.520f + miR.519b + miR.26a + miR.29a + miR.362 + miR.369.3p + miR.376a* + miR.494 + miR.639 + miR.660 + miR.338 | 0,81 | 0,328620869 |
| miR.609 + miR.520f + miR.220a + miR.519b + miR.130a + miR.26a + miR.29a + miR.362 + miR.369.3p + miR.376a* + miR.518c + miR.639 + miR.660 + miR.338 | 0,81 | 0,341948655 |
| miR.609 + miR.520f + miR.519b + miR.130a + miR.26a + miR.29a + miR.362 + miR.369.3p + miR.376a* + miR.494 + miR.518c + miR.639 + miR.660 + miR.338 | 0,81 | 0,488586805 |
| miR.609 + miR.603 + miR.520f + miR.220a + miR.519b + miR.130a + miR.26a + miR.29a + miR.362 + miR.369.3p + miR.376a* + miR.518c + miR.660 + miR.338 | 0,81 | 0,368413093 |
| miR.558 + miR.609 + miR.603 + miR.520f + miR.519b + miR.130a + miR.26a + miR.29a + miR.362 + miR.376a* + miR.518c + miR.639 + miR.660 + miR.338 | 0,81 | 0,058905172 |
| miR.609 + miR.603 + miR.520f + miR.220a + miR.130a + miR.26a + miR.29a + miR.369.3p + miR.376a* + miR.494 + miR.518c + miR.639 + miR.660 + miR.338 | 0,81 | 0,082143766 |
| miR.609 + miR.603 + miR.520f + miR.220a + miR.519b + miR.130a + miR.26a + miR.29a + miR.362 + miR.369.3p + miR.376a* + miR.518c + miR.639 + miR.660 | 0,81 | 0,222557881 |
| miR.558 + miR.609 + miR.603 + miR.520f + miR.519b + miR.130a + miR.26a + miR.29a + miR.362 + miR.376a* + miR.494 + miR.518c + miR.639 + miR.660 | 0,81 | 0,085203912 |
| miR.558 + miR.609 + miR.520f + miR.220a + miR.519b + miR.130a + miR.26a + miR.362 + miR.369.3p + miR.376a* + miR.518c + miR.639 + miR.660 + miR.338 | 0,81 | 0,180493636 |
| miR.558 + miR.609 + miR.520f + miR.220a + miR.130a + miR.26a + miR.362 + miR.369.3p + miR.376a* + miR.494 + miR.518c + miR.639 + miR.660 + miR.338 | 0,81 | 0,16247103 |
| miR.558 + miR.609 + miR.520f + miR.519b + miR.130a + miR.26a + miR.362 + miR.369.3p + miR.376a* + miR.494 + miR.518c + miR.639 + miR.660 + miR.338 | 0,81 | 0,235809401 |
| miR.558 + miR.609 + miR.603 + miR.519b + miR.130a + miR.26a + miR.29a + miR.362 + miR.376a^{*} + miR.494 + miR.518c + miR.639 + miR.660 + miR.338 | 0,81 | 0,026730385 |
| miR.609 + miR.603 + miR.220a + miR.519b + miR.130a + miR.26a + miR.29a + miR.362 + miR.369.3p + miR.376a* + miR.518c + miR.639 + miR.660 + miR.338 | 0,81 | 0,425981471 |
| miR.558 + miR.609 + miR.520f + miR.220a + miR.519b + miR.26a + miR.362 + miR.369.3p + miR.376a* + miR.494 + miR.518c + miR.639 + miR.660 + miR.338 | 0,81 | 0,162260334 |
| miR.609 + miR.603 + miR.520f + miR.220a + miR.519b + miR.130a + miR.26a + miR.29a + miR.369.3p + miR.376a* + miR.518c + miR.639 + miR.660 + miR.338 | 0,81 | 0,214705087 |
| miR.558 + miR.609 + miR.603 + miR.520f + miR.220a + miR.130a + miR.26a + miR.362 + miR.369.3p + miR.376a* + miR.494 + miR.518c + miR.639 + miR.660 | 0,81 | 0,023712127 |
| miR.558 + miR.609 + miR.603 + miR.520f + miR.220a + miR.519b + miR.130a + miR.362 + miR.369.3p + miR.376a* + miR.494 + miR.518c + miR.639 + miR.660 | 0,81 | 0,024618759 |
| miR.558 + miR.609 + miR.603 + miR.520f + miR.220a + miR.519b + miR.130a + miR.26a + miR.362 + miR.369.3p + miR.376a* + miR.518c + miR.639 + miR.660 | 0,81 | 0,024608771 |
| miR.609 + miR.520f + miR.220a + miR.519b + miR.130a + miR.26a + miR.29a + miR.362 + miR.369.3p + miR.376a* + miR.494 + miR.518c + miR.660 + miR.338 | 0,81 | 0,414060871 |
| miR.558 + miR.609 + miR.603 + miR.520f + miR.220a + miR.519b + miR.130a + miR.26a + miR.369.3p + miR.376a* + miR.518c + miR.639 + miR.660 + miR.338 | 0,81 | 0,129481869 |
| miR.609 + miR.603 + miR.520f + miR.220a + miR.519b + miR.130a + miR.26a + miR.29a + miR.362 + miR.369.3p + miR.376a* + miR.494 + miR.518c + miR.639 | 0,81 | 0,080918235 |
| miR.558 + miR.609 + miR.520f + miR.220a + miR.519b + miR.130a + miR.26a + miR.29a + miR.362 + miR.369.3p + miR.376a* + miR.494 + miR.660 + miR.338 | 0,81 | 0,306562122 |
| miR.609 + miR.603 + miR.519b + miR.130a + miR.26a + miR.29a + miR.362 + miR.369.3p + miR.376a* + miR.494 + miR.518c + miR.639 + miR.660 + miR.338 | 0,81 | 0,109526284 |
| miR.558 + miR.609 + miR.603 + miR.520f + miR.519b + miR.130a + miR.26a + miR.29a + miR.362 + miR.369.3p + miR.376a* + miR.494 + miR.660 + miR.338 | 0,81 | 0,33160657 |
| miR.558 + miR.609 + miR.603 + miR.220a + miR.519b + miR.26a + miR.362 + miR.369.3p + miR.376a* + miR.494 + miR.518c + miR.639 + miR.660 + miR.338 | 0,8 | 0,077144205 |
| miR.558 + miR.609 + miR.603 + miR.520f + miR.220a + miR.519b + miR.130a + miR.369.3p + miR.376a* + miR.494 + miR.518c + miR.639 + miR.660 + miR.338 | 0,8 | 0,12378441 |
| miR.558 + miR.609 + miR.603 + miR.520f + miR.220a + miR.519b + miR.26a + miR.29a + miR.362 + miR.369.3p + miR.376a* + miR.494 + miR.660 + miR.338 | 0,8 | 0,344327611 |
| miR.609 + miR.603 + miR.520f + miR.519b + miR.130a + miR.26a + miR.29a + miR.362 + miR.369.3p + miR.376a* + miR.494 + miR.518c + miR.660 + miR.338 | 0,8 | 0,354329436 |
| miR.558 + miR.609 + miR.603 + miR.520f + miR.220a + miR.130a + miR.26a + miR.369.3p + miR.376a* + miR.494 + miR.518c + miR.639 + miR.660 + miR.338 | 0,8 | 0,098351722 |
| miR.558 + miR.609 + miR.603 + miR.520f + miR.220a + miR.519b + miR.130a + miR.29a + miR.362 + miR.369.3p + miR.376a* + miR.494 + miR.660 + miR.338 | 0,8 | 0,263240613 |
| miR.558 + miR.609 + miR.603 + miR.220a + miR.519b + miR.130a + miR.26a + miR.362 + miR.369.3p + miR.376a* + miR.518c + miR.639 + miR.660 + miR.338 | 0,8 | 0,078061857 |
| miR.558 + miR.609 + miR.603 + miR.220a + miR.130a + miR.26a + miR.362 + miR.369.3p + miR.376a* + miR.494 + miR.518c + miR.639 + miR.660 + miR.338 | 0,8 | 0,071091157 |
| miR.558 + miR.609 + miR.603 + miR.520f + miR.220a + miR.519b + miR.130a + miR.29a + miR.362 + miR.369.3p + miR.376a* + miR.639 + miR.660 + miR.338 | 0,8 | 0,245793065 |
| miR.558 + miR.609 + miR.603 + miR.520f + miR.220a + miR.130a + miR.26a + miR.29a + miR.362 + miR.369.3p + miR.376a* + miR.494 + miR.660 + miR.338 | 0,8 | 0,35909622 |
| miR.609 + miR.520f + miR.220a + miR.519b + miR.130a + miR.26a + miR.29a + miR.362 + miR.369.3p + miR.376a* + miR.494 + miR.518c + miR.639 + miR.660 | 0,8 | 0,046802378 |
| miR.558 + miR.609 + miR.520f + miR.220a + miR.519b + miR.130a + miR.26a + miR.29a + miR.362 + miR.369.3p + miR.376a* + miR.639 + miR.660 + miR.338 | 0,8 | 0,297700793 |
| miR.558 + miR.609 + miR.603 + miR.520f + miR.519b + miR.130a + miR.26a + miR.29a + miR.362 + miR.369.3p + miR.376a* + miR.639 + miR.660 + miR.338 | 0,8 | 0,311833614 |
| miR.558 + miR.609 + miR.603 + miR.520f + miR.220a + miR.519b + miR.26a + miR.29a + miR.362 + miR.369.3p + miR.376a* + miR.639 + miR.660 + miR.338 | 0,8 | 0,276015157 |
| miR.609 + miR.603 + miR.520f + miR.220a + miR.519b + miR.130a + miR.26a + miR.362 + miR.369.3p + miR.376a* + miR.518c + miR.639 + miR.660 + miR.338 | 0,8 | 0,177294609 |
| miR.609 + miR.603 + miR.520f + miR.220a + miR.130a + miR.26a + miR.362 + miR.369.3p + miR.376a* + miR.494 + miR.518c + miR.639 + miR.660 + miR.338 | 0,8 | 0,188464045 |
| miR.609 + miR.603 + miR.520f + miR.220a + miR.519b + miR.130a + miR.29a + miR.362 + miR.369.3p + miR.376a* + miR.494 + miR.518c + miR.639 + miR.338 | 0,8 | 0,055149277 |
| miR.609 + miR.603 + miR.520f + miR.220a + miR.519b + miR.26a + miR.29a + miR.362 + miR.369.3p + miR.376a* + miR.494 + miR.518c + miR.639 + miR.338 | 0,8 | 0,058530001 |
| miR.558 + miR.609 + miR.603 + miR.520f + miR.519b + miR.130a + miR.26a + miR.369.3p + miR.376a* + miR.494 + miR.518c + miR.639 + miR.660 + miR.338 | 0,8 | 0,073419161 |
| miR.609 + miR.603 + miR.520f + miR.220a + miR.519b + miR.130a + miR.362 + miR.369.3p + miR.376a* + miR.494 + miR.518c + miR.639 + miR.660 + miR.338 | 0,8 | 0,160884494 |
| miR.558 + miR.609 + miR.603 + miR.520f + miR.220a + miR.130a + miR.26a + miR.29a + miR.362 + miR.369.3p + miR.376a* + miR.639 + miR.660 + miR.338 | 0,8 | 0,251950223 |
| miR.558 + miR.609 + miR.603 + miR.220a + miR.519b + miR.130a + miR.26a + miR.362 + miR.369.3p + miR.376a* + miR.494 + miR.518c + miR.639 + miR.660 | 0,8 | 0,037889128 |
| miR.609 + miR.603 + miR.520f + miR.220a + miR.519b + miR.130a + miR.26a + miR.29a + miR.362 + miR.369.3p + miR.376a* + miR.494 + miR.518c + miR.338 | 0,8 | 0,059988822 |
| miR.558 + miR.609 + miR.603 + miR.520f + miR.220a + miR.130a + miR.26a + miR.362 + miR.369.3p + miR.376a* + miR.494 + miR.518c + miR.660 + miR.338 | 0,8 | 0,249427092 |
| miR.609 + miR.603 + miR.520f + miR.220a + miR.519b + miR.130a + miR.29a + miR.362 + miR.376a* + miR.494 + miR.518c + miR.639 + miR.660 + miR.338 | 0,8 | 0,046130925 |
| miR.558 + miR.609 + miR.603 + miR.520f + miR.220a + miR.519b + miR.130a + miR.26a + miR.362 + miR.369.3p + miR.376a* + miR.518c + miR.660 + miR.338 | 0,8 | 0,30411399 |
| miR.609 + miR.603 + miR.520f + miR.220a + miR.519b + miR.130a + miR.26a + miR.29a + miR.369.3p + miR.376a* + miR.494 + miR.518c + miR.639 + miR.338 | 0,8 | 0,05301622 |
| miR.558 + miR.609 + miR.603 + miR.520f + miR.220a + miR.519b + miR.26a + miR.369.3p + miR.376a* + miR.494 + miR.518c + miR.639 + miR.660 + miR.338 | 0,8 | 0,076763417 |
| miR.609 + miR.603 + miR.520f + miR.519b + miR.130a + miR.26a + miR.29a + miR.369.3p + miR.376a* + miR.494 + miR.518c + miR.639 + miR.660 + miR.338 | 0,8 | 0,091403692 |
| miR.558 + miR.609 + miR.603 + miR.520f + miR.220a + miR.519b + miR.130a + miR.26a + miR.369.3p + miR.376a* + miR.494 + miR.518c + miR.639 + miR.660 | 0,8 | 0,036778697 |
| miR.609 + miR.603 + miR.520f + miR.220a + miR.130a + miR.26a + miR.29a + miR.362 + miR.369.3p + miR.376a* + miR.494 + miR.518c + miR.639 + miR.338 | 0,8 | 0,03378263 |
| miR.609 + miR.603 + miR.520f + miR.220a + miR.519b + miR.130a + miR.26a + miR.29a + miR.362 + miR.376a* + miR.494 + miR.518c + miR.639 + miR.660 | 0,8 | 0,05034036 |
| miR.609 + miR.603 + miR.520f + miR.519b + miR.130a + miR.26a + miR.362 + miR.369.3p + miR.376a* + miR.494 + miR.518c + miR.639 + miR.660 + miR.338 | 0,8 | 0,171090226 |
| miR.609 + miR.603 + miR.520f + miR.220a + miR.519b + miR.130a + miR.26a + miR.29a + miR.362 + miR.376a* + miR.494 + miR.518c + miR.660 + miR.338 | 0,8 | 0,087606136 |
| miR.609 + miR.220a + miR.519b + miR.130a + miR.26a + miR.29a + miR.362 + miR.369.3p + miR.376a* + miR.494 + miR.518c + miR.639 + miR.660 + miR.338 | 0,8 | 0,224158548 |
| miR.558 + miR.609 + miR.603 + miR.520f + miR.220a + miR.519b + miR.130a + miR.362 + miR.369.3p + miR.376a* + miR.494 + miR.518c + miR.660 + miR.338 | 0,8 | 0,253686996 |
| miR.609 + miR.603 + miR.520f + miR.519b + miR.130a + miR.26a + miR.29a + miR.362 + miR.369.3p + miR.376a* + miR.494 + miR.518c + miR.639 + miR.660 | 0,8 | 0,006637411 |
| miR.609 + miR.603 + miR.220a + miR.519b + miR.130a + miR.26a + miR.29a + miR.362 + miR.369.3p + miR.376a* + miR.494 + miR.518c + miR.639 + miR.338 | 0,8 | 0,086002133 |
| miR.609 + miR.603 + miR.520f + miR.220a + miR.519b + miR.26a + miR.29a + miR.362 + miR.376a* + miR.494 + miR.518c + miR.639 + miR.660 + miR.338 | 0,8 | 0,055090064 |
| miR.609 + miR.603 + miR.520f + miR.220a + miR.519b + miR.130a + miR.26a + miR.29a + miR.376a* + miR.494 + miR.518c + miR.639 + miR.660 + miR.338 | 0,8 | 0,052475325 |
| miR.558 + miR.609 + miR.603 + miR.520f + miR.220a + miR.519b + miR.26a + miR.362 + miR.369.3p + miR.376a* + miR.494 + miR.518c + miR.639 + miR.660 | 0,8 | 0,013558968 |
| miR.558 + miR.609 + miR.603 + miR.520f + miR.220a + miR.519b + miR.130a + miR.26a + miR.369.3p + miR.376a* + miR.494 + miR.518c + miR.660 + miR.338 | 0,8 | 0,104124958 |
| miR.558 + miR.609 + miR.603 + miR.519b + miR.130a + miR.26a + miR.362 + miR.369.3p + miR.376a* + miR.494 + miR.518c + miR.639 + miR.660 + miR.338 | 0,8 | 0,060776353 |
| miR.609 + miR.520f + miR.220a + miR.519b + miR.130a + miR.26a + miR.29a + miR.369.3p + miR.376a* + miR.494 + miR.518c + miR.639 + miR.660 + miR.338 | 0,8 | 0,109279266 |
| miR.609 + miR.603 + miR.520f + miR.220a + miR.130a + miR.26a + miR.29a + miR.362 + miR.376a* + miR.494 + miR.518c + miR.639 + miR.660 + miR.338 | 0,8 | 0,023839263 |
| miR.558 + miR.609 + miR.603 + miR.520f + miR.220a + miR.519b + miR.130a + miR.26a + miR.369.3p + miR.376a* + miR.494 + miR.518c + miR.639 + miR.338 | 0,8 | 0,023504599 |
| miR.609 + miR.603 + miR.520f + miR.220a + miR.519b + miR.26a + miR.29a + miR.362 + miR.369.3p + miR.376a* + miR.494 + miR.639 + miR.660 + miR.338 | 0,8 | 0,325057189 |
| miR.609 + miR.603 + miR.520f + miR.220a + miR.130a + miR.26a + miR.29a + miR.362 + miR.369.3p + miR.376a* + miR.494 + miR.639 + miR.660 + miR.338 | 0,8 | 0,334934386 |
| miR.609 + miR.603 + miR.520f + miR.519b + miR.130a + miR.26a + miR.29a + miR.362 + miR.369.3p + miR.376a* + miR.494 + miR.639 + miR.660 + miR.338 | 0,8 | 0,342581789 |
| miR.558 + miR.609 + miR.603 + miR.520f + miR.220a + miR.130a + miR.26a + miR.362 + miR.369.3p + miR.376a* + miR.494 + miR.518c + miR.639 + miR.338 | 0,8 | 0,007685147 |
| miR.609 + miR.603 + miR.520f + miR.220a + miR.519b + miR.130a + miR.26a + miR.29a + miR.362 + miR.369.3p + miR.376a* + miR.518c + miR.639 + miR.338 | 0,8 | 0,100091253 |
| miR.558 + miR.609 + miR.603 + miR.520f + miR.220a + miR.519b + miR.130a + miR.26a + miR.362 + miR.369.3p + miR.376a* + miR.518c + miR.639 + miR.338 | 0,8 | 0,020942311 |
| miR.558 + miR.609 + miR.603 + miR.520f + miR.220a + miR.519b + miR.130a + miR.362 + miR.369.3p + miR.376a* + miR.494 + miR.518c + miR.639 + miR.338 | 0,8 | 0,024987059 |
| miR.558 + miR.609 + miR.603 + miR.520f + miR.519b + miR.130a + miR.26a + miR.362 + miR.369.3p + miR.376a* + miR.494 + miR.518c + miR.660 + miR.338 | 0,8 | 0,173225559 |
| miR.558 + miR.609 + miR.603 + miR.220a + miR.130a + miR.26a + miR.29a + miR.362 + miR.369.3p + miR.376a* + miR.494 + miR.639 + miR.660 + miR.338 | 0,8 | 0,11496419 |
| miR.558 + miR.609 + miR.603 + miR.519b + miR.130a + miR.26a + miR.29a + miR.362 + miR.369.3p + miR.376a* + miR.494 + miR.639 + miR.660 + miR.338 | 0,8 | 0,110893359 |
| miR.558 + miR.609 + miR.603 + miR.520f + miR.519b + miR.130a + miR.26a + miR.362 + miR.369.3p + miR.376a* + miR.494 + miR.518c + miR.639 + miR.660 | 0,8 | 0,009546435 |
| miR.609 + miR.603 + miR.520f + miR.519b + miR.130a + miR.26a + miR.29a + miR.362 + miR.369.3p + miR.376a* + miR.494 + miR.518c + miR.639 + miR.338 | 0,8 | 0,049406253 |
| miR.558 + miR.609 + miR.603 + miR.520f + miR.220a + miR.130a + miR.26a + miR.362 + miR.376a* + miR.494 + miR.518c + miR.639 + miR.660 + miR.338 | 0,8 | 0,06378174 |
| miR.609 + miR.603 + miR.520f + miR.220a + miR.519b + miR.26a + miR.362 + miR.369.3p + miR.376a* + miR.494 + miR.518c + miR.639 + miR.660 + miR.338 | 0,8 | 0,107424295 |
| miR.609 + miR.520f + miR.220a + miR.519b + miR.130a + miR.26a + miR.29a + miR.362 + miR.369.3p + miR.376a* + miR.494 + miR.639 + miR.660 + miR.338 | 0,8 | 0,308850265 |
| miR.609 + miR.603 + miR.520f + miR.220a + miR.519b + miR.130a + miR.29a + miR.362 + miR.369.3p + miR.376a* + miR.494 + miR.639 + miR.660 + miR.338 | 0,8 | 0,300731562 |
| miR.558 + miR.609 + miR.220a + miR.519b + miR.130a + miR.26a + miR.29a + miR.362 + miR.369.3p + miR.376a* + miR.494 + miR.639 + miR.660 + miR.338 | 0,8 | 0,136518793 |
| miR.558 + miR.609 + miR.603 + miR.520f + miR.220a + miR.519b + miR.130a + miR.26a + miR.362 + miR.369.3p + miR.376a* + miR.494 + miR.518c + miR.660 | 0,8 | 0,05083898 |
| miR.558 + miR.609 + miR.603 + miR.520f + miR.220a + miR.519b + miR.130a + miR.26a + miR.29a + miR.362 + miR.369.3p + miR.376a* + miR.660 + miR.338 | 0,8 | 0,309992112 |
| miR.558 + miR.609 + miR.603 + miR.220a + miR.519b + miR.130a + miR.362 + miR.369.3p + miR.376a* + miR.494 + miR.518c + miR.639 + miR.660 + miR.338 | 0,8 | 0,056993528 |
| miR.609 + miR.603 + miR.520f + miR.220a + miR.519b + miR.130a + miR.26a + miR.29a + miR.362 + miR.376a* + miR.494 + miR.518c + miR.639 + miR.338 | 0,8 | 0,040920039 |
| miR.609 + miR.603 + miR.220a + miR.519b + miR.130a + miR.26a + miR.29a + miR.362 + miR.376a* + miR.494 + miR.518c + miR.639 + miR.660 + miR.338 | 0,8 | 0,042466516 |
| miR.558 + miR.609 + miR.603 + miR.520f + miR.519b + miR.130a + miR.26a + miR.362 + miR.369.3p + miR.376a* + miR.494 + miR.518c + miR.639 + miR.338 | 0,8 | 0,011241388 |
| miR.558 + miR.609 + miR.603 + miR.520f + miR.220a + miR.519b + miR.130a + miR.26a + miR.362 + miR.376a* + miR.518c + miR.639 + miR.660 + miR.338 | 0,8 | 0,076611718 |
| miR.609 + miR.603 + miR.520f + miR.519b + miR.130a + miR.26a + miR.29a + miR.362 + miR.376a* + miR.494 + miR.518c + miR.639 + miR.660 + miR.338 | 0,8 | 0,01127434 |
| miR.558 + miR.609 + miR.603 + miR.220a + miR.519b + miR.130a + miR.26a + miR.369.3p + miR.376a* + miR.494 + miR.518c + miR.639 + miR.660 + miR.338 | 0,8 | 0,057831206 |
| miR.558 + miR.609 + miR.603 + miR.520f + miR.220a + miR.519b + miR.130a + miR.26a + miR.376a* + miR.494 + miR.518c + miR.639 + miR.660 + miR.338 | 0,8 | 0,088643936 |
| miR.558 + miR.609 + miR.603 + miR.520f + miR.519b + miR.130a + miR.26a + miR.362 + miR.376a* + miR.494 + miR.518c + miR.639 + miR.660 + miR.338 | 0,8 | 0,052153102 |
| miR.558 + miR.609 + miR.603 + miR.520f + miR.220a + miR.519b + miR.130a + miR.26a + miR.362 + miR.369.3p + miR.376a* + miR.494 + miR.518c + miR.338 | 0,8 | 0,026733007 |
| miR.558 + miR.609 + miR.603 + miR.520f + miR.220a + miR.519b + miR.130a + miR.26a + miR.362 + miR.376a* + miR.494 + miR.518c + miR.639 + miR.338 | 0,8 | 0,05819873 |
| miR.558 + miR.609 + miR.520f + miR.220a + miR.519b + miR.130a + miR.26a + miR.362 + miR.369.3p + miR.376a* + miR.494 + miR.518c + miR.639 + miR.660 | 0,8 | 0,010025775 |
| miR.558 + miR.609 + miR.603 + miR.220a + miR.519b + miR.130a + miR.26a + miR.362 + miR.369.3p + miR.376a* + miR.494 + miR.518c + miR.660 + miR.338 | 0,8 | 0,078466893 |
| miR.558 + miR.609 + miR.603 + miR.520f + miR.220a + miR.519b + miR.130a + miR.362 + miR.376a* + miR.494 + miR.518c + miR.639 + miR.660 + miR.338 | 0,8 | 0,084470249 |
| miR.558 + miR.609 + miR.520f + miR.220a + miR.519b + miR.130a + miR.26a + miR.362 + miR.369.3p + miR.376a* + miR.494 + miR.518c + miR.660 + miR.338 | 0,8 | 0,293490283 |
| miR.609 + miR.520f + miR.220a + miR.519b + miR.130a + miR.26a + miR.362 + miR.369.3p + miR.376a* + miR.494 + miR.518c + miR.639 + miR.660 + miR.338 | 0,8 | 0,13971314 |
| miR.558 + miR.609 + miR.603 + miR.220a + miR.519b + miR.130a + miR.29a + miR.362 + miR.369.3p + miR.376a* + miR.494 + miR.639 + miR.660 + miR.338 | 0,8 | 0,068026115 |
| miR.558 + miR.609 + miR.220a + miR.519b + miR.130a + miR.26a + miR.362 + miR.369.3p + miR.376a* + miR.494 + miR.518c + miR.639 + miR.660 + miR.338 | 0,8 | 0,061260888 |
| miR.558 + miR.609 + miR.603 + miR.520f + miR.220a + miR.519b + miR.26a + miR.362 + miR.376a* + miR.494 + miR.518c + miR.639 + miR.660 + miR.338 | 0,8 | 0,046879914 |
| miR.558 + miR.609 + miR.520f + miR.220a + miR.519b + miR.130a + miR.26a + miR.369.3p + miR.376a* + miR.494 + miR.518c + miR.639 + miR.660 + miR.338 | 0,8 | 0,108256788 |
| miR.558 + miR.609 + miR.603 + miR.520f + miR.519b + miR.130a + miR.26a + miR.29a + miR.369.3p + miR.376a* + miR.494 + miR.639 + miR.660 + miR.338 | 0,8 | 0,065432315 |
| miR.558 + miR.609 + miR.603 + miR.520f + miR.519b + miR.130a + miR.26a + miR.29a + miR.362 + miR.369.3p + miR.376a* + miR.494 + miR.639 + miR.660 | 0,8 | 0,01172865 |
| miR.558 + miR.609 + miR.603 + miR.520f + miR.220a + miR.519b + miR.130a + miR.26a + miR.362 + miR.369.3p + miR.376a* + miR.494 + miR.518c + miR.639 | 0,8 | 0,030836129 |
| miR.609 + miR.603 + miR.520f + miR.220a + miR.519b + miR.130a + miR.26a + miR.29a + miR.362 + miR.376a* + miR.518c + miR.639 + miR.660 + miR.338 | 0,8 | 0,095192448 |
| miR.558 + miR.609 + miR.603 + miR.520f + miR.220a + miR.130a + miR.26a + miR.29a + miR.362 + miR.369.3p + miR.376a* + miR.494 + miR.639 + miR.660 | 0,8 | 0,013700819 |
| miR.609 + miR.603 + miR.220a + miR.519b + miR.130a + miR.26a + miR.362 + miR.369.3p + miR.376a* + miR.494 + miR.518c + miR.639 + miR.660 + miR.338 | 0,8 | 0,053720299 |
| miR.558 + miR.609 + miR.603 + miR.520f + miR.220a + miR.519b + miR.26a + miR.362 + miR.369.3p + miR.376a* + miR.494 + miR.518c + miR.639 + miR.338 | 0,8 | 0,015063919 |
| miR.558 + miR.609 + miR.603 + miR.520f + miR.220a + miR.519b + miR.130a + miR.29a + miR.369.3p + miR.376a* + miR.494 + miR.639 + miR.660 + miR.338 | 0,8 | 0,043809313 |
| miR.558 + miR.609 + miR.603 + miR.520f + miR.220a + miR.130a + miR.26a + miR.362 + miR.369.3p + miR.376a* + miR.494 + miR.639 + miR.660 + miR.338 | 0,8 | 0,177177824 |
| miR.558 + miR.609 + miR.603 + miR.520f + miR.519b + miR.130a + miR.26a + miR.362 + miR.369.3p + miR.376a* + miR.494 + miR.639 + miR.660 + miR.338 | 0,8 | 0,190168183 |
| miR.558 + miR.609 + miR.603 + miR.520f + miR.220a + miR.519b + miR.26a + miR.29a + miR.369.3p + miR.376a* + miR.494 + miR.639 + miR.660 + miR.338 | 0,8 | 0,090096711 |
| miR.558 + miR.609 + miR.520f + miR.220a + miR.519b + miR.130a + miR.26a + miR.29a + miR.362 + miR.376a* + miR.494 + miR.639 + miR.660 + miR.338 | 0,8 | 0,058666008 |
| miR.558 + miR.609 + miR.603 + miR.520f + miR.220a + miR.519b + miR.130a + miR.29a + miR.362 + miR.369.3p + miR.376a* + miR.494 + miR.639 + miR.660 | 0,8 | 0,013176111 |
| miR.558 + miR.609 + miR.520f + miR.220a + miR.519b + miR.130a + miR.26a + miR.29a + miR.362 + miR.369.3p + miR.376a* + miR.494 + miR.639 + miR.660 | 0,8 | 0,017767517 |
| miR.609 + miR.603 + miR.520f + miR.220a + miR.519b + miR.130a + miR.26a + miR.29a + miR.362 + miR.369.3p + miR.376a* + miR.494 + miR.660 + miR.338 | 0,8 | 0,361479845 |
| miR.609 + miR.603 + miR.520f + miR.220a + miR.519b + miR.130a + miR.26a + miR.369.3p + miR.376a* + miR.494 + miR.518c + miR.639 + miR.660 + miR.338 | 0,8 | 0,059371244 |
| miR.558 + miR.609 + miR.603 + miR.520f + miR.220a + miR.130a + miR.26a + miR.29a + miR.362 + miR.376a* + miR.494 + miR.639 + miR.660 + miR.338 | 0,8 | 0,017379743 |
| miR.558 + miR.609 + miR.603 + miR.520f + miR.220a + miR.519b + miR.130a + miR.29a + miR.362 + miR.376a* + miR.494 + miR.639 + miR.660 + miR.338 | 0,8 | 0,01317354 |
| miR.558 + miR.609 + miR.603 + miR.220a + miR.519b + miR.130a + miR.26a + miR.362 + miR.369.3p + miR.376a* + miR.494 + miR.518c + miR.639 + miR.338 | 0,8 | 0,015154989 |
| miR.609 + miR.520f + miR.220a + miR.519b + miR.130a + miR.26a + miR.29a + miR.362 + miR.376a* + miR.494 + miR.518c + miR.639 + miR.660 + miR.338 | 0,8 | 0,039426385 |
| miR.609 + miR.603 + miR.520f + miR.220a + miR.519b + miR.130a + miR.26a + miR.362 + miR.369.3p + miR.376a* + miR.494 + miR.518c + miR.639 + miR.660 | 0,8 | 0,017648939 |
| miR.558 + miR.609 + miR.520f + miR.220a + miR.519b + miR.130a + miR.26a + miR.29a + miR.369.3p + miR.376a* + miR.494 + miR.639 + miR.660 + miR.338 | 0,8 | 0,102227385 |
| miR.558 + miR.609 + miR.603 + miR.520f + miR.220a + miR.519b + miR.26a + miR.362 + miR.369.3p + miR.376a* + miR.494 + miR.639 + miR.660 + miR.338 | 0,8 | 0,176365286 |
| miR.558 + miR.609 + miR.603 + miR.520f + miR.220a + miR.519b + miR.130a + miR.26a + miR.29a + miR.369.3p + miR.376a* + miR.494 + miR.660 + miR.338 | 0,8 | 0,110508988 |
| miR.558 + miR.609 + miR.603 + miR.520f + miR.220a + miR.519b + miR.130a + miR.26a + miR.362 + miR.369.3p + miR.376a* + miR.639 + miR.660 + miR.338 | 0,8 | 0,261071933 |
| miR.558 + miR.609 + miR.603 + miR.520f + miR.220a + miR.519b + miR.130a + miR.26a + miR.362 + miR.376a* + miR.494 + miR.518c + miR.660 + miR.338 | 0,8 | 0,078135033 |
| miR.558 + miR.609 + miR.603 + miR.520f + miR.220a + miR.130a + miR.26a + miR.29a + miR.369.3p + miR.376a* + miR.494 + miR.639 + miR.660 + miR.338 | 0,8 | 0,028352292 |
| miR.558 + miR.609 + miR.603 + miR.220a + miR.519b + miR.130a + miR.26a + miR.29a + miR.362 + miR.369.3p + miR.376a* + miR.494 + miR.660 + miR.338 | 0,8 | 0,09826272 |
| miR.558 + miR.609 + miR.603 + miR.520f + miR.220a + miR.519b + miR.26a + miR.29a + miR.362 + miR.369.3p + miR.376a* + miR.494 + miR.639 + miR.660 | 0,8 | 0,027125524 |
| miR.558 + miR.609 + miR.603 + miR.220a + miR.519b + miR.26a + miR.29a + miR.362 + miR.369.3p + miR.376a* + miR.494 + miR.639 + miR.660 + miR.338 | 0,8 | 0,107146535 |
| miR.558 + miR.609 + miR.603 + miR.220a + miR.130a + miR.26a + miR.29a + miR.362 + miR.369.3p + miR.494 + miR.518c + miR.639 + miR.660 + miR.338 | 0,8 | 0,01698892 |
| miR.558 + miR.609 + miR.603 + miR.220a + miR.519b + miR.130a + miR.26a + miR.29a + miR.362 + miR.369.3p + miR.494 + miR.518c + miR.639 + miR.660 | 0,8 | 0,010566286 |
| miR.558 + miR.609 + miR.603 + miR.520f + miR.220a + miR.519b + miR.130a + miR.26a + miR.29a + miR.369.3p + miR.376a* + miR.494 + miR.639 + miR.338 | 0,8 | 0,030742831 |
| miR.558 + miR.609 + miR.520f + miR.220a + miR.519b + miR.130a + miR.26a + miR.362 + miR.369.3p + miR.376a* + miR.494 + miR.639 + miR.660 + miR.338 | 0,8 | 0,214792956 |
| miR.558 + miR.609 + miR.603 + miR.520f + miR.220a + miR.519b + miR.26a + miR.362 + miR.369.3p + miR.376a* + miR.494 + miR.518c + miR.660 + miR.338 | 0,8 | 0,061100427 |
| miR.558 + miR.609 + miR.520f + miR.220a + miR.519b + miR.130a + miR.26a + miR.362 + miR.376a* + miR.494 + miR.518c + miR.639 + miR.660 + miR.338 | 0,8 | 0,091496082 |
| miR.558 + miR.609 + miR.603 + miR.520f + miR.519b + miR.130a + miR.26a + miR.29a + miR.362 + miR.369.3p + miR.376a* + miR.494 + miR.639 + miR.338 | 0,8 | 0,026191542 |
| miR.558 + miR.609 + miR.603 + miR.520f + miR.220a + miR.519b + miR.130a + miR.29a + miR.362 + miR.369.3p + miR.494 + miR.518c + miR.639 + miR.660 | 0,8 | 0,006908093 |
| miR.558 + miR.609 + miR.603 + miR.520f + miR.220a + miR.130a + miR.26a + miR.29a + miR.362 + miR.369.3p + miR.518c + miR.639 + miR.660 + miR.338 | 0,8 | 0,015580289 |
| miR.558 + miR.609 + miR.603 + miR.520f + miR.220a + miR.519b + miR.130a + miR.26a + miR.362 + miR.376a* + miR.494 + miR.518c + miR.639 + miR.660 | 0,8 | 0,055096981 |
| miR.558 + miR.609 + miR.603 + miR.520f + miR.220a + miR.130a + miR.26a + miR.29a + miR.362 + miR.369.3p + miR.494 + miR.518c + miR.639 + miR.660 | 0,8 | 0,009438241 |
| miR.558 + miR.609 + miR.603 + miR.520f + miR.220a + miR.519b + miR.130a + miR.26a + miR.29a + miR.362 + miR.369.3p + miR.376a* + miR.494 + miR.660 | 0,8 | 0,033681962 |
| miR.558 + miR.609 + miR.603 + miR.520f + miR.220a + miR.519b + miR.130a + miR.29a + miR.362 + miR.369.3p + miR.376a* + miR.494 + miR.639 + miR.338 | 0,8 | 0,026398977 |
| miR.609 + miR.603 + miR.520f + miR.220a + miR.519b + miR.130a + miR.26a + miR.362 + miR.369.3p + miR.376a* + miR.494 + miR.518c + miR.660 + miR.338 | 0,8 | 0,262528653 |
| miR.558 + miR.609 + miR.603 + miR.220a + miR.519b + miR.130a + miR.26a + miR.29a + miR.362 + miR.369.3p + miR.494 + miR.518c + miR.660 + miR.338 | 0,8 | 0,030567393 |
| miR.558 + miR.609 + miR.603 + miR.220a + miR.519b + miR.130a + miR.26a + miR.29a + miR.362 + miR.369.3p + miR.518c + miR.639 + miR.660 + miR.338 | 0,8 | 0,031207729 |
| miR.558 + miR.609 + miR.603 + miR.520f + miR.220a + miR.519b + miR.26a + miR.29a + miR.362 + miR.376a* + miR.494 + miR.639 + miR.660 + miR.338 | 0,8 | 0,055830281 |
| miR.558 + miR.609 + miR.603 + miR.520f + miR.519b + miR.130a + miR.26a + miR.29a + miR.362 + miR.376a* + miR.494 + miR.639 + miR.660 + miR.338 | 0,8 | 0,051520906 |
| miR.558 + miR.609 + miR.603 + miR.520f + miR.220a + miR.519b + miR.26a + miR.29a + miR.362 + miR.369.3p + miR.376a* + miR.494 + miR.639 + miR.338 | 0,8 | 0,022972698 |
| miR.558 + miR.609 + miR.603 + miR.520f + miR.220a + miR.519b + miR.130a + miR.26a + miR.29a + miR.362 + miR.369.3p + miR.518c + miR.639 + miR.660 | 0,8 | 0,009348737 |
| miR.558 + miR.609 + miR.603 + miR.520f + miR.220a + miR.519b + miR.130a + miR.26a + miR.29a + miR.362 + miR.376a* + miR.494 + miR.660 + miR.338 | 0,8 | 0,113170361 |
| miR.558 + miR.609 + miR.603 + miR.220a + miR.519b + miR.130a + miR.26a + miR.29a + miR.369.3p + miR.494 + miR.518c + miR.639 + miR.660 + miR.338 | 0,8 | 0,028719048 |
| miR.558 + miR.609 + miR.603 + miR.520f + miR.220a + miR.26a + miR.29a + miR.362 + miR.369.3p + miR.494 + miR.518c + miR.639 + miR.660 + miR.338 | 0,8 | 0,012465825 |
| miR.609 + miR.603 + miR.520f + miR.220a + miR.519b + miR.130a + miR.26a + miR.29a + miR.362 + miR.369.3p + miR.376a* + miR.639 + miR.660 + miR.338 | 0,8 | 0,372109999 |
| miR.558 + miR.609 + miR.603 + miR.520f + miR.220a + miR.519b + miR.130a + miR.29a + miR.362 + miR.494 + miR.518c + miR.639 + miR.660 + miR.338 | 0,8 | 0,014803783 |
| miR.558 + miR.609 + miR.603 + miR.520f + miR.220a + miR.519b + miR.130a + miR.29a + miR.362 + miR.369.3p + miR.494 + miR.518c + miR.660 + miR.338 | 0,8 | 0,019467446 |
| miR.558 + miR.609 + miR.603 + miR.520f + miR.220a + miR.519b + miR.130a + miR.26a + miR.29a + miR.369.3p + miR.494 + miR.518c + miR.660 + miR.338 | 0,8 | 0,023002139 |
| miR.558 + miR.609 + miR.603 + miR.520f + miR.220a + miR.519b + miR.26a + miR.29a + miR.362 + miR.369.3p + miR.518c + miR.639 + miR.660 + miR.338 | 0,8 | 0,019047463 |
| miR.558 + miR.609 + miR.603 + miR.520f + miR.220a + miR.519b + miR.130a + miR.362 + miR.369.3p + miR.376a* + miR.494 + miR.639 + miR.660 + miR.338 | 0,8 | 0,178460774 |
| miR.558 + miR.609 + miR.603 + miR.220a + miR.519b + miR.26a + miR.29a + miR.362 + miR.369.3p + miR.494 + miR.518c + miR.639 + miR.660 + miR.338 | 0,8 | 0,028736225 |
| miR.558 + miR.609 + miR.603 + miR.520f + miR.220a + miR.519b + miR.130a + miR.26a + miR.29a + miR.362 + miR.369.3p + miR.376a* + miR.494 + miR.338 | 0,8 | 0,035327934 |
| miR.558 + miR.609 + miR.603 + miR.520f + miR.220a + miR.519b + miR.130a + miR.29a + miR.369.3p + miR.494 + miR.518c + miR.639 + miR.660 + miR.338 | 0,8 | 0,014218463 |
| miR.558 + miR.609 + miR.603 + miR.520f + miR.220a + miR.519b + miR.130a + miR.26a + miR.29a + miR.362 + miR.369.3p + miR.494 + miR.518c + miR.660 | 0,8 | 0,022209399 |
| miR.558 + miR.609 + miR.603 + miR.520f + miR.220a + miR.519b + miR.26a + miR.29a + miR.362 + miR.369.3p + miR.494 + miR.518c + miR.639 + miR.660 | 0,8 | 0,010788415 |
| miR.558 + miR.609 + miR.603 + miR.220a + miR.519b + miR.130a + miR.29a + miR.362 + miR.369.3p + miR.494 + miR.518c + miR.639 + miR.660 + miR.338 | 0,8 | 0,018060186 |
| miR.558 + miR.609 + miR.603 + miR.520f + miR.220a + miR.519b + miR.130a + miR.26a + miR.29a + miR.494 + miR.518c + miR.639 + miR.660 + miR.338 | 0,8 | 0,019141951 |
| miR.558 + miR.609 + miR.603 + miR.520f + miR.220a + miR.519b + miR.130a + miR.26a + miR.29a + miR.362 + miR.494 + miR.518c + miR.660 + miR.338 | 0,8 | 0,024049426 |
| miR.558 + miR.609 + miR.603 + miR.520f + miR.220a + miR.519b + miR.130a + miR.26a + miR.29a + miR.362 + miR.494 + miR.518c + miR.639 + miR.660 | 0,8 | 0,01129977 |
| miR.558 + miR.609 + miR.603 + miR.520f + miR.220a + miR.130a + miR.29a + miR.362 + miR.369.3p + miR.494 + miR.518c + miR.639 + miR.660 + miR.338 | 0,8 | 0,004248511 |
| miR.558 + miR.609 + miR.603 + miR.520f + miR.220a + miR.519b + miR.130a + miR.26a + miR.29a + miR.376a* + miR.494 + miR.639 + miR.660 + miR.338 | 0,8 | 0,068004904 |
| miR.558 + miR.609 + miR.603 + miR.520f + miR.220a + miR.519b + miR.130a + miR.29a + miR.362 + miR.369.3p + miR.518c + miR.639 + miR.660 + miR.338 | 0,8 | 0,007728063 |
| miR.558 + miR.609 + miR.603 + miR.520f + miR.220a + miR.519b + miR.130a + miR.26a + miR.29a + miR.362 + miR.369.3p + miR.518c + miR.660 + miR.338 | 0,8 | 0,038155902 |
| miR.558 + miR.609 + miR.603 + miR.520f + miR.130a + miR.26a + miR.29a + miR.362 + miR.369.3p + miR.494 + miR.518c + miR.639 + miR.660 + miR.338 | 0,8 | 0,005150207 |
| miR.609 + miR.603 + miR.520f + miR.220a + miR.519b + miR.130a + miR.26a + miR.362 + miR.369.3p + miR.376a* + miR.494 + miR.518c + miR.639 + miR.338 | 0,8 | 0,01856064 |
| miR.558 + miR.609 + miR.603 + miR.520f + miR.220a + miR.519b + miR.130a + miR.26a + miR.29a + miR.369.3p + miR.494 + miR.518c + miR.639 + miR.660 | 0,8 | 0,01055317 |
| miR.558 + miR.609 + miR.603 + miR.520f + miR.220a + miR.519b + miR.26a + miR.29a + miR.362 + miR.369.3p + miR.494 + miR.518c + miR.660 + miR.338 | 0,8 | 0,039104023 |
| miR.558 + miR.609 + miR.603 + miR.520f + miR.519b + miR.130a + miR.26a + miR.29a + miR.362 + miR.369.3p + miR.494 + miR.518c + miR.639 + miR.660 | 0,8 | 0,005817217 |
| miR.558 + miR.609 + miR.603 + miR.520f + miR.220a + miR.519b + miR.130a + miR.26a + miR.29a + miR.369.3p + miR.518c + miR.639 + miR.660 + miR.338 | 0,8 | 0,021453956 |
| miR.558 + miR.609 + miR.520f + miR.220a + miR.519b + miR.130a + miR.26a + miR.362 + miR.369.3p + miR.376a* + miR.494 + miR.518c + miR.639 + miR.338 | 0,8 | 0,018697239 |
| miR.558 + miR.609 + miR.520f + miR.220a + miR.519b + miR.130a + miR.26a + miR.29a + miR.362 + miR.369.3p + miR.494 + miR.518c + miR.639 + miR.660 | 0,8 | 0,00494647 |
| miR.558 + miR.609 + miR.603 + miR.220a + miR.519b + miR.130a + miR.26a + miR.29a + miR.362 + miR.494 + miR.518c + miR.639 + miR.660 + miR.338 | 0,8 | 0,026107071 |
| miR.558 + miR.609 + miR.603 + miR.520f + miR.519b + miR.130a + miR.29a + miR.362 + miR.369.3p + miR.494 + miR.518c + miR.639 + miR.660 + miR.338 | 0,8 | 0,002805467 |
| miR.609 + miR.520f + miR.220a + miR.519b + miR.130a + miR.26a + miR.29a + miR.362 + miR.369.3p + miR.376a* + miR.494 + miR.518c + miR.639 + miR.338 | 0,8 | 0,03136434 |
| miR.558 + miR.609 + miR.603 + miR.520f + miR.220a + miR.130a + miR.26a + miR.29a + miR.362 + miR.369.3p + miR.494 + miR.518c + miR.660 + miR.338 | 0,8 | 0,028805568 |
| miR.558 + miR.609 + miR.603 + miR.220a + miR.519b + miR.130a + miR.26a + miR.29a + miR.362 + miR.369.3p + miR.376a* + miR.494 + miR.639 + miR.660 | 0,8 | 0,012489899 |
| miR.558 + miR.609 + miR.520f + miR.220a + miR.519b + miR.130a + miR.29a + miR.362 + miR.369.3p + miR.494 + miR.518c + miR.639 + miR.660 + miR.338 | 0,8 | 0,003124611 |
| miR.558 + miR.609 + miR.520f + miR.220a + miR.519b + miR.130a + miR.26a + miR.29a + miR.362 + miR.369.3p + miR.376a* + miR.494 + miR.639 + miR.338 | 0,8 | 0,025730939 |
| miR.558 + miR.609 + miR.603 + miR.520f + miR.220a + miR.130a + miR.26a + miR.29a + miR.369.3p + miR.494 + miR.518c + miR.639 + miR.660 + miR.338 | 0,8 | 0,009684082 |
| miR.558 + miR.609 + miR.603 + miR.520f + miR.220a + miR.130a + miR.26a + miR.29a + miR.362 + miR.369.3p + miR.376a* + miR.494 + miR.639 + miR.338 | 0,8 | 0,007678091 |
| miR.558 + miR.609 + miR.603 + miR.519b + miR.130a + miR.26a + miR.29a + miR.362 + miR.369.3p + miR.494 + miR.518c + miR.639 + miR.660 + miR.338 | 0,8 | 0,006249143 |
| miR.558 + miR.609 + miR.520f + miR.220a + miR.130a + miR.26a + miR.29a + miR.362 + miR.369.3p + miR.494 + miR.518c + miR.639 + miR.660 + miR.338 | 0,8 | 0,008091387 |
| miR.558 + miR.609 + miR.603 + miR.520f + miR.220a + miR.519b + miR.26a + miR.29a + miR.369.3p + miR.494 + miR.518c + miR.639 + miR.660 + miR.338 | 0,8 | 0,019583277 |
| miR.558 + miR.609 + miR.603 + miR.520f + miR.220a + miR.130a + miR.26a + miR.29a + miR.362 + miR.494 + miR.518c + miR.639 + miR.660 + miR.338 | 0,8 | 0,008544846 |
| miR.558 + miR.609 + miR.603 + miR.520f + miR.220a + miR.519b + miR.130a + miR.26a + miR.29a + miR.369.3p + miR.376a* + miR.639 + miR.660 + miR.338 | 0,8 | 0,050638514 |
| miR.558 + miR.609 + miR.603 + miR.520f + miR.220a + miR.519b + miR.26a + miR.29a + miR.362 + miR.494 + miR.518c + miR.639 + miR.660 + miR.338 | 0,8 | 0,019945097 |
| miR.558 + miR.609 + miR.603 + miR.220a + miR.519b + miR.130a + miR.26a + miR.362 +miR.376a* + miR.494 + miR.518c + miR.639 + miR.660 + miR.338 | 0,8 | 0,053220027 |
| miR.558 + miR.609 + miR.603 + miR.520f + miR.220a + miR.519b + miR.29a + miR.362 + miR.369.3p + miR.494 + miR.518c + miR.639 + miR.660 + miR.338 | 0,8 | 0,011098878 |
| miR.558 + miR.609 + miR.603 + miR.520f + miR.220a + miR.519b + miR.130a + miR.26a + miR.29a + miR.362 + miR.518c + miR.639 + miR.660 + miR.338 | 0,8 | 0,014815491 |
| miR.558 + miR.609 + miR.603 + miR.520f + miR.220a + miR.519b + miR.130a + miR.26a + miR.29a + miR.362 + miR.376a* + miR.494 + miR.639 + miR.338 | 0,8 | 0,0229879 |
| miR.558 + miR.609 + miR.603 + miR.520f + miR.519b + miR.130a + miR.26a + miR.29a + miR.362 + miR.369.3p + miR.518c + miR.639 + miR.660 + miR.338 | 0,8 | 0,005321563 |
| miR.558 + miR.609 + miR.520f + miR.220a + miR.519b + miR.130a + miR.26a + miR.29a + miR.362 + miR.369.3p + miR.518c + miR.639 + miR.660 + miR.338 | 0,8 | 0,006604092 |
| miR.558 + miR.609 + miR.220a + miR.519b + miR.130a + miR.26a + miR.29a + miR.362 + miR.369.3p + miR.494 + miR.518c + miR.639 + miR.660 + miR.338 | 0,8 | 0,010124967 |
| miR.558 + miR.609 + miR.520f + miR.519b + miR.130a + miR.26a + miR.29a + miR.362 + miR.369.3p + miR.494 + miR.518c + miR.639 + miR.660 + miR.338 | 0,8 | 0,003326403 |
| miR.558 + miR.609 + miR.520f + miR.220a + miR.519b + miR.26a + miR.29a + miR.362 + miR.369.3p + miR.494 + miR.518c + miR.639 + miR.660 + miR.338 | 0,8 | 0,008559942 |
| miR.558 + miR.609 + miR.520f + miR.220a + miR.519b + miR.130a + miR.26a + miR.29a + miR.362 + miR.494 + miR.518c + miR.639 + miR.660 + miR.338 | 0,8 | 0,003364812 |
| miR.558 + miR.609 + miR.603 + miR.520f + miR.519b + miR.130a + miR.26a + miR.29a + miR.362 + miR.494 + miR.518c + miR.639 + miR.660 + miR.338 | 0,8 | 0,00434136 |
| miR.609 + miR.603 + miR.520f + miR.220a + miR.519b + miR.130a + miR.26a + miR.362 + miR.376a* + miR.494 + miR.518c + miR.639 + miR.660 + miR.338 | 0,8 | 0,052467223 |
| miR.558 + miR.609 + miR.603 + miR.520f + miR.220a + miR.519b + miR.130a + miR.26a + miR.29a + miR.362 + miR.369.3p + miR.376a* + miR.639 + miR.660 | 0,8 | 0,020388689 |
| miR.558 + miR.609 + miR.520f + miR.220a + miR.519b + miR.130a + miR.26a + miR.29a + miR.362 + miR.369.3p + miR.494 + miR.518c + miR.660 + miR.338 | 0,8 | 0,01053064 |
| miR.558 + miR.609 + miR.603 + miR.520f + miR.519b + miR.26a + miR.29a + miR.362 + miR.369.3p + miR.494 + miR.518c + miR.639 + miR.660 + miR.338 | 0,8 | 0,005301543 |
| miR.558 + miR.609 + miR.603 + miR.520f + miR.519b + miR.130a + miR.26a + miR.29a + miR.369.3p + miR.494 + miR.518c + miR.639 + miR.660 + miR.338 | 0,8 | 0,007014815 |
| miR.558 + miR.609 + miR.520f + miR.220a + miR.519b + miR.130a + miR.26a + miR.29a + miR.369.3p + miR.494 + miR.518c + miR.639 + miR.660 + miR.338 | 0,8 | 0,009009583 |
| miR.558 + miR.609 + miR.603 + miR.520f + miR.220a + miR.519b + miR.130a + miR.26a + miR.29a + miR.362 + miR.369.3p + miR.376a* + miR.639 + miR.338 | 0,8 | 0,022578146 |
| miR.558 + miR.609 + miR.603 + miR.520f + miR.220a + miR.519b + miR.130a + miR.26a + miR.29a + miR.362 + miR.376a* + miR.494 + miR.639 + miR.660 | 0,8 | 0,003876995 |
| miR.609 + miR.603 + miR.520f + miR.220a + miR.519b + miR.130a + miR.26a + miR.362 + miR.369.3p + miR.376a* + miR.494 + miR.639 + miR.660 + miR.338 | 0,8 | 0,176166811 |
| miR.558 + miR.609 + miR.603 + miR.520f + miR.519b + miR.130a + miR.26a + miR.29a + miR.362 + miR.369.3p + miR.494 + miR.518c + miR.660 + miR.338 | 0,8 | 0,011991996 |
| miR.558 + miR.609 + miR.603 + miR.520f + miR.220a + miR.519b + miR.130a + miR.26a + miR.362 + miR.369.3p + miR.376a* + miR.494 + miR.660 + miR.338 | 0,8 | 0,233348404 |
| miR.558 + miR.609 + miR.603 + miR.520f + miR.220a + miR.519b + miR.130a + miR.26a + miR.29a + miR.369.3p + miR.376a* + miR.494 + miR.639 + miR.660 | 0,8 | 0,009690995 |
| miR.558 + miR.609 + miR.603 + miR.220a + miR.519b + miR.130a + miR.26a + miR.29a + miR.362 + miR.369.3p + miR.376a* + miR.639 + miR.660 + miR.338 | 0,8 | 0,048559007 |
| miR.558 + miR.609 + miR.603 + miR.520f + miR.220a + miR.519b + miR.130a + miR.26a + miR.29a + miR.362 + miR.369.3p + miR.494 + miR.518c + miR.338 | 0,79 | 0,032484662 |
| miR.558 + miR.609 + miR.603 + miR.220a + miR.519b + miR.130a + miR.26a + miR.29a + miR.362 + miR.369.3p + miR.494 + miR.518c + miR.639 + miR.338 | 0,79 | 0,033583739 |
| miR.558 + miR.609 + miR.603 + miR.520f + miR.220a + miR.519b + miR.130a + miR.26a + miR.29a + miR.362 + miR.494 + miR.518c + miR.639 + miR.338 | 0,79 | 0,031807754 |
| miR.558 + miR.609 + miR.603 + miR.520f + miR.220a + miR.519b + miR.130a + miR.26a + miR.29a + miR.362 + miR.369.3p + miR.376a* + miR.494 + miR.639 | 0,79 | 0,006085582 |
| miR.558 + miR.609 + miR.603 + miR.520f + miR.220a + miR.519b + miR.130a + miR.26a + miR.29a + miR.362 + miR.376a* + miR.639 + miR.660 + miR.338 | 0,79 | 0,029731462 |
| miR.558 + miR.609 + miR.603 + miR.220a + miR.519b + miR.130a + miR.26a + miR.29a + miR.362 + miR.369.3p + miR.376a* + miR.494 + miR.639 + miR.338 | 0,79 | 0,012041757 |
| miR.609 + miR.603 + miR.520f + miR.220a + miR.519b + miR.130a + miR.26a + miR.29a + miR.362 + miR.369.3p + miR.376a* + miR.494 + miR.639 + miR.660 | 0,79 | 0,021500963 |
| miR.558 + miR.609 + miR.603 + miR.520f + miR.220a + miR.519b + miR.130a + miR.26a + miR.29a + miR.362 + miR.369.3p + miR.518c + miR.639 + miR.338 | 0,79 | 0,027971802 |
| miR.558 + miR.609 + miR.603 + miR.520f + miR.220a + miR.519b + miR.130a + miR.26a + miR.369.3p + miR.376a* + miR.494 + miR.639 + miR.660 + miR.338 | 0,79 | 0,053000691 |
| miR.558 + miR.609 + miR.603 + miR.520f + miR.220a + miR.519b + miR.130a + miR.29a + miR.362 + miR.369.3p + miR.494 + miR.518c + miR.639 + miR.338 | 0,79 | 0,047142301 |
| miR.609 + miR.603 + miR.220a + miR.519b + miR.130a + miR.26a + miR.29a + miR.362 + miR.369.3p + miR.376a* + miR.494 + miR.639 + miR.660 + miR.338 | 0,79 | 0,108525289 |
| miR.609 + miR.603 + miR.520f + miR.220a + miR.519b + miR.130a + miR.26a + miR.29a + miR.369.3p + miR.376a* + miR.494 + miR.639 + miR.660 + miR.338 | 0,79 | 0,060410062 |
| miR.609 + miR.603 + miR.520f + miR.220a + miR.519b + miR.130a + miR.26a + miR.29a + miR.362 + miR.376a* + miR.494 + miR.639 + miR.660 + miR.338 | 0,79 | 0,024830622 |
| miR.558 + miR.609 + miR.603 + miR.220a + miR.519b + miR.130a + miR.26a + miR.29a + miR.369.3p + miR.376a* + miR.494 + miR.639 + miR.660 + miR.338 | 0,79 | 0,003332699 |
| miR.558 + miR.609 + miR.603 + miR.520f + miR.220a + miR.519b + miR.26a + miR.29a + miR.362 + miR.369.3p + miR.494 + miR.518c + miR.639 + miR.338 | 0,79 | 0,022325889 |
| miR.558 + miR.609 + miR.603 + miR.520f + miR.220a + miR.519b + miR.130a + miR.26a + miR.29a + miR.369.3p + miR.494 + miR.518c + miR.639 + miR.338 | 0,79 | 0,032533787 |
| miR.558 + miR.609 + miR.603 + miR.520f + miR.220a + miR.519b + miR.130a + miR.26a + miR.362 + miR.376a* + miR.494 + miR.639 + miR.660 + miR.338 | 0,79 | 0,056348072 |
| miR.558 + miR.609 + miR.603 + miR.520f + miR.220a + miR.130a + miR.26a + miR.29a + miR.362 + miR.369.3p + miR.494 + miR.518c + miR.639 + miR.338 | 0,79 | 0,012745843 |
| miR.558 + miR.609 + miR.520f + miR.220a + miR.519b + miR.130a + miR.26a + miR.29a + miR.362 + miR.369.3p + miR.494 + miR.518c + miR.639 + miR.338 | 0,79 | 0,021305139 |
| miR.558 + miR.609 + miR.603 + miR.520f + miR.220a + miR.519b + miR.130a + miR.26a + miR.29a + miR.362 + miR.369.3p + miR.494 + miR.518c + miR.639 | 0,79 | 0,025363655 |
| miR.558 + miR.609 + miR.603 + miR.520f + miR.220a + miR.519b + miR.130a + miR.26a + miR.362 + miR.369.3p + miR.376a* + miR.494 + miR.639 + miR.338 | 0,79 | 0,008398289 |
| miR.558 + miR.609 + miR.603 + miR.520f + miR.220a + miR.519b + miR.130a + miR.26a + miR.362 + miR.369.3p + miR.376a* + miR.494 + miR.639 + miR.660 | 0,79 | 0,023442674 |
| miR.558 + miR.609 + miR.603 + miR.520f + miR.220a + miR.130a + miR.26a + miR.362 + miR.369.3p + miR.494 + miR.518c + miR.639 + miR.660 + miR.338 | 0,79 | 0,004306052 |
| miR.558 + miR.609 + miR.603 + miR.520f + miR.220a + miR.519b + miR.130a + miR.26a + miR.362 + miR.369.3p + miR.494 + miR.518c + miR.639 + miR.660 | 0,79 | 0,003934717 |
| miR.609 + miR.603 + miR.520f + miR.220a + miR.519b + miR.130a + miR.26a + miR.29a + miR.362 + miR.369.3p + miR.376a* + miR.494 + miR.639 + miR.338 | 0,79 | 0,03639979 |
| miR.558 + miR.609 + miR.603 + miR.220a + miR.519b + miR.130a + miR.26a + miR.362 + miR.369.3p + miR.376a* + miR.494 + miR.639 + miR.660 + miR.338 | 0,79 | 0,061479947 |
| miR.558 + miR.609 + miR.603 + miR.520f + miR.519b + miR.130a + miR.26a + miR.29a + miR.362 + miR.369.3p + miR.494 + miR.518c + miR.639 + miR.338 | 0,79 | 0,023303609 |
| miR.558 + miR.609 + miR.603 + miR.520f + miR.220a + miR.519b + miR.130a + miR.26a + miR.362 + miR.369.3p + miR.518c + miR.639 + miR.660 + miR.338 | 0,79 | 0,005058528 |
| miR.558 + miR.609 + miR.603 + miR.520f + miR.220a + miR.519b + miR.130a + miR.362 + miR.369.3p + miR.494 + miR.518c + miR.639 + miR.660 + miR.338 | 0,79 | 0,005366844 |
| miR.558 + miR.609 + miR.603 + miR.520f + miR.220a + miR.519b + miR.26a + miR.362 + miR.369.3p + miR.494 + miR.518c + miR.639 + miR.660 + miR.338 | 0,79 | 0,005184261 |
| miR.558 + miR.609 + miR.603 + miR.220a + miR.519b + miR.130a + miR.26a + miR.362 + miR.369.3p + miR.494 + miR.518c + miR.639 + miR.660 + miR.338 | 0,79 | 0,00768243 |
| miR.609 + miR.603 + miR.520f + miR.220a + miR.519b + miR.26a + miR.29a + miR.362 + miR.369.3p + miR.494 + miR.518c + miR.639 + miR.660 + miR.338 | 0,79 | 0,007849931 |
| miR.609 + miR.603 + miR.520f + miR.220a + miR.519b + miR.130a + miR.29a + miR.362 + miR.369.3p + miR.494 + miR.518c + miR.639 + miR.660 + miR.338 | 0,79 | 0,00790819 |
| miR.609 + miR.603 + miR.520f + miR.220a + miR.130a + miR.26a + miR.29a + miR.362 + miR.369.3p + miR.494 + miR.518c + miR.639 + miR.660 + miR.338 | 0,79 | 0,005320446 |
| miR.609 + miR.603 + miR.520f + miR.220a + miR.519b + miR.130a + miR.26a + miR.29a + miR.362 + miR.369.3p + miR.494 + miR.518c + miR.639 + miR.660 | 0,79 | 0,004224297 |
| miR.558 + miR.609 + miR.603 + miR.520f + miR.220a + miR.519b + miR.130a + miR.26a + miR.362 + miR.494 + miR.518c + miR.639 + miR.660 + miR.338 | 0,79 | 0,005290236 |
| miR.609 + miR.603 + miR.520f + miR.220a + miR.519b + miR.130a + miR.26a + miR.29a + miR.362 + miR.369.3p + miR.494 + miR.518c + miR.660 + miR.338 | 0,79 | 0,006989892 |
| miR.558 + miR.609 + miR.603 + miR.520f + miR.220a + miR.519b + miR.130a + miR.26a + miR.369.3p + miR.494 + miR.518c + miR.639 + miR.660 + miR.338 | 0,79 | 0,007191019 |
| miR.558 + miR.603 + miR.220a + miR.519b + miR.130a + miR.26a + miR.29a + miR.362 + miR.369.3p + miR.376a* + miR.494 + miR.518c + miR.639 + miR.660 | 0,79 | 0,051643591 |
| miR.558 + miR.609 + miR.603 + miR.220a + miR.519b + miR.130a + miR.26a + miR.29a + miR.362 + miR.376a* + miR.494 + miR.639 + miR.660 + miR.338 | 0,79 | 0,000961238 |
| miR.558 + miR.603 + miR.220a + miR.519b + miR.130a + miR.26a + miR.29a + miR.362 + miR.369.3p + miR.376a* + miR.518c + miR.639 + miR.660 + miR.338 | 0,79 | 0,065690861 |
| miR.558 + miR.603 + miR.520f + miR.220a + miR.519b + miR.130a + miR.29a + miR.362 + miR.369.3p + miR.376a* + miR.518c + miR.639 + miR.660 + miR.338 | 0,79 | 0,036512774 |
| miR.558 + miR.603 + miR.520f + miR.220a + miR.519b + miR.130a + miR.26a + miR.29a + miR.362 + miR.369.3p + miR.376a* + miR.518c + miR.639 + miR.660 | 0,79 | 0,042057641 |
| miR.558 + miR.603 + miR.520f + miR.220a + miR.519b + miR.26a + miR.29a + miR.362 + miR.369.3p + miR.376a* + miR.494 + miR.518c + miR.639 + miR.660 | 0,79 | 0,044866686 |
| miR.558 + miR.603 + miR.520f + miR.220a + miR.519b + miR.130a + miR.29a + miR.362 + miR.369.3p + miR.376a* + miR.494 + miR.518c + miR.639 + miR.660 | 0,79 | 0,03365196 |
| miR.609 + miR.603 + miR.220a + miR.519b + miR.130a + miR.26a + miR.29a + miR.362 + miR.369.3p + miR.494 + miR.518c + miR.639 + miR.660 + miR.338 | 0,79 | 0,005141548 |
| miR.558 + miR.609 + miR.603 + miR.520f + miR.519b + miR.130a + miR.26a + miR.362 + miR.369.3p + miR.494 + miR.518c + miR.639 + miR.660 + miR.338 | 0,79 | 0,00343947 |
| miR.558 + miR.603 + miR.520f + miR.220a + miR.519b + miR.29a + miR.362 + miR.369.3p + miR.376a* + miR.494 + miR.518c + miR.639 + miR.660 + miR.338 | 0,79 | 0,033810602 |
| miR.609 + miR.603 + miR.520f + miR.220a + miR.519b + miR.130a + miR.26a + miR.29a + miR.362 + miR.494 + miR.518c + miR.639 + miR.660 + miR.338 | 0,79 | 0,004106011 |
| miR.558 + miR.603 + miR.520f + miR.220a + miR.519b + miR.130a + miR.26a + miR.29a + miR.362 + miR.369.3p + miR.376a* + miR.494 + miR.518c + miR.639 | 0,79 | 0,040317621 |
| miR.609 + miR.603 + miR.520f + miR.220a + miR.519b + miR.130a + miR.26a + miR.29a + miR.362 + miR.369.3p + miR.518c + miR.639 + miR.660 + miR.338 | 0,79 | 0,007217829 |
| miR.558 + miR.603 + miR.520f + miR.220a + miR.519b + miR.130a + miR.26a + miR.29a + miR.362 + miR.369.3p + miR.376a* + miR.518c + miR.639 + miR.338 | 0,79 | 0,053493608 |
| miR.558 + miR.603 + miR.220a + miR.519b + miR.130a + miR.26a + miR.29a + miR.362 + miR.369.3p + miR.376a* + miR.494 + miR.518c + miR.639 + miR.338 | 0,79 | 0,046238119 |
| miR.558 + miR.603 + miR.520f + miR.220a + miR.519b + miR.130a + miR.29a + miR.362 + miR.369.3p + miR.376a* + miR.494 + miR.518c + miR.639 + miR.338 | 0,79 | 0,02486941 |
| miR.558 + miR.603 + miR.520f + miR.220a + miR.519b + miR.26a + miR.29a + miR.362 + miR.369.3p + miR.376a* + miR.518c + miR.639 + miR.660 + miR.338 | 0,79 | 0,063449632 |
| miR.609 + miR.603 + miR.520f + miR.220a + miR.519b + miR.130a + miR.26a + miR.29a + miR.369.3p + miR.494 + miR.518c + miR.639 + miR.660 + miR.338 | 0,79 | 0,002560894 |
| miR.558 + miR.603 + miR.520f + miR.220a + miR.519b + miR.130a + miR.26a + miR.29a + miR.362 + miR.369.3p + miR.376a* + miR.494 + miR.518c + miR.338 | 0,79 | 0,045666719 |
| miR.558 + miR.603 + miR.520f + miR.220a + miR.519b + miR.130a + miR.26a + miR.29a + miR.362 + miR.369.3p + miR.376a* + miR.494 + miR.518c + miR.660 | 0,79 | 0,045427381 |
| miR.558 + miR.603 + miR.220a + miR.519b + miR.26a + miR.29a + miR.362 + miR.369.3p + miR.376a* + miR.494 + miR.518c + miR.639 + miR.660 + miR.338 | 0,79 | 0,068865951 |
| miR.558 + miR.603 + miR.220a + miR.519b + miR.130a + miR.26a + miR.29a + miR.362 + miR.369.3p + miR.376a* + miR.494 + miR.518c + miR.660 + miR.338 | 0,79 | 0,04441265 |
| miR.558 + miR.603 + miR.520f + miR.220a + miR.519b + miR.26a + miR.29a + miR.362 + miR.369.3p + miR.376a* + miR.494 + miR.518c + miR.660 + miR.338 | 0,79 | 0,06287616 |
| miR.558 + miR.603 + miR.520f + miR.220a + miR.519b + miR.130a + miR.29a + miR.369.3p + miR.376a* + miR.494 + miR.518c + miR.639 + miR.660 + miR.338 | 0,79 | 0,026585437 |
| miR.558 + miR.609 + miR.520f + miR.220a + miR.519b + miR.130a + miR.26a + miR.362 + miR.369.3p + miR.494 + miR.518c + miR.639 + miR.660 + miR.338 | 0,79 | 0,004117076 |
| miR.558 + miR.603 + miR.520f + miR.220a + miR.519b + miR.130a + miR.26a + miR.29a + miR.362 + miR.369.3p + miR.376a* + miR.518c + miR.660 + miR.338 | 0,79 | 0,053326778 |
| miR.558 + miR.603 + miR.220a + miR.519b + miR.130a + miR.29a + miR.362 + miR.369.3p + miR.376a* + miR.494 + miR.518c + miR.639 + miR.660 + miR.338 | 0,79 | 0,039165413 |
| miR.558 + miR.603 + miR.520f + miR.220a + miR.519b + miR.130a + miR.26a + miR.29a + miR.369.3p + miR.376a* + miR.494 + miR.518c + miR.639 + miR.660 | 0,79 | 0,037618768 |
| miR.558 + miR.603 + miR.520f + miR.220a + miR.519b + miR.26a + miR.29a + miR.362 + miR.369.3p + miR.376a* + miR.494 + miR.518c + miR.639 + miR.338 | 0,79 | 0,047400005 |
| miR.558 + miR.609 + miR.603 + miR.520f + miR.220a + miR.519b + miR.130a + miR.26a + miR.362 + miR.369.3p + miR.494 + miR.518c + miR.660 + miR.338 | 0,79 | 0,013092072 |
| miR.609 + miR.520f + miR.220a + miR.519b + miR.130a + miR.26a + miR.29a + miR.362 + miR.369.3p + miR.494 + miR.518c + miR.639 + miR.660 + miR.338 | 0,79 | 0,000581235 |
| miR.558 + miR.603 + miR.520f + miR.220a + miR.519b + miR.130a + miR.26a + miR.29a + miR.369.3p + miR.376a* + miR.518c + miR.639 + miR.660 + miR.338 | 0,79 | 0,034082818 |
| miR.558 + miR.603 + miR.520f + miR.220a + miR.519b + miR.130a + miR.29a + miR.362 + miR.369.3p + miR.376a* + miR.494 + miR.518c + miR.660 + miR.338 | 0,79 | 0,030151994 |
| miR.558 + miR.603 + miR.520f + miR.220a + miR.519b + miR.26a + miR.29a + miR.369.3p + miR.376a* + miR.494 + miR.518c + miR.639 + miR.660 + miR.338 | 0,79 | 0,037594907 |
| miR.558 + miR.603 + miR.520f + miR.220a + miR.519b + miR.130a + miR.26a + miR.29a + miR.369.3p + miR.376a* + miR.494 + miR.518c + miR.639 + miR.338 | 0,79 | 0,035674694 |
| miR.558 + miR.603 + miR.520f + miR.220a + miR.519b + miR.130a + miR.26a + miR.362 + miR.369.3p + miR.376a* + miR.494 + miR.518c + miR.639 + miR.660 | 0,79 | 0,03884114 |
| miR.558 + miR.603 + miR.220a + miR.519b + miR.130a + miR.26a + miR.29a + miR.369.3p + miR.376a* + miR.494 + miR.518c + miR.639 + miR.660 + miR.338 | 0,79 | 0,037521581 |
| miR.558 + miR.603 + miR.520f + miR.220a + miR.519b + miR.130a + miR.26a + miR.29a + miR.369.3p + miR.376a* + miR.494 + miR.518c + miR.660 + miR.338 | 0,79 | 0,04121026 |
| miR.558 + miR.603 + miR.520f + miR.220a + miR.519b + miR.130a + miR.26a + miR.362 + miR.369.3p + miR.376a* + miR.518c + miR.639 + miR.660 + miR.338 | 0,79 | 0,046840855 |
| miR.558 + miR.603 + miR.520f + miR.220a + miR.519b + miR.26a + miR.362 + miR.369.3p + miR.376a* + miR.494 + miR.518c + miR.639 + miR.660 + miR.338 | 0,79 | 0,045365571 |
| miR.609 + miR.603 + miR.520f + miR.519b + miR.130a + miR.26a + miR.29a + miR.362 + miR.369.3p + miR.494 + miR.518c + miR.639 + miR.660 + miR.338 | 0,79 | 0,002045457 |
| miR.558 + miR.603 + miR.520f + miR.220a + miR.519b + miR.130a + miR.362 + miR.369.3p + miR.376a* + miR.494 + miR.518c + miR.639 + miR.660 + miR.338 | 0,79 | 0,035765779 |
| miR.558 + miR.603 + miR.220a + miR.519b + miR.130a + miR.26a + miR.362 + miR.369.3p + miR.376a* + miR.494 + miR.518c + miR.639 + miR.660 + miR.338 | 0,79 | 0,052336529 |
| miR.558 + miR.603 + miR.520f + miR.220a + miR.519b + miR.130a + miR.26a + miR.362 + miR.369.3p + miR.376a* + miR.494 + miR.518c + miR.639 + miR.338 | 0,79 | 0,036657315 |
| miR.558 + miR.603 + miR.220a + miR.130a + miR.26a + miR.29a + miR.362 + miR.369.3p + miR.376a* + miR.494 + miR.518c + miR.639 + miR.660 + miR.338 | 0,79 | 0,030590384 |
| miR.558 + miR.609 + miR.603 + miR.520f + miR.220a + miR.519b + miR.130a + miR.26a + miR.362 + miR.369.3p + miR.494 + miR.518c + miR.639 + miR.338 | 0,79 | 0,006176138 |
| miR.558 + miR.603 + miR.520f + miR.220a + miR.130a + miR.26a + miR.29a + miR.362 + miR.369.3p + miR.376a* + miR.494 + miR.518c + miR.639 + miR.660 | 0,78 | 0,030580245 |
| miR.558 + miR.603 + miR.520f + miR.220a + miR.26a + miR.29a + miR.362 + miR.369.3p + miR.376a* + miR.494 + miR.518c + miR.639 + miR.660 + miR.338 | 0,78 | 0,029932015 |
| miR.558 + miR.603 + miR.520f + miR.220a + miR.519b + miR.130a + miR.26a + miR.362 + miR.369.3p + miR.376a* + miR.494 + miR.518c + miR.660 + miR.338 | 0,78 | 0,038297104 |
| miR.558 + miR.603 + miR.520f + miR.220a + miR.519b + miR.130a + miR.26a + miR.369.3p + miR.376a* + miR.494 + miR.518c + miR.639 + miR.660 + miR.338 | 0,78 | 0,035548638 |
| miR.558 + miR.603 + miR.520f + miR.220a + miR.130a + miR.26a + miR.362 + miR.369.3p + miR.376a* + miR.494 + miR.518c + miR.639 + miR.660 + miR.338 | 0,78 | 0,021213589 |
| miR.558 + miR.603 + miR.520f + miR.220a + miR.130a + miR.26a + miR.29a + miR.362 + miR.369.3p + miR.376a* + miR.518c + miR.639 + miR.660 + miR.338 | 0,78 | 0,025821406 |
| miR.558 + miR.603 + miR.520f + miR.220a + miR.130a + miR.26a + miR.29a + miR.362 + miR.369.3p + miR.376a* + miR.494 + miR.518c + miR.639 + miR.338 | 0,78 | 0,011696159 |
| miR.558 + miR.603 + miR.520f + miR.220a + miR.130a + miR.29a + miR.362 + miR.369.3p + miR.376a* + miR.494 + miR.518c + miR.639 + miR.660 + miR.338 | 0,78 | 0,018113387 |
| miR.558 + miR.603 + miR.520f + miR.220a + miR.519b + miR.130a + miR.29a + miR.362 + miR.376a* + miR.494 + miR.518c + miR.639 + miR.660 + miR.338 | 0,78 | 0,015425693 |
| miR.558 + miR.603 + miR.220a + miR.519b + miR.130a + miR.26a + miR.29a + miR.362 + miR.376a* + miR.494 + miR.518c + miR.639 + miR.660 + miR.338 | 0,78 | 0,023097884 |
| miR.558 + miR.603 + miR.520f + miR.220a + miR.519b + miR.130a + miR.26a + miR.29a + miR.362 + miR.376a* + miR.494 + miR.518c + miR.639 + miR.660 | 0,78 | 0,024569319 |
| miR.558 + miR.603 + miR.520f + miR.220a + miR.130a + miR.26a + miR.29a + miR.369.3p + miR.376a* + miR.494 + miR.518c + miR.639 + miR.660 + miR.338 | 0,78 | 0,008888786 |
| miR.558 + miR.603 + miR.520f + miR.220a + miR.519b + miR.130a + miR.26a + miR.29a + miR.362 + miR.376a* + miR.494 + miR.518c + miR.660 + miR.338 | 0,78 | 0,020894465 |
| miR.558 + miR.603 + miR.520f + miR.220a + miR.519b + miR.130a + miR.26a + miR.29a + miR.362 + miR.376a* + miR.494 + miR.518c + miR.639 + miR.338 | 0,78 | 0,013299731 |
| miR.558 + miR.603 + miR.520f + miR.220a + miR.519b + miR.130a + miR.26a + miR.29a + miR.376a* + miR.494 + miR.518c + miR.639 + miR.660 + miR.338 | 0,78 | 0,013858012 |
| miR.558 + miR.603 + miR.520f + miR.220a + miR.519b + miR.130a + miR.26a + miR.29a + miR.362 + miR.376a* + miR.518c + miR.639 + miR.660 + miR.338 | 0,78 | 0,017900737 |
| miR.558 + miR.603 + miR.520f + miR.220a + miR.519b + miR.26a + miR.29a + miR.362 + miR.376a* + miR.494 + miR.518c + miR.639 + miR.660 + miR.338 | 0,78 | 0,017263067 |
| miR.609 + miR.603 + miR.520f + miR.220a + miR.519b + miR.130a + miR.26a + miR.362 + miR.369.3p + miR.494 + miR.518c + miR.639 + miR.660 + miR.338 | 0,78 | 0,005967248 |
| miR.558 + miR.603 + miR.520f + miR.519b + miR.130a + miR.26a + miR.29a + miR.362 + miR.369.3p + miR.376a* + miR.518c + miR.639 + miR.660 + miR.338 | 0,78 | 0,023698113 |
| miR.558 + miR.603 + miR.520f + miR.519b + miR.130a + miR.29a + miR.362 + miR.369.3p + miR.376a* + miR.494 + miR.518c + miR.639 + miR.660 + miR.338 | 0,78 | 0,021111105 |
| miR.558 + miR.603 + miR.520f + miR.220a + miR.130a + miR.26a + miR.29a + miR.362 + miR.369.3p + miR.376a* + miR.494 + miR.518c + miR.660 + miR.338 | 0,78 | 0,016725226 |
| miR.558 + miR.603 + miR.520f + miR.519b + miR.26a + miR.29a + miR.362 + miR.369.3p + miR.376a* + miR.494 + miR.518c + miR.639 + miR.660 + miR.338 | 0,78 | 0,019684948 |
| miR.558 + miR.603 + miR.520f + miR.519b + miR.130a + miR.26a + miR.29a + miR.362 + miR.369.3p + miR.376a* + miR.494 + miR.518c + miR.639 + miR.338 | 0,78 | 0,018456147 |
| miR.558 + miR.609 + miR.603 + miR.520f + miR.519b + miR.130a + miR.26a + miR.29a + miR.362 + miR.369.3p + miR.494 + miR.639 + miR.660 + miR.338 | 0,78 | 0,006056795 |
| miR.558 + miR.609 + miR.603 + miR.520f + miR.220a + miR.519b + miR.130a + miR.29a + miR.362 + miR.369.3p + miR.494 + miR.639 + miR.660 + miR.338 | 0,78 | 0,004485767 |
| miR.609 + miR.603 + miR.520f + miR.220a + miR.519b + miR.130a + miR.26a + miR.29a + miR.362 + miR.369.3p + miR.494 + miR.518c + miR.639 + miR.338 | 0,78 | 0,003432279 |
| miR.558 + miR.609 + miR.520f + miR.220a + miR.519b + miR.130a + miR.26a + miR.29a + miR.362 + miR.369.3p + miR.494 + miR.639 + miR.660 + miR.338 | 0,78 | 0,003576897 |
| miR.558 + miR.603 + miR.520f + miR.220a + miR.519b + miR.130a + miR.26a + miR.362 + miR.376a* + miR.494 + miR.518c + miR.639 + miR.660 + miR.338 | 0,78 | 0,01023259 |
| miR.558 + miR.603 + miR.520f + miR.519b + miR.130a + miR.26a + miR.362 + miR.369.3p + miR.376a* + miR.494 + miR.518c + miR.639 + miR.660 + miR.338 | 0,78 | 0,013120117 |
| miR.558 + miR.603 + miR.519b + miR.130a + miR.26a + miR.29a + miR.362 + miR.369.3p + miR.376a* + miR.494 + miR.518c + miR.639 + miR.660 + miR.338 | 0,78 | 0,039952459 |
| miR.558 + miR.609 + miR.603 + miR.520f + miR.220a + miR.130a + miR.26a + miR.29a + miR.362 + miR.369.3p + miR.494 + miR.639 + miR.660 + miR.338 | 0,78 | 0,0032938 |
| miR.558 + miR.603 + miR.520f + miR.130a + miR.26a + miR.29a + miR.362 + miR.369.3p + miR.376a* + miR.494 + miR.518c + miR.639 + miR.660 + miR.338 | 0,78 | 0,009754549 |
| miR.558 + miR.609 + miR.603 + miR.520f + miR.220a + miR.519b + miR.130a + miR.26a + miR.29a + miR.362 + miR.369.3p + miR.494 + miR.639 + miR.660 | 0,78 | 0,005980976 |
| miR.558 + miR.609 + miR.603 + miR.520f + miR.220a + miR.519b + miR.130a + miR.26a + miR.29a + miR.362 + miR.494 + miR.639 + miR.660 + miR.338 | 0,78 | 0,00562775 |
| miR.558 + miR.603 + miR.520f + miR.519b + miR.130a + miR.26a + miR.29a + miR.362 + miR.369.3p + miR.376a* + miR.494 + miR.518c + miR.639 + miR.660 | 0,78 | 0,022485569 |
| miR.603 + miR.520f + miR.220a + miR.519b + miR.26a + miR.29a + miR.362 + miR.369.3p + miR.376a* + miR.494 + miR.518c + miR.639 + miR.660 + miR.338 | 0,78 | 0,015872651 |
| miR.558 + miR.609 + miR.603 + miR.520f + miR.220a + miR.519b + miR.26a + miR.29a + miR.362 + miR.369.3p + miR.494 + miR.639 + miR.660 + miR.338 | 0,78 | 0,00451448 |
| miR.603 + miR.520f + miR.220a + miR.519b + miR.130a + miR.26a + miR.29a + miR.362 + miR.369.3p + miR.376a* + miR.518c + miR.639 + miR.660 + miR.338 | 0,78 | 0,01868585 |
| miR.603 + miR.520f + miR.220a + miR.519b + miR.130a + miR.29a + miR.362 + miR.369.3p + miR.376a* + miR.494 + miR.518c + miR.639 + miR.660 + miR.338 | 0,78 | 0,014360381 |
| miR.603 + miR.520f + miR.220a + miR.519b + miR.130a + miR.26a + miR.29a + miR.362 + miR.369.3p + miR.376a* + miR.494 + miR.518c + miR.639 + miR.660 | 0,78 | 0,013393427 |
| miR.603 + miR.220a + miR.519b + miR.130a + miR.26a + miR.29a + miR.362 + miR.369.3p + miR.376a* + miR.494 + miR.518c + miR.639 + miR.660 + miR.338 | 0,78 | 0,019659735 |
| miR.603 + miR.520f + miR.220a + miR.519b + miR.130a + miR.26a + miR.362 + miR.369.3p + miR.376a* + miR.494 + miR.518c + miR.639 + miR.660 + miR.338 | 0,78 | 0,017653305 |
| miR.558 + miR.603 + miR.220a + miR.519b + miR.130a + miR.26a + miR.29a + miR.362 + miR.369.3p + miR.494 + miR.518c + miR.639 + miR.660 + miR.338 | 0,78 | 0,027608733 |
| miR.558 + miR.609 + miR.603 + miR.520f + miR.220a + miR.519b + miR.130a + miR.26a + miR.29a + miR.362 + miR.369.3p + miR.494 + miR.660 + miR.338 | 0,78 | 0,006852495 |
| miR.558 + miR.603 + miR.520f + miR.220a + miR.519b + miR.130a + miR.26a + miR.29a + miR.362 + miR.369.3p + miR.494 + miR.518c + miR.660 + miR.338 | 0,78 | 0,025823419 |
| miR.558 + miR.603 + miR.520f + miR.519b + miR.130a + miR.26a + miR.29a + miR.369.3p + miR.376a* + miR.494 + miR.518c + miR.639 + miR.660 + miR.338 | 0,78 | 0,015729623 |
| miR.558 + miR.603 + miR.520f + miR.519b + miR.130a + miR.26a + miR.29a + miR.362 + miR.369.3p + miR.376a* + miR.494 + miR.518c + miR.660 + miR.338 | 0,78 | 0,03289423 |
| miR.558 + miR.603 + miR.520f + miR.220a + miR.130a + miR.26a + miR.29a + miR.362 + miR.376a* + miR.494 + miR.518c + miR.639 + miR.660 + miR.338 | 0,78 | 0,001930455 |
| miR.603 + miR.520f + miR.220a + miR.519b + miR.130a + miR.26a + miR.29a + miR.362 + miR.369.3p + miR.376a* + miR.494 + miR.518c + miR.639 + miR.338 | 0,78 | 0,00955262 |
| miR.603 + miR.520f + miR.220a + miR.130a + miR.26a + miR.29a + miR.362 + miR.369.3p + miR.376a* + miR.494 + miR.518c + miR.639 + miR.660 + miR.338 | 0,78 | 0,011944047 |
| miR.558 + miR.603 + miR.520f + miR.220a + miR.519b + miR.130a + miR.26a + miR.29a + miR.369.3p + miR.494 + miR.518c + miR.639 + miR.660 + miR.338 | 0,78 | 0,019952903 |
| miR.558 + miR.603 + miR.520f + miR.220a + miR.519b + miR.130a + miR.26a + miR.29a + miR.362 + miR.369.3p + miR.494 + miR.518c + miR.639 + miR.338 | 0,78 | 0,030585667 |
| miR.603 + miR.520f + miR.220a + miR.519b + miR.130a + miR.26a + miR.29a + miR.362 + miR.369.3p + miR.376a* + miR.494 + miR.518c + miR.660 + miR.338 | 0,78 | 0,02036552 |
| miR.558 + miR.603 + miR.520f + miR.220a + miR.519b + miR.130a + miR.26a + miR.29a + miR.362 + miR.369.3p + miR.518c + miR.639 + miR.660 + miR.338 | 0,78 | 0,022737674 |
| miR.558 + miR.609 + miR.603 + miR.520f + miR.220a + miR.519b + miR.130a + miR.26a + miR.29a + miR.362 + miR.369.3p + miR.639 + miR.660 + miR.338 | 0,78 | 0,002284354 |
| miR.558 + miR.603 + miR.520f + miR.220a + miR.519b + miR.130a + miR.29a + miR.362 + miR.369.3p + miR.494 + miR.518c + miR.639 + miR.660 + miR.338 | 0,78 | 0,017434767 |
| miR.558 + miR.603 + miR.520f + miR.220a + miR.519b + miR.130a + miR.26a + miR.29a + miR.362 + miR.369.3p + miR.494 + miR.518c + miR.639 + miR.660 | 0,78 | 0,019895237 |
| miR.603 + miR.520f + miR.220a + miR.519b + miR.130a + miR.26a + miR.29a + miR.369.3p + miR.376a* + miR.494 + miR.518c + miR.639 + miR.660 + miR.338 | 0,78 | 0,006389498 |
| miR.558 + miR.603 + miR.520f + miR.220a + miR.519b + miR.26a + miR.29a + miR.362 + miR.369.3p + miR.494 + miR.518c + miR.639 + miR.660 + miR.338 | 0,78 | 0,023506388 |
| miR.558 + miR.603 + miR.520f + miR.220a + miR.519b + miR.130a + miR.26a + miR.29a + miR.362 + miR.494 + miR.518c + miR.639 + miR.660 + miR.338 | 0,78 | 0,012217828 |
| miR.558 + miR.603 + miR.520f + miR.519b + miR.130a + miR.26a + miR.29a + miR.362 + miR.376a* + miR.494 + miR.518c + miR.639 + miR.660 + miR.338 | 0,78 | 0,004743746 |
| miR.558 + miR.603 + miR.520f + miR.220a + miR.519b + miR.130a + miR.26a + miR.362 + miR.369.3p + miR.494 + miR.518c + miR.639 + miR.660 + miR.338 | 0,78 | 0,010958841 |
| miR.558 + miR.609 + miR.603 + miR.220a + miR.519b + miR.130a + miR.26a + miR.29a + miR.362 + miR.369.3p + miR.494 + miR.639 + miR.660 + miR.338 | 0,78 | 0,004310703 |
| miR.558 + miR.609 + miR.603 + miR.520f + miR.220a + miR.519b + miR.130a + miR.26a + miR.29a + miR.369.3p + miR.494 + miR.639 + miR.660 + miR.338 | 0,78 | 0,001676862 |
| miR.558 + miR.603 + miR.520f + miR.220a + miR.130a + miR.26a + miR.29a + miR.362 + miR.369.3p + miR.494 + miR.518c + miR.639 + miR.660 + miR.338 | 0,77 | 0,010269422 |
| miR.558 + miR.520f + miR.220a + miR.519b + miR.130a + miR.29a + miR.362 + miR.369.3p + miR.376a* + miR.494 + miR.518c + miR.639 + miR.660 + miR.338 | 0,77 | 0,010915222 |
| miR.558 + miR.520f + miR.220a + miR.519b + miR.130a + miR.26a + miR.29a + miR.362 + miR.369.3p + miR.376a* + miR.518c + miR.639 + miR.660 + miR.338 | 0,77 | 0,018014321 |
| miR.558 + miR.609 + miR.603 + miR.520f + miR.220a + miR.519b + miR.130a + miR.26a + miR.362 + miR.369.3p + miR.494 + miR.639 + miR.660 + miR.338 | 0,77 | 0,00816644 |
| miR.558 + miR.520f + miR.220a + miR.519b + miR.26a + miR.29a + miR.362 + miR.369.3p + miR.376a* + miR.494 + miR.518c + miR.639 + miR.660 + miR.338 | 0,77 | 0,011387087 |
| miR.558 + miR.520f + miR.220a + miR.519b + miR.130a + miR.26a + miR.362 + miR.369.3p + miR.376a* + miR.494 + miR.518c + miR.639 + miR.660 + miR.338 | 0,77 | 0,011993783 |
| miR.558 + miR.520f + miR.220a + miR.130a + miR.26a + miR.29a + miR.362 + miR.369.3p + miR.376a* + miR.494 + miR.518c + miR.639 + miR.660 + miR.338 | 0,77 | 0,010071406 |
| miR.558 + miR.520f + miR.220a + miR.519b + miR.130a + miR.26a + miR.29a + miR.362 + miR.369.3p + miR.376a* + miR.494 + miR.518c + miR.639 + miR.660 | 0,77 | 0,011844227 |
| miR.558 + miR.520f + miR.220a + miR.519b + miR.130a + miR.26a + miR.29a + miR.362 + miR.369.3p + miR.376a* + miR.494 + miR.518c + miR.639 + miR.338 | 0,77 | 0,022616737 |
| miR.603 + miR.520f + miR.220a + miR.519b + miR.130a + miR.26a + miR.29a + miR.362 + miR.376a* + miR.494 + miR.518c + miR.639 + miR.660 + miR.338 | 0,77 | 0,002355668 |
| miR.558 + miR.520f + miR.519b + miR.130a + miR.26a + miR.29a + miR.362 + miR.369.3p + miR.376a* + miR.494 + miR.518c + miR.639 + miR.660 + miR.338 | 0,77 | 0,002827991 |
| miR.558 + miR.603 + miR.520f + miR.519b + miR.130a + miR.26a + miR.29a + miR.362 + miR.369.3p + miR.494 + miR.518c + miR.639 + miR.660 + miR.338 | 0,77 | 0,008202613 |
| miR.603 + miR.520f + miR.519b + miR.130a + miR.26a + miR.29a + miR.362 + miR.369.3p + miR.376a* + miR.494 + miR.518c + miR.639 + miR.660 + miR.338 | 0,77 | 0,002968385 |
| miR.558 + miR.520f + miR.220a + miR.519b + miR.130a + miR.26a + miR.29a + miR.369.3p + miR.376a* + miR.494 + miR.518c + miR.639 + miR.660 + miR.338 | 0,77 | 0,014012981 |
| miR.558 + miR.520f + miR.220a + miR.519b + miR.130a + miR.26a + miR.29a + miR.362 + miR.369.3p + miR.376a* + miR.494 + miR.518c + miR.660 + miR.338 | 0,77 | 0,014131251 |
| miR.609 + miR.603 + miR.520f + miR.220a + miR.519b + miR.130a + miR.26a + miR.29a + miR.362 + miR.369.3p + miR.494 + miR.639 + miR.660 + miR.338 | 0,77 | 0,003183899 |
| miR.558 + miR.609 + miR.603 + miR.520f + miR.220a + miR.519b + miR.130a + miR.26a + miR.29a + miR.362 + miR.369.3p + miR.494 + miR.639 + miR.338 | 0,77 | 0,001665673 |
| miR.558 + miR.220a + miR.519b + miR.130a + miR.26a + miR.29a + miR.362 + miR.369.3p + miR.376a* + miR.494 + miR.518c + miR.639 + miR.660 + miR.338 | 0,77 | 0,018514106 |
| miR.558 + miR.520f + miR.220a + miR.519b + miR.130a + miR.26a + miR.29a + miR.362 + miR.376a* + miR.494 + miR.518c + miR.639 + miR.660 + miR.338 | 0,77 | 0,004861981 |
| miR.603 + miR.520f + miR.220a + miR.519b + miR.130a + miR.26a + miR.29a + miR.362 + miR.369.3p + miR.494 + miR.518c + miR.639 + miR.660 + miR.338 | 0,77 | 0,001455528 |
| miR.558 + miR.520f + miR.220a + miR.519b + miR.130a + miR.26a + miR.29a + miR.362 + miR.369.3p + miR.494 + miR.518c + miR.639 + miR.660 + miR.338 | 0,77 | 0,004785135 |
| miR.520f + miR.220a + miR.519b + miR.130a + miR.26a + miR.29a + miR.362 + miR.369.3p + miR.376a* + miR.494 + miR.518c + miR.639 + miR.660 + miR.338 | 0,76 | 0,000466356 |
| miR.558 + miR.603 + miR.520f + miR.519b + miR.130a + miR.26a + miR.29a + miR.362 + miR.369.3p + miR.376a* + miR.494 + miR.639 + miR.660 + miR.338 | 0,76 | 0,001480874 |
| miR.558 + miR.603 + miR.520f + miR.220a + miR.519b + miR.130a + miR.26a + miR.362 + miR.369.3p + miR.376a* + miR.494 + miR.639 + miR.660 + miR.338 | 0,76 | 0,001666107 |
| miR.558 + miR.603 + miR.520f + miR.220a + miR.519b + miR.26a + miR.29a + miR.362 + miR.369.3p + miR.376a* + miR.494 + miR.639 + miR.660 + miR.338 | 0,76 | 0,002647249 |
| miR.558 + miR.603 + miR.520f + miR.220a + miR.519b + miR.130a + miR.29a + miR.362 + miR.369.3p + miR.376a* + miR.494 + miR.639 + miR.660 + miR.338 | 0,76 | 0,002038337 |
| miR.558 + miR.603 + miR.520f + miR.220a + miR.519b + miR.130a + miR.26a + miR.29a + miR.362 + miR.369.3p + miR.376a* + miR.639 + miR.660 + miR.338 | 0,75 | 0,000724525 |
| miR.558 + miR.603 + miR.520f + miR.220a + miR.130a + miR.26a + miR.29a + miR.362 + miR.369.3p + miR.376a* + miR.494 + miR.639 + miR.660 + miR.338 | 0,75 | 0,000438559 |
| miR.558 + miR.603 + miR.520f + miR.220a + miR.519b + miR.130a + miR.26a + miR.29a + miR.362 + miR.369.3p + miR.376a* + miR.494 + miR.660 + miR.338 | 0,75 | 0,004191131 |
| miR.558 + miR.603 + miR.520f + miR.220a + miR.519b + miR.130a + miR.26a + miR.29a + miR.362 + miR.369.3p + miR.376a* + miR.494 + miR.639 + miR.338 | 0,75 | 0,001704329 |
| miR.558 + miR.603 + miR.520f + miR.220a + miR.519b + miR.130a + miR.26a + miR.29a + miR.362 + miR.369.3p + miR.376a* + miR.494 + miR.639 + miR.660 | 0,75 | 0,000807655 |
| miR.558 + miR.520f + miR.220a + miR.519b + miR.130a + miR.26a + miR.29a + miR.362 + miR.369.3p + miR.376a* + miR.494 + miR.639 + miR.660 + miR.338 | 0,75 | 0,001349938 |
| miR.558 + miR.603 + miR.520f + miR.220a + miR.519b + miR.130a + miR.26a + miR.29a + miR.362 + miR.376a* + miR.494 + miR.639 + miR.660 + miR.338 | 0,75 | 0,000296002 |
| miR.603 + miR.520f + miR.220a + miR.519b + miR.130a + miR.26a + miR.29a + miR.362 + miR.369.3p + miR.376a* + miR.494 + miR.639 + miR.660 + miR.338 | 0,75 | 0,00077583 |
| miR.558 + miR.603 + miR.520f + miR.220a + miR.519b + miR.130a + miR.26a + miR.29a + miR.369.3p + miR.376a* + miR.494 + miR.639 + miR.660 + miR.338 | 0,75 | 0,000781236 |
| miR.558 + miR.603 + miR.220a + miR.519b + miR.130a + miR.26a + miR.29a + miR.362 + miR.369.3p + miR.376a* + miR.494 + miR.639 + miR.660 + miR.338 | 0,75 | 0,002537045 |
| miR.558 + miR.603 + miR.520f + miR.220a + miR.519b + miR.130a + miR.26a + miR.29a + miR.362 + miR.369.3p + miR.494 + miR.639 + miR.660 + miR.338 | 0,74 | 0,00066002 |

**Table 15: clusters of15 miRNAs**

| **Cluster of 15 miRs** | **c_tau level** | **pv compared to top signature miR.558 + miR.609 + miR.603 + miR.520f + miR.130a + miR.26a + miR.29a + miR.362 + miR.369.3p + miR.376a* + miR.494 + miR.518c + miR.639 + miR.660 + miR.338** |
|---|---|---|
| miR.558 + miR.609 + miR.603 + miR.520f + miR.130a + miR.26a + miR.29a + miR.362 + miR.369.3p + miR.376a. + miR.494 + miR.518c + miR.639 + miR.660 + miR.338 | 0,82 | NaN |
| miR.558 + miR.609 + miR.603 + miR.520f + miR.519b + miR.130a + miR.29a + miR.362 + miR.369.3p + miR.376a. + miR.494 + miR.518c + miR.639 + miR.660 + miR.338 | 0,82 | 0,417893112 |
| miR.558 + miR.609 + miR.603 + miR.220a + miR.130a + miR.26a + miR.29a + miR.362 + miR.369.3p + miR.376a. + miR.494 + miR.518c + miR.639 + miR.660 + miR.338 | 0,82 | 0,421947834 |
| miR.558 + miR.609 + miR.603 + miR.520f + miR.220a + miR.130a + miR.29a + miR.362 + miR.369.3p + miR.376a. + miR.494 + miR.518c + miR.639 + miR.660 + miR.338 | 0,82 | 0,456723659 |
| miR.558 + miR.609 + miR.603 + miR.520f + miR.220a + miR.26a + miR.29a + miR.362 + miR.369.3p + miR.376a. + miR.494 + miR.518c + miR.639 + miR.660 + miR.338 | 0,82 | 0,436472042 |
| miR.558 + miR.609 + miR.603 + miR.519b + miR.130a + miR.26a + miR.29a + miR.362 + miR.369.3p + miR.376a. + miR.494 + miR.518c + miR.639 + miR.660 + miR.338 | 0,82 | 0,266119713 |
| miR.558 + miR.609 + miR.520f + miR.519b + miR.130a + miR.26a + miR.29a + miR.362 + miR.369.3p + miR.376a. + miR.494 + miR.518c + miR.639 + miR.660 + miR.338 | 0,82 | 0,026919383 |
| miR.558 + miR.609 + miR.603 + miR.520f + miR.220a + miR.519b + miR.29a + miR.362 + miR.369.3p + miR.376a. + miR.494 + miR.518c + miR.639 + miR.660 + miR.338 | 0,82 | 0,450229229 |
| miR.558 + miR.609 + miR.603 + miR.520f + miR.220a + miR.130a + miR.26a + miR.29a + miR.362 + miR.369.3p + miR.376a. + miR.518c + miR.639 + miR.660 + miR.338 | 0,82 | 0,450046797 |
| miR.558 + miR.609 + miR.520f + miR.220a + miR.130a + miR.26a + miR.29a + miR.362 + miR.369.3p + miR.376a. + miR.494 + miR.518c + miR.639 + miR.660 + miR.338 | 0,82 | 0,006693682 |
| miR.558 + miR.609 + miR.520f + miR.220a + miR.519b + miR.130a + miR.29a + miR.362 + miR.369.3p + miR.376a. + miR.494 + miR.518c + miR.639 + miR.660 + miR.338 | 0,82 | 0,009272355 |
| miR.558 + miR.609 + miR.603 + miR.520f + miR.220a + miR.519b + miR.26a + miR.29a + miR.362 + miR.369.3p + miR.376a. + miR.518c + miR.639 + miR.660 + miR.338 | 0,82 | 0,455293954 |
| miR.558 + miR.609 + miR.603 + miR.220a + miR.519b + miR.26a + miR.29a + miR.362 + miR.369.3p + miR.376a. + miR.494 + miR.518c + miR.639 + miR.660 + miR.338 | 0,81 | 0,482287213 |
| miR.558 + miR.609 + miR.603 + miR.220a + miR.519b + miR.130a + miR.29a + miR.362 + miR.369.3p + miR.376a. + miR.494 + miR.518c + miR.639 + miR.660 + miR.338 | 0,81 | 0,316214873 |
| miR.558 + miR.609 + miR.520f + miR.220a + miR.519b + miR.26a + miR.29a + miR.362 + miR.369.3p + miR.376a. + miR.494 + miR.518c + miR.639 + miR.660 + miR.338 | 0,81 | 0,074783672 |
| miR.558 + miR.609 + miR.603 + miR.520f + miR.220a + miR.519b + miR.130a + miR.29a + miR.362 + miR.369.3p + miR.376a. + miR.518c + miR.639 + miR.660 + miR.338 | 0,81 | 0,435288591 |
| miR.558 + miR.609 + miR.603 + miR.520f + miR.519b + miR.26a + miR.29a + miR.362 + miR.369.3p + miR.376a. + miR.494 + miR.518c + miR.639 + miR.660 + miR.338 | 0,81 | 0,478845578 |
| miR.558 + miR.609 + miR.603 + miR.520f + miR.220a + miR.519b + miR.130a + miR.29a + miR.369.3p + miR.376a. + miR.494 + miR.518c + miR.639 + miR.660 + miR.338 | 0,81 | 0,210546057 |
| miR.558 + miR.609 + miR.603 + miR.520f + miR.220a + miR.519b + miR.26a + miR.29a + miR.362 + miR.369.3p + miR.376a. + miR.494 + miR.518c + miR.660 + miR.338 | 0,81 | 0,468227262 |
| miR.558 + miR.609 + miR.603 + miR.520f + miR.220a + miR.519b + miR.26a + miR.29a + miR.369.3p + miR.376a. + miR.494 + miR.518c + miR.639 + miR.660 + miR.338 | 0,81 | 0,223550252 |
| miR.558 + miR.609 + miR.603 + miR.220a + miR.519b + miR.130a + miR.26a + miR.29a + miR.362 + miR.369.3p + miR.376a. + miR.494 + miR.518c + miR.660 + miR.338 | 0,81 | 0,286745317 |
| miR.558 + miR.609 + miR.603 + miR.520f + miR.220a + miR.519b + miR.130a + miR.29a + miR.362 + miR.369.3p + miR.376a. + miR.494 + miR.518c + miR.660 + miR.338 | 0,81 | 0,391989353 |
| miR.558 + miR.609 + miR.520f + miR.220a + miR.519b + miR.130a + miR.26a + miR.29a + miR.362 + miR.369.3p + miR.376a. + miR.518c + miR.639 + miR.660 + miR.338 | 0,81 | 0,103368158 |
| miR.558 + miR.609 + miR.603 + miR.220a + miR.519b + miR.130a + miR.26a + miR.29a + miR.362 + miR.369.3p + miR.376a. + miR.518c + miR.639 + miR.660 + miR.338 | 0,81 | 0,489753522 |
| miR.558 + miR.609 + miR.603 + miR.520f + miR.220a + miR.130a + miR.26a + miR.29a + miR.369.3p + miR.376a. + miR.494 + miR.518c + miR.639 + miR.660 + miR.338 | 0,81 | 0,092748321 |
| miR.558 + miR.609 + miR.603 + miR.520f + miR.519b + miR.130a + miR.26a + miR.29a + miR.362 + miR.369.3p + miR.376a. + miR.518c + miR.639 + miR.660 + miR.338 | 0,81 | 0,474651411 |
| miR.558 + miR.609 + miR.603 + miR.220a + miR.519b + miR.130a + miR.26a + miR.29a + miR.369.3p + miR.376a. + miR.494 + miR.518c + miR.639 + miR.660 + miR.338 | 0,81 | 0,162700387 |
| miR.558 + miR.609 + miR.603 + miR.520f + miR.220a + miR.519b + miR.130a + miR.26a + miR.29a + miR.369.3p + miR.376a. + miR.494 + miR.518c + miR.660 + miR.338 | 0,81 | 0,17522995 |
| miR.558 + miR.609 + miR.220a + miR.519b + miR.130a + miR.26a + miR.29a + miR.362 + miR.369.3p + miR.376a. + miR.494 + miR.518c + miR.639 + miR.660 + miR.338 | 0,81 | 0,34357476 |
| miR.558 + miR.609 + miR.603 + miR.520f + miR.220a + miR.130a + miR.26a + miR.29a + miR.362 + miR.369.3p + miR.376a. + miR.494 + miR.518c + miR.660 + miR.338 | 0,81 | 0,412954075 |
| miR.558 + miR.609 + miR.603 + miR.220a + miR.519b + miR.130a + miR.26a + miR.29a + miR.362 + miR.369.3p + miR.376a. + miR.494 + miR.518c + miR.639 + miR.660 | 0,81 | 0,277631728 |
| miR.558 + miR.609 + miR.603 + miR.520f + miR.220a + miR.519b + miR.130a + miR.26a + miR.29a + miR.362 + miR.369.3p + miR.376a. + miR.518c + miR.660 + miR.338 | 0,81 | 0,450828429 |
| miR.558 + miR.609 + miR.603 + miR.520f + miR.220a + miR.519b + miR.26a + miR.29a + miR.362 + miR.369.3p + miR.376a. + miR.494 + miR.518c + miR.639 + miR.660 | 0,81 | 0,288365965 |
| miR.558 + miR.609 + miR.603 + miR.520f + miR.220a + miR.130a + miR.26a + miR.29a + miR.362 + miR.369.3p + miR.376a. + miR.494 + miR.518c + miR.639 + miR.660 | 0,81 | 0,2350529 |
| miR.558 + miR.609 + miR.603 + miR.520f + miR.220a + miR.519b + miR.130a + miR.26a + miR.29a + miR.369.3p + miR.376a. + miR.494 + miR.518c + miR.639 + miR.660 | 0,81 | 0,215400116 |
| miR.558 + miR.609 + miR.603 + miR.520f + miR.220a + miR.519b + miR.130a + miR.26a + miR.29a + miR.369.3p + miR.376a. + miR.518c + miR.639 + miR.660 + miR.338 | 0,81 | 0,194690338 |
| miR.558 + miR.609 + miR.603 + miR.520f + miR.220a + miR.519b + miR.130a + miR.26a + miR.29a + miR.362 + miR.369.3p + miR.376a. + miR.494 + miR.518c + miR.660 | 0,81 | 0,309617959 |
| miR.558 + miR.609 + miR.520f + miR.220a + miR.519b + miR.130a + miR.26a + miR.29a + miR.362 + miR.369.3p + miR.376a. + miR.494 + miR.518c + miR.660 + miR.338 | 0,81 | 0,161293467 |
| miR.558 + miR.609 + miR.603 + miR.520f + miR.220a + miR.519b + miR.130a + miR.29a + miR.362 + miR.369.3p + miR.376a. + miR.494 + miR.518c + miR.639 + miR.660 | 0,81 | 0,215282667 |
| miR.558 + miR.609 + miR.603 + miR.520f + miR.220a + miR.519b + miR.130a + miR.26a + miR.29a + miR.362 + miR.369.3p + miR.376a. + miR.494 + miR.518c + miR.338 | 0,81 | 0,10211321 |
| miR.558 + miR.609 + miR.603 + miR.520f + miR.220a + miR.519b + miR.130a + miR.26a + miR.29a + miR.369.3p + miR.376a. + miR.494 + miR.518c + miR.639 + miR.338 | 0,81 | 0,093394764 |
| miR.558 + miR.609 + miR.603 + miR.520f + miR.220a + miR.519b + miR.130a + miR.29a + miR.362 + miR.369.3p + miR.376a. + miR.494 + miR.518c + miR.639 + miR.338 | 0,81 | 0,081563215 |
| miR.558 + miR.609 + miR.603 + miR.520f + miR.220a + miR.519b + miR.26a + miR.29a + miR.362 + miR.369.3p + miR.376a. + miR.494 + miR.518c + miR.639 + miR.338 | 0,81 | 0,093913311 |
| miR.558 + miR.609 + miR.603 + miR.220a + miR.519b + miR.130a + miR.26a + miR.29a + miR.362 + miR.369.3p + miR.376a. + miR.494 + miR.518c + miR.639 + miR.338 | 0,81 | 0,107763407 |
| miR.558 + miR.609 + miR.603 + miR.520f + miR.220a + miR.519b + miR.130a + miR.26a + miR.29a + miR.362 + miR.369.3p + miR.376a. + miR.518c + miR.639 + miR.660 | 0,81 | 0,327963791 |
| miR.558 + miR.609 + miR.603 + miR.520f + miR.519b + miR.130a + miR.26a + miR.29a + miR.369.3p + miR.376a. + miR.494 + miR.518c + miR.639 + miR.660 + miR.338 | 0,81 | 0,118805836 |
| miR.558 + miR.609 + miR.603 + miR.520f + miR.220a + miR.130a + miR.26a + miR.29a + miR.362 + miR.369.3p + miR.376a. + miR.494 + miR.518c + miR.639 + miR.338 | 0,81 | 0,036016323 |
| miR.558 + miR.609 + miR.603 + miR.520f + miR.220a + miR.519b + miR.130a + miR.26a + miR.29a + miR.362 + miR.369.3p + miR.376a. + miR.494 + miR.518c + miR.639 | 0,81 | 0,150167898 |
| miR.558 + miR.609 + miR.520f + miR.220a + miR.519b + miR.130a + miR.26a + miR.29a + miR.369.3p + miR.376a. + miR.494 + miR.518c + miR.639 + miR.660 + miR.338 | 0,81 | 0,331081172 |
| miR.558 + miR.609 + miR.603 + miR.520f + miR.519b + miR.130a + miR.26a + miR.29a + miR.362 + miR.369.3p + miR.376a. + miR.494 + miR.518c + miR.660 + miR.338 | 0,81 | 0,413196791 |
| miR.558 + miR.609 + miR.603 + miR.520f + miR.519b + miR.130a + miR.26a + miR.29a + miR.362 + miR.369.3p + miR.376a. + miR.494 + miR.518c + miR.639 + miR.660 | 0,81 | 0,03822282 |
| miR.558 + miR.609 + miR.603 + miR.220a + miR.519b + miR.130a + miR.26a + miR.29a + miR.362 + miR.376a. + miR.494 + miR.518c + miR.639 + miR.660 + miR.338 | 0,81 | 0,109920647 |
| miR.558 + miR.609 + miR.603 + miR.520f + miR.220a + miR.519b + miR.130a + miR.26a + miR.29a + miR.362 + miR.369.3p + miR.376a. + miR.518c + miR.639 + miR.338 | 0,81 | 0,127195311 |
| miR.558 + miR.609 + miR.603 + miR.520f + miR.220a + miR.519b + miR.130a + miR.26a + miR.29a + miR.376a. + miR.494 + miR.518c + miR.639 + miR.660 + miR.338 | 0,81 | 0,112211762 |
| miR.558 + miR.609 + miR.603 + miR.520f + miR.220a + miR.519b + miR.130a + miR.29a + miR.362 + miR.376a. + miR.494 + miR.518c + miR.639 + miR.660 + miR.338 | 0,81 | 0,125745555 |
| miR.558 + miR.609 + miR.520f + miR.220a + miR.519b + miR.130a + miR.26a + miR.29a + miR.362 + miR.369.3p + miR.376a. + miR.494 + miR.518c + miR.639 + miR.660 | 0,81 | 0,376610793 |
| miR.558 + miR.609 + miR.603 + miR.520f + miR.220a + miR.519b + miR.26a + miR.29a + miR.362 + miR.376a. + miR.494 + miR.518c + miR.639 + miR.660 + miR.338 | 0,81 | 0,112351353 |
| miR.558 + miR.609 + miR.603 + miR.520f + miR.220a + miR.519b + miR.130a + miR.26a + miR.29a + miR.362 + miR.376a. + miR.494 + miR.518c + miR.639 + miR.338 | 0,81 | 0,096797657 |
| miR.558 + miR.609 + miR.603 + miR.520f + miR.220a + miR.519b + miR.130a + miR.26a + miR.29a + miR.362 + miR.376a. + miR.494 + miR.518c + miR.660 + miR.338 | 0,81 | 0,149699339 |
| miR.558 + miR.609 + miR.603 + miR.520f + miR.519b + miR.130a + miR.26a + miR.29a + miR.362 + miR.369.3p + miR.376a. + miR.494 + miR.518c + miR.639 + miR.338 | 0,81 | 0,063984212 |
| miR.558 + miR.609 + miR.603 + miR.520f + miR.220a + miR.519b + miR.130a + miR.26a + miR.29a + miR.362 + miR.376a. + miR.494 + miR.518c + miR.639 + miR.660 | 0,81 | 0,131446617 |
| miR.558 + miR.609 + miR.603 + miR.520f + miR.220a + miR.130a + miR.26a + miR.29a + miR.362 + miR.376a. + miR.494 + miR.518c + miR.639 + miR.660 + miR.338 | 0,81 | 0,010680595 |
| miR.609 + miR.603 + miR.520f + miR.220a + miR.519b + miR.26a + miR.29a + miR.362 + miR.369.3p + miR.376a. + miR.494 + miR.518c + miR.639 + miR.660 + miR.338 | 0,81 | 0,144240395 |
| miR.609 + miR.603 + miR.520f + miR.220a + miR.519b + miR.130a + miR.29a + miR.362 + miR.369.3p + miR.376a. + miR.494 + miR.518c + miR.639 + miR.660 + miR.338 | 0,81 | 0,132825914 |
| miR.609 + miR.603 + miR.520f + miR.220a + miR.130a + miR.26a + miR.29a + miR.362 + miR.369.3p + miR.376a. + miR.494 + miR.518c + miR.639 + miR.660 + miR.338 | 0,81 | 0,161500162 |
| miR.558 + miR.609 + miR.520f + miR.220a + miR.519b + miR.130a + miR.26a + miR.29a + miR.362 + miR.376a. + miR.494 + miR.518c + miR.639 + miR.660 + miR.338 | 0,81 | 0,175203922 |
| miR.558 + miR.609 + miR.603 + miR.520f + miR.220a + miR.519b + miR.130a + miR.362 + miR.369.3p + miR.376a. + miR.494 + miR.518c + miR.639 + miR.660 + miR.338 | 0,81 | 0,167563806 |
| miR.609 + miR.603 + miR.520f + miR.220a + miR.519b + miR.130a + miR.26a + miR.29a + miR.362 + miR.369.3p + miR.376a. + miR.494 + miR.518c + miR.660 + miR.338 | 0,81 | 0,393067943 |
| miR.558 + miR.609 + miR.603 + miR.520f + miR.220a + miR.130a + miR.26a + miR.362 + miR.369.3p + miR.376a. + miR.494 + miR.518c + miR.639 + miR.660 + miR.338 | 0,81 | 0,149893937 |
| miR.609 + miR.603 + miR.220a + miR.519b + miR.130a + miR.26a + miR.29a + miR.362 + miR.369.3p + miR.376a. + miR.494 + miR.518c + miR.639 + miR.660 + miR.338 | 0,81 | 0,34632805 |
| miR.558 + miR.609 + miR.603 + miR.520f + miR.220a + miR.519b + miR.130a + miR.26a + miR.362 + miR.369.3p + miR.376a. + miR.518c + miR.639 + miR.660 + miR.338 | 0,81 | 0,145848675 |
| miR.558 + miR.609 + miR.520f + miR.220a + miR.519b + miR.130a + miR.26a + miR.29a + miR.362 + miR.369.3p + miR.376a. + miR.494 + miR.518c + miR.639 + miR.338 | 0,81 | 0,167860153 |
| miR.558 + miR.609 + miR.603 + miR.520f + miR.220a + miR.519b + miR.26a + miR.362 + miR.369.3p + miR.376a. + miR.494 + miR.518c + miR.639 + miR.660 + miR.338 | 0,81 | 0,126816594 |
| miR.609 + miR.603 + miR.520f + miR.519b + miR.130a + miR.26a + miR.29a + miR.362 + miR.369.3p + miR.376a. + miR.494 + miR.518c + miR.639 + miR.660 + miR.338 | 0,81 | 0,370477332 |
| miR.558 + miR.609 + miR.603 + miR.520f + miR.519b + miR.130a + miR.26a + miR.362 + miR.369.3p + miR.376a. + miR.494 + miR.518c + miR.639 + miR.660 + miR.338 | 0,81 | 0,228251369 |
| miR.558 + miR.609 + miR.603 + miR.520f + miR.220a + miR.519b + miR.130a + miR.26a + miR.29a + miR.362 + miR.376a. + miR.518c + miR.639 + miR.660 + miR.338 | 0,81 | 0,073192445 |
| miR.609 + miR.603 + miR.520f + miR.220a + miR.519b + miR.130a + miR.26a + miR.29a + miR.362 + miR.369.3p + miR.376a. + miR.494 + miR.518c + miR.639 + miR.660 | 0,81 | 0,116546595 |
| miR.609 + miR.603 + miR.520f + miR.220a + miR.519b + miR.130a + miR.26a + miR.29a + miR.369.3p + miR.376a. + miR.494 + miR.518c + miR.639 + miR.660 + miR.338 | 0,81 | 0,122133192 |
| miR.558 + miR.609 + miR.603 + miR.520f + miR.220a + miR.130a + miR.26a + miR.29a + miR.362 + miR.369.3p + miR.376a. + miR.494 + miR.639 + miR.660 + miR.338 | 0,81 | 0,219796182 |
| miR.609 + miR.520f + miR.220a + miR.519b + miR.130a + miR.26a + miR.29a + miR.362 + miR.369.3p + miR.376a. + miR.494 + miR.518c + miR.639 + miR.660 + miR.338 | 0,81 | 0,295356576 |
| miR.558 + miR.609 + miR.603 + miR.520f + miR.220a + miR.519b + miR.130a + miR.29a + miR.362 + miR.369.3p + miR.376a. + miR.494 + miR.639 + miR.660 + miR.338 | 0,81 | 0,225754346 |
| miR.558 + miR.609 + miR.603 + miR.520f + miR.519b + miR.130a + miR.26a + miR.29a + miR.362 + miR.376a. + miR.494 + miR.518c + miR.639 + miR.660 + miR.338 | 0,81 | 0,045119668 |
| miR.609 + miR.603 + miR.520f + miR.220a + miR.519b + miR.130a + miR.26a + miR.29a + miR.362 + miR.369.3p + miR.376a. + miR.518c + miR.639 + miR.660 + miR.338 | 0,81 | 0,212558942 |
| miR.558 + miR.609 + miR.520f + miR.220a + miR.519b + miR.130a + miR.26a + miR.29a + miR.362 + miR.369.3p + miR.376a. + miR.494 + miR.639 + miR.660 + miR.338 | 0,81 | 0,263873769 |
| miR.558 + miR.609 + miR.603 + miR.520f + miR.519b + miR.130a + miR.26a + miR.29a + miR.362 + miR.369.3p + miR.376a. + miR.494 + miR.639 + miR.660 + miR.338 | 0,81 | 0,263452602 |
| miR.558 + miR.609 + miR.603 + miR.520f + miR.220a + miR.519b + miR.26a + miR.29a + miR.362 + miR.369.3p + miR.376a. + miR.494 + miR.639 + miR.660 + miR.338 | 0,81 | 0,31341978 |
| miR.558 + miR.609 + miR.520f + miR.220a + miR.519b + miR.130a + miR.26a + miR.362 + miR.369.3p + miR.376a. + miR.494 + miR.518c + miR.639 + miR.660 + miR.338 | 0,81 | 0,198337901 |
| miR.558 + miR.609 + miR.603 + miR.520f + miR.220a + miR.519b + miR.130a + miR.26a + miR.369.3p + miR.376a. + miR.494 + miR.518c + miR.639 + miR.660 + miR.338 | 0,81 | 0,172671019 |
| miR.558 + miR.609 + miR.603 + miR.520f + miR.220a + miR.519b + miR.130a + miR.26a + miR.362 + miR.369.3p + miR.376a. + miR.494 + miR.518c + miR.639 + miR.660 | 0,81 | 0,018337047 |
| miR.558 + miR.609 + miR.603 + miR.520f + miR.220a + miR.519b + miR.130a + miR.26a + miR.29a + miR.362 + miR.369.3p + miR.376a. + miR.494 + miR.660 + miR.338 | 0,81 | 0,364987235 |
| miR.558 + miR.609 + miR.603 + miR.220a + miR.519b + miR.130a + miR.26a + miR.362 + miR.369.3p + miR.376a. + miR.494 + miR.518c + miR.639 + miR.660 + miR.338 | 0,8 | 0,070908309 |
| miR.558 + miR.609 + miR.603 + miR.520f + miR.220a + miR.519b + miR.130a + miR.26a + miR.29a + miR.362 + miR.369.3p + miR.376a. + miR.639 + miR.660 + miR.338 | 0,8 | 0,301858502 |
| miR.609 + miR.603 + miR.520f + miR.220a + miR.519b + miR.130a + miR.26a + miR.362 + miR.369.3p + miR.376a. + miR.494 + miR.518c + miR.639 + miR.660 + miR.338 | 0,8 | 0,172914559 |
| miR.609 + miR.603 + miR.520f + miR.220a + miR.519b + miR.130a + miR.26a + miR.29a + miR.362 + miR.369.3p + miR.376a. + miR.494 + miR.518c + miR.639 + miR.338 | 0,8 | 0,085241504 |
| miR.558 + miR.609 + miR.603 + miR.520f + miR.220a + miR.519b + miR.130a + miR.26a + miR.362 + miR.369.3p + miR.376a. + miR.494 + miR.518c + miR.660 + miR.338 | 0,8 | 0,275912582 |
| miR.609 + miR.603 + miR.520f + miR.220a + miR.519b + miR.130a + miR.26a + miR.29a + miR.362 + miR.376a. + miR.494 + miR.518c + miR.639 + miR.660 + miR.338 | 0,8 | 0,048725879 |
| miR.609 + miR.603 + miR.520f + miR.220a + miR.519b + miR.130a + miR.26a + miR.29a + miR.362 + miR.369.3p + miR.376a. + miR.494 + miR.639 + miR.660 + miR.338 | 0,8 | 0,340248591 |
| miR.558 + miR.609 + miR.603 + miR.520f + miR.220a + miR.519b + miR.130a + miR.26a + miR.362 + miR.369.3p + miR.376a. + miR.494 + miR.518c + miR.639 + miR.338 | 0,8 | 0,023945648 |
| miR.558 + miR.609 + miR.603 + miR.220a + miR.519b + miR.130a + miR.26a + miR.29a + miR.362 + miR.369.3p + miR.376a. + miR.494 + miR.639 + miR.660 + miR.338 | 0,8 | 0,119519147 |
| miR.558 + miR.609 + miR.603 + miR.520f + miR.220a + miR.519b + miR.130a + miR.26a + miR.362 + miR.376a. + miR.494 + miR.518c + miR.639 + miR.660 + miR.338 | 0,8 | 0,103363765 |
| miR.558 + miR.609 + miR.603 + miR.520f + miR.220a + miR.519b + miR.130a + miR.26a + miR.29a + miR.369.3p + miR.376a. + miR.494 + miR.639 + miR.660 + miR.338 | 0,8 | 0,105124135 |
| miR.558 + miR.609 + miR.603 + miR.520f + miR.220a + miR.519b + miR.130a + miR.26a + miR.362 + miR.369.3p + miR.376a. + miR.494 + miR.639 + miR.660 + miR.338 | 0,8 | 0,198217613 |
| miR.558 + miR.609 + miR.603 + miR.520f + miR.220a + miR.519b + miR.130a + miR.26a + miR.29a + miR.362 + miR.369.3p + miR.376a. + miR.494 + miR.639 + miR.660 | 0,8 | 0,008765638 |
| miR.558 + miR.609 + miR.603 + miR.220a + miR.519b + miR.130a + miR.26a + miR.29a + miR.362 + miR.369.3p + miR.494 + miR.518c + miR.639 + miR.660 + miR.338 | 0,8 | 0,021270043 |
| miR.558 + miR.609 + miR.603 + miR.520f + miR.220a + miR.519b + miR.130a + miR.26a + miR.29a + miR.362 + miR.369.3p + miR.494 + miR.518c + miR.639 + miR.660 | 0,8 | 0,010274001 |
| miR.558 + miR.609 + miR.603 + miR.520f + miR.220a + miR.519b + miR.130a + miR.29a + miR.362 + miR.369.3p + miR.494 + miR.518c + miR.639 + miR.660 + miR.338 | 0,8 | 0,008719557 |
| miR.558 + miR.609 + miR.603 + miR.520f + miR.220a + miR.130a + miR.26a + miR.29a + miR.362 + miR.369.3p + miR.494 + miR.518c + miR.639 + miR.660 + miR.338 | 0,8 | 0,012052399 |
| miR.558 + miR.609 + miR.603 + miR.520f + miR.220a + miR.519b + miR.130a + miR.26a + miR.29a + miR.362 + miR.369.3p + miR.376a. + miR.494 + miR.639 + miR.338 | 0,8 | 0,022360041 |
| miR.558 + miR.609 + miR.603 + miR.520f + miR.220a + miR.519b + miR.130a + miR.26a + miR.29a + miR.362 + miR.369.3p + miR.518c + miR.639 + miR.660 + miR.338 | 0,8 | 0,011327887 |
| miR.558 + miR.609 + miR.603 + miR.520f + miR.220a + miR.519b + miR.130a + miR.26a + miR.29a + miR.362 + miR.376a. + miR.494 + miR.639 + miR.660 + miR.338 | 0,8 | 0,075661668 |
| miR.558 + miR.609 + miR.603 + miR.520f + miR.220a + miR.519b + miR.130a + miR.26a + miR.29a + miR.362 + miR.369.3p + miR.494 + miR.518c + miR.660 + miR.338 | 0,8 | 0,024467973 |
| miR.558 + miR.609 + miR.603 + miR.520f + miR.220a + miR.519b + miR.130a + miR.26a + miR.29a + miR.369.3p + miR.494 + miR.518c + miR.639 + miR.660 + miR.338 | 0,8 | 0,020813306 |
| miR.558 + miR.609 + miR.603 + miR.520f + miR.220a + miR.519b + miR.130a + miR.26a + miR.29a + miR.362 + miR.494 + miR.518c + miR.639 + miR.660 + miR.338 | 0,8 | 0,01599405 |
| miR.558 + miR.609 + miR.603 + miR.520f + miR.220a + miR.519b + miR.26a + miR.29a + miR.362 + miR.369.3p + miR.494 + miR.518c + miR.639 + miR.660 + miR.338 | 0,8 | 0,014343674 |
| miR.558 + miR.609 + miR.603 + miR.520f + miR.519b + miR.130a + miR.26a + miR.29a + miR.362 + miR.369.3p + miR.494 + miR.518c + miR.639 + miR.660 + miR.338 | 0,8 | 0,003784491 |
| miR.558 + miR.609 + miR.520f + miR.220a + miR.519b + miR.130a + miR.26a + miR.29a + miR.362 + miR.369.3p + miR.494 + miR.518c + miR.639 + miR.660 + miR.338 | 0,8 | 0,004771994 |
| miR.558 + miR.609 + miR.603 + miR.520f + miR.220a + miR.519b + miR.130a + miR.26a + miR.29a + miR.362 + miR.369.3p + miR.494 + miR.518c + miR.639 + miR.338 | 0,79 | 0,025855658 |
| miR.558 + miR.609 + miR.603 + miR.520f + miR.220a + miR.519b + miR.130a + miR.26a + miR.362 + miR.369.3p + miR.494 + miR.518c + miR.639 + miR.660 + miR.338 | 0,79 | 0,003847267 |
| miR.609 + miR.603 + miR.520f + miR.220a + miR.519b + miR.130a + miR.26a + miR.29a + miR.362 + miR.369.3p + miR.494 + miR.518c + miR.639 + miR.660 + miR.338 | 0,79 | 0,006399787 |
| miR.558 + miR.603 + miR.220a + miR.519b + miR.130a + miR.26a + miR.29a + miR.362 + miR.369.3p + miR.376a. + miR.494 + miR.518c + miR.639 + miR.660 + miR.338 | 0,79 | 0,052750788 |
| miR.558 + miR.603 + miR.520f + miR.220a + miR.519b + miR.130a + miR.26a + miR.29a + miR.362 + miR.369.3p + miR.376a. + miR.518c + miR.639 + miR.660 + miR.338 | 0,79 | 0,045179266 |
| miR.558 + miR.603 + miR.520f + miR.220a + miR.519b + miR.130a + miR.29a + miR.362 + miR.369.3p + miR.376a. + miR.494 + miR.518c + miR.639 + miR.660 + miR.338 | 0,79 | 0,02591738 |
| miR.558 + miR.603 + miR.520f + miR.220a + miR.519b + miR.130a + miR.26a + miR.29a + miR.362 + miR.369.3p + miR.376a. + miR.494 + miR.518c + miR.639 + miR.660 | 0,79 | 0,043496008 |
| miR.558 + miR.603 + miR.520f + miR.220a + miR.519b + miR.130a + miR.26a + miR.29a + miR.362 + miR.369.3p + miR.376a. + miR.494 + miR.518c + miR.639 + miR.338 | 0,79 | 0,034857672 |
| miR.558 + miR.603 + miR.520f + miR.220a + miR.519b + miR.26a + miR.29a + miR.362 + miR.369.3p + miR.376a. + miR.494 + miR.518c + miR.639 + miR.660 + miR.338 | 0,79 | 0,040182398 |
| miR.558 + miR.603 + miR.520f + miR.220a + miR.519b + miR.130a + miR.26a + miR.29a + miR.362 + miR.369.3p + miR.376a. + miR.494 + miR.518c + miR.660 + miR.338 | 0,79 | 0,05514381 |
| miR.558 + miR.603 + miR.520f + miR.220a + miR.519b + miR.130a + miR.26a + miR.29a + miR.369.3p + miR.376a. + miR.494 + miR.518c + miR.639 + miR.660 + miR.338 | 0,79 | 0,035721744 |
| miR.558 + miR.603 + miR.520f + miR.220a + miR.519b + miR.130a + miR.26a + miR.362 + miR.369.3p + miR.376a. + miR.494 + miR.518c + miR.639 + miR.660 + miR.338 | 0,79 | 0,037218329 |
| miR.558 + miR.603 + miR.520f + miR.220a + miR.130a + miR.26a + miR.29a + miR.362 + miR.369.3p + miR.376a. + miR.494 + miR.518c + miR.639 + miR.660 + miR.338 | 0,78 | 0,020642923 |
| miR.558 + miR.603 + miR.520f + miR.220a + miR.519b + miR.130a + miR.26a + miR.29a + miR.362 + miR.376a. + miR.494 + miR.518c + miR.639 + miR.660 + miR.338 | 0,78 | 0,014050884 |
| miR.558 + miR.603 + miR.520f + miR.519b + miR.130a + miR.26a + miR.29a + miR.362 + miR.369.3p + miR.376a. + miR.494 + miR.518c + miR.639 + miR.660 + miR.338 | 0,78 | 0,021301208 |
| miR.558 + miR.609 + miR.603 + miR.520f + miR.220a + miR.519b + miR.130a + miR.26a + miR.29a + miR.362 + miR.369.3p + miR.494 + miR.639 + miR.660 + miR.338 | 0,78 | 0,005883105 |
| miR.603 + miR.520f + miR.220a + miR.519b + miR.130a + miR.26a + miR.29a + miR.362 + miR.369.3p + miR.376a. + miR.494 + miR.518c + miR.639 + miR.660 + miR.338 | 0,78 | 0,013306274 |
| miR.558 + miR.603 + miR.520f + miR.220a + miR.519b + miR.130a + miR.26a + miR.29a + miR.362 + miR.369.3p + miR.494 + miR.518c + miR.639 + miR.660 + miR.338 | 0,78 | 0,017843499 |
| miR.558 + miR.520f + miR.220a + miR.519b + miR.130a + miR.26a + miR.29a + miR.362 + miR.369.3p + miR.376a. + miR.494 + miR.518c + miR.639 + miR.660 + miR.338 | 0,77 | 0,010286083 |
| miR.558 + miR.603 + miR.520f + miR.220a + miR.519b + miR.130a + miR.26a + miR.29a + miR.362 + miR.369.3p + miR.376a. + miR.494 + miR.639 + miR.660 + miR.338 | 0,76 | 0,00164717 |

| **Table 16: clusters of 16 miRNAs** | | |
|---|---|---|
| **Cluster of 16 miRs** | **c_ tau level** | **pv compared to top signature miR.558 + miR.609 + miR.603 + miR.520f + miR.220a + miR.130a + miR.26a + miR.29a + miR.362 + miR.369.3 p + miR.376a* + miR.494 + miR.518c + miR.639 + miR.660 + miR.338** |
| miR.558 + miR.609 + miR.603 + miR.520f + miR.220a + miR.130a + miR.26a + miR.29a + miR.362 + miR.369.3p + miR.376a. + miR.494 + miR.518c + miR.639 + miR.660 + miR.338 | 0,82 | NaN |
| miR.558 + miR.609 + miR.603 + miR.520f + miR.519b + miR.130a + miR.26a + miR.29a + miR.362 + miR.369.3p + miR.376a. + miR.494 + miR.518c + miR.639 + miR.660 + miR.338 | 0,82 | 0,346794554 |
| miR.558 + miR.609 + miR.603 + miR.520f + miR.220a + miR.519b + miR.130a + miR.29a + miR.362 + miR.369.3p + miR.376a. + miR.494 + miR.518c + miR.639 + miR.660 + miR.338 | 0,82 | 0,374050841 |
| miR.558 + miR.609 + miR.603 + miR.220a + miR.519b + miR.130a + miR.26a + miR.29a + miR.362 + miR.369.3p + miR.376a. + miR.494 + miR.518c + miR.639 + miR.660 + miR.338 | 0,82 | 0,310289635 |
| miR.558 + miR.609 + miR.603 + miR.520f + miR.220a + miR.519b + miR.26a + miR.29a + miR.362 + miR.369.3p + miR.376a. + miR.494 + miR.518c + miR.639 + miR.660 + miR.338 | 0,82 | 0,366102054 |
| miR.558 + miR.609 + miR.520f + miR.220a + miR.519b + miR.130a + miR.26a + miR.29a + miR.362 + miR.369.3p + miR.376a. + miR.494 + miR.518c + miR.639 + miR.660 + miR.338 | 0,82 | 0,158144613 |
| miR.558 + miR.609 + miR.603 + miR.520f + miR.220a + miR.519b + miR.130a + miR.26a + miR.29a + miR.362 + miR.369.3p + miR.376a. + miR.518c + miR.639 + miR.660 + miR.338 | 0,82 | 0,445078393 |
| miR.558 + miR.609 + miR.603 + miR.520f + miR.220a + miR.519b + miR.130a + miR.26a + miR.29a + miR.369.3p + miR.376a. + miR.494 + miR.518c + miR.639 + miR.660 + miR.338 | 0,81 | 0,109507894 |
| miR.558 + miR.609 + miR.603 + miR.520f + miR.220a + miR.519b + miR.130a + miR.26a + miR.29a + miR.362 + miR.369.3p + miR.376a. + miR.494 + miR.518c + miR.660 + miR.338 | 0,81 | 0,200650952 |
| miR.558 + miR.609 + miR.603 + miR.520f + miR.220a + miR.519b + miR.130a + miR.26a + miR.29a + miR.362 + miR.369.3p + miR.376a. + miR.494 + miR.518c + miR.639 + miR.660 | 0,81 | 0,217785753 |
| miR.558 + miR.609 + miR.603 + miR.520f + miR.220a + miR.519b + miR.130a + miR.26a + miR.29a + miR.362 + miR.369.3p + miR.376a. + miR.494 + miR.518c + miR.639 + miR.338 | 0,81 | 0,066505939 |
| miR.558 + miR.609 + miR.603 + miR.520f + miR.220a + miR.519b + miR.130a + miR.26a + miR.29a + miR.362 + miR.376a. + miR.494 + miR.518c + miR.639 + miR.660 + miR.338 | 0,81 | 0,073885357 |
| miR.609 + miR.603 + miR.520f + miR.220a + miR.519b + miR.130a + miR.26a + miR.29a + miR.362 + miR.369.3p + miR.376a. + miR.494 + miR.518c + miR.639 + miR.660 + miR.338 | 0,81 | 0,303472946 |
| miR.558 + miR.609 + miR.603 + miR.520f + miR.220a + miR.519b + miR.130a + miR.26a + miR.362 + miR.369.3p + miR.376a. + miR.494 + miR.518c + miR.639 + miR.660 + miR.338 | 0,81 | 0,185015053 |
| miR.558 + miR.609 + miR.603 + miR.520f + miR.220a + miR.519b + miR.130a + miR.26a + miR.29a + miR.362 + miR.369.3p + miR.376a. + miR.494 + miR.639 + miR.660 + miR.338 | 0,81 | 0,216679209 |
| miR.558 + miR.609 + miR.603 + miR.520f + miR.220a + miR.519b + miR.130a + miR.26a + miR.29a + miR.362 + miR.369.3p + miR.494 + miR.518c + miR.639 + miR.660 + miR.338 | 0,8 | 0,008870546 |
| miR.558 + miR.603 + miR.520f + miR.220a + miR.519b + miR.130a + miR.26a + miR.29a + miR.362 + miR.369.3p + miR.376a. + miR.494 + miR.518c + miR.639 + miR.660 + miR.338 | 0,79 | 0,038816435 |

| **Table 17: best clusters of miRNAs** | | | |
|---|---|---|---|
| **Comparaison between the top clusters of miRs** | **c_tau level** | **pv compared to UICC** | **pv compared to top signature miR.558 + miR.609 + miR.603 + miR.520f + miR.220a + miR.519b + miR.130a + miR.26a + miR.29a + miR.362 + miR.369.3p + miR.376a .+ miR.494 + miR.518c + miR.639 + miR.660 + miR.338** |
| miR.558 + miR.609 + miR.603 + miR.520f + miR.220a + miR.519b + miR.130a + miR.26a + miR.29a + miR.362 + miR.369.3p + miR.376a.+ miR.494 + miR.518c + miR.639 + miR.660 + miR.338 | 0,82 | 3,89631E-09 | NaN |
| miR.558 + miR.609 + miR.603 + miR.520f + miR.220a + miR.130a + miR.26a + miR.29a + miR.362 + miR.369.3p + miR.376a. + miR.494 + miR.518c + miR.639 + miR.660 + miR.338 | 0,82 | 5,31322E-09 | 0,273786656 |
| miR.558 + miR.609 + miR.603 + miR.520f + miR.130a + miR.26a + miR.29a + miR.362 + miR.369.3p + miR.376a. + miR.494 + miR.518c + miR.639 + miR.660 + miR.338 | 0,82 | 1,07178E-09 | 0,46959299 |
| miR.558 + miR.609 + miR.603 + miR.520f + miR.130a + miR.29a + miR.362 + miR.369.3p + miR.376a. + miR.494 + miR.518c + miR.639 + miR.660 + miR.338 | 0,82 | 1,57648E-09 | 0,496622615 |
| miR.558 + miR.609 + miR.520f + miR.130a + miR.29a + miR.362 + miR.369.3p + miR.376a. + miR.494 + miR.518c + miR.639 + miR.660 + miR.338 | 0,82 | 3,57144E-09 | 0,358285772 |
| miR.558 + miR.609 + miR.520f + miR.220a + miR.29a + miR.362 + miR.369.3p + miR.376a. + miR.518c + miR.639 + miR.660 + miR.338 | 0,81 | 4,82435E-09 | 0,231087673 |
| miR.558 + miR.609 + miR.520f + miR.29a + miR.362 + miR.369.3p + miR.376a. + miR.518c + miR.639 + miR.660 + miR.338 | 0,81 | 3,58012E-09 | 0,455646227 |
| miR.558 + miR.609 + miR.603 + miR.220a + miR.26a + miR.29a + miR.369.3p + miR.376a. + miR.518c + miR.660 | 0,81 | 2,32944E-08 | 0,062628078 |
| miR.558 + miR.609 + miR.603 + miR.220a + miR.26a + miR.29a + miR.369.3p + miR.376a. + miR.518c | 0,81 | 1,12929E-08 | 0,021753942 |
| miR.558 + miR.609 + miR.603 + miR.220a + miR.29a + miR.369.3p + miR.376a. + miR.518c | 0,81 | 1,05401E-08 | 0,003784432 |
| miR.558 + miR.609 + miR.220a + miR.29a + miR.369.3p + miR.376a. + miR.518c | 0,8 | 1,70664E-07 | 0,016562471 |
| miR.558 + miR.609 + miR.220a + miR.29a + miR.376a. + miR.518c | 0,8 | 2,75189E-07 | 0,005183149 |
| miR.558 + miR.609 + miR.29a + miR.376a. + miR.518c | 0,79 | 8,33665E-07 | 0,007189529 |
| miR.609 + miR.29a + miR.376a. + miR.518c | 0,77 | 0,000258704 | 0,001288932 |
| miR.558 + miR.609 + miR.518c | 0,76 | 3,78 | 8,30 |
| | | 08E-05 | 546E-05 |
| miR.603 + miR.518c | 0,73 | 1,38325E-05 | 8,07086E-06 |
| miR.609 | 0,66 | 0,071282935 | 7,46688E-10 |
| UICC | 0,59 | NaN | 3,89631E-09 |

### REFERENCES:

Throughout this application, various references describe the state of the art to which this invention pertains. The disclosures of these references are hereby incorporated by reference into the present disclosure.
Galon J, Costes A, Sanchez-Cabo F, Kirilovsky A, Mlecnik B, Lagorce-Pagès C, Tosolini M, Camus M, Berger A, Wind P, Zinzindohoué F, Bruneval P, Cugnenc PH, Trajanoski Z, Fridman WH, Pages F. Type, density, and location of immune cells within human colorectal tumors predict clinical outcome. Science. 2006 Sep 29;313(5795):1960-4.
Harrell FE Jr, Lee KL, Mark DB.Multivariable prognostic models: issues in developing models, evaluating assumptions and adequacy, and measuring and reducing errors. Stat Med. 1996 Feb 28;15(4):361-87. Review.
Heagerty PJ, Zheng Y. Survival model predictive accuracy and ROC curves. Biometrics. 2005 Mar;61(1):92-105.
Pencina MJ, D'Agostino RB Sr, D'Agostino RB Jr, Vasan RS. Evaluating the added predictive ability of a new marker: from area under the ROC curve to reclassification and beyond. Stat Med. 2008 Jan30;27(2): 157-72; discussion 207-12.

## Claims

1. A method for predicting the outcome of a cancer in a patient comprising a step consisting of determining the expression level of a miRNA cluster in a tumor sample obtained from said patient, wherein said miRNA cluster comprises miR.494.

2. The method according to claim 1 wherein said miRNA cluster consists in the combination miR.558 + miR.609 + miR.520f + miR.130a + miR.29a + miR.362 + miR.369.3p + miR.376a. + miR.494 + miR.518c + miR.639 + miR.660 + miR.338.

3. The method according to claim 1 wherein said miRNA cluster consists in the combination miR.558 + miR.609 + miR.603 + miR.520f + miR.130a + miR.29a + miR.362 + miR.369.3p + miR.376a. + miR.494 + miR.518c + miR.639 + miR.660 + miR.338.

4. The method according to claim 1 wherein said miRNA cluster consists in the combination miR.558 + miR.609 + miR.603 + miR.520f + miR.130a + miR.26a + miR.29a + miR.362 + miR.369.3p + miR.376a. + miR.494 + miR.518c + miR.639 + miR.660 + miR.338.

5. The method according to claim 1 wherein said miRNA cluster consists in the combination miR.558 + miR.609 + miR.603 + miR.520f + miR.220a + miR.130a + miR.26a + miR.29a + miR.362 + miR.369.3p + miR.376a. + miR.494 + miR.518c + miR.639 + miR.660 + miR.338.

6. The method according to claim 1 wherein said miRNA cluster consists in the combination miR.558 + miR.609 + miR.603 + miR.520f + miR.220a + miR.519b + miR.130a + miR.26a + miR.29a + miR.362 + miR.369.3p + miR.376a*+ miR.494 + miR.518c + miR.639 + miR.660 + miR.338.

7. The method according to any of claims 1 to 6 which further comprises a step consisting of comparing the expression level of said miRNA cluster determined in the sample of the patient with a reference expression level of said miRNA cluster.

8. The method according to any of claims 1 to 7 wherein said cancer is a colorectal cancer.

## Patentansprüche

1. Ein Verfahren zur Vorhersage der Folgen einer Krebserkrankung bei einem Patienten, umfassend einen Schritt, bestehend aus der Bestimmung des Expressionsniveaus eines miRNA-Clusters in einer Tumorprobe, erhalten aus diesem Patienten, wobei dieses miRNA-Cluster miR.494 enthält.

2. Das Verfahren nach Anspruch 1, wobei dieses miRNA-Cluster aus der Kombination von miR.558 + miR.609 + miR.520f + miR.130a + miR.29a + miR.362 + miR.369.3p + miR.376a. + miR.494 + miR.518c + miR.639 + miR.660 + miR.338 besteht.

3. Das Verfahren nach Anspruch 1, wobei dieses miRNA-Cluster aus der Kombination von miR.558 + miR.609 + miR.603 + miR.520f + miR.130a + miR.29a + miR.362 + miR.369.3p + miR.376a. + miR.494 + miR.518c + miR.639 + miR.660 + miR.338 besteht.

4. Das Verfahren nach Anspruch 1, wobei dieses miRNA-Cluster aus der Kombination von miR.558 + miR.609 + miR.603 + miR.520f + miR.130a + miR.26a + miR.29a + miR.362 + miR.369.3p + miR.376a. + miR.494 + miR.518c + miR.639 + miR.660 + miR.338 besteht.

5. Das Verfahren nach Anspruch 1, wobei dieses miRNA-Cluster aus der Kombination von miR.558 + miR.609 + miR.603 + miR.520f + miR.220a + miR.130a + miR.26a + miR.29a + miR.362 + miR.369.3p + miR.376a. + miR.494 + miR.518c + miR.639 + miR.660 + miR.338 besteht.

6. Das Verfahren nach Anspruch 1, wobei dieses miRNA-Cluster aus der Kombination von miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338 besteht.

7. Das Verfahren nach einem der Ansprüche 1 bis 6, welches weiterhin einen Schritt, bestehend aus dem Vergleich des Expressionsniveaus dieses miRNA-Clusters, wie in der Probe des Patienten bestimmt, mit einem Referenzexpressionslevel dieses miRNA-Clusters, umfasst.

8. Das Verfahren nach einem der Ansprüche 1 bis 7, wobei diese Krebserkrankung ein kolorektaler Krebs ist.

## Revendications

1. Procédé de prévision du résultat d'un cancer chez un patient comprenant une étape consistant à déterminer le niveau d'expression d'une grappe de miARN dans un échantillon de tumeur obtenu à partir dudit patient, dans lequel ladite grappe de miARN comprend du miR.494.

2. Procédé selon la revendication 1 dans lequel ladite grappe de miARN est constituée de la combinaison miR.558 + miR.609 + miR.520f + miR.130a + miR.29a + miR.362 + miR.369.3p + miR.376a + miR.494 + miR.518c + miR.639 + miR.660 + miR.338.

3. Procédé selon la revendication 1 dans lequel ladite grappe de miARN est constituée de la combinaison miR.558 + miR.609 + miR.603 + miR.520f + miR.130a + miR.29a + miR.362 + miR.369.3p + miR.376a + miR.494 + miR.518c + miR.639 + miR.660 + miR.338.

4. Procédé selon la revendication 1 dans lequel ladite grappe de miARN est constituée de la combinaison miR.558 + miR.609 + miR.603 + miR.520f + miR.130a + miR.26a + miR.29a + miR.362 + miR.369.3p + miR.376a + miR.494 + miR.518c + miR.639 + miR.660 + miR.338.

5. Procédé selon la revendication 1 dans lequel ladite grappe de miARN est constituée de la combinaison miR.558 + miR.609 + miR.603 + miR.520f + miR.220a + miR.130a + miR.26a + miR.29a + miR.362 + miR.369.3p + miR.376a + miR.494 + miR.518c + miR.639 + miR.660 + miR.338.

6. Procédé selon la revendication 1 dans lequel ladite grappe de miARN est constituée de la combinaison miR.558 + miR.609 + miR.603 + miR.520f + miR.220a + miR.519b + miR.130a + miR.26a + miR.29a + miR.362 + miR.369.3p + miR.376a* + miR.494 + miR.518c + miR.639 + miR.660 + miR.338.

7. Procédé selon l'une quelconque des revendications 1 à 6 qui comprend en outre une étape consistant à comparer le niveau d'expression de ladite grappe de miARN déterminé dans l'échantillon du patient à un niveau d'expression de référence de ladite grappe de miARN.

8. Procédé selon l'une quelconque des revendications 1 à 7 dans lequel ledit cancer est un cancer colorectal.
